(19) Europäisches Patentamt / European Patent Office / Office européen des brevets

(11) **EP 3 011 035 B1**

(12) **EUROPEAN PATENT SPECIFICATION**

(45) Date of publication and mention
of the grant of the patent:
**13.05.2020  Bulletin 2020/20**

(21) Application number: **14738672.6**

(22) Date of filing: **10.06.2014**

(51) Int Cl.:
*C12N 15/63* (2006.01)     *C12N 15/10* (2006.01)
*C40B 40/08* (2006.01)     *C12N 9/22* (2006.01)

(86) International application number:
**PCT/US2014/041790**

(87) International publication number:
**WO 2014/204723 (24.12.2014 Gazette 2014/52)**

(54) **ASSAY FOR QUANTITATIVE EVALUATION OF TARGET SITE CLEAVAGE BY ONE OR MORE CRISPR-CAS GUIDE SEQUENCES**

TEST ZUR QUANTITATIVEN BEWERTUNG DER ZIELSTELLENSPALTUNG DURCH EINE ODER MEHRERE CRISPR-CAS FÜHRUNGSSEQUENZEN

TEST POUR L'ÉVALUATION QUANTITATIVE DU CLIVAGE DE SITES CIBLES PAR UNE OU PLUSIEURS SÉQUENCES GUIDES DU SYSTÈME CRISPR-CAS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2013   US 201361836123 P**
**12.12.2013   US 201361915397 P**

(43) Date of publication of application:
**27.04.2016   Bulletin 2016/17**

(73) Proprietors:
- **The Broad Institute, Inc.**
  **Cambridge, MA 02142 (US)**
- **Massachusetts Institute of Technology**
  **Cambridge, MA 02139 (US)**
- **The Brigham and Women's Hospital, Inc.**
  **Boston, MA 02115 (US)**

(72) Inventors:
- **ZHANG, Feng**
  **Cambrige, MA 02139 (US)**
- **EBERT, Benjamin, Levine**
  **Brookline, MA 02445 (US)**
- **HECKL, Dirk**
  **29352 Adelheidsdorf (DE)**

(74) Representative: **Icely, Dominic Michael**
**The IP Asset Partnership Limited**
**Prama House**
**267 Banbury Road**
**Oxford OX2 7HT (GB)**

(56) References cited:
- **GAJ THOMAS ET AL: "ZFN, TALEN, and CRISPR/Cas-based methods for genome engineering", TRENDS IN BIOTECHNOLOGY, ELSEVIER PUBLICATIONS, CAMBRIDGE, GB, vol. 31, no. 7, 9 May 2013 (2013-05-09), pages 397-405, XP028571313, ISSN: 0167-7799, DOI: 10.1016/J.TIBTECH.2013.04.004**
- **JANSSEN K P ET AL: "Mouse models of K-ras-initiated carcinogenesis", BBA - REVIEWS ON CANCER, ELSEVIER SCIENCE BV, AMSTERDAM, NL, vol. 1756, no. 2, 25 November 2005 (2005-11-25), pages 145-154, XP027829577, ISSN: 0304-419X [retrieved on 2005-11-25]**
- **B. C. LEWIS: "The c-myc and PyMT oncogenes induce different tumor types in a somatic mouse model for pancreatic cancer", GENES & DEVELOPMENT, vol. 17, no. 24, 15 December 2003 (2003-12-15), pages 3127-3138, XP55049590, ISSN: 0890-9369, DOI: 10.1101/gad.1140403**
- **L. CONG ET AL: "Multiplex Genome Engineering Using CRISPR/Cas Systems", SCIENCE, vol. 339, no. 6121, 3 January 2013 (2013-01-03), pages 819-823, XP055102030, ISSN: 0036-8075, DOI: 10.1126/science.1231143**
- **P. MALI ET AL: "RNA-Guided Human Genome Engineering via Cas9", SCIENCE, vol. 339, no. 6121, 3 January 2013 (2013-01-03), pages 823-826, XP055111247, ISSN: 0036-8075, DOI: 10.1126/science.1232033**

**(Cont. next page)**

- **LOMBARDO ANGELO ET AL: "Gene editing in human stem cells using zinc finger nucleases and integrase-defective lentiviral vector delivery",** NATURE BIOTECHNOLOGY, NATURE PUBLISHING GROUP, NEW YORK, NY, US, vol. 25, no. 11, 1 November 2007 (2007-11-01), pages 1298-1306, XP002465562, ISSN: 1087-0156, DOI: 10.1038/NBT1353
- **L. CONG ET AL: "Supplementary Material to : Multiplex Genome Engineering Using CRISPR/Cas Systems",** SCIENCE, vol. 339, no. 6121, 3 January 2013 (2013-01-03), pages 1-25, XP002730884, ISSN: 0036-8075, DOI: 10.1126/science.1231143
- **WANG HAOYI ET AL: "One-Step Generation of Mice Carrying Mutations in Multiple Genes by CRISPR/Cas-Mediated Genome Engineering",** CELL, CELL PRESS, US, vol. 153, no. 4, 2 May 2013 (2013-05-02), pages 910-918, XP028538358, ISSN: 0092-8674, DOI: 10.1016/J.CELL.2013.04.025
- **SHALEM OPHIR ET AL: "Genome-Scale CRISPR-Cas9 Knockout Screening in Human Cells",** January 2014 (2014-01), SCIENCE (WASHINGTON D C), VOL. 343, NR. 6166, PAGE(S) 84-87, XP002723432, ISSN: 0036-8075(print) the whole document
- **HSU PATRICK D ET AL: "Development and Applications of CRISPR-Cas9 for Genome Engineering",** CELL, vol. 157, no. 6, 5 June 2014 (2014-06-05), pages 1262-1278, XP028849523, ISSN: 0092-8674, DOI: 10.1016/J.CELL.2014.05.010
- **LUKE A. GILBERT ET AL: "CRISPR-Mediated Modular RNA-Guided Regulation of Transcription in Eukaryotes",** CELL, vol. 154, no. 2, 1 July 2013 (2013-07-01), pages 442-451, XP055115843, ISSN: 0092-8674, DOI: 10.1016/j.cell.2013.06.044
- **MALINA ABBA ET AL: "Repurposing CRISPR/Cas9 for in situ functional assays",** 1 December 2013 (2013-12-01), GENES & DEVELOPMENT, VOL. 27, NR. 23, PAGE(S) 2602-2614 ISSN: 0890-9369(print)
- **N. Manjunath ET AL: "Newer Gene Editing Technologies toward HIV Gene Therapy",** Viruses, vol. 5, no. 11, 14 November 2013 (2013-11-14), pages 2748-2766, XP055110267, DOI: 10.3390/v5112748
- **Hirotaka Ebina ET AL: "Harnessing the CRISPR/Cas9 system to disrupt latent HIV-1 provirus",** Scientific Reports, vol. 3, 26 August 2013 (2013-08-26), XP055110157, DOI: 10.1038/srep02510
- **MANNELL HANNA ET AL: "Targeted Endothelial Gene Delivery by Ultrasonic Destruction of Magnetic Microbubbles Carrying Lentiviral Vectors",** PHARMACEUTICAL RESEARCH (DORDRECHT), vol. 29, no. 5, May 2012 (2012-05), pages 1282-1294, ISSN: 0724-8741(print)

**Description**

FIELD OF THE INVENTION

The present disclosure generally relates to the delivery, engineering, optimization and therapeutic applications of systems, methods, compositions and vectors used for the control of gene expression involving sequence targeting, such as genome perturbation or gene-editing, that relate to Clustered Regularly Interspaced Short Palindromic Repeats (CRISPR) and components thereof.

STATEMENT AS TO FEDERALLY SPONSORED RESEARCH

This invention was made with government support under the NIH Pioneer Award (1DP1MH100706) and NIH (P01 CA108631) grant awarded by the National Institutes of Health. The government has certain rights in the invention.

BACKGROUND OF THE INVENTION

Recent advances in genome sequencing techniques and analysis methods have significantly accelerated the ability to catalog and map genetic factors associated with a diverse range of biological functions and diseases. Precise genome targeting technologies are needed to enable systematic reverse engineering of causal genetic variations by allowing selective perturbation of individual genetic elements, as well as to advance synthetic biology, biotechnological, and medical applications. Although genome-editing techniques such as designer zinc fingers, transcription activator-like effectors (TALEs), or homing meganucleases are available for producing targeted genome perturbations, there remains a need for new genome engineering technologies that are affordable, easy to set up, scalable, and amenable to targeting multiple positions within the eukaryotic genome.

SUMMARY OF THE INVENTION

The CRISPR-Cas system does not require the generation of customized proteins to target specific sequences but rather a single Cas enzyme can be programmed by a short RNA molecule to recognize a specific DNA target. Adding the CRISPR-Cas system to the repertoire of genome sequencing techniques and analysis methods may significantly simplify the methodology and accelerate the ability to catalog and map genetic factors associated with a diverse range of biological functions and diseases. To utilize the CRISPR-Cas system effectively for genome editing without deleterious effects, it is critical to understand aspects of engineering, optimization and tissue/organ specific delivery of these genome engineering tools, which are aspects of the claimed invention.

There exists a pressing need for alternative and robust systems and techniques for sequence targeting with a wide array of applications. Aspects of this invention address this need and provides related advantages. An exemplary CRISPR complex comprises a CRISPR enzyme complexed with a guide sequence hybridized to a target sequence within the target polynucleotide. The guide sequence is linked to a tracr mate sequence, which in turn hybridizes to a tracr sequence.

In one aspect, the disclosure provides methods for using one or more elements of a CRISPR system. The CRISPR complex provides an effective means for modifying a target polynucleotide. The CRISPR complex has a wide variety of utilities including modifying (e.g., deleting, inserting, translocating, inactivating, activating) a target polynucleotide in a multiplicity of cell types in various tissues and organs. As such the CRISPR complex has a broad spectrum of applications in, e.g., gene or genome editing, gene therapy, generation of cell and animal model systems, drug discovery, drug screening, disease diagnosis, and prognosis.

Aspects of the disclosure relate to the CRISPR-Cas system having the utility of engineering primary cells that recapitulate the genetic complexity of human disease. This complexity arises from the interaction of multiple genetic lesions, which is particularly true in the case of proliferative disorders like cancer.

The disclosure comprehends a method of inducing a proliferative condition in a an organism comprising: isolating a first population of cells from the organism, transducing the first population of cells with a non-naturally occurring or engineered composition comprising a vector system comprising one or more vectors comprising I. a first regulatory element operably linked to a CRISPR-Cas system chimeric RNA (chiRNA) polynucleotide sequence, wherein the polynucleotide sequence comprises (a) three or more guide sequences capable of hybridizing to three or more target sequences in genome of the organism, (b) a tracr mate sequence, and (c) a tracr sequence, and II. a second regulatory element operably linked to an enzyme-coding sequence encoding a CRISPR enzyme comprising at least one or more nuclear localization sequences (NLSs), wherein (a), (b) and (c) are arranged in a 5' to 3' orientation, wherein components I and II are located on the same or different vectors of the system, wherein when transcribed, the tracr mate sequence hybridizes to the tracr sequence and the guide sequence direct sequence-specific binding of CRISPR complexes to the target sequence, wherein the CRISPR complex comprises the CRISPR enzyme complexed with (1) the guide sequence that is hybridizableto the target sequence, and (2) the tracr mate sequence that is hybridized to the tracr sequence, wherein the CRISPR enzyme alters the genome of the first population of cells to obtain a second population of cells, and transplanting the second population of cells into the organism thereby inducing the proliferative condition. In an embodiment may be a cancer, particularly a myeloid malignancy. In another embodiment the first population of cells may be a population of stem cells, particulary a population of hematopoietic stem and progenitor cells. In a preferred embodiment the vector system is a lentiviral vector system. In further embodiments the first regulatory element may be a Pol III promoters such as a U6 promoter and the second regulatory element may be an EFS promoter. In a further aspect the polynucleotide sequence comprises 4, 5, 6, 7, 8, 9 or 10 guide sequences. The disclosure also comprehends that the target sequences may be associated with tumor suppressor genes. In one embodiment, the organism is a mouse.

The disclosure also comprehends a method of interrogating function of one or more genes in a proliferative condition in an organism comprising: inducing the proliferative condition by a method comprising isolating a first population of cells from the organism, transducing the first population of cells with a non-naturally occurring or engineered composition comprising a vector system comprising one or more vectors comprising I. a first regulatory element operably linked to a CRISPR-Cas system chiRNA polynucleotide sequence, wherein the polynucleotide sequence comprises (a) three or more guide sequences capable of hybridizing to three or more target sequences in genome of the organism, (b) a tracr mate sequence, and (c) a tracr sequence, and II. a second regulatory element operably linked to an enzyme-coding sequence encoding a CRISPR enzyme comprising at least one or more NLSs, wherein (a), (b) and (c) are arranged in a 5' to 3' orientation, wherein components I and II are located on the same or different vectors of the system, wherein when transcribed, the tracr mate sequence hybridizes to the tracr sequence and the guide sequence direct sequence-specific binding of CRISPR complexes to the target sequence, wherein the CRISPR complex comprises the CRISPR enzyme complexed with (1) the guide sequence that is hybridizableto the target sequence, and (2) the tracr mate sequence that is hybridized to the tracr sequence, wherein the CRISPR enzyme alters the genome of the first population of cells to obtain a second population of cells, transplanting the second population of cells into the organism thereby inducing the proliferative condition; and determining changes in expression of the one or more genes in the proliferative condition thereby interrogating the function of one or more genes. In an embodiment may be a cancer, particularly a myeloid malignancy. In another embodiment the first population of cells may be a population of stem cells, particulary a population of hematopoietic stem and progenitor cells. In a preferred embodiment the vector system is a lentiviral vector system. In further embodiments the first regulatory element may be a Pol III promoter such as a U6 or a H1 promoter and the second regulatory element may be an EFS promoter. In a further aspect the polynucleotide sequence comprises 4, 5, 6, 7, 8, 9 or 10 guide sequences. The disclosure also comprehends that the target sequences may be associated with tumor suppressor genes. In one embodiment, the organism is a mouse.

The genes that are useful for this invention are any of the genes that are recurrently mutated in cancer. Embodiments herein are relevant for any gene(s) with loss-of-function mutation(s) in cancer. This is sometimes referred to as a tumor suppressor, but probably more accurate to refer to gene(s) with loss-of-function mutation(s). Loss-of-function mutation(s) result in the gene product having less or no function. When the allele has a complete loss of function (null allele) it is often called an amorphic mutation. Phenotypes associated with such mutations are most often recessive. (Exceptions are when the organism is haploid, or when the reduced dosage of a normal gene product is not enough for a normal phenotype (this is called haploinsufficiency).) The CRISPR-Cas9 system of the instant disclosure and the utilization of the system in the practice of the instant invention also can be used to introduce specific mutations, e.g., cut or nick, insertion, deletion, substitution. The list of genes with loss-of-function mutation is large - in the hundreds. Indeed, the Bioinformatics and Systems Medicine Laboratory of Vanderbilt University Medical Center has a website, the TS GENE TUMOR SUPPRESSOR GENE DATABASE, http://bioinfo.mc.vanderbilt.edu/TSGene/index.html, that includes 716 human genes (637 coding and 79 non-coding genes), 628 mouse genes, and 567 rat genes. The invention is applicable to each of those genes. The invention is applicable to any gene for which loss of function mutation(s) leads to neoplasm or tumor growth or cancer; and the skilled person well appreciates loss of function mutation(s) in cancer (as well as gain of function mutations in cancer and the invention can be analogously applicable to such mutations). See Komarova Journal of Statistical Physics, July 2007, Volume 128, Issue 1-2, pp 413-446, "Loss- and Gain-of-Function Mutations in Cancer: Mass-action, Spatial and Hierarchical Models." The term "tumor suppressor" is herein used as understood in the art, namely to include loss of function mutation(s) in or involved in or that leads to or contributes to or is a factor in neoplasm or tumor growth or cancer, including tumor formation and/or spread and/or Hyperplasia (altered cell divides in an uncontrolled manner leading to an excess of cells in that region of the tissue) and/or Dysplasia (additional genetic changes in the hyperplastic cells lead to increasingly abnormal growth; cells and the the tissue no longer look normal; cells and the tissue may become disorganized) and/or Carcinoma in situ (additional changes make the cells and tissues appear even more abnormal; cells are spread over a larger area and the region of the tissue involved primarily contains altered cells; cells often 'regress' or become more primitive in their capabilities, e.g., a liver cell that no longer makes liver-specific proteins; cells may be de-differentiated or anaplastic and/or 'benign tumor(s)' and/or Cancer (including Malignant tumor(s)).

The disclosure provides a non-naturally occurring or engineered composition for generating an *in vivo, in vitro* or *ex vivo* proliferative condition comprising multiple genetic lesions in a population of cells comprising a viral vector such as a lentiviral vector system comprising one or more lentiviral vectors comprising I. a Pol III promoter operably linked to a CRISPR-Cas system chiRNA polynucleotide sequence, wherein the polynucleotide sequence comprises (a) three or more guide sequences capable of hybridizing to three or more target sequences in genome of the organism, (b) a tracr mate sequence, and (c) a tracr sequence, and II. a EFS promoter operably linked to an enzyme-coding sequence encoding a CRISPR enzyme comprising at least one or more NLSs, wherein (a), (b) and (c) are arranged in a 5' to 3' orientation, wherein components I and II are located on the same or different vectors of the system, wherein when transcribed, the tracr mate sequence hybridizes to the tracr sequence and the guide sequence direct sequence-specific binding of CRISPR complexes to the target sequence, and wherein the CRISPR complex comprises the CRISPR enzyme complexed with (1) the guide sequence that is hybridizable to the target sequence, and (2) the tracr mate sequence that is hybridized to the tracr sequence. In an embodiment may be a cancer, particularly a myeloid malignancy. In another embodiment the first population of cells may be a population of stem cells, particulary a population of hematopoietic stem and progenitor cells.17. The composition of claim 12, wherein the polynucleotide sequence comprises 4, 5, 6, 7, 8, 9 or 10 guide sequences. In a further aspect the polynucleotide sequence comprises 4, 5, 6, 7, 8, 9 or 10 guide sequences. The disclosure also comprehends that the target sequences may be associated with tumor suppressor genes.

Aspects of the disclosure relate to Cas9 enzymes having improved target specificity in a CRISPR-Cas9 system having guide RNAs having optimal activity, smaller in length than wild-type Cas9 enzymes and nucleic acid molecules coding therefor, and chimeric Cas9 enzymes, as well as methods of improving the target specificity of a Cas9 enzyme or of designing a CRISPR-Cas9 system comprising designing or preparing guide RNAs having optimal activity and/or selecting or preparing a Cas9 enzyme having a smaller size or length than wild-type Cas9 whereby packaging a nucleic acid coding therefor into a delivery vector is advanced as there is less coding therefor in the delivery vector than for wild-type Cas9, and/or generating chimeric Cas9 enzymes.

Also provided are uses of the present sequences, vectors, enzymes or systems, in medicine. Also provided are uses of the same in gene or genome editing.

In an additional aspect of the disclosure, a Cas9 enzyme may comprise one or more mutations and may be used as a generic DNA binding protein with or without fusion to a functional domain. The mutations may be artificially introduced mutations or gain- or loss-of-function mutations. The mutations may include but are not limited to mutations in one of the catalytic domains (D10 and H840). Further mutations have been characterized and may be used in one or more compositions of the disclosure. In one aspect of the disclosure, the mutated Cas9 enzyme may be fused to a protein domain, e.g., such as a transcriptional activation domain. In one aspect of the disclosure, the transcriptional activation domain may be VP64. In other aspects of the disclosure, the transcriptional repressor domain may be KRAB or SID4X. Other aspects relate to the mutated Cas 9 enzyme being fused to domains which include but are not limited to a transcriptional activator, repressor, a recombinase, a transposase, a histone remodeler, a DNA methyltransferase, a cryptochrome, a light inducible/controllable domain or a chemically inducible/controllable domain. In one aspect, the Cas enzyme has at least one or more mutations in a catalytic domain. In one aspect, the Cas enzyme is a nickase.

In a further embodiment, the disclosure provides for methods to generate mutant tracrRNA and direct repeat sequences or mutant chimeric guide sequences that allow for enhancing performance of these RNAs in cells. Aspects also provide for selection of said sequences.

Aspects also provide for methods of simplifying the cloning and delivery of components of the CRISPR complex. In the preferred embodiment of the disclosure, a suitable promoter, such as a Pol III promoter, e.g., a H1 or a U6 promoter, is amplified with a DNA oligo and positioned contiguous to and upstream of a sequence encoding the guide RNA. The resulting PCR product can then be transfected into cells to drive expression of the guide RNA. Aspects of the disclosure also relate to the guide RNA being transcribed *in vitro* or ordered from a synthesis company and directly transfected.

In one aspect, the disclosure provides for methods to improve activity by using a more active polymerase. In one aspect, a T7 promoter may be inserted contiguous to and upstream of a sequence encoding a guide RNA. In a preferred embodiment, the expression of guide RNAs under the control of the T7 promoter is driven by the expression of the T7 polymerase in the cell. In an advantageous embodiment, the cell is a eukaryotic cell. In a preferred embodiment the eukaryotic cell is a human cell. In a more preferred embodiment the human cell is a patient specific cell.

In one aspect, the disclosure provides for methods of reducing the toxicity of Cas enzymes. In certain aspects, the Cas enzyme is any Cas9 as described herein, for instance any naturally-occurring bacterial Cas9 as well as any chimaeras, mutants, homologs or orthologs. In one aspect, the Cas enzyme is a nickase. In a preferred embodiment, the Cas9 is delivered into the cell in the form of mRNA. This allows for the transient expression of the enzyme thereby reducing toxicity. In another embodiment, the Cas9 is delivered into the cell in the nucleotide construct that encodes and expresses the Cas9 enzyme. In another preferred embodiment, the disclosure also provides for methods of expressing Cas9 under the control of an inducible promoter the constructs used therein.

In another aspect, the disclosure provides for methods of improving the *in vivo* applications of the CRISPR-Cas system. In the preferred embodiment, the Cas enzyme is wildtype Cas9 or any of the modified versions described herein, including any naturally-occurring bacterial Cas9 as well as any chimaeras, mutants, homologs or orthologs. In one aspect, the Cas enzyme is a nickase. An advantageous aspect provides for the selection of Cas9 homologs that are easily packaged into viral vectors for delivery. Cas9 orthologs typically share the general organization of 3-4 RuvC domains and a HNH domain. The 5' most RuvC domain cleaves the non-complementary strand, and the HNH domain cleaves the complementary strand. All notations are in reference to the guide sequence.

The catalytic residue in the 5' RuvC domain is identified through homology comparison of the Cas9 of interest with other Cas9 orthologs (from S. pyogenes type II CRISPR locus, S. thermophilus CRISPR locus 1, S. thermophilus CRISPR locus 3, and Franciscilla novicida type II CRISPR locus), and the conserved Asp residue is mutated to alanine to convert Cas9 into a complementary-strand nicking enzyme. Similarly, the conserved His and Asn residues in the HNH domains are mutated to Alanine to convert Cas9 into a non-complementary-strand nicking enzyme.

In some embodiments, the CRISPR enzyme is a type I or III CRISPR enzyme, preferably a type II CRISPR enzyme. This type II CRISPR enzyme may be any Cas enzyme. A Cas enzyme may be identified Cas9 as this can refer to the general class of enzymes that share homology to the biggest nuclease with multiple nuclease domains from the type II CRISPR system. Most preferably, the Cas9 enzyme is from, or is derived from, spCas9 or saCas9. By derived, Applicants mean that the derived enzyme is largely based, in the sense of having a high degree of sequence homology with, a wildtype enzyme, but that it has been mutated (modified) in some way as described herein

It will be appreciated that the terms Cas and CRISPR enzyme are generally used herein interchangeably, unless otherwise apparent. As mentioned above, many of the residue numberings used herein refer to the Cas9 enzyme from the type II CRISPR locus in *Streptococcus pyogenes* (annotated alternatively as SpCas9 or spCas9). However, it will be appreciated that this disclosure includes many more Cas9s from other species of microbes, such as SpCas9 derived from S. pyogenes, SaCas9 derived from S. aureus, St1Cas9 derived from S. thermophilus and so forth. Further examples are provided herein.

An example of a codon optimized sequence, in this instance optimized for humans (i.e. being optimized for expression in humans) is provided herein, e.g., see the SaCas9 human codon optimized sequence. Whilst this is preferred, it will be appreciated that other examples are possible and codon optimization for a host species other than human, or for codon optimization for specific organs such as the brain, is known.

In further embodiments, the disclosure provides for methods of enhancing the function of Cas9 by generating chimeric Cas9 proteins. These methods may comprise fusing N-terminal fragments of one Cas9 homolog with C-terminal fragments of another Cas9 homolog. These methods also allow for the selection of new properties displayed by the chimeric proteins.

It will be appreciated that in the present methods, where the organism is an animal or a plant, the modification may occur *ex vivo* or *in vitro,* for instance in a cell culture and in some instances not *in vivo.* In other embodiments, it may occur *in vivo.*

In one aspect, the disclosure provides a method of modifying an organism or a non-human organism by manipulation of a target sequence in a genomic locus of interest comprising: delivering a non-naturally occurring or engineered composition comprising:

A) -

I. a CRISPR-Cas system chimeric RNA (chiRNA) polynucleotide sequence, wherein the polynucleotide sequence comprises:

(a) a guide sequence capable of hybridizing to a target sequence in a eukaryotic cell,
(b) a tracr mate sequence, and
(c) a tracr sequence, and

II. a polynucleotide sequence encoding a CRISPR enzyme comprising at least one or more nuclear localization sequences,

wherein (a), (b) and (c) are arranged in a 5' to 3' orientation,
wherein when transcribed, the tracr mate sequence hybridizes to the tracr sequence and the guide sequence directs sequence-specific binding of a CRISPR complex to the target sequence, and
wherein the CRISPR complex comprises the CRISPR enzyme complexed with (1) the guide sequence that is hybridizable to the target sequence, and (2) the tracr mate sequence that is hybridized to the tracr sequence and the polynucleotide sequence encoding a CRISPR enzyme is DNA or RNA, or
(B)

I. polynucleotides comprising:

 (a) a guide sequence capable of hybridizing to a target sequence in a prokaryotic cell, and
 (b) at least one or more tracr mate sequences,

II. a polynucleotide sequence encoding a CRISPR enzyme, and
III. a polynucleotide sequence comprising a tracr sequence,

wherein when transcribed, the tracr mate sequence hybridizes to the tracr sequence and the guide sequence directs sequence-specific binding of a CRISPR complex to the target sequence, and
wherein the CRISPR complex comprises the CRISPR enzyme complexed with (1) the guide sequence that is hybridizableto the target sequence, and (2) the tracr mate sequence that is hybridized to the tracr sequence, and the polynucleotide sequence encoding a CRISPR enzyme is DNA or RNA.

Any or all of the polynucleotide sequence encoding a CRISPR enzyme, guide sequence, tracr mate sequence or tracr sequence, may be RNA, DNA or a combination of RNA and DNA. In one aspect, the polynucleotides comprising the sequence encoding a CRISPR enzyme, the guide sequence, tracr mate sequence or tracr sequence are RNA. In one aspect, the polynucleotides comprising the sequence encoding a CRISPR enzyme, the guide sequence, tracr mate sequence or tracr sequence are DNA. In one aspect, the polynucleotides are a mixture of DNA and RNA, wherein some of the polynucleotides comprising the sequence encoding one or more of the CRISPR enzyme, the guide sequence, tracr mate sequence or tracr sequence are DNA and some of the polynucleotides are RNA. In one aspect, the polynucleotide comprising the sequence encoding the CRISPR enzyme is a DNA and the guide sequence, tracr mate sequence or tracr sequence are RNA. The one or more polynucleotides comprising the sequence encoding a CRISPR enzyme, the guide sequence, tracr mate sequence or tracr sequence may be delivered via nanoparticles, exosomes, microvesciles, or a gene-gun.

It will be appreciated that where reference is made to a polynucleotide, where that polynucleotide is RNA and is said to 'comprise' a feature such as a tracr mate sequence, the RNA sequence includes the feature. Where the polynucleotide is DNA and is said to comprise a feature such as a tracr mate sequence, the DNA sequence is or can be transcribed into the RNA that comprises the feature at issue. Where the feature is a protein, such as the CRISPR enzyme, the DNA or RNA sequence referred to is, or can be, translated (and in the case of DNA transcribed first). Furthermore, in cases where an RNA encoding the CRISPR enzyme is provided to a cell, it is understood that the RNA is capable of being translated by the cell into which it is delivered.

Accordingly, in certain embodiments the disclosure provides method of modifying an organism, e.g., mammal including human or a non-human mammal or organism by manipulation of a target sequence in a genomic locus of interest comprising delivering a non-naturally occurring or engineered composition comprising a viral or plasmid vector system comprising one or more viral or plasmid vectors operably encoding a composition for expression thereof, wherein the composition comprises: (A) a non-naturally occurring or engineered composition comprising a vector system comprising one or more vectors comprising I. a first regulatory element operably linked to a CRISPR-Cas system chimeric RNA (chiRNA) polynucleotide sequence, wherein the polynucleotide sequence comprises (a) a guide sequence capable of hybridizing to a target sequence in a eukaryotic cell, (b) a tracr mate sequence, and (c) a tracr sequence, and II. a second regulatory element operably linked to an enzyme-coding sequence encoding a CRISPR enzyme comprising at least one or more nuclear localization sequences (or optionally at least one or more nuclear localization sequences as some embodiments can involve no NLS), wherein (a), (b) and (c) are arranged in a 5' to 3' orientation, wherein components I and II are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridizableto the target sequence, and (2) the tracr mate sequence that is hybridized to the tracr sequence, or (B) a non-naturally occurring or engineered composition comprising a vector system comprising one or more vectors comprising I. a first regulatory element operably linked to (a) a guide sequence capable of hybridizing to a target sequence in a prokaryotic cell, and (b) at least one or more tracr mate sequences, II. a second regulatory element operably linked to an enzyme-coding sequence encoding a CRISPR enzyme, and III. a third regulatory element operably linked to a tracr sequence, wherein components I, II and III are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridizableto the target sequence, and (2) the tracr mate sequence that is hybridized to the tracr sequence. In one embodiment, the vector system comprises a viral vector system, e.g., an AAV vector or AAV vector system or a lentivirus-derived vector system. In one aspect, the Cas enzyme is a nickase.

Preferably, the vector is a viral vector, such as a lenti- or baculo- or preferably adeno-viral/adeno-associated viral vectors, but other means of delivery are known (such as yeast systems, microvesicles, gene guns/means of attaching vectors to gold nanoparticles) and are provided. In some embodiments, one or more of the viral or plasmid vectors may be delivered via nanoparticles, exosomes, microvescicles, or a gene-gun.

By manipulation of a target sequence, Applicants mean the alteration of the target sequence, which may include the epigenetic manipulation of a target sequence. This epigenetic manipulation may be of the chromatin state of a target sequence, such as by modification of the methylation state of the target sequence (i.e. addition or removal of methylation or methylation patterns or CpG islands), histone modification, increasing or reducing accessibility to the target sequence, or by promoting or reducing 3D folding.

It will be appreciated that where reference is made to a method of modifying an organism or mammal including human or a non-human mammal or organism by manipulation of a target sequence in a genomic locus of interest, this may apply to the organism (or mammal) as a whole or just a single cell or population of cells from that organism (if the organism is multicellular). In the case of humans, for instance, Applicants envisage, *inter alia,* a single cell or a population of cells and these may preferably be modified *ex vivo* and then re-introduced. In this case, a biopsy or other tissue or biological fluid sample may be necessary. Stem cells are also particularly preferred in this regard. But, of course, *in vivo* embodiments are also envisaged.

In certain embodiments the disclosure provides a method of treating or inhibiting a condition caused by a defect in a target sequence in a genomic locus of interest in an organism (e.g., mammal or human) or a non-human organism (e.g., mammal, such as a mouse) in need thereof comprising modifying the organism or a non-human organism by manipulation of the target sequence and wherein the condition is susceptible to treatment or inhibition by manipulation of the target sequence comprising providing treatment comprising: delivering a non-naturally occurring or engineered composition comprising an adenoviral, an AAV or lentiviral vector system comprising one or more vectors comprising operably encoding a composition for expression thereof, wherein the target sequence is manipulated by the composition when expressed, wherein the composition comprises: (A) a non-naturally occurring or engineered composition comprising a vector system comprising one or more vectors comprising I. a first regulatory element operably linked to a CRISPR-Cas system chimeric RNA (chiRNA) polynucleotide sequence, wherein the polynucleotide sequence comprises (a) a guide sequence capable of hybridizing to a target sequence in a eukaryotic cell, (b) a tracr mate sequence, and (c) a tracr sequence, and II. a second regulatory element operably linked to an enzyme-coding sequence encoding a CRISPR enzyme comprising at least one or more nuclear localization sequences (or optionally at least one or more nuclear localization sequences as some embodments can involve no NLS) wherein (a), (b) and (c) are arranged in a 5' to 3' orientation, wherein components I and II are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridizable to the target sequence, and (2) the tracr mate sequence that is hybridized to the tracr sequence, or (B) a non-naturally occurring or engineered composition comprising a vector system comprising one or more vectors comprising I. a first regulatory element operably linked to (a) a guide sequence capable of hybridizing to a target sequence in a prokaryotic cell, and (b) at least one or more tracr mate sequences, II. a second regulatory element operably linked to an enzyme-coding sequence encoding a CRISPR enzyme, and III. a third regulatory element operably linked to a tracr sequence, wherein components I, II and III are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridizable to the target sequence, and (2) the tracr mate sequence that is hybridized to the tracr sequence. In one embodiment, the vector system comprises a viral vector system, e.g., an AAV vector or AAV vector system or a lentivirus-derived vector system. In one embodiment, the Cas enzyme is a nickase.

Some methods can include inducing expression. In some methods the organism or subject is a eukaryote (including mammal including human) or a non-human eukaryote or a non-human animal or a non-human mammal. In some methods the organism or subject is a plant In some methods the organism or subject is a mammal or a non-human mammal. In some methods the organism or subject is algae. In some methods the viral vector is an AAV. In some methods the CRISPR enzyme is a Cas9. In some methods the CRISPR enzyme copmprises one or more mutations in one of the catalytic domains. In some methods the CRISPR enzyme is a Cas9 nickase. In some methods the expression of the guide sequence is under the control of the T7 promoter that is driven by the expression of T7 polymerase. In some methods the expression of the guide sequence is under the control of a U6 promoter.

By manipulation of a target sequence, Applicants mean the alteration of the target sequence, which may include the epigenetic manipulation of a target sequence. This epigenetic manipulation may be of the chromatin state of a target sequence, such as by modification of the methylation state of the target sequence (i.e. addition or removal of methylation or methylation patterns or CpG islands), histone modification, increasing or reducing accessibility to the target sequence, or by promoting or reducing 3D folding.

It will be appreciated that where reference is made to a method of modifying an organism or a non-human organism by manipulation of a target sequence in a genomic locus of interest, this may apply to the organism as a whole or just a single cell or population of cells from that organism (if the organism is multicellular). In the case of humans, for instance, Applicants envisage, *inter alia*, a single cell or a population of cells and these may preferably be modified *ex vivo* and then re-introduced. In this case, a biopsy or other tissue or biological fluid sample may be necessary. Stem cells are also particularly preferred in this regard. But, of course, *in vivo* embodiments are also envisaged.

In preferred embodiments, the disclosure provides methods and compositions related to generating models to study proliferative disorders. These models maybe encompassed in non-human organisms, e.g. mouse, rat, primates etc. Examples of such proliferative disorders or cancers include, but are not limited to, a carcinoma, for example a carcinoma of the bladder, breast, colon (e.g. colorectal carcinomas such as colon adenocarcinoma and colon adenoma), kidney, epidermis, liver, lung, for example adenocarcinoma, small cell lung cancer and non-small cell lung carcinomas, oesophagus, gall bladder, ovary, pancreas e.g. exocrine pancreatic carcinoma, stomach, cervix, thyroid, prostate, gastrointestinal system, e.g. gastrointestinal stromal tumours, or skin, for example squamous cell carcinoma; a hematopoieitic tumour of lymphoid lineage, for example leukaemia, acute lymphocytic leukaemia, chronic lymphocytic leukaemia, B-cell lymphoma (such as diffuse large B cell lymphoma), T-cell lymphoma, Hodgkin's lymphoma, non-Hodgkin's lymphoma, hairy cell lymphoma, or Burkitt's lymphoma; a hematopoieitic tumour of myeloid lineage, including, but not limited to acute myeloid leukaemia, chronic myeloid leukaemias, myelogenous leukaemias, and Imatinib sensitive and refractory chronic myelogenous leukaemias, myelodysplastic syndrome, Bortezomib sensitive and refractory multiple myeloma, myeloproliferative disease or promyelocytic leukaemia; thyroid follicular cancer; a tumour of mesenchymal origin, for example fibrosarcoma or rhabdomyosarcoma; a tumour of the central or peripheral nervous system, for example astrocytoma, neuroblastoma, glioma (a High grade glioma) or schwannoma; melanoma (e.g. malignant or metastatic melanoma); seminoma; teratocarcinoma; osteosarcoma; keratoacanthoma; thyroid follicular cancer; or Kaposi's sarcoma. A further example of a tumour of mesenchymal origin is Ewing's sarcoma.

One sub-group of cancers includes a carcinoma, for example a carcinoma of the bladder, breast, colon (e.g. colorectal carcinomas such as colon adenocarcinoma and colon adenoma), kidney, epidermis, liver, lung, for example adenocarcinoma, small cell lung cancer and non-small cell lung carcinomas, gall bladder, ovary, pancreas e.g. exocrine pancreatic carcinoma, stomach, thyroid, prostate, gastrointestinal system, e.g. gastrointestinal stromal tumours, or skin, for example squamous cell carcinoma; a hematopoieitic tumour of lymphoid lineage, for example leukaemia, acute lymphocytic leukaemia, chronic lymphocytic leukaemia, B-cell lymphoma (such as diffuse large B cell lymphoma), T-cell lymphoma, Hodgkin's lymphoma, non-Hodgkin's lymphoma, hairy cell lymphoma, or Burkitt's lymphoma; a hematopoieitic tumour of myeloid lineage, including acute myeloid leukaemia, chronic myeloid leukaemias, myelogenous leukaemias, and Imatinib sensitive and refractory chronic myelogenous leukaemias, myelodysplastic syndrome, Bortezomib sensitive and refractory multiple myeloma, myeloproliferative disease or promyelocytic leukaemia; thyroid follicular cancer; a tumour of mesenchymal origin, for example fibrosarcoma or rhabdomyosarcoma; a tumour of the central or peripheral nervous system, for example astrocytoma, glioma (a High grade glioma); melanoma (e.g. malignant or metastatic melanoma); osteosarcoma; or thyroid follicular cancer. A further example of a tumour of mesenchymal origin is Ewing's sarcoma.

One group of cancers consists of solid tumours selected from metastatic breast cancer which is HER2 positive; adenocarcinoma of the prostate; metastatic melanoma; non-small cell carcinoma of the lung (NSCLC); small cell carcinoma of the lung (SCLC); high grade gliomas; gastrointestinal stromal tumors (GIST); colorectal cancer; glioblastoma; melanoma; metastatic thyroid cancer; prostate cancer; and rectal cancer.

Within this group of cancers, a particular subgroup consists of colorectal cancer; glioblastoma; melanoma; metastatic thyroid cancer; prostate cancer; and rectal cancer.

One group of cancers includes human breast cancers (e.g. primary breast tumours, node-negative breast cancer, invasive duct adenocarcinomas of the breast, non-endometrioid breast cancers); and mantle cell lymphomas. In addition, other cancers are colorectal and endometrial cancers. Another sub-set of cancers includes hematopoietic tumours of both lymphoid and myeloid lineage, for example acute lymphoblastic leukemia, chronic lymphocytic leukaemia (Both T and B cell), acute myeloid leukaemia, chronic myeloid leukaemia, mantle cell lymphoma and B-cell lymphoma (such as diffuse large B cell lymphoma) and optionally further includes chronic myelogenous leukaemia and multiple myeloma. A sub-set of cancers consists of ErbB2-positive breast, prostate, lung, and gastric cancer; chronic myeloid leukemia; androgen receptor dependent prostate cancer; Flt3-dependent acute myeloid leukaemia; melanoma associated with Braf mutation; multiple myeloma; velcade refractory multiple myeloma; and gastrointestinal stromal tumours (GIST).

Another sub-set of cancers consists of hormone refractory prostate cancer, metastatic melanoma, HER2 positive breast cancer, mutant EGFR positive non-small cell lung carcinoma and Gleevec resistant gastrointestinal stromal tumours. A further sub-set of cancers consists of hormone refractory prostate cancer, metastatic melanoma, HER2 positive breast cancer, mutant EGFR positive non-small cell lung carcinoma, Small Cell Lung Carcinoma and gastrointestinal stromal tumours.

In certain embodiments the disclosure provides a method of treating or inhibiting a condition caused by a defect in a target sequence in a genomic locus of interest in an organism or a non-human organism in need thereof comprising modifying the organism or a non-human organism by manipulation of the target sequence and wherein the condition is susceptible to treatment or inhibition by manipulation of the target sequence comprising providing treatment comprising: delivering a non-naturally occurring or engineered composition comprising an AAV or a lentiviral vector system comprising one or more AAV or lentiviral vectors comprising operably encoding a composition for expression thereof, wherein the target sequence is manipulated by the composition when expressed, wherein the composition comprises: (A) a non-naturally occurring or engineered composition comprising a vector system comprising one or more vectors comprising I. a first regulatory element operably linked to a CRISPR-Cas system chimeric RNA (chiRNA) polynucleotide sequence, wherein the polynucleotide sequence comprises (a) a guide sequence capable of hybridizing to a target sequence in a eukaryotic cell, (b) a tracr mate sequence, and (c) a tracr sequence, and II. a second regulatory element operably linked to an enzyme-coding sequence encoding a CRISPR enzyme comprising at least one or more nuclear localization sequences (or optionally at least one or more nuclear localization sequences as some embodiments can involve no NLS) wherein (a), (b) and (c) are arranged in a 5' to 3' orientation, wherein components I and II are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridizable to the target sequence, and (2) the tracr mate sequence that is hybridized to the tracr sequence, or (B) a non-naturally occurring or engineered composition comprising a vector system comprising one or more vectors comprising I. a first regulatory element operably linked to (a) a guide sequence capable of hybridizing to a target sequence in a prokaryotic cell, and (b) at least one or more tracr mate sequences, II. a second regulatory element operably linked to an enzyme-coding sequence encoding a CRISPR enzyme, and III. a third regulatory element operably linked to a tracr sequence, wherein components I, II and III are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridizable to the target sequence, and (2) the tracr mate sequence that is hybridized to the tracr sequence. In one embodiment, the vector system comprises a viral vector system, e.g., an AAV vector or AAV vector system or a lentivirus-derived vector system. In one aspect, the Cas enzyme is a nickase.

Some methods can include inducing expression. In some methods the organism or subject is a eukaryote or a non-human eukaryote or a non-human animal. In some methods the organism or subject is a plant. In some methods the organism or subject is a mammal or a non-human mammal. In some methods the organism or subject is algae. In some methods the viral vector is an AAV or a lentiviral vector. In some methods of the invention the CRISPR enzyme is a Cas9. In some methods the expression of the guide sequence is under the control of the T7 promoter is driven by the expression of T7 polymerase.

The disclosure in some embodiments comprehends a method of delivering a CRISPR enzyme comprising delivering to a cell mRNA encoding the CRISPR enzyme. In some of these methods the CRISPR enzyme is a Cas9.

The disclosure in some embodiments comprehends a method of preparing the AAV or lentivirus vector comprising transfecting one or more plasmid(s) containing or consisting essentially of nucleic acid molecule(s) coding for the AAV or lentivirus into AAV- or lentivirus-infetable cells, and supplying AAV rep and/or cap obligatory for replication and packaging of the AAV or supplying the essential proteins that may be needed for packaging, replication and transduction of a lentivirus (e.g. gag, pol, rev). In some embodiments the AAV rep and/or cap obligatory for replication and packaging of the AAV are supplied by transfecting the cells with helper plasmid(s) or helper virus(es). In some embodiments the helper virus is a poxvirus, adenovirus, herpesvirus or baculovirus. In some embodiments the poxvirus is a vaccinia virus. In some embodiments the cells are mammalian cells. And in some embodiments the cells are insect cells and the helper virus is baculovirus.

In plants, pathogens are often host-specific. For example, *Fusarium oxysporum* f. sp. *lycopersici* causes tomato wilt but attacks only tomato, and *F. oxysporum f. dianthii Puccinia graminis* f. sp. *tritici* attacks only wheat. Plants have existing and induced defenses to resist most pathogens. Mutations and recombination events across plant generations lead to genetic variability that gives rise to susceptibility, especially as pathogens reproduce with more frequency than plants. In plants there can be non-host resistance, e.g., the host and pathogen are incompatible. There can also be Horizontal Resistance, e.g., partial resistance against all races of a pathogen, typically controlled by many genes and Vertical Resistance, e.g., complete resistance to some races of a pathogen but not to other races, typically controlled by a few genes. In a Gene-for-Gene level, plants and pathogens evolve together, and the genetic changes in one balance changes in other. Accordingly, using Natural Variability, breeders combine most useful genes for Yield, Quality, Uniformity, Hardiness, Resistance. The sources of resistance genes include native or foreign Varieties, Heirloom Varieties, Wild Plant Relatives, and Induced Mutations, e.g., treating plant material with mutagenic agents. Using the present invention, plant breeders are provided with a new tool to induce mutations. Accordingly, one skilled in the art can analyze the genome of sources of resistance genes, and in Varieties having desired characteristics or traits employ the present invention to induce the rise of resistance genes, with more precision than previous mutagenic agents and hence accelerate and improve plant breeding programs. In some methods of the invention the viral vector is a tobacco mosaic virus vector or an Agrobacterium Ti or Ri plasmid.

The disclosure further comprehends a composition or a CRISPR enzyme thereof (including or alternatively mRNA encoding the CRISPR enzyme) for use in medicine. In some embodiments the disclosure comprehends a composition or a CRISPR enzyme thereof (including or alternatively mRNA encoding the CRISPR enzyme) for use in a method according to the disclosure. In some embodiments the disclosure provides for the use of a composition or a CRISPR enzyme thereof (including or alternatively mRNA encoding the CRISPR enzyme) in *ex vivo* gene or genome editing. In certain embodiments the disclosure comprehends use of a composition or a CRISPR enzyme thereof (including or alternatively mRNA encoding the CRISPR enzyme) in the manufacture of a medicament for *ex vivo* gene or genome editing or for use in a method according of the disclosure. In some methods the CRISPR enzyme comprises one or more mutations in one of the catalytic domains. In some methods the CRISPR enzyme is a nickase. The disclosure comprehends in some embodiments a composition or a CRISPR enzyme thereof (including or alternatively mRNA encoding the CRISPR enzyme), wherein the target sequence is flanked at its 3' end by 5'-motif termed a proto-spacer adjacent motif (PAM), especially where the Cas9 is (or is derived from) *S. pyogenes* or *S. aureus* Cas9. For example, a suitable PAM is 5'-NRG or 5'-NNGRR (where N is any Nucleotide) for SpCas9 or SaCas9 enzymes (or derived enzymes), respectively, as mentioned below.

It will be appreciated that SpCas9 or SaCas9 are those from or derived from *S. pyogenes* or *S. aureus* Cas9.

Apects of the disclosure comprehend improving the specificity of a CRISPR enzyme, e.g. Cas9, mediated gene targeting and reducing the likelihood of off-target modification by the CRISPR enzyme, e.g. Cas9. The disclosure in some embodiments comprehends a method of modifying an organism or a non-human organism by minimizing off-target modifications by manipulation of a first and a second target sequence on opposite strands of a DNA duplex in a genomic locus of interest in a cell comprising delivering a non-naturally occurring or engineered composition comprising:

I. a first CRISPR-Cas system chimeric RNA (chiRNA) polynucleotide sequence, wherein the first polynucleotide sequence comprises:

(a) a first guide sequence capable of hybridizing to the first target sequence,
(b) a first tracr mate sequence, and
(c) a first tracr sequence,

II. a second CRISPR-Cas system chiRNA polynucleotide sequence, wherein the second polynucleotide sequence comprises:

(a) a second guide sequence capable of hybridizing to the second target sequence,
(b) a second tracr mate sequence, and
(c) a second tracr sequence, and

III. a polynucleotide sequence encoding a CRISPR enzyme comprising at least one or more nuclear localization sequences and comprising one or more mutations, wherein (a), (b) and (c) are arranged in a 5' to 3' orientation, wherein when transcribed, the first and the second tracr mate sequence hybridize to the first and second tracr sequence respectively and the first and the second guide sequence directs sequence-specific binding of a first and a second CRISPR complex to the first and second target sequences respectively, wherein the first CRISPR complex comprises the CRISPR enzyme complexed with (1) the first guide sequence that is hybridizableto the first target sequence, and (2) the first tracr mate sequence that is hybridized to the first tracr sequence, wherein the second CRISPR complex comprises the CRISPR enzyme complexed with (1) the second guide sequence that is hybridizableto the second target sequence, and (2) the second tracr mate sequence that is hybridized to the second tracr sequence, wherein the polynucleotide sequence encoding a CRISPR enzyme is DNA or RNA, and wherein the first guide sequence directs cleavage of one strand of the DNA duplex near the first target sequence and the second guide sequence directs cleavage of the opposite strand near the second target sequence, thereby inducing an offset double strand break in the DNA duplex, thereby modifying the organism or the non-human organism by minimizing off-target modifications. In one aspect, the first nick and the second nick in the DNA is offset relative to each other by at least one base pair of the duplex. In one aspect, the first nick and the second nick are offset relative to each other so that the resulting DNA break has a 3' overhang. In one aspct, the first nick and the second nick are offset relative to each other so that the resulting DNA break has a 5' overhang. In one aspect, the first nick and the second nick are positioned relative to each other such that the overhang is at least 1 nt, at least 10 nt, at least 15 nt, at least 26 nt, at least 30 nt, at least 50 nt or more that at least 50 nt. Additional aspects comprising the resulting offset double nicked DNA strand can be appreciated by one skilled in the art, and exemplary uses of the double nick system are provided herein.

In some methods of the invention any or all of the polynucleotide sequence encoding the CRISPR enzyme, the first and the second guide sequence, the first and the second tracr mate sequence or the first and the second tracr sequence, is/are RNA. In further embodiments of the invention the polynucleotides comprising the sequence encoding the CRISPR enzyme, the first and the second guide sequence, the first and the second tracr mate sequence or the first and the second tracr sequence, is/are RNA and are delivered via nanoparticles, exosomes, microvesciles, or a gene-gun. In certain embodiments of the invention, the first and second tracr mate sequence share 100% identity and/or the first and second tracr sequence share 100% identity. In preferred embodiments of the invention the CRISPR enzyme is a Cas9 enzyme, e.g. SpCas9. In an aspect of the invention the CRISPR enzyme comprises one or more mutations in a catalytic domain, wherein the one or more mutations are selected from the group consisting of D10A, E762A, H840A, N854A, N863A and D986A. In a highly preferred embodiment the CRISPR enzyme has the D10A mutation.

In preferred methods the first guide sequence directing cleavage of one strand of the DNA duplex near the first target sequence and the second guide sequence directing cleavage of the opposite strand near the second target sequence results in a 5' overhang. In embodiments the 5' overhang is at most 200 base pairs, preferably at most 100 base pairs, or more preferably at most 50 base pairs. In embodiments the 5' overhang is at least 26 base pairs, preferably at least 30 base pairs or more preferably 34-50 base pairs.

The in some embodiments comprehends a method of modifying an organism or a non-human organism by minimizing off-target modifications by manipulation of a first and a second target sequence on opposite strands of a DNA duplex in a genomic locus of interest in a cell, the method comprising delivering a non-naturally occurring or engineered composition comprising a vector system comprising one or more vectors comprising

    I. a first regulatory element operably linked to

        (a) a first guide sequence capable of hybridizing to the first target sequence, and
        (b) at least one or more tracr mate sequences,

    II. a second regulatory element operably linked to

        (a) a second guide sequence capable of hybridizing to the second target sequence, and
        (b) at least one or more tracr mate sequences,

    III. a third regulatory element operably linked to an enzyme-coding sequence encoding a CRISPR enzyme, and
    IV. a fourth regulatory element operably linked to a tracr sequence,

wherein components I, II, III and IV are located on the same or different vectors of the system,
when transcribed, the tracr mate sequence hybridizes to the tracr sequence and the first and the second guide sequence directs sequence-specific binding of a first and a second CRISPR complex to the first and second target sequences respectively, wherein the first CRISPR complex comprises the CRISPR enzyme complexed with (1) the first guide sequence that is hybridizableto the first target sequence, and (2) the tracr mate sequence that is hybridized to the tracr sequence, wherein the second CRISPR complex comprises the CRISPR enzyme complexed with (1) the second guide sequence that is hybridizableto the second target sequence, and (2) the tracr mate sequence that is hybridized to the tracr sequence, wherein the polynucleotide sequence encoding a CRISPR enzyme is DNA or RNA, and wherein the first guide sequence directs cleavage of one strand of the DNA duplex near the first target sequence and the second guide sequence directs cleavage of the opposite strand of the DNA duplex near the second target sequence inducing an offset double strand break, thereby modifying the organism or the non-human organism by minimizing off-target modifications.

In some methods any or all of the polynucleotide sequence encoding the CRISPR enzyme, the first and the second guide sequence, the first and the second tracr mate sequence or the first and the second tracr sequence, is/are RNA. In further embodiments the first and second tracr mate sequence share 100% identity and/or the first and second tracr sequence share 100% identity. In preferred embodiments the CRISPR enzyme is a Cas9 enzyme, e.g. SpCas9. In an aspect the CRISPR enzyme comprises one or more mutations in a catalytic domain, wherein the one or more mutations are selected from the group consisting of D10A, E762A, H840A, N854A, N863A and D986A. In a highly preferred embodiment the CRISPR enzyme has the D10A mutation. In a further embodiment of the disclosure, one or more of the viral vectors are delivered via nanoparticles, exosomes, microvesicles, or a gene-gun.

In preferred methods the first guide sequence directing cleavage of one strand of the DNA duplex near the first target sequence and the second guide sequence directing cleavage of the opposite strand near the second target sequence results in a 5' overhang. In embodiments the 5' overhang is at most 200 base pairs, preferably at most 100 base pairs, or more preferably at most 50 base pairs. In embodiments the 5' overhang is at least 26 base pairs, preferably at least 30 base pairs or more preferably 34-50 base pairs.

The disclosure in some embodiments comprehends a method of modifying a genomic locus of interest by minimizing off-target modifications by introducing into a cell, said cell containing and expressing a double stranded DNA molecule encoding the gene product, an engineered, non-naturally occurring CRISPR-Cas system comprising a Cas protein having one or more mutations in a catalytic domain and two guide RNAs that target a first strand and a second strand of the DNA molecule respectively, whereby the guide RNAs target the DNA molecule encoding the gene product and the Cas protein nicks each of the first strand and the second strand of the DNA molecule encoding the gene product, whereby expression of the gene product is altered; and, wherein the Cas protein and the two guide RNAs do not naturally occur together.

In preferred methods the Cas protein nicking each of the first strand and the second strand of the DNA molecule encoding the gene product results in a 5' overhang. In embodiments the 5' overhang is at most 200 base pairs, preferably at most 100 base pairs, or more preferably at most 50 base pairs. In embodiments the 5' overhang is at least 26 base pairs, preferably at least 30 base pairs or more preferably 34-50 base pairs.

Embodiments also comprehend the guide RNAs comprising a guide sequence fused to a tracr mate sequence and a tracr sequence. In an aspect the Cas protein is codon optimized for expression in a eukaryotic cell, wherein it may be a mammalian cell or a human cell or a specific tissue-type cell. In further embodiments the Cas protein is a type II CRISPR-Cas protein, e.g. a Cas 9 protein. In a highly preferred embodiment the Cas protein is a Cas9 protein, e.g. SpCas9. In aspects the Cas protein has one or more mutations selected from the group consisting of D10A, E762A, H840A, N854A, N863A and D986A. In a highly preferred embodiment the Cas protein has the D10A mutation. In many aspects of the disclosure, the Cas protein is a nickase.

Aspects relate to the expression of the gene product being decreased or a template polynucleotide being further introduced into the DNA molecule encoding the gene product or an intervening sequence being excised precisely by allowing the fragment comprising the DNA sequence between the two nick sites to be lost and the remaining two 5' overhangs to reanneal and ligate or the activity or function of the gene product being altered or the expression of the gene product being increased. In an embodiment the gene product is a protein. In an embodiment the gene product is a microRNA. In one embodiment, the regulatory region of a gene is targeted, thereby modifying the expression of the corresponding gene product.

The disclosure also comprehends an engineered, non-naturally occurring CRISPR-Cas system comprising a Cas protein having one or more mutations and two guide RNAs that target a first strand and a second strand respectively of a double stranded DNA molecule encoding a gene product in a cell, whereby the guide RNAs target the DNA molecule encoding the gene product and the Cas protein nicks each of the first strand and the second strand of the DNA duplex molecule encoding the gene product, whereby expression of the gene product is altered; and, wherein the Cas protein and the two guide RNAs do not naturally occur together.

In aspects the guide RNAs may comprise a guide sequence fused to a tracr mate sequence and a tracr sequence. In an embodiment the Cas protein is a type II CRISPR-Cas protein. In an aspect the Cas protein is codon optimized for expression in a eukaryotic cell, wherein it may be a mammalian cell or a human cell or a cell of a specific tissue type. In further embodiments the Cas protein is a type II CRISPR-Cas protein, e.g. a Cas 9 protein. In a highly preferred embodiment the Cas protein is a Cas9 protein, e.g. SpCas9. In aspects the Cas protein has one or more mutations selected from the group consisting of D10A, E762A, H840A, N854A, N863A and D986A. In a highly preferred embodiment the Cas protein has the D10A mutation. In many embodiments the Cas protein is a nickase.

Aspects relate to the expression of the gene product being decreased or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased or decreased. In an embodiment the gene product is a protein. In an embodiment the gene product is a microRNA. In one embodiment, the regulatory region of a gene is targeted, thereby modifying the expression of the corresponding gene product.

The disclosure also comprehends an engineered, non-naturally occurring vector system comprising one or more vectors comprising:

    a) a first regulatory element operably linked to each of two CRISPR-Cas system guide RNAs that target a first strand and a second strand respectively of a double stranded DNA molecule encoding a gene product,
    b) a second regulatory element operably linked to a Cas protein,

wherein components (a) and (b) are located on same or different vectors of the system, whereby the guide RNAs target the DNA molecule encoding the gene product and the Cas protein nicks each of the first strand and the second strand of the DNA molecule encoding the gene product, whereby expression of the gene product is altered; and, wherein the Cas protein and the two guide RNAs do not naturally occur together.

In aspects the guide RNAs may comprise a guide sequence fused to a tracr mate sequence and a tracr sequence. In an embodiment the Cas protein is a type II CRISPR-Cas protein. In an aspect the Cas protein is codon optimized for expression in a eukaryotic cell, wherein it may be a mammalian cell or a human cell. In further embodiments the Cas protein is a type II CRISPR-Cas protein, e.g. a Cas 9 protein. In a highly preferred embodiment the Cas protein is a Cas9 protein, e.g. SpCas9. In aspects the Cas protein has one or more mutations selected from the group consisting of D10A, E762A, H840A, N854A, N863A and D986A. In a highly preferred embodiment the Cas protein has the D10A mutation.

Aspects relate to the expression of the gene product being decreased or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased. In an embodiment the gene product is a protein. In an embodiment the gene product is a microRNA. In one embodiment, the regulatory region of a gene is targeted, thereby modifying the expression of the corresponding gene product. In preferred embodiments the vectors of the system are viral vectors. In a further embodiment, the vectors of the system are delivered via nanoparticles, exosomes, microvesicles, or a gene-gun.

In one aspect, the disclosure provides a method of modifying a target polynucleotide in a eukaryotic cell. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence. In some embodiments, said cleavage comprises cleaving one or two strands at the location of the target sequence by said CRISPR enzyme. In some embodiments, said cleavage results in decreased transcription of a target gene. In some embodiments, the method further comprises repairing said cleaved target polynucleotide by homologous recombination with an exogenous template polynucleotide, wherein said repair results in a mutation comprising an insertion, deletion, or substitution of one or more nucleotides of said target polynucleotide. In some embodiments, said mutation results in one or more amino acid changes in a protein expressed from a gene comprising the target sequence. In some embodiments, the method further comprises delivering one or more vectors to said eukaryotic cell, wherein the one or more vectors drive expression of one or more of: the CRISPR enzyme, the guide sequence linked to the tracr mate sequence, and the tracr sequence. In some embodiments, said vectors are delivered to the eukaryotic cell in an organism. In some embodiments, said modifying takes place in said eukaryotic cell in a cell culture. In some embodiments, the method further comprises isolating said eukaryotic cell from an organism prior to said modifying. In some embodiments, the method further comprises returning said eukaryotic cell and/or cells derived therefrom to said organism.

In one aspect, the disclosure provides a method of modifying expression of a polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a CRISPR enzyme complexed with a guide sequence hybridized to a target sequence within said polynucleotide, wherein said guide sequence is linked to a tracr mate sequence which in turn hybridizes to a tracr sequence. In some embodiments, the method further comprises delivering one or more vectors to said eukaryotic cells, wherein the one or more vectors drive expression of one or more of: the CRISPR enzyme, the guide sequence linked to the tracr mate sequence, and the tracr sequence.

In one aspect, the disclosure provides a method of generating a model eukaryotic cell comprising a mutated disease gene. In some embodiments, a disease gene is any gene associated an increase in the risk of having or developing a disease. In some embodiments, the method comprises (a) introducing one or more vectors into a eukaryotic cell, wherein the one or more vectors drive expression of one or more of: a CRISPR enzyme, a guide sequence linked to a tracr mate sequence, and a tracr sequence; and (b) allowing a CRISPR complex to bind to a target polynucleotide to effect cleavage of the target polynucleotide within said disease gene, wherein the CRISPR complex comprises the CRISPR enzyme complexed with (1) the guide sequence that is hybridizable to the target sequence within the target polynucleotide, and (2) the tracr mate sequence that is hybridized to the tracr sequence, thereby generating a model eukaryotic cell comprising a mutated disease gene. In some embodiments, said cleavage comprises cleaving one or two strands at the location of the target sequence by said CRISPR enzyme. In some embodiments, said cleavage results in decreased transcription of a target gene. In some embodiments, the method further comprises repairing said cleaved target polynucleotide by homologous recombination with an exogenous template polynucleotide, wherein said repair results in a mutation comprising an insertion, deletion, or substitution of one or more nucleotides of said target polynucleotide. In some embodiments, said mutation results in one or more amino acid changes in a protein expression from a gene comprising the target sequence.

In one aspect the disclosure provides for a method of selecting one or more prokaryotic cell(s) by introducing one or more mutations in a gene in the one or more prokaryotic cell (s), the method comprising: introducing one or more vectors into the prokaryotic cell (s), wherein the one or more vectors drive expression of one or more of: a CRISPR enzyme, a guide sequence linked to a tracr mate sequence, a tracr sequence, and an editing template; wherein the editing template comprises the one or more mutations that abolish CRISPR enzyme cleavage; allowing homologous recombination of the editing template with the target polynucleotide in the cell(s) to be selected; allowing a CRISPR complex to bind to a target polynucleotide to effect cleavage of the target polynucleotide within said gene, wherein the CRISPR complex comprises the CRISPR enzyme complexed with (1) the guide sequence that is hybridizable to the target sequence within the target polynucleotide, and (2) the tracr mate sequence that is hybridized to the tracr sequence, wherein binding of the CRISPR complex to the target polynucleotide induces cell death, thereby allowing one or more prokaryotic cell(s) in which one or more mutations have been introduced to be selected. In a preferred embodiment, the CRISPR enzyme is Cas9. In another aspect the cell to be selected may be a eukaryotic cell. Aspects allow for selection of specific cells without requiring a selection marker or a two-step process that may include a counter-selection system.

In one aspect, the disclosure provides for methods of modifying a target polynucleotide in a eukaryotic cell. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence.

In other embodiments, this disclosure provides a method of modifying expression of a polynucleotide in a eukaryotic cell. The method comprises increasing or decreasing expression of a target polynucleotide by using a CRISPR complex that binds to the polynucleotide.

Where desired, to effect the modification of the expression in a cell, one or more vectors comprising a tracr sequence, a guide sequence linked to the tracr mate sequence, a sequence encoding a CRISPR enzyme is delivered to a cell. In some methods, the one or more vectors comprises a regulatory element operably linked to an enzyme-coding sequence encoding said CRISPR enzyme comprising a nuclear localization sequence; and a regulatory element operably linked to a tracr mate sequence and one or more insertion sites for inserting a guide sequence upstream of the tracr mate sequence. When expressed, the guide sequence directs sequence-specific binding of a CRISPR complex to a target sequence in a cell. Typically, the CRISPR complex comprises a CRISPR enzyme complexed with (1) the guide sequence that is hybridizable to the target sequence, and (2) the tracr mate sequence that is hybridized to the tracr sequence. In one embodiment, the vector system comprises a viral vector system, e.g., an AAV vector or AAV vector system or a lentivirus-derived vector system.

In some methods, a target polynucleotide can be inactivated to effect the modification of the expression in a cell. For example, upon the binding of a CRISPR complex to a target sequence in a cell, the target polynucleotide is inactivated such that the sequence is not transcribed, the coded protein is not produced, or the sequence does not function as the wild-type sequence does. For example, a protein or microRNA coding sequence may be inactivated such that the protein or microRNA or pre-microRNA transcript is not produced.

In certain embodiments, the CRISPR enzyme comprises one or more mutations D10A, E762A, H840A, N854A, N863A or D986A and/or the one or more mutations is in a RuvC1 or HNH domain of the CRISPR enzyme or is a mutation as otherwise as discussed herein. In some embodiments, the CRISPR enzyme has one or more mutations in a catalytic domain, wherein when transcribed, the tracr mate sequence hybridizes to the tracr sequence and the guide sequence directs sequence-specific binding of a CRISPR complex to the target sequence, and wherein the enzyme further comprises a functional domain. In some embodiments, , the mutated Cas9 enzyme may be fused to a protein domain, e.g., such as a transcriptional activation domain. In one aspect, a transcriptional activation domain is VP64. In some embodiments, a transcription repression domains is KRAB. In some embodiments, a transcription repression domain is SID, or concatemers of SID (i.e. SID4X). In some embodiments, an epigenetic modifying enzyme is provided. In some embodiments, an activation domain is provided, which may be the P65 activation domain.

In some embodiments, the CRISPR enzyme is a type I or III CRISPR enzyme, preferably a type II CRISPR enzyme. This type II CRISPR enzyme may be any Cas enzyme. A Cas enzyme may be identified Cas9 as this can refer to the general class of enzymes that share homology to the biggest nuclease with multiple nuclease domains from the type II CRISPR system. Most preferably, the Cas9 enzyme is from, or is derived from, spCas9 or saCas9. By derived, Applicants mean that the derived enzyme is largely based, in the sense of having a high degree of sequence homology with, a wildtype enzyme, but that it has been mutated (modified) in some way as described herein.

It will be appreciated that the terms Cas and CRISPR enzyme are generally used herein interchangeably, unless otherwise apparent. As mentioned above, many of the residue numberings used herein refer to the Cas9 enzyme from the type II CRISPR locus in *Streptococcus pyogenes*. However, it will be appreciated that this disclosure includes many more Cas9s from other species of microbes, such as SpCas9, SaCa9, St1Cas9 and so forth.

An example of a codon optimized sequence, in this instance optimized for humans (i.e. being optimized for expression in humans) is provided herein, see the SaCas9 human codon optimized sequence. Whilst this is preferred, it will be appreciated that other examples are possible and codon optimization for a host species other than human, or for codon optimization for specific organs or tissues such as the brain, is known.

Preferably, delivery is in the form of a vector which may be a viral vector, such as a lenti- or baculo- or preferably adeno-viral/adeno-associated viral vectors, but other means of delivery are known (such as yeast systems, microvesicles, gene guns/means of attaching vectors to gold nanoparticles) and are provided. A vector may mean not only a viral or yeast system (for instance, where the nucleic acids of interest may be operably linked to and under the control (in terms of expression, such as to ultimately provide a processed RNA) a promoter), but also direct delivery of nucleic acids into a host cell. While in herein methods the vector may be a viral vector and this is advantageously an AAV, other viral vectors as herein discussed can be employed. For example, baculoviruses may be used for expression in insect cells. These insect cells may, in turn be useful for producing large quantities of further vectors, such as AAV vectors adapted for delivery of the present invention. Also envisaged is a method of delivering the present CRISPR enzyme comprising delivering to a cell mRNA encoding the CRISPR enzyme. It will be appreciated that the CRISPR enzyme is truncated, comprised of less than one thousand amino acids or less than four thousand amino acids, is a nuclease or nickase, is codon-optimized comprises one or more mutations, and/or comprises a chimeric CRISPR enzyme, or the other options as herein discussed. AAV viral vectors or lentiviral vectors are preferred.

In certain embodiments, the target sequence is flanked or followed, at its 3' end, by a PAM suitable for the CRISPR enzyme, typically a Cas and in particular a Cas9.

For example, a suitable PAM is 5'-NRG or 5'-NNGRR for SpCas9 or SaCas9 enzymes (or derived enzymes), respectively.

It will be appreciated that SpCas9 or SaCas9 are those from or derived from *S. pyogenes* or *S. aureus* Cas9.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the disclosure.

These and other embodiments are disclosed or are obvious from and encompassed by, the following Detailed Description.

BRIEF DESCRIPTION OF THE DRAWINGS

The novel features of the disclosure are set forth with particularity in the appended claims. A better understanding of the features and advantages of the present invention will be obtained by reference to the following detailed description that sets forth illustrative embodiments, in which the principles of the invention are utilized, and the accompanying drawings of which:

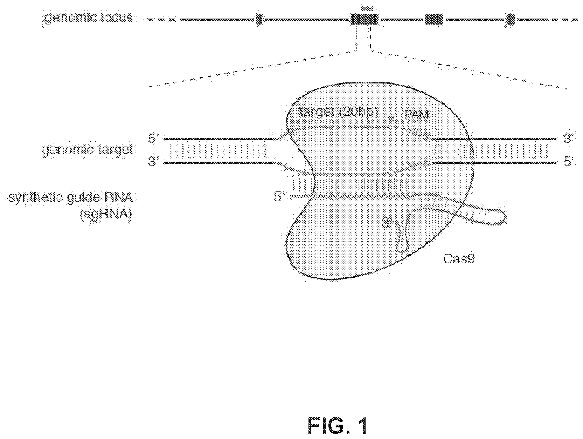

**Figure 1** shows a schematic model of the CRISPR system. The Cas9 nuclease from Streptococcus pyogenes (yellow) is targeted to genomic DNA by a synthetic guide RNA (sgRNA) consisting of a 20-nt guide sequence (blue) and a scaffold (red). The guide sequence base-pairs with the DNA target (blue), directly upstream of a requisite 5'-NGG protospacer adjacent motif (PAM; magenta), and Cas9 mediates a double-stranded break (DSB) ~3 bp upstream of the PAM (red triangle).

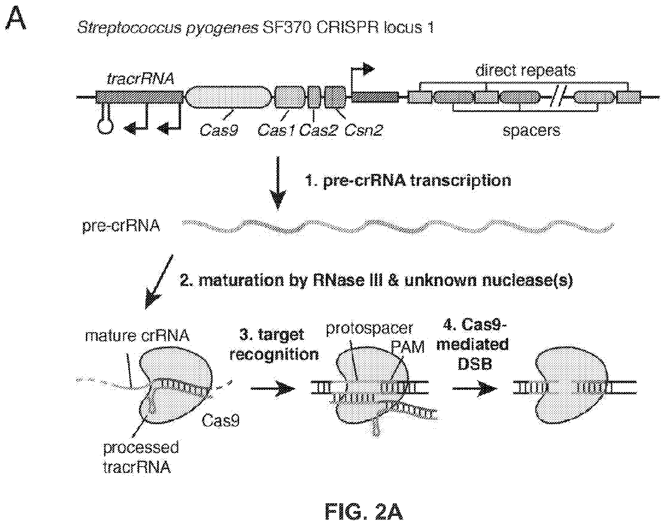

**Figure 2A-F** shows an exemplary CRISPR system, a possible mechanism of action, an example adaptation for expression in eukaryotic cells, and results of tests assessing nuclear localization and CRISPR activity.

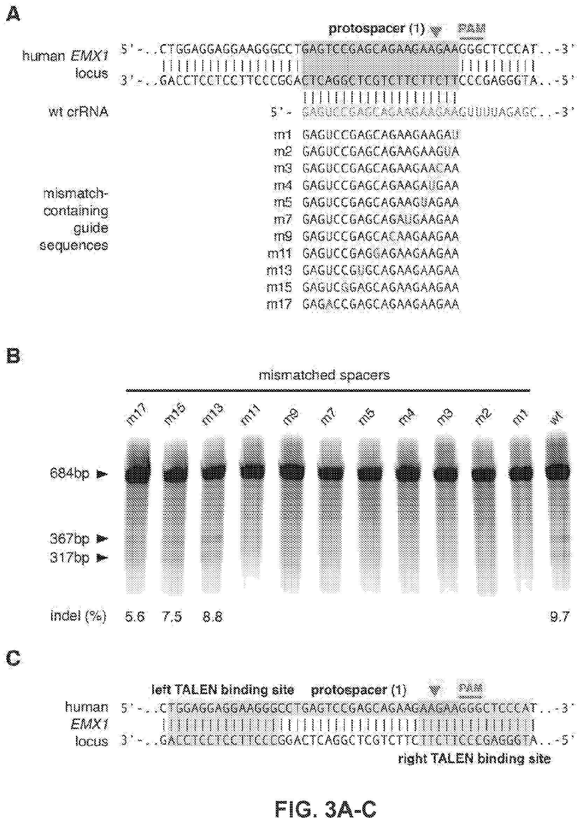

**Figure 3A-D** shows results of an evaluation of SpCas9 specificity for an example target.

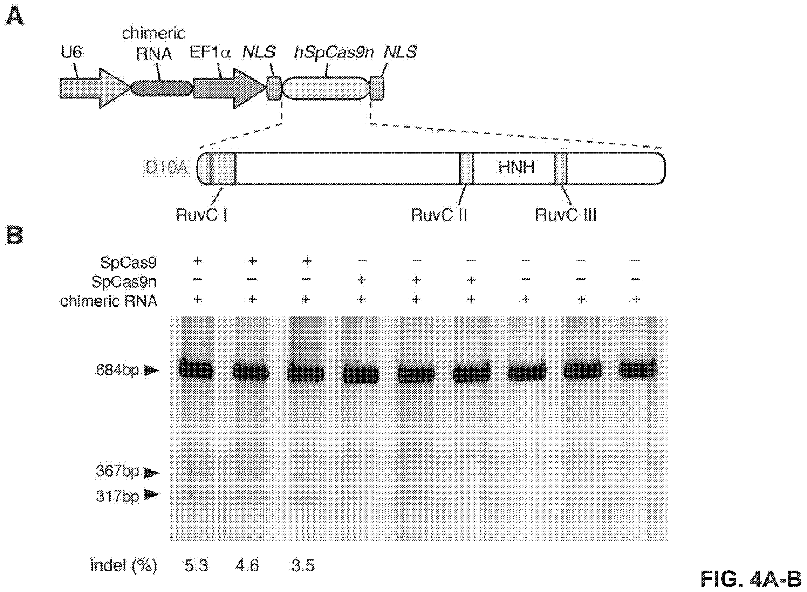

**Figure 4A-G** show an exemplary vector system and results for its use in directing homologous recombination in eukaryotic cells.

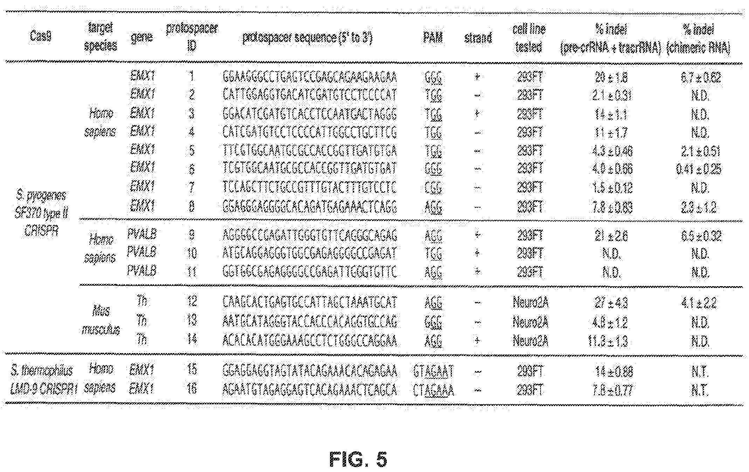

**Figure 5** provides a table of protospacer sequences and summarizes modification efficiency results for protospacer targets designed based on exemplary S. pyogenes and S. thermophilus CRISPR systems with corresponding PAMs against loci in human and mouse genomes. Cells were transfected with Cas9 and either pre-crRNA/tracrRNA or chimeric RNA, and analyzed 72 hours after transfection. Percent indels are calculated based on Surveyor assay results from indicated cell lines (N=3 for all protospacer targets, errors are S.E.M., N.D. indicates not detectable using the Surveyor assay, and N.T. indicates not tested in this study).

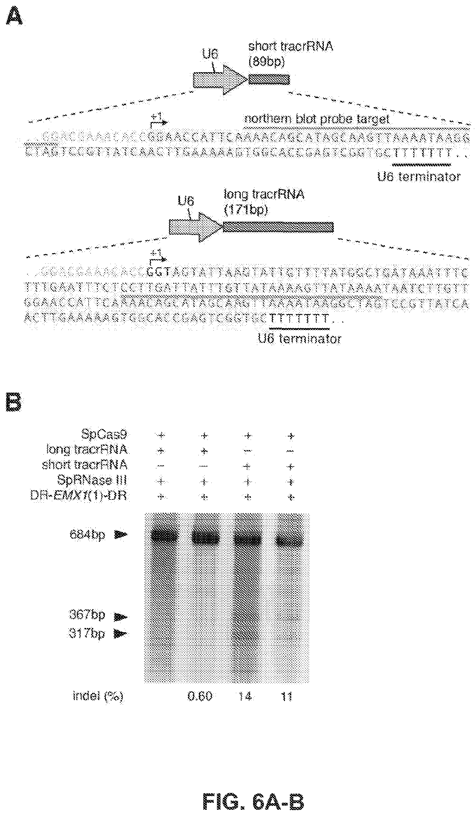

**Figure 6A-C** shows a comparison of different tracrRNA transcripts for Cas9-mediated gene targeting.

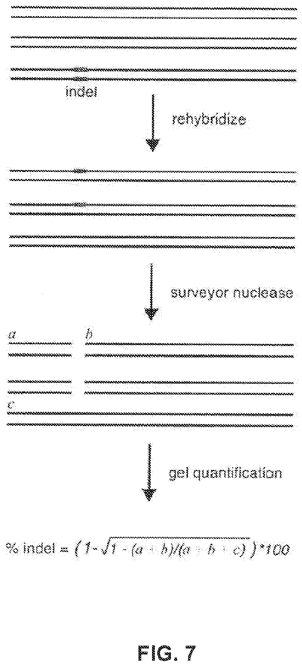

**Figure 7** shows a schematic of a surveyor nuclease assay for detection of double strand break-induced micro-insertions and -deletions.

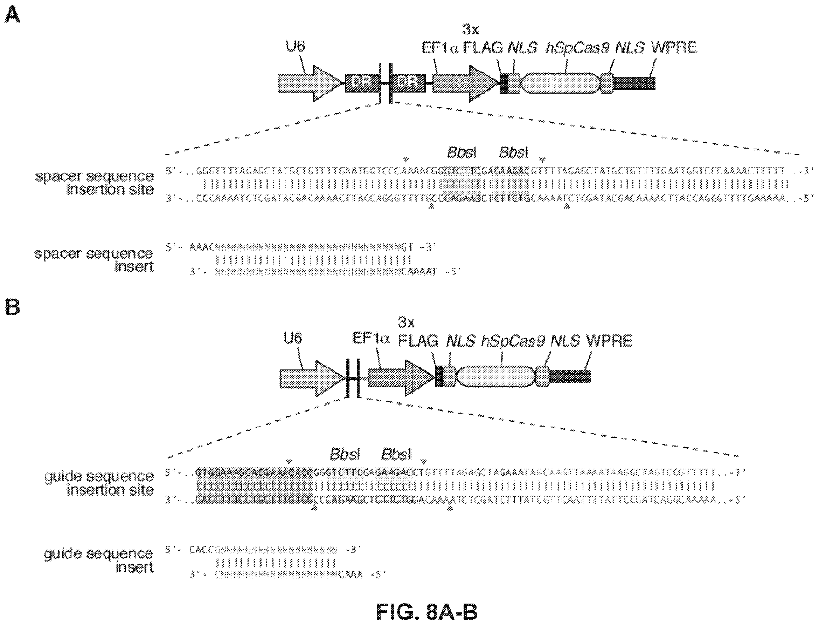

**Figure 8A-B** shows exemplary bicistronic expression vectors for expression of CRISPR system elements in eukaryotic cells.

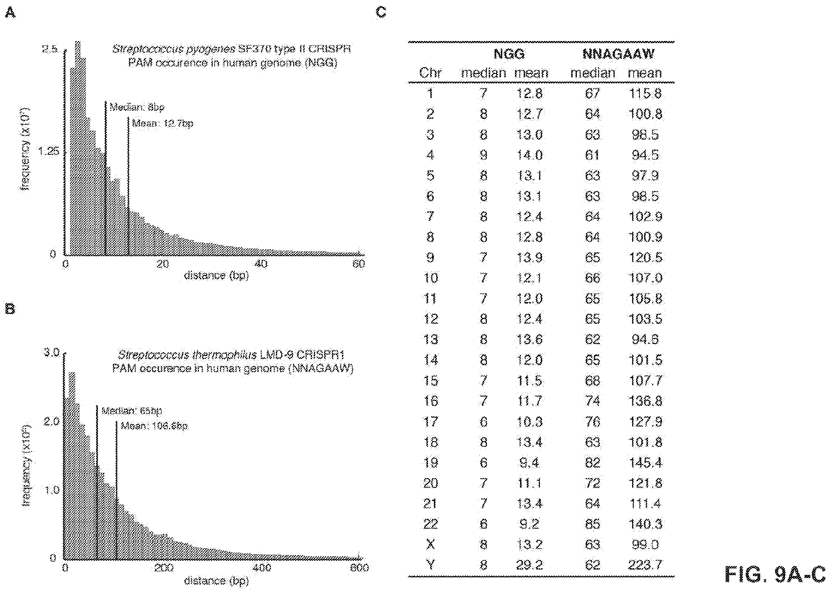

**Figure 9A-C** shows histograms of distances between adjacent S. pyogenes SF370 locus 1 PAM (NGG) (Figure 9A) and S. thermophilus LMD9 locus 2 PAM (NNAGAAW) (Figure 9B) in the human genome; and distances for each PAM by chromosome (Chr) (Figure 9C).

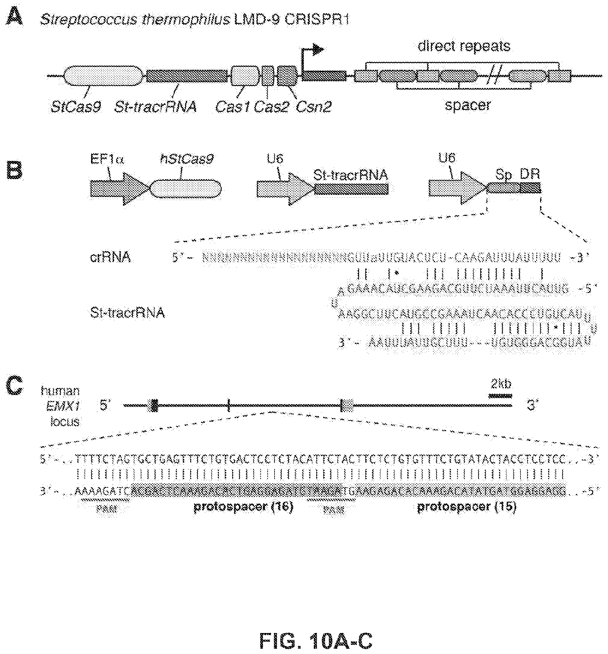

**Figure 10A-D** shows an exemplary CRISPR system, an example adaptation for expression in eukaryotic cells, and results of tests assessing CRISPR activity.

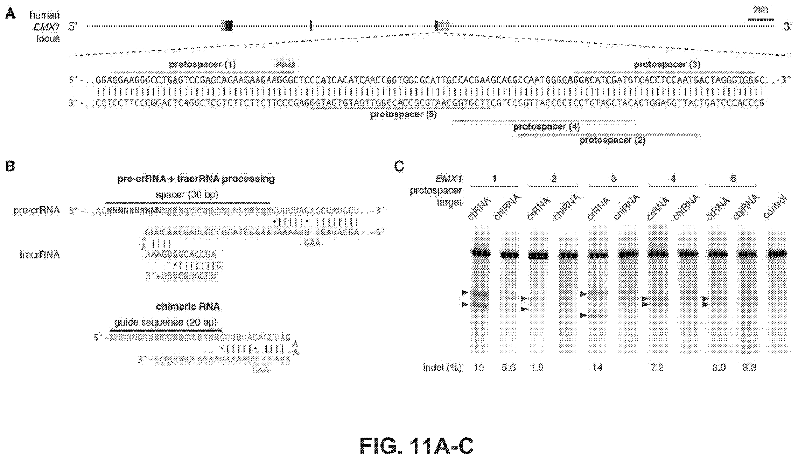

**Figure 11A-C** shows exemplary manipulations of a CRISPR system for targeting of genomic loci in mammalian cells.

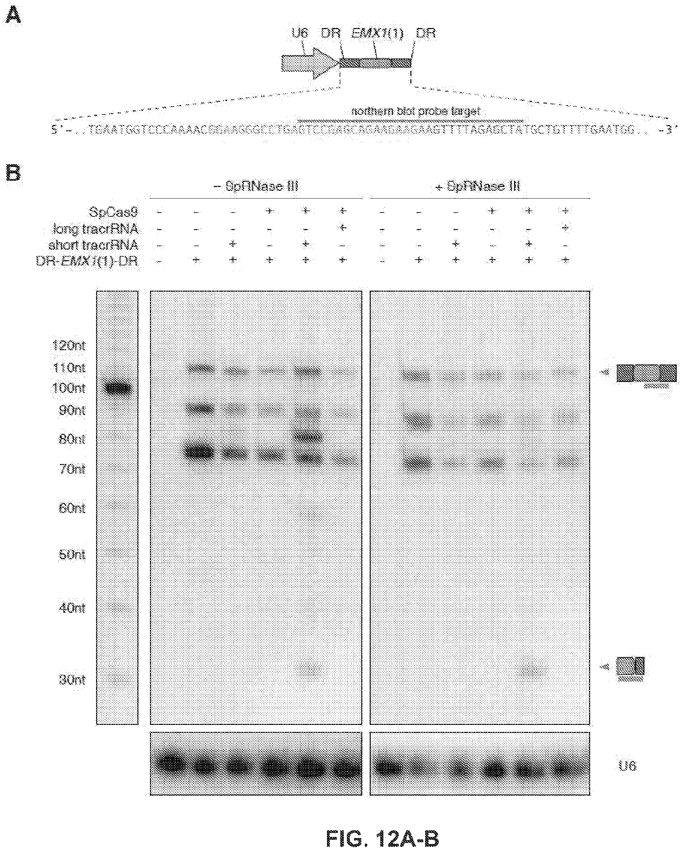

**Figure 12A-B** shows the results of a Northern blot analysis of crRNA processing in mammalian cells.

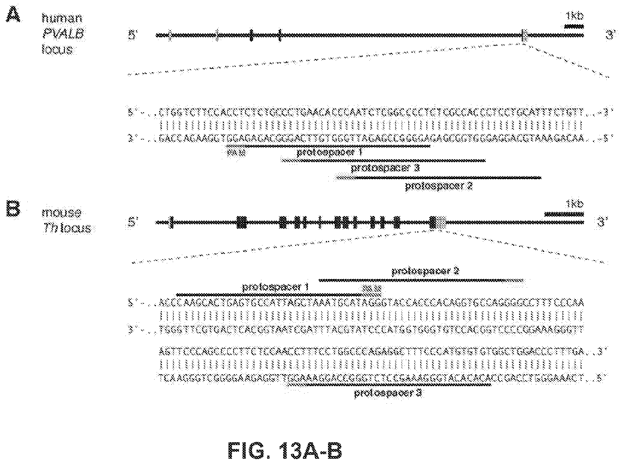

**Figure 13A-B** shows an exemplary selection of protospacers in the human PVALB and mouse Th loci.

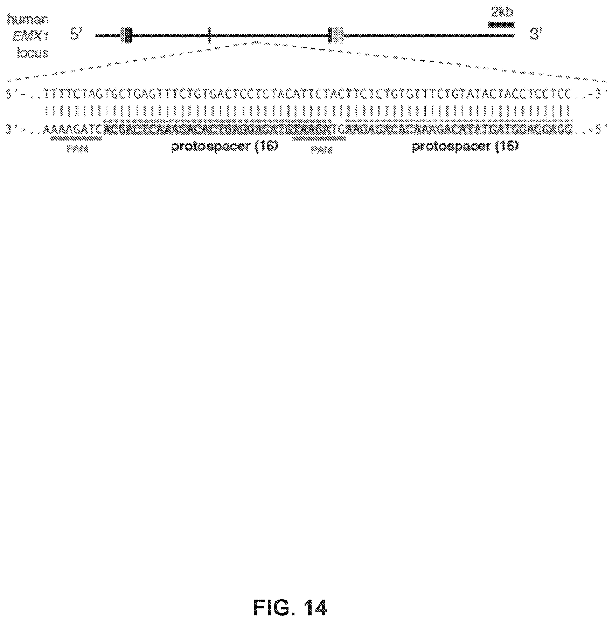

**Figure 14** shows example protospacer and corresponding PAM sequence targets of the S. thermophilus CRISPR system in the human EMX1 locus.

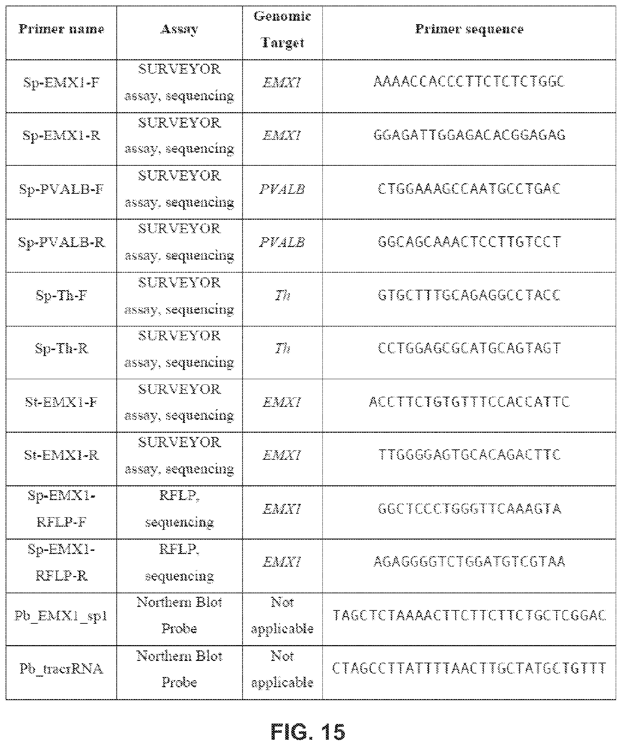

**Figure 15** provides a table of sequences for primers and probes used for Surveyor, RFLP, genomic sequencing, and Northern blot assays.

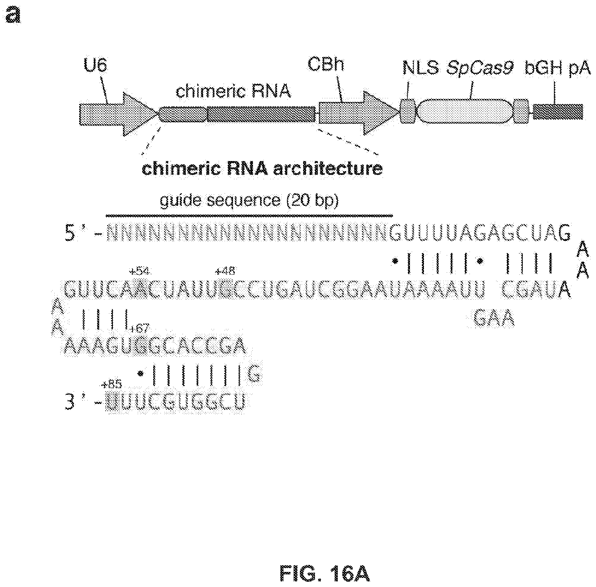

**Figure 16A-C** shows exemplary manipulation of a CRISPR system with chimeric RNAs and results of SURVEYOR assays for system activity in eukaryotic cells.

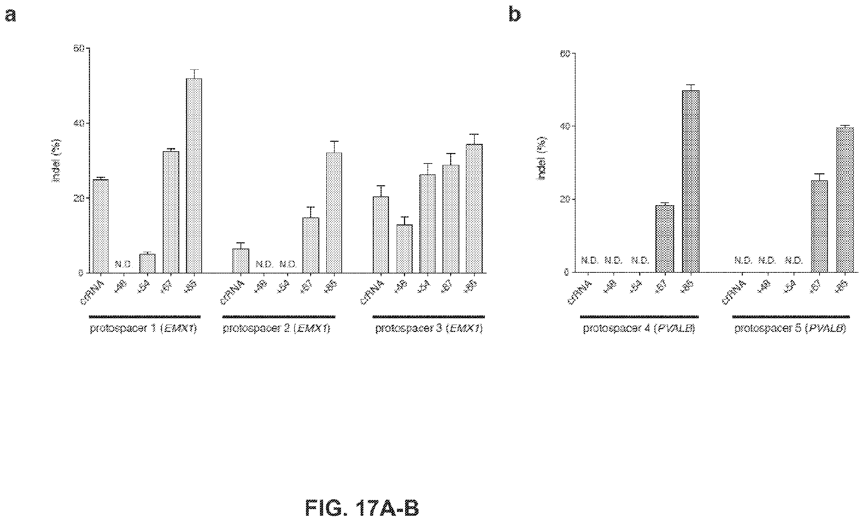

**Figure 17A-B** shows a graphical representation of the results of SURVEYOR assays for CRISPR system activity in eukaryotic cells.

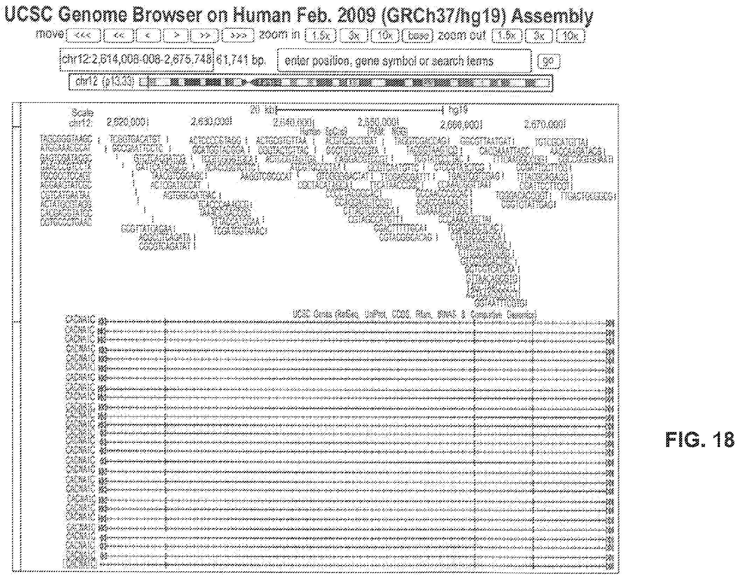

**Figure 18** shows an exemplary visualization of some S. pyogenes Cas9 target sites in the human genome using the UCSC genome browser.

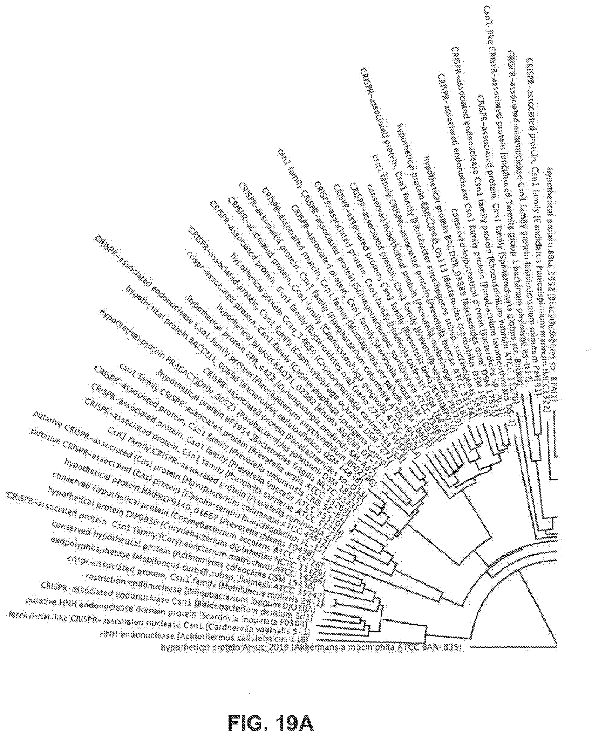

**Figure 19A-D** shows a circular depiction of the phylogenetic analysis revealing five families of Cas9s, including three groups of large Cas9s (-1400 amino acids) and two of small Cas9s (~1100 amino acids).

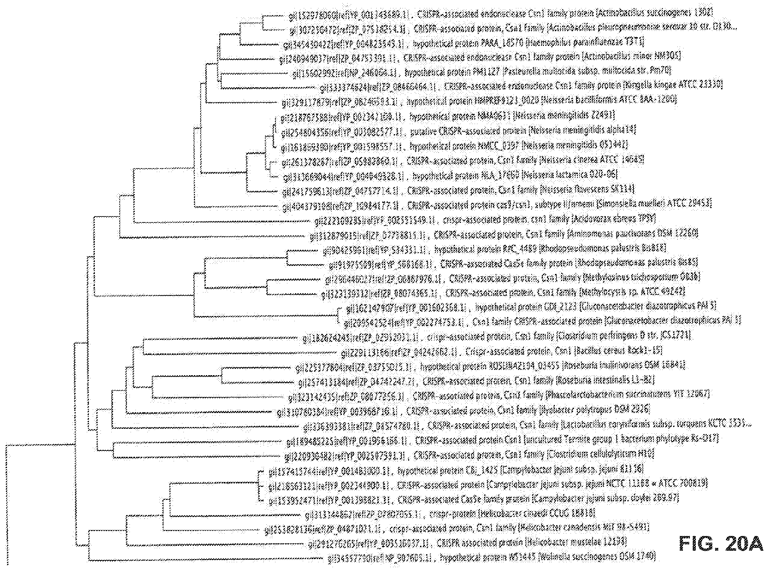

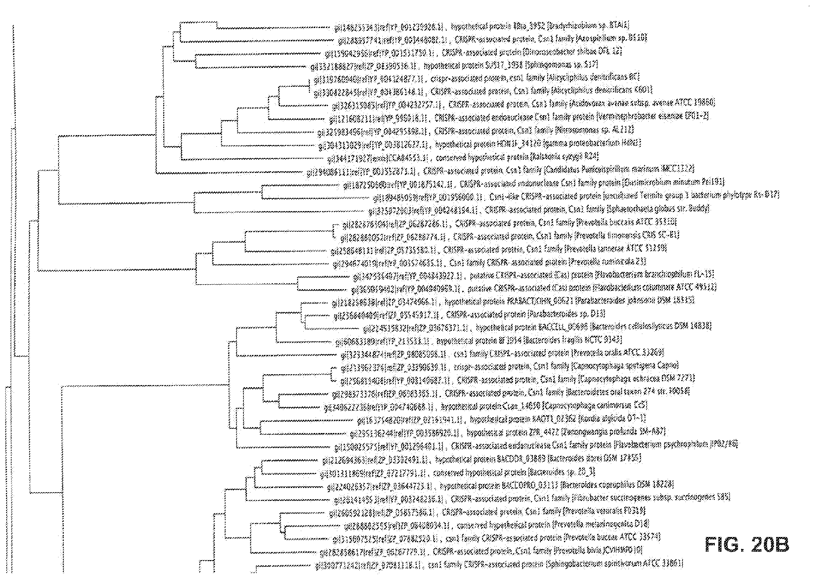

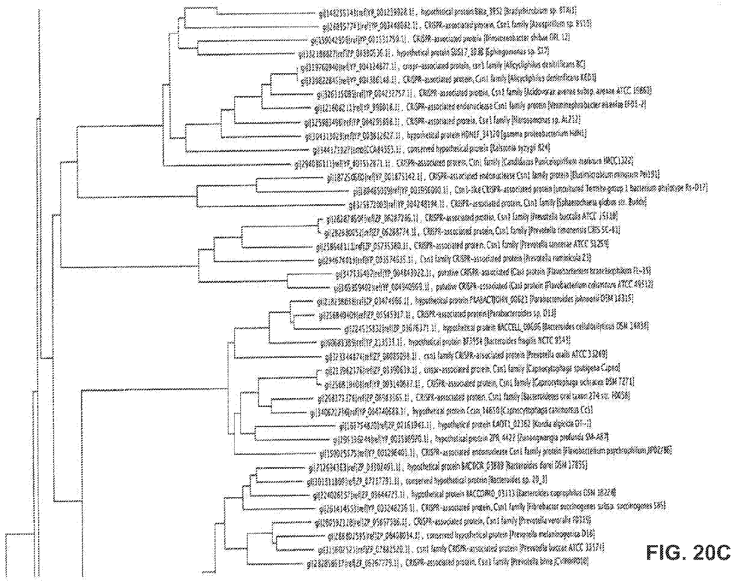

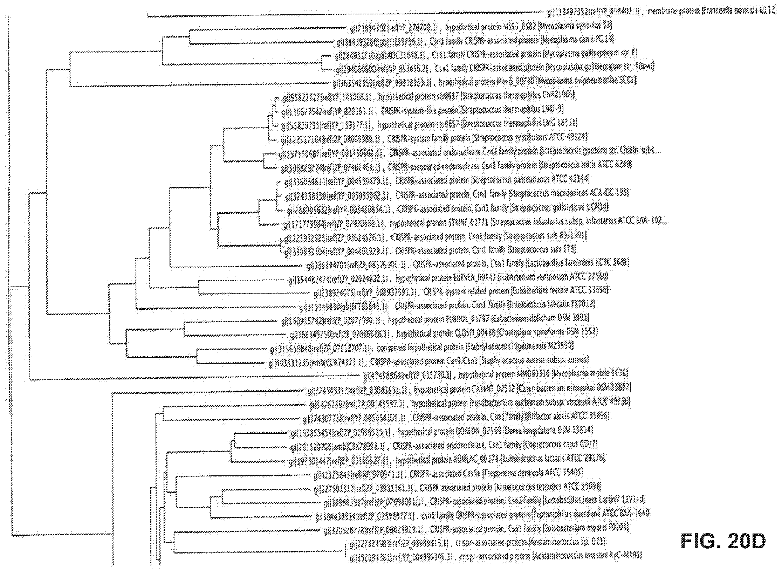

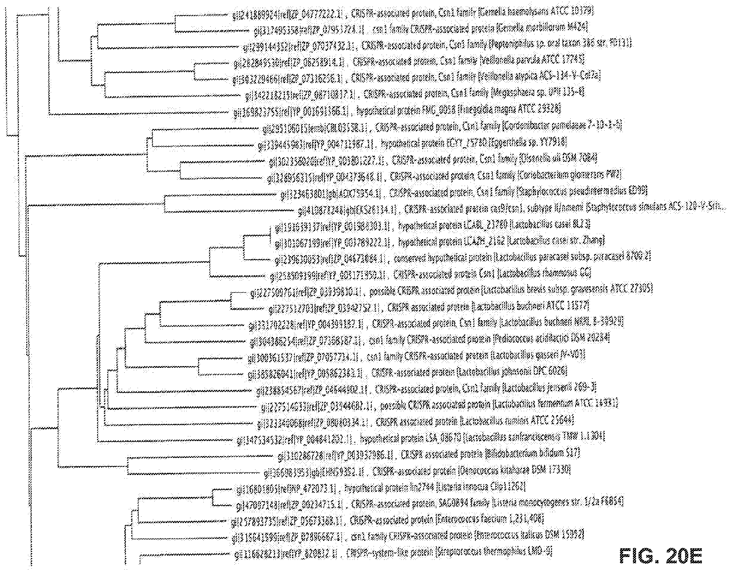

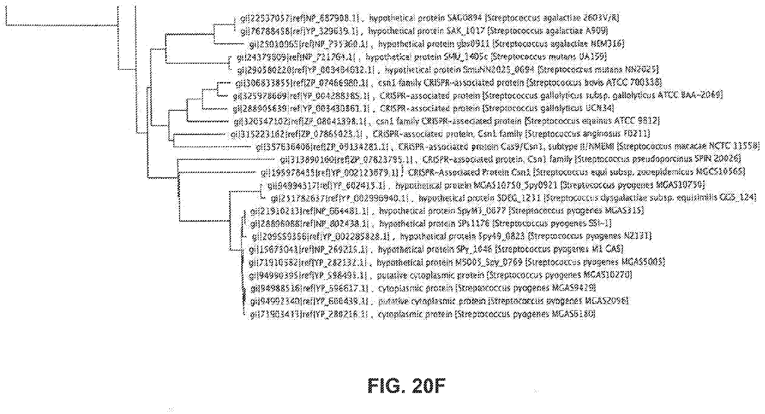

**Figure 20A-F** shows the linear depiction of the phylogenetic analysis revealing five families of Cas9s, including three groups of large Cas9s (~1400 amino acids) and two of small Cas9s (-1100 amino acids).

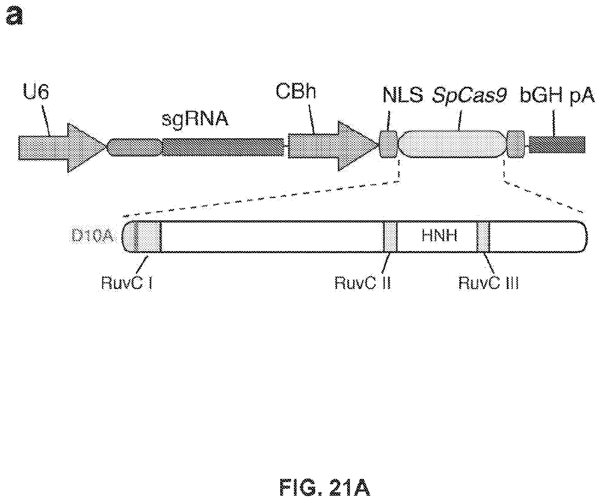

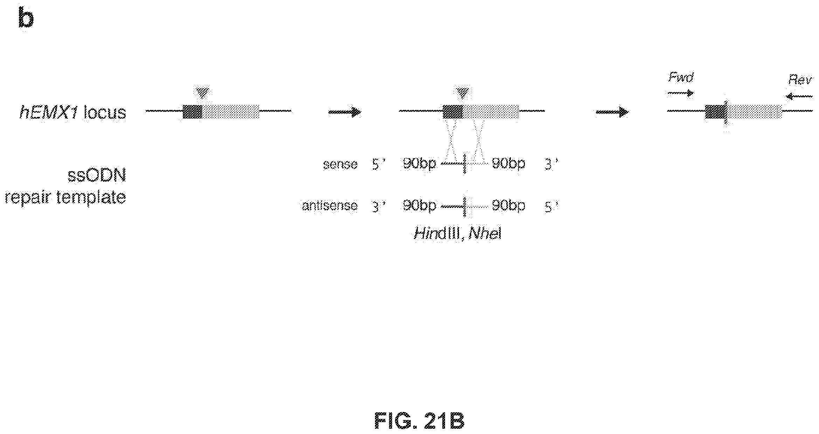

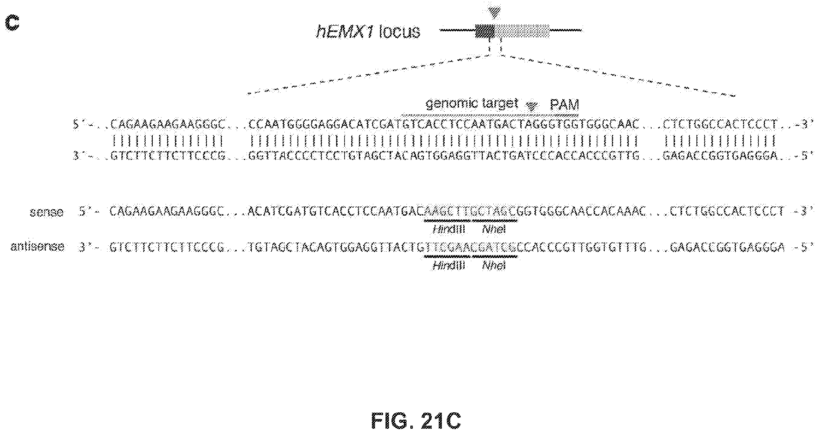

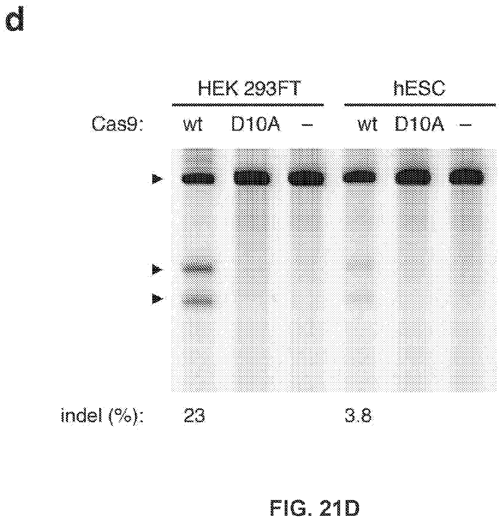

**Figure 21A-D** shows genome editing via homologous recombination. (a) Schematic of SpCas9 nickase, with D10A mutation in the RuvC I catalytic domain. (b) Schematic representing homologous recombination (HR) at the human EMX1 locus using either sense or antisense single stranded oligonucleotides as repair templates. Red arrow above indicates sgRNA cleavage site; PCR primers for genotyping (Tables J and K) are indicated as arrows in right panel. (c) Sequence of region modified by HR. d, SURVEYOR assay for wildtype (wt) and nickase (D10A) SpCas9-mediated indels at the EMX1 target 1 locus (n=3). Arrows indicate positions of expected fragment sizes.

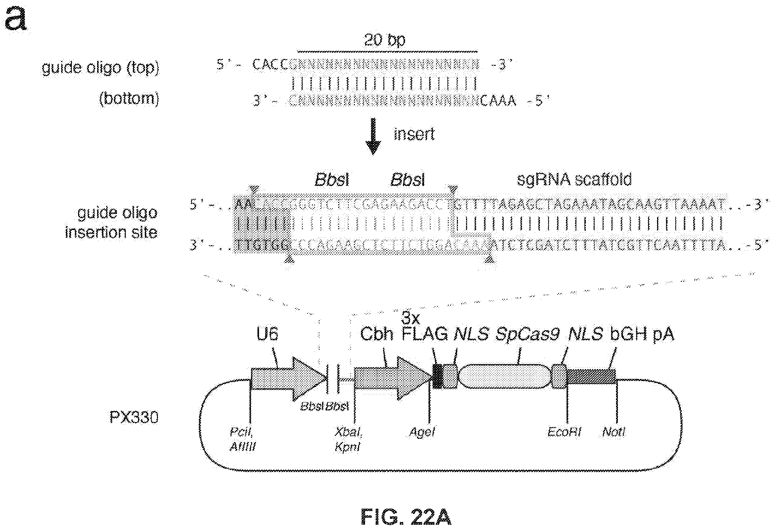

**Figure 22A-B** shows single vector designs for SpCas9.

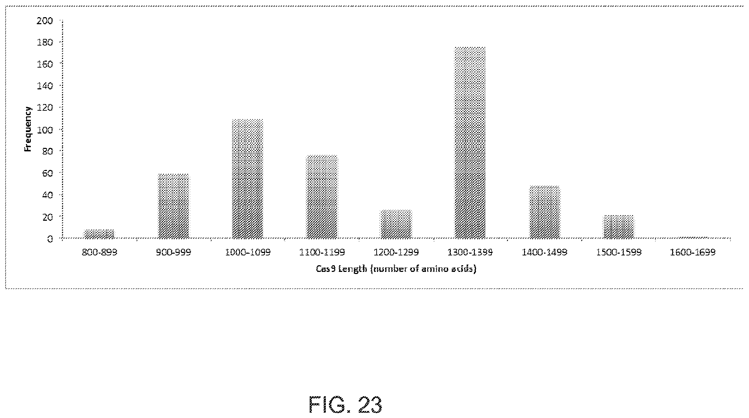

**Figure 23** shows a graph representing the length distribution of Cas9 orthologs.

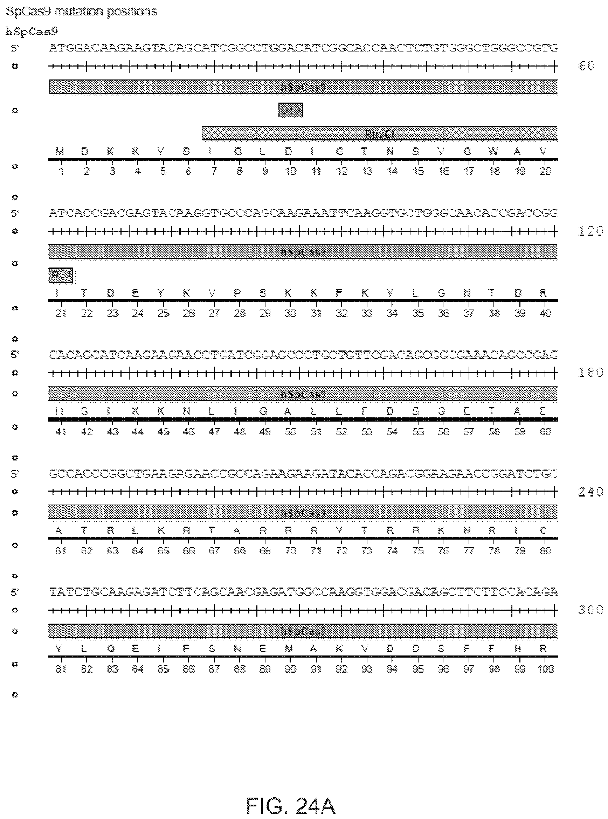

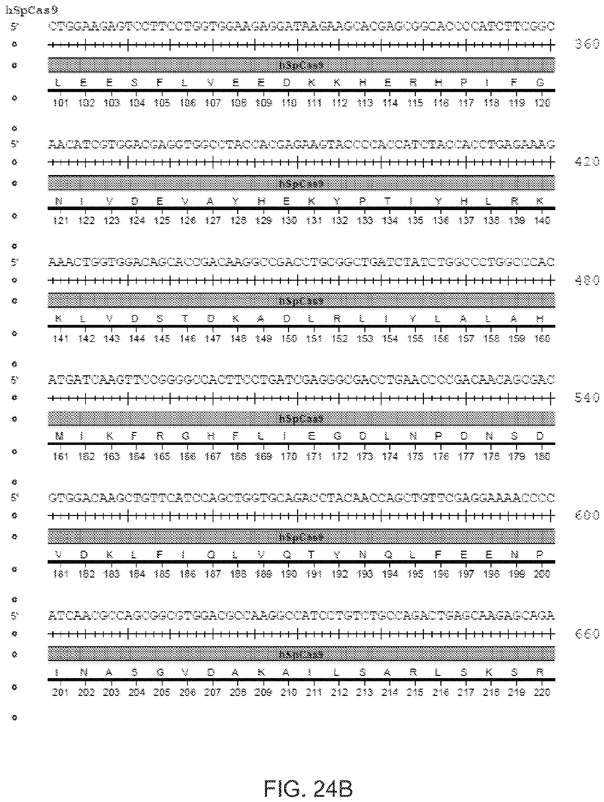

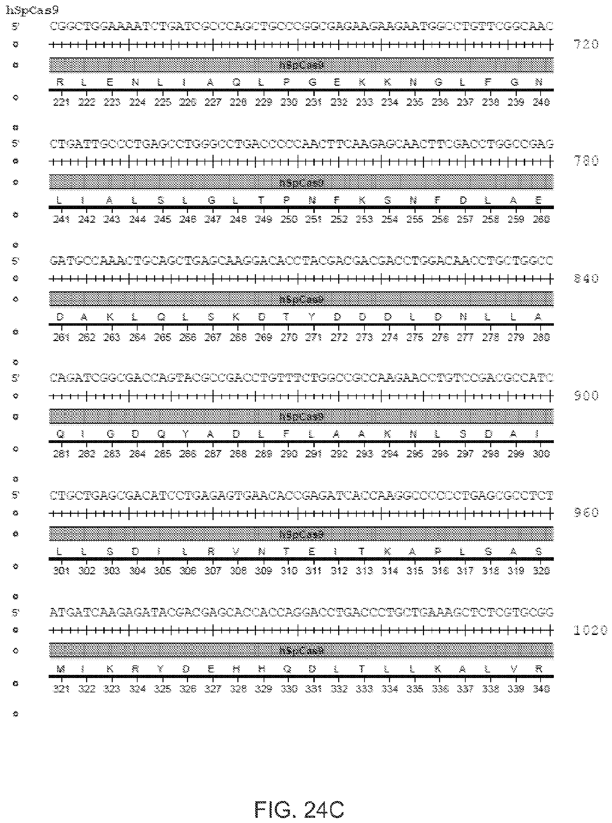

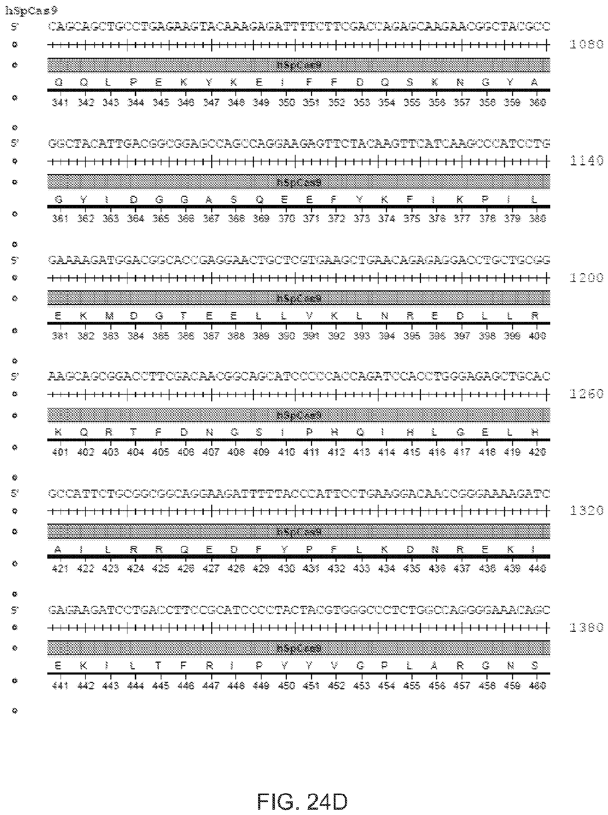

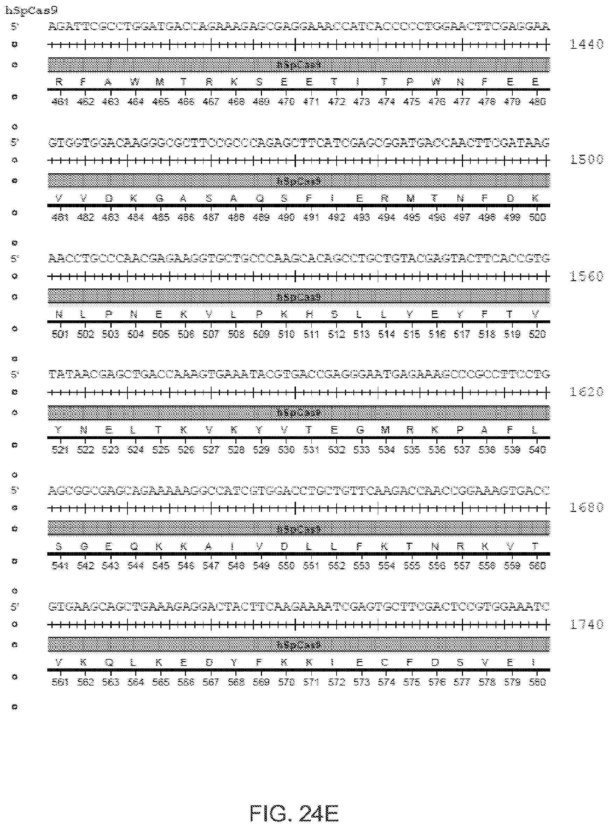

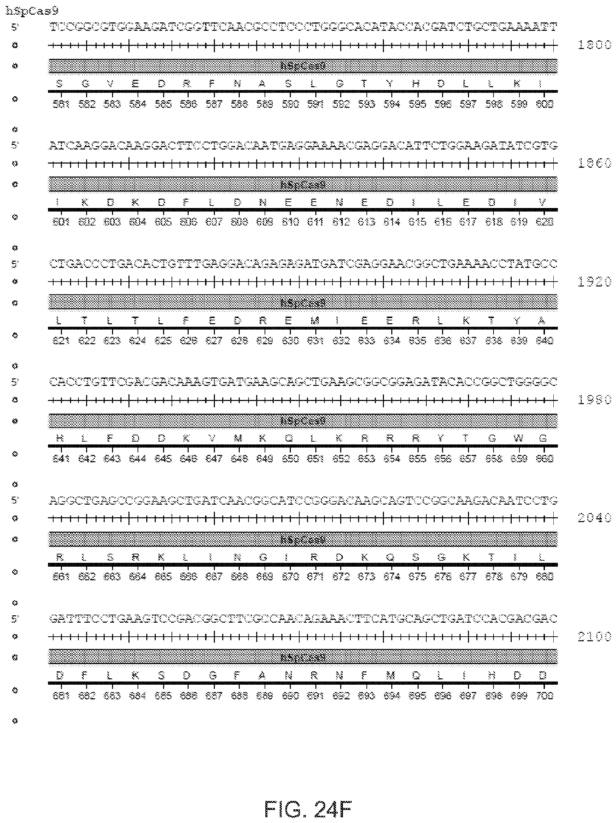

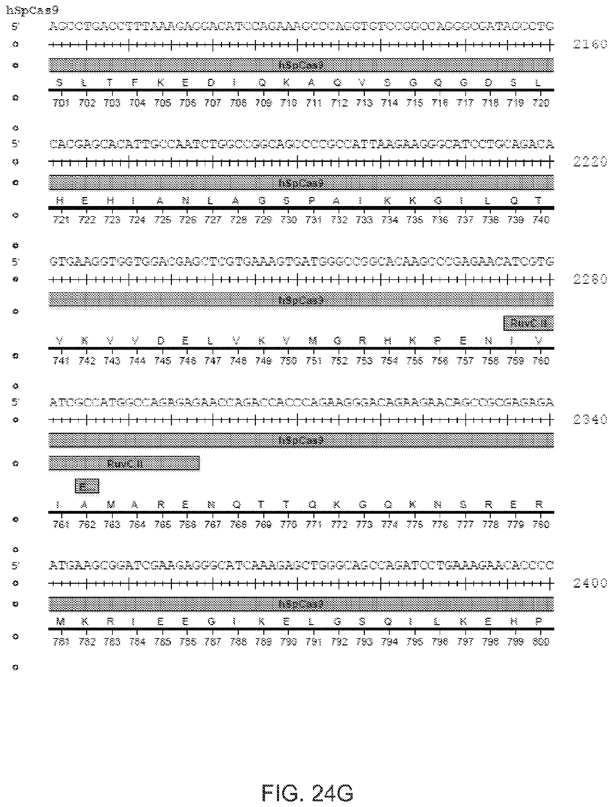

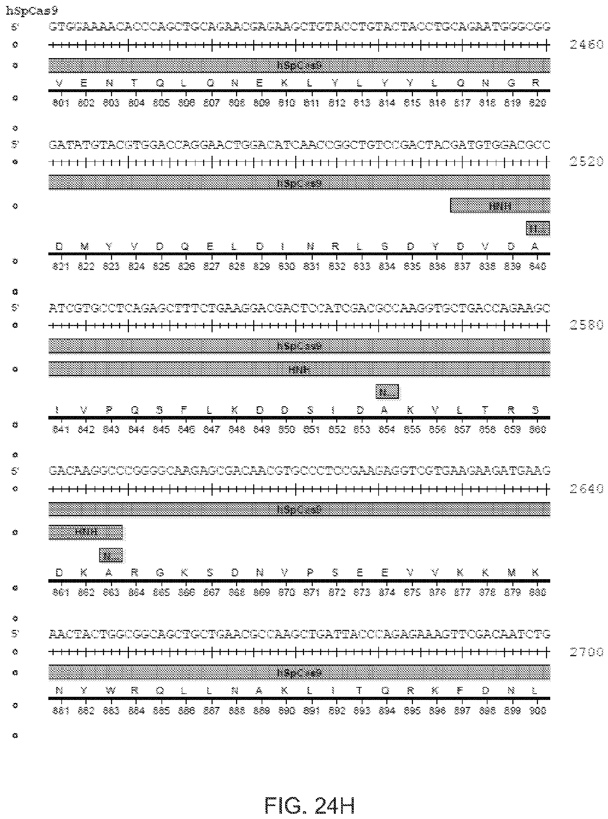

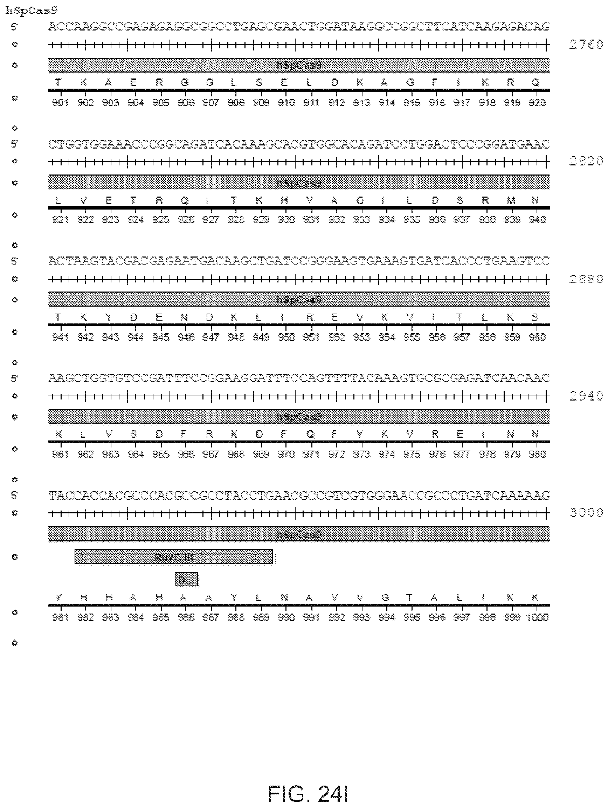

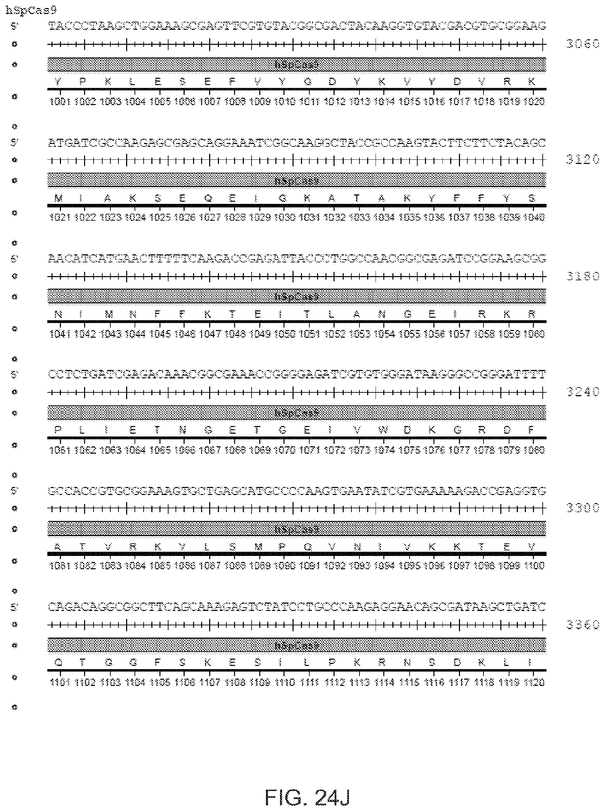

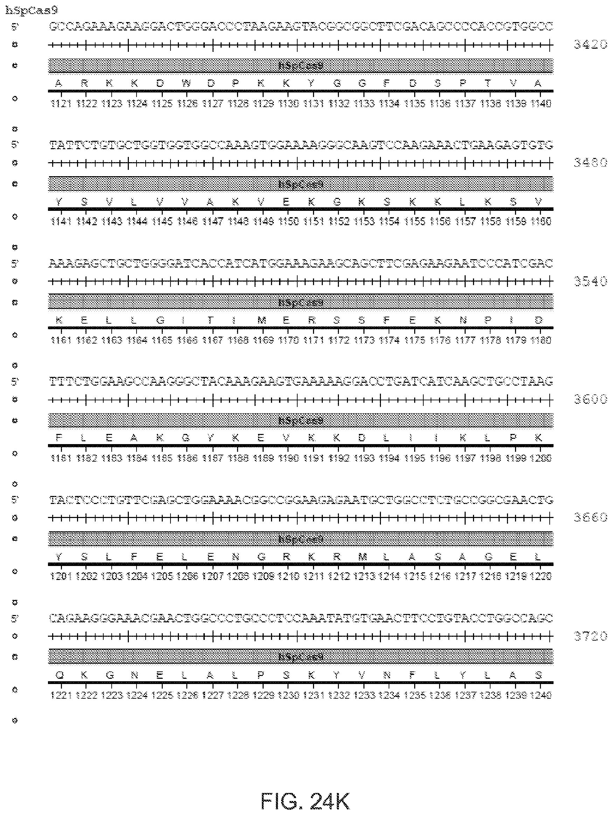

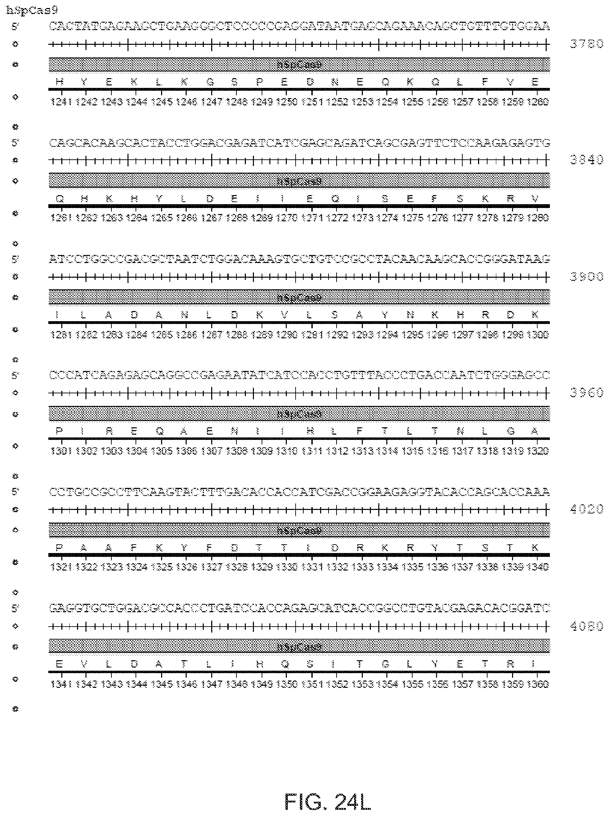

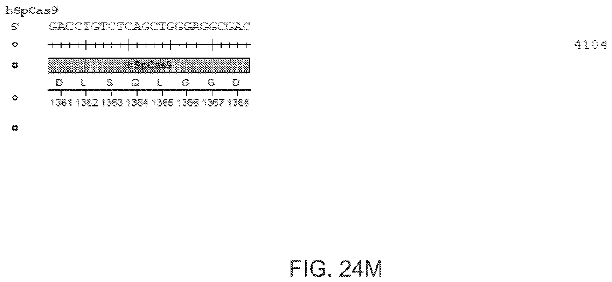

**Figure 24A-M** shows sequences where the mutation points are located within the SpCas9 gene.

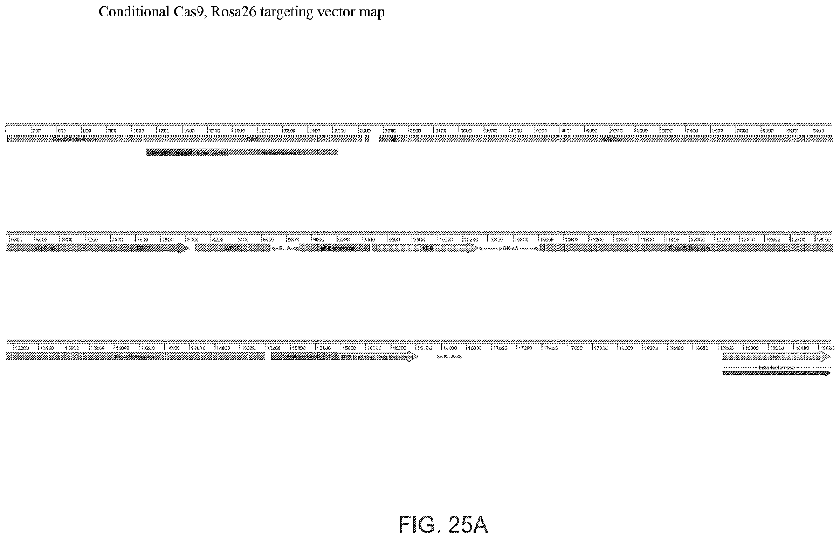

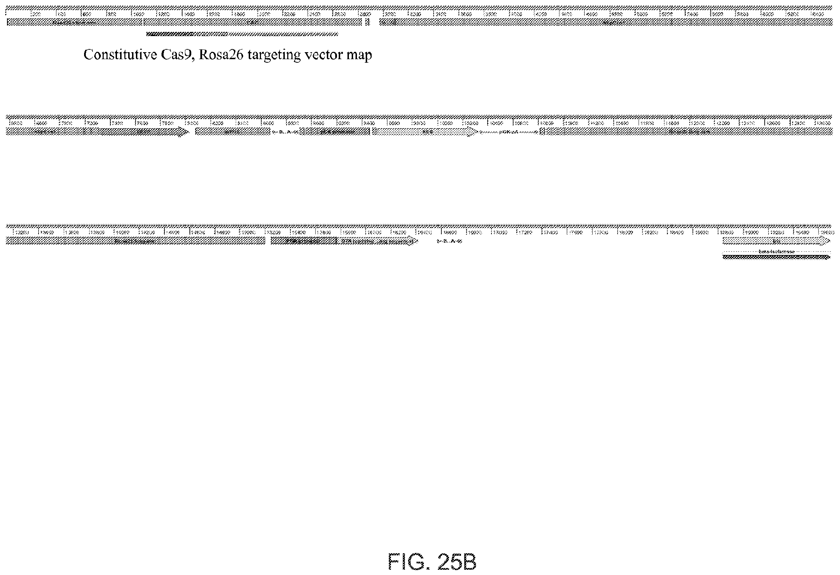

**Figure 25A-B** (A) shows the Conditional Cas9, Rosa26 targeting vector map and (B) shows the Constitutive Cas9, Rosa26 targeting vector map.

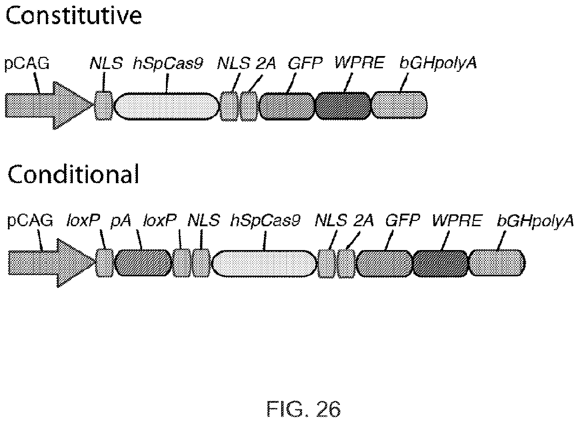

**Figure 26** shows a schematic of the important elements in the Constitutive and Conditional Cas9 constructs.

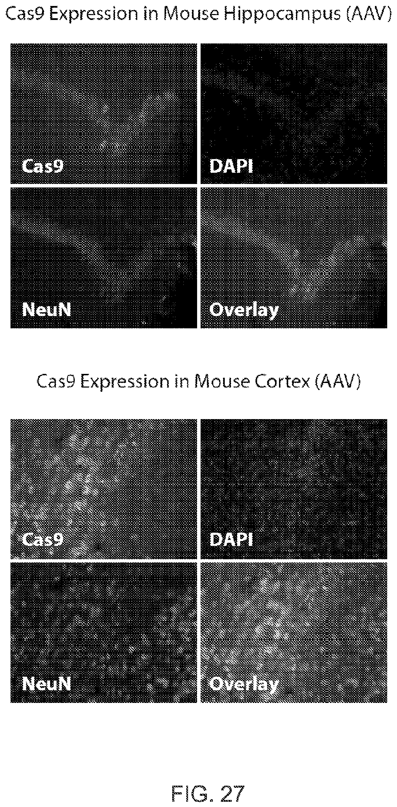

**Figure 27** shows delivery and *in vivo* mouse brain Cas9 expression data.

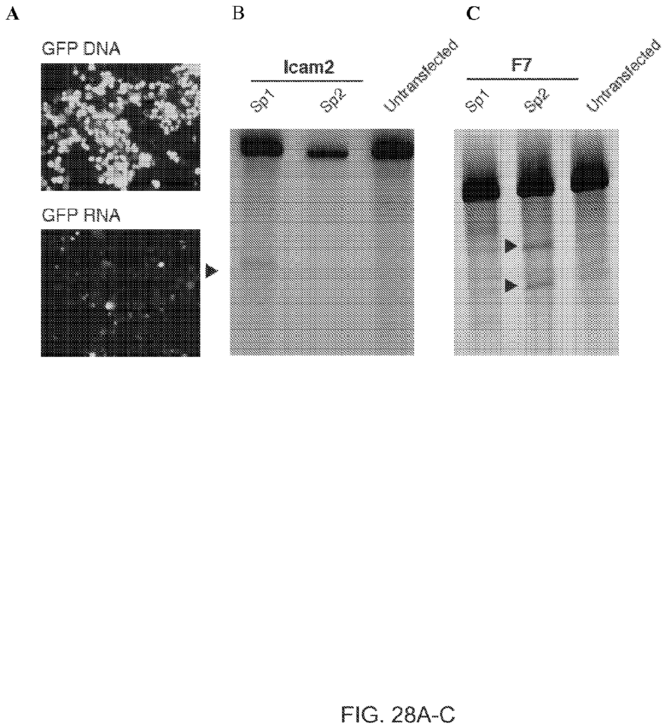

**Figure 28A-C** shows RNA delivery of Cas9 and chimeric RNA into cells (A) Delivery of a GFP reporter as either DNA or mRNA into Neuro-2A cells. (B) Delivery of Cas9 and chimeric RNA against the Icam2 gene as RNA results in cutting for one of two spacers tested. (C) Delivery of Cas9 and chimeric RNA against the F7 gene as RNA results in cutting for one of two spacers tested.

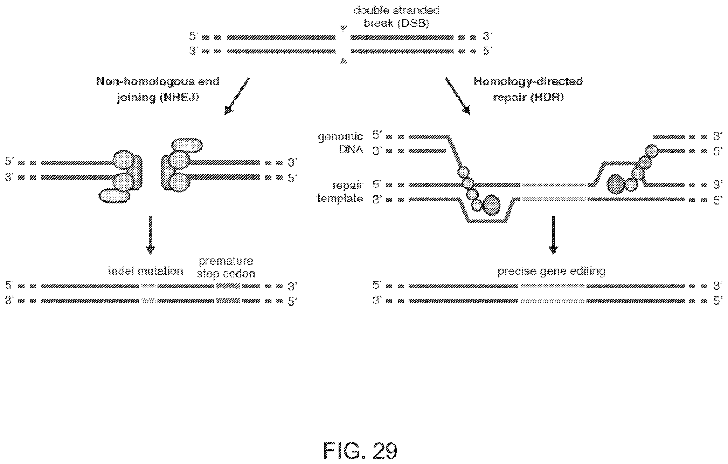

**Figure 29** shows how DNA double-strand break (DSB) repair promotes gene editing. In the error-prone non-homologous end joining (NHEJ) pathway, the ends of a DSB are processed by endogenous DNA repair machineries and rejoined together, which can result in random insertion/deletion (indel) mutations at the site of junction. Indel mutations occurring within the coding region of a gene can result in frame-shift and a premature stop codon, leading to gene knockout. Alternatively, a repair template in the form of a plasmid or single-stranded oligodeoxynucleotides (ssODN) can be supplied to leverage the homology-directed repair (HDR) pathway, which allows high fidelity and precise editing.

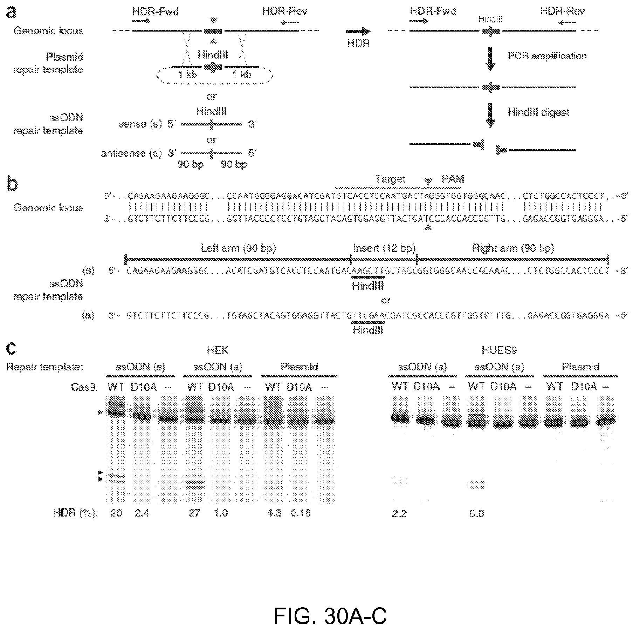

**Figure 30A-C** shows anticipated results for HDR in HEK and HUES9 cells. (a) Either a targeting plasmid or an ssODN (sense or antisense) with homology arms can be used to edit the sequence at a target genomic locus cleaved by Cas9 (red triangle). To assay the efficiency of HDR, Applicants introduced a HindIII site (red bar) into the target locus, which was PCR-amplified with primers that anneal outside of the region of homology. Digestion of the PCR product with HindIII reveals the occurrence of HDR events. (b) ssODNs, oriented in either the sense or the antisense (s or a) direction relative to the locus of interest, can be used in combination with Cas9 to achieve efficient HDR-mediated editing at the target locus. A minimal homology region of 40 bp, and preferably 90 bp, is recommended on either side of the modification (red bar). (c) Example of the effect of ssODNs on HDR in the EMX1 locus is shown using both wild-type Cas9 and Cas9 nickase (D10A). Each ssODN contains homology arms of 90 bp flanking a 12-bp insertion of two restriction sites.

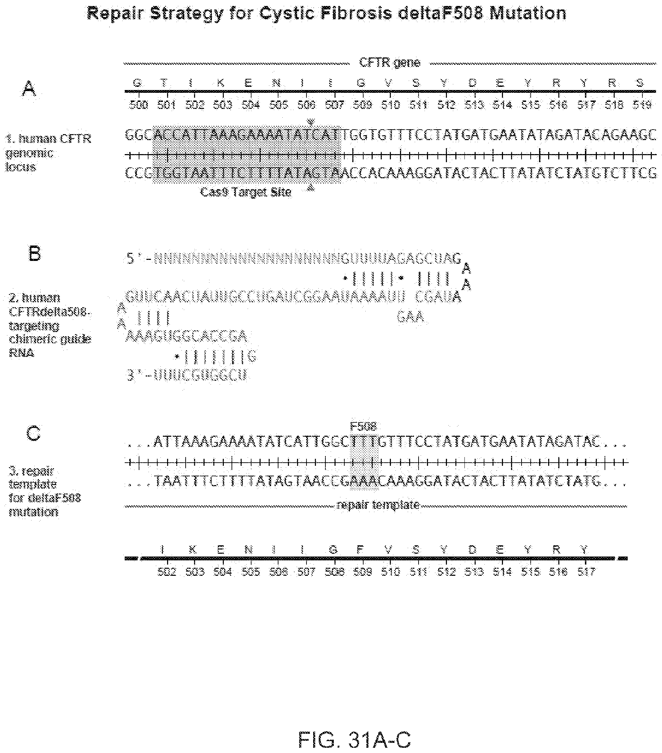

**Figure 31A-C** shows the repair strategy for Cystic Fibrosis delta F508 mutation.

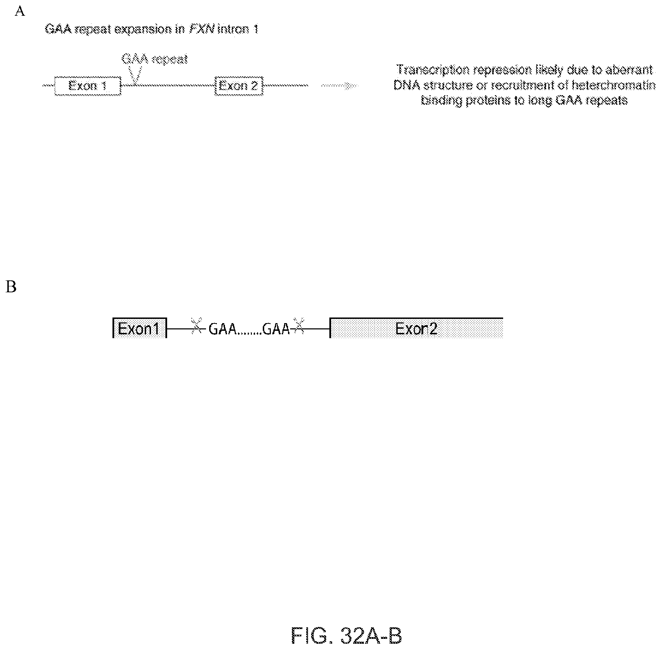

**Figure 32A-B** (a) shows a schematic of the GAA repeat expansion in FXN intron 1 and (b) shows a schematic of the strategy adopted to excise the GAA expansion region using the CRISPR/Cas system.

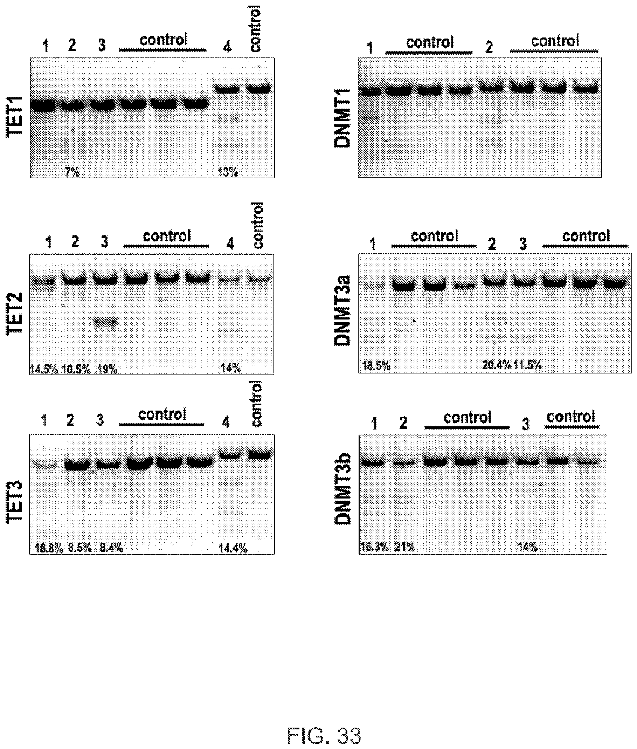

**Figure 33** shows a screen for efficient SpCas9 mediated targeting of Tet1-3 and Dnmt1, 3a and 3b gene loci. Surveyor assay on DNA from transfected N2A cells demonstrates efficient DNA cleavage by using different gRNAs.

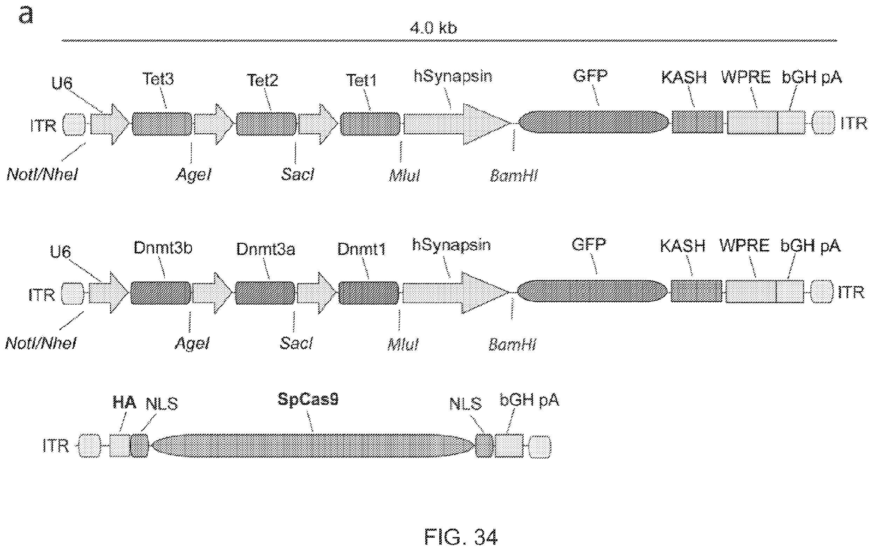

**Figure 34** shows a strategy of multiplex genome targeting using a 2-vector system in an AAV1/2 delivery system. Tet1-3 and Dnmt1, 3a and 3b gRNA under the control of the U6 promoter. GFP-KASH under the control of the human synapsin promoter. Restriction sides shows simple gRNA replacement strategy by subcloning. HA-tagged SpCas9 flanked by two nuclear localization signals (NLS) is shown. Both vectors are delivered into the brain by AAV1/2 virus in a 1:1 ratio.

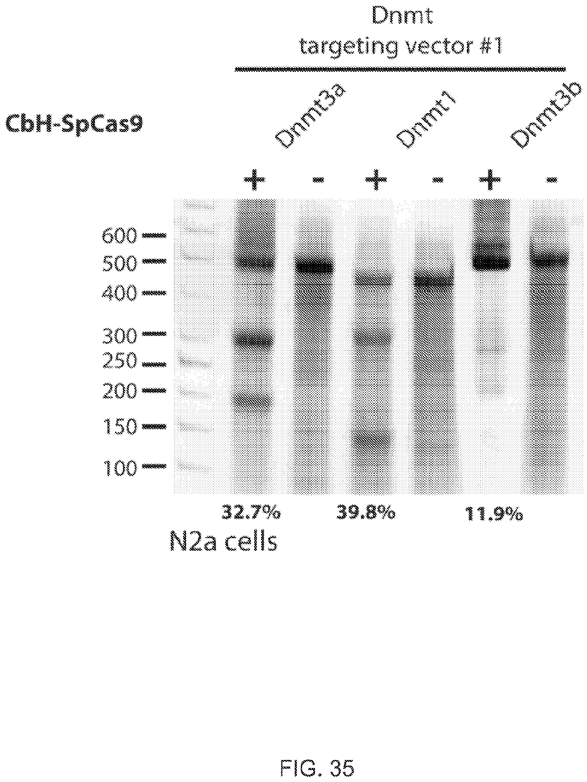

**Figure 35** shows verification of multiplex DNMT targeting vector #1 functionality using Surveyor assay. N2A cells were co-transfected with the DNMT targeting vector #1 (+) and the SpCas9 encoding vector for testing SpCas9 mediated cleavage of DNMTs genes family loci. gRNA only (-) is negative control. Cells were harvested for DNA purification and downstream processing 48 h after transfection.

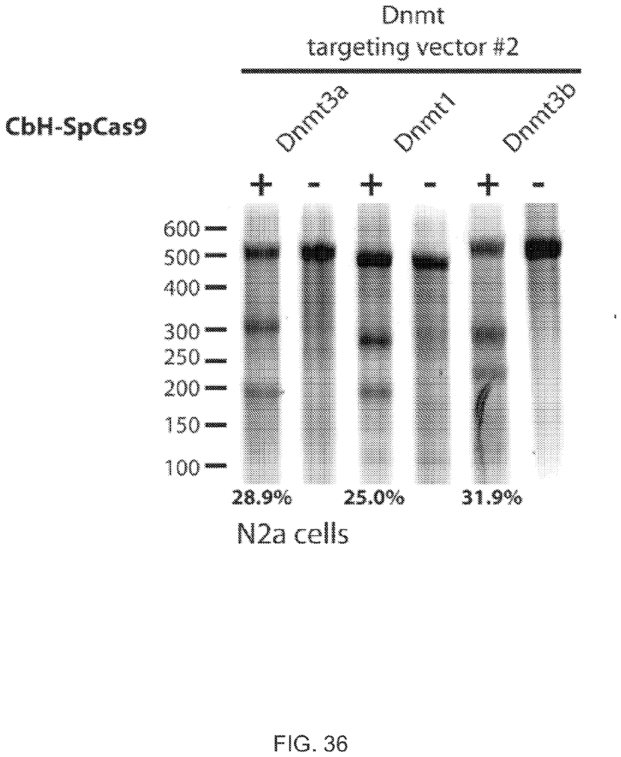

**Figure 36** shows verification of multiplex DNMT targeting vector #2 functionality using Surveyor assay. N2A cells were co-transfected with the DNMT targeting vector #1 (+) and the SpCas9 encoding vector for testing SpCas9 mediated cleavage of DNMTs genes family loci. gRNA only (-) is negative control. Cells were harvested for DNA purification and downstream processing 48 h after transfection.

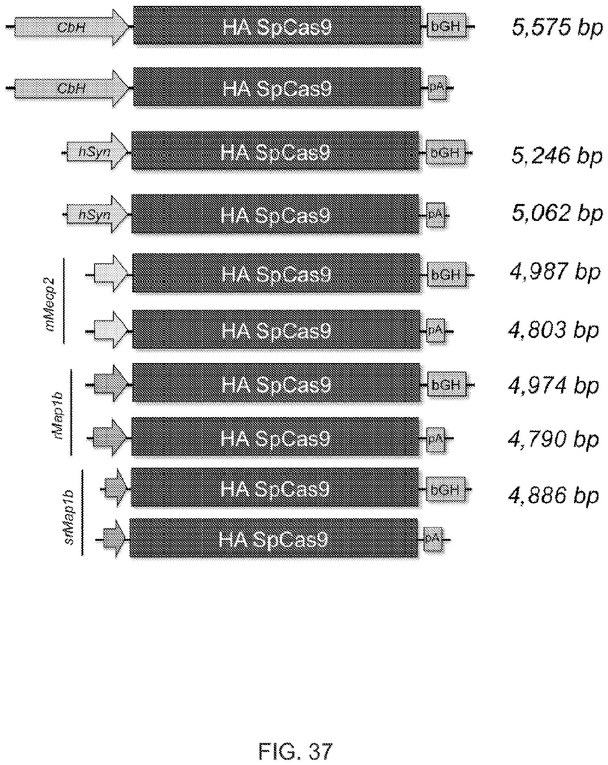

**Figure 37** shows schematic overview of short promoters and short polyA versions used for HA-SpCas9 expression in vivo. Sizes of the encoding region from L-ITR to R-ITR are shown on the right.

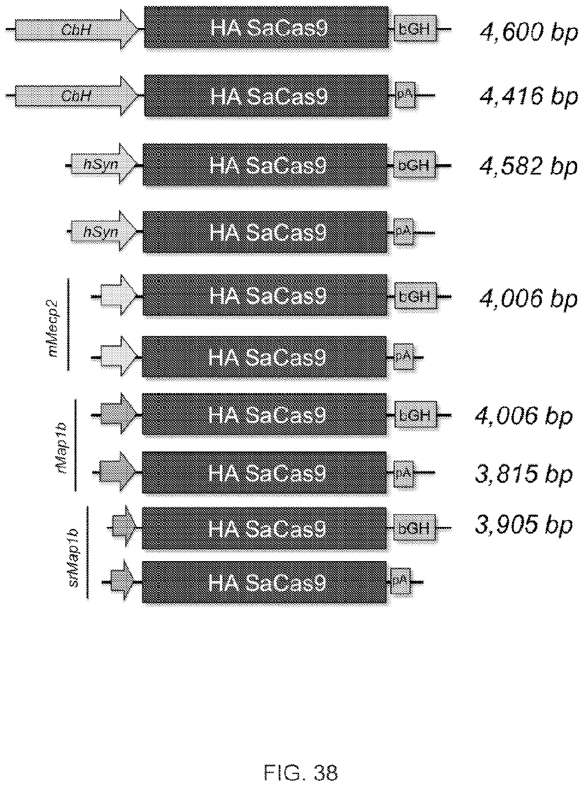

**Figure 38** shows schematic overview of short promoters and short polyA versions used for HA-SaCas9 expression in vivo. Sizes of the encoding region from L-ITR to R-ITR are shown on the right.

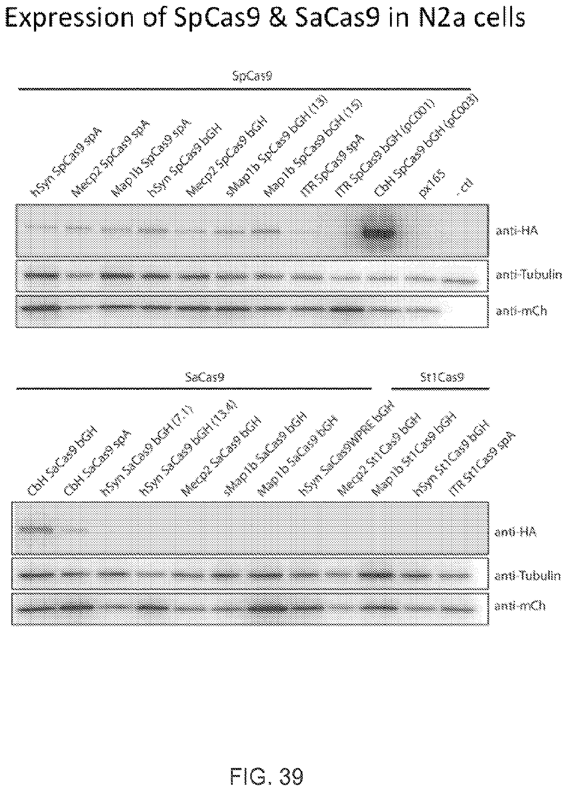

**Figure 39** shows expression of SpCas9 and SaCas9 in N2A cells. Representative Western blot of HA-tagged SpCas9 and SaCas9 versions under the control of different short promoters and with or short polyA (spA) sequences. Tubulin is loading control. mCherry (mCh) is a transfection control. Cells were harvested and further processed for Western blotting 48 h after transfection.

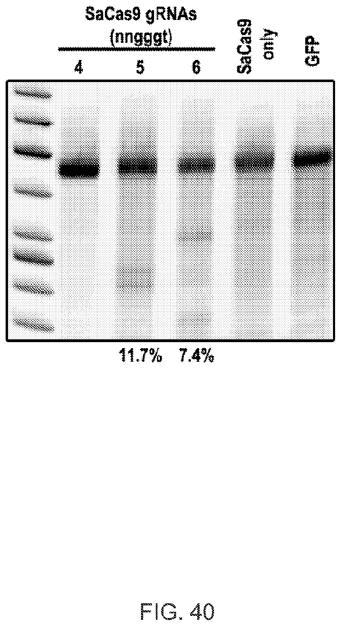

**Figure 40** shows screen for efficient SaCas9 mediated targeting of Tet3 gene locus. Surveyor assay on DNA from transfected N2A cells demonstrates efficient DNA cleavage by using different gRNAs with NNGGGT sequence. GFP transfected cells and cells expressing only SaCas9 are controls.

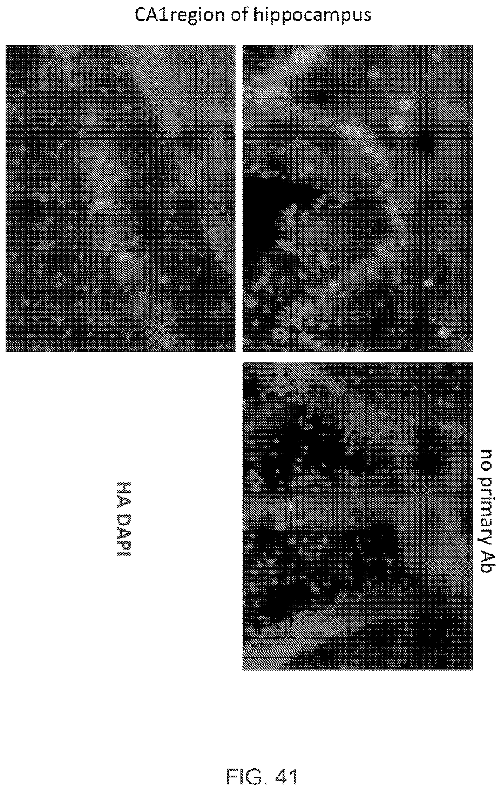

**Figure 41** shows expression of HA-SaCas9 in the mouse brain. Animals were injected into dentate gyri with virus driving expression of HA-SaCas9 under the control of human Synapsin promoter. Animals were sacrificed 2 weeks after surgery. HA tag was detected using rabbit monoclonal antibody C29F4 (Cell Signaling). Cell nuclei stained in blue with DAPI stain.

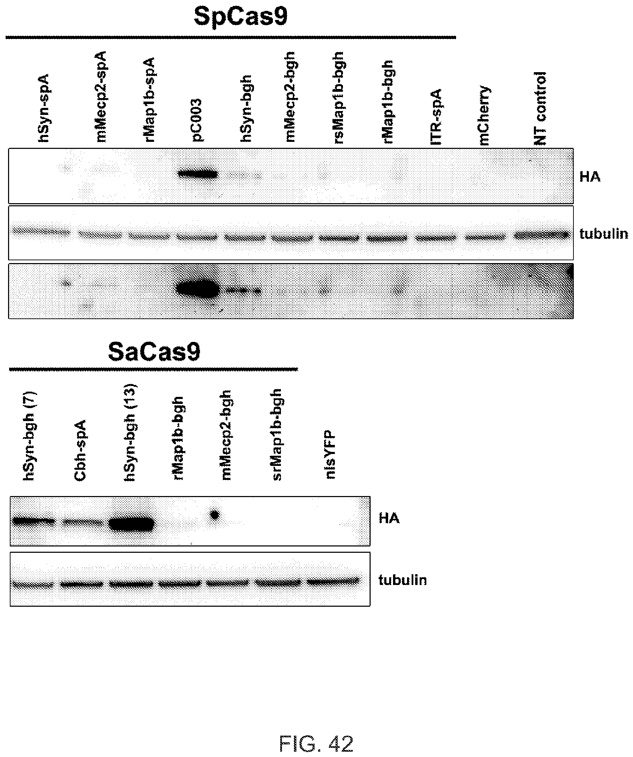

**Figure 42** shows expression of SpCas9 and SaCas9 in cortical primary neurons in culture 7 days after transduction. Representative Western blot of HA-tagged SpCas9 and SaCas9 versions under the control of different promoters and with bgh or short polyA (spA) sequences. Tubulin is loading control.

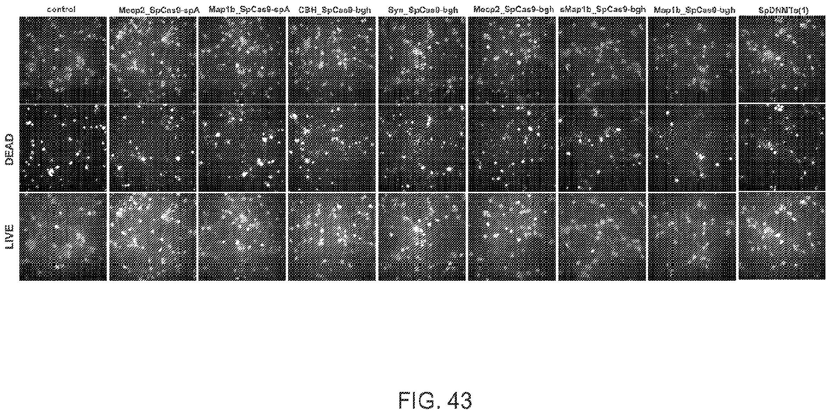

**Figure 43** shows LIVE/DEAD stain of primary cortical neurons 7 days after transduction with AAV1 particles carrying SpCas9 with different promoters and multiplex gRNAs constructs (example shown on the last panel for DNMTs). Neurons after AAV transduction were compared with control untransduced neurons. Red nuclei indicate permeabilized, dead cells (second line of panels). Live cells are marked in green color (third line of panels).

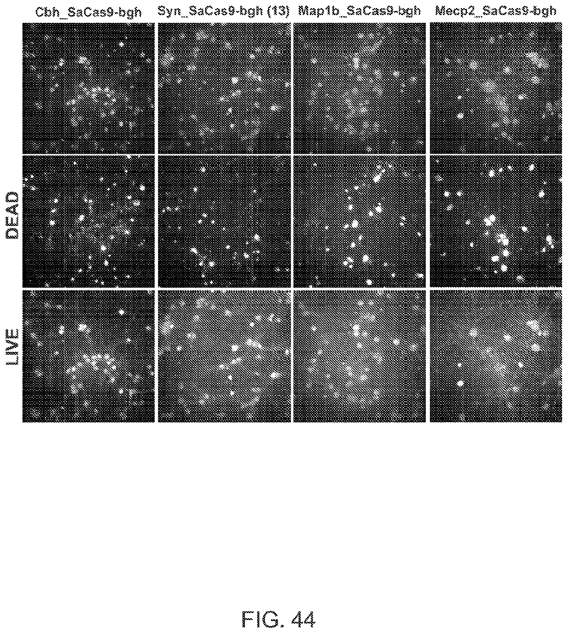

**Figure 44** shows LIVE/DEAD stain of primary cortical neurons 7 days after transduction with AAV1 particles carrying SaCas9 with different promoters. Red nuclei indicate permeabilized, dead cells (second line of panels). Live cells are marked in green color (third line of panels).

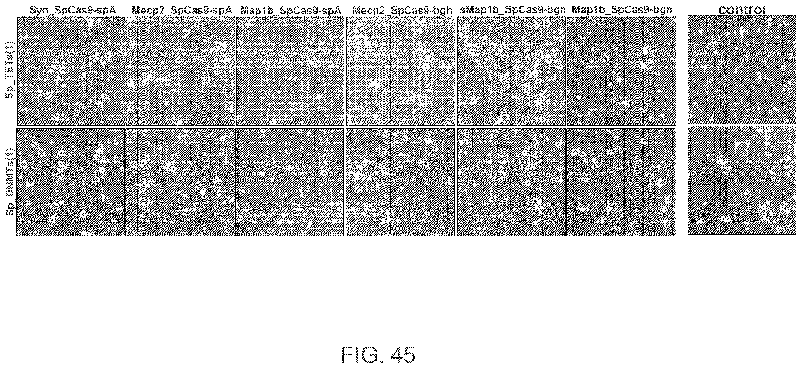

**Figure 45** shows comparison of morphology of neurons after transduction with AAV1 virus carrying SpCas9 and gRNA multiplexes for TETs and DNMTs genes loci. Neurons without transduction are shown as a control.

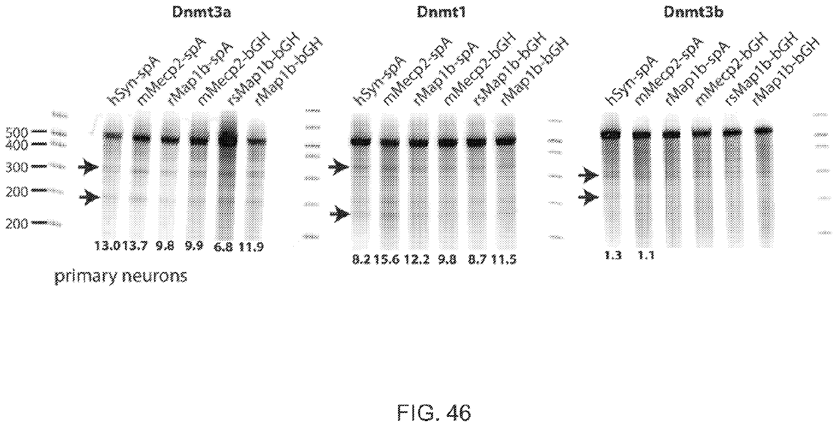

**Figure 46** shows verification of multiplex DNMT targeting vector #1 functionality using Surveyor assay in primary cortical neurons. Cells were co-transduced with the DNMT targeting vector #1 and the SpCas9 viruses with different promoters for testing SpCas9 mediated cleavage of DNMTs genes family loci.

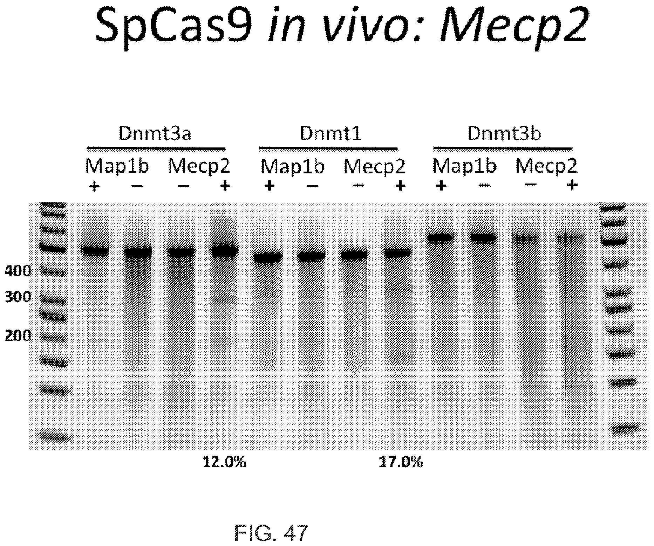

**Figure 47** shows in vivo efficiency of SpCas9 cleavage in the brain. Mice were injected with AAV1/2 virus carrying gRNA multiplex targeting DNMT family genes loci together with SpCas9 viruses under control of 2 different promoters: mouse Mecp2 and rat Maplb. Two weeks after injection brain tissue was extracted and nuclei were prepped and sorted using FACS, based on the GFP expression driven by Synapsin promoter from gRNA multiplex construct After gDNA extraction Surveyor assay was run. + indicates GFP positive nuclei and - control, GFP-negative nuclei from the same animal. Numbers on the gel indicate assessed SpCas9 efficiency.

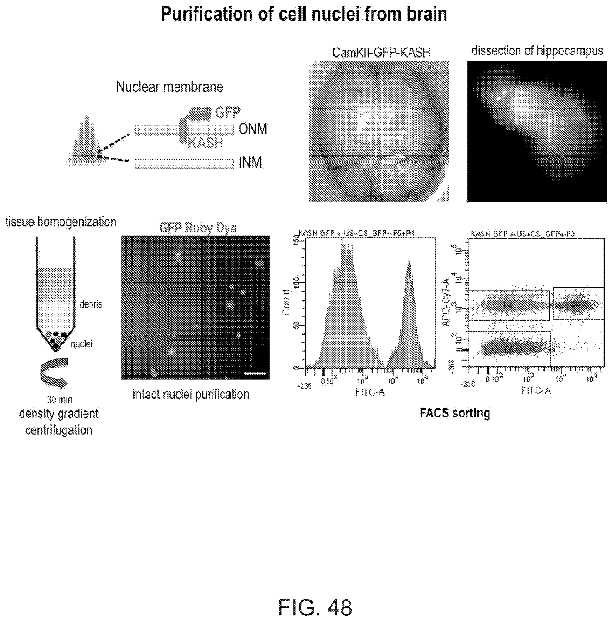

**Figure 48** shows purification of GFP-KASH labeled cell nuclei from hippocampal neurons. The outer nuclear membrane (ONM) of the cell nuclear membrane is tagged with a fusion of GFP and the KASH protein transmembrane domain. Strong GFP expression in the brain after one week of stereotactic surgery and AAV1/2 injection. Density gradient centrifugation step to purify cell nuclei from intact brain. Purified nuclei are shown. Chromatin stain by Vybrant® DyeCycle™ Ruby Stain is shown in red, GFP labeled nuclei are green. Representative FACS profile of GFP+ and GFP- cell nuclei (Magenta: Vybrant® DyeCycle™ Ruby Stain, Green: GFP).

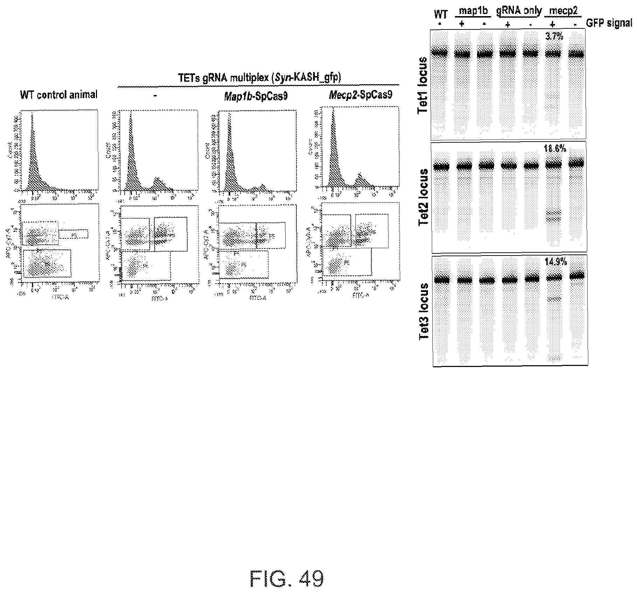

**Figure 49** shows efficiency of SpCas9 cleavage in the mouse brain. Mice were injected with AAV1/2 virus carrying gRNA multiplex targeting TET family genes loci together with SpCas9 viruses under control of 2 different promoters: mouse Mecp2 and rat Maplb. Three weeks after injection brain tissue was extracted, nuclei were prepped and sorted using FACS, based on the GFP expression driven by Synapsin promoter from gRNA multiplex construct After gDNA extraction Surveyor assay was run. + indicates GFP positive nuclei and - control, GFP-negative nuclei from the same animal. Numbers on the gel indicate assessed SpCas9 efficiency.

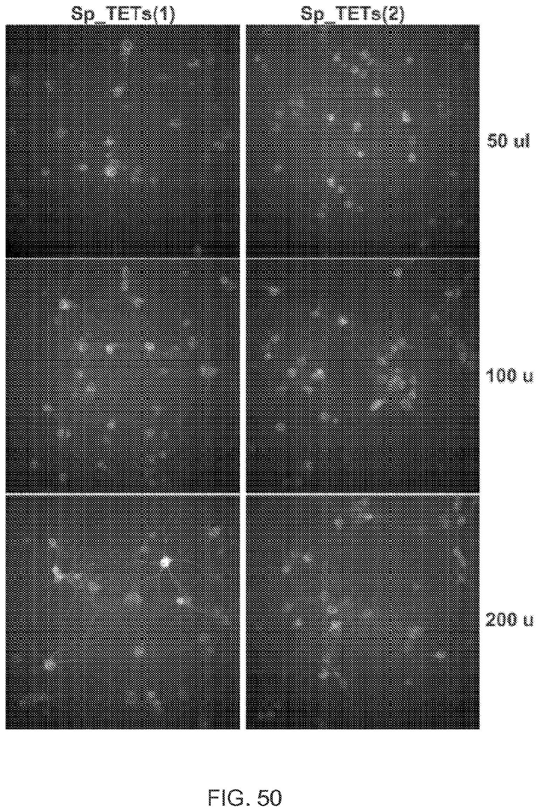

**Figure 50** shows GFP-KASH expression in cortical neurons in culture. Neurons were transduced with AAV1 virus carrying gRNA multiplex constructs targeting TET genes loci. The strongest signal localize around cells nuclei due to KASH domain localization.

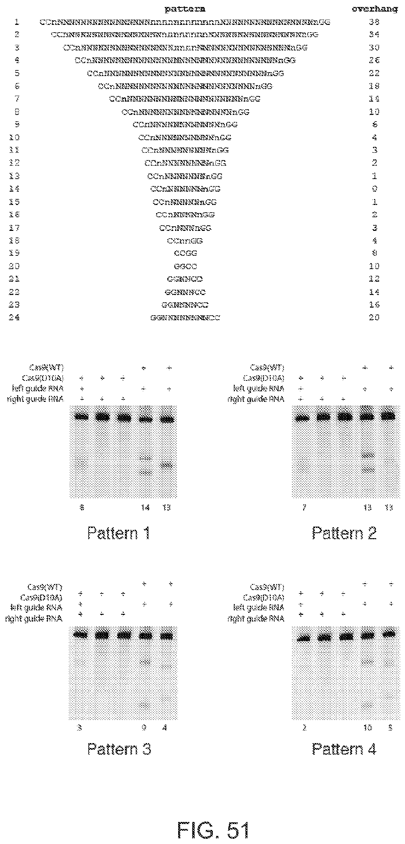

**Figure 51** shows (top) a list of spacing (as indicated by the pattern of arrangement for two PAM sequences) between pairs of guide RNAs. Only guide RNA pairs satisfying patterns 1, 2, 3, 4 exhibited indels when used with SpCas9(D10A) nickase. (bottom) Gel images showing that combination of SpCas9(D10A) with pairs of guide RNA satisfying patterns 1, 2, 3, 4 led to the formation of indels in the target site.

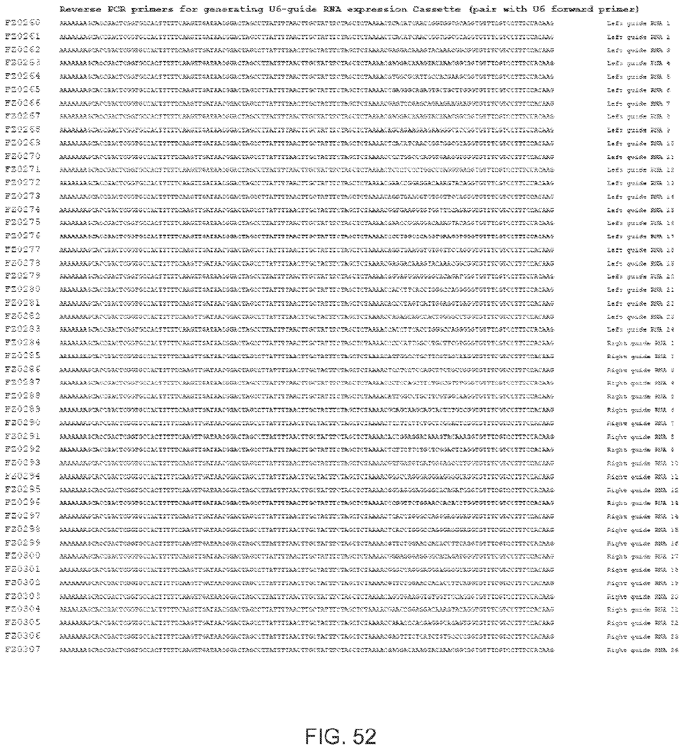

**Figure 52** shows a list of U6 reverse primer sequences used to generate U6-guide RNA expression casssettes. Each primer needs to be paired with the U6 forward primer "gcactgagggcctatttcccatgattc" (SEQ ID NO: _) to generate amplicons containing U6 and the desired guide RNA.

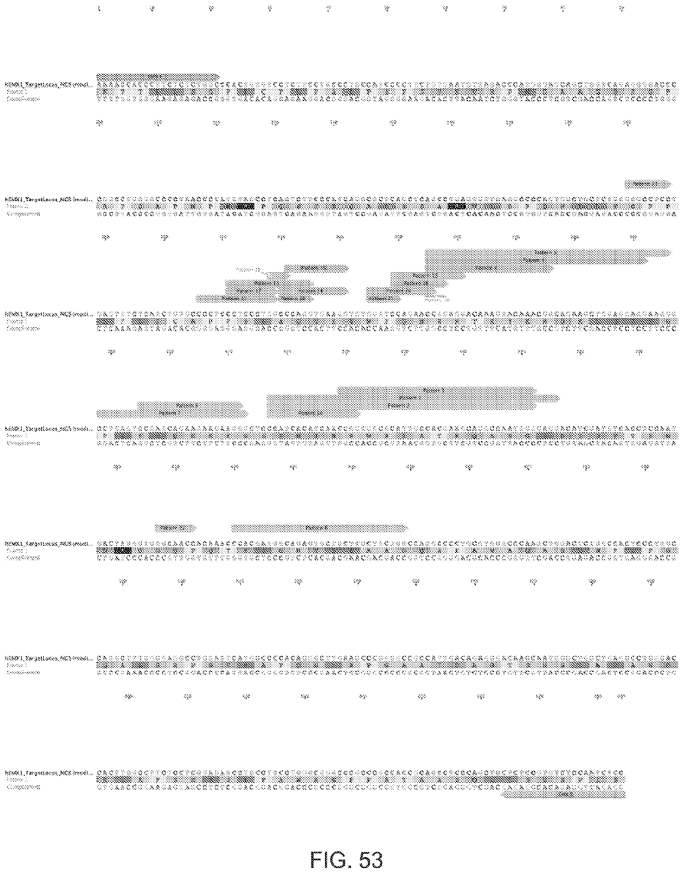

**Figure 53** shows a Genomic sequence map from the human Emx1 locus showing the locations of the 24 patterns listed in Fig. 51.

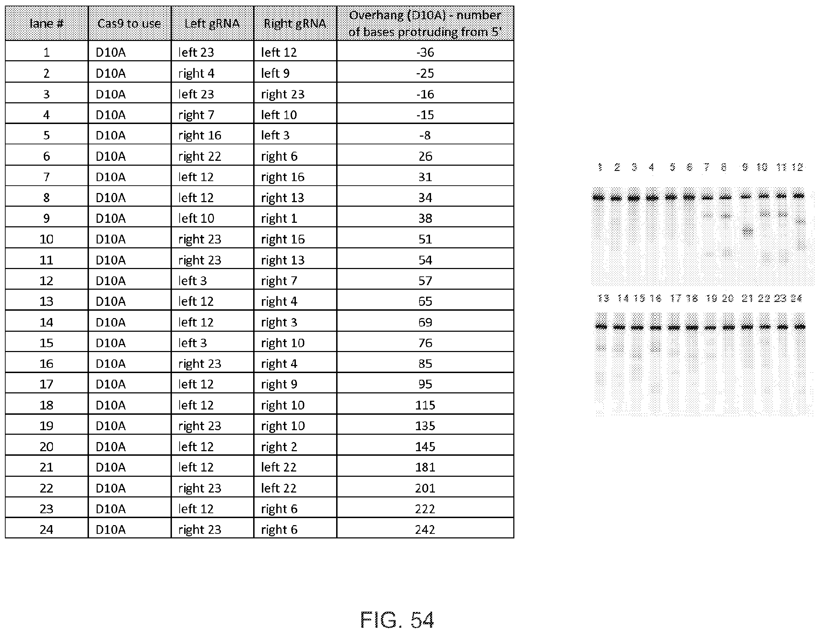

**Figure 54** shows on (right) a gel image indicating the formation of indels at the target site when variable 5' overhangs are present after cleavage by the Cas9 nickase targeted by different pairs of guide RNAs. On (left) a table indicating the lane numbers of the gel on the right and various parameters including identifying the guide RNA pairs used and the length of the 5' overhang present following cleavage by the Cas9 nickase.

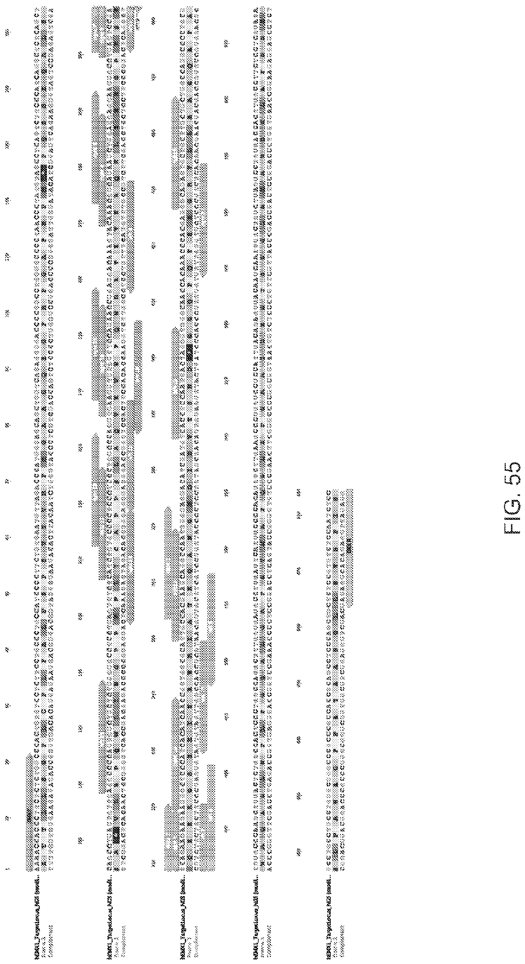

**Figure 55** shows a Genomic sequence map from the human Emx1 locus showing the locations of the different pairs of guide RNAs that result in the gel patterns of Fig. 54 (right) and which are further described in Example 35.

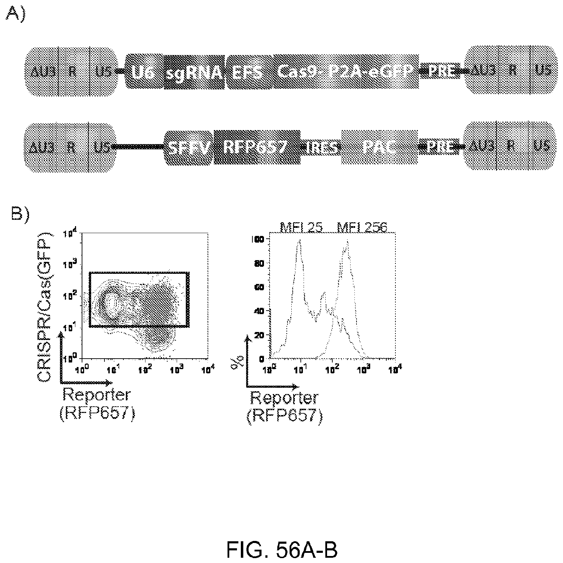

**Figure 56A-E** shows stable modification of hematopoietic stem cells by a lentiviral CRISPR-Cas delivery system A) Depiction of a lentiviral vector for bi-cistronic expression of the sgRNA from a U6 promoter (U6) and Cas9 from a short EF1a promoter (EFS) with a fluorescent protein marker (eGFP) from a picorna virus derived 2A auto-cleavage site (P2A). A lentiviral vector that allows incorporation of target sites into the coding sequence of a fluorescent protein (RFP657) is depicted at the bottom. A Puromycin resistance gene (PAC) is expressed from an internal ribosomal entry site (IRES) B) Super-transduction of a reporter cell line with an eGFP tagged CRISPR/Cas vector targeting the respective site (blue) induces loss of reporter fluorescence. A non-targeting CRISPR/Cas vector (red) does not affect reporter fluorescence. Quantification by plotting the mean fluorescent intensity (MFI) of the reporter in targeting (blue) vs non-targeting (red) sgRNA transduced cells (black box, left plot). C) Efficacy of CRISPR/Cas spacers for recurrently mutated genes in myeloid malignancy. Efficacy was assessed with the RFP657 reporter system described in A) and B). Results were normalized to non-targeting spacer. Spacers marked in red were used in following experiments. D) Peripheral blood CRISPR/Cas vector marking tracked over a period of 19 weeks. A significant increase of CRISPR/Cas expressing cells with a spacer targeting Runxl (n=4) was observed in comparison to mice expressing a non-targeting spacer (n=4). E) Stable expression of the CRISPR/Cas vector after 19 weeks in hematopoietic stem and progenitor cells. Overlay of eGFP expression in CRISPR/Cas transduced cells (blue) and non-transduced cells (red) of the respective cell population. MPP=Multipotent progenitors; ST-HSC=short-term HSCs; LT-HSC=long-term HSCs; LT-HSC CD34-=most quiescent long-term-HSCs.

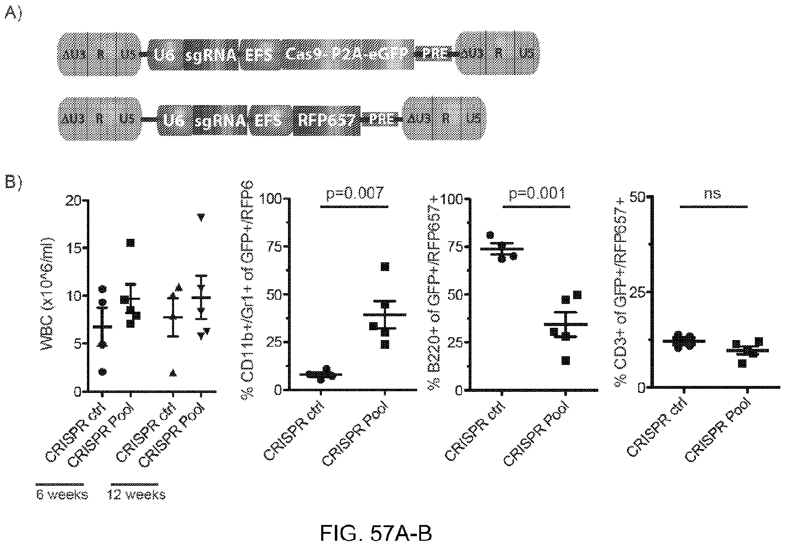

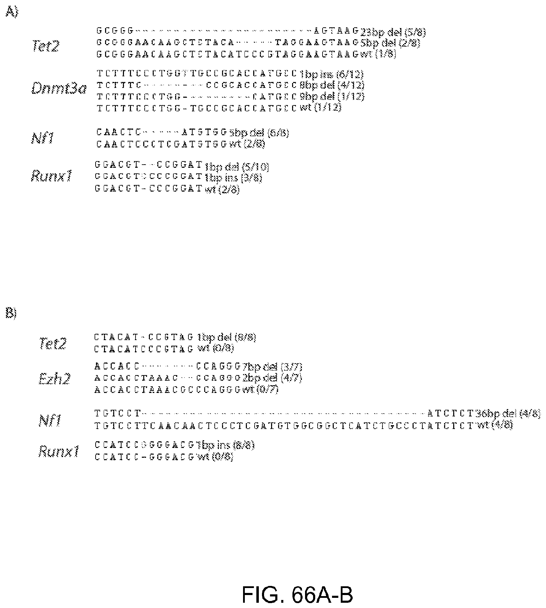

**Figure 57A-D** shows multiplex gene targeting induces clonal development *in vivo* A) Schematic for multiplex gene targeting with two lentiviral vectors: a CRISPR/Cas-eGFP vector expressing sgRNA, Cas9 and eGFP, and a vector expressing additional sgRNAs with a RFP657 fluorescent reporter gene from a short EFla promoter. B) Multiplexed gene targeting in mice (n=5) significantly increases myelomonocytic cells (Gr1+/CD11b+), decreases B-cells (B220+), and increases white blood cell counts at 12 weeks post-transplant in comparison to mice that were transplanted with control sgRNA vector transduced cells (n=4). C) Peripheral blood CRISPR/Cas-eGFP vector expression (x-axis) and expression of a pool of vectors expressing RFP657 (y-axis) over time starting 4 weeks post-transplant. Clonal populations were detected by the pattern of eGFP/RFP657 expression. Two clones were isolated (red gates indicate clones) and genomic regions of targeted genes were sequenced (displayed in red box). Mono- and bi-allelic deletions in *Tet2, Dnmt3a, Nf1* and *Runx1* were detected in one case. Detected mutations at 8 weeks are shown below. Sequences of the deletions are shown in Fig. 66A. D) Another clone was analyzed as in panel C, and was found to have mutations in Tet2, Ezh2, Nf1, and Runxl B-II. Detected mutations at 8 weeks are shown below. Sequences of the deletions are shown in Fig. 66B.

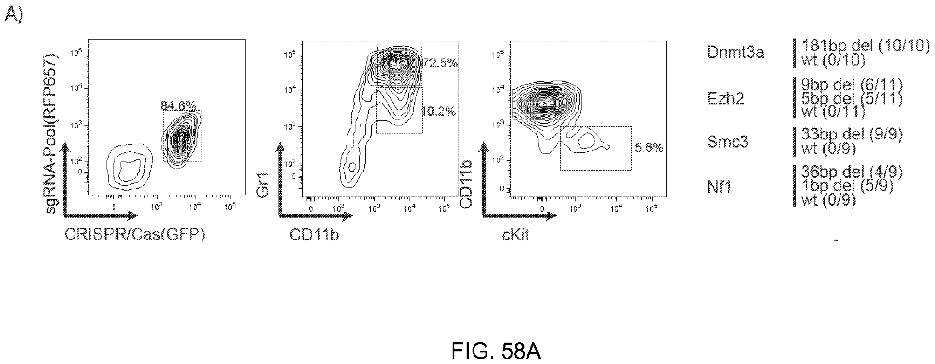

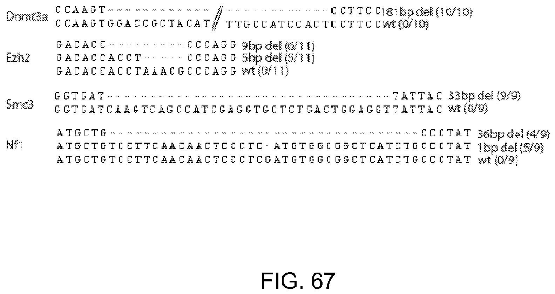

**Figure 58A-G** shows myeloid malignancy modeling with multiplex genome editing A) Flow cytometric analysis of a diseased C57B1/6 mouse for expression of the introduced CRISPR/Cas vectors and for myeloid (Gr1+/CD11b+). Detected mutations are shown in the right panel (displayed in red box). Sequence alignments are shown in Fig. 67. B) Peripheral blood smear (left; May-Gruenwald/Giemsa (MGG); 400x) and BM (right; Hematoxylin-Eosin(HE), 400x) showing granulocytic cells and blasts. C) Western blots of Ezh2, Smc3, and activated Erkl/2 (pErk) protein levels in the leukemic BM compared to normal C57B1/6 wild-type BM. D) Serial replating capacity of normal BM compared to the leukemic BM. E) Representative flow cytometry of secondary transplant recipient mouse peripheral blood. F) Lineage distribution in peripheral blood from secondary mice 8 weeks post-transplant (n=5). G) Histopathology analysis of blood smear (MGG; 400x) from a secondary mouse shows myelo-monocytic leukocytosis and blast cells in peripheral blood.

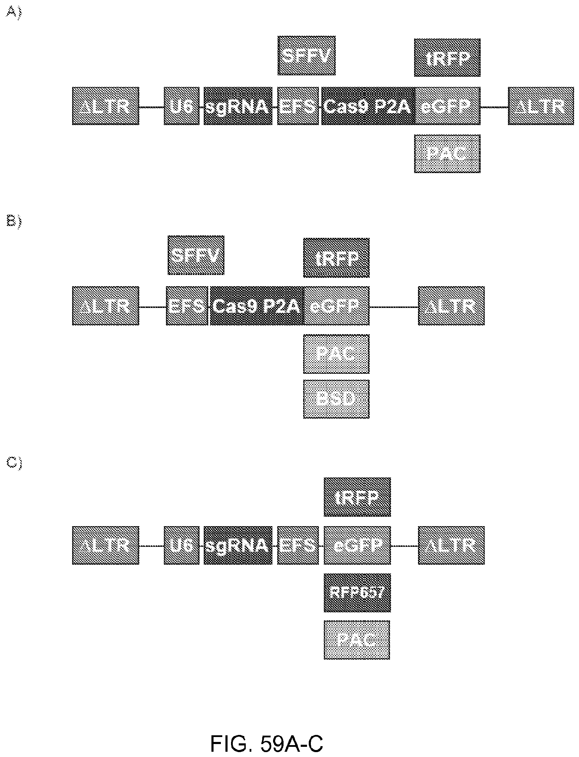

**Figure 59A-C** shows a modular CRISPR/Cas lentiviral vector system for genome engineering A) Schematic depiction of a lentiviral vector for bi-cistronic expression of the sgRNA from a U6 promoter (U6) and expression of Cas9 from a short EF1a promoter (EFS) or Spleen Focus Forming Virus (SFFV) promoter with a fluorescent protein marker (eGFP or tagRFP) or drug selectable marker (Purmomycin acetyl transferase (PAC) or Blasticidin deaminase (BSD)) from a picorna virus derived 2A auto-cleavage site (P2A). B) Depiction of a lentiviral vector for expression of Cas9 without the sgRNA. C) Depiction of a lentiviral vector for expression of the sgRNA only with a fluorescent protein marker or selectable marker form an EFS promoter.

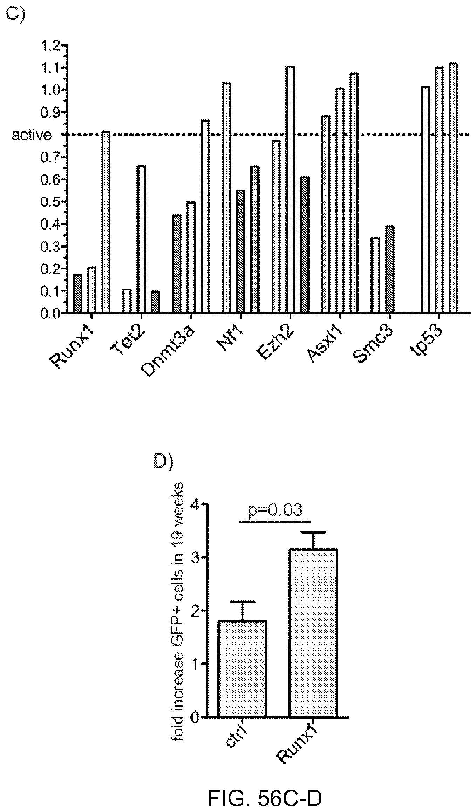

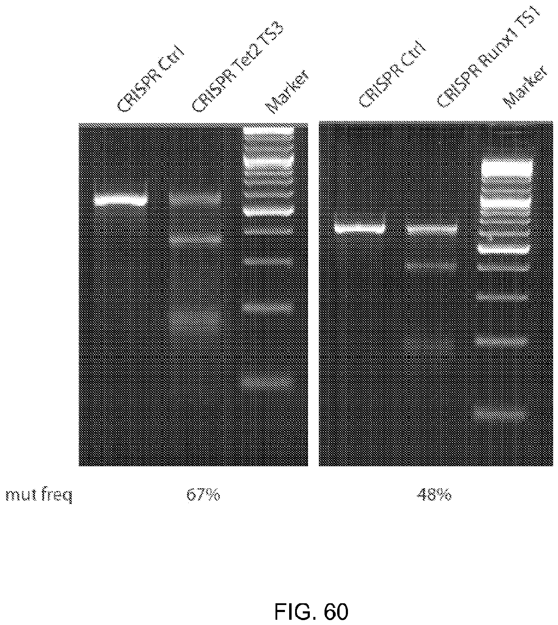

**Figure 60** shows that Lentiviral CRISPR-Cas mediates highly efficient genome editing at endogenous loci Surveyor Assay on targeted region of Tet2 target site 3(TS3) (left, marked red in Fig. 56C) and Runxl target site 1 (TS1) (right, marked red in Fig. 56C) one week post-transduction of murine BaF3 cells with a lentiviral CRISPR-Cas vector. CRISPR/Cas expressing cells were purified by FACS. The percentage of cleaved PCR product is noted below. Control sgRNA transduced cells served as negative control.

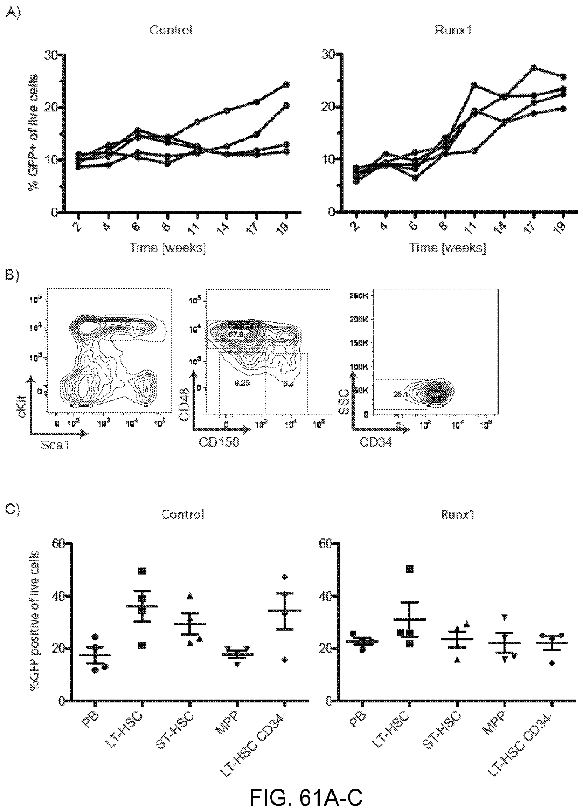

**Figure 61A-C** shows CRISPR/Cas mediated knock out of Runxl in Flt3-ITD heterozygous HSCs induces a competitive advantage A) Flow cytometric assessment of CRISPR/Cas transduced cells in the peripheral blood of mice over a period of 19 weeks after transplantation (control sgRNA left; Runxl sgRNA right). B) Gating scheme of HSPC populations in mice transduced with CRISPR/Cas lentiviral vectors. C) Summarized CRISPR/Cas vector expression in different HSPC populations at 19 weeks post-transplantation for control sgRNA (left) or Runxl sgRNA (right). MPP = Multipotent progenitors (LSK CD150- CD48+); ST-HSC = short-term HSCs (LSK CD150- CD48-); LT-HSC = long-term HSCs (LSK CD150+ CD48-); LT-HSC CD34- = most quiescent long-term-HSCs (LSK CD150+ CD48- CD34-).

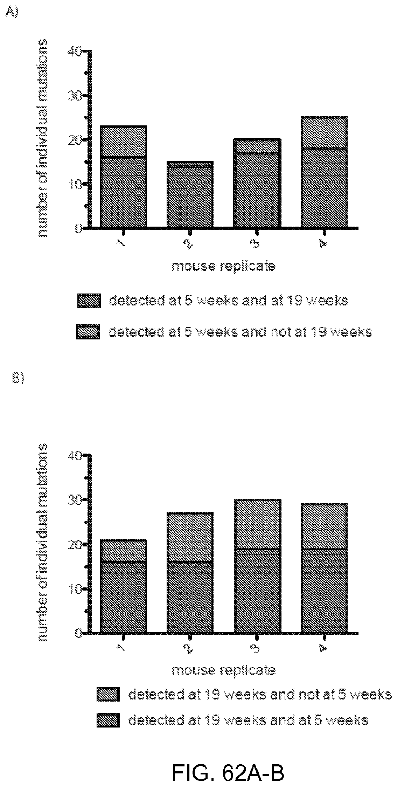

**Figure 62A-B** shows stable genome modification in long-term repopulating HSCs in-vivo by Next Generation Sequencing. Mutational analysis of peripheral blood samples at 5 weeks post-transplant and 19 weeks post-transplant. A) Samples were analyzed to detect all unique mutations from each mouse at 5 weeks and at 19 weeks for mutations at the Runx1 target site. Mutations detected at both time points and only at 5 weeks are shown. B) Mutations found at 19 weeks but not at 5 weeks are shown.

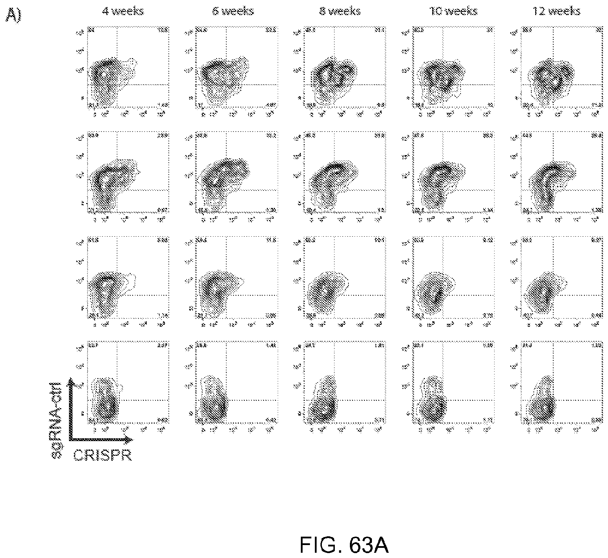

**Figure 63A-B** shows flow cytometry based clonality analysis of C57B1/6 mice shows expansion of CRISPR/Cas engineered populations over time Flow cytometric assessment of CRISPR/Cas vector expression (eGFP) and sgRNAonly vector expression (RFP657) for control sgRNA (A) and pooled sgRNAs (B) in C57B1/6 wild-type mice. Each row shows vector expression at the given time point from an individual mouse.

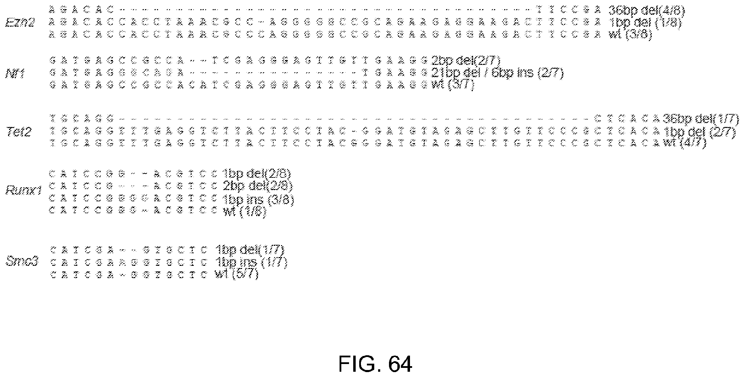

**Figure 64** shows sequence alignment of targeted gene loci in a putatively clonal CRISPR/Cas modified population with dim Cas9-eGFP expression shows gene editing Alignment of sequences obtained for targeted genes from unsorted peripheral blood of a mouse that presented with expanding RFP657 positive and eGFP-dim/negative population. The ratio of the number of bacterial clones with each sequence over the total clones sequenced for the respective gene is shown following each sequence.

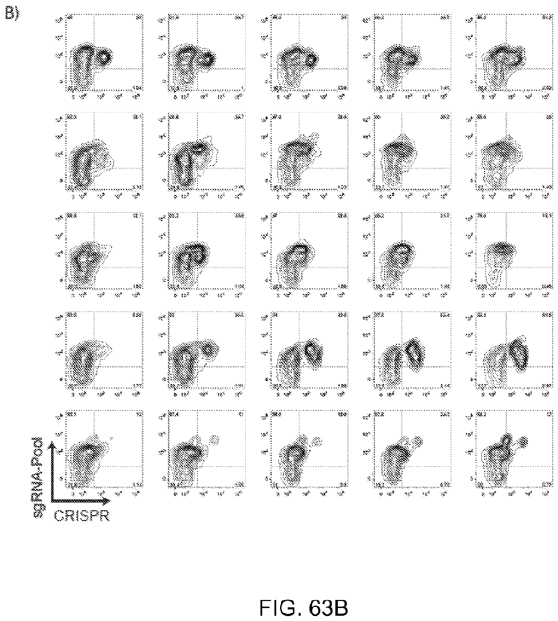

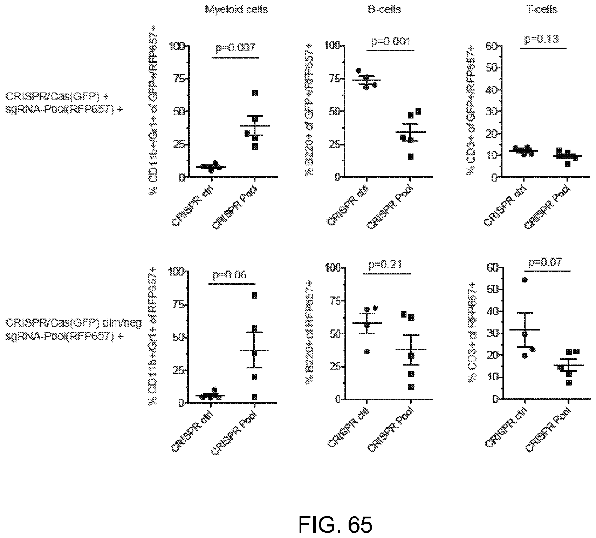

**Figure 65** shows Myeloid lineage skewing induced by multiplex CRISPR/Cas in eGFP-high and eGFP-dim CRISPR/Cas expressing populations Flow cytometric assessment of mature hematopoietic lineage (myeloid = CD11b+/Gr1+; B-cells = B220 (CD45R)+; T-cells = CD3e+) distribution in mice transduced with sgRNA-control or sgRNA-pool 10 weeks post-transplant. Mice are shown in Fig. 63. Upper panel shows lineages distribution in GFP+ / RFP657+ populations in mice with pooled targeting CRISPR/Cas vectors compared to CRISPR/Cas non-targeting controls. Lower panel shows lineages distribution in GFP-dim/neg / RFP657+ populations in mice with pooled targeting CRISPR/Cas vectors compared to CRISPR/Cas non-targeting controls. Statistical differences were using t-test (two-sided, unpaired). Welch correction was performed if statistical significant variance was detected.

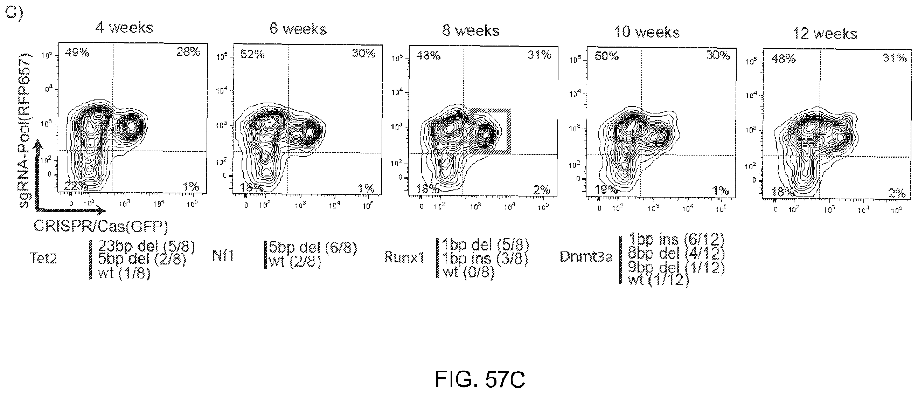

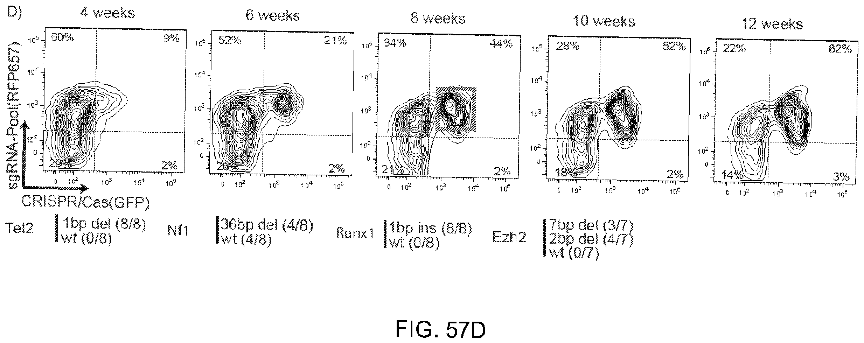

**Figure 66A-B** shows Sequence alignments of genes mutated in putatively clonal hematopoietic CRISPR/Cas modified populations identified after multiplex CRISPR/Cas genome engineering A) Alignment of sequences obtained for targeted genes from sorted cell populations shown in Fig. 57. The ratio of the number of bacterial clones with each sequence over the total clones sequenced for the respective gene is shown following each sequence. A) Genes found to be mutated in the clone shown in Fig. 57C. B) Genes found to be mutated for the clone shown in Fig. 57D.

**Figure 67** shows sequence alignments of *Dnmt3a, Ezh2, Smc3,* and *Nf1* mutations in AML cells. Alignment of sequences obtained for targeted genes from sorted leukemic cells shown in Fig. 58A. The ratio of the number of bacterial clones with each sequence over the total clones sequenced for the respective gene is shown following each sequence.

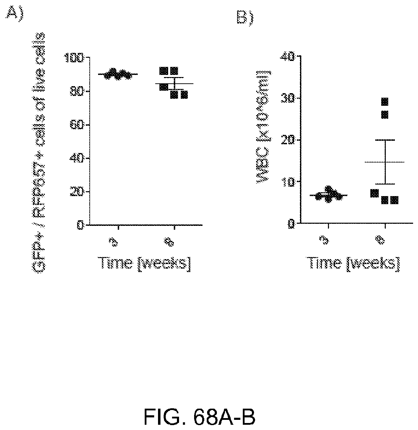

**Figure 68A-B** shows lentivirus CRISPR-Cas induced AML engrafts secondary recipients A) Engraftment of primary leukemic cells in secondary recipients was assessed by flow cytometry of peripheral blood for eGFP and RFP657 expressing cells at 3 and 8 weeks post transplant. B) White blood cell counts (WBC) of secondary recipients was assessed at 3 and 8 weeks post transplant.

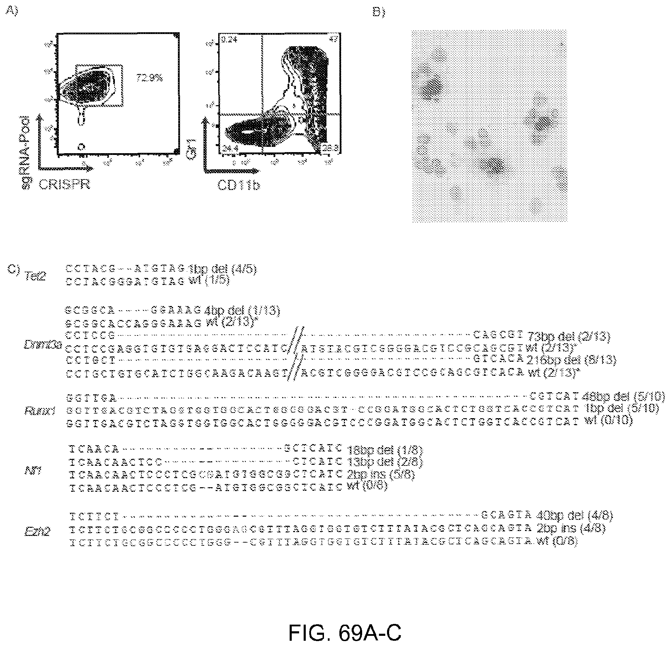

**Figure 69A-C** shows characterization of a CRISPR/Cas induced myeloid leukemia in a C57B1/6 with Tet2, Dnmt3a, Runxl, Nf1, and Ezh2 mutation A) Flow cytometric assessment of CRISPR/Cas vector expression (eGFP) and sgRNA-only vector expression (RFP657) for pooled sgRNAs in a leukemic mouse with leukocytosis (33.6x10^6/ml) and splenomegaly (412mg). Expression of myeloid cell markers (right). B) Peripheral blood smear (MGG; 400X) showing myelo-monocytic phenotype with low blast cell percentage. C) Mutation analysis on targeted genomic regions showed mutations in *Tet2, Dnmt3a, Runx1, Nf1,* and *Ezh2* (*cumulative number of wildtype sequences detected are given. Alignment of wildtype sequence is shown for all mutations with different length shown).

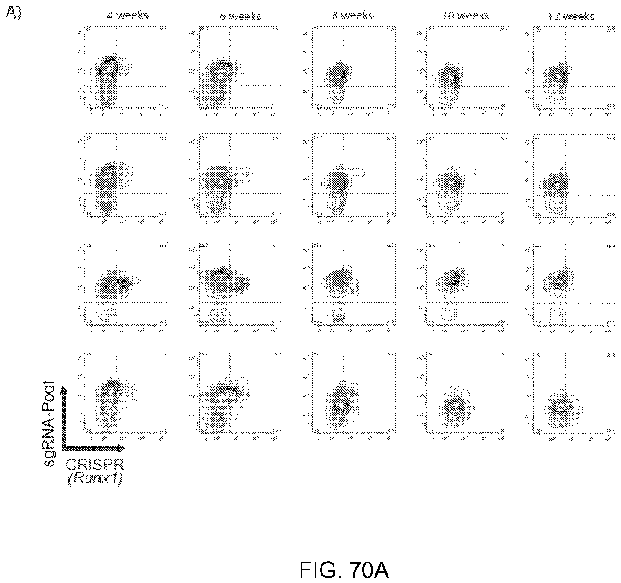

**Figure 70A-B** shows flow cytometry based clonality analysis of F1t3-ITD heterozygous mice shows expansion of CRISPR/Cas engineered populations over time. Flow cytometric assessment of CRISPR/Cas vector expression (eGFP) and sgRNAonly vector expression (RFP657) for pooled sgRNAs in Flt3-ITD heterozygous mice. A) The Runx1 targeting sgRNA was provided with the Cas9. B) The Tet2 sgRNA was provided with the Cas9. Each row shows vector expression at the given time point from an individual mouse.

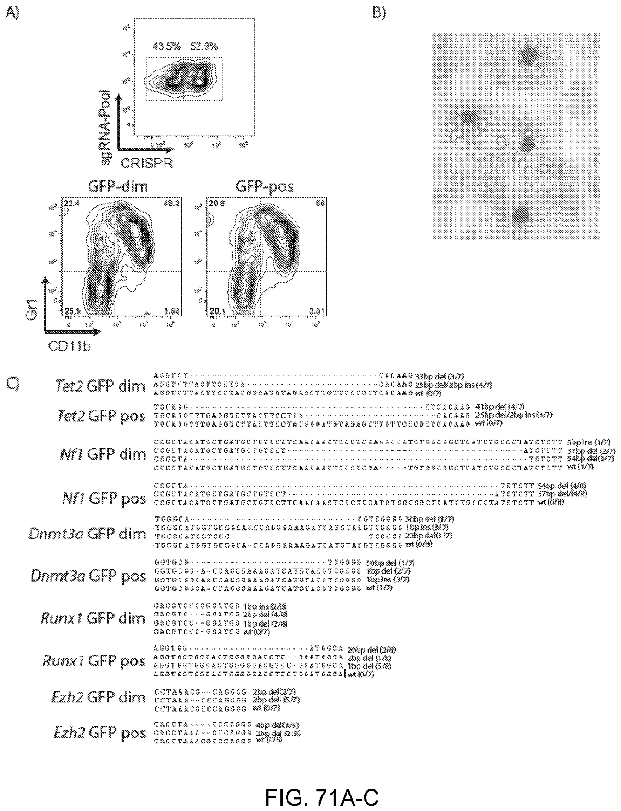

**Figure 71A-C** shows characterization of a CRISPR/Cas induced myeloid leukemia in a F1t3-ITD heterozygous mouse with Tet2, Dnmt3a, Runx1, Nf1, and Ezh2 mutation A) Flow cytometric assessment of CRISPR/Cas vector expression (eGFP) and sgRNA-only vector expression (RFP657) for pooled sgRNAs in a leukemic mouse that presented with mild leukocytosis (15.4x10^6/ml) and splenomegaly (326mg). Myeloid cell marker expression (lower panel) was assessed both in GFP-dim cells (left) and GFP-pos cells (right). B) Blood smear (MGG; 400X) shows a myelo-monocytic phenotype with high blast cell percentage. C) Mutation analysis on targeted genomic regions. Mutations were assessed for both flow sorted GFP-dim and GFP-pos cells. Redundant mutation on one allele in *Tet2, Dnmt3a, Runx1, Nf1,* and *Ezh2* suggests subclonal architecture.

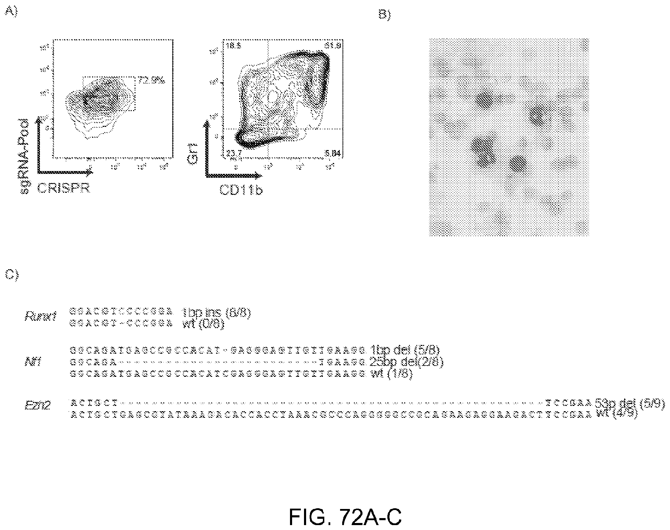

**Figure 72A-C** shows characterization of a CRISPR/Cas induced myeloid leukemia in a Flt3-ITD heterozygous mouse with *Runx1, Nf1,* and *Ezh2* mutation A) Flow cytometric assessment of CRISPR/Cas vector expression (eGFP) and sgRNA-only vector expression (RFP657) for pooled sgRNAs in a leukemic mouse with leukocytosis (40.4x10^6/ml) and splenomegaly (533mg). Expression of myeloid cell markers (right). B) Blood smear (MGG; 400X) showed myelo-monocytic phenotype with intermediate blast cell percentage. C) Mutation analysis on targeted genomic regions showed mutations in *Runx1, Nf1,* and *Ezh2.*

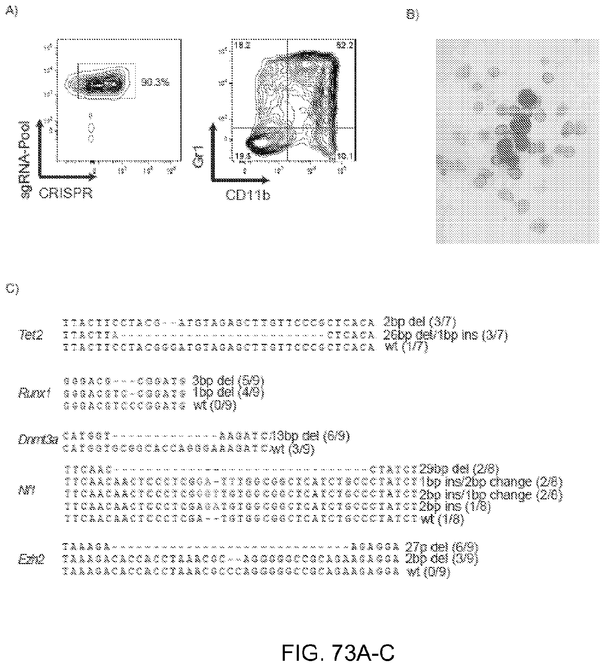

**Figure 73A-C** shows characterization of a CRISPR/Cas induced myeloid leukemia in a Flt3-ITD heterozygous mouse with Tet2, Dnmt3a, Runx1, Nf1, and Ezh2 mutation A) Flow cytometric assessment of CRISPR/Cas vector expression (eGFP) and sgRNA-only vector expression (RFP657) for pooled sgRNAs in a leukemic mouse with leukocytosis (78.1^6/ml) and splenomegaly (1036mg) and myeloid cell marker expression. Expression of myeloid cell markers (right). B) Blood smear (MGG; 400X) showed myelo-monocytic phenotype with intermediate blast cell percentage. C) Mutation analysis on targeted genomic regions showed mutations in *Tet2, Dnmt3a, Runx1, Nf1,* and *Ezh2.*

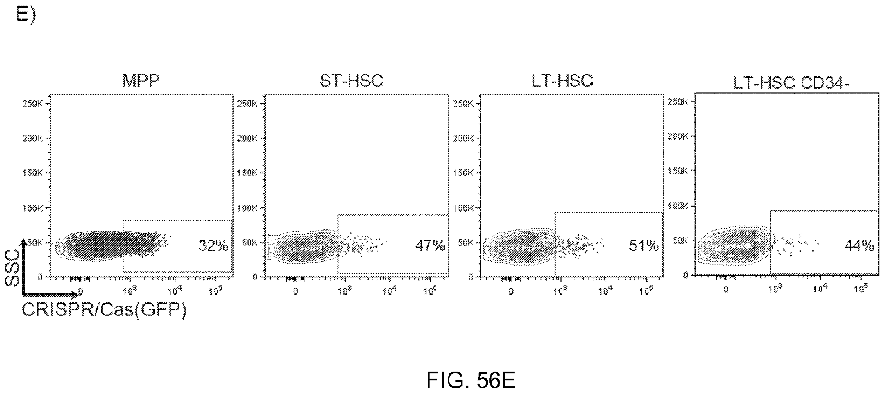

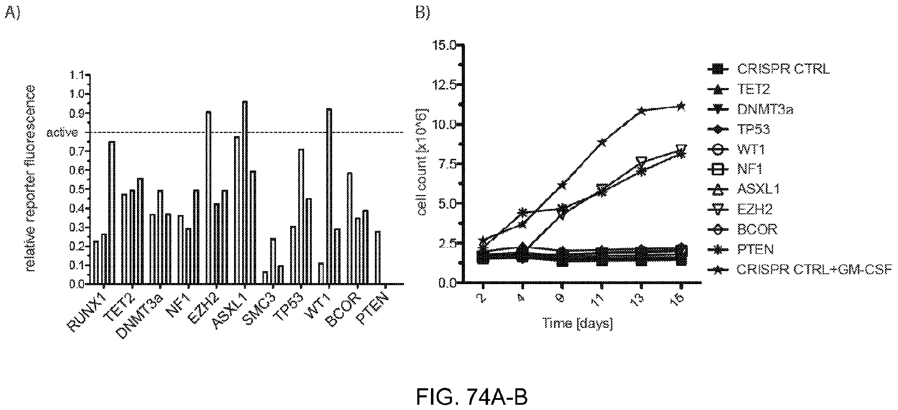

**Figure 74A-B** shows Lentiviral delivered CRISPR-Cas mediates transformation of human leukemia cells. A) Reporter assay based efficiency testing of lentiviral delivered Cas9 and sgRNAs targeting human tumor suppressor genes and transcription factor genes recurrently mutated in leukemia. Efficacy has been assessed as shown in Figure 56. B) Cumulative cell count over time of isogenic cell lines generated from cytokine dependent human erythroleukemia cell line TF1 after cytokine deprivation. Genes targeted are shown on the right. Transformation to growth factor independence was observed after CRISPR-Cas mediated targeting of PTEN or EZH2.

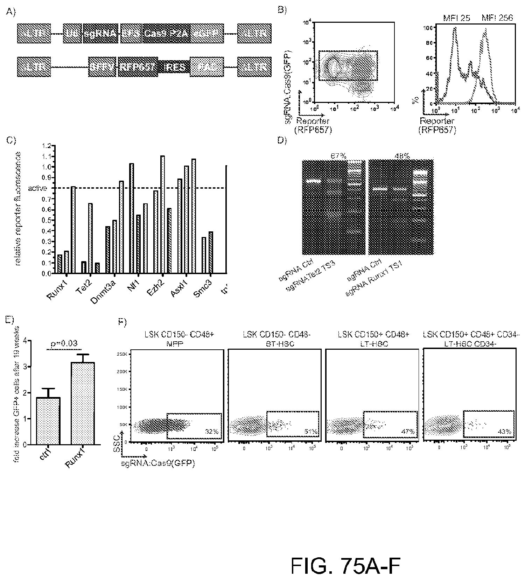

**Figure 75A-E** shows stable modification of hematopoietic stem cells by a lentiviral sgRNA:Cas9 delivery system, including: A) Depiction of a lentiviral vector for bi-cistronic expression of the sgRNA from a U6 promoter (U6) and *cas9* from a short *EF1a* promoter (EFS) with a fluorescent protein marker (eGFP) from a picorna virus derived 2A auto-cleavage site (P2A). A lentiviral vector that allows incorporation of target sites into the coding sequence of a fluorescent protein (RFP657) is depicted at the bottom. A Puromycin resistance gene (PAC) is expressed from an internal ribosomal entry site (IRES) B) Super-transduction of a reporter cell line with an eGFP tagged sgRNA:Cas9 vector targeting the respective site (blue) induces loss of reporter fluorescence. A non-targeting sgRNA:Cas9 vector (red) does not affect reporter fluorescence. Quantification by plotting the MFI of the reporter in targeting (blue) vs non-targeting (red) sgRNA transduced cells (black box, left plot). C) Efficacy of spacers for recurrently mutated genes in myeloid malignancy. Efficacy was assessed with the RFP657 reporter system described in A) and B). Results were normalized to non-targeting spacer. Spacer marked in red were used in following experiments. D) Surveyor assay based confirmation of target site cleavage at genomic loci for Tet2 and Runxl spacers indicated in panel C compared to non-targeting spacer. Percentages above are quantified cleavage efficacies. E) Peripheral blood sgRNA:Cas9 vector marking tracked over a period of 19 weeks. A significant increase of sgRNA:Cas9 expressing cells with a spacer targeting *Runx1* (n=4) was observed in comparison to mice expressing a non-targeting spacer (n=4). F) Stable expression of the sgRNA:Cas9 vector after 19 weeks in hematopoietic stem and progenitor cells. Overlay of eGFP expression in sgRNA:Cas9 transduced cells (blue) and non-transduced cells (red) of the respective cell population. MPP=Multipotent progenitors; ST-HSC=short-term HSCs; LT-HSC=long-term HSCs; LT-HSC CD34-=most quiescent long-term-HSCs

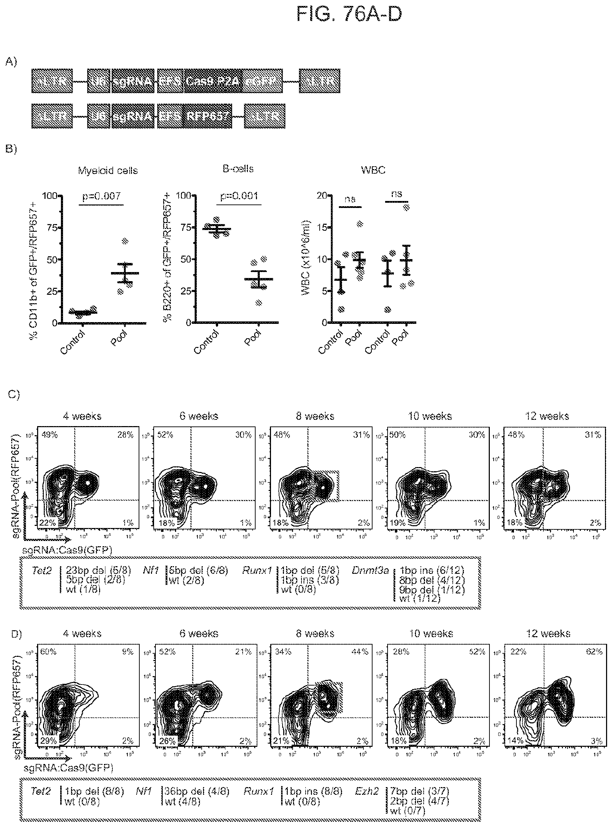

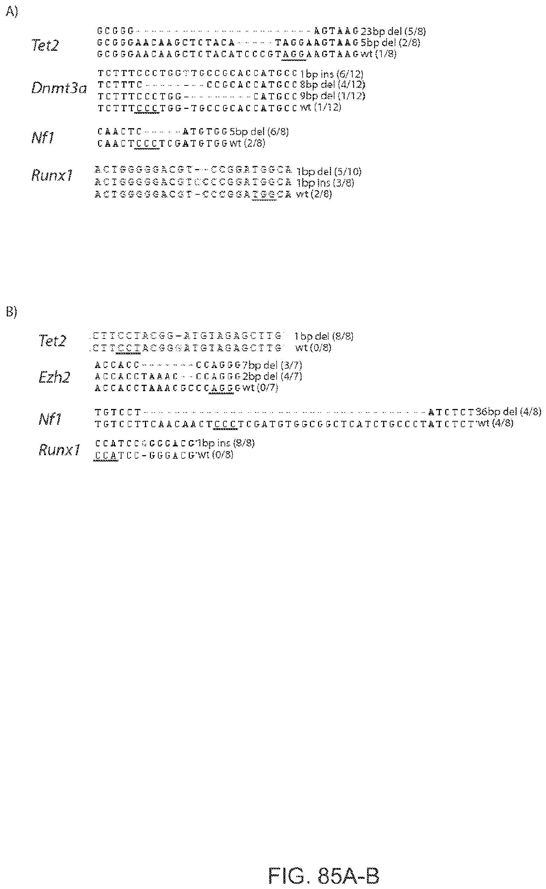

**Figure 76A-D** shows multiplex gene targeting induces clonal development *in vivo:* A) Schematic for multiplex gene targeting with two lentiviral vectors: a sgRNA:Cas9-eGFP vector expressing sgRNA, Cas9 and eGFP, and a vector expressing additional sgRNAs with a RFP657 fluorescent reporter gene from a short EFla promoter. B) Multiplexed gene targeting in mice (n=5) significantly increases myelomonocytic cells (CD11b+), decreases B-cells (B220+), and increases white blood cell counts at 12 weeks post-transplant in comparison to mice that were transplanted with control sgRNA vector transduced cells (n=4). C) Peripheral blood sgRNA:Cas-eGFP vector expression (x-axis) and expression of a pool of vectors expressing RFP657 (y-axis) over time starting 4 weeks post-transplant. Clonal populations were detected by the pattern of eGFP/RFP657 expression. Two clones were isolated (red gates indicate clones) and genomic regions of targeted genes were sequenced (displayed in red box). Mono- and bi-allelic deletions in *Tet2, Dnmt3a, Nf1* and *Runx1* were detected in one case. Detected mutations at 8 weeks are shown below. Sequences of the deletions are shown in Figure 85A. D) Another clone was analyzed as in panel C, and was found to have mutations in *Tet2, Ezh2, Nf1,* and *Runx1* B-II. Detected mutations at 8 weeks are shown below. Sequences of the deletions are shown in Figure 85B.

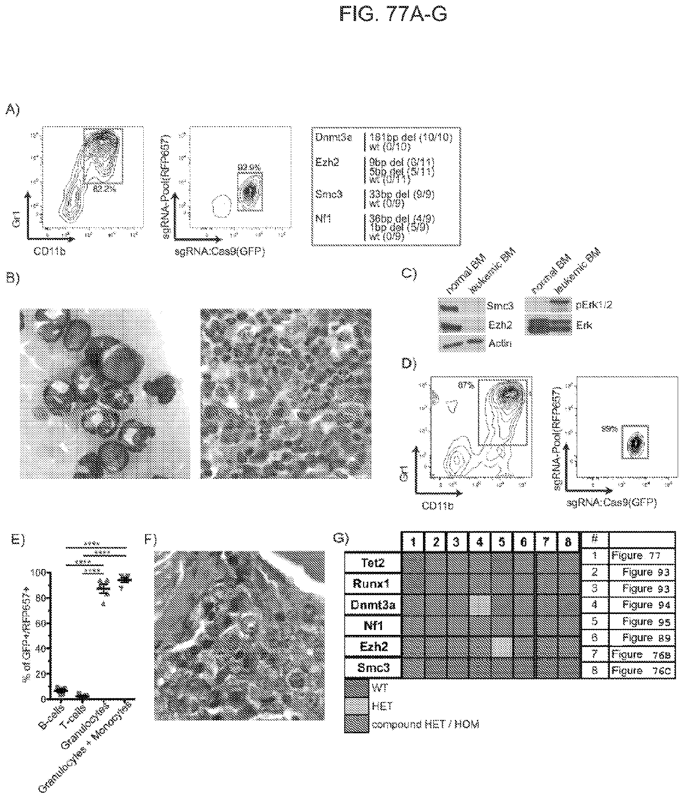

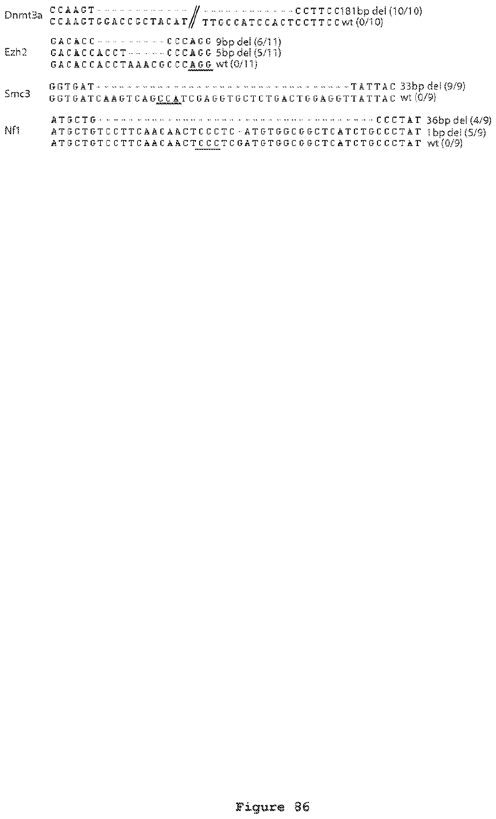

**Figure 77A-G** shows myeloid malignancy modeling with multiplex genome editing, including: A) Flow cytometric analysis of a diseased C57B1/6 mouse for expression of the introduced Cas9:sgRNA vectors and for myeloid (Gr1+/CD11b+). Detected mutations are shown in the right panel (displayed in red box). Sequence alignments are shown in Figure 86. B) Peripheral blood smear (left; May-Gruenwald/Giemsa (MGG); 1000x) and BM (right; Hematoxylin-Eosin(HE), 1000x) showing granulocytic cells and blasts. C) Western blots of Ezh2, Smc3, and activated Erk1/2 (pErk) protein levels in the leukemic BM compared to normal C57B1/6 wild-type BM. D) Representative flow cytometry of secondary transplant recipient mouse peripheral blood. E) Lineage distribution in peripheral blood from secondary mice 8 weeks post-transplant (n=5). F) Histopathology analysis of BM (MGG; 400x) from a secondary mouse that succumbed to leukemia. G) Heatmap summarizing mutations detected in all mice. Each column presents clone. Targeted genes are presented in rows. Color legend is given below. A table indicating where individual clones have been presented is given on the right.

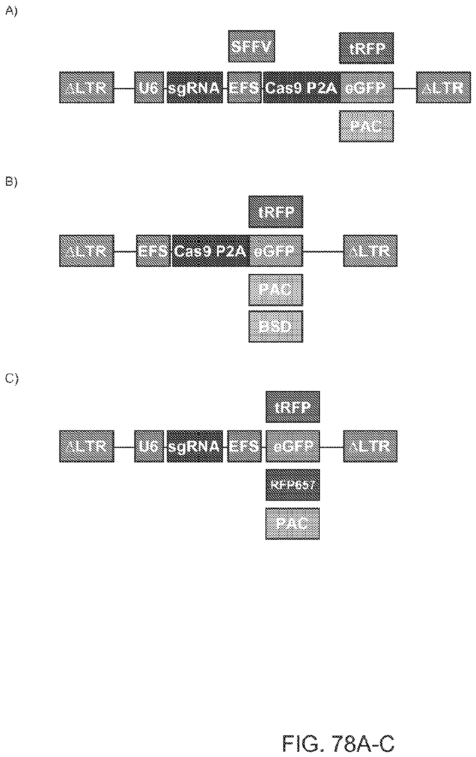

**Figure 78A-C** shows a modular CRISPR/Cas lentiviral vector system for genome engineering, including: A) Schematic depiction of a lentiviral vector for bi-cistronic expression of the sgRNA from a U6 promoter (U6) and expression of Cas9 from a short EFla promoter (EFS) or Spleen Focus Forming Virus (SFFV) promoter with a fluorescent protein marker (eGFP or tagRFP) or drug selectable marker (Purmomycin acetyl transferase (PAC) or Blasticidin deaminase (BSD)) from a picorna virus derived 2A auto-cleavage site (P2A). B) Depiction of a lentiviral vector for expression of Cas9 without the sgRNA. C) Depiction of a lentiviral vector for expression of the sgRNA only with a fluorescent protein marker or selectable marker form an EFS promoter.

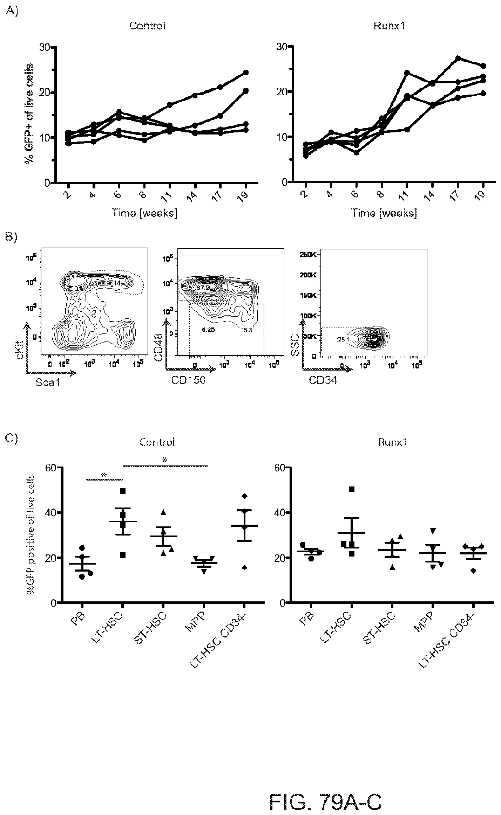

**Figure 79A-C** shows sgRNA:Cas9 mediated knock out of *Runx1* in Flt3-ITD heterozygous HSCs induces a competitive advantage including: A) Flow cytometric assessment of sgRNA:Cas9 transduced cells in the peripheral blood of mice over 19 weeks (control sgRNA left; Runxl sgRNA right). B) Gating scheme of HSPC populations in mice transduced with sgRNA:Cas9 lentiviral vectors. C) Summarized sgRNA:Cas9 vector expression in different HSPC populations at 19 weeks post transplantation for control sgRNA (left) or Runxl sgRNA (right). Significant differences are indicated where applicable (t-test, two sided, unpaired; Welch corrected for comparisons with signicantly different variances). MPP = Multipotent progenitors (LSK CD150- CD48+); ST-HSC = short-term HSCs (LSK CD150- CD48-); LT-HSC = long-term HSCs (LSK CD150+ CD48-); LT-HSC CD34- = most quiescent long-term-HSCs (LSK CD150+ CD48- CD34-).

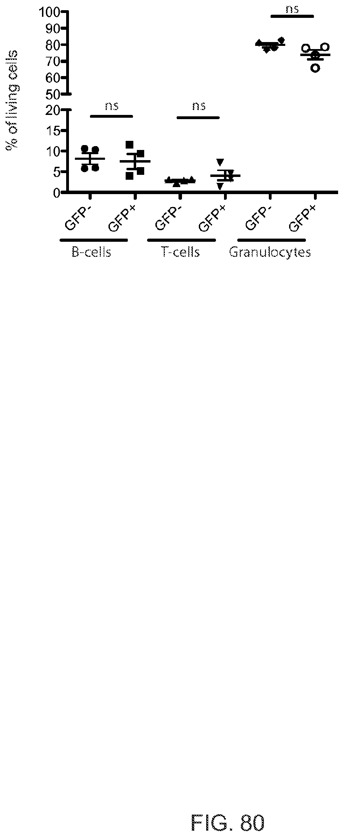

**Figure 80** shows sgRNA:Cas9 expression does not alter lineage maturation in the BM. Flow cytometry based quantification of mature hematopoietic lineages within the GFP negative and GFP positive population in the BM of mice expressing Cas9 (GFP+) and a control sgRNA shows no significant differences (t-test, two sided, unpaired) in the maturation potential of Cas9 expressing cells.

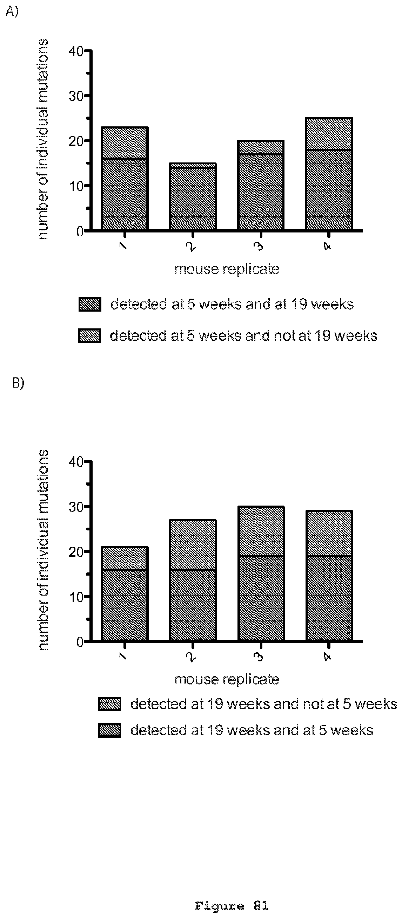

**Figure 81A-B** shows that next Generation Sequencing shows stable genome modification in long-term repopulating HSCs in-vivo Mutational analysis of peripheral blood samples at 5 weeks post-transplant and 19 weeks post-transplant, including: A) Samples were analyzed to detect all unique mutations from each mouse at 5 weeks and at 19 weeks for mutations at the Runxl target site. Mutations detected at both time points and only at 5 weeks are shown. B) Mutations found at 19 weeks but not at 5 weeks are shown.

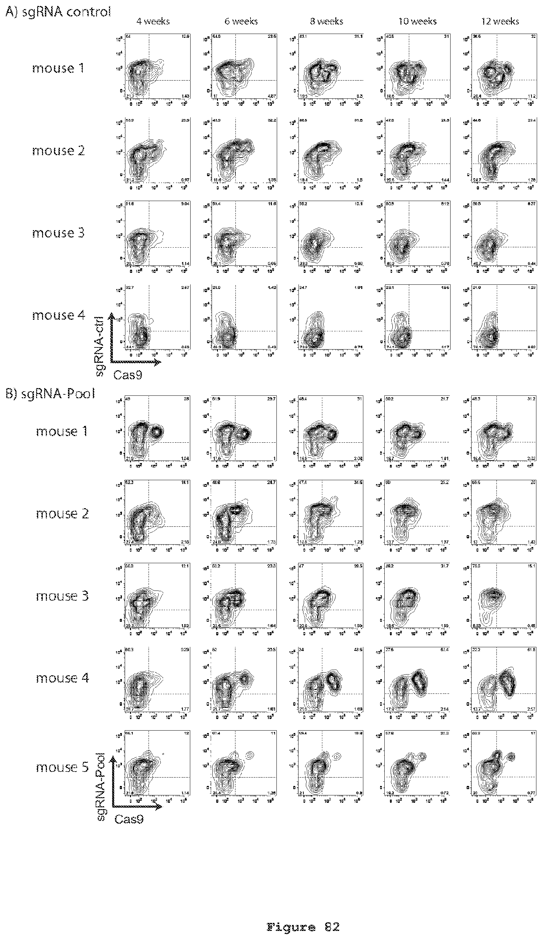

**Figure 82** - shows flow cytometry based clonality analysis of C57B1/6 mice shows expansion of sgRNA:Cas9 engineered populations over time Flow cytometric assessment of sgRNA:Cas9 vector expression (eGFP) and sgRNAonly vector expression (RFP657) for control sgRNA (A) and pooled sgRNAs (B) in C57B1/6 wild-type mice. Each row shows vector expression at the given time point from an individual mouse.

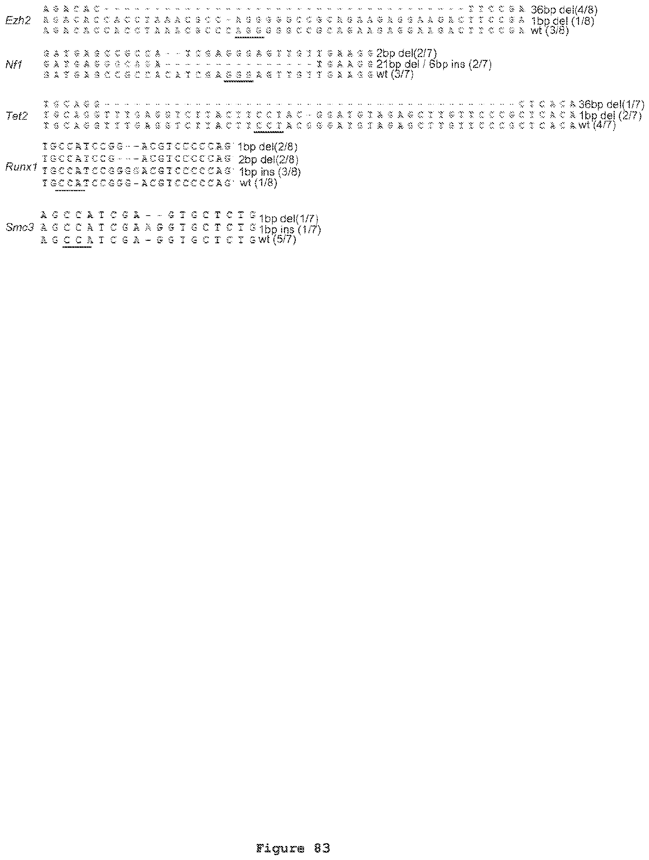

**Figure 83** - shows sequence alignment of targeted gene loci in a putatively clonal sgRNA :Cas9 modified population with dim Cas9 - eGFP expression and shows gene editing Alignment of sequences obtained for targeted genes from sorted cell populations shown in Figure 76. Ratio of bacterial clones with presented adjacent sequence out of the total clones sequenced for the respective gene shown behind each sequence A) Genes found mutated in the clone shown in Figure 76C. B) Genes found mutated for the clone shown in Figure 76D. PAM underlined

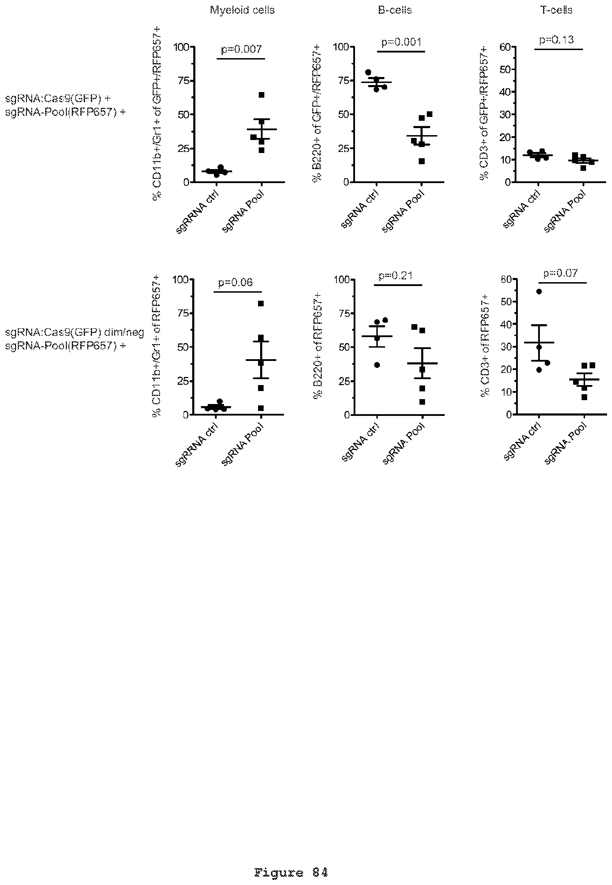

**Figure 84** - shows myeloid lineage skewing induced by multiplex CRISPR/Cas in eGFP-high and eGFP-dim sgRNA:Cas9 expressing populations. Flow cytometric assessment of mature hematopoietic lineage distribution in mice transduced with sgRNA-control or sgRNA-pool 10 weeks post transplant (myeloid = CD11b+/Gr1+; B-cells=B220+; T-cells=CD3+. Mice are shown in Figure 82. Upper panel shows lineage distribution in the GFP+ / RFP657+ population of mice with the targeting sgRNA-Pool (sgRNA-Pool) compared to the nontargeting control (sgRNA-ctrl). Lower Panel shows lineage distribution in the GFP-dim/neg / RFP657+ population of mice with the targeting sgRNA-Pool (sgRNA-Pool) compared to the non-targeting control (sgRNA-ctrl). Statistical differences are indicated (t-test, two sided, unpaired; Welch corrected for comparisons with significantly different variances).

**Figure 85A-B** shows sequence alignments of genes mutated in putatively clonal hematopoietic sgRNA:Cas9 modified populations identified after multiplex sgRNA:Cas9 genome engineering A) Alignment of sequences obtained for targeted genes from sorted cell populations shown in Figure 76. The ratio of the number of bacterial clones with each sequence over the total clones sequenced for the respective gene is shown following each sequence. A) Genes found to be mutated in the clone shown in Figure 76C. B) Genes found to be mutated for the clone shown in Figure 76D. PAM underlined.

**Figure 86** shows sequence alignments of Dnmt3a, Ezh2, Smc3, and Nf1 mutations in AML cells Alignment of sequences obtained for targeted genes from sorted leukemic cells shown in Figure 77A. The ratio of the number of bacterial clones with each sequence over the total clones sequenced for the respective gene is shown following each sequence. PAM underlined (due to deletion size not possible for Dnmt3a).

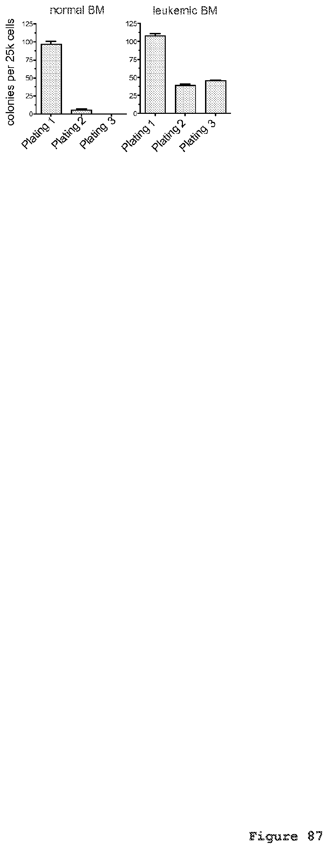

**Figure 87** shows leukemia induced by sgRNA:Cas9 genome editing shows enhanced replating capacity Serial replating capacity of normal BM compared to the leukemic BM cells from the leukemia presented in Figure 77.

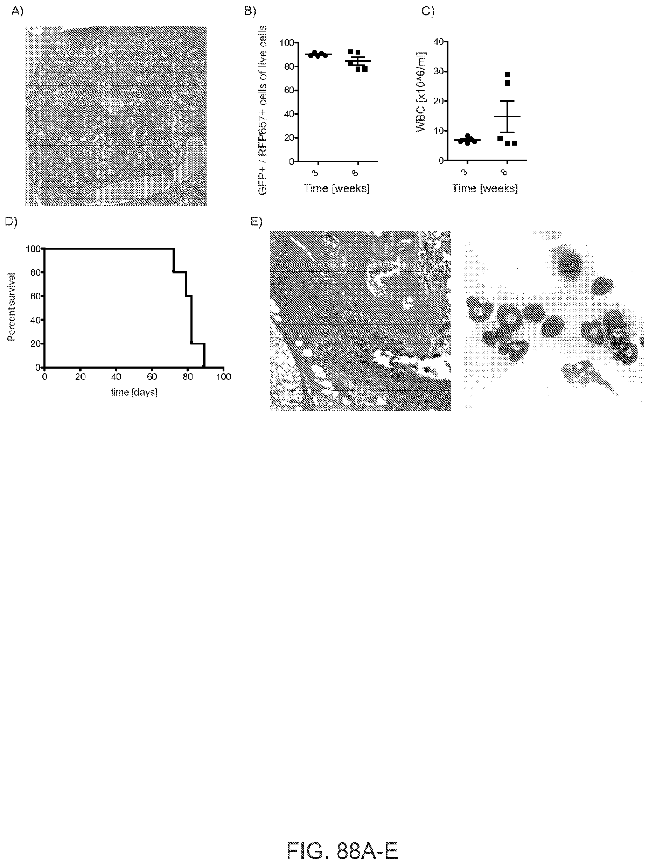

**Figure 88A-E** shows primary sgRNA:Cas9 induced leukemia engrafts in secondary mice and re-initiates disease. A) Low magnification (200x, HE) BM histology of the leukemic mouse presented in Figure 77. B) Engraftment of primary leukemia cells in sublethally irradiated secondary recipients as assessed by flow cytometry for eGFP and RFP657 at 3 and 8 weeks post transplant C) WBC counts of the same secondary mice at 3 and 8 weeks post transplant. D) Survival of the secondary mice. E) Low magnification (200x, HE) BM histology of a leukemic secondary mouse (left) and high magnification peripheral blood smear (MGG, 1000x) of a leukemic secondary mouse (right).

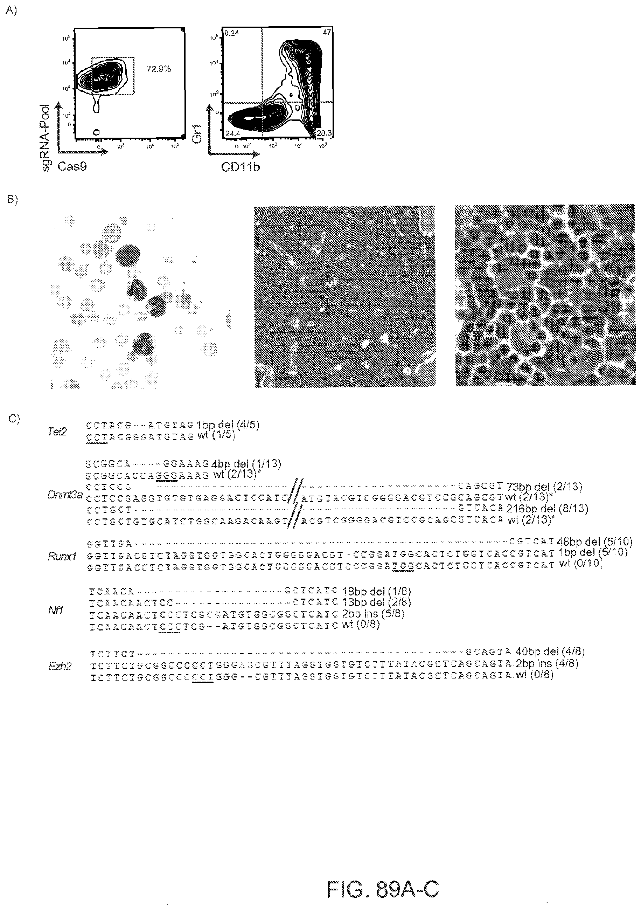

**Figure 89A-C** shows characterization of a CRISPR/Cas induced myeloid leukemia in a C57Bl/6 with Tet2, Dnmt3a, Runxl, Nf1, and Ezh2 mutation. A) Flow cytometric assessment of sgRNA:Cas9 vector expression (eGFP),sgRNA-only vector expression (RFP657) for pooled sgRNAs (left), and myeloid lineage marker expression (right) in a leukemic mouse transplanted with C57Bl/6 donor cells that presented with leukocytosis ($33.6 \times 10^6$/ml) and splenomegaly (412mg). B) High magnification peripheral blood smear (MGG, 1000x) of the leukemic mouse (left). Low magnification (200x, HE) BM histology (middle) and high magnification BM histology (10 00x, HE) (right) of the leukemic mouse. C) Mutation analysis on targeted genomic regions showed mutations in Tet2, Dnmt3a, Runxl, Nf1, and Ezh2. PAM underlined (due to deletion size not possible for some Dnmt3a sequences).

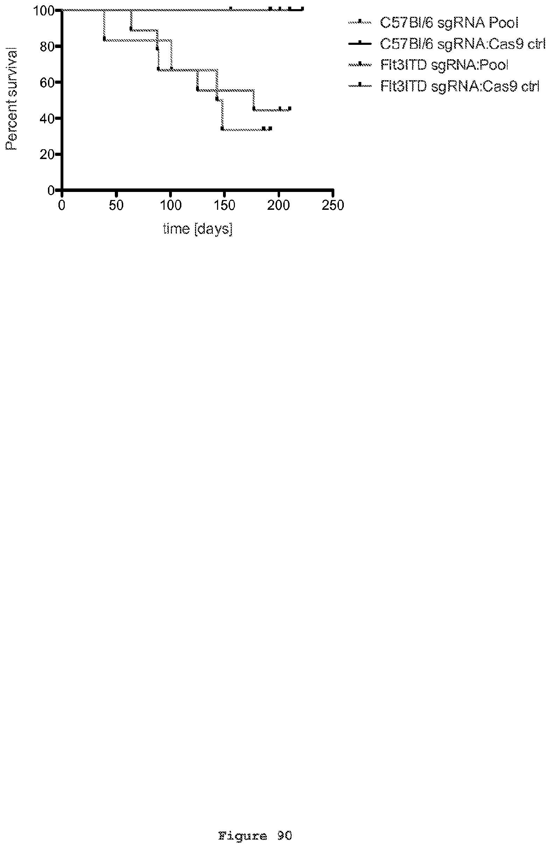

**Figure 90** shows survival of mice transplanted with pooled sgRNAs targeting tumor supressor genes. Survival of C57B1/6 mice with the targeting sgRNA-pool and Cas9 or non-targeting sgRNA and Cas9, and Flt3-ITD heterozygous mice transplanted targeting sgRNA-Pool.

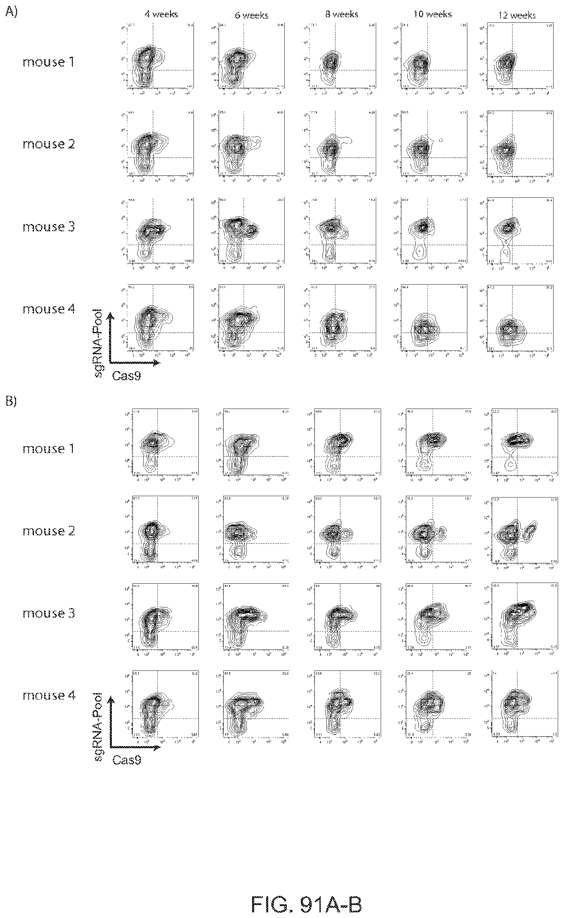

**Figure 91A-B** shows flow cytometry based clonality analysis of Fit 3 - ITD heterozygous mice shows expansion of sgRNA:Cas9 engineered populations over time. Flow cytometric assessment of sgRNA:Cas9 vector expression (eGFP) and sgRNAonly vector expression (RFP657) for pooled sgRNAs in Flt3-ITD heterozygous mice. A) The Runxl targeting sgRNA was provided with the Cas9. B) The Tet2 sgRNA was provided with the Cas9. Each row shows vector expression at the given time point from an individual mouse.

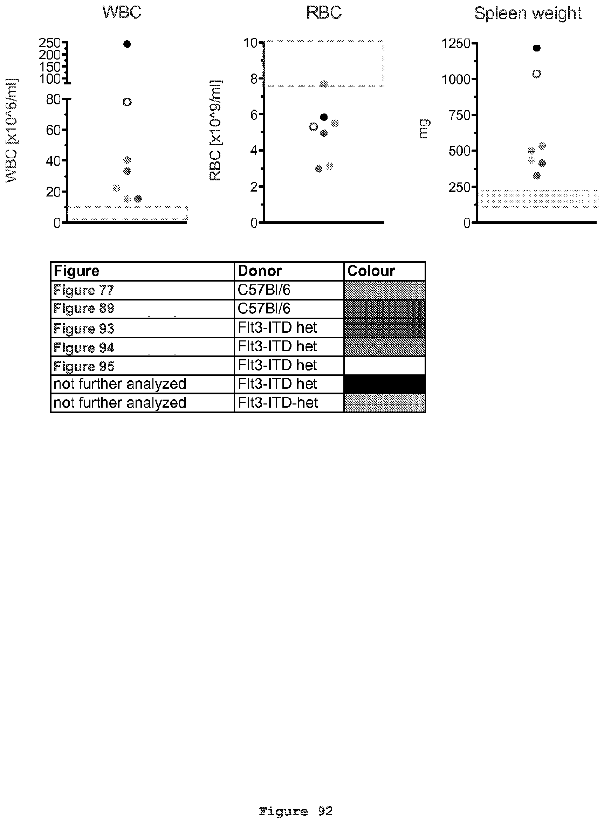

**Figure 92** shows summarized blood counts and spleen weights from leukemic Mice. Summarized WBC (left), RBC (middle), and spleen weight of leukemic mice presented in the study. Each mouse is presented in all three plots by one colour. A legend where data has been presented is given below. Range of control mice for each value is given as grey boxes. Control spleen weights are from Flt3-ITD control mice presented in Figure 75E/F and Figure 77.

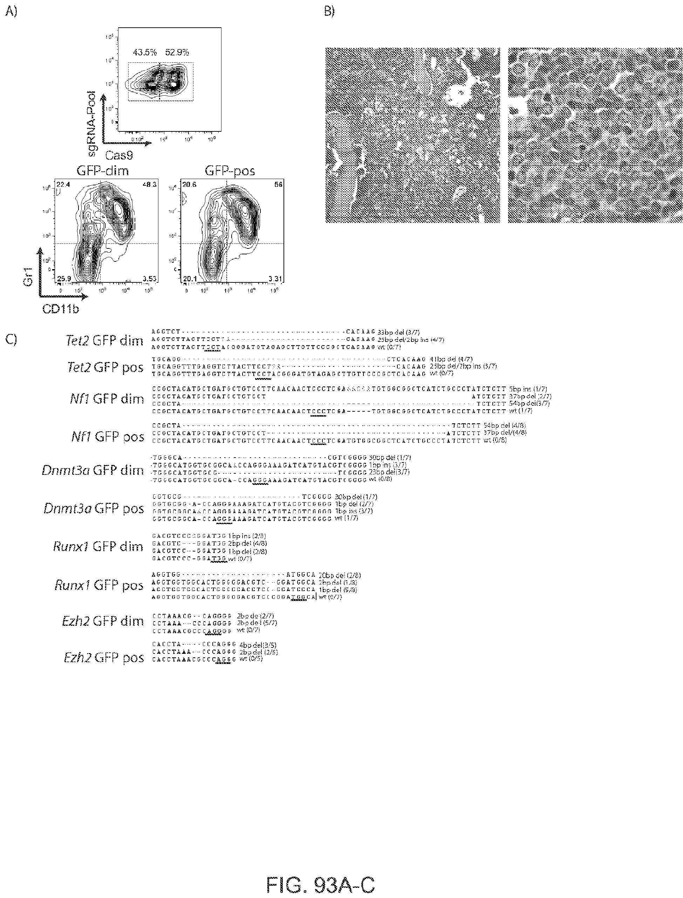

**Figure 93A-C** shows characterization of a Cas9:sgRNA induced myeloid leukemia in a F1t3-ITD heterozygous mouseA) Flow cytometric assessment of sgRNA:Cas9 vector expression (eGFP), sgRNA-only vector expression (RFP657) (upper panel) in a leukemic mouse that presented with mild leukocytosis ($15.4 \times 10^6$/ml) and splenomegaly (326mg). Myeloid marker expression (lower panel) was assessed both in GFP-dim cells (left) and GFP-pos cells (right). B) Low magnification (200x, HE) BM histology (left) and high magnification BM histology (1000x, HE) (right) of the leukemic mouse. C) Mutation analysis on targeted genomic regions. Mutations were assessed for both flow sorted GFP-dim and GFP-pos cells. Redundant mutation on one allele in Tet2, Nfl, Dnmt3a, Runxl, and Ezh2 suggests subclonal architecture. Figure 93 - Characterization of a Cas9:sgRNA induced myeloid leukemia in a Flt3-ITD heterozygous mouse PAM underlined.

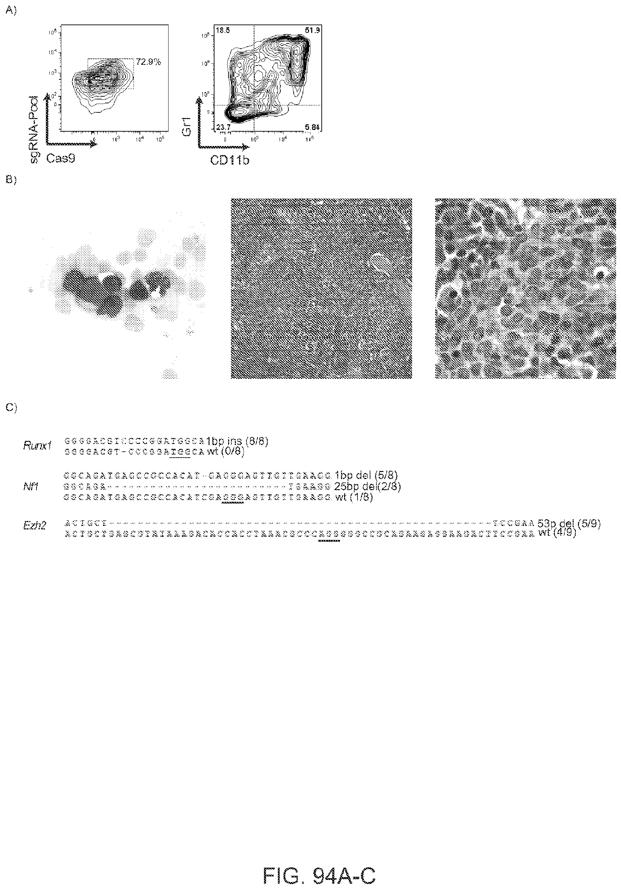

**Figure 94A-C** shows characterization of a sgRNA:Cas9 induced myeloid leukemia in a Flt3-ITD heterozygous mouse A) Flow cytometric assessment of sgRNA:Cas9 vector expression (eGFP), sgRNA-only vector expression (RFP657) (left) and myeloid lineage marker expression (right) in a leukemic mouse in a leukemic mouse with leukocytosis ($40.4 \times 10^6$/ml) and splenomegaly (533mg). Expression of myeloid markers (right). B) High magnification peripheral blood smear (MGG, 1000x) of the leukemic mouse (left). Low magnification (200x, HE) BM histology (middle) and high magnification BM histology 1000x, HE) (right) of the leukemic mouse. C) Mutation analysis on targeted genomic regions showed mutations in Runxl, Nf1, and Ezh2. PAM underlined.

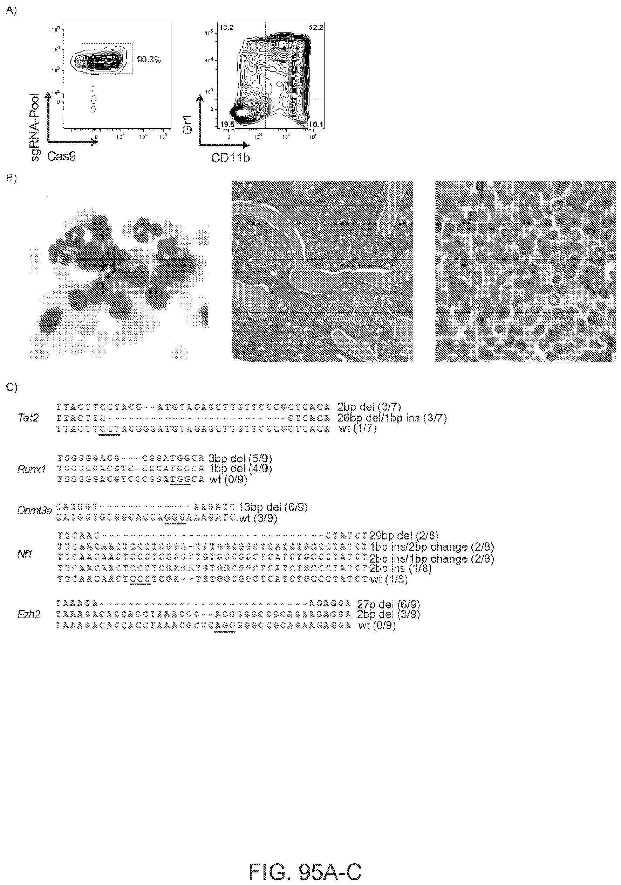

**Figure 95A-C** shows characterization of a sgRNA:Cas9 induced myeloid leukemia in a Flt3-ITD heterozygous mouse with A) Flow cytometric assessment of sgRNA:Cas9 vector expression (eGFP), sgRNA-only vector expression (RFP657) (left) and myeloid lineage marker expression (right) in a leukemic mouse in a leukemic mouse with leukocytosis (78.1^6/ml) and splenomegaly (1036mg) and myeloid marker expression. Expression of myeloid markers (right). B) High magnification peripheral blood smear (MGG, 1000x) of the leukemic mouse (left). Low magnification (200x, HE) BM histology (middle) and high magnification BM histology 1000x, HE) (right) of the leukemic mouse. C) Mutation analysis on targeted genomic regions showed mutations in Tet2, Runxl, Dnmt3a, Nf1, and Ezh2. PAM underlined.

The figures herein are for illustrative purposes only and are not necessarily drawn to scale.

DETAILED DESCRIPTION OF THE INVENTION

Also with respect to general information on CRISPR-Cas Systems, mention is made of:

> Multiplex genome engineering using CRISPR/Cas systems. Cong, L., Ran, F.A., Cox, D., Lin, S., Barretto, R., Habib, N., Hsu, P.D., Wu, X., Jiang, W., Marraffini, L.A., & Zhang, F. Science Feb 15;339(6121):819-23 (2013);

> RNA-guided editing of bacterial genomes using CRISPR-Cas systems. Jiang W., Bikard D., Cox D., Zhang F, Marraffini LA. Nat Biotechnol Mar;31(3):233-9 (2013);

> One-Step Generation of Mice Carrying Mutations in Multiple Genes by CRISPR/Cas-Mediated Genome Engineering. Wang H., Yang H., Shivalila CS., Dawlaty MM., Cheng AW., Zhang F., Jaenisch R. Cell May 9;153(4):910-8 (2013);

> Optical control of mammalian endogenous transcription and epigenetic states. Konermann S, Brigham MD, Trevino AE, Hsu PD, Heidenreich M, Cong L, Platt RJ, Scott DA, Church GM, Zhang F. Nature. 2013 Aug 22;500(7463):472-6. doi: 10.1038/Nature12466. Epub 2013 Aug 23;

> Double Nicking by RNA-Guided CRISPR Cas9 for Enhanced Genome Editing Specificity. Ran, FA., Hsu, PD., Lin, CY., Gootenberg, JS., Konermann, S., Trevino, AE., Scott, DA., Inoue, A., Matoba, S., Zhang, Y., & Zhang, F. Cell Aug 28. pii: S0092-8674(13)01015-5. (2013);

> DNA targeting specificity of RNA-guided Cas9 nucleases. Hsu, P., Scott, D., Weinstein, J., Ran, FA., Konermann, S., Agarwala, V., Li, Y., Fine, E., Wu, X., Shalem, O., Cradick, TJ., Marraffini, LA., Bao, G., & Zhang, F. Nat Biotechnol doi:10.1038/nbt.2647 (2013);

> Genome engineering using the CRISPR-Cas9 system. Ran, FA., Hsu, PD., Wright, J., Agarwala, V., Scott, DA., Zhang, F. Nature Protocols Nov;8(11):2281-308. (2013);

> Genome-Scale CRISPR-Cas9 Knockout Screening in Human Cells. Shalem, O., Sanjana, NE., Hartenian, E., Shi, X., Scott, DA., Mikkelson, T., Heckl, D., Ebert, BL., Root, DE., Doench, JG., Zhang, F. Science Dec 12. (2013). [Epub ahead of print];

> Crystal structure of cas9 in complex with guide RNA and target DNA. Nishimasu, H., Ran, FA., Hsu, PD., Konermann, S., Shehata, SI., Dohmae, N., Ishitani, R., Zhang, F., Nureki, O. Cell Feb 27. (2014). 156(5):935-49;

> Genome-wide binding of the CRISPR endonuclease Cas9 in mammalian cells. Wu X., Scott DA., Kriz AJ., Chiu AC., Hsu PD., Dadon DB., Cheng AW., Trevino AE., Konermann S., Chen S., Jaenisch R., Zhang F., Sharp PA. Nat Biotechnol. (2014) Apr 20. doi: 10.1038/nbt.2889, and

> Development and Applications of CRISPR-Cas9 for Genome Engineering, Hsu et al, Cell 157, 1262-1278 (June 5, 2014) (Hsu 2014),

each of which is discussed briefly below:

■ Cong *et al.* engineered type II CRISPR/Cas systems for use in eukaryotic cells based on both *Streptococcus thermophilus* Cas9 and also *Streptoccocus pyogenes* Cas9 and demonstrated that Cas9 nucleases can be directed by short RNAs to induce precise cleavage of DNA in human and mouse cells. Their study further showed that Cas9 as converted into a nicking enzyme can be used to facilitate homology-directed repair in eukaryotic cells with minimal mutagenic activity. Additionally, their study demonstrated that multiple guide sequences can be encoded into a single CRISPR array to enable simultaneous editing of several at endogenous genomic loci sites within the mammalian genome, demonstrating easy programmability and wide applicability of the RNA-guided nuclease technology. This ability to use RNA to program sequence specific DNA cleavage in cells defined a new class of genome engineering tools. These studies further showed that other CRISPR loci are likely to be transplantable into mammalian cells and can also mediate mammalian genome cleavage. Importantly, it can be envisaged that several aspects of the CRISPR/Cas system can be further improved to increase its efficiency and versatility.

- Jiang *et al.* used the clustered, regularly interspaced, short palindromic repeats (CRISPR)-associated Cas9 endonuclease complexed with dual-RNAs to introduce precise mutations in the genomes of *Streptococcus pneumoniae* and *Escherichia coli*. The approach relied on dual-RNA:Cas9-directed cleavage at the targeted genomic site to kill unmutated cells and circumvents the need for selectable markers or counter-selection systems. The study reported reprogramming dual-RNA:Cas9 specificity by changing the sequence of short CRISPR RNA (crRNA) to make single- and multinucleotide changes carried on editing templates. The study showed that simultaneous use of two crRNAs enabled multiplex mutagenesis. Furthermore, when the approach was used in combination with recombineering, in *S. pneumoniae,* nearly 100% of cells that were recovered using the described approach contained the desired mutation, and in *E. coli,* 65% that were recovered contained the mutation.

- Konermann *et al.* addressed the need in the art for versatile and robust technologies that enable optical and chemical modulation of DNA-binding domains based CRISPR Cas9 enzyme and also Transcriptional Activator Like Effectors

- As discussed in the present specification, the Cas9 nuclease from the microbial CRISPR-Cas system is targeted to specific genomic loci by a 20 nt guide sequence, which can tolerate certain mismatches to the DNA target and thereby promote undesired off-target mutagenesis. To address this, Ran *et al.* described an approach that combined a Cas9 nickase mutant with paired guide RNAs to introduce targeted double-strand breaks. Because individual nicks in the genome are repaired with high fidelity, simultaneous nicking *via* appropriately offset guide RNAs is required for double-stranded breaks and extends the number of specifically recognized bases for target cleavage. The authors demonstrated that using paired nicking can reduce off-target activity by 50- to 1,500-fold in cell lines and to facilitate gene knockout in mouse zygotes without sacrificing on-target cleavage efficiency. This versatile strategy enables a wide variety of genome editing applications that require high specificity.

- Hsu *et al.* characterized SpCas9 targeting specificity in human cells to inform the selection of target sites and avoid off-target effects. The study evaluated >700 guide RNA variants and SpCas9-induced indel mutation levels at >100 predicted genomic off-target loci in 293T and 293FT cells. The authors that SpCas9 tolerates mismatches between guide RNA and target DNA at different positions in a sequence-dependent manner, sensitive to the number, position and distribution of mismatches. The authors further showed that SpCas9-mediated cleavage is unaffected by DNA methylation and that the dosage of SpCas9 and sgRNA can be titrated to minimize off-target modification. Additionally, to facilitate mammalian genome engineering applications, the authors reported providing a web-based software tool to guide the selection and validation of target sequences as well as off-target analyses.

- Ran *et al.* described a set of tools for Cas9-mediated genome editing via non-homologous end joining (NHEJ) or homology-directed repair (HDR) in mammalian cells, as well as generation of modified cell lines for downstream functional studies. To minimize off-target cleavage, the authors further described a double-nicking strategy using the Cas9 nickase mutant with paired guide RNAs. The protocol by the authors provided experimentally derived guidelines for the selection of target sites, evaluation of cleavage efficiency and analysis of off-target activity. The studies showed that beginning with target design, gene modifications can be achieved within as little as 1-2 weeks, and modified clonal cell lines can be derived within 2-3 weeks.

- Shalem *et al.* described a new way to interrogate gene function on a genome-wide scale. Their studies showed that delivery of a genome-scale CRISPR-Cas9 knockout (GeCKO) library targeted 18,080 genes with 64,751 unique guide sequences enabled both negative and positive selection screening in human cells. First, the authors showed use of the GeCKO library to identify genes essential for cell viability in cancer and pluripotent stem cells. Next, in a melanoma model, the authors screened for genes whose loss is involved in resistance to vemurafenib, a therapeutic that inhibits mutant protein kinase BRAF. Their studies showed that the highest-ranking candidates included previously validated genes NF1 and MED12 as well as novel hits NF2, CUL3, TADA2B, and TADA1. The authors observed a high level of consistency between independent guide RNAs targeting the same gene and a high rate of hit confirmation, and thus demonstrated the promise of genome-scale screening with Cas9.

- Nishimasu *et al.* reported the crystal structure of *Streptococcus pyogenes* Cas9 in complex with sgRNA and its target DNA at 2.5 A° resolution. The structure revealed a bilobed architecture composed of target recognition and nuclease lobes, accommodating the sgRNA:DNA heteroduplex in a positively charged groove at their interface. Whereas the recognition lobe is essential for binding sgRNA and DNA, the nuclease lobe contains the HNH and RuvC nuclease domains, which are properly positioned for cleavage of the complementary and non-complementary strands of the target DNA, respectively. The nuclease lobe also contains a carboxyl-terminal domain responsible for the interaction with the protospacer adjacent motif (PAM). This high-resolution structure and accompanying functional analyses have revealed the molecular mechanism of RNA-guided DNA targeting by Cas9, thus paving the way for the rational design of new, versatile genome-editing technologies.

- Wu *et al.* mapped genome-wide binding sites of a catalytically inactive Cas9 (dCas9) from *Streptococcus pyogenes* loaded with single guide RNAs (sgRNAs) in mouse embryonic stem cells (mESCs). The authors showed that each of the four sgRNAs tested targets dCas9 to between tens and thousands of genomic sites, frequently characterized by a 5-nucleotide seed region in the sgRNA and an NGG protospacer adjacent motif (PAM). Chromatin inaccessibility decreases dCas9 binding to other sites with matching seed sequences; thus 70% of off-target sites are associated with genes. The authors showed that targeted sequencing of 295 dCas9 binding sites in mESCs transfected with catalytically active Cas9 identified only one site mutated above background levels. The authors proposed a two-state model for Cas9 binding and cleavage, in which a seed match triggers binding but extensive pairing with target DNA is required for cleavage.

■ Hsu 2014 is a review article that discusses generally CRISPR-Cas9 history from yogurt to genome editing, including genetic screening of cells, that is in the information, data and findings of the applications in the lineage of this specification filed prior to June 5, 2014. The general teachings of Hsu 2014 do not involve the specific models, animals of the instant specification.

The disclosure relates to the engineering and optimization of systems, methods and compositions used for the control of gene expression involving sequence targeting, such as genome perturbation or gene-editing, that relate to the CRISPR-Cas system and components thereof. In advantageous embodiments, the Cas enzyme is Cas9. In one aspect, the Cas enzyme is a nickase.

An advantage of the present methods is that the CRISPR system avoids off-target binding and its resulting side effects. This is achieved using systems arranged to have a high degree of sequence specificity for the target DNA.

Cas9 optimization may be used to enhance function or to develop new functions, one can generate chimeric Cas9 proteins. Examples that the Applicants have generated are provided in Example 6. Chimeric Cas9 proteins can be made by combining fragments from different Cas9 homologs. For example, two example chimeric Cas9 proteins from the Cas9s described herein. For example, Applicants fused the N-term of St1Cas9 (fragment from this protein is in bold) with C-term of SpCas9. The benefit of making chimeric Cas9s include any or all of: reduced toxicity; improved expression in eukaryotic cells; enhanced specificity; reduced molecular weight of protein, make protein smaller by combining the smallest domains from different Cas9 homologs; and/or altering the PAM sequence requirement. In one aspect, the chimeric Cas enzyme is a nickase.

The Cas9 may be used as a generic DNA binding protein. For example, and as shown in Example 7, Aplicants used Cas9 as a generic DNA binding protein by mutating the two catalytic domains (D10 and H840) responsible for cleaving both strands of the DNA target. In order to upregulate gene transcription at a target locus Applicants fused the transcriptional activation domain (VP64) to Cas9. Other transcriptional activation domains are known. As shown in Example 17, transcriptional activation is possible. As also shown in Example 17, gene repression (in this case of the beta-catenin gene) is possible using a Cas9 repressor (DNA-binding domain) that binds to the target gene sequence, thus repressing its activity.

Transgenic animals are also provided. Preferred examples include animals comprising Cas9, in terms of polynucleotides encoding Cas9 or the protein itself. Mice, rats and rabbits are preferred. To generate transgenic mice with the constructs, as exemplified herein one may inject pure, linear DNA into the pronucleus of a zygote from a pseudo pregnant female, e.g. a CB56 female. Founders may then be identified, genotyped, and backcrossed to CB57 mice. The constructs may then be cloned and optionally verified, for instance by Sanger sequencing. Knock outs are envisaged where for instance one or more genes are knocked out in a model. However, knockins are also envisaged (alone or in combination). An example Knock-in Cas9 mouse was generated and this is exemplified. To generate Cas9 knock in mice one may target the same constitutive and conditional constructs to the Rosa26 locus, as described herein (Figs. 25A-B and 26).

Utility of the conditional Cas9 mouse: Applicants have shown in 293 cells that the Cas9 conditional expression construct can be activated by co-expression with Cre. Applicants also show that the correctly targeted R1 mESCs can have active Cas9 when Cre is expressed. Because Cas9 is followed by the P2A peptide cleavage sequence and then EGFP Applicants identify successful expression by observing EGFP. Applicants have shown Cas9 activation in mESCs. This same concept is what makes the conditional Cas9 mouse so useful. Applicants may cross their conditional Cas9 mouse with a mouse that ubiquitously expresses Cre (ACTB-Cre line) and may arrive at a mouse that expresses Cas9 in every cell. It should only take the delivery of chimeric RNA to induce genome editing in embryonic or adult mice. Interestingly, if the conditional Cas9 mouse is crossed with a mouse expressing Cre under a tissue specific promoter, there should only be Cas9 in the tissues that also express Cre. This approach may be used to edit the genome in only precise tissues by delivering chimeric RNA to the same tissue.

As mentioned above, transgenic animals are also provided, as are transgenic plants, especially crops and algae. The transgenic may be useful in applications outside of providing a disease model. These may include food of feed production through expression of, for instance, higher protein, carbohydrate, nutrient or vitamins levels than would normally be seen in the wildtype. In this regard, transgenic plants, especially pulses and tubers, and animals, especially mammals such as livestock (cows, sheep, goats and pigs), but also poultry and edible insects, are preferred.

Transgenic algae or other plants such as rape may be particularly useful in the production of vegetable oils or biofuels such as alcohols (especially methanol and ethanol), for instance. These may be engineered to express or overexpress high levels of oil or alcohols for use in the oil or biofuel industries.

In terms of in *vivo* delivery, AAV is advantageous over other viral vectors for a couple of reasons:

- ◦ Low toxicity (this may be due to the purification method not requiring ultra centrifugation of cell particles that can activate the immune response)
- ◦ Low probability of causing insertional mutagenesis because it doesn't integrate into the host genome.

AAV has a packaging limit of 4.5 or 4.75 Kb. This means that Cas9 as well as a promoter and transcription terminator have to be all fit into the same viral vector. Constructs larger than 4.5 or 4.75 Kb will lead to significantly reduced virus production. SpCas9 is quite large, the gene itself is over 4.1 Kb, which makes it difficult for packing into AAV. Therefore embodiments include utilizing homologs of Cas9 that are shorter. For example:

| Species | Cas9 Size |
| --- | --- |
| Corynebacter diphtheriae | 3252 |
| Eubacterium ventriosum | 3321 |
| Streptococcus pasteurianus | 3390 |
| Lactobacillus farciminis | 3378 |
| Sphaerochaeta globus | 3537 |
| Azospirillum B510 | 3504 |
| Gluconacetobacter diazotrophicus | 3150 |
| Neisseria cinerea | 3246 |
| Roseburia intestinalis | 3420 |
| Parvibaculum lavamentivorans | 3111 |
| Staphylococcus aureus | 3159 |
| Nitratifractor salsuginis DSM 16511 | 3396 |
| Campylobacter lari CF89-12 | 3009 |
| Streptococcus thermophilus LMD-9 | 3396 |

These species are therefore, in general, preferred Cas9 species. Applicants have shown delivery and *in vivo* mouse brain Cas9 expression data.

Two ways to package Cas9 coding nucleic acid molecules, e.g., DNA, into viral vectors to mediate genome modification *in vivo* are preferred:

To achieve NHEJ-mediated gene knockout:

Single virus vector:
Vector containing two or more expression cassettes:

Promoter-Cas9 coding nucleic acid molecule -terminator
Promoter-gRNA1-terminator
Promoter-gRNA2-terminator
Promoter-gRNA(N)-terminator (up to size limit of vector)

Double virus vector:

Vector 1 containing one expression cassette for driving the expression of Cas9 Promoter-Cas9 coding nucleic acid molecule-terminator
Vector 2 containing one more expression cassettes for driving the expression of one or more guideRNAs
Promoter-gRNA1-terminator
Promoter-gRNA(N)-terminator (up to size limit of vector)

To mediate homology-directed repair. In addition to the single and double virus vector approaches described above, an additional vector is used to deliver a homology-direct repair template.

Promoters used to drive Cas9 coding nucleic acid molecule expression can include:

AAV ITR can serve as a promoter: this is advantageous for eliminating the need for an additional promoter element (which can take up space in the vector). The additional space freed up can be used to drive the expression of additional elements (gRNA, etc.). Also, ITR activity is relatively weaker, so can be used to reduce toxicity due to over expression of Cas9;

For ubiquitous expression, can use promoters: CMV, CAG, CBh, PGK, SV40, Ferritin heavy or light chains, etc.;

For brain expression, can use promoters: SynapsinI for all neurons, CaMKIIalpha for excitatory neurons, GAD67 or GAD65 or VGAT for GABAergic neurons, etc.;

For liver expression, can use Albumin promoter;

For lung expression, can use SP-B;

For endothelial cells, can use ICAM;

For hematopoietic cells can use IFNbeta or CD45; and

For Osteoblasts can use OG-2;

Promoter used to drive guide RNA can include:

Pol III promoters such as U6 or HI;

Use of Pol II promoter and intronic cassettes to express gRNA.

As to AAV, the AAV can be AAV1, AAV2, AAV5 or any combination thereof. One can select the AAV of the AAV with regard to the cells to be targeted; e.g., one can select AAV serotypes 1, 2, 5 or a hybrid or capsid AAV1, AAV2, AAV5 or any combination thereof for targeting brain or neuronal cells; and one can select AAV4 for targeting cardiac tissue. AAV8 is useful for delivery to the liver. The above promoters and vectors are preferred individually.

RNA delivery is also a useful method of *in vivo* delivery. Fig. 27 shows delivery and *in vivo* mouse brain Cas9 expression data. It is possible to deliver Cas9 and gRNA (and, for instance, HR repair template) into cells using liposomes or nanoparticles. Thus delivery of the CRISPR enzyme, such as a Cas9 and/or delivery of the RNAs may be in RNA form and via microvesicles, liposomes or nanoparticles. For example, Cas9 mRNA and gRNA can be packaged into liposomal particles for delivery in vivo. Liposomal transfection reagents such as Invivofectamine from Life Technologies and other reagents on the market can effectively deliver RNA molecules into the liver.

Enhancing NHEJ or HR efficiency is also helpful for delivery. It is preferred that NHEJ efficiency is enhanced by co-expressing end-processing enzymes such as Trex2 (Dumitrache et al. Genetics. 2011 August; 188(4): 787-797). It is preferred that HR efficiency is increased by transiently inhibiting NHEJ machineries such as Ku70 and Ku86. HR efficiency can also be increased by co-expressing prokaryotic or eukaryotic homologous recombination enzymes such as RecBCD, RecA.

Various means of delivery are described herein, and further discussed in this section.

Viral delivery: The CRISPR enzyme, for instance a Cas9, and/or any of the present RNAs, for instance a guide RNA, can be delivered using adeno associated virus (AAV), lentivirus, adenovirus or other viral vector types, or combinations thereof. Cas9 and one or more guide RNAs can be packaged into one or more viral vectors. In some embodiments, the viral vector is delivered to the tissue of interest by, for example, an intramuscular injection, while other times the viral delivery is via intravenous, transdermal, intranasal, oral, mucosal, or other delivery methods. Such delivery may be either via a single dose, or multiple doses. One skilled in the art understands that the actual dosage to be delivered herein may vary greatly depending upon a variety of factors, such as the vector chose, the target cell, organism, or tissue, the general condition of the organism or subject to be treated, the degree of transformation/modification sought, the administration route, the administration mode, the type of transformation/modification sought, etc.

Such a dosage may further contain, for example, a carrier (water, saline, ethanol, glycerol, lactose, sucrose, calcium phosphate, gelatin, dextran, agar, pectin, peanut oil, sesame oil, etc.), a diluent, a pharmaceutically-acceptable carrier (e.g., phosphate-buffered saline), a pharmaceutically-acceptable excipient, an adjuvant to enhance antigenicity, an immunostimulatory compound or molecule, and/or other compounds known in the art The adjuvant herein may contain a suspension of minerals (alum, aluminum hydroxide, aluminum phosphate) on which antigen is adsorbed; or water-in-oil emulsion in which antigen solution is emulsified in oil (MF-59, Freund's incomplete adjuvant), sometimes with the inclusion of killed mycobacteria (Freund's complete adjuvant) to further enhance antigenicity (inhibits degradation of antigen and/or causes influx of macrophages). Adjuvants also include immunostimulatory molecules, such as cytokines, costimulatory molecules, and for example, immunostimulatory DNA or RNA molecules, such as CpG oligonucleotides. Such a dosage formulation is readily ascertainable by one skilled in the art. The dosage may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include microcrystalline cellulose, carboxymethylcellulose sodium, polysorbate 80, phenylethyl alcohol, chlorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991).

In an embodiment herein the delivery is via a recombinant viral vector delivery system derived from an adenovirus or a lentivirus, which may be at a single booster dose containing at least $1 \times 10^5$ particles (also referred to as particle units, pu) of adenoviral or lentiviral vector. In an embodiment herein, the dose preferably is at least about $1 \times 10^6$ particles (for example, about $1 \times 10^6$-$1 \times 10^{12}$ particles), more preferably at least about $1 \times 10^7$ particles, more preferably at least about $1 \times 10^8$ particles (e.g., about $1 \times 10^8$-$1 \times 10^{11}$ particles or about $1 \times 10^8$-$1 \times 10^{12}$ particles), and most preferably at least about $1 \times 10^°$ particles (e.g., about $1^* \times 10^9$-$1 \times 10^{10}$ particles or about $1 \times 10^9$-$1 \times 10^{12}$ particles), or even at least about $1 \times 10^{10}$ particles (e.g., about $1 \times 10^{10}$-$1 \times 10^{12}$ particles) of the adenoviral or lentiviral vector. Alternatively, the dose comprises no more than about $1 \times 10^{14}$ particles, preferably no more than about $1 \times 10^{13}$ particles, even more preferably no more than about $1 \times 10^{12}$ particles, even more preferably no more than about $1 \times 10^{11}$ particles, and most preferably no more than about $1 \times 10^{10}$ particles (e.g., no more than about $1 \times 10^9$ articles). Thus, the dose may contain a single dose of adenoviral vector with, for example, about $1 \times 10^6$ particle units (pu), about $2 \times 10^6$ pu, about $4 \times 10^6$ pu, about $1 \times 10^7$ pu, about $2 \times 10^7$ pu, about $4 \times 10^7$ pu, about $1 \times 10^8$ pu, about $2 \times 10^8$ pu, about $4 \times 10^8$ pu, about $1 \times 10^9$ pu, about $2 \times 10^9$ pu, about $4 \times 10^9$ pu, about $1 \times 10^{10}$ pu, about $2 \times 10^{10}$ pu, about $4 \times 10^{10}$ pu, about $1 \times 10^{11}$ pu, about $2 \times 10^{11}$ pu, about $4 \times 10^{11}$ pu, about $1 \times 10^{12}$ pu, about $2 \times 10^{12}$ pu, or about $4 \times 10^{12}$ pu of adenoviral or lentiviral vector. See, for example, the adenoviral vectors in U.S. Patent No. 8,454,972 B2 to Nabel, et. al., granted on June 4, 2013; and the dosages at col 29, lines 36-58 thereof. In an embodiment herein, the adenovirus is delivered via multiple doses. Similarly, consider U.S. Patent No. 7,250,299 to Naldini et. al. granted on July 31, 2007 and U.S. Patent No. 7,226,780 to Arya granted on June 5, 2007 for dosage of lentiviral vectors.

In an embodiment herein, the delivery is via a recombinant viral vector delivery system derived from an AAV or a lentivirus. A therapeutically effective dosage for in vivo delivery of the AAV or lentivirus to a human is believed to be in the range of from about 20 to about 50 ml of saline solution containing from about $1 \times 10^{10}$ to about $1 \times 10^{10}$ functional AAV/ml or lentivirus/ml solution. The dosage may be adjusted to balance the therapeutic benefit against any side effects. In an embodiment herein, the AAV dose is generally in the range of concentrations of from about $1 \times 10^5$ to $1 \times 10^{50}$ genomes AAV, from about $1 \times 10^8$ to $1 \times 10^{20}$ genomes AAV, from about $1 \times 10^{10}$ to about $1 \times 10^{16}$ genomes, or about $1 \times 10^{11}$ to about $1 \times 10^{16}$ genomes AAV. A human dosage may be about $1 \times 10^{13}$ genomes AAV. Such concentrations may be delivered in from about 0.001 ml to about 100 ml, about 0.05 to about 50 ml, or about 10 to about 25 ml of a carrier solution. Other effective dosages can be readily established by one of ordinary skill in the art through routine trials establishing dose response curves. See, for example, U.S. Patent No. 8,404,658 B2 to Hajjar, et al., granted on March 26, 2013, at col. 27, lines 45-60.

In an embodiment herein the delivery is via a plasmid. In such plasmid compositions, the dosage should be a sufficient amount of plasmid to elicit a response. For instance, suitable quantities of plasmid DNA in plasmid compositions can be from about 0.1 to about 2 mg, or from about 1 μg to about 10 μg.

The doses herein are based on an average 70 kg individual. The frequency of administration is within the ambit of the medical or veterinary practitioner (e.g., physician, veterinarian), or scientist skilled in the art.

Cas9 and one or more guide RNA can be delivered using a vector delivery system derived from an adeno associated virus (AAV), lentivirus, adenovirus or other plasmid or viral vector types, using formulations and doses from, for example, US Patents Nos. 8,454,972 (formulations, doses for adenovirus), 8,404,658 (formulations, doses for AAV) and 5,846,946 (formulations, doses for DNA plasmids) and from clinical trials and publications regarding the clinical trials involving lentivirus, AAV and adenovirus. For examples, for AAV, the route of administration, formulation and dose can be as in US Patent No. 8,454,972 and as in clinical trials involving AAV. For Adenovirus, the route of administration, formulation and dose can be as in US Patent No. 8,404,658 and as in clinical trials involving adenovirus. For plasmid delivery, the route of administration, formulation and dose can be as in US Patent No 5,846,946 and as in clinical studies involving plasmids. Doses may be based on or extrapolated to an average 70 kg individual, and can be adjusted for patients, organisms or subjects, mammals of different weight and species. Frequency of administration is within the ambit of the medical or veterinary practitioner (e.g., physician, veterinarian), depending on usual factors including the age, sex, general health, other conditions of the patient or subject and the particular condition or symptoms being addressed.

The viral vectors can be injected into the tissue of interest. For cell-type specific genome modification, the expression of Cas9 can be driven by a cell-type specific promoter. For example, liver-specific expression might use the Albumin promoter and neuron-specific expression might use the Synapsin I promoter.

<u>RNA delivery</u>: The CRISPR enzyme, for instance a Cas9, and/or any of the present RNAs, for instance a guide RNA, can also be delivered in the form of RNA. Cas9 mRNA can be generated using *in vitro* transcription. For example, Cas9 mRNA can be synthesized using a PCR cassette containing the following elements: T7_promoter-kozak sequence (GCCACC)-Cas9-3' UTR from beta globin-polyA tail (a string of 120 or more adenines). The cassette can be used for transcription by T7 polymerase. Guide RNAs can also be transcribed using in vitro transcription from a cassette containing T7_promoter-GG-guide RNA sequence.

To enhance expression and reduce toxicity, the CRISPR enzyme and/or guide RNA can be modified using pseudo-U or 5-Methyl-C.

CRISPR enzyme mRNA and guide RNA may be delivered simultaneously using nanoparticles or lipid envelopes.

For example, Su X, Fricke J, Kavanagh DG, Irvine DJ ("In vitro and in vivo mRNA delivery using lipid-enveloped pH-responsive polymer nanoparticles" Mol Pharm. 2011 Jun 6;8(3):774-87. doi: 10.1021/mp100390w. Epub 2011 Apr 1) describes biodegradable core-shell structured nanoparticles with a poly($\beta$-amino ester) (PBAE) core enveloped by a phospholipid bilayer shell. These were developed for in vivo mRNA delivery. The pH-responsive PBAE component was chosen to promote endosome disruption, while the lipid surface layer was selected to minimize toxicity of the polycation core. Such are, therefore, preferred for delivering RNA.

Furthermore, Michael S D Kormann et al. ("Expression of therapeutic proteins after delivery of chemically modified mRNA in mice: Nature Biotechnology, Volume:29, Pages: 154-157 (2011) Published online 09 January 2011) describes the use of lipid envelopes to deliver RNA. Use of lipid envelopes is also preferred.

mRNA delivery methods are especially promising for liver delivery currently.

CRISPR enzyme mRNA and guide RNA might also be delivered separately. CRISPR enzyme mRNA can be delivered prior to the guide RNA to give time for CRISPR enzyme to be expressed. CRISPR enzyme mRNA might be administered 1-12 hours (preferably around 2-6 hours) prior to the administration of guide RNA.

Alternatively, CRISPR enzyme mRNA and guide RNA can be administered together. Advantageously, a second booster dose of guide RNA can be administered 1-12 hours (preferably around 2-6 hours) after the initial administration of CRISPR enzyme mRNA + guide RNA.

Additional administrations of CRISPR enzyme mRNA and/or guide RNA might be useful to achieve the most efficient levels of genome modification.

For minimization of toxicity and off-target effect, it will be important to control the concentration of CRISPR enzyme mRNA and guide RNA delivered. Optimal concentrations of CRISPR enzyme mRNA and guide RNA can be determined by testing different concentrations in a cellular or animal model and using deep sequencing the analyze the extent of modification at potential off-target genomic loci. For example, for the guide sequence targeting 5'-GAGTCCGAGCAGAAGAAGAA-3' (SEQ ID NO:_) in the EMX1 gene of the human genome, deep sequencing can be used to assess the level of modification at the following two off-target loci, 1: 5'-GAGTCCTAGCAGGAGAAGAA-3'(SEQ ID NO:_) and 2: 5'-GAGTCTAAGCAGAAGAAGAA-3' (SEQ ID NO:_). The concentration that gives the highest level of on-target modification while minimizing the level of off-target modification should be chosen for in vivo delivery.

Alternatively, to minimize the level of toxicity and off-target effect, CRISPR enzyme nickase mRNA (for example S. pyogenes Cas9 with the D10A mutation) can be delivered with a pair of guide RNAs targeting a site of interest. The two guide RNAs need to be spaced as follows. Guide sequences in red (single underline) and blue (double underline) respectively (these examples are based on the PAM requirement for *Streptococcus pyogenes* Cas9.

| Overhang length (bp) | Guide RNA design (guide sequence and PAM color coded) (SEQ ID NOs:_) |
|---|---|
| 14 | 5'-NNNNNNNNNNNNNNNNNNNNNCCNNNNNNNNNNNNNNNNNNNNGGNNNNNNNNNNNNNNNN-3'<br>3'-NNNNNNNNNNNNNNNNNNNNNNGGNNNNNNNNNNNNNNNNNNNNNNCCNNNNNNNNNNNNNNNNNN-5' |
| 13 | 5'-NNNNNNNNNNNNNNNNNNNNNNCCNNNNNNNNNNNNNNNNNNNNGGNNNNNNNNNNNNNNNNN-3'<br>3'-NNNNNNNNNNNNNNNNNNNNNGGNNNNNNNNNNNNNNNNNNNNNCCNNNNNNNNNNNNNNNNNN-5' |
| 12 | 5'-NNNNNNNNNNNNNNNNNNNNNNNCCNNNNNNNNNNNNNNNNNNNNGGNNNNNNNNNNNNNNNN-3'<br>3'-NNNNNNNNNNNNNNNNNNNNNGGNNNNNNNNNNNNNNNNNNNNNCCNNNNNNNNNNNNNNNNN-5' |
| 11 | 5'-NNNNNNNNNNNNNNNNNNNNNNNCCNNNNNNNNNNNNNNNNNNNGGNNNNNNNNNNNNNNNN-3'<br>3'-NNNNNNNNNNNNNNNNNNNNGGNNNNNNNNNNNNNNNNNNNNNCCNNNNNNNNNNNNNNNNN-5' |
| 10 | 5'-NNNNNNNNNNNNNNNNNNNNNNNCCNNNNNNNNNNNNNNNNNNNGGNNNNNNNNNNNNNNNNN-3'<br>3'-NNNNNNNNNNNNNNNNNNNNGGNNNNNNNNNNNNNNNNNNNCCNNNNNNNNNNNNNNNNN-5' |
| 9 | 5'-NNNNNNNNNNNNNNNNNNNNNNNCCNNNNNNNNNNNNNNNNNNNGGNNNNNNNNNNNNNNNNN-3'<br>3'-NNNNNNNNNNNNNNNNNNNNGGNNNNNNNNNNNNNNNNNCCNNNNNNNNNNNNNNNNNNN-5' |
| 8 | 5'-NNNNNNNNNNNNNNNNNNNNNNCCNNNNNNNNNNNNNNNNNNNGGNNNNNNNNNNNNNNNNN-3'<br>3'-NNNNNNNNNNNNNNNNNNNNGGNNNNNNNNNNNNNNNCCNNNNNNNNNNNNNNNNNNNN-5' |
| 7 | 5'-NNNNNNNNNNNNNNNNNNNNNNCCNNNNNNNNNNNNNNNGGNNNNNNNNNNNNNNNNNNN-3'<br>3'-NNNNNNNNNNNNNNNNNNGGNNNNNNNNNNNNNNNCCNNNNNNNNNNNNNNNNNNN-5' |
| 6 | 5'-NNNNNNNNNNNNNNNNNNNNNCCNNNNNNNNNNNNNNNNNNGGNNNNNNNNNNNNNNNNNNN-3'<br>3'-NNNNNNNNNNNNNNNNNNGGNNNNNNNNNNNNNCCNNNNNNNNNNNNNNNNNNNNNN-5' |
| 5 | 5'-NNNNNNNNNNNNNNNNNNNCCNNNNNNNNNNNNNNNNNGGNNNNNNNNNNNNNNNNNNNNN-3'<br>3'-NNNNNNNNNNNNNNNNNGGNNNNNNNNNNNNNNNNNNCCNNNNNNNNNNNNNNNNNNNNNNNNNN-5' |
| 4 | 5'-NNNNNNNNNNNNNNNNNNCCNNNNNNNNNNGGNNNNNNNNNNNNNNNNNNNNNNNN-3'<br>3'-NNNNNNNNNNNNNNNGGNNNNNNNNNNCCNNNNNNNNNNNNNNNNNNNNNNNN-5' |
| 3 | 5'-NNNNNNNNNNNNNNNNNNNNCCNNNNNNNNNNNGGNNNNNNNNNNNNNNNNNNNNNNNN-3' |

(continued)

| Overhang length (bp) | Guide RNA design (guide sequence and PAM color coded) (SEQ ID NOs:_) |
|---|---|
| | 3'-NNNNNNNNNNNNNNNNNNNNNGGNNNNNNNNNNCCNNNNNNNNNNNNNNNNNNNNNNNNN-5' |
| 2 | 5'-NNNNNNNNNNNNNNNNNNNNCCNNNNNNNNNNGGNNNNNNNNNNNNNNNNNNNNNNNNNN-3' |
| | 3'-NNNNNNNNNNNNNNNNNNNNGGNNNNNNNNNNCCNNNNNNNNNNNNNNNNNNNNNNNNNN-5' |
| 1 | 5'-NNNNNNNNNNNNNNNNNNNNCCNNNNNNNNNNGGNNNNNNNNNNNNNNNNNNNNNNNNNN-3' |
| | 3'-NNNNNNNNNNNNNNNNNNNNNGGNNNNNNNNNNCCNNNNNNNNNNNNNNNNNNNNNNNNN-5' |
| blunt | 5'-NNNNNNNNNNNNNNNNNNNNNCCNNNNNNNNNGGNNNNNNNNNNNNNNNNNNNNNNNNNN-3' |
| | 3'-NNNNNNNNNNNNNNNNNNNNNGGNNNNNNNNNCCNNNNNNNNNNNNNNNNNNNNNNNNNN-5' |
| 1 | 5'-NNNNNNNNNNNNNNNNNNNNNCCNNNNNNNNGGNNNNNNNNNNNNNNNNNNNNNNNNNNN-3' |
| | 3'-NNNNNNNNNNNNNNNNNNNNNGGNNNNNNNNCCNNNNNNNNNNNNNNNNNNNNNNNNNNN-5' |
| 2 | 5'-NNNNNNNNNNNNNNNNNNNNNCCNNNNNNGGNNNNNNNNNNNNNNNNNNNNNNNNNNNNN-3' |
| | 3'-NNNNNNNNNNNNNNNNNNNNNGGNNNNNNCCNNNNNNNNNNNNNNNNNNNNNNNNNNNNN-5' |
| 3 | 5'-NNNNNNNNNNNNNNNNNNNNNCCNNNNNGGNNNNNNNNNNNNNNNNNNNNNNNNNNNNNN-3' |
| | 3'-NNNNNNNNNNNNNNNNNNNNNGGNNNNNCCNNNNNNNNNNNNNNNNNNNNNNNNNNNNNN-5' |
| 4 | 5'-NNNNNNNNNNNNNNNNNNNNNCCNNNNGGNNNNNNNNNNNNNNNNNNNNNNNNNNNNNNN-3' |
| | 3'-NNNNNNNNNNNNNNNNNNNNNGGNNNNCCNNNNNNNNNNNNNNNNNNNNNNNNNNNNNNN-5' |
| 5 | 5'-NNNNNNNNNNNNNNNNNNNNNCCNNNGGNNNNNNNNNNNNNNNNNNNNNNNNNNNNNNNN-3' |
| | 3'-NNNNNNNNNNNNNNNNNNNNNGGNNNCCNNNNNNNNNNNNNNNNNNNNNNNNNNNNNNNN-5' |
| 6 | 5'-NNNNNNNNNNNNNNNNNNNNNCCNNGGNNNNNNNNNNNNNNNNNNNNNNNNNNNNNNNNN-3' |
| | 3'-NNNNNNNNNNNNNNNNNNNNNGGNNCCNNNNNNNNNNNNNNNNNNNNNNNNNNNNNNNNN-5' |
| 7 | 5'-NNNNNNNNNNNNNNNNNNNNNCCNGGNNNNNNNNNNNNNNNNNNNNNNNNNNNNNNNNNN-3' |
| | 3'-NNNNNNNNNNNNNNNNNNNNNGGNCCNNNNNNNNNNNNNNNNNNNNNNNNNNNNNNNNNN-5' |
| 8 | 5'-NNNNNNNNNNNNNNNNNNNNNCCGGNNNNNNNNNNNNNNNNNNNNNNNNNNNNNNNNNNN-3' |

| Overhang length (bp) | Guide RNA design (guide sequence and PAM color coded) (SEQ ID NOs:_) |
|---|---|
|  | 3'-NNNNNNNNNNNNNNNNNNNNGGCCNNNNNNNNNNNNNNNNNNNNNNNNNNNNNNNNNNNNN-5' |
| 12 | 5'-NNNNNNNNNNNNNNNNNNNNNNGGNNNNNNNNNNNNNNNNNNNNNNNNNNNNNNNNNNNNN-3' |
|  | 3'-NNNNNNNNNNNNNNNNNNNNNNNCCGGNNNNNNNNNNNNNNNNNNNNNNNNNNNNNNNNNN-5' |
| 13 | 5'-NNNNNNNNNNNNNNNNNNNNNNNCGGNNNNNNNNNNNNNNNNNNNNNNNNNNNNNNNNNNN-3' |
|  | 3'-NNNNNNNNNNNNNNNNNNNNNNNNCCNGGNNNNNNNNNNNNNNNNNNNNNNNNNNNNNNNN-5' |
| 14 | 5'-NNNNNNNNNNNNNNNNNNNNNNNNCGGNNNNNNNNNNNNNNNNNNNNNNNNNNNNNNNNNN-3' |
|  | 3'-NNNNNNNNNNNNNNNNNNNNNNNNCCNNGGNNNNNNNNNNNNNNNNNNNNNNNNNNNNNNN-5' |
| 15 | 5'-NNNNNNNNNNNNNNNNNNNNNNNNNCGGNNNNNNNNNNNNNNNNNNNNNNNNNNNNNNNNN-3' |
|  | 3'-NNNNNNNNNNNNNNNNNNNNNNNNNCCNNNGGNNNNNNNNNNNNNNNNNNNNNNNNNNNNN-5' |
| 16 | 5'-NNNNNNNNNNNNNNNNNNNNNNNNNCGGNNNNNNNNNNNNNNNNNNNNNNNNNNNNNNNNN-3' |
|  | 3'-NNNNNNNNNNNNNNNNNNNNNNNNNCCNNNNGGNNNNNNNNNNNNNNNNNNNNNNNNNNNN-5' |
| 17 | 5'-NNNNNNNNNNNNNNNNNNNNNNNNNNCGGNNNNNNNNNNNNNNNNNNNNNNNNNNNNNNNN-3' |
|  | 3'-NNNNNNNNNNNNNNNNNNNNNNNNNNCCNNNNNGGNNNNNNNNNNNNNNNNNNNNNNNNNN-5' |

EP 3 011 035 B1

Further interrogation of the system have given Applicants evidence of the 5' overhang (see, e.g., Ran et al., Cell. 2013 Sep 12;154(6):1380-9). Applicants have further identified parameters that relate to efficient cleavage by the Cas9 nickase mutant when combined with two guide RNAs and these parameters include but are not limited to the length of the 5' overhang. In embodiments of the invention the 5' overhang is at most 200 base pairs, preferably at most 100 base pairs, or more preferably at most 50 base pairs. In embodiments of the invention the 5' overhang is at least 26 base pairs, preferably at least 30 base pairs or more preferably 34-50 base pairs or 1-34 base pairs. In other preferred methods of the invention the first guide sequence directing cleavage of one strand of the DNA duplex near the first target sequence and the second guide sequence directing cleavage of the opposite strand near the second target sequence results in a blunt cut or a 3' overhang. In embodiments of the invention the 3' overhang is at most 150, 100 or 25 base pairs or at least 15, 10 or 1 base pairs. In preferred embodiments the 3' overhang is 1-100 basepairs.

Aspects of the invention relate to the expression of the gene product being decreased or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased. In an embodiment of the gene product is a protein. In an embodiment the gene product is a microRNA. In one embodiment, the regulatory region of a gene is targeted, thereby modifying the expression of the corresponding gene product.

Only sgRNA pairs creating 5' overhangs with less than 8bp overlap between the guide sequences (offset greater than about 8 bp) were able to mediate detectable indel formation. Importantly, each guide used in these assays is able to efficiently induce indels when paired with wildtype Cas9, indicating that the relative positions of the guide pairs are the most important parameters in predicting double nicking activity.

Since Cas9n and Cas9H840A nick opposite strands of DNA, substitution of Cas9n with Cas9H840A with a given sgRNA pair should result in the inversion of the overhang type. For example, a pair of sgRNAs that will generate a 5' overhang with Cas9n should in principle generate the corresponding 3' overhang instead. Therefore, sgRNA pairs that lead to the generation of a 3' overhang with Cas9n might be used with Cas9H840A to generate a 5' overhang. Applicants tested Cas9H840A with a set of sgRNA pairs designed to generate both 5' and 3' overhangs (offset range from -278 to +58 bp), but, unexpectedly, were unable to observe indel formation. Further work may be needed to identify the necessary design rules for sgRNA pairing to allow double nicking by Cas9H840A.

Additional delivery options for the brain include encapsulation of CRISPR enzyme and guide RNA in the form of either DNA or RNA into liposomes and conjugating to molecular Trojan horses for trans-blood brain barrier (BBB) delivery. Molecular Trojan horses have been shown to be effective for delivery of B-gal expression vectors into the brain of non-human primates. The same approach can be used to delivery vectors containing CRISPR enzyme and guide RNA. For instance, Xia CF and Boado RJ, Pardridge WM ("Antibody-mediated targeting of siRNA via the human insulin receptor using avidin-biotin technology." Mol Pharm. 2009 May-Jun;6(3):747-51. doi: 10.1021/mp800194) describes how delivery of short interfering RNA (siRNA) to cells in culture, and in vivo, is possible with combined use of a receptor-specific monoclonal antibody (mAb) and avidin-biotin technology. The authors also report that because the bond between the targeting mAb and the siRNA is stable with avidin-biotin technology, and RNAi effects at distant sites such as brain are observed in vivo following an intravenous administration of the targeted siRNA.

Zhang Y, Schlachetzki F, Pardridge WM. ("Global non-viral gene transfer to the primate brain following intravenous administration." Mol Ther. 2003 Jan;7(1):11-8.) describe how expression plasmids encoding reporters such as luciferase were encapsulated in the interior of an "artificial virus" comprised of an 85 nm pegylated immunoliposome, which was targeted to the rhesus monkey brain in vivo with a monoclonal antibody (MAb) to the human insulin receptor (HIR). The HIRMAb enables the liposome carrying the exogenous gene to undergo transcytosis across the blood-brain barrier and endocytosis across the neuronal plasma membrane following intravenous injection. The level of luciferase gene expression in the brain was 50-fold higher in the rhesus monkey as compared to the rat. Widespread neuronal expression of the beta-galactosidase gene in primate brain was demonstrated by both histochemistry and confocal microscopy. The authors indicate that this approach makes feasible reversible adult transgenics in 24 hours. Accordingly, the use of immunoliposome is preferred. These may be used in conjunction with antibodies to target specific tissues or cell surface proteins.

Other means of delivery or RNA are also preferred, such as via nanoparticles (Cho, S., Goldberg, M., Son, S., Xu, Q., Yang, F., Mei, Y., Bogatyrev, S., Langer, R. and Anderson, D., Lipid-like nanoparticles for small interfering RNA delivery to endothelial cells, Advanced Functional Materials, 19: 3112-3118, 2010) or exosomes (Schroeder, A., Levins, C., Cortez, C., Langer, R., and Anderson, D., Lipid-based nanotherapeutics for siRNA delivery, Journal of Internal Medicine, 267: 9-21, 2010, PMID: 20059641). Indeed, exozomes have been shown to be particularly useful in delivery siRNA, a system with some parallels to the CRISPR system. For instance, El-Andaloussi S, et al. ("Exosome-mediated delivery of siRNA in vitro and in vivo." Nat Protoc. 2012 Dec;7(12):2112-26. doi: 10.1038/nprot.2012.131. Epub 2012 Nov 15.) describe how exosomes are promising tools for drug delivery across different biological barriers and can be harnessed for delivery of siRNA in vitro and in vivo. Their approach is to generate targeted exosomes through transfection of an expression vector, comprising an exosomal protein fused with a peptide ligand. The exosomes are then purify and characterized from transfected cell supernatant, then siRNA is loaded into the exosomes.

Targeted deletion of genes is preferred. Examples are exemplified in Example 18. Preferred are, therefore, genes involved in cholesterol biosynthesis, fatty acid biosynthesis, and other metabolic disorders, genes encoding mis-folded proteins involved in amyloid and other diseases, oncogenes leading to cellular transformation, latent viral genes, and genes leading to dominant-negative disorders, amongst other disorders. As exemplified here, Applicants prefer gene delivery of a CRISPR-Cas system to the liver, brain, ocular, epithelial, hematopoetic, or another tissue of a organism, subject or a patient in need thereof, suffering from metabolic disorders, amyloidosis and protein-aggregation related diseases, cellular transformation arising from genetic mutations and translocations, dominant negative effects of gene mutations, latent viral infections, and other related symptoms, using either viral or nanoparticle delivery system.

Therapeutic applications of the CRISPR-Cas system include Glaucoma, Amyloidosis, and Huntington's disease. These are exemplified in Example 20 and the features described therein are preferred alone or in combination.

As an example, chronic infection by HIV-1 may be treated or prevented. In order to accomplish this, one may generate CRISPR-Cas guide RNAs that target the vast majority of the HIV-1 genome while taking into account HIV-1 strain variants for maximal coverage and effectiveness. One may accomplish delivery of the CRISPR-Cas system by conventional adenoviral or lentiviral-mediated infection of the host immune system. Depending on approach, host immune cells could be a) isolated, transduced with CRISPR-Cas, selected, and re-introduced in to the host or b) transduced in vivo by systemic delivery of the CRISPR-Cas system. The first approach allows for generation of a resistant immune population whereas the second is more likely to target latent viral reservoirs within the host This is discussed in more detail in the Examples section.

It is also envisaged that the present disclosure generates a gene knockout cell library. Each cell may have a single gene knocked out. This is exemplified in Example 23.

One may make a library of ES cells where each cell has a single gene knocked out, and the entire library of ES cells will have every single gene knocked out. This library is useful for the screening of gene function in cellular processes as well as diseases. To make this cell library, one may integrate Cas9 driven by an inducible promoter (e.g. doxycycline inducible promoter) into the ES cell. In addition, one may integrate a single guide RNA targeting a specific gene in the ES cell. To make the ES cell library, one may simply mix ES cells with a library of genes encoding guide RNAs targeting each gene in the human genome. One may first introduce a single BxB1 attB site into the AAVS1 locus of the human ES cell. Then one may use the BxB1 integrase to facilitate the integration of individual guide RNA genes into the BxB1 attB site in AAVS1 locus. To facilitate integration, each guide RNA gene may be contained on a plasmid that carries of a single attP site. This way BxB1 will recombine the attB site in the genome with the attP site on the guide RNA containing plasmid. To generate the cell library, one may take the library of cells that have single guide RNAs integrated and induce Cas9 expression. After induction, Cas9 mediates double strand break at sites specified by the guide RNA.

Chronic administration of protein therapeutics may elicit unacceptable immune responses to the specific protein. The immunogenicity of protein drugs can be ascribed to a few immunodominant helper T lymphocyte (HTL) epitopes. Reducing the MHC binding affinity of these HTL epitopes contained within these proteins can generate drugs with lower immunogenicity (Tangri S, et al. ("Rationally engineered therapeutic proteins with reduced immunogenicity" J Immunol. 2005 Mar 15;174(6):3187-96.) In the present disclosure the immunogenicity of the CRISPR enzyme in particular may be reduced following the approach first set out in Tangri et al with respect to erythropoietin and subsequently developed. Accordingly, directed evolution or rational design may be used to reduce the immunogenicity of the CRISPR enzyme (for instance a Cas9) in the host species (human or other species).

In Example 28, Applicants used 3 guideRNAs of interest and were able to visualize efficient DNA cleavage *in vivo* occurring only in a small subset of cells. Essentially, Applicants have shown targeted *in vivo* cleavage. In particular, this provides proof of concept that specific targeting in higher organisms such as mammals can also be achieved. It also highlights multiplex aspect in that multiple guide sequences (i.e. separate targets) can be used simultaneously (in the sense of co-delivery). In other words, Applicants used a multiple approach, with several different sequences targeted at the same time, but independently.

A suitable example of a protocol for producing AAV, a preferred vector is provided in Example 34.

Nucleotide repeat disorders are preferred conditions to be treated. These are also exemplified herein.

According to another aspect, a method of gene therapy for the treatment of an organism or a subject having a mutation in the CFTR gene is provided and comprises administering a therapeutically effective amount of a CRISPR-Cas gene therapy particle, optionally via a biocompatible pharmaceutical carrier, to the cells of an organism or a subject. Preferably, the target DNA comprises the mutation deltaF508. In general, it is of preferred that the mutation is repaired to the wildtype. In this case, the mutation is a deletion of the three nucleotides that comprise the codon for phenylalanine (F) at position 508. Accordingly, repair in this instance requires reintroduction of the missing codon into the mutant.

To implement this Gene Repair Strategy, it is preferred that an adenovirus/AAV vector system is introduced into the host cell, cells or patient. Preferably, the system comprises a Cas9 (or Cas9 nickase) and the guide RNA along with a adenovirus/AAV vector system comprising the homology repair template containing the F508 residue. This may be introduced into the organism or subject via one of the methods of delivery discussed earlier. The CRISPR-Cas system may be guided by the CFTRdelta 508 chimeric guide RNA. It targets a specific site of the CFTR genomic locus to be nicked or cleaved. After cleavage, the repair template is inserted into the cleavage site via homologous recombination correcting the deletion that results in cystic fibrosis or causes cystic fibrosis related symptoms. This strategy to direct delivery and provide systemic introduction of CRISPR systems with appropriate guide RNAs can be employed to target genetic mutations to edit or otherwise manipulate genes that cause metabolic, liver, kidney and protein diseases and disorders such as those in Table B.

For an example of CFTRdelta508 chimeric guide RNA, see Example 22 which demonstrates gene transfer or gene delivery of a CRISPR-Cas system in airways of an organism, a subject or a patient in need thereof, suffering from cystic fibrosis or from cystic fibrosis (CF) related symptoms, using adeno-associated virus (AAV) particles. In particular, they exemplify a repair strategy for Cystic Fibrosis delta F508 mutation. This type of strategy should apply across all organisms. With particular reference to CF, suitable patients may include: Human, non-primate human, canine, feline, bovine, equine and other domestic animals. In this instance, Applicants utilized a CRISPR-Cas system comprising a Cas9 enzyme to target deltaF508 or other CFTR-inducing mutations.

The treated subjects in this instance receive pharmaceutically effective amount of aerosolized AAV vector system per lung endobronchially delivered while spontaneously breathing. As such, aerosolized delivery is preferred for AAV delivery in general. An adenovirus or an AAV particle may be used for delivery. Suitable gene constructs, each operably linked to one or more regulatory sequences, may be cloned into the delivery vector. In this instance, the following constructs are provided as examples: Cbh or EFIa promoter for Cas9, U6 or HI promoter for chimeric guide RNA),: A preferred arrangement is to use a CFTRdelta508 targeting chimeric guide, a repair template for deltaF508 mutation and a codon optimized Cas9 enzyme (preferred Cas9s are those with nuclease or nickase activity) with optionally one or more nuclear localization signal or sequence(s) (NLS(s)), e.g., two (2) NLSs. Constructs without NLS are also envisaged.

In order to identify the Cas9 target site, Applicants analyzed the human CFTR genomic locus and identified the Cas9 target site. Preferably, in general and in this CF case, the PAM may contain a NGG or a NNAGAAW motif.

Accordingly, in the case of CF, the present method comprises manipulation of a target sequence in a genomic locus of interest comprising delivering a non-naturally occurring or engineered composition comprising a viral vector system comprising one or more viral vectors operably encoding a composition for expression thereof, wherein the composition comprises:

a non-naturally occurring or engineered composition comprising a vector system comprising one or more vectors comprising

I. a first regulatory element operably linked to a CRISPR-Cas system chimeric RNA (chiRNA) polynucleotide sequence, wherein the polynucleotide sequence comprises

(a) a guide sequence capable of hybridizing to the CF target sequence in a suitable mammalian cell,
(b) a tracr mate sequence, and
(c) a tracr sequence, and

II. a second regulatory element operably linked to an enzyme-coding sequence encoding a CRISPR enzyme comprising at least one or more nuclear localization sequences,

wherein (a), (b) and (c) are arranged in a 5' to 3' orientation,
wherein components I and II are located on the same or different vectors of the system, wherein when transcribed, the tracr mate sequence hybridizes to the tracr sequence and the guide sequence directs sequence-specific binding of a CRISPR complex to the target sequence, and
wherein the CRISPR complex comprises the CRISPR enzyme complexed with (1) the guide sequence that is hybridizable to the target sequence, and (2) the tracr mate sequence that is hybridized to the tracr sequence. In respect of CF, preferred target DNA sequences comprise the CFTRdelta508 mutation. A preferred PAM is described above. A preferred CRISPR enzyme is any Cas (described herein, but particularly that described in Example 22).

Alternatives to CF include any genetic disorder and examples of these are well known. Another preferred method or use is for correcting defects in the EMP2A and EMP2B genes that have been identified to be associated with Lafora disease.

In some embodiments, a "guide sequence" may be distinct from "guide RNA". A guide sequence may refer to an approx. 20bp sequence, within the guide RNA, that specifies the target site.

In some embodiments, the Cas9 is (or is derived from) the SpCas9. In such embodiments, preferred mutations are at any or all or positions 10, 762, 840, 854, 863 and/or 986 of SpCas9 or corresponding positions in other Cas9s (which may be ascertained for instance by standard sequence comparison tools. In particular, any or all of the following mutations are preferred in SpCas9: D10A, E762A, H840A, N854A, N863A and/or D986A; as well as conservative substitution for any of the replacement amino acids is also envisaged. The same (or conservative substitutions of these mutations) at corresponding positions in other Cas9s are also preferred. Particularly preferred are D10 and H840 in SpCas9. However, in other Cas9s, residues corresponding to SpCas9 D10 and H840 are also preferred. These are advantageous as they provide nickase activity.

It will be readily apparent that a host of other diseases can be treated in a similar fashion. Some examples of genetic diseases caused by mutations are provided herein, but many more are known. The above strategy can be applied to these diseases.

The disclosure uses nucleic acids to bind target DNA sequences. This is advantageous as nucleic acids are much easier and cheaper to produce and the specificity can be varied according to the length of the stretch where homology is sought. Complex 3-D positioning of multiple fingers, for example is not required.

The terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), microRNA (miRNA), ribozymes, cDNA, recombinant polynucleotides, branched polynucleotides, plasmids, vectors, isolated DNA of any sequence, isolated RNA of any sequence, nucleic acid probes, and primers. The term also encompasses nucleic-acid-like structures with synthetic backbones, see, e.g., Eckstein, 1991; Baserga et al., 1992; Milligan, 1993; WO 97/03211; WO 96/39154; Mata, 1997; Strauss-Soukup, 1997; and Samstag, 1996. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component

As used herein the term "wild type" is a term of the art understood by skilled persons and means the typical form of an organism, strain, gene or characteristic as it occurs in nature as distinguished from mutant or variant forms.

As used herein the term "variant" should be taken to mean the exhibition of qualities that have a pattern that deviates from what occurs in nature.

The terms "non-naturally occurring" or "engineered" are used interchangeably and indicate the involvement of the hand of man. The terms, when referring to nucleic acid molecules or polypeptides mean that the nucleic acid molecule or the polypeptide is at least substantially free from at least one other component with which they are naturally associated in nature and as found in nature.

"Complementarity" refers to the ability of a nucleic acid to form hydrogen bond(s) with another nucleic acid sequence by either traditional Watson-Crick base pairing or other non-traditional types. A percent complementarity indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

As used herein, "stringent conditions" for hybridization refer to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993), Laboratory Techniques In Biochemistry And Molecular Biology-Hybridization With Nucleic Acid Probes Part I, Second Chapter "Overview of principles of hybridization and the strategy of nucleic acid probe assay", Elsevier, N.Y. Where reference is made to a polynucleotide sequence, then complementary or partially complementary sequences are also envisaged. These are preferably capable of hybridising to the reference sequence under highly stringent conditions. Generally, in order to maximize the hybridization rate, relatively low-stringency hybridization conditions are selected: about 20 to 25° C. lower than the thermal melting point ($T_m$). The $T_m$ is the temperature at which 50% of specific target sequence hybridizes to a perfectly complementary probe in solution at a defined ionic strength and pH. Generally, in order to require at least about 85% nucleotide complementarity of hybridized sequences, highly stringent washing conditions are selected to be about 5 to 15° C. lower than the $T_m$. In order to require at least about 70% nucleotide complementarity of hybridized sequences, moderately-stringent washing conditions are selected to be about 15 to 30° C. lower than the $T_m$. Highly permissive (very low stringency) washing conditions may be as low as 50° C. below the $T_m$, allowing a high level of mis-matching between hybridized sequences. Those skilled in the art will recognize that other physical and chemical parameters in the hybridization and wash stages can also be altered to affect the outcome of a detectable hybridization signal from a specific level of homology between target and probe sequences. Preferred highly stringent conditions comprise incubation in 50% formamide, 5×SSC, and 1% SDS at 42° C., or incubation in 5×SSC and 1% SDS at 65° C., with wash in 0.2×SSC and 0.1% SDS at 65° C.

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PCR, or the cleavage of a polynucleotide by an enzyme. A sequence capable of hybridizing with a given sequence is referred to as the "complement" of the given sequence.

As used herein, the term "genomic locus" or "locus" (plural loci) is the specific location of a gene or DNA sequence on a chromosome. A "gene" refers to stretches of DNA or RNA that encode a polypeptide or an RNA chain that has functional role to play in an organism and hence is the molecular unit of heredity in living organisms. For the purpose of this invention it may be considered that genes include regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

As used herein, "expression of a genomic locus" or "gene expression" is the process by which information from a gene is used in the synthesis of a functional gene product. The products of gene expression are often proteins, but in non-protein coding genes such as rRNA genes or tRNA genes, the product is functional RNA. The process of gene expression is used by all known life - eukaryotes (including multicellular organisms), prokaryotes (bacteria and archaea) and viruses to generate functional products to survive. As used herein "expression" of a gene or nucleic acid encompasses not only cellular gene expression, but also the transcription and translation of nucleic acid(s) in cloning systems and in any other context. As used herein, "expression" also refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The terms "polypeptide", "peptide" and "protein" are used interchangeably herein to refer to polymers of amino acids of any length. The polymer may be linear or branched, it may comprise modified amino acids, and it may be interrupted by non amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" includes natural and/or unnatural or synthetic amino acids, including glycine and both the D or L optical isomers, and amino acid analogs and peptidomimetics.

As used herein, the term "domain" or "protein domain" refers to a part of a protein sequence that may exist and function independently of the rest of the protein chain.

As described in aspects sequence identity is related to sequence homology. Homology comparisons may be conducted by eye, or more usually, with the aid of readily available sequence comparison programs. These commercially available computer programs may calculate percent (%) homology between two or more sequences and may also calculate the sequence identity shared by two or more amino acid or nucleic acid sequences. In some preferred embodiments, the capping region of the dTALEs described herein have sequences that are at least 95% identical or share identity to the capping region amino acid sequences provided herein.

Sequence homologies may be generated by any of a number of computer programs known in the art, for example BLAST or FASTA, etc. A suitable computer program for carrying out such an alignment is the GCG Wisconsin Bestfit package (University of Wisconsin, U.S.A; Devereux et al., 1984, Nucleic Acids Research 12:387). Examples of other software than may perform sequence comparisons include, but are not limited to, the BLAST package (see Ausubel et al., 1999 ibid - Chapter 18), FASTA (Atschul et al., 1990, J. Mol. Biol., 403-410) and the GENEWORKS suite of comparison tools. Both BLAST and FASTA are available for offline and online searching (see Ausubel et al., 1999 ibid, pages 7-58 to 7-60). However it is preferred to use the GCG Bestfit program.

Percentage (%) sequence homology may be calculated over contiguous sequences, i.e., one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion may cause the following amino acid residues to be put out of alignment, thus potentially resulting in a large reduction in % homology when a global alignment is performed. Consequently, most sequence comparison methods are designed to produce optimal alignments that take into consideration possible insertions and deletions without unduly penalizing the overall homology or identity score. This is achieved by inserting "gaps" in the sequence alignment to try to maximize local homology or identity.

However, these more complex methods assign "gap penalties" to each gap that occurs in the alignment so that, for the same number of identical amino acids, a sequence alignment with as few gaps as possible - reflecting higher relatedness between the two compared sequences - may achieve a higher score than one with many gaps. "Affinity gap costs" are typically used that charge a relatively high cost for the existence of a gap and a smaller penalty for each subsequent residue in the gap. This is the most commonly used gap scoring system. High gap penalties may, of course, produce optimized alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum % homology therefore first requires the production of an optimal alignment, taking into consideration gap penalties. A suitable computer program for carrying out such an alignment is the GCG Wisconsin Bestfit package (Devereux et al., 1984 Nuc. Acids Research 12 p387). Examples of other software than may perform sequence comparisons include, but are not limited to, the BLAST package (see Ausubel et al., 1999 Short Protocols in Molecular Biology, 4th Ed. - Chapter 18), FASTA (Altschul et al., 1990 J Mol. Biol. 403-410) and the GENEWORKS suite of comparison tools. Both BLAST and FASTA are available for offline and online searching (see Ausubel et al., 1999, Short Protocols in Molecular Biology, pages 7-58 to 7-60). However, for some applications, it is preferred to use the GCG Bestfit program. A new tool, called BLAST 2 Sequences is also available for comparing protein and nucleotide sequences (see FEMS Microbiol Lett. 1999 174(2): 247-50; FEMS Microbiol Lett. 1999 177(1): 187-8 and the website of the National Center for Biotechnology information at the website of the National Institutes for Health).

Although the final % homology may be measured in terms of identity, the alignment process itself is typically not based on an all-or-nothing pair comparison. Instead, a scaled similarity score matrix is generally used that assigns scores to each pair-wise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table, if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1), 237-244). Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in amino acid properties (such as polarity, charge, solubility, hydrophobicity, hydrophilicity, and/or the amphipathic nature of the residues) and it is therefore useful to group amino acids together in functional groups. Amino acids may be grouped together based on the properties of their side chains alone. However, it is more useful to include mutation data as well. The sets of amino acids thus derived are likely to be conserved for structural reasons. These sets may be described in the form of a Venn diagram (Livingstone C.D. and Barton G.J. (1993) "Protein sequence alignments: a strategy for the hierarchical analysis of residue conservation" Comput Appl. Biosci. 9: 745-756) (Taylor W.R. (1986) "The classification of amino acid conservation" J. Theor. Biol. 119; 205-218). Conservative substitutions may be made, for example according to the table below which describes a generally accepted Venn diagram grouping of amino acids.

| Set | | Sub-set | |
|---|---|---|---|
| Hydrophobic | F W Y H K M I L V A G C | Aromatic | F W Y H |
| | | Aliphatic | I L V |
| Polar | W Y H K R E D C S T N Q | Charged | H K R E D |
| | | Positively charged | H K R |
| | | Negatively charged | E D |
| Small | V C A G S P T N D | Tiny | A G S |

Embodiments of the invention include sequences (both polynucleotide or polypeptide) which may comprise homologous substitution (substitution and replacement are both used herein to mean the interchange of an existing amino acid residue or nucleotide, with an alternative residue or nucleotide) that may occur i.e., like-for-like substitution in the case of amino acids such as basic for basic, acidic for acidic, polar for polar, etc. Non-homologous substitution may also occur i.e., from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, which involves the presence of one or more amino acid residues in peptoid form, may be well understood by those skilled in the art For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The practice of the present invention employs, unless otherwise indicated, conventional techniques of immunology, biochemistry, chemistry, molecular biology, microbiology, cell biology, genomics and recombinant DNA, which are within the skill of the art. See Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I. Freshney, ed. (1987)).

In one aspect, the disclosure provides for vectors that are used in the engineering and optimization of CRISPR-Cas systems.

A used herein, a "vector" is a tool that allows or facilitates the transfer of an entity from one environment to another. It is a replicon, such as a plasmid, phage, or cosmid, into which another DNA segment may be inserted so as to bring about the replication of the inserted segment. Generally, a vector is capable of replication when associated with the proper control elements. In general, the term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g. circular); nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Another type of vector is a viral vector, wherein virally-derived DNA or RNA sequences are present in the vector for packaging into a virus (e.g. retroviruses, replication defective retroviruses, adenoviruses, replication defective adenoviruses, and adeno-associated viruses (AAVs)). Viral vectors also include polynucleotides carried by a virus for transfection into a host cell. Certain vectors are capable of autonomous replication in a host cell into which they are introduced (e.g. bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors." Common expression vectors of utility in recombinant DNA techniques are often in the form of plasmids.

Recombinant expression vectors can comprise a nucleic acid in a form suitable for expression of the nucleic acid in a host cell, which means that the recombinant expression vectors include one or more regulatory elements, which may be selected on the basis of the host cells to be used for expression, that is operatively-linked to the nucleic acid sequence to be expressed. Within a recombinant expression vector, "operably linked" is intended to mean that the nucleotide sequence of interest is linked to the regulatory element(s) in a manner that allows for expression of the nucleotide sequence (e.g. in an in vitro transcription/translation system or in a host cell when the vector is introduced into the host cell). With regards to recombination and cloning methods, mention is made of U.S. patent application 10/815,730, published September 2, 2004 as US 2004-0171156 A1.

Aspects relate to bicistronic vectors for chimeric RNA and Cas9. Bicistronic expression vectors for chimeric RNA and Cas9 are preferred. In general and particularly in this embodiment Cas9 is preferably driven by the CBh promoter. The chimeric RNA may preferably be driven by a Pol III promoter such as a HI or a U6 promoter. Ideally the two are combined. The chimeric guide RNA typically consists of a 20bp guide sequence (Ns) and this may be joined to the tracr sequence (running from the first "U" of the lower strand to the end of the transcript). The tracr sequence may be truncated at various positions as indicated. The guide and tracr sequences are separated by the tracr-mate sequence, which may be GUUUUAGAGCUA. This may be followed by the loop sequence GAAA as shown. Both of these are preferred examples. Applicants have demonstrated Cas9-mediated indels at the human *EMX1* and *PVALB* loci by SURVEYOR assays. ChiRNAs are indicated by their "+n" designation, and crRNA refers to a hybrid RNA where guide and tracr sequences are expressed as separate transcripts. Throughout this application, chimeric RNA may also be called single guide, or synthetic guide RNA (sgRNA). The loop is preferably GAAA, but it is not limited to this sequence or indeed to being only 4bp in length. Indeed, preferred loop forming sequences for use in hairpin structures are four nucleotides in length, and most preferably have the sequence GAAA. However, longer or shorter loop sequences may be used, as may alternative sequences. The sequences preferably include a nucleotide triplet (for example, AAA), and an additional nucleotide (for example C or G). Examples of loop forming sequences include CAAA and AAAG.

The term "regulatory element" is intended to include promoters, enhancers, internal ribosomal entry sites (IRES), and other expression control elements (e.g. transcription termination signals, such as polyadenylation signals and poly-U sequences). Such regulatory elements are described, for example, in Goeddel, GENE EXPRESSION TECHNOLOGY: METHODS IN ENZYMOLOGY 185, Academic Press, San Diego, Calif. (1990). Regulatory elements include those that direct constitutive expression of a nucleotide sequence in many types of host cell and those that direct expression of the nucleotide sequence only in certain host cells (e.g., tissue-specific regulatory sequences). A tissue-specific promoter may direct expression primarily in a desired tissue of interest, such as muscle, neuron, bone, skin, blood, specific organs (e.g. liver, pancreas), or particular cell types (e.g. lymphocytes). Regulatory elements may also direct expression in a temporal-dependent manner, such as in a cell-cycle dependent or developmental stage-dependent manner, which may or may not also be tissue or cell-type specific. In some embodiments, a vector comprises one or more pol III promoter (e.g. 1, 2, 3, 4, 5, or more pol I promoters), one or more pol II promoters (e.g. 1, 2, 3, 4, 5, or more pol II promoters), one or more pol I promoters (e.g. 1, 2, 3, 4, 5, or more pol I promoters), or combinations thereof. Examples of pol III promoters include, but are not limited to, U6 and H1 promoters. Examples of pol II promoters include, but are not limited to, the retroviral Rous sarcoma virus (RSV) LTR promoter (optionally with the RSV enhancer), the cytomegalovirus (CMV) promoter (optionally with the CMV enhancer) [see, e.g., Boshart et al, Cell, 41:521-530 (1985)], the SV40 promoter, the dihydrofolate reductase promoter, the β-actin promoter, the phosphoglycerol kinase (PGK) promoter, and the EFla promoter. Also encompassed by the term "regulatory element" are enhancer elements, such as WPRE; CMV enhancers; the R-U5' segment in LTR of HTLV-I (Mol. Cell. Biol., Vol. 8(1), p. 466-472, 1988); SV40 enhancer; and the intron sequence between exons 2 and 3 of rabbit β-globin (Proc. Natl. Acad. Sci. USA., Vol. 78(3), p. 1527-31, 1981). It will be appreciated by those skilled in the art that the design of the expression vector can depend on such factors as the choice of the host cell to be transformed, the level of expression desired, etc. A vector can be introduced into host cells to thereby produce transcripts, proteins, or peptides, including fusion proteins or peptides, encoded by nucleic acids as described herein (e.g., clustered regularly interspersed short palindromic repeats (CRISPR) transcripts, proteins, enzymes, mutant forms thereof, fusion proteins thereof, etc.). With regards to regulatory sequences, mention is made of U.S. patent application 10/491,026. With regards to promoters, mention is made of PCT publication WO 2011/028929 and U.S. application 12/511, 940.

Vectors can be designed for expression of CRISPR transcripts (e.g. nucleic acid transcripts, proteins, or enzymes) in prokaryotic or eukaryotic cells. For example, CRISPR transcripts can be expressed in bacterial cells such as Escherichia coli, insect cells (using baculovirus expression vectors), yeast cells, or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated in vitro, for example using T7 promoter regulatory sequences and T7 polymerase.

Vectors may be introduced and propagated in a prokaryote or prokaryotic cell. In some embodiments, a prokaryote is used to amplify copies of a vector to be introduced into a eukaryotic cell or as an intermediate vector in the production of a vector to be introduced into a eukaryotic cell (e.g. amplifying a plasmid as part of a viral vector packaging system). In some embodiments, a prokaryote is used to amplify copies of a vector and express one or more nucleic acids, such as to provide a source of one or more proteins for delivery to a host cell or host organism. Expression of proteins in prokaryotes is most often carried out in Escherichia coli with vectors containing constitutive or inducible promoters directing the expression of either fusion or non-fusion proteins. Fusion vectors add a number of amino acids to a protein encoded therein, such as to the amino terminus of the recombinant protein. Such fusion vectors may serve one or more purposes, such as: (i) to increase expression of recombinant protein; (ii) to increase the solubility of the recombinant protein; and (iii) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Example fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion E. coli expression vectors include pTrc (Amrann et al., (1988) Gene 69:301-315) and pET 11d (Studier et al., GENE EXPRESSION TECHNOLOGY: METHODS IN ENZYMOLOGY 185, Academic Press, San Diego, Calif. (1990) 60-89).

In some embodiments, a vector is a yeast expression vector. Examples of vectors for expression in yeast Saccharomyces cerivisae include pYepSecl (Baldari, et al., 1987. EMBO J. 6: 229-234), pMFa (Kuijan and Herskowitz, 1982. Cell 30: 933-943), pJRY88 (Schultz et al., 1987. Gene 54: 113-123), pYES2 (Invitrogen Corporation, San Diego, Calif.), and picZ (InVitrogen Corp, San Diego, Calif.).

In some embodiments, a vector drives protein expression in insect cells using baculovirus expression vectors. Baculovirus vectors available for expression of proteins in cultured insect cells (e.g., SF9 cells) include the pAc series (Smith, et al., 1983. Mol. Cell. Biol. 3: 2156-2165) and the pVL series (Lucklow and Summers, 1989. Virology 170: 31-39).

In some embodiments, a vector is capable of driving expression of one or more sequences in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are typically provided by one or more regulatory elements. For example, commonly used promoters are derived from polyoma, adenovirus 2, cytomegalovirus, simian virus 40, and others disclosed herein and known in the art. For other suitable expression systems for both prokaryotic and eukaryotic cells see, e.g., Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

In some embodiments, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Baneiji, et al., 1983. Cell 33: 729-740; Queen and Baltimore, 1983. Cell 33: 741-748), neuron-specific promoters (e.g., the neurofilament promoter; Byrne and Ruddle, 1989. Proc. Natl. Acad. Sci. USA 86: 5473-5477), pancreas-specific promoters (Edlund, et al., 1985. Science 230: 912-916), and mammary gland-specific promoters (e.g., milk whey promoter; U.S. Pat. No. 4,873,316 and European Application Publication No. 264,166). Developmentally-regulated promoters are also encompassed, e.g., the murine hox promoters (Kessel and Gruss, 1990. Science 249: 374-379) and the $\alpha$-fetoprotein promoter (Campes and Tilghman, 1989. Genes Dev. 3: 537-546). With regards to these prokaryotic and eukaryotic vectors, mention is made of U.S. Patent 6,750,059. Other embodiments of the invention may relate to the use of viral vectors, with regards to which mention is made of U.S. Patent application 13/092,085. Tissue-specific regulatory elements are known in the art and in this regard, mention is made of U.S. Patent 7,776,321.

In some embodiments, a regulatory element is operably linked to one or more elements of a CRISPR system so as to drive expression of the one or more elements of the CRISPR system. In general, CRISPRs (Clustered Regularly Interspaced Short Palindromic Repeats), also known as SPIDRs (SPacer Interspersed Direct Repeats), constitute a family of DNA loci that are usually specific to a particular bacterial species. The CRISPR locus comprises a distinct class of interspersed short sequence repeats (SSRs) that were recognized in E. coli (Ishino et al., J. Bacteriol., 169:5429-5433 [1987]; and Nakata et al., J. Bacteriol., 171:3553-3556 [1989]), and associated genes. Similar interspersed SSRs have been identified in Haloferax mediterranei, Streptococcus pyogenes, Anabaena, and Mycobacterium tuberculosis (See, Groenen et al., Mol. Microbiol., 10:1057-1065 [1993]; Hoe et al., Emerg. Infect. Dis., 5:254-263 [1999]; Masepohl et al., Biochim. Biophys. Acta 1307:26-30 [1996]; and Mojica et al., Mol. Microbiol., 17:85-93 [1995]). The CRISPR loci typically differ from other SSRs by the structure of the repeats, which have been termed short regularly spaced repeats (SRSRs) (Janssen et al., OMICS J. Integ. Biol., 6:23-33 [2002]; and Mojica et al., Mol. Microbiol., 36:244-246 [2000]). In general, the repeats are short elements that occur in clusters that are regularly spaced by unique intervening sequences with a substantially constant length (Mojica et al., [2000], supra). Although the repeat sequences are highly conserved between strains, the number of interspersed repeats and the sequences of the spacer regions typically differ from strain to strain (van Embden et al., J. Bacteriol., 182:2393-2401 [2000]). CRISPR loci have been identified in more than 40 prokaryotes (See e.g., Jansen et al., Mol. Microbiol., 43:1565-1575 [2002]; and Mojica et al., [2005]) including, but not limited to Aeropyrum, Pyrobaculum, Sulfolobus, Archaeoglobus, Halocarcula, Methanobacterium, Methanococcus, Methanosarcina, Methanopyrus, Pyrococcus, Picrophilus, Thermoplasma, Corynebacterium, Mycobacterium, Streptomyces, Aquifex, Porphyromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myxococcus, Campylobacter, Wolinella, Acinetobacter, Erwinia, Escherichia, Legionella, Methylococcus, Pasteurella, Photobacterium, Salmonella, Xanthomonas, Yersinia, Treponema, and Thermotoga.

In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or other sequences and transcripts from a CRISPR locus. In embodiments the terms guide sequence and guide RNA are used interchangeably. In some embodiments, one or more elements of a CRISPR system is derived from a type I, type II, or type III CRISPR system. In some embodiments, one or more elements of a CRISPR system is derived from a particular organism comprising an endogenous CRISPR system, such as Streptococcus pyogenes. In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise any polynucleotide, such as DNA or RNA polynucleotides. In some embodiments, a target sequence is located in the nucleus or cytoplasm of a cell.

In some embodiments, direct repeats may be identified *in silico* by searching for repetitive motifs that fulfill any or all of the following criteria:

1. found in a 2Kb window of genomic sequence flanking the type II CRISPR locus;
2. span from 20 to 50 bp; and
3. interspaced by 20 to 50 bp.

In some embodiments, 2 of these criteria may be used, for instance 1 and 2, 2 and 3, or 1 and 3. In some embodiments, all 3 criteria may be used.

In some embodiments, candidate tracrRNA may be subsequently predicted by sequences that fulfill any or all of the following criteria:

1. sequence homology to direct repeats (motif search in Geneious with up to 18-bp mismatches);
2. presence of a predicted Rho-independent transcriptional terminator in direction of transcription; and
3. stable hairpin secondary structure between tracrRNA and direct repeat.

In some embodiments, 2 of these criteria may be used, for instance 1 and 2, 2 and 3, or 1 and 3. In some embodiments, all 3 criteria may be used.

In some embodiments, chimeric synthetic guide RNAs (sgRNAs) designs may incorporate at least 12 bp of duplex structure between the direct repeat and tracrRNA.

In preferred embodiments the CRISPR system is a type II CRISPR system and the Cas enzyme is Cas9, which catalyzes DNA cleavage. Enzymatic action by Cas9 derived from Streptococcus pyogenes or any closely related Cas9 generates double stranded breaks at target site sequences which hybridize to 20 nucleotides of the guide sequence and that have a protospacer-adjacent motif (PAM) sequence (examples include NGG/NRG or a PAM that can be determined as described herein) following the 20 nucleotides of the target sequence. CRISPR activity through Cas9 for site-specific DNA recognition and cleavage is defined by the guide sequence, the tracr sequence that hybridizes in part to the guide sequence and the PAM sequence. More aspects of the CRISPR system are described in Karginov and Hannon, The CRISPR system: small RNA-guided defence in bacteria and archaea, Mole Cell 2010, January 15; 37(1): 7.

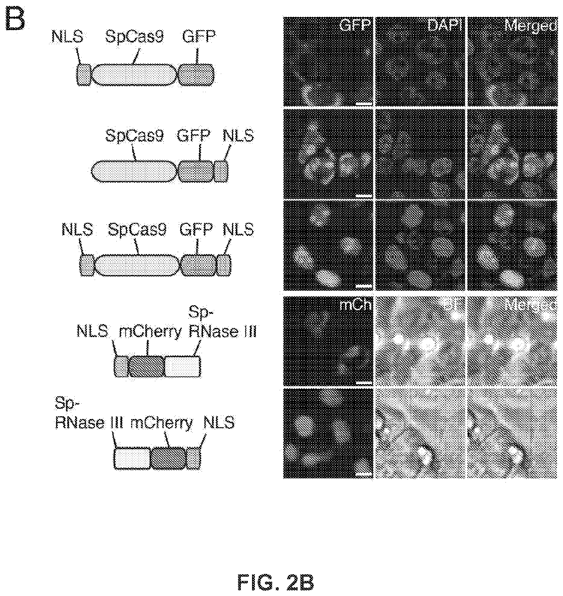

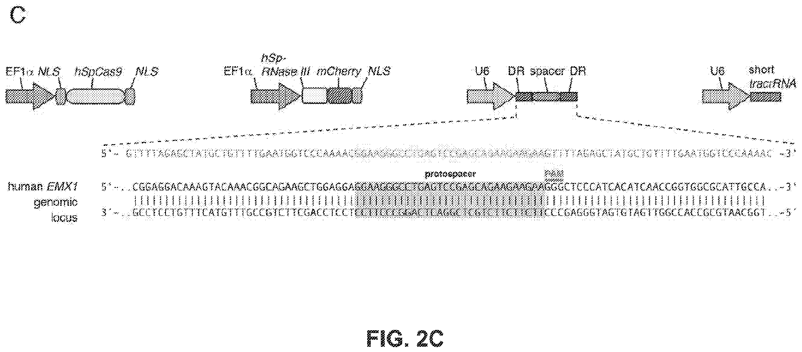

The type II CRISPR locus from Streptococcus pyogenes SF370, which contains a cluster of four genes Cas9, Cas1, Cas2, and Csn1, as well as two non-coding RNA elements, tracrRNA and a characteristic array of repetitive sequences (direct repeats) interspaced by short stretches of non-repetitive sequences (spacers, about 30bp each). In this system, targeted DNA double-strand break (DSB) is generated in four sequential steps (Fig. 2A). First, two non-coding RNAs, the pre-crRNA array and tracrRNA, are transcribed from the CRISPR locus. Second, tracrRNA hybridizes to the direct repeats of pre-crRNA, which is then processed into mature crRNAs containing individual spacer sequences. Third, the mature crRNA:tracrRNA complex directs Cas9 to the DNA target consisting of the protospacer and the corresponding PAM via heteroduplex formation between the spacer region of the crRNA and the protospacer DNA. Finally, Cas9 mediates cleavage of target DNA upstream of PAM to create a DSB within the protospacer (Fig. 2A). Fig. 2B demonstrates the nuclear localization of the codon optimized Cas9. To promote precise transcriptional initiation, the RNA polymerase III-based U6 promoter was selected to drive the expression of tracrRNA (Fig. 2C). Similarly, a U6 promoter-based construct was developed to express a pre-crRNA array consisting of a single spacer flanked by two direct repeats (DRs, also encompassed by the term "tracr-mate sequences"; Fig. 2C). The initial spacer was designed to target a 33-base-pair (bp) target site (30-bp protospacer plus a 3-bp CRISPR motif (PAM) sequence satisfying the NGG recognition motif of Cas9) in the human EMX1 locus (Fig. 2C), a key gene in the development of the cerebral cortex.

Typically, in the context of an endogenous CRISPR system, formation of a CRISPR complex (comprising a guide sequence hybridized to a target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. Without wishing to be bound by theory, the tracr sequence, which may comprise or consist of all or a portion of a wild-type tracr sequence (e.g. about or more than about 20, 26, 32, 45, 48, 54, 63, 67, 85, or more nucleotides of a wild-type tracr sequence), may also form part of a CRISPR complex, such as by hybridization along at least a portion of the tracr sequence to all or a portion of a tracr mate sequence that is operably linked to the guide sequence. In some embodiments, one or more vectors driving expression of one or more elements of a CRISPR system are introduced into a host cell such that expression of the elements of the CRISPR system direct formation of a CRISPR complex at one or more target sites. For example, a Cas enzyme, a guide sequence linked to a tracr-mate sequence, and a tracr sequence could each be operably linked to separate regulatory elements on separate vectors. Alternatively, two or more of the elements expressed from the same or different regulatory elements, may be combined in a single vector, with one or more additional vectors providing any components of the CRISPR system not included in the first vector. CRISPR system elements that are combined in a single vector may be arranged in any suitable orientation, such as one element located 5' with respect to ("upstream" of) or 3' with respect to ("downstream" of) a second element. The coding sequence of one element may be located on the same or opposite strand of the coding sequence of a second element, and oriented in the same or opposite direction. In some embodiments, a single promoter drives expression of a transcript encoding a CRISPR enzyme and one or more of the guide sequence, tracr mate sequence (optionally operably linked to the guide sequence), and a tracr sequence embedded within one or more intron sequences (e.g. each in a different intron, two or more in at least one intron, or all in a single intron). In some embodiments, the CRISPR enzyme, guide sequence, tracr mate sequence, and tracr sequence are operably linked to and expressed from the same promoter.

In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. In some embodiments, a vector comprises an insertion site upstream of a tracr mate sequence, and optionally downstream of a regulatory element operably linked to the tracr mate sequence, such that following insertion of a guide sequence into the insertion site and upon expression the guide sequence directs sequence-specific binding of a CRISPR complex to a target sequence in a eukaryotic cell. In some embodiments, a vector comprises two or more insertion sites, each insertion site being located between two tracr mate sequences so as to allow insertion of a guide sequence at each site. In such an arrangement, the two or more guide sequences may comprise two or more copies of a single guide sequence, two or more different guide sequences, or combinations of these. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell.

In some embodiments, a vector comprises a regulatory element operably linked to an enzyme-coding sequence encoding a CRISPR enzyme, such as a Cas protein. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologues thereof, or modified versions thereof. In some embodiments, the unmodified CRISPR enzyme has DNA cleavage activity, such as Cas9. In some embodiments, the CRISPR enzyme directs cleavage of one or both strands at the location of a target sequence, such as within the target sequence and/or within the complement of the target sequence. In some embodiments, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, a vector encodes a CRISPR enzyme that is mutated to with respect to a corresponding wild-type enzyme such that the mutated CRISPR enzyme lacks the ability to cleave one or both strands of a target polynucleotide containing a target sequence. For example, an aspartate-to-alanine substitution (D10A) in the RuvC I catalytic domain of Cas9 from S. pyogenes converts Cas9 from a nuclease that cleaves both strands to a nickase (cleaves a single strand). Other examples of mutations that render Cas9 a nickase include, without limitation, H840A, N854A, and N863A. As a further example, two or more catalytic domains of Cas9 (RuvC I, RuvC II, and RuvC III or the HNH domain) may be mutated to produce a mutated Cas9 substantially lacking all DNA cleavage activity. In some embodiments, a D10A mutation is combined with one or more of H840A, N854A, or N863A mutations to produce a Cas9 enzyme substantially lacking all DNA cleavage activity. In some embodiments, a CRISPR enzyme is considered to substantially lack all DNA cleavage activity when the DNA cleavage activity of the mutated enzyme is less than about 25%, 10%, 5%, 1%, 0.1%, 0.01%, or lower with respect to its non-mutated form.

An aspartate-to-alanine substitution (D10A) in the RuvC I catalytic domain of SpCas9 was engineered to convert the nuclease into a nickase (SpCas9n) (see e.g. Sapranauskas et al., 2011, Nucleic Acis Research, 39: 9275; Gasiunas et al., 2012, Proc. Natl. Acad. Sci. USA, 109:E2579), such that nicked genomic DNA undergoes the high-fidelity homology-directed repair (HDR). Surveyor assay confirmed that SpCas9n does not generate indels at the EMX1 protospacer target. Co-expression of EMX1-targeting chimeric crRNA (having the tracrRNA component as well) with SpCas9 produced indels in the target site, whereas co-expression with SpCas9n did not (n=3). Moreover, sequencing of 327 amplicons did not detect any indels induced by SpCas9n. The same locus was selected to test CRISPR-mediated HR by co-transfecting HEK 293FT cells with the chimeric RNA targeting EMX1, hSpCas9 or hSpCas9n, as well as a HR template to introduce a pair of restriction sites (HindIII and NheI) near the protospacer.

Preferred orthologs are described herein. A Cas enzyme may be identified Cas9 as this can refer to the general class of enzymes that share homology to the biggest nuclease with multiple nuclease domains from the type II CRISPR system. Most preferably, the Cas9 enzyme is from, or is derived from, spCas9 or saCas9. By derived, Applicants mean that the derived enzyme is largely based, in the sense of having a high degree of sequence homology with, a wildtype enzyme, but that it has been mutated (modified) in some way as described herein.

It will be appreciated that the terms Cas and CRISPR enzyme are generally used herein interchangeably, unless otherwise apparent. As mentioned above, many of the residue numberings used herein refer to the Cas9 enzyme from the type II CRISPR locus in *Streptococcus pyogenes.* However, it will be appreciated that this disclosure includes many more Cas9s from other species of microbes, such as SpCas9, SaCa9, St1Cas9 and so forth.

An example of a codon optimized sequence, in this instance optimized for humans (i.e. being optimized for expression in humans) is provided herein, see the SaCas9 human codon optimized sequence. Whilst this is preferred, it will be appreciated that other examples are possible and codon optimization for a host species_other than human, or for codon optimization for specific organs such as the brain, is known.

In some embodiments, an enzyme coding sequence encoding a CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human mammal or primate. In some embodiments, processes for modifying the germ line genetic identity of human beings and/or processes for modifying the genetic identity of animals which are likely to cause them suffering without any substantial medical benefit to man or animal, and also animals resulting from such processes, may be excluded.

In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database" available at www.kazusa.orjp/codon/ (visited Jul. 9, 2002), and these tables can be adapted in a number of ways. See Nakamura, Y., et al. "Codon usage tabulated from the international DNA sequence databases: status for the year 2000"Nucl. Acids Res. 28:292 (2000). Computer algorithms for codon optimizing a particular sequence for expression in a particular host cell are also available, such as Gene Forge (Aptagen; Jacobus, PA), are also available. In some embodiments, one or more codons (e.g. 1, 2, 3, 4, 5, 10, 15, 20, 25, 50, or more, or all codons) in a sequence encoding a CRISPR enzyme correspond to the most frequently used codon for a particular amino acid.

In some embodiments, a vector encodes a CRISPR enzyme comprising one or more nuclear localization sequences (NLSs), such as about or more than about 1, 2, 3, 4, 5, 6, 7, 8, 9, 10, or more NLSs. In some embodiments, the CRISPR enzyme comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g. one or more NLS at the amino-terminus and one or more NLS at the carboxy terminus). When more than one NLS is present, each may be selected independently of the others, such that a single NLS may be present in more than one copy and/or in combination with one or more other NLSs present in one or more copies. In a preferred embodiment the CRISPR enzyme comprises at most 6 NLSs. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C-terminus. Non-limiting examples of NLSs include an NLS sequence derived from: the NLS of the SV40 virus large T-antigen, having the amino acid sequence PKKKRKV (SEQ ID NO:_); the NLS from nucleoplasmin (e.g. the nucleoplasmin bipartite NLS with the sequence KRPAATKKAGQAKKKK) (SEQ ID NO:_); the c-myc NLS having the amino acid sequence PAAKRVKLD (SEQ ID NO:_)or RQRRNELKRSP (SEQ ID NO:_); the hRNPA1 M9 NLS having the sequence NQSSNFGPMKGGNFGGRSSGPYGGGGQYFAKPRNQGGY (SEQ ID NO:_); the sequence RMRIZFKNKGKDTAELRRRRVEVSVELRKAKKDEQILKRRNV (SEQ ID NO:_) of the IBB domain from importin-alpha; the sequences VSRKRPRP (SEQ ID NO:_) and PPKKARED (SEQ ID NO:_) of the myoma T protein; the sequence POPKKKPL (SEQ ID NO:_) of human p53; the sequence SALIKKKKKMAP (SEQ ID NO:_) of mouse c-abl IV; the sequences DRLRR (SEQ ID NO:_) and PKQKKRK (SEQ ID NO:_) of the influenza virus NS1; the sequence RKLKKKIKKL (SEQ ID NO:_) of the Hepatitis virus delta antigen; the sequence REKKKFLKRR (SEQ ID NO:_) of the mouse Mx1 protein; the sequence KRKGDEVDGVDEVAKKKSKK (SEQ ID NO:_) of the human poly(ADP-ribose) polymerase; and the sequence RKCLQAGMNLEARKTKK (SEQ ID NO:_) of the steroid hormone receptors (human) glucocorticoid.

In general, the one or more NLSs are of sufficient strength to drive accumulation of the CRISPR enzyme in a detectable amount in the nucleus of a eukaryotic cell. In general, strength of nuclear localization activity may derive from the number of NLSs in the CRISPR enzyme, the particular NLS(s) used, or a combination of these factors. Detection of accumulation in the nucleus may be performed by any suitable technique. For example, a detectable marker may be fused to the CRISPR enzyme, such that location within a cell may be visualized, such as in combination with a means for detecting the location of the nucleus (e.g. a stain specific for the nucleus such as DAPI). Cell nuclei may also be isolated from cells, the contents of which may then be analyzed by any suitable process for detecting protein, such as immunohistochemistry, Western blot, or enzyme activity assay. Accumulation in the nucleus may also be determined indirectly, such as by an assay for the effect of CRISPR complex formation (e.g. assay for DNA cleavage or mutation at the target sequence, or assay for altered gene expression activity affected by CRISPR complex formation and/or CRISPR enzyme activity), as compared to a control no exposed to the CRISPR enzyme or complex, or exposed to a CRISPR enzyme lacking the one or more NLSs.

In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies; available at www.novocraftcom), ELAND (Illumina, San Diego, CA), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

A guide sequence may be selected to target any target sequence. In some embodiments, the target sequence is a sequence within a genome of a cell. Exemplary target sequences include those that are unique in the target genome. For example, for the *S. pyogenes* Cas9, a unique target sequence in a genome may include a Cas9 target site of the form MMMMMMMMNNNNNNNNNNNNXGG (SEQ ID NO:_) where NNNNNNNNNNNNXGG (N is A, G, T, or C; and X can be anything) (SEQ ID NO:_) has a single occurrence in the genome. A unique target sequence in a genome may include an *S. pyogenes* Cas9 target site of the form MMMMMMMMMNNNNNNNNNNNNXGG where NNNNNNNNNNNNXGG (N is A, G, T, or C; and X can be anything) (SEQ ID NO:_) has a single occurrence in the genome. For the S. *thermophilus* CRISPR1 Cas9, a unique target sequence in a genome may include a Cas9 target site of the form MMMMMMMMNNNNNNNNNNNNXXAGAAW (SEQ ID NO:_) where NNNNNNNNNNNNXXAGAAW (N is A, G, T, or C; X can be anything; and W is A or T) (SEQ ID NO:_) has a single occurrence in the genome. A unique target sequence in a genome may include an *S. thermophilus* CRISPR1 Cas9 target site of the form MMMMMMMMMNNNNNNNNNNNNXXAGAAW (SEQ ID NO:_) where NNNNNNNNNNNNXXAGAAW (N is A, G, T, or C; X can be anything; and W is A or T) (SEQ ID NO:_) has a single occurrence in the genome. For the *S. pyogenes* Cas9, a unique target sequence in a genome may include a Cas9 target site of the form MMMMMMMMMNNNNNNNNNNNNXGGXG (SEQ ID NO:_) where NNNNNNNNNNNNXGGXG (N is A, G, T, or C; and X can be anything) (SEQ ID NO:_) has a single occurrence in the genome. A unique target sequence in a genome may include an *S. pyogenes* Cas9 target site of the form MMMMMMMMMNNNNNNNNNNNNXGGXG (SEQ ID NO:_) where NNNNNNNNNNNNXGGXG (N is A, G, T, or C; and X can be anything) (SEQ ID NO:_) has a single occurrence in the genome. In each of these sequences "M" may be A, G, T, or C, and need not be considered in identifying a sequence as unique.

In some embodiments, a guide sequence is selected to reduce the degree secondary structure within the guide sequence. In some embodiments, about or less than about 75%, 50%, 40%, 30%, 25%, 20%, 15%, 10%, 5%, 1%, or fewer of the nucleotides of the guide sequence participate in self-complementary base pairing when optimally folded. Optimal folding may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold,

developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62).

In general, a tracr mate sequence includes any sequence that has sufficient complementarity with a tracr sequence to promote one or more of: (1) excision of a guide sequence flanked by tracr mate sequences in a cell containing the corresponding tracr sequence; and (2) formation of a CRISPR complex at a target sequence, wherein the CRISPR complex comprises the tracr mate sequence hybridized to the tracr sequence. In general, degree of complementarity is with reference to the optimal alignment of the tracr mate sequence and tracr sequence, along the length of the shorter of the two sequences. Optimal alignment may be determined by any suitable alignment algorithm, and may further account for secondary structures, such as self-complementarity within either the tracr sequence or tracr mate sequence. In some embodiments, the degree of complementarity between the tracr sequence and tracr mate sequence along the length of the shorter of the two when optimally aligned is about or more than about 25%, 30%, 40%, 50%, 60%, 70%, 80%, 90%, 95%, 97.5%, 99%, or higher. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and tracr mate sequence are contained within a single transcript, such that hybridization between the two produces a transcript having a secondary structure, such as a hairpin. In an embodiment the transcript or transcribed polynucleotide sequence has at least two or more hairpins. In preferred embodiments, the transcript has two, three, four or five hairpins. In a further embodiment the transcript has at most five hairpins. In a hairpin structure the portion of the sequence 5' of the final "N" and upstream of the loop corresponds to the tracr mate sequence, and the portion of the sequence 3' of the loop corresponds to the tracr sequence Further non-limiting examples of single polynucleotides comprising a guide sequence, a tracr mate sequence, and a tracr sequence are as follows (listed 5' to 3'), where "N" represents a base of a guide sequence, the first block of lower case letters represent the tracr mate sequence, and the second block of lower case letters represent the tracr sequence, and the final poly-T sequence represents the transcription terminator: (1) NNNNNNNNNNNNNNNNNNNNgttttgtactctcaagattaGAAAtaaatcttgca- gaagctacaaagata a ggcttcatgccgaaatcaacaccctgtcattttatggcaggtgtgtttcgttatttaaTTTTTT (SEQ ID NO:_); (2) NNNNNNNNNNNNNNNNNNNNgttttgtactctca GAAA tgcagaagctacaaagata aggcttcatgccg aaatcaacaccctgtcattttat- ggcaggtgtgtttcgttatttaaTTTTTT (SEQ ID NO:_); (3) NNNNNNNNNNNNNNNNNNNNgttttgtactctcaGAAA tgcagaagcta- caaaga taaggcttcatgccg aaatcaacaccctgtcattttatggcaggtgtTTTTTT (SEQ ID NO:_); (4) NNNNNNNNNNNNNNNNNNNNgttttagagctaGAAA tagcaagttaaaataa ggctagtccgttatca actt gaaaaagtggcaccgagtcggtgcTTTTTT (SEQ ID NO:_); (5) NNNNNNNNN NNNNNNNNNNNgttttagagctaGAAATAG caagttaaaataaggctagtccgttat caac ttgaaaaagtg TTTTTTT (SEQ ID NO:_); and (6) NNNNNNNNNNNN NNNNNNNNNgtttta gagctagAAATAGcaagt- taaaataaggctagtccgttatcaTT TTTTTT (SEQ ID NO:_). In some embodiments, sequences (1) to (3) are used in combination with Cas9 from S. *thermophilus* CRISPR1. In some embodiments, sequences (4) to (6) are used in combination with Cas9 from *S. pyogenes.* In some embodiments, the tracr sequence is a separate transcript from a transcript comprising the tracr mate sequence.

In some embodiments, a recombination template is also provided. A recombination template may be a component of another vector as described herein, contained in a separate vector, or provided as a separate polynucleotide. In some embodiments, a recombination template is designed to serve as a template in homologous recombination, such as within or near a target sequence nicked or cleaved by a CRISPR enzyme as a part of a CRISPR complex. A template polynucleotide may be of any suitable length, such as about or more than about 10, 15, 20, 25, 50, 75, 100, 150, 200, 500, 1000, or more nucleotides in length. In some embodiments, the template polynucleotide is complementary to a portion of a polynucleotide comprising the target sequence. When optimally aligned, a template polynucleotide might overlap with one or more nucleotides of a target sequences (e.g. about or more than about 1, 5, 10, 15, 20, or more nucleotides). In some embodiments, when a template sequence and a polynucleotide comprising a target sequence are optimally aligned, the nearest nucleotide of the template polynucleotide is within about 1, 5, 10, 15, 20, 25, 50, 75, 100, 200, 300, 400, 500, 1000, 5000, 10000, or more nucleotides from the target sequence.

In some embodiments, the CRISPR enzyme is part of a fusion protein comprising one or more heterologous protein domains (e.g. about or more than about 1, 2, 3, 4, 5, 6, 7, 8, 9, 10, or more domains in addition to the CRISPR enzyme). A CRISPR enzyme fusion protein may comprise any additional protein sequence, and optionally a linker sequence between any two domains. Examples of protein domains that may be fused to a CRISPR enzyme include, without limitation, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporter genes include, but are not limited to, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP). A CRISPR enzyme may be fused to a gene sequence encoding a protein or a fragment of a protein that bind DNA molecules or bind other cellular molecules, including but not limited to maltose binding protein (MBP), S-tag, Lex A DNA binding domain (DBD) fusions, GAL4 DNA binding domain fusions, and herpes simplex virus (HSV) BP16 protein fusions. Additional domains that may form part of a fusion protein comprising a CRISPR enzyme are described in US20110059502. In some embodiments, a tagged CRISPR enzyme is used to identify the location of a target sequence.

In some embodiments, a CRISPR enzyme may form a component of an inducible system. The inducible nature of the system would allow for spatiotemporal control of gene editing or gene expression using a form of energy. The form of energy may include but is not limited to electromagnetic radiation, sound energy, chemical energy and thermal energy. Examples of inducible system include tetracycline inducible promoters (Tet-On or Tet-Off), small molecule two-hybrid transcription activations systems (FKBP, ABA, etc), or light inducible systems (Phytochrome, LOV domains, or cryptochrome).In one embodiment, the CRISPR enzyme may be a part of a Light Inducible Transcriptional Effector (LITE) to direct changes in transcriptional activity in a sequence-specific manner. The components of a light may include a CRISPR enzyme, a light-responsive cytochrome heterodimer (e.g. from Arabidopsis thaliana), and a transcriptional activation/repression domain. Further examples of inducible DNA binding proteins and methods for their use are provided in US 61/736465 and US 61/721,283.

In some aspects, the disclosure provides methods comprising delivering one or more polynucleotides, such as or one or more vectors as described herein, one or more transcripts thereof, and/or one or proteins transcribed therefrom, to a host cell. In some aspects, the in disclosure further provides cells produced by such methods, and animals comprising or produced from such cells. In some embodiments, a CRISPR enzyme in combination with (and optionally complexed with) a guide sequence is delivered to a cell. Conventional viral and non-viral based gene transfer methods can be used to introduce nucleic acids in mammalian cells or target tissues. Such methods can be used to administer nucleic acids encoding components of a CRISPR system to cells in culture, or in a host organism. Non-viral vector delivery systems include DNA plasmids, RNA (e.g. a transcript of a vector described herein), naked nucleic acid, and nucleic acid complexed with a delivery vehicle, such as a liposome. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Böhm (eds) (1995); and Yu et al., Gene Therapy 1:13-26 (1994).

Methods of non-viral delivery of nucleic acids include lipofection, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat Nos. 5,049,386, 4,946,787; and 4,897,355) and lipofection reagents are sold commercially (e.g., Transfectam™ and Lipofectin™). Cationic and neutral lipids that are suitable for efficient receptor-recognition lipofection of polynucleotides include those of Felgner, WO 91/17424; WO 91/16024. Delivery can be to cells (e.g. in vitro or ex vivo administration) or target tissues (e.g. in vivo administration).

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids take advantage of highly evolved processes for targeting a virus to specific cells in the body and trafficking the viral payload to the nucleus. Viral vectors can be administered directly to patients (in vivo) or they can be used to treat cells in vitro, and the modified cells may optionally be administered to patients (ex vivo). Conventional viral based systems could include retroviral, lentivirus, adenoviral, adeno-associated and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system would therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immuno deficiency virus (SIV), human immuno deficiency virus (HIV), and combinations thereof (see, e.g., Buchscher et al., J. Virol. 66:2731-2739 (1992); Johann et al., J. Virol. 66:1635-1640 (1992); Sommnerfelt et al., Virol.

176:58-59 (1990); Wilson et al., J. Virol. 63:2374-2378 (1989); Miller et al., J. Virol. 65:2220-2224 (1991); PCT/US94/05700).

In applications where transient expression is preferred, adenoviral based systems may be used. Adenoviral based vectors are capable of very high transduction efficiency in many cell types and do not require cell division. With such vectors, high titer and levels of expression have been obtained. This vector can be produced in large quantities in a relatively simple system.

Adeno-associated virus ("AAV") vectors may also be used to transduce cells with target nucleic acids, e.g., in the in vitro production of nucleic acids and peptides, and for in vivo and ex vivo gene therapy procedures (see, e.g., West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

Packaging cells are typically used to form virus particles that are capable of infecting a host cell. Such cells include 293 cells, which package adenovirus, and ψ2 cells or PA317 cells, which package retrovirus. Viral vectors used in gene therapy are usually generated by producer a cell line that packages a nucleic acid vector into a viral particle. The vectors typically contain the minimal viral sequences required for packaging and subsequent integration into a host, other viral sequences being replaced by an expression cassette for the polynucleotide(s) to be expressed. The missing viral functions are typically supplied in trans by the packaging cell line. For example, AAV vectors used in gene therapy typically only possess ITR sequences from the AAV genome which are required for packaging and integration into the host genome. Viral DNA is packaged in a cell line, which contains a helper plasmid encoding the other AAV genes, namely rep and cap, but lacking ITR sequences. The cell line may also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV.

Accordingly, AAV is considered an ideal candidate for use as a transducing vector. Such AAV transducing vectors can comprise sufficient cis-acting functions to replicate in the presence of adenovirus or herpesvirus or poxvirus (e.g., vaccinia virus) helper functions provided in trans. Recombinant AAV (rAAV) can be used to carry exogenous genes into cells of a variety of lineages. In these vectors, the AAV cap and/or rep genes are deleted from the viral genome and replaced with a DNA segment of choice. Current AAV vectors may accommodate up to 4300 bases of inserted DNA.

There are a number of ways to produce rAAV, and the disclosure provides rAAV and methods for preparing rAAV. For example, plasmid(s) containing or consisting essentially of the desired viral construct are transfected into AAV-infected cells. In addition, a second or additional helper plasmid is cotransfected into these cells to provide the AAV rep and/or cap genes which are obligatory for replication and packaging of the recombinant viral construct. Under these conditions, the rep and/or cap proteins of AAV act in trans to stimulate replication and packaging of the rAAV construct. Two to Three days after transfection, rAAV is harvested. Traditionally rAAV is harvested from the cells along with adenovirus. The contaminating adenovirus is then inactivated by heat treatment. In the instant disclosure rAAV is advantageously harvested not from the cells themselves, but from cell supernatant. Accordingly, in an initial aspect the disclosure provides for preparing rAAV, and in addition to the foregoing, rAAV can be prepared by a method that comprises or consists essentially of: infecting susceptible cells with a rAAV containing exogenous DNA including DNA for expression, and helper virus (e.g., adenovirus, herpesvirus, poxvirus such as vaccinia virus) wherein the rAAV lacks functioning cap and/or rep (and the helper virus (e.g., adenovirus, herpesvirus, poxvirus such as vaccinia virus) provides the cap and/or rev function that the rAAV lacks); or infecting susceptible cells with a rAAV containing exogenous DNA including DNA for expression, wherein the recombinant lacks functioning cap and/or rep, and transfecting said cells with a plasmid supplying cap and/or rep function that the rAAV lacks; or infecting susceptible cells with a rAAV containing exogenous DNA including DNA for expression, wherein the recombinant lacks functioning cap and/or rep, wherein said cells supply cap and/or rep function that the recombinant lacks; or transfecting the susceptible cells with an AAV lacking functioning cap and/or rep and plasmids for inserting exogenous DNA into the recombinant so that the exogenous DNA is expressed by the recombinant and for supplying rep and/or cap functions whereby transfection results in an rAAV containing the exogenous DNA including DNA for expression that lacks functioning cap and/or rep.

The rAAV can be from an AAV as herein described, and advantageously can be an rAAV1, rAAV2, AAV5 or rAAV having hybrid or capsid which may comprise AAV1, AAV2, AAV5 or any combination thereof. One can select the AAV of the rAAV with regard to the cells to be targeted by the rAAV; e.g., one can select AAV serotypes 1, 2, 5 or a hybrid or capsid AAV1, AAV2, AAV5 or any combination thereof for targeting brain or neuronal cells; and one can select AAV4 for targeting cardiac tissue.

In addition to 293 cells, other cells that can be used in the practice of the invention and the relative infectivity of certain AAV serotypes in vitro as to these cells (see Grimm, D. et al, J. Virol. 82: 5887-5911 (2008)) are as follows:

| Cell Line | AAV-1 | AAV-2 | AAV-3 | AAV-4 | AAV-5 | AAV-6 | AAV-8 | AAV-9 |
|---|---|---|---|---|---|---|---|---|
| Huh-7 | 13 | 100 | 2.5 | 0.0 | 0.1 | 10 | 0.7 | 0.0 |
| HEK293 | 25 | 100 | 2.5 | 0.1 | 0.1 | 5 | 0.7 | 0.1 |
| HeLa | 3 | 100 | 2.0 | 0.1 | 6.7 | 1 | 0.2 | 0.1 |
| HepG2 | 3 | 100 | 16.7 | 0.3 | 1.7 | 5 | 0.3 | ND |
| Hep1A | 20 | 100 | 0.2 | 1.0 | 0.1 | 1 | 0.2 | 0.0 |
| 911 | 17 | 100 | 11 | 0.2 | 0.1 | 17 | 0.1 | ND |
| CHO | 100 | 100 | 14 | 1.4 | 333 | 50 | 10 | 1.0 |
| COS | 33 | 100 | 33 | 3.3 | 5.0 | 14 | 2.0 | 0.5 |
| MeWo | 10 | 100 | 20 | 0.3 | 6.7 | 10 | 1.0 | 0.2 |
| NIH3T3 | 10 | 100 | 2.9 | 2.9 | 0.3 | 10 | 0.3 | ND |
| A549 | 14 | 100 | 20 | ND | 0.5 | 10 | 0.5 | 0.1 |
| HT1180 | 20 | 100 | 10 | 0.1 | 0.3 | 33 | 0.5 | 0.1 |
| Monocytes | 1111 | 100 | ND | ND | 125 | 1429 | ND | ND |
| Immature DC | 2500 | 100 | ND | ND | 222 | 2857 | ND | ND |
| Mature DC | 2222 | 100 | ND | ND | 333 | 3333 | ND | ND |

The disclosure provides rAAV that contains or consists essentially of an exogenous nucleic acid molecule encoding a CRISPR (Clustered Regularly Interspaced Short Palindromic Repeats) system, e.g., a plurality of cassettes comprising or consisting a first cassette comprising or consisting essentially of a promoter, a nucleic acid molecule encoding a CRISPR-associated (Cas) protein (putative nuclease or helicase proteins), e.g., Cas9 and a terminator, and a two, or more, advantageously up to the packaging size limit of the vector, e.g., in total (including the first cassette) five, cassettes comprising or consisting essentially of a promoter, nucleic acid molecule encoding guide RNA (gRNA) and a terminator (e.g., each cassette schematically represented as Promoter-gRNA1-terminator, Promoter-gRNA2-terminator ... Promoter-gRNA(N)-terminator (where N is a number that can be inserted that is at an upper limit of the packaging size limit of the vector), or two or more individual rAAVs, each containing one or more than one cassette of a CRISPR system, e.g., a first rAAV containing the first cassette comprising or consisting essentially of a promoter, a nucleic acid molecule encoding Cas, e.g., Cas9 and a terminator, and a second rAAV containing a plurality, four, cassettes comprising or consisting essentially of a promoter, nucleic acid molecule encoding guide RNA (gRNA) and a terminator (e.g., each cassette schematically represented as Promoter-gRNA1-terminator, Promoter-gRNA2-terminator ... Promoter-gRNA(N)-terminator (where N is a number that can be inserted that is at an upper limit of the packaging size limit of the vector). As rAAV is a DNA virus, the nucleic acid molecules in the herein discussion concerning AAV or rAAV are advantageously DNA. The promoter is in some embodiments advantageously human Synapsin I promoter (hSyn).

Lentiviruses are complex retroviruses that have the ability to infect and express their genes in both mitotic and post-mitotic cells. The most commonly known lentivirus is the human immunodeficiency virus (HIV), which uses the envelope glycoproteins of other viruses to target a broad range of cell types.

Lentiviruses may be prepared as follows. After cloning pCasES10 (which contains a lentiviral transfer plasmid backbone), HEK293FT at low passage (p=5) were seeded in a T-75 flask to 50% confluence the day before transfection in DMEM with 10% fetal bovine serum and without antibiotics. After 20 hours, media was changed to OptiMEM (serum-free) media and transfection was done 4 hours later. Cells were transfected with 10 μg of lentiviral transfer plasmid (pCasES10) and the following packaging plasmids: 5 μg of pMD2.G (VSV-g pseudotype), and 7.5ug of psPAX2 (gag/pol/rev/tat). Transfection was done in 4mL OptiMEM with a cationic lipid delivery agent (50uL Lipofectamine 2000 and 100ul Plus reagent). After 6 hours, the media was changed to antibiotic-free DMEM with 10% fetal bovine serum. Preparation of lentiviral vectors is further described in Example 36.

Lentivirus may be purified as follows. Viral supernatants were harvested after 48 hours. Supernatants were first cleared of debris and filtered through a 0.45um low protein binding (PVDF) filter. They were then spun in a ultracentrifuge for 2 hours at 24,000 rpm. Viral pellets were resuspended in 50ul of DMEM overnight at 4C. They were then aliquotted and immediately frozen at -80C.

In another embodiment, minimal non-primate lentiviral vectors based on the equine infectious anemia virus (EIAV) are also contemplated, especially for ocular gene therapy (see, e.g., Balagaan, J Gene Med 2006; 8: 275 - 285, Published online 21 November 2005 in Wiley InterScience (www.interscience.wiley.com). DOI: 10.1002/jgm.845). In another embodiment, RetinoStat®, an equine infectious anemia virus-based lentiviral gene therapy vector that expresses angiostatic proteins endostain and angiostatin that is delivered via a subretinal injection for the treatment of the web form of age-related macular degeneration is also contemplated (see, e.g., Binley et al., HUMAN GENE THERAPY 23:980-991 (September 2012)) may be modified for the CRISPR-Cas system.

In another embodiment, self-inactivating lentiviral vectors with an siRNA targeting a common exon shared by HIV tat/rev, a nucleolar-localizing TAR decoy, and an anti-CCR5-specific hammerhead ribozyme (see, e.g., DiGiusto et al. (2010) Sci Transl Med 2:36ra43) may be adapted to the CRISPR-Cas system. A minimum of $2.5 \times 106$ CD34+ cells per kilogram patient weight may be collected and prestimulated for 16 to 20 hours in X-VIVO 15 medium (Lonza) containing 2mML-glutamine, stem cell factor (100 ng/ml), Flt-3 ligand (Flt-3L) (100 ng/ml), and thrombopoietin (10 ng/ml) (CellGenix) at a density of $2 \times 106$ cells/ml. Prestimulated cells may be transduced with lentiviral at a multiplicity of infection of 5 for 16 to 24 hours in 75-cm2 tissue culture flasks coated with fibronectin (25 mg/cm2) (RetroNectin, Takara Bio Inc.).

Lentiviral vectors have been disclosed as in the treatment for Parkinson's Disease, see, e.g., US Patent Publication No. 20120295960 and US Patent Nos. 7303910 and 7351585. Lentiviral vectors have also been disclosed for the treatment of ocular diseases, see e.g., US Patent Publication Nos. 20060281180, 20090007284, US20110117189; US20090017543; US20070054961, US20100317109. Lentiviral vectors have also been disclosed for delivery to the train, see, e.g., US Patent Publication Nos. US20110293571; US20110293571, US20040013648, US20070025970, US20090111106 and US Patent No. US7259015.

Additional methods for the delivery of nucleic acids to cells are known to those skilled in the art. See, for example, US20030087817.

In some embodiments, a host cell is transiently or non-transiently transfected with one or more vectors described herein. In some embodiments, a cell is transfected as it naturally occurs in an organism. In some embodiments, a cell that is transfected is taken from an organism. In some embodiments, the cell is derived from cells taken from a an organism, such as a cell line. A wide variety of cell lines for tissue culture are known in the art Examples of cell lines include, but are not limited to, C8161, CCRF-CEM, MOLT, mIMCD-3, NHDF, HeLa-S3, Huh1, Huh4, Huh7, HUVEC, HASMC, HEKn, HEKa, MiaPaCell, Panc1, PC-3, TF1, CTLL-2, C1R, Rat6, CV1, RPTE, A10, T24, J82, A375, ARH-77, Calu1, SW480, SW620, SKOV3, SK-UT, CaCo2, P388D1, SEM-K2, WEHI-231, HB56, TIB55, Jurkat, J45.01, LRMB, Bcl-1, BC-3, IC21, DLD2, Raw264.7, NRK, NRK-52E, MRC5, MEF, Hep G2, HeLa B, HeLa T4, COS, COS-1, COS-6, COS-M6A, BS-C-1 monkey kidney epithelial, BALB/ 3T3 mouse embryo fibroblast, 3T3 Swiss, 3T3-L1, 132-d5 human fetal fibroblasts; 10.1 mouse fibroblasts, 293-T, 3T3, 721, 9L, A2780, A2780ADR, A2780cis, A172, A20, A253, A431, A-549, ALC, B16, B35, BCP-1 cells, BEAS-2B, bEnd.3, BHK-21, BR 293, BxPC3, C3H-10T1/2, C6/36, Cal-27, CHO, CHO-7, CHO-IR, CHO-K1, CHO-K2, CHO-T, CHO Dhfr -/-, COR-L23, COR-L23/CPR, COR-L23/5010, COR-L23/R23, COS-7, COV-434, CML T1, CMT, CT26, D17, DH82, DU145, DuCaP, EL4, EM2, EM3, EMT6/AR1, EMT6/AR10.0, FM3, H1299, H69, HB54, HB55, HCA2, HEK-293, HeLa, Hepalclc7, HL-60, HMEC, HT-29, Jurkat, JY cells, K562 cells, Ku812, KCL22, KG1, KYO1, LNCap, Ma-Mel 1-48, MC-38, MCF-7, MCF-10A, MDA-MB-231, MDA-MB-468, MDA-MB-435, MDCK II, MDCK II, MOR/0.2R, MONO-MAC 6, MTD-1A, MyEnd, NCI-H69/CPR, NCI-H69/LX10, NCI-H69/LX20, NCI-H69/LX4, NIH-3T3, NALM-1, NW-145, OPCN / OPCT cell lines, Peer, PNT-1A / PNT 2, RenCa, RIN-5F, RMA/RMAS, Saos-2 cells, Sf-9, SkBr3, T2, T-47D, T84, THP1 cell line, U373, U87, U937, VCaP, Vero cells, WM39, WT-49, X63, YAC-1, YAR, and transgenic varieties thereof. Cell lines are available from a variety of sources known to those with skill in the art (see, e.g., the American Type Culture Collection (ATCC) (Manassus, Va.)). In some embodiments, a cell transfected with one or more vectors described herein is used to establish a new cell line comprising one or more vector-derived sequences. In some embodiments, a cell transiently transfected with the components of a CRISPR system as described herein (such as by transient transfection of one or more vectors, or transfection with RNA), and modified through the activity of a CRISPR complex, is used to establish a new cell line comprising cells containing the modification but lacking any other exogenous sequence. In some embodiments, cells transiently or non-transiently transfected with one or more vectors described herein, or cell lines derived from such cells are used in assessing one or more test compounds.

In some embodiments, one or more vectors described herein are used to produce a non-human transgenic animal. In some embodiments, the transgenic animal is a mammal, such as a mouse, rat, or rabbit. Methods for producing transgenic animals are known in the art, and generally begin with a method of cell transfection, such as described herein.

In one aspect, the disclosure provides for methods of modifying a target polynucleotide in a eukaryotic cell, which may be *in vivo*, *ex vivo* or *in vitro*. In some embodiments, the method comprises sampling a cell or population of cells from a human or non-human animal, and modifying the cell or cells. Culturing may occur at any stage *ex vivo*. The cell or cells may even be re-introduced into the non-human animal. For re-introduced cells it is particularly preferred that the cells are stem cells.

In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence.

In one aspect, the disclosure provides a method of modifying expression of a polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a CRISPR enzyme complexed with a guide sequence hybridized to a target sequence within said polynucleotide, wherein said guide sequence is linked to a tracr mate sequence which in turn hybridizes to a tracr sequence. Similar considerations and conditions apply as above for methods of modifying a target polynucleotide. In fact, these sampling, culturing and re-introduction options apply across the aspects of the present disclosure.

Indeed, in any aspect the CRISPR complex may comprise a CRISPR enzyme complexed with a guide sequence hybridized to a target sequence, wherein said guide sequence may be linked to a tracr mate sequence which in turn may hybridize to a tracr sequence. Similar considerations and conditions apply as above for methods of modifying a target polynucleotide.

In one aspect, the disclosure provides kits containing any one or more of the elements disclosed in the above methods and compositions. Elements may be provided individually or in combinations, and may be provided in any suitable container, such as a vial, a bottle, or a tube. In some embodiments, the kit includes instructions in one or more languages, for example in more than one language.

In some embodiments, a kit comprises one or more reagents for use in a process utilizing one or more of the elements described herein. Reagents may be provided in any suitable container. For example, a kit may provide one or more reaction or storage buffers. Reagents may be provided in a form that is usable in a particular assay, or in a form that requires addition of one or more other components before use (e.g. in concentrate or lyophilized form). A buffer can be any buffer, , including but not limited to a sodium carbonate buffer, a sodium bicarbonate buffer, a borate buffer, a Tris buffer, a MOPS buffer, a HEPES buffer, and combinations thereof. In some embodiments, the buffer is alkaline. In some embodiments, the buffer has a pH from about 7 to about 10. In some embodiments, the kit comprises one or more oligonucleotides corresponding to a guide sequence for insertion into a vector so as to operably link the guide sequence and a regulatory element. In some embodiments, the kit comprises a homologous recombination template polynucleotide.

In one aspect, the disclosure provides methods for using one or more elements of a CRISPR system. The CRISPR complex provides an effective means for modifying a target polynucleotide. The CRISPR complex has a wide variety of utility including modifying (e.g., deleting, inserting, translocating, inactivating, activating) a target polynucleotide in a multiplicity of cell types. As such the CRISPR complex has a broad spectrum of applications in, e.g., gene therapy, drug screening, disease diagnosis, and prognosis. An exemplary CRISPR complex comprises a CRISPR enzyme complexed with a guide sequence hybridized to a target sequence within the target polynucleotide. The guide sequence is linked to a tracr mate sequence, which in turn hybridizes to a tracr sequence.

In one embodiment, this disclosure provides a method of cleaving a target polynucleotide. The method comprises modifying a target polynucleotide using a CRISPR complex that binds to the target polynucleotide and effect cleavage of said target polynucleotide. Typically, the CRISPR complex when introduced into a cell, creates a break (e.g., a single or a double strand break) in the genome sequence. For example, the method can be used to cleave a disease gene in a cell.

The break created by the CRISPR complex can be repaired by a repair processes such as the error prone non-homologous end joining (NHEJ) pathway or the high fidelity homology-directed repair (HDR) (Fig. 29). During these repair process, an exogenous polynucleotide template can be introduced into the genome sequence. In some methods, the HDR process is used modify genome sequence. For example, an exogenous polynucleotide template comprising a sequence to be integrated flanked by an upstream sequence and a downstream sequence is introduced into a cell. The upstream and downstream sequences share sequence similarity with either side of the site of integration in the chromosome.

Where desired, a donor polynucleotide can be DNA, e.g., a DNA plasmid, a bacterial artificial chromosome (BAC), a yeast artificial chromosome (YAC), a viral vector, a linear piece of DNA, a PCR fragment, a naked nucleic acid, or a nucleic acid complexed with a delivery vehicle such as a liposome or poloxamer.

The exogenous polynucleotide template comprises a sequence to be integrated (e.g., a mutated gene). The sequence for integration may be a sequence endogenous or exogenous to the cell. Examples of a sequence to be integrated include polynucleotides encoding a protein or a non-coding RNA (e.g., a microRNA). Thus, the sequence for integration may be operably linked to an appropriate control sequence or sequences. Alternatively, the sequence to be integrated may provide a regulatory function.

The upstream and downstream sequences in the exogenous polynucleotide template are selected to promote recombination between the chromosomal sequence of interest and the donor polynucleotide. The upstream sequence is a nucleic acid sequence that shares sequence similarity with the genome sequence upstream of the targeted site for integration. Similarly, the downstream sequence is a nucleic acid sequence that shares sequence similarity with the chromosomal sequence downstream of the targeted site of integration. The upstream and downstream sequences in the exogenous polynucleotide template can have 75%, 80%, 85%, 90%, 95%, or 100% sequence identity with the targeted genome sequence. Preferably, the upstream and downstream sequences in the exogenous polynucleotide template have about 95%, 96%, 97%, 98%, 99%, or 100% sequence identity with the targeted genome sequence. In some methods, the upstream and downstream sequences in the exogenous polynucleotide template have about 99% or 100% sequence identity with the targeted genome sequence.

An upstream or downstream sequence may comprise from about 20 bp to about 2500 bp, for example, about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, or 2500 bp. In some methods, the exemplary upstream or downstream sequence have about 200 bp to about 2000 bp, about 600 bp to about 1000 bp, or more particularly about 700 bp to about 1000 bp.

In some methods, the exogenous polynucleotide template may further comprise a marker. Such a marker may make it easy to screen for targeted integrations. Examples of suitable markers include restriction sites, fluorescent proteins, or selectable markers. The exogenous polynucleotide template can be constructed using recombinant techniques (see, for example, Sambrook et al., 2001 and Ausubel et al., 1996).

In an exemplary method for modifying a target polynucleotide by integrating an exogenous polynucleotide template, a double stranded break is introduced into the genome sequence by the CRISPR complex, the break is repaired via homologous recombination an exogenous polynucleotide template such that the template is integrated into the genome. The presence of a double-stranded break facilitates integration of the template.

In other embodiments, this disclosure provides a method of modifying expression of a polynucleotide in a eukaryotic cell. The method comprises increasing or decreasing expression of a target polynucleotide by using a CRISPR complex that binds to the polynucleotide.

In some methods, a target polynucleotide can be inactivated to effect the modification of the expression in a cell. For example, upon the binding of a CRISPR complex to a target sequence in a cell, the target polynucleotide is inactivated such that the sequence is not transcribed, the coded protein is not produced, or the sequence does not function as the wild-type sequence does. For example, a protein or microRNA coding sequence may be inactivated such that the protein or microRNA or pre-microRNA transcript is not produced.

In some methods, a control sequence can be inactivated such that it no longer functions as a control sequence. As used herein, "control sequence" refers to any nucleic acid sequence that effects the transcription, translation, or accessibility of a nucleic acid sequence. Examples of a control sequence include, a promoter, a transcription terminator, and an enhancer are control sequences.

The inactivated target sequence may include a deletion mutation (i.e., deletion of one or more nucleotides), an insertion mutation (i.e., insertion of one or more nucleotides), or a nonsense mutation (i.e., substitution of a single nucleotide for another nucleotide such that a stop codon is introduced). In some methods, the inactivation of a target sequence results in "knock-out" of the target sequence.

A method of the disclosure may be used to create a plant, an animal or cell that may be used as a disease model. As used herein, "disease" refers to a disease, disorder, or indication in a subject. For example, a method of the disclosure may be used to create an animal or cell that comprises a modification in one or more nucleic acid sequences associated with a disease, or a plant, animal or cell in which the expression of one or more nucleic acid sequences associated with a disease are altered. Such a nucleic acid sequence may encode a disease associated protein sequence or may be a disease associated control sequence. Accordingly, it is understood that in embodiments a plant, subject, patient, organism or cell can be a non-human subject, patient, organism or cell. Thus, the disclosure provides a plant, animal or cell, produced by the present methods, or a progeny thereof. The progeny may be a clone of the produced plant or animal, or may result from sexual reproduction by crossing with other individuals of the same species to introgress further desirable traits into their offspring. The cell may be *in vivo* or *ex vivo* in the cases of multicellular organisms, particularly animals or plants. In the instance where the cell is in cultured, a cell line may be established if appropriate culturing conditions are met and preferably if the cell is suitably adapted for this purpose (for instance a stem cell). Bacterial cell lines produced by the disclosure are also envisaged. Hence, cell lines are also envisaged.

In some methods, the disease model can be used to study the effects of mutations on the animal or cell and development and/or progression of the disease using measures commonly used in the study of the disease. Alternatively, such a disease model is useful for studying the effect of a pharmaceutically active compound on the disease.

In some methods, the disease model can be used to assess the efficacy of a potential gene therapy strategy. That is, a disease-associated gene or polynucleotide can be modified such that the disease development and/or progression is inhibited or reduced. In particular, the method comprises modifying a disease-associated gene or polynucleotide such that an altered protein is produced and, as a result, the animal or cell has an altered response. Accordingly, in some methods, a genetically modified animal may be compared with an animal predisposed to development of the disease such that the effect of the gene therapy event may be assessed.

In another embodiment, this disclosure provides a method of developing a biologically active agent that modulates a cell signaling event associated with a disease gene. The method comprises contacting a test compound with a cell comprising one or more vectors that drive expression of one or more of a CRISPR enzyme, a guide sequence linked to a tracr mate sequence, and a tracr sequence; and detecting a change in a readout that is indicative of a reduction or an augmentation of a cell signaling event associated with, e.g., a mutation in a disease gene contained in the cell.

A cell model or animal model can be constructed in combination with the method for screening a cellular function change. Such a model may be used to study the effects of a genome sequence modified by the CRISPR complex on a cellular function of interest. For example, a cellular function model may be used to study the effect of a modified genome sequence on intracellular signaling or extracellular signaling. Alternatively, a cellular function model may be used to study the effects of a modified genome sequence on sensory perception. In some such models, one or more genome sequences associated with a signaling biochemical pathway in the model are modified.

Several disease models have been specifically investigated. These include *de novo* autism risk genes CHD8, KATNAL2, and SCN2A; and the syndromic autism (Angelman Syndrome) gene UBE3A. These genes and resulting autism models are of course preferred, but serve to show the broad applicability of the invention across genes and corresponding models.

An altered expression of one or more genome sequences associated with a signaling biochemical pathway can be determined by assaying for a difference in the mRNA levels of the corresponding genes between the test model cell and a control cell, when they are contacted with a candidate agent. Alternatively, the differential expression of the sequences associated with a signaling biochemical pathway is determined by detecting a difference in the level of the encoded polypeptide or gene product.

To assay for an agent-induced alteration in the level of mRNA transcripts or corresponding polynucleotides, nucleic acid contained in a sample is first extracted according to standard methods in the art. For instance, mRNA can be isolated using various lytic enzymes or chemical solutions according to the procedures set forth in Sambrook et al. (1989), or extracted by nucleic-acid-binding resins following the accompanying instructions provided by the manufacturers. The mRNA contained in the extracted nucleic acid sample is then detected by amplification procedures or conventional hybridization assays (e.g. Northern blot analysis) according to methods widely known in the art or based on the methods exemplified herein.

For purpose of this disclosure amplification means any method employing a primer and a polymerase capable of replicating a target sequence with reasonable fidelity. Amplification may be carried out by natural or recombinant DNA polymerases such as TaqGold™, T7 DNA polymerase, Klenow fragment of E.coli DNA polymerase, and reverse transcriptase. A preferred amplification method is PCR. In particular, the isolated RNA can be subjected to a reverse transcription assay that is coupled with a quantitative polymerase chain reaction (RT-PCR) in order to quantify the expression level of a sequence associated with a signaling biochemical pathway.

Detection of the gene expression level can be conducted in real time in an amplification assay. In one aspect, the amplified products can be directly visualized with fluorescent DNA-binding agents including but not limited to DNA intercalators and DNA groove binders. Because the amount of the intercalators incorporated into the double-stranded DNA molecules is typically proportional to the amount of the amplified DNA products, one can conveniently determine the amount of the amplified products by quantifying the fluorescence of the intercalated dye using conventional optical systems in the art. DNA-binding dye suitable for this application include SYBR green, SYBR blue, DAPI, propidium iodine, Hoeste, SYBR gold, ethidium bromide, acridines, proflavine, acridine orange, acriflavine, fluorcoumanin, ellipticine, daunomycin, chloroquine, distamycin D, chromomycin, homidium, mithramycin, ruthenium polypyridyls, anthramycin, and the like.

In another aspect, other fluorescent labels such as sequence specific probes can be employed in the amplification reaction to facilitate the detection and quantification of the amplified products. Probe-based quantitative amplification relies on the sequence-specific detection of a desired amplified product. It utilizes fluorescent, target-specific probes (e.g., TaqMan® probes) resulting in increased specificity and sensitivity. Methods for performing probe-based quantitative amplification are well established in the art and are taught in U.S. Patent No. 5,210,015.

In yet another aspect, conventional hybridization assays using hybridization probes that share sequence homology with sequences associated with a signaling biochemical pathway can be performed. Typically, probes are allowed to form stable complexes with the sequences associated with a signaling biochemical pathway contained within the biological sample derived from the test subject in a hybridization reaction. It will be appreciated by one of skill in the art that where antisense is used as the probe nucleic acid, the target polynucleotides provided in the sample are chosen to be complementary to sequences of the antisense nucleic acids. Conversely, where the nucleotide probe is a sense nucleic acid, the target polynucleotide is selected to be complementary to sequences of the sense nucleic acid.

Hybridization can be performed under conditions of various stringency. Suitable hybridization conditions for the practice of the present disclosure are such that the recognition interaction between the probe and sequences associated with a signaling biochemical pathway is both sufficiently specific and sufficiently stable. Conditions that increase the stringency of a hybridization reaction are widely known and published in the art See, for example, (Sambrook, et al., (1989); Nonradioactive In Situ Hybridization Application Manual, Boehringer Mannheim, second edition). The hybridization assay can be formed using probes immobilized on any solid support, including but are not limited to nitrocellulose, glass, silicon, and a variety of gene arrays. A preferred hybridization assay is conducted on high-density gene chips as described in U.S. Patent No. 5,445,934.

For a convenient detection of the probe-target complexes formed during the hybridization assay, the nucleotide probes are conjugated to a detectable label. Detectable labels suitable for use in the present disclosure include any composition detectable by photochemical, biochemical, spectroscopic, immunochemical, electrical, optical or chemical means. A wide variety of appropriate detectable labels are known in the art, which include fluorescent or chemiluminescent labels, radioactive isotope labels, enzymatic or other ligands. In preferred embodiments, one will likely desire to employ a fluorescent label or an enzyme tag, such as digoxigenin, β-galactosidase, urease, alkaline phosphatase or peroxidase, avidin/biotin complex.

The detection methods used to detect or quantify the hybridization intensity will typically depend upon the label selected above. For example, radiolabels may be detected using photographic film or a phosphoimager. Fluorescent markers may be detected and quantified using a photodetector to detect emitted light. Enzymatic labels are typically detected by providing the enzyme with a substrate and measuring the reaction product produced by the action of the enzyme on the substrate; and finally colorimetric labels are detected by simply visualizing the colored label.

An agent-induced change in expression of sequences associated with a signaling biochemical pathway can also be determined by examining the corresponding gene products. Determining the protein level typically involves a) contacting the protein contained in a biological sample with an agent that specifically bind to a protein associated with a signaling biochemical pathway; and (b) identifying any agent:protein complex so formed. In one aspect of this embodiment, the agent that specifically binds a protein associated with a signaling biochemical pathway is an antibody, preferably a monoclonal antibody.

The reaction is performed by contacting the agent with a sample of the proteins associated with a signaling biochemical pathway derived from the test samples under conditions that will allow a complex to form between the agent and the proteins associated with a signaling biochemical pathway. The formation of the complex can be detected directly or indirectly according to standard procedures in the art. In the direct detection method, the agents are supplied with a detectable label and unreacted agents may be removed from the complex; the amount of remaining label thereby indicating the amount of complex formed. For such method, it is preferable to select labels that remain attached to the agents even during stringent washing conditions. It is preferable that the label does not interfere with the binding reaction. In the alternative, an indirect detection procedure may use an agent that contains a label introduced either chemically or enzymatically. A desirable label generally does not interfere with binding or the stability of the resulting agent:polypeptide complex. However, the label is typically designed to be accessible to an antibody for an effective binding and hence generating a detectable signal.

A wide variety of labels suitable for detecting protein levels are known in the art Non-limiting examples include radioisotopes, enzymes, colloidal metals, fluorescent compounds, bioluminescent compounds, and chemiluminescent compounds.

The amount of agent:polypeptide complexes formed during the binding reaction can be quantified by standard quantitative assays. As illustrated above, the formation of agent:polypeptide complex can be measured directly by the amount of label remained at the site of binding. In an alternative, the protein associated with a signaling biochemical pathway is tested for its ability to compete with a labeled analog for binding sites on the specific agent. In this competitive assay, the amount of label captured is inversely proportional to the amount of protein sequences associated with a signaling biochemical pathway present in a test sample.

A number of techniques for protein analysis based on the general principles outlined above are available in the art. They include but are not limited to radioimmunoassays, ELISA (enzyme linked immunoradiometric assays), "sandwich" immunoassays, immunoradiometric assays, in situ immunoassays (using e.g., colloidal gold, enzyme or radioisotope labels), western blot analysis, immunoprecipitation assays, immunofluorescent assays, and SDS-PAGE.

Antibodies that specifically recognize or bind to proteins associated with a signaling biochemical pathway are preferable for conducting the aforementioned protein analyses. Where desired, antibodies that recognize a specific type of post-translational modifications (e.g., signaling biochemical pathway inducible modifications) can be used. Post-translational modifications include but are not limited to glycosylation, lipidation, acetylation, and phosphorylation. These antibodies may be purchased from commercial vendors. For example, anti-phosphotyrosine antibodies that specifically recognize tyrosine-phosphorylated proteins are available from a number of vendors including Invitrogen and Perkin Elmer. Anti-phosphotyrosine antibodies are particularly useful in detecting proteins that are differentially phosphorylated on their tyrosine residues in response to an ER stress. Such proteins include but are not limited to eukaryotic translation initiation factor 2 alpha (eIF-2a). Alternatively, these antibodies can be generated using conventional polyclonal or monoclonal antibody technologies by immunizing a host animal or an antibody-producing cell with a target protein that exhibits the desired post-translational modification.

In practicing the subject method, it may be desirable to discern the expression pattern of an protein associated with a signaling biochemical pathway in different bodily tissue, in different cell types, and/or in different subcellular structures. These studies can be performed with the use of tissue-specific, cell-specific or subcellular structure specific antibodies capable of binding to protein markers that are preferentially expressed in certain tissues, cell types, or subcellular structures.

An altered expression of a gene associated with a signaling biochemical pathway can also be determined by examining a change in activity of the gene product relative to a control cell. The assay for an agent-induced change in the activity of a protein associated with a signaling biochemical pathway will dependent on the biological activity and/or the signal transduction pathway that is under investigation. For example, where the protein is a kinase, a change in its ability to phosphorylate the downstream substrate(s) can be determined by a variety of assays known in the art. Representative assays include but are not limited to immunoblotting and immunoprecipitation with antibodies such as anti-phosphotyrosine antibodies that recognize phosphorylated proteins. In addition, kinase activity can be detected by high throughput chemiluminescent assays such as AlphaScreen™ (available from Perkin Elmer) and eTag™ assay (Chan-Hui, et al. (2003) Clinical Immunology 111: 162-174).

Where the protein associated with a signaling biochemical pathway is part of a signaling cascade leading to a fluctuation of intracellular pH condition, pH sensitive molecules such as fluorescent pH dyes can be used as the reporter molecules. In another example where the protein associated with a signaling biochemical pathway is an ion channel, fluctuations in membrane potential and/or intracellular ion concentration can be monitored. A number of commercial kits and high-throughput devices are particularly suited for a rapid and robust screening for modulators of ion channels. Representative instruments include FLIPRTM (Molecular Devices, Inc.) and VIPR (Aurora Biosciences). These instruments are capable of detecting reactions in over 1000 sample wells of a microplate simultaneously, and providing real-time measurement and functional data within a second or even a minisecond.

In practicing any of the methods disclosed herein, a suitable vector can be introduced to a cell or an embryo via one or more methods known in the art, including without limitation, microinjection, electroporation, sonoporation, biolistics, calcium phosphate-mediated transfection, cationic transfection, liposome transfection, dendrimer transfection, heat shock transfection, nucleofection transfection, magnetofection, lipofection, impalefection, optical transfection, proprietary agent-enhanced uptake of nucleic acids, and delivery via liposomes, immunoliposomes, virosomes, or artificial virions. In some methods, the vector is introduced into an embryo by microinjection. The vector or vectors may be microinjected into the nucleus or the cytoplasm of the embryo. In some methods, the vector or vectors may be introduced into a cell by nucleofection.

The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA).

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA).

The target polynucleotide of a CRISPR complex may include a number of disease-associated genes and polynucleotides as well as signaling biochemical pathway-associated genes and polynucleotides as listed in US provisional patent applications 61/736,527 and 61/748,427 having Broad reference BI-2011/008/WSGR Docket No. 44063-701.101 and BI-2011/008/WSGR Docket No. 44063-701.102 respectively, both entitled SYSTEMS METHODS AND COMPOSITIONS FOR SEQUENCE MANIPULATION filed on December 12, 2012 and January 2, 2013, respectively.

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

Examples of disease-associated genes and polynucleotides are listed in Tables A and B. Disease specific information is available from McKusick-Nathans Institute of Genetic Medicine, Johns Hopkins University (Baltimore, Md.) and National Center for Biotechnology Information, National Library of Medicine (Bethesda, Md.), available on the World Wide Web. Examples of signaling biochemical pathway-associated genes and polynucleotides are listed in Table C.

Mutations in these genes and pathways can result in production of improper proteins or proteins in improper amounts which affect function. Further examples of genes, diseases and proteins are disclosed in US Provisional applications 61/736,527 filed on December 12, 2012 and 61/748,427 filed on January 2, 2013. Such genes, proteins and pathways may be the target polynucleotide of a CRISPR complex.

**Table A**

| DISEASE/DISORDERS | GENE(S) |
| --- | --- |
| Neoplasia | PTEN; ATM; ATR; EGFR; ERBB2; ERBB3; ERBB4; Notch1; Notch2; Notch3; Notch4; AKT; AKT2; AKT3; HIF; HIF1a; HIF3a; Met; HRG; Bcl2; PPAR alpha; PPAR gamma; WT1 (Wilms Tumor); FGF Receptor Family members (5 members: 1, 2, 3, 4, 5); CDKN2a; APC; RB (retinoblastoma); MEN1; VHL; BRCA1; BRCA2; AR (Androgen Receptor); TSG101; IGF; IGF Receptor; Igf1 (4 variants); Igf2 (3 variants); Igf 1 Receptor; Igf 2 Receptor; Bax; Bcl2; caspases family (9 members: 1, 2, 3, 4, 6, 7, 8, 9, 12); Kras; Apc |
| Age-related Macular Degeneration | Abcr; Ccl2; Cc2; cp (ceruloplasmin); Timp3; cathepsinD; Vldlr; Ccr2 |
| Schizophrenia | Neuregulin1 (Nrg1); Erb4 (receptor for Neuregulin); Complexin1 (Cplx1); Tph1 Tryptophan hydroxylase; Tph2 Tryptophan hydroxylase 2; Neurexin 1; GSK3; GSK3a; GSK3b |
| Disorders | 5-HTT (Slc6a4); COMT; DRD (Drd1a); SLC6A3; DAOA; DTNBP1; Dao (Dao1) |
| Trinucleotide Repeat Disorders | HTT (Huntington's Dx); SBMA/SMAX1/AR (Kennedy's Dx); FXN/X25 (Friedrich's Ataxia); ATX3 (Machado-Joseph's Dx); ATXN1 and ATXN2 (spinocerebellar ataxias); DMPK (myotonic dystrophy); Atrophin-1 and Atn1 (DRPLA Dx); CBP (Creb-BP - global instability); VLDLR (Alzheimer's); Atxn7; Atxn10 |
| Fragile X Syndrome | FMR2; FXR1; FXR2; mGLUR5 |
| Secretase Related Disorders | APH-1 (alpha and beta); Presenilin (PsenI); nicastrin (Ncstn); PEN-2 |
| Others | Nos1; Parp1; Nat1; Nat2 |
| Prion - related disorders | Prp |
| ALS | SOD1; ALS2; STEX; FUS; TARDBP; VEGF (VEGF-a; |

(continued)

| DISEASE/DISORDERS | GENE(S) |
|---|---|
|  | VEGF-b; VEGF-c) |
| Drug addiction | Prkce (alcohol); Drd2; Drd4; ABAT (alcohol); GRIA2; |
|  | Grm5; Grin1; Htr1b; Grin2a; Drd3; Pdyn; Grial (alcohol) |
| Autism | Mecp2; BZRAP1; MDGA2; Sema5A; Neurexin 1; Fragile X |
|  | (FMR2 (AFF2); FXR1; FXR2; Mglur5) |
| Alzheimer's Disease | E1; CHIP; UCH; UBB; Tau; LRP; PICALM; Clusterin; PS1; |
|  | SORL1; CR1; Vldlr; Uba1; Uba3; CHIP28 (Aqp1, |
|  | Aquaporin 1); Uchl1; Uchl3; APP |
| Inflammation | IL-10; IL-1 (IL-1a; IL-1b); IL-13; IL-17 (IL-17a (CTLA8); IL- |
|  | 17b; IL-17c; IL-17d; IL-17f); II-23; Cx3crl; ptpn22; TNFa; |
|  | NOD2/CARD15 for IBD; IL-6; IL-12 (IL-12a; IL-12b); |
|  | CTLA4; Cx3cl1 |
| Parkinson's Disease | x-Synuclein; DJ-1; LRRK2; Parkin; PINK1 |

**Table B:**

| Blood and coagulation diseases and disorders | Anemia (CDAN1, CDA1, RPS19, DBA, PKLR, PK1, NT5C3, UMPH1, PSN1, RHAG, RH50A, NRAMP2, SPTB, ALAS2, ANH1, ASB, ABCB7, ABC7, ASAT); Bare lymphocyte syndrome (TAPBP, TPSN, TAP2, ABCB3, PSF2, RING11, MHC2TA, C2TA, RFX5, RFXAP, RFX5), Bleeding disorders (TBXA2R, P2RX1, P2X1); Factor H and factor H-like 1 (HF1, CFH, HUS); Factor V and factor VIII (MCFD2); Factor VII deficiency (F7); Factor X deficiency (F10); Factor XI deficiency (F11); Factor XII deficiency (F12, HAF); Factor XIIIA deficiency (F13A1, F13A); Factor XIIIB deficiency (F13B); Fanconi anemia (FANCA, FACA, FA1, FA, FAA, FAAP95, FAAP90, FLJ34064, FANCB, FANCC, FACC, BRCA2, FANCD1, FANCD2, FANCD, FACD, FAD, FANCE, FACE, FANCF, XRCC9, FANCG, BRIP1, BACH1, FANCJ, PHF9, FANCL, FANCM, KIAA1596); Hemophagocytic lymphohistiocytosis disorders (PRF1, HPLH2, UNC13D, MUNC13-4, HPLH3, HLH3, FHL3); Hemophilia A (F8, F8C, HEMA); Hemophilia B (F9, HEMB), Hemorrhagic disorders (PI, ATT, F5); Leukocyde deficiencies and disorders (ITGB2, CD18, LCAMB, LAD, EIF2B1, EIF2BA, EIF2B2, EIF2B3, EIF2B5, LVWM, CACH, CLE, EIF2B4); Sickle cell anemia (HBB); Thalassemia (HBA2, HBB, HBD, LCRB, HBA1). |
|---|---|
|  |  |
| Cell dysregulation and oncology diseases and disorders | B-cell non-Hodgkin lymphoma (BCL7A, BCL7); Leukemia (TAL1, TCL5, SCL, TAL2, FLT3, NBS1, NBS, ZNFN1A1, IK1, LYF1, HOXD4, HOX4B, BCR, CML, PHL, ALL, ARNT, KRAS2, RASK2, GMPS, AF10, ARHGEF12, LARG, KIAA0382, CALM, CLTH, CEBPA, CEBP, CHIC2, BTL, FLT3, KIT, PBT, LPP, NPM1, NUP214, D9S46E, CAN, CAIN, RUNX1, CBFA2, AML1, WHSC1L1, NSD3, FLT3, AF1Q, NPM1, NUMA1, ZNF145, PLZF, PML, MYL, STAT5B, AF10, CALM, CLTH, ARL11, ARLTS1, P2RX7, P2X7, BCR, CML, PHL, ALL, GRAF, NF1, VRNF, WSS, NFNS, PTPN11, PTP2C, SHP2 NS1, BCL2, CCND1, PRAD1, BCL1, TCRA, GATA1, GF1, ERYF1, NFE1, ABL1, NQO1, DIA4, NMOR1, NUP214, D9S46E, CAN, CAIN). |
|  |  |

(continued)

| Inflammation and immune related diseases and disorders | AIDS (KIR3DL1, NKAT3, NKB1, AMB11, KIR3DS1, IFNG, CXCL12, SDF1); Autoimmune lymphoproliferative syndrome (TNFRSF6, APT1, FAS, CD95, ALPS1A); Combined immunodeficiency, (IL2RG, SCIDX1, SCIDX, IMD4); HIV-1 (CCL5, SCYA5, D17S136E, TCP228), HIV susceptibility or infection (IL10, CSIF, CMKBR2, CCR2, CMKBR5, CCCKR5 (CCR5)); Immunodeficiencies (CD3E, CD3G, AICDA, AID, HIGM2, TNFRSF5, CD40, UNG, DGU, HIGM4, TNFSF5, CD40LG, HIGM1, IGM, FOXP3, IPEX, AIID, XPID, PIDX, TNFRSF14B, TACI); Inflammation (IL-10, IL-1 (IL-1a, IL-1b), IL-13, IL-17 (IL-17a (CTLA8), IL-17b, IL-17c, IL-17d, IL-17f), Il-23, Cx3cr1, ptpn22, TNFa, NOD2/CARD15 for IBD, IL-6, IL-12 (IL-12a, IL-12b), CTLA4, Cx3cl1); Severe combined immunodeficiencies (SCIDs)(JAK3, JAKL, DCLRE1C, ARTEMIS, SCIDA, RAG1, RAG2, ADA, PTPRC, CD45, LCA, IL7R, CD3D, T3D, IL2RG, SCIDX1, SCIDX, IMD4). |
| --- | --- |
| | |
| Metabolic, liver, kidney and protein diseases and disorders | Amyloid neuropathy (TTR, PALB); Amyloidosis (APOA1, APP, AAA, CVAP, AD1, GSN, FGA, LYZ, TTR, PALB); Cirrhosis (KRT18, KRT8, CIRH1A, NAIC, TEX292, KIAA1988); Cystic fibrosis (CFTR, ABCC7, CF, MRP7); Glycogen storage diseases (SLC2A2, GLUT2, G6PC, G6PT, G6PT1, GAA, LAMP2, LAMPB, AGL, GDE, GBE1, GYS2, PYGL, PFKM); Hepatic adenoma, 142330 (TCF1, HNF1A, MODY3), Hepatic failure, early onset, and neurologic disorder (SCOD1, SCO1), Hepatic lipase deficiency (LIPC), Hepatoblastoma, cancer and carcinomas (CTNNB1, PDGFRL, PDGRL, PRLTS, AXIN1, AXIN, CTNNB1, TP53, P53, LFS1, IGF2R, MPRI, MET, CASP8, MCH5; Medullary cystic kidney disease (UMOD, HNFJ, FJHN, MCKD2, ADMCKD2); Phenylketonuria (PAH, PKU1, QDPR, DHPR, PTS); Polycystic kidney and hepatic disease (FCYT, PKHD1, ARPKD, PKD1, PKD2, PKD4, PKDTS, PRKCSH, G19P1, PCLD, SEC63). |
| | |
| Muscular / Skeletal diseases and disorders | Becker muscular dystrophy (DMD, BMD, MYF6), Duchenne Muscular Dystrophy (DMD, BMD); Emery-Dreifuss muscular dystrophy (LMNA, LMN1, EMD2, FPLD, CMD1A, HGPS, LGMD1B, LMNA, LMN1, EMD2, FPLD, CMD1A); Facioscapulohumeral muscular dystrophy (FSHMD1A, FSHD1A); Muscular dystrophy (FKRP, MDC1C, LGMD2I, LAMA2, LAMM, LARGE, KIAA0609, MDC1D, FCMD, TTID, MYOT, CAPN3, CANP3, DYSF, LGMD2B, SGCG, LGMD2C, DMDA1, SCG3, SGCA, ADL, DAG2, LGMD2D, DMDA2, SGCB, LGMD2E, SGCD, SGD, LGMD2F, CMD1L, TCAP, LGMD2G, CMD1N, TRIM32, HT2A, LGMD2H, FKRP, MDC1C, LGMD2I, TTN, CMD1G, TMD, LGMD2J, POMT1, CAV3, LGMD1C, SEPN1, SELN, RSMD1, PLEC1, PLTN, EBS1); Osteopetrosis (LRP5, BMND1, LRP7, LR3, OPPG, VBCH2, CLCN7, CLC7, OPTA2, OSTM1, GL, TCIRG1, TIRC7, OC116, OPTB1); Muscular atrophy (VAPB, VAPC, ALS8, SMN1, SMA1, SMA2, SMA3, SMA4, BSCL2, SPG17, GARS, SMAD1, CMT2D, HEXB, IGHMBP2, SMUBP2, CATF1, SMARD1). |
| | |

(continued)

| Neurological and neuronal diseases and disorders | ALS (SOD1, ALS2, STEX, FUS, TARDBP, VEGF (VEGF-a, VEGF-b, VEGF-c); Alzheimer disease (APP, AAA, CVAP, AD1, APOE, AD2, PSEN2, AD4, STM2, APBB2, FE65L1, NOS3, PLAU, URK, ACE, DCP1, ACE1, MPO, PACIP1, PAXIP1L, PTIP, A2M, BLMH, BMH, PSEN1, AD3); Autism (Mecp2, BZRAP1, MDGA2, Sema5A, Neurexin 1, GLO1, MECP2, RTT, PPMX, MRX16, MRX79, NLGN3, NLGN4, KIAA1260, AUTSX2); Fragile X Syndrome (FMR2, FXR1, FXR2, mGLUR5); Huntington's disease and disease like disorders (HD, IT15, PRNP, PRIP, JPH3, JP3, HDL2, TBP, SCA17); Parkinson disease (NR4A2, NURR1, NOT, TINUR, SNCAIP, TBP, SCA17, SNCA, NACP, PARK1, PARK4, DJ1, PARK7, LRRK2, PARK8, PINK1, PARK6, UCHL1, PARK5, SNCA, NACP, PARK1, PARK4, PRKN, PARK2, PDJ, DBH, NDUFV2); Rett syndrome (MECP2, RTT, PPMX, MRX16, MRX79, CDKL5, STK9, MECP2, RTT, PPMX, MRX16, MRX79, x-Synuclein, DJ-1); Schizophrenia (Neuregulin1 (Nrg1), Erb4 (receptor for Neuregulin), Complexin1 (Cplx1), Tph1 Tryptophan hydroxylase, Tph2, Tryptophan hydroxylase 2, Neurexin 1, GSK3, GSK3a, GSK3b, 5-HTT (Slc6a4), COMT, DRD (Drdla), SLC6A3, DAOA, DTNBP1, Dao (Dao1)); Secretase Related Disorders (APH-1 (alpha and beta), Presenilin (Psen1), nicastrin, (Ncstn), PEN-2, Nosl, Parp1, Natl, Nat2); Trinucleotide Repeat Disorders (HTT (Huntington's Dx), SBMA/SMAX1/AR (Kennedy's Dx), FXN/X25 (Friedrich's Ataxia), ATX3 (Machado-Joseph's Dx), ATXN1 and ATXN2 (spinocerebellar ataxias), DMPK (myotonic dystrophy), Atrophin-1 and Atn1 (DRPLA Dx), CBP (Creb-BP - global instability), VLDLR (Alzheimer's), Atxn7, Atxn10). |
|---|---|
| | |
| Occular diseases and disorders | Age-related macular degeneration (Abcr, Ccl2, Cc2, cp (ceruloplasmin), Timp3, cathepsinD, Vldlr, Ccr2); Cataract (CRYAA, CRYA1, CRYBB2, CRYB2, PITX3, BFSP2, CP49, CP47, CRYAA, CRYA1, PAX6, AN2, MGDA, CRYBA1, CRYB1, CRYGC, CRYG3, CCL, LIM2, MP19, CRYGD, CRYG4, BFSP2, CP49, CP47, HSF4, CTM, HSF4, CTM, MIP, AQP0, CRYAB, CRYA2, CTPP2, CRYBB1, CRYGD, CRYG4, CRYBB2, CRYB2, CRYGC, CRYG3, CCL, CRYAA, CRYA1, GJA8, CX50, CAE1, GJA3, CX46, CZP3, CAE3, CCM1, CAM, KRIT1); Corneal clouding and dystrophy (APOA1, TGFBI, CSD2, CDGG1, CSD, BIGH3, CDG2, TACSTD2, TROP2, M1S1, VSX1, RINX, PPCD, PPD, KTCN, COL8A2, FECD, PPCD2, PIP5K3, CFD); Cornea plana congenital (KERA, CNA2); Glaucoma (MYOC, TIGR, GLC1A, JOAG, GPOA, OPTN, GLC1E, FIP2, HYPL, NRP, CYP1B1, GLC3A, OPA1, NTG, NPG, CYP1B1, GLC3A); Leber congenital amaurosis (CRB1, RP12, CRX, CORD2, CRD, RPGRIP1, LCA6, CORD9, RPE65, RP20, AIPL1, LCA4, GUCY2D, GUC2D, LCA1, CORD6, RDH12, LCA3); Macular dystrophy (ELOVL4, ADMD, STGD2, STGD3, RDS, RP7, PRPH2, PRPH, AVMD, AOFMD, VMD2). |

**Table C:**

| CELLULAR FUNCTION | GENES |
|---|---|
| PI3K/AKT Signaling | PRKCE; ITGAM; ITGA5; IRAK1; PRKAA2; EIF2AK2; |
| | PTEN; EIF4E; PRKCZ; GRK6; MAPK1; TSC1; PLK1; |
| | AKT2; IKBKB; PIK3CA; CDK8; CDKN1B; NFKB2; BCL2; |
| | PIK3CB; PPP2R1A; MAPK8; BCL2L1; MAPK3; TSC2; |
| | ITGA1; KRAS; EIF4EBP1; RELA; PRKCD; NOS3; |
| | PRKAA1; MAPK9; CDK2; PPP2CA; PIM1; ITGB7; |
| | YWHAZ; ILK; TP53; RAF1; IKBKG; RELB; DYRK1A; |
| | CDKN1A; ITGB1; MAP2K2; JAK1; AKT1; JAK2; PIK3R1; |
| | CHUK; PDPK1; PPP2R5C; CTNNB1; MAP2K1; NFKB1; |
| | PAK3; ITGB3; CCND1; GSK3A; FRAP1; SFN; ITGA2; |
| | TTK; CSNK1A1; BRAF; GSK3B; AKT3; FOXO1; SGK; |
| | HSP90AA1; RPS6KB1 |

(continued)

| CELLULAR FUNCTION | GENES |
|---|---|
| ERK/MAPK Signaling | PRKCE; ITGAM; ITGA5; HSPB1; IRAK1; PRKAA2; |
| | EIF2AK2; RAC1; RAP1A; TLN1; EIF4E; ELK1; GRK6; |
| | MAPK1; RAC2; PLK1; AKT2; PIK3CA; CDK8; CREB1; |
| | PRKCI; PTK2; FOS; RPS6KA4; PIK3CB; PPP2R1A; |
| | PIK3C3; MAPK8; MAPK3; ITGA1; ETS1; KRAS; MYCN; |
| | EIF4EBP1; PPARG; PRKCD; PRKAA1; MAPK9; SRC; |
| | CDK2; PPP2CA; PIM1; PIK3C2A; ITGB7; YWHAZ; |
| | PPP1CC; KSR1; PXN; RAF1; FYN; DYRK1A; ITGB1; |
| | MAP2K2; PAK4; PIK3R1; STAT3; PPP2R5C; MAP2K1; |
| | PAK3; ITGB3; ESR1; ITGA2; MYC; TTK; CSNK1A1; |
| | CRKL; BRAF; ATF4; PRKCA; SRF; STAT1; SGK |
| Glucocorticoid Receptor Signaling | RAC1; TAF4B; EP300; SMAD2; TRAF6; PCAF; ELK1; |
| | MAPK1; SMAD3; AKT2; IKBKB; NCOR2; UBE2I; |
| | PIK3CA; CREB1; FOS; HSPA5; NFKB2; BCL2; |
| | MAP3K14; STAT5B; PIK3CB; PIK3C3; MAPK8; BCL2L1; |
| | MAPK3; TSC22D3; MAPK10; NRIP1; KRAS; MAPK13; |
| | RELA; STAT5A; MAPK9; NOS2A; PBX1; NR3C1; |
| | PIK3C2A; CDKN1C; TRAF2; SERPINE1; NCOA3; |
| | MAPK14; TNF; RAF1; IKBKG; MAP3K7; CREBBP; |
| | CDKN1A; MAP2K2; JAK1; IL8; NCOA2; AKT1; JAK2; |
| | PIK3R1; CHUK; STAT3; MAP2K1; NFKB1; TGFBR1; |
| | ESR1; SMAD4; CEBPB; JUN; AR; AKT3; CCL2; MMP1; |
| | STAT1; IL6; HSP90AA1 |
| Axonal Guidance Signaling | PRKCE; ITGAM; ROCK1; ITGA5; CXCR4; ADAM12; |
| | IGF1; RAC1; RAP1A; EIF4E; PRKCZ; NRP1; NTRK2; |
| | ARHGEF7; SMO; ROCK2; MAPK1; PGF; RAC2; |
| | PTPN11; GNAS; AKT2; PIK3CA; ERBB2; PRKCI; PTK2; |
| | CFL1; GNAQ; PIK3CB; CXCL12; PIK3C3; WNT11; |
| | PRKD1; GNB2L1; ABL1; MAPK3; ITGA1; KRAS; RHOA; |
| | PRKCD; PIK3C2A; ITGB7; GLI2; PXN; VASP; RAF1; |
| | FYN; ITGB1; MAP2K2; PAK4; ADAM17; AKT1; PIK3R1; |
| | GLI1; WNT5A; ADAM10; MAP2K1; PAK3; ITGB3; |
| | CDC42; VEGFA; ITGA2; EPHA8; CRKL; RND1; GSK3B; |
| | AKT3; PRKCA |
| Ephrin Receptor Signaling | PRKCE; ITGAM; ROCK1; ITGA5; CXCR4; IRAK1; |
| | PRKAA2; EIF2AK2; RAC1; RAP1A; GRK6; ROCK2; |
| | MAPK1; PGF; RAC2; PTPN11; GNAS; PLK1; AKT2; |
| | DOK1; CDK8; CREB1; PTK2; CFL1; GNAQ; MAP3K14; |

(continued)

| CELLULAR FUNCTION | GENES |
|---|---|
| | CXCL12; MAPK8; GNB2L1; ABL1; MAPK3; ITGA1; |
| | KRAS; RHOA; PRKCD; PRKAA1; MAPK9; SRC; CDK2; |
| | PIM1; ITGB7; PXN; RAF1; FYN; DYRK1A; ITGB1; |
| | MAP2K2; PAK4; AKT1; JAK2; STAT3; ADAM10; |
| | MAP2K1; PAK3; ITGB3; CDC42; VEGFA; ITGA2; |
| | EPHA8; TTK; CSNK1A1; CRKL; BRAF; PTPN13; ATF4; |
| | AKT3; SGK |
| Actin Cytoskeleton Signaling | ACTN4; PRKCE; ITGAM; ROCK1; ITGA5; IRAK1; |
| | PRKAA2; EIF2AK2; RAC1; INS; ARHGEF7; GRK6; |
| | ROCK2; MAPK1; RAC2; PLK1; AKT2; PIK3CA; CDK8; |
| | PTK2; CFL1; P1K3CB; MYH9; DIAPH1; PIK3C3; MAPK8; |
| | F2R; MAPK3; SLC9A1; ITGA1; KRAS; RHOA; PRKCD; |
| | PRKAA1; MAPK9; CDK2; PIM1; PIK3C2A; ITGB7; |
| | PPP1CC; PXN; VIL2; RAF1; GSN; DYRK1A; ITGB1; |
| | MAP2K2; PAK4; PIP5K1A; PIK3R1; MAP2K1; PAK3; |
| | ITGB3; CDC42; APC; ITGA2; TTK; CSNK1A1; CRKL; |
| | BRAF; VAV3; SGK |
| Huntington's Disease Signaling | PRKCE; IGF1; EP300; RCOR1; PRKCZ; HDAC4; TGM2; |
| | MAPK1; CAPNS1; AKT2; EGFR; NCOR2; SP1; CAPN2; |
| | PIK3CA; HDAC5; CREB1; PRKCI; HSPA5; REST; |
| | GNAQ; PIK3CB; PIK3C3; MAPK8; IGF1R; PRKD1; |
| | GNB2L1; BCL2L1; CAPN1; MAPK3; CASP8; HDAC2; |
| | HDAC7A; PRKCD; HDAC11; MAPK9; HDAC9; PIK3C2A; |
| | HDAC3; TP53; CASP9; CREBBP; AKT1; PIK3R1; |
| | PDPK1; CASP1; APAF1; FRAP1; CASP2; JUN; BAX; |
| | ATF4; AKT3; PRKCA; CLTC; SGK; HDAC6; CASP3 |
| Apoptosis Signaling | PRKCE; ROCK1; BID; IRAK1; PRKAA2; EIF2AK2; BAK1; |
| | BIRC4; GRK6; MAPK1; CAPNS1; PLK1; AKT2; IKBKB; |
| | CAPN2; CDK8; FAS; NFKB2; BCL2; MAP3K14; MAPK8; |
| | BCL2L1; CAPN1; MAPK3; CASP8; KRAS; RELA; |
| | PRKCD; PRKAA1; MAPK9; CDK2; PIM1; TP53; TNF; |
| | RAF1; IKBKG; RELB; CASP9; DYRK1A; MAP2K2; |
| | CHUK; APAF1; MAP2K1; NFKB1; PAK3; LMNA; CASP2; |
| | BIRC2; TTK; CSNK1A1; BRAF; BAX; PRKCA; SGK; |
| | CASP3; BIRC3; PARP1 |
| B Cell Receptor Signaling | RAC1; PTEN; LYN; ELK1; MAPK1; RAC2; PTPN11; |
| | AKT2; IKBKB; PIK3CA; CREB1; SYK; NFKB2; CAMK2A; |
| | MAP3K14; PIK3CB; PIK3C3; MAPK8; BCL2L1; ABL1; |

(continued)

| CELLULAR FUNCTION | GENES |
|---|---|
| | MAPK3; ETS1; KRAS; MAPK13; RELA; PTPN6; MAPK9; |
| | EGR1; PIK3C2A; BTK; MAPK14; RAF1; IKBKG; RELB; |
| | MAP3K7; MAP2K2; AKT1; PIK3R1; CHUK; MAP2K1; |
| | NFKB1; CDC42; GSK3A; FRAP1; BCL6; BCL10; JUN; |
| | GSK3B; ATF4; AKT3; VAV3; RPS6KB1 |
| Leukocyte Extravasation Signaling | ACTN4; CD44; PRKCE; ITGAM; ROCK1; CXCR4; CYBA; |
| | RAC1; RAP1A; PRKCZ; ROCK2; RAC2; PTPN11; |
| | MMP14; PIK3CA; PRKCI; PTK2; PIK3CB; CXCL12; |
| | PIK3C3; MAPK8; PRKD1; ABL1; MAPK10; CYBB; |
| | MAPK13; RHOA; PRKCD; MAPK9; SRC; PIK3C2A; BTK; |
| | MAPK14; NOX1; PXN; VIL2; VASP; ITGB1; MAP2K2; |
| | CTNND1; PIK3R1; CTNNB1; CLDN1; CDC42; F11R; ITK; |
| | CRKL; VAV3; CTTN; PRKCA; MMP1; MMP9 |
| Integrin Signaling | ACTN4; ITGAM; ROCK1; ITGA5; RAC1; PTEN; RAP1A; |
| | TLN1; ARHGEF7; MAPK1; RAC2; CAPNS1; AKT2; |
| | CAPN2; PIK3CA; PTK2; PIK3CB; PIK3C3; MAPK8; |
| | CAV1; CAPN1; ABL1; MAPK3; ITGA1; KRAS; RHOA; |
| | SRC; PIK3C2A; ITGB7; PPP1CC; ILK; PXN; VASP; |
| | RAF1; FYN; ITGB1; MAP2K2; PAK4; AKT1; PIK3R1; |
| | TNK2; MAP2K1; PAK3; ITGB3; CDC42; RND3; ITGA2; |
| | CRKL; BRAF; GSK3B; AKT3 |
| Acute Phase Response Signaling | IRAK1; SOD2; MYD88; TRAF6; ELK1; MAPK1; PTPN11; |
| | AKT2; IKBKB; PIK3CA; FOS; NFKB2; MAP3K14; |
| | PIK3CB; MAPK8; RIPK1; MAPK3; IL6ST; KRAS; |
| | MAPK13; IL6R; RELA; SOCS1; MAPK9; FTL; NR3C1; |
| | TRAF2; SERPINE1; MAPK14; TNF; RAF1; PDK1; |
| | IKBKG; RELB; MAP3K7; MAP2K2; AKT1; JAK2; PIK3R1; |
| | CHUK; STAT3; MAP2K1; NFKB1; FRAP1; CEBPB; JUN; |
| | AKT3; IL1R1; IL6 |
| PTEN Signaling | ITGAM; ITGA5; RAC1; PTEN; PRKCZ; BCL2L11; |
| | MAPK1; RAC2; AKT2; EGFR; IKBKB; CBL; PIK3CA; |
| | CDKN1B; PTK2; NFKB2; BCL2; PIK3CB; BCL2L1; |
| | MAPK3; ITGA1; KRAS; ITGB7; ILK; PDGFRB; INSR; |
| | RAF1; IKBKG; CASP9; CDKN1A; ITGB1; MAP2K2; |
| | AKT1; PIK3R1; CHUK; PDGFRA; PDPK1; MAP2K1; |
| | NFKB1; ITGB3; CDC42; CCND1; GSK3A; ITGA2; |
| | GSK3B; AKT3; FOXO1; CASP3; RPS6KB1 |
| p53 Signaling | PTEN; EP300; BBC3; PCAF; FASN; BRCA1; GADD45A; |

(continued)

| CELLULAR FUNCTION | GENES |
|---|---|
| | BIRC5; AKT2; PIK3CA; CHEK1; TP53INP1; BCL2; |
| | PIK3CB; PIK3C3; MAPK8; THBS1; ATR; BCL2L1; E2F1; |
| | PMAIP1; CHEK2; TNFRSF10B; TP73; RB1; HDAC9; |
| | CDK2; PIK3C2A; MAPK14; TP53; LRDD; CDKN1A; |
| | HIPK2; AKT1; PIK3R1; RRM2B; APAF1; CTNNB1; |
| | SIRT1; CCND1; PRKDC; ATM; SFN; CDKN2A; JUN; |
| | SNAI2; GSK3B; BAX; AKT3 |
| Aryl Hydrocarbon Receptor Signaling | HSPB1; EP300; FASN; TGM2; RXRA; MAPK1; NQO1; |
| | NCOR2; SP1; ARNT; CDKN1B; FOS; CHEK1; |
| | SMARCA4; NFKB2; MAPK8; ALDH1A1; ATR; E2F1; |
| | MAPK3; NRIP1; CHEK2; RELA; TP73; GSTP1; RB1; |
| | SRC; CDK2; AHR; NFE2L2; NCOA3; TP53; TNF; |
| | CDKN1A; NCOA2; APAF1; NFKB1; CCND1; ATM; ESR1; |
| | CDKN2A; MYC; JUN; ESR2; BAX; IL6; CYP1B1; |
| | HSP90AA1 1 |
| Xenobiotic Metabolism Signaling | PRKCE; EP300; PRKCZ; RXRA; MAPK1; NQO1; |
| | NCOR2; PIK3CA; ARNT; PRKCI; NFKB2; CAMK2A; |
| | PIK3CB; PPP2R1A; PIK3C3; MAPK8; PRKD1; |
| | ALDH1A1; MAPK3; NRIP1; KRAS; MAPK13; PRKCD; |
| | GSTP1; MAPK9; NOS2A; ABCB1; AHR; PPP2CA; FTL; |
| | NFE2L2; PIK3C2A; PPARGC1A; MAPK14; TNF; RAF1; |
| | CREBBP; MAP2K2; PIK3R1; PPP2R5C; MAP2K1; |
| | NFKB1; KEAP1; PRKCA; EIF2AK3; IL6; CYP1B1; |
| | HSP90AA1 1 |
| SAPK/JNK Signaling | PRKCE; IRAK1; PRKAA2; EIF2AK2; RAC1; ELK1; |
| | GRK6; MAPK1; GADD45A; RAC2; PLK1; AKT2; PIK3CA; |
| | FADD; CDK8; PIK3CB; PIK3C3; MAPK8; RIPK1; |
| | GNB2L1; IRS1; MAPK3; MAPK10; DAXX; KRAS; |
| | PRKCD; PRKAA1; MAPK9; CDK2; PIM1; PIK3C2A; |
| | TRAF2; TP53; LCK; MAP3K7; DYRK1A; MAP2K2; |
| | PIK3R1; MAP2K1; PAK3; CDC42; JUN; TTK; CSNK1A1; |
| | CRKL; BRAF; SGK |
| PPAr/RXR Signaling | PRKAA2; EP300; INS; SMAD2; TRAF6; PPARA; FASN; |
| | RXRA; MAPK1; SMAD3; GNAS; IKBKB; NCOR2; |
| | ABCA1; GNAQ; NFKB2; MAP3K14; STAT5B; MAPK8; |
| | IRS1; MAPK3; KRAS; RELA; PRKAA1; PPARGC1A; |
| | NCOA3; MAPK14; INSR; RAF1; IKBKG; RELB; MAP3K7; |
| | CREBBP; MAP2K2; JAK2; CHUK; MAP2K1; NFKB1; |

(continued)

| CELLULAR FUNCTION | GENES |
|---|---|
| | TGFBR1; SMAD4; JUN; IL1R1; PRKCA; IL6; HSP90AA1; |
| | ADIPOQ |
| NF-KB Signaling | IRAK1; EIF2AK2; EP300; INS; MYD88; PRKCZ; TRAF6; |
| | TBK1; AKT2; EGFR; IKBKB; PIK3CA; BTRC; NFKB2; |
| | MAP3K14; PIK3CB; PIK3C3; MAPK8; RIPK1; HDAC2; |
| | KRAS; RELA; PIK3C2A; TRAF2; TLR4; PDGFRB; TNF; |
| | INSR; LCK; IKBKG; RELB; MAP3K7; CREBBP; AKT1; |
| | PIK3R1; CHUK; PDGFRA; NFKB1; TLR2; BCL10; |
| | GSK3B; AKT3; TNFAIP3; IL1R1 |
| Neuregulin Signaling | ERBB4; PRKCE; ITGAM; ITGA5; PTEN; PRKCZ; ELK1; |
| | MAPK1; PTPN11; AKT2; EGFR; ERBB2; PRKCI; |
| | CDKN1B; STAT5B; PRKD1; MAPK3; ITGA1; KRAS; |
| | PRKCD; STAT5A; SRC; ITGB7; RAF1; ITGB1; MAP2K2; |
| | ADAM17; AKT1; PIK3R1; PDPK1; MAP2K1; ITGB3; |
| | EREG; FRAP1; PSEN1; ITGA2; MYC; NRG1; CRKL; |
| | AKT3; PRKCA; HSP90AA1; RPS6KB1 |
| Wnt & Beta catenin | CD44; EP300; LRP6; DVL3; CSNK1E; GJA1; SMO; |
| Signaling | AKT2; PIN1; CDH1; BTRC; GNAQ; MARK2; PPP2R1A; |
| | WNT11; SRC; DKK1; PPP2CA; SOX6; SFRP2; ILK; |
| | LEF1; SOX9; TP53; MAP3K7; CREBBP; TCF7L2; AKT1; |
| | PPP2R5C; WNT5A; LRP5; CTNNB1; TGFBR1; CCND1; |
| | GSK3A; DVL1; APC; CDKN2A; MYC; CSNK1A1; GSK3B; |
| | AKT3; SOX2 |
| Insulin Receptor Signaling | PTEN; INS; EIF4E; PTPN1; PRKCZ; MAPK1; TSC1; |
| | PTPN11; AKT2; CBL; PIK3CA; PRKCI; PIK3CB; PIK3C3; |
| | MAPK8; IRS1; MAPK3; TSC2; KRAS; EIF4EBP1; |
| | SLC2A4; PIK3C2A; PPP1CC; INSR; RAF1; FYN; |
| | MAP2K2; JAK1; AKT1; JAK2; PIK3R1; PDPK1; MAP2K1; |
| | GSK3A; FRAP1; CRKL; GSK3B; AKT3; FOXO1; SGK; |
| | RPS6KB1 |
| IL-6 Signaling | HSPB1; TRAF6; MAPKAPK2; ELK1; MAPK1; PTPN11; |
| | IKBKB; FOS; NFKB2; MAP3K14; MAPK8; MAPK3; |
| | MAPK10; IL6ST; KRAS; MAPK13; IL6R; RELA; SOCS1; |
| | MAPK9; ABCB1; TRAF2; MAPK14; TNF; RAF1; IKBKG; |
| | RELB; MAP3K7; MAP2K2; IL8; JAK2; CHUK; STAT3; |
| | MAP2K1; NFKB1; CEBPB; JUN; IL1R1; SRF; IL6 |
| Hepatic Cholestasis | PRKCE; IRAK1; INS; MYD88; PRKCZ; TRAF6; PPARA; |
| | RXRA; IKBKB; PRKCI; NFKB2; MAP3K14; MAPK8; |

(continued)

| CELLULAR FUNCTION | GENES |
|---|---|
| | PRKD1; MAPK10; RELA; PRKCD; MAPK9; ABCB1; |
| | TRAF2; TLR4; TNF; INSR; IKBKG; RELB; MAP3K7; IL8; |
| | CHUK; NR1H2; TJP2; NFKB1; ESR1; SREBF1; FGFR4; |
| | JUN; IL1R1; PRKCA; IL6 |
| IGF-1 Signaling | IGF1; PRKCZ; ELK1; MAPK1; PTPN11; NEDD4; AKT2; |
| | PIK3CA; PRKCI; PTK2; FOS; PIK3CB; PIK3C3; MAPK8; |
| | IGF1R; IRS1; MAPK3; IGFBP7; KRAS; PIK3C2A; |
| | YWHAZ; PXN; RAF1; CASP9; MAP2K2; AKT1; PIK3R1; |
| | PDPK1; MAP2K1; IGFBP2; SFN; JUN; CYR61; AKT3; |
| | FOXO1; SRF; CTGF; RPS6KB1 |
| NRF2-mediated Oxidative | PRKCE; EP300; SOD2; PRKCZ; MAPK1; SQSTM1; |
| Stress Response | NQO1; PIK3CA; PRKCI; FOS; PIK3CB; PIK3C3; MAPK8; |
| | PRKD1; MAPK3; KRAS; PRKCD; GSTP1; MAPK9; FTL; |
| | NFE2L2; PIK3C2A; MAPK14; RAF1; MAP3K7; CREBBP; |
| | MAP2K2; AKT1; PIK3R1; MAP2K1; PPIB; JUN; KEAP1; |
| | GSK3B; ATF4; PRKCA; EIF2AK3; HSP90AA1 |
| Hepatic Fibrosis/Hepatic | EDN1; IGF1; KDR; FLT1; SMAD2; FGFR1; MET; PGF; |
| Stellate Cell Activation | SMAD3; EGFR; FAS; CSF1; NFKB2; BCL2; MYH9; |
| | IGF1R; IL6R; RELA; TLR4; PDGFRB; TNF; RELB; IL8; |
| | PDGFRA; NFKB1; TGFBR1; SMAD4; VEGFA; BAX; |
| | IL1R1; CCL2; HGF; MMP1; STAT1; IL6; CTGF; MMP9 |
| PPAR Signaling | EP300; INS; TRAF6; PPARA; RXRA; MAPK1; IKBKB; |
| | NCOR2; FOS; NFKB2; MAP3K14; STAT5B; MAPK3; |
| | NRIP1; KRAS; PPARG; RELA; STAT5A; TRAF2; |
| | PPARGC1A; PDGFRB; TNF; INSR; RAF1; IKBKG; |
| | RELB; MAP3K7; CREBBP; MAP2K2; CHUK; PDGFRA; |
| | MAP2K1; NFKB1; JUN; IL1R1; HSP90AA1 |
| Fc Epsilon RI Signaling | PRKCE; RAC1; PRKCZ; LYN; MAPK1; RAC2; PTPN11; |
| | AKT2; PIK3CA; SYK; PRKCI; PIK3CB; PIK3C3; MAPK8; |
| | PRKD1; MAPK3; MAPK10; KRAS; MAPK13; PRKCD; |
| | MAPK9; PIK3C2A; BTK; MAPK14; TNF; RAF1; FYN; |
| | MAP2K2; AKT1; PIK3R1; PDPK1; MAP2K1; AKT3; |
| | VAV3; PRKCA |
| G-Protein Coupled | PRKCE; RAP1A; RGS16; MAPK1; GNAS; AKT2; IKBKB; |
| Receptor Signaling | PIK3CA; CREB1; GNAQ; NFKB2; CAMK2A; PIK3CB; |
| | PIK3C3; MAPK3; KRAS; RELA; SRC; PIK3C2A; RAF1; |
| | IKBKG; RELB; FYN; MAP2K2; AKT1; PIK3R1; CHUK; |
| | PDPK1; STAT3; MAP2K1; NFKB1; BRAF; ATF4; AKT3; |

(continued)

| CELLULAR FUNCTION | GENES |
|---|---|
| | PRKCA |
| Inositol Phosphate | PRKCE; IRAK1; PRKAA2; EIF2AK2; PTEN; GRK6; |
| Metabolism | MAPK1; PLK1; AKT2; PIK3CA; CDK8; PIK3CB; PIK3C3; |
| | MAPK8; MAPK3; PRKCD; PRKAA1; MAPK9; CDK2; |
| | PIM1; PIK3C2A; DYRK1A; MAP2K2; PIP5K1A; PIK3R1; |
| | MAP2K1; PAK3; ATM; TTK; CSNK1A1; BRAF; SGK |
| PDGF Signaling | EIF2AK2; ELK1; ABL2; MAPK1; PIK3CA; FOS; PIK3CB; |
| | PIK3C3; MAPK8; CAV1; ABL1; MAPK3; KRAS; SRC; |
| | PIK3C2A; PDGFRB; RAF1; MAP2K2; JAK1; JAK2; |
| | PIK3R1; PDGFRA; STAT3; SPHK1; MAP2K1; MYC; |
| | JUN; CRKL; PRKCA; SRF; STAT1; SPHK2 |
| VEGF Signaling | ACTN4; ROCK1; KDR; FLT1; ROCK2; MAPK1; PGF; |
| | AKT2; PIK3CA; ARNT; PTK2; BCL2; PIK3CB; PIK3C3; |
| | BCL2L1; MAPK3; KRAS; HIF1A; NOS3; PIK3C2A; PXN; |
| | RAF1; MAP2K2; ELAVL1; AKT1; PIK3R1; MAP2K1; SFN; |
| | VEGFA; AKT3; FOXO1; PRKCA |
| Natural Killer Cell Signaling | PRKCE; RAC1; PRKCZ; MAPK1; RAC2; PTPN11; |
| | KIR2DL3; AKT2; PIK3CA; SYK; PRKCI; P1K3CB; |
| | PIK3C3; PRKD1; MAPK3; KRAS; PRKCD; PTPN6; |
| | PIK3C2A; LCK; RAF1; FYN; MAP2K2; PAK4; AKT1; |
| | PIK3R1; MAP2K1; PAK3; AKT3; VAV3; PRKCA |
| Cell Cycle: G1/S | HDAC4; SMAD3; SUV39H1; HDAC5; CDKN1B; BTRC; |
| Checkpoint Regulation | ATR; ABL1; E2F1; HDAC2; HDAC7A; RB1; HDAC11; |
| | HDAC9; CDK2; E2F2; HDAC3; TP53; CDKN1A; CCND1; |
| | E2F4; ATM; RBL2; SMAD4; CDKN2A; MYC; NRG1; |
| | GSK3B; RBL1; HDAC6 |
| T Cell Receptor Signaling | RAC1; ELK1; MAPK1; IKBKB; CBL; PIK3CA; FOS; |
| | NFKB2; PIK3CB; PIK3C3; MAPK8; MAPK3; KRAS; |
| | RELA; PIK3C2A; BTK; LCK; RAF1; IKBKG; RELB; FYN; |
| | MAP2K2; PIK3R1; CHUK; MAP2K1; NFKB1; ITK; BCL10; |
| | JUN; VAV3 |
| Death Receptor Signaling | CRADD; HSPB1; BID; BIRC4; TBK1; IKBKB; FADD; |
| | FAS; NFKB2; BCL2; MAP3K14; MAPK8; RIPK1; CASP8; |
| | DAXX; TNFRSF10B; RELA; TRAF2; TNF; IKBKG; RELB; |
| | CASP9; CHUK; APAF1; NFKB1; CASP2; BIRC2; CASP3; |
| | BIRC3 |
| FGF Signaling | RAC1; FGFR1; MET; MAPKAPK2; MAPK1; PTPN11; |
| | AKT2; PIK3CA; CREB1; PIK3CB; PIK3C3; MAPK8; |

(continued)

| CELLULAR FUNCTION | GENES |
|---|---|
| | MAPK3; MAPK13; PTPN6; PIK3C2A; MAPK14; RAF1; |
| | AKT1; PIK3R1; STAT3; MAP2K1; FGFR4; CRKL; ATF4; |
| | AKT3; PRKCA; HGF |
| GM-CSF Signaling | LYN; ELK1; MAPK1; PTPN11; AKT2; PIK3CA; CAMK2A; |
| | STAT5B; PIK3CB; PIK3C3; GNB2L1; BCL2L1; MAPK3; |
| | ETS1; KRAS; RUNX1; PIM1; PIK3C2A; RAF1; MAP2K2; |
| | AKT1; JAK2; PIK3R1; STAT3; MAP2K1; CCND1; AKT3; |
| | STAT1 |
| Amyotrophic Lateral | BID; IGF1; RAC1; BIRC4; PGF; CAPNS1; CAPN2; |
| Sclerosis Signaling | PIK3CA; BCL2; PIK3CB; PIK3C3; BCL2L1; CAPN1; |
| | PIK3C2A; TP53; CASP9; PIK3R1; RAB5A; CASP1; |
| | APAF1; VEGFA; BIRC2; BAX; AKT3; CASP3; BIRC3 |
| JAK/Stat Signaling | PTPN1; MAPK1; PTPN11; AKT2; PIK3CA; STAT5B; |
| | P1K3CB; PIK3C3; MAPK3; KRAS; SOCS1; STAT5A; |
| | PTPN6; PIK3C2A; RAF1; CDKN1A; MAP2K2; JAK1; |
| | AKT1; JAK2; PIK3R1; STAT3; MAP2K1; FRAP1; AKT3; |
| | STAT1 |
| Nicotinate and Nicotinamide | PRKCE; IRAKI; PRKAA2; EIF2AK2; GRK6; MAPK1; |
| Metabolism | PLK1; AKT2; CDK8; MAPK8; MAPK3; PRKCD; PRKAA1; |
| | PBEF1; MAPK9; CDK2; PIM1; DYRK1A; MAP2K2; |
| | MAP2K1; PAK3; NT5E; TTK; CSNK1A1; BRAF; SGK |
| Chemokine Signaling | CXCR4; ROCK2; MAPK1; PTK2; FOS; CFL1; GNAQ; |
| | CAMK2A; CXCL12; MAPK8; MAPK3; KRAS; MAPK13; |
| | RHOA; CCR3; SRC; PPP1CC; MAPK14; NOX1; RAF1; |
| | MAP2K2; MAP2K1; JUN; CCL2; PRKCA |
| IL-2 Signaling | ELK1; MAPK1; PTPN11; AKT2; PIK3CA; SYK; FOS; |
| | STAT5B; PIK3CB; PIK3C3; MAPK8; MAPK3; KRAS; |
| | SOCS1; STAT5A; PIK3C2A; LCK; RAF1; MAP2K2; |
| | JAK1; AKT1; PIK3R1; MAP2K1; JUN; AKT3 |
| Synaptic Long Term | PRKCE; IGF1; PRKCZ; PRDX6; LYN; MAPK1; GNAS; |
| Depression | PRKCI; GNAQ; PPP2R1A; IGF1R; PRKD1; MAPK3; |
| | KRAS; GRN; PRKCD; NOS3; NOS2A; PPP2CA; |
| | YWHAZ; RAF1; MAP2K2; PPP2R5C; MAP2K1; PRKCA |
| Estrogen Receptor | TAF4B; EP300; CARM1; PCAF; MAPK1; NCOR2; |
| Signaling | SMARCA4; MAPK3; NRIP1; KRAS; SRC; NR3C1; |
| | HDAC3; PPARGC1A; RBM9; NCOA3; RAF1; CREBBP; |
| | MAP2K2; NCOA2; MAP2K1; PRKDC; ESR1; ESR2 |
| Protein Ubiquitination | TRAF6; SMURF1; BIRC4; BRCA1; UCHL1; NEDD4; |

(continued)

| CELLULAR FUNCTION | GENES |
|---|---|
| Pathway | CBL; UBE2I; BTRC; HSPA5; USP7; USP10; FBXW7; |
| | USP9X; STUB1; USP22; B2M; BIRC2; PARK2; USP8; |
| | USP1; VHL; HSP90AA1; BIRC3 |
| IL-10 Signaling | TRAF6; CCR1; ELK1; IKBKB; SP1; FOS; NFKB2; |
| | MAP3K14; MAPK8; MAPK13; RELA; MAPK14; TNF; |
| | IKBKG; RELB; MAP3K7; JAK1; CHUK; STAT3; NFKB1; |
| | JUN; IL1R1; IL6 |
| VDR/RXR Activation | PRKCE; EP300; PRKCZ; RXRA; GADD45A; HES1; |
| | NCOR2; SP1; PRKCI; CDKN1B; PRKD1; PRKCD; |
| | RUNX2; KLF4; YY1; NCOA3; CDKN1A; NCOA2; SPP1; |
| | LRP5; CEBPB; FOXO1; PRKCA |
| TGF-beta Signaling | EP300; SMAD2; SMURF1; MAPK1; SMAD3; SMAD1; |
| | FOS; MAPK8; MAPK3; KRAS; MAPK9; RUNX2; |
| | SERPINE1; RAF1; MAP3K7; CREBBP; MAP2K2; |
| | MAP2K1; TGFBR1; SMAD4; JUN; SMAD5 |
| Toll-like Receptor Signaling | IRAK1; EIF2AK2; MYD88; TRAF6; PPARA; ELK1; |
| | IKBKB; FOS; NFKB2; MAP3K14; MAPK8; MAPK13; |
| | RELA; TLR4; MAPK14; IKBKG; RELB; MAP3K7; CHUK; |
| | NFKB1; TLR2; JUN |
| p38 MAPK Signaling | HSPB1; IRAK1; TRAF6; MAPKAPK2; ELK1; FADD; FAS; |
| | CREB1; DDIT3; RPS6KA4; DAXX; MAPK13; TRAF2; |
| | MAPK14; TNF; MAP3K7; TGFBR1; MYC; ATF4; IL1R1; |
| | SRF; STAT1 |
| Neurotrophin/TRK Signaling | NTRK2; MAPK1; PTPN11; P1K3CA; CREB1; FOS; |
| | PIK3CB; PIK3C3; MAPK8; MAPK3; KRAS; PIK3C2A; |
| | RAF1; MAP2K2; AKT1; PIK3R1; PDPK1; MAP2K1; |
| | CDC42; JUN; ATF4 |
| FXR/RXR Activation | INS; PPARA; FASN; RXRA; AKT2; SDC1; MAPK8; |
| | APOB; MAPK10; PPARG; MTTP; MAPK9; PPARGC1A; |
| | TNF; CREBBP; AKT1; SREBF1; FGFR4; AKT3; FOXO1 |
| Synaptic Long Term | PRKCE; RAP1A; EP300; PRKCZ; MAPK1; CREB1; |
| Potentiation | PRKCI; GNAQ; CAMK2A; PRKD1; MAPK3; KRAS; |
| | PRKCD; PPP1CC; RAF1; CREBBP; MAP2K2; MAP2K1; |
| | ATF4; PRKCA |
| Calcium Signaling | RAP1A; EP300; HDAC4; MAPK1; HDAC5; CREB1; |
| | CAMK2A; MYH9; MAPK3; HDAC2; HDAC7A; HDAC11; |
| | HDAC9; HDAC3; CREBBP; CALR; CAMKK2; ATF4; |
| | HDAC6 |

(continued)

| CELLULAR FUNCTION | GENES |
|---|---|
| EGF Signaling | ELK1; MAPK1; EGFR; PIK3CA; FOS; PIK3CB; P1K3C3; |
| | MAPK8; MAPK3; PIK3C2A; RAF1; JAK1; PIK3R1; |
| | STAT3; MAP2K1; JUN; PRKCA; SRF; STAT1 |
| Hypoxia Signaling in the | EDN1; PTEN; EP300; NQO1; UBE2I; CREB1; ARNT; |
| Cardiovascular System | HIF1A; SLC2A4; NOS3; TP53; LDHA; AKT1; ATM; |
| | VEGFA; JUN; ATF4; VHL; HSP90AA1 |
| LPS/IL-1 Mediated Inhibition | IRAK1; MYD88; TRAF6; PPARA; RXRA; ABCA1; |
| of RXR Function | MAPK8; ALDH1A1; GSTP1; MAPK9; ABCB1; TRAF2; |
| | TLR4; TNF; MAP3K7; NR1H2; SREBF1; JUN; IL1R1 |
| LXR/RXR Activation | FASN; RXRA; NCOR2; ABCA1; NFKB2; IRF3; RELA; |
| | NOS2A; TLR4; TNF; RELB; LDLR; NR1H2; NFKB1; |
| | SREBF1; IL1R1; CCL2; IL6; MMP9 |
| Amyloid Processing | PRKCE; CSNK1E; MAPK1; CAPNS1; AKT2; CAPN2; |
| | CAPN1; MAPK3; MAPK13; MAPT; MAPK14; AKT1; |
| | PSEN1; CSNK1A1; GSK3B; AKT3; APP |
| IL-4 Signaling | AKT2; PIK3CA; PIK3CB; PIK3C3; IRS1; KRAS; SOCS1; |
| | PTPN6; NR3C1; PIK3C2A; JAK1; AKT1; JAK2; PIK3R1; |
| | FRAP1; AKT3; RPS6KB1 |
| Cell Cycle: G2/M DNA | EP300; PCAF; BRCA1; GADD45A; PLK1; BTRC; |
| Damage Checkpoint | CHEK1; ATR; CHEK2; YWHAZ; TP53; CDKN1A; |
| Regulation | PRKDC; ATM; SFN; CDKN2A |
| Nitric Oxide Signaling in the | KDR; FLT1; PGF; AKT2; PIK3CA; PIK3CB; PIK3C3; |
| Cardiovascular System | CAV1; PRKCD; NOS3; PIK3C2A; AKT1; PIK3R1; |
| | VEGFA; AKT3; HSP90AA1 |
| Purine Metabolism | NME2; SMARCA4; MYH9; RRM2; ADAR; EIF2AK4; |
| | PKM2; ENTPD1; RAD51; RRM2B; TJP2; RAD51C; |
| | NT5E; POLD1; NME1 |
| cAMP-mediated Signaling | RAP1A; MAPK1; GNAS; CREB1; CAMK2A; MAPK3; |
| | SRC; RAF1; MAP2K2; STAT3; MAP2K1; BRAF; ATF4 |
| Mitochondrial Dysfunction | SOD2; MAPK8; CASP8; MAPK10; MAPK9; CASP9; |
| | PARK7; PSEN1; PARK2; APP; CASP3 |
| Notch Signaling | HES1; JAG1; NUMB; NOTCH4; ADAM1 7; NOTCH2; |
| | PSEN1; NOTCH3; NOTCH1; DLL4 |
| Endoplasmic Reticulum | HSPA5; MAPK8; XBP1; TRAF2; ATF6; CASP9; ATF4; |
| Stress Pathway | EIF2AK3; CASP3 |
| Pyrimidine Metabolism | NME2; AICDA; RRM2; EIF2AK4; ENTPD1; RRM2B; |
| NT5E; | NT5E; POLD1; NME1 |
| Parkinson's Signaling | UCHL1; MAPK8; MAPK13; MAPK14; CASP9; PARK7; |

(continued)

| CELLULAR FUNCTION | GENES |
|---|---|
|  | PARK2; CASP3 |
| Cardiac & Beta Adrenergic | GNAS; GNAQ; PPP2R1A; GNB2L1; PPP2CA; PPP1CC; |
| Signaling | PPP2R5C |
| Glycolysis/Gluconeogene sis | HK2; GCK; GPI; ALDH1A1; PKM2; LDHA; HK1 |
| Interferon Signaling | IRF1; SOCS1; JAK1; JAK2; IFITM1; STAT1; IFIT3 |
| Sonic Hedgehog Signaling | ARRB2; SMO; GLI2; DYRK1A; GLI1; GSK3B; DYRK1B |
| Glycerophospholipid | PLD1; GRN; GPAM; YWHAZ; SPHK1; SPHK2 |
| Metabolism |  |
| Phospholipid Degradation | PRDX6; PLD1; GRN; YWHAZ; SPHK1; SPHK2 |
| Tryptophan Metabolism | SIAH2; PRMT5; NEDD4; ALDH1A1; CYP1B1; SIAH1 |
| Lysine Degradation | SUV39H1; EHMT2; NSD1; SETD7; PPP2R5C |
| Nucleotide Excision Repair | ERCC5; ERCC4; XPA; XPC; ERCC1 |
| Pathway |  |
| Starch and Sucrose | UCHL1; HK2; GCK; GPI; HK1 |
| Metabolism |  |
| Aminosugars Metabolism | NQO1; HK2; GCK; HK1 |
| Arachidonic Acid | PRDX6; GRN; YWHAZ; CYP1B1 |
| Metabolism |  |
| Circadian Rhythm Signaling | CSNK1E; CREB1; ATF4; NR1D1 |
| Coagulation System | BDKRB1; F2R; SERPINE1; F3 |
| Dopamine Receptor | PPP2R1A; PPP2CA; PPP1CC; PPP2R5C |
| Signaling |  |
| Glutathione Metabolism | IDH2; GSTP1; ANPEP; IDH1 |
| Glycerolipid Metabolism | ALDH1A1; GPAM; SPHK1; SPHK2 |
| Linoleic Acid Metabolism | PRDX6; GRN; YWHAZ; CYP1B1 |
| Methionine Metabolism | DNMT1; DNMT3B; AHCY; DNMT3A |
| Pyruvate Metabolism | GLO1; ALDH1A1; PKM2; LDHA |
| Arginine and Proline | ALDH1A1; NOS3; NOS2A |
| Metabolism |  |
| Eicosanoid Signaling | PRDX6; GRN; YWHAZ |
| Fructose and Mannose | HK2; GCK; HK1 |
| Metabolism |  |
| Galactose Metabolism | HK2; GCK; HK1 |
| Stilbene, Coumarine and | PRDX6; PRDX1; TYR |
| Lignin Biosynthesis |  |
| Antigen Presentation | CALR; B2M |
| Pathway |  |
| Biosynthesis of Steroids | NQO1; DHCR7 |

(continued)

| CELLULAR FUNCTION | GENES |
|---|---|
| Butanoate Metabolism | ALDH1A1; NLGN1 |
| Citrate Cycle | IDH2; IDH1 |
| Fatty Acid Metabolism | ALDH1A1; CYP1B1 |
| Glycerophospholipid Metabolism | PRDX6; CHKA |
| Histidine Metabolism | PRMT5; ALDH1A1 |
| Inositol Metabolism | ERO1L; APEX1 |
| Metabolism of Xenobiotics by Cytochrome p450 | GSTP1; CYP1B1 |
| Methane Metabolism | PRDX6; PRDX1 |
| Phenylalanine Metabolism | PRDX6; PRDX1 |
| Propanoate Metabolism | ALDH1A1; LDHA |
| Selenoamino Acid Metabolism | PRMT5; AHCY |
| Sphingolipid Metabolism | SPHK1; SPHK2 |
| Aminophosphonate Metabolism | PRMT5 |
| Androgen and Estrogen Metabolism | PRMT5 |
| Ascorbate and Aldarate Metabolism | ALDH1A1 |
| Bile Acid Biosynthesis | ALDH1A1 |
| Cysteine Metabolism | LDHA |
| Fatty Acid Biosynthesis | FASN |
| Glutamate Receptor Signaling | GNB2L1 |
| NRF2-mediated Oxidative Stress Response | PRDX1 |
| Pentose Phosphate Pathway | GPI |
| Pentose and Glucuronate Interconversions | UCHL1 |
| Retinol Metabolism | ALDH1A1 |
| Riboflavin Metabolism | TYR |
| Tyrosine Metabolism | PRMT5, TYR |
| Ubiquinone Biosynthesis | PRMT5 |
| Valine, Leucine and Isoleucine Degradation | ALDH1A1 |

(continued)

| CELLULAR FUNCTION | GENES |
|---|---|
| Glycine, Serine and Threonine Metabolism | CHKA |
| Lysine Degradation | ALDH1A1 |
| Pain/Taste | TRPM5; TRPA1 |
| Pain | TRPM7; TRPC5; TRPC6; TRPC1; Cnr1; cnr2; Grk2; |
| | Trpa1; Pomc; Cgrp; Crf; Pka; Era; Nr2b; TRPM5; Prkaca; |
| | Prkacb; Prkar1a; Prkar2a |
| Mitochondrial Function | AIF; CytC; SMAC (Diablo); Aifm-1; Aifm-2 |
| Developmental Neurology | BMP-4; Chordin (Chrd); Noggin (Nog); WNT (Wnt2; |
| | Wnt2b; Wnt3a; Wnt4; Wnt5a; Wnt6; Wnt7b; Wnt8b; |
| | Wnt9a; Wnt9b; Wnt10a; Wnt10b; Wnt16); beta-catenin; |
| | Dkk-1; Frizzled related proteins; Otx-2; Gbx2; FGF-8; |
| | Reelin: Dab1: unc-86 (Pou4f1 or Brn3a): Numb: Reln |

Embodiments also relate to methods and compositions related to knocking out genes, amplifying genes and repairing particular mutations associated with DNA repeat instability and neurological disorders (Robert D. Wells, Tetsuo Ashizawa, Genetic Instabilities and Neurological Diseases, Second Edition, Academic Press, Oct 13, 2011 - Medical). Specific aspects of tandem repeat sequences have been found to be responsible for more than twenty human diseases (New insights into repeat instability: role of RNA•DNA hybrids. McIvor EI, Polak U, Napierala M. RNA Biol. 2010 Sep-Oct;7(5):551-8). The CRISPR-Cas system may be harnessed to correct these defects of genomic instability.

A further aspect relates to utilizing the CRISPR-Cas system for correcting defects in the EMP2A and EMP2B genes that have been identified to be associated with Lafora disease. Lafora disease is an autosomal recessive condition which is characterized by progressive myoclonus epilepsy which may start as epileptic seizures in adolescence. A few cases of the disease may be caused by mutations in genes yet to be identified. The disease causes seizures, muscle spasms, difficulty walking, dementia, and eventually death. There is currently no therapy that has proven effective against disease progression. Other genetic abnormalities associated with epilepsy may also be targeted by the CRISPR-Cas system and the underlying genetics is further described in Genetics of Epilepsy and Genetic Epilepsies, edited by Giuliano Avanzini, Jeffrey L. Noebels, Mariani Foundation Paediatric Neurology:20; 2009).

In yet another aspect the CRISPR-Cas system may be used to correct ocular defects that arise from several genetic mutations further described in Genetic Diseases of the Eye, Second Edition, edited by Elias I. Traboulsi, Oxford University Press, 2012.

Several further aspects relate to correcting defects associated with a wide range of genetic diseases which are further described on the website of the National Institutes of Health under the topic subsection Genetic Disorders (website at health.nih.gov/topic/GeneticDisorders). The genetic brain diseases may include but are not limited to Adrenoleukodystrophy, Agenesis of the Corpus Callosum, Aicardi Syndrome, Alpers' Disease, Alzheimer's Disease, Barth Syndrome, Batten Disease, CADASIL, Cerebellar Degeneration, Fabry's Disease, Gerstmann-Straussler-Scheinker Disease, Huntington's Disease and other Triplet Repeat Disorders, Leigh's Disease, Lesch-Nyhan Syndrome, Menkes Disease, Mitochondrial Myopathies and NINDS Colpocephaly. These diseases are further described on the website of the National Institutes of Health under the subsection Genetic Brain Disorders.

In some embodiments, the condition may be neoplasia. In some embodiments, where the condition is neoplasia, the genes to be targeted are any of those listed in Table A (in this case PTEN and so forth). In some embodiments, the condition may be Age-related Macular Degeneration. In some embodiments, the condition may be a Schizophrenic Disorder. In some embodiments, the condition may be a Trinucleotide Repeat Disorder. In some embodiments, the condition may be Fragile X Syndrome. In some embodiments, the condition may be a Secretase Related Disorder. In some embodiments, the condition may be a Prion - related disorder. In some embodiments, the condition may be ALS. In some embodiments, the condition may be a drug addiction. In some embodiments, the condition may be Autism. In some embodiments, the condition may be Alzheimer's Disease. In some embodiments, the condition may be inflammation. In some embodiments, the condition may be Parkinson's Disease.

Examples of proteins associated with Parkinson's disease include but are not limited to α-synuclein, DJ-1, LRRK2, PINK1, Parkin, UCHL1, Synphilin-1, and NURR1.

Examples of addiction-related proteins may include ABAT for example.

Examples of inflammation-related proteins may include the monocyte chemoattractant protein-1 (MCP1) encoded by the Ccr2 gene, the C-C chemokine receptor type 5 (CCR5) encoded by the Ccr5 gene, the IgG receptor IIB (FCGR2b, also termed CD32) encoded by the Fcgr2b gene, or the Fc epsilon R1g (FCER1g) protein encoded by the Fcer1g gene, for example.

Examples of cardiovascular diseases associated proteins may include IL1B (interleukin 1, beta), XDH (xanthine dehydrogenase), TP53 (tumor protein p53), PTGIS (prostaglandin I2 (prostacyclin) synthase), MB (myoglobin), IL4 (interleukin 4), ANGPT1 (angiopoietin 1), ABCG8 (ATP-binding cassette, sub-family G (WHITE), member 8), or CTSK (cathepsin K), for example.

Examples of Alzheimer's disease associated proteins may include the very low density lipoprotein receptor protein (VLDLR) encoded by the VLDLR gene, the ubiquitin-like modifier activating enzyme 1 (UBA1) encoded by the UBA1 gene, or the NEDD8-activating enzyme E1 catalytic subunit protein (UBE1C) encoded by the UBA3 gene, for example.

Examples of proteins associated Autism Spectrum Disorder may include the benzodiazapine receptor (peripheral) associated protein 1 (BZRAP1) encoded by the BZRAP1 gene, the AF4/FMR2 family member 2 protein (AFF2) encoded by the AFF2 gene (also termed MFR2), the fragile X mental retardation autosomal homolog 1 protein (FXR1) encoded by the FXR1 gene, or the fragile X mental retardation autosomal homolog 2 protein (FXR2) encoded by the FXR2 gene, for example.

Examples of proteins associated Macular Degeneration may include the ATP-binding cassette, sub-family A (ABC1) member 4 protein (ABCA4) encoded by the ABCR gene, the apolipoprotein E protein (APOE) encoded by the APOE gene, or the chemokine (C-C motif) Ligand 2 protein (CCL2) encoded by the CCL2 gene, for example.

Examples of proteins associated Schizophrenia may include NRG1, ErbB4, CPLX1, TPH1, TPH2, NRXN1, GSK3A, BDNF, DISC1, GSK3B, and combinations thereof.

Examples of proteins involved in tumor suppression may include ATM (ataxia telangiectasia mutated), ATR (ataxia telangiectasia and Rad3 related), EGFR (epidermal growth factor receptor), ERBB2 (v-erb-b2 erythroblastic leukemia viral oncogene homolog 2), ERBB3 (v-erb-b2 erythroblastic leukemia viral oncogene homolog 3), ERBB4 (v-erb-b2 erythroblastic leukemia viral oncogene homolog 4), Notch 1, Notch2, Notch 3, or Notch 4, for example.

Examples of proteins associated with a secretase disorder may include PSENEN (presenilin enhancer 2 homolog (C. elegans)), CTSB (cathepsin B), PSEN1 (presenilin 1), APP (amyloid beta (A4) precursor protein), APH1B (anterior pharynx defective 1 homolog B (C. elegans)), PSEN2 (presenilin 2 (Alzheimer disease 4)), or BACE1 (beta-site APP-cleaving enzyme 1), for example.

Examples of proteins associated with Amyotrophic Lateral Sclerosis may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins associated with prion diseases may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins related to neurodegenerative conditions in prion disorders may include A2M (Alpha-2-Macroglobulin), AATF (Apoptosis antagonizing transcription factor), ACPP (Acid phosphatase prostate), ACTA2 (Actin alpha 2 smooth muscle aorta), ADAM22 (ADAM metallopeptidase domain), ADORA3 (Adenosine A3 receptor), or ADRA1D (Alpha-ID adrenergic receptor for Alpha-1D adrenoreceptor), for example.

Examples of proteins associated with Immunodeficiency may include A2M [alpha-2-macroglobulin]; AANAT [arylalkylamine N-acetyltransferase]; ABCA1 [ATP-binding cassette, sub-family A (ABC1), member 1]; ABCA2 [ATP-binding cassette, sub-family A (ABC1), member 2]; or ABCA3 [ATP-binding cassette, sub-family A (ABC1), member 3]; for example.

Examples of proteins associated with Trinucleotide Repeat Disorders include AR (androgen receptor), FMR1 (fragile X mental retardation 1), HTT (huntingtin), or DMPK (dystrophia myotonica-protein kinase), FXN (frataxin), ATXN2 (ataxin 2), for example.

Examples of proteins associated with Neurotransmission Disorders include SST (somatostatin), NOS1 (nitric oxide synthase 1 (neuronal)), ADRA2A (adrenergic, alpha-2A-, receptor), ADRA2C (adrenergic, alpha-2C-, receptor), TACR1 (tachykinin receptor 1), or HTR2c (5-hydroxytryptamine (serotonin) receptor 2C), for example.

Examples of neurodevelopmental-associated sequences include A2BP1 [ataxin 2-binding protein 1], AADAT [aminoadipate aminotransferase], AANAT [arylalkylamine N-acetyltransferase], ABAT [4-aminobutyrate aminotransferase], ABCA1 [ATP-binding cassette, sub-family A (ABC1), member 1], or ABCA13 [ATP-binding cassette, sub-family A (ABC1), member 13], for example.

Further examples of preferred conditions treatable with the present system include may be selected from: Aicardi-Goutières Syndrome; Alexander Disease; Allan-Herndon-Dudley Syndrome; POLG-Related Disorders; Alpha-Mannosidosis (Type II and III); Alström Syndrome; Angelman; Syndrome; Ataxia-Telangiectasia; Neuronal Ceroid-Lipofuscinoses; Beta-Thalassemia; Bilateral Optic Atrophy and (Infantile) Optic Atrophy Type 1; Retinoblastoma (bilateral); Canavan Disease; Cerebrooculofacioskeletal Syndrome 1 [COFS1]; Cerebrotendinous Xanthomatosis; Cornelia de Lange Syndrome; MAPT-Related Disorders; Genetic Prion Diseases; Dravet Syndrome; Early-Onset Familial Alzheimer Disease; Friedreich Ataxia [FRDA]; Fryns Syndrome; Fucosidosis; Fukuyama Congenital Muscular Dystrophy; Galactosialidosis; Gaucher Disease; Organic Acidemias; Hemophagocytic Lymphohistiocytosis; Hutchinson-Gilford Progeria Syndrome; Mucolipidosis II; Infantile Free Sialic Acid Storage Disease; PLA2G6-Associated Neurodegeneration; Jervell and Lange-Nielsen Syndrome; Junctional Epidermolysis Bullosa; Huntington Disease; Krabbe Disease (Infantile); Mitochondrial DNA-Associated Leigh Syndrome and NARP; Lesch-Nyhan Syndrome; LIS1-Associated Lissencephaly; Lowe Syndrome; Maple Syrup Urine Disease; MECP2 Duplication Syndrome; ATP7A-Related Copper Transport Disorders; LAMA2-Related Muscular Dystrophy; Arylsulfatase A Deficiency; Mucopolysaccharidosis Types I, II or III; Peroxisome Biogenesis Disorders, Zellweger Syndrome Spectrum; Neurodegeneration with Brain Iron Accumulation Disorders; Acid Sphingomyelinase Deficiency; Niemann-Pick Disease Type C; Glycine Encephalopathy; ARX-Related Disorders; Urea Cycle Disorders; COL1A1/2-Related Osteogenesis Imperfecta; Mitochondrial DNA Deletion Syndromes; PLP1-Related Disorders; Perry Syndrome; Phelan-McDermid Syndrome; Glycogen Storage Disease Type II (Pompe Disease) (Infantile); MAPT-Related Disorders; MECP2-Related Disorders; Rhizomelic Chondrodysplasia Punctata Type 1; Roberts Syndrome; Sandhoff Disease; Schindler Disease - Type 1; Adenosine Deaminase Deficiency; Smith-Lemli-Opitz Syndrome; Spinal Muscular Atrophy; Infantile-Onset Spinocerebellar Ataxia; Hexosaminidase A Deficiency; Thanatophoric Dysplasia Type 1; Collagen Type VI-Related Disorders; Usher Syndrome Type I; Congenital Muscular Dystrophy; Wolf-Hirschhorn Syndrome; Lysosomal Acid Lipase Deficiency; and Xeroderma Pigmentosum.

As will be apparent, it is envisaged that the present system can be used to target any polynucleotide sequence of interest. Some examples of conditions or diseases that might be usefully treated using the present system are included in the Tables above and examples of genes currently associated with those conditions are also provided there. However, the genes exemplified are not exhaustive.

For example, "wild type StCas9" refers to wild type Cas9 from S thermophilus, the protein sequence of which is given in the SwissProt database under accession number G3ECR1. Similarly, S pyogenes Cas9 is included in SwissProt under accession number Q99ZW2.

The ability to use CRISPR-Cas systems to perform efficient and cost effective gene editing and manipulation will allow the rapid selection and comparison of single and and multiplexed genetic manipulations to transform such genomes for improved production and enhanced traits. In this regard reference is made to US patents and publications: US Patent No. 6,603,061 - Agrobacterium-Mediated Plant Transformation Method; US Patent No. 7,868,149 - Plant Genome Sequences and Uses Thereof and US 2009/0100536 - Transgenic Plants with Enhanced Agronomic Traits. In the practice of the invention, the contents and disclosure of Morrell et al "Crop genomics:advances and applications" Nat Rev Genet. 2011 Dec 29;13(2):85-96 are also referred to.

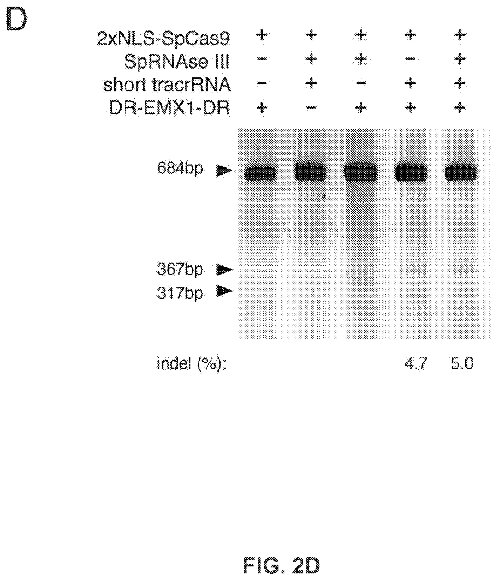

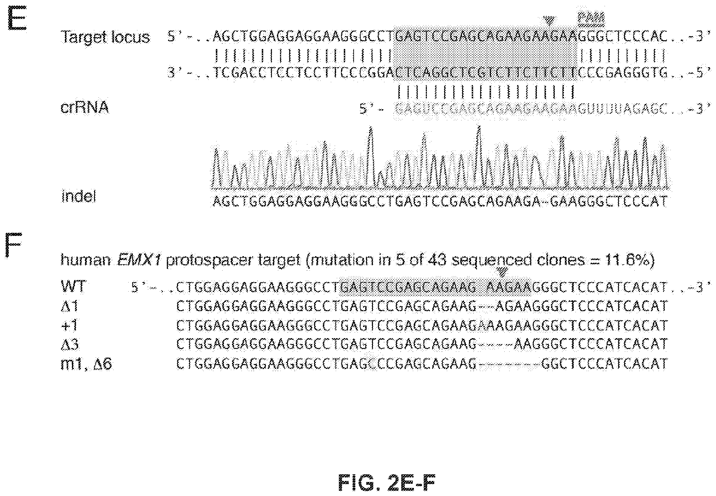

Examples of genes to be targeted include the genes provided in Lawrence et al; Nature 23 Jan. 2014; Vol 505, pages 495-503 as provided in Figure 2, and Supplementary Figures 2, 3, 4, and 5. Examples of genes to be targeted include the genes provided in Ioannidis et al., JNCI, Vol. 102, Issue 12, June 16, 2010 Table 1.

Examples of genes to be targeted include the genes encoding the proteins involved in tumor suppression or any tumor suppressor gene(s), as that term is used herein, including, for example, any gene of the TS GENE TUMOR SUPPRESSOR GENE DATABASE, http://bioinfo.mc.vanderbilt.edu/TSGene/index.html, on the filing day of this application, including 716 human genes (637 coding and 79 non-coding genes), 628 mouse genes, and 567 rat genes, involved in "loss-of-function" or is a tumor suppressor gene consistent with the use of the term in this disclosure, or ATM (ataxia telangiectasia mutated), ATR (ataxia telangiectasia and Rad3 related), EGFR (epidermal growth factor receptor), ERBB2 (v-erb-b2 erythroblastic leukemia viral oncogene homolog 2), ERBB3 (v-erb-b2 erythroblastic leukemia viral oncogene homolog 3), ERBB4 (v-erb-b2 erythroblastic leukemia viral oncogene homolog 4), Notch 1, Notch2, Notch 3, or Notch 4.

Examples of genes to be targeted include any gene of the TS GENE TUMOR SUPPRESSOR GENE DATABASE, http://bioinfo.mc.vanderbilt.edu/TSGene/index.html, on the filing day of this application including 716 human genes (637 coding and 79 non-coding genes), 628 mouse genes, and 567 rat genes, involved in "loss-of-function" or is a tumor suppressor gene consistent with the use of the term in this disclosure, or Tet2, Runx1, Dnmt3a, Nf1, Ezh2, Smc3 genes.

The current disclosure further comprises cells and non-human animals obtainable by the methods which include genomic modifications for expression of CRISPR enzyme for generation of complex disease models, including proliferative conditions including the further administration to said cells / animals three or more sgRNA targeting genes of the invention, including the genes of Tables A, B, and C, as well genes provided in Lawrence et al; Nature 23 Jan. 2014; Vol 505, pages 495-503 as provided in Figure 2, and Supplementary Figures 2, 3, 4, and 5.; genes provided in Ioannidis et al., JNCI, Vol. 102, Issue 12, June 16, 2010, Table 1; gene(s) encoding the protein(s) involved in tumor suppression or any tumor suppressor gene, as that term is herin understood in view of the foregoing discussion of "loss-of-function gene(s)", including, for example, any gene of the TS GENE TUMOR SUPPRESSOR GENE DATABASE, http://bioinfo.mc.vanderbilt.edu/TSGene/index.html, on the filing day of this application including 716 human genes (637 coding and 79 non-coding genes), 628 mouse genes, and 567 rat genes, involved in "loss-of-function" or is a tumor suppressor gene consistent with the use of the term in this disclosure, or ATM (ataxia telangiectasia mutated), ATR (ataxia telangiectasia and Rad3 related), EGFR (epidermal growth factor receptor), ERBB2 (v-erb-b2 erythroblastic leukemia viral oncogene homolog 2), ERBB3 (v-erb-b2 erythroblastic leukemia viral oncogene homolog 3), ERBB4 (v-erb-b2 erythroblastic leukemia viral oncogene homolog 4), Notch 1, Notch2, Notch 3, or Notch 4.

The current disclosure is further defined, inter alia, by the following items:

1) A vector system comprising one or more vectors, wherein said vector system comprises:

Component I comprising a first regulatory element operably linked to a CRISPR-Cas system chimeric RNA (chiRNA) polynucleotide sequence, wherein the polynucleotide sequence comprises:

(a) three or more guide sequences capable of hybridizing to three or more target sequences in the genome of an organism,
(b) a tracr mate sequence, and
(c) a tracr sequence, and

wherein (a) the guide sequences, (b) tracr mate sequence and (c) tracr sequence are arranged in a 5' to 3' orientation; and
Component II comprising a second regulatory element operably linked to an enzyme-coding nucleotide sequence encoding a CRISPR enzyme, wherein the enzyme-coding nucleotide sequence comprises at least one or more nuclear localization sequences (NLSs),

such that when the vector or vectors are transcribed after having been transduced into a population of cells from the organism, the tracr mate sequence hybridizes to the tracr sequence and the guide sequences direct sequence-specific binding of CRISPR enzyme to the target sequences, thereby forming CRISPR complexes;
wherein a CRISPR complex comprises the CRISPR enzyme complexed with (1) the guide sequence that is hybridizableto the target sequence, and (2) the tracr mate sequence that is hybridized to the tracr sequence; and
such that the CRISPR enzyme alters the genome of the transduced cell to obtain a genetically altered population of cells.
2) The vector system of item 1, wherein the population of cells is a population of stem cells.
3) The vector system of item 1 or 2, wherein the population of cells is a population of hematopoietic stem and progenitor cells.
4) The vector system of any one of the above items, wherein the vector system is a lentiviral vector system.
5) The vector system of any one of the above items, wherein the first regulatory element is a U6 promoter.
6) The vector system of any one of the above items, wherein the second regulatory element is an EFS promoter.
7) The vector system of any one of the above items, wherein the polynucleotide sequence comprises at least 4, at least 5, at least 6, at least 7, at least 8, at least 9 or at least 10 guide sequences.
8) The vector system of any one of the above items, wherein the polynucleotide sequence comprises 3, 4, 5, 6, 7, 8, 9 or 10 guide sequences.
9) The vector system of item 8, wherein the polynucleotide sequence comprises 3 guide sequences.
10) The vector system of item 8, wherein the polynucleotide sequence comprises 4 guide sequences.
11) The vector system of item 8, wherein the polynucleotide sequence comprises 5 guide sequences.
12) The vector system of item 8, wherein the polynucleotide sequence comprises 6 guide sequences.
13) The vector system of any one of the above items, wherein the target sequences are associated with tumor suppressor genes.
14) The vector system of any one of the above items, wherein the target sequences are tumor suppressor genes.
15) The vector system of items 1-12, wherein the target sequences are genes selected from the group consisting of Tet2, Runx1, Dnmt3a, Nf1, Ezh2, Smc3 genes.
16) The vector system of items 1-12, wherein the target sequences are genes selected from the group consisting of Tet2, Runx1, Dnmt3a, Nf1, Ezh2, Smc3, NF1, MED12, NF2, CUL3, TADA2B, TADA1, BCR, ABL1 genes, and genes of the following proteins ATM (ataxia telangiectasia mutated), ATR (ataxia telangiectasia and Rad3 related), EGFR (epidermal growth factor receptor), ERBB2 (v-erb-b2 erythroblastic leukemia viral oncogene homolog 2), ERBB3 (v-erb-b2 erythroblastic leukemia viral oncogene homolog 3), ERBB4 (v-erb-b2 erythroblastic leukemia viral oncogene homolog 4), Notch 1, Notch2, Notch 3, and Notch 4.

17) The vector system of items 1-12, wherein the target sequences are genes selected from the group consisting of those genes provided in Tables A, B, and C.

18) The vector system of items 1-12, wherein the target sequences are genes selected from the group consisting of genes of the following proteins ATM (ataxia telangiectasia mutated), ATR (ataxia telangiectasia and Rad3 related), EGFR (epidermal growth factor receptor), ERBB2 (v-erb-b2 erythroblastic leukemia viral oncogene homolog 2), ERBB3 (v-erb-b2 erythroblastic leukemia viral oncogene homolog 3), ERBB4 (v-erb-b2 erythroblastic leukemia viral oncogene homolog 4), Notch 1, Notch2, Notch 3, and Notch 4.

19) The vector system of items 1-12, wherein the target sequences are genes selected from the group consisting of ACO1 (aconitase 1, soluble); ACVR1B (activin A receptor, type IB); ACVR2B (activin A receptor, type IIB); ADNP (activity-dependent neuroprotector homeobox); AJUBA (jub, ajuba homolog (Xenopus laevis)); AKT1 (v-akt murine thymoma viral oncogene homolog 1); ALK (anaplastic lymphoma receptor tyrosine kinase); ALKBH6 (alkB, alkylation repair homolog 6 (E. coli)); ALPK2 (alpha-kinase 2); ANK3 (ankyrin 3, node of Ranvier (ankyrin G)); APC (adenomatous polyposis coli); APOL2 (apolipoprotein L, 2); ARHGAP35 (glucocorticoid receptor DNA binding factor 1); ARID1A (AT rich interactive domain 1A (SWI-like)); ARID2 (AT rich interactive domain 2 (ARID, RFX-like)); ARID5B (AT rich interactive domain 5B (MRF1-like)); ASXL1 (additional sex combs like 1 (Drosophila)); ASXL2 (additional sex combs like 2 (Drosophila)); ATM (ataxia telangiectasia mutated); ATP5B (ATP synthase, H+ transporting, mitochondrial F1 complex, beta polypeptide); AXIN2 (axin 2 (conductin, axil)); AZGP1 (alpha-2-glycoprotein 1, zinc-binding); B2M (beta-2-microglobulin); BAP1 (BRCA1 associated protein-1 (ubiquitin carboxy-terminal hydrolase)); BCLAF1 (BCL2-associated transcription factor 1); BCOR (BCL6 co-repressor); BHMT2 (betaine-homocysteine methyltransferase 2); BRAF (v-raf murine sarcoma viral oncogene homolog B1); BRCA1 (breast cancer 1, early onset); BRE (brain and reproductive organ-expressed (TNFRSF1A modulator)); C3orf70 (chromosome 3 open reading frame 70); CAP2 (CAP, adenylate cyclase-associated protein, 2 (yeast)); CARD11 (caspase recruitment domain family, member 11); CASP8 (caspase 8, apoptosis-related cysteine peptidase); CBFB (core-binding factor, beta subunit); CCDC1020 (coiled-coil domain containing 120); CCDC6 (coiled-coil domain containing 6); CCND1 (cyclin D1); CD1D (CD1d molecule); CD70 (CD70 molecule); CD79B (CD79b molecule, immunoglobulin-associated beta); CDC27 (cell division cycle 27 homolog (S. cerevisiae)); CDH1 (cadherin 1, type 1, E-cadherin (epithelial)); CDK12 (cyclin-dependent kinase 12); CDK4 (cyclin-dependent kinase 4); CDKN1A (cyclin-dependent kinase inhibitor 1A (p21, Cipl)); CDKN1B (cyclin-dependent kinase inhibitor 1B (p27, Kip1)); CDKN2A (cyclin-dependent kinase inhibitor 2A (melanoma, p16, inhibits CDK4)); CEBPA (CCAAT/enhancer binding protein (C/EBP), alpha); CEP76 (centrosomal protein 76kDa); CHD4 (chromodomain helicase DNA binding protein 4); CHD8 (chromodomain helicase DNA binding protein 8); CNBD1 (cyclic nucleotide binding domain containing 1); CNKSR1 (connector enhancer of kinase suppressor of Ras 1); COL5A1 (collagen, type V, alpha 1); COL5A3 (collagen, type V, alpha 3); CREBBP (CREB binding protein (Rubinstein-Taybi syndrome)); CTCF (CCCTC-binding factor (zinc finger protein)); CTNNB1 (catenin (cadherin-associated protein), beta 1, 88kDa); CUL4B (cullin 4B); CUX1 (cut-like homeobox 1); DDX3X (DEAD (Asp-Glu-Ala-Asp) box polypeptide 3, X-linked); DDX5 (DEAD (Asp-Glu-Ala-Asp) box polypeptide 5); DIAPH1 (diaphanous homolog 1 (Drosophila)); DIS3 (DIS3 mitotic control homolog (S. cerevisiae)); DNAH12 (dynein, axonemal, heavy chain 12); DNER (delta/notch-like EGF repeat containing); DNMT3A (DNA (cytosine-5-)-methyltransferase 3 alpha); EGFR (epidermal growth factor receptor (erythroblastic leukemia viral (v-erb-b) oncogene homolog, avian)); EIF2S2 (eukaryotic translation initiation factor 2, subunit 2 beta, 38kDa); ELF3 (E74-like factor 3 (ets domain transcription factor, epithelial-specific )); EP300 (E1A binding protein p300); EPHA2 (EPH receptor A2); ERBB2 (v-erb-b2 erythroblastic leukemia viral oncogene homolog 2, neuro/glioblastoma derived oncogene homolog (avian)); ERBB3 (v-erb-b2 erythroblastic leukemia viral oncogene homolog 3 (avian)); ERCC2 (excision repair cross-complementing rodent repair deficiency, complementation group 2 (xeroderma pigmentosum D)); EZH1 (enhancer of zeste homolog 1 (Drosophila)); EZH2 (enhancer of zeste homolog 2 (Drosophila)); EZR (ezrin); FAM166A (family with sequence similarity 166, member A); FAM46C (family with sequence similarity 46, member C);FAT1 (FAT tumor suppressor homolog 1 (Drosophila)); FBXW7 (F-box and WD repeat domain containing 7); FGFBP1 (fibroblast growth factor binding protein 1); FGFR2 (fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FGFR3 (fibroblast growth factor receptor 3 (achondroplasia, thanatophoric dwarfism));FLG (filaggrin); FLT3 (fms-related tyrosine kinase 3); FOXA1 (forkhead box A1); FOXQ1 (forkhead box Q1); FRMD7 (FERM domain containing 7); GATA3 (GATA binding protein 3); GNA13 (guanine nucleotide binding protein (G protein), alpha 13); GNB1 (guanine nucleotide binding protein (G protein), beta polypeptide 1); GNPTAB (N-acetylglucosamine-1-phosphate transferase, alpha and beta subunits); GOT1 (glutamic-oxaloacetic transaminase 1, soluble (aspartate aminotransferase 1)); GPS2 (G protein pathway suppressor 2); GUSB (glucuronidase, beta); HIST1H1E (histone cluster 1, H1e); HIST1H3B (histone cluster 1, H3b); HIST1H4E (histone cluster 1, H4e); HLA-A (major histocompatibility complex, class I, A); HLA-B (major histocompatibility complex, class I, B); HRAS (v-Ha-ras Harvey rat sarcoma viral oncogene homolog); HSP90AB1 (heat shock protein 90kDa alpha (cytosolic), class B member 1); IDH1 (isocitrate dehydrogenase 1 (NADP+), soluble); IDH2 (isocitrate dehydrogenase 2 (NADP+), mitochondrial); IL7R (interleukin 7 receptor); ING1 (inhibitor of growth family, member 1); INPPL1 (inositol polyphosphate phosphatase-like 1); INTS12 (integrator complex subunit 12); IPO7 (importin 7); IRF4 (interferon regulatory factor 4); IRF6 (interferon regulatory factor 6); ITGB7 (integrin, beta 7); ITPKB (inositol 1,4,5-trisphosphate 3-kinase B); KDM5C (lysine (K)-specific demethylase 5C); KDM6A (lysine (K)-specific demethylase 6A); KEAP1 (kelch-like ECH-associated protein 1); KEL (Kell blood group, metallo-endopeptidase); KIT (v-kit Hardy-Zuckerman 4 feline sarcoma viral oncogene homolog); KLHL8 (kelch-like 8 (Drosophila)); KRAS (v-Ki-ras2 Kirsten rat sarcoma viral oncogene homolog); LCTL (lactase-like); MAP2K1 (mitogen-activated protein kinase kinase 1); MAP2K4 (mitogen-activated protein kinase kinase 4); MAP3K1 (mitogen-activated protein kinase kinase kinase 1); MAP4K3 (mitogen-activated protein kinase kinase kinase 3); MBD1 (methyl-CpG binding domain protein 1); MED12 (mediator complex subunit 12); MED23 (mediator complex subunit 23); MET (met proto-oncogene (hepatocyte growth factor receptor)); MAG (MAX gene associated); MICALCL (MICAL C-terminal like); MLL (myeloid/lymphoid or mixed-lineage leukemia (trithorax homolog, Drosophila)); MLL2 (myeloid/lymphoid or mixed-lineage leukemia 2); MLL3 (myeloid/lymphoid or mixed-lineage leukemia 3); MLL4 (myeloid/lymphoid or mixed-lineage leukemia 2); MORC4 (MORC family CW-type zinc finger 4); MPO (myeloperoxidase); MTOR (mechanistic target of rapamycin (serine/threonine kinase)); MUC17 (mucin 17, cell surface associated); MXRA5 (matrix-remodelling associated 5); MYB (v-myb myeloblastosis viral oncogene homolog (avian)); MYCN (v-myc myelocytomatosis viral related oncogene, neuroblastoma derived (avian)); MYD88 (myeloid differentiation primary response gene (88)); MYOCD (myocardin); NBPF1 (neuroblastoma breakpoint family, member 1); NCOR1 (nuclear receptor co-repressor 1); NF1 (neurofibromin 1 (neurofibromatosis, von Recklinghausen disease, Watson disease)); NFE2L2 (nuclear factor (erythroid-derived 2)-like 2); NOTCH1 (Notch homolog 1, translocation-associated (Drosophila)); NPM1 (nucleophosmin (nucleolar phosphoprotein B23, numatrin)); NRAS (neuroblastoma RAS viral (v-ras) oncogene homolog); NSD1 (nuclear receptor binding SET domain protein 1); NTN4 (netrin 4); NUP210L (nucleoporin 210kDa-like); ODAM (odontogenic, ameloblast asssociated); OMA1 (OMA1 homolog, zinc metallopeptidase (S. cerevisiae)); OR4A16 (olfactory receptor, family 4, subfamily A, member 16); OR52N1 (olfactory receptor, family 52, subfamily N, member 1); OTUD7A (OTU domain containing 7A); PAPD5 (PAP associated domain containing 5); PBRM1 (polybromo 1); PCBP1 (poly(rC) binding protein 1); PDAP1 (PDGFA associated protein 1); PDCD2L (programmed cell death 2-like); PDSS2 (prenyl (decaprenyl) diphosphate synthase, subunit 2); PHF6 (PHD finger protein 6); PIK3CA (phosphoinositide-3-kinase, catalytic, alpha polypeptide); PIK3R1 (phosphoinositide-3-kinase, regulatory subunit 1 (alpha)); PLCG2 (phospholipase C, gamma 2 (phosphatidylinositol-specific)); POLE (polymerase (DNA directed), epsilon); POU2AF1 (POU class 2 associating factor 1); POU2F2 (POU class 2 homeobox 2); PPM1D (protein phosphatase ID magnesium-dependent, delta isoform); PPP2R1A (protein phosphatase 2 (formerly 2A), regulatory subunit A , alpha isoform); PPP6C (protein phosphatase 6, catalytic subunit); PRDM1 (PR domain containing 1, with ZNF domain); PTEN (phosphatase and tensin homolog (mutated in multiple advanced cancers 1)); PTPN11 (protein tyrosine phosphatase, non-receptor type 11 (Noonan syndrome 1)); QKI (quaking homolog, KH domain RNA binding (mouse)); RAB40A (RAB40A, member RAS oncogene family); RAC1 (ras-related C3 botulinum toxin substrate 1 (rho family, small GTP binding protein Rac1)); RAD21 (RAD21 homolog (S. pombe)); RASA1 (RAS p21 protein activator (GTPase activating protein) 1); RB1 (retinoblastoma 1 (including osteosarcoma)); RBM10 (RNA binding motif protein 10); RHEB (Ras homolog enriched in brain); RHOA (ras homolog gene family, member A); RIT1 (Ras-like without CAAX 1); RPL5 (ribosomal protein L5); RPS15 (ribosomal protein S15); RPS2 (ribosomal protein S2); RSBN1L (round spermatid basic protein 1-like); RUNX1 (runt-related transcription factor 1 (acute myeloid leukemia 1; aml1 oncogene)); RXRA (retinoid X receptor, alpha); SACS (spastic ataxia of Charlevoix-Saguenay (sacsin)); SELP (selectin P (granule membrane protein 140kDa, antigen CD62)); SEPT12 (septin 12); SERPINB13 (serpin peptidase inhibitor, clade B (ovalbumin), member 13); SETD2 (SET domain containing 2); SETDB1 (SET domain, bifurcated 1); SF3B1 (splicing factor 3b, subunit 1, 155kDa); SFRS2 (splicing factor, arginine/serine-rich 2); SGK1 (serum/glucocorticoid regulated kinase 1); SIRT4 (sirtuin (silent mating type information regulation 2 homolog) 4 (S. cerevisiae)); SLC1A3 (solute carrier family 1 (glial high affinity glutamate transporter), member 3); SLC26A3 (solute carrier family 26, member 3); SLC44A3 (solute carrier family 44, member 3); SLC4A5 (solute carrier family 4, sodium bicarbonate cotransporter, member 5); SMAD2 (SMAD family member 2); SMAD4 (SMAD family member 4); SMARCA4 (SWI/SNF related, matrix associated, actin dependent regulator of chromatin, subfamily a, member 4); SMARCB1 (SWI/SNF related, matrix associated, actin dependent regulator of chromatin, subfamily b, member 1); SMC1A (structural maintenance of chromosomes 1A); SMC3 (structural maintenance of chromosomes 3); SNX25 (sorting nexin 25); SOS1 (son of sevenless homolog 1 (Drosophila)); SOX17 (SRY (sex determining region Y)-box 17); SPEN (spen homolog, transcriptional regulator (Drosophila)); SPOP (speckle-type POZ protein); STAG2 (stromal antigen 2); STK11 (serine/threonine kinase 11); STK19 (serine/threonine kinase 19); STX2 (syntaxin 2); TAP1 (transporter 1, ATP-binding cassette, sub-family B (MDR/TAP)); TBC1D12 (TBC1 domain family, member 12); TBL1XR1 (transducin (beta)-like 1 X-linked receptor 1); TBX3 (T-box 3 (ulnar mammary syndrome)); TCEB1 (transcription elongation factor B (SIII), polypeptide 1 (15kDa, elongin C)); TCF7L2 (transcription factor 7-like 2 (T-cell specific, HMG-box)); TCP11L2 (t-complex 11 (mouse)-like 2); TDRD10 (tudor domain containing 10); TET2 (tet oncogene family member 2); TGFBR2 (transforming growth factor, beta receptor II (70/80kDa)); TIMM17A (translocase of inner mitochondrial membrane 17 homolog A (yeast)); TNF (tumor necrosis factor (TNF superfamily, member 2)); TNFRSF14 (tumor necrosis factor receptor superfamily, member 14 (herpesvirus entry mediator)); TP53 (tumor protein p53); TP53BP1 (tumor protein p53 binding protein 1); TPX2 (TPX2, microtubule-associated, homolog (Xenopus laevis)); TRAF3 (TNF receptor-associated factor 3); TRIM23 (tripartite motif-containing 23); TSC1 (tuberous sclerosis 1); TTLL9 (tubulin tyrosine ligase-like family, member 9); TXNDC8 (thioredoxin domain containing 8 (spermatozoa)); U2AF1 (U2 small nuclear RNA auxiliary factor 1); VHL (von Hippel-Lindau tumor suppressor); WASF3 (WAS protein family, member 3); WT1 (Wilms tumor 1); XIRP2 (xin actin-binding repeat containing 2); XPO1 (exportin 1 (CRM1 homolog, yeast)); ZFHX3 (zinc finger homeobox 3); ZNF180 (zinc finger protein 180); ZNF471 (zinc finger protein 471); ZNF483 (zinc finger protein 483); ZNF620 (zinc finger protein 620); ZNF750 (zinc finger protein 750); and ZRANB3 (zinc finger, RAN-binding domain containing 3).

20) The vector system of items 1-12, wherein the target sequences are genes selected from the group consisting of FLT3, DNMT3A, NPM1, IDH2, IDH1, TET2, NRAS, RUNX1, WT1, U2AF1, TP53, KRAS, PTPN11, KIT, SMC3, STAG2, PHF6, RAD21, CEBPA, ASXL1, SFRS2, SMC1A, PAPD5, EZH2, PDSS2, MXRA5, and KDM6A.

21) The vector system of items 1-12, wherein the target sequences are genes selected from the group consisting of TP53, KDM6A, RB1, PIK3CA, ARID1A, MLL2, CDKN1A, ERCC2, STAG2, RXRA, TBC1D12, NFE2L2, C3orf70, ERBB3, ELF3, FBXW7, FGFR3, FOXQ1, CREBBP, HRAS, SNX25, TSC1, MGA, EZR, DDX5, MLL, RHOA, PHF6, MLL3, BCLAF1, TGFBR2, EPHA2, SETD2, and CDKN2A.

22) The vector system of items 1-12, wherein the target sequences are genes selected from the group consisting of PIK3CA, TP53, GATA3, MAP3K1, PTEN, AKT1, CTCF, CBFB, MLL3, MAP2K4, RUNX1, CDH1, SF3B1, PIK3R1, ARID1A, NCOR1, KRAS, SPEN, RB1, MLL, ERBB2, TBL1XR1, CDKN1B, HIST1H3B, FOXA1, CASP8, MED23, TBX3, CUL4B, STAG2, MYB, RAB40A, EP300, FGFR2, GNPTAB, ERBB3, and ACVR1B.

23) The vector system of items 1-12, wherein the target sequences are genes selected from the group consisting of CDKN1B, ALK, and SMARCB1.

24) The vector system of items 1-12, wherein the target sequences are genes selected from the group consisting of SF3B1, TP53, MYD88, XPO1, HIST1H1E, RPS15, RPS2 NRAS, DDX3X, SPEN, ATM, MED12, IDH1, SETDB1, and JAK1.

25) The vector system of items 1-12, wherein the target sequences are genes selected from the group consisting of APC, TP53, FBXW7, SMAD4, NRAS, SMAD2, TCF7L2, BRAF, KRAS, PIK3CA, PCBP1, ARID1A, ACVR1B, ERBB3, CASP8, ELF3, TRIM23, CDC27, B2M, NTN4, AXIN2, SIRT4, GOT1 RBM10, BCLAF1, BCOR, MAP2K4, IDH2, PTEN, ERBB2, ARID2, CTNNB1, TRAF3, CNBD1, and CD70.

26) The vector system of items 1-12, wherein the target sequences are genes selected from the group consisting of TP53, MYD88, TNFRSF14, CD79B, MLL2, EZH2, CARD11, CREBBP, TNF, CD70, POU2F2, ITPKB, HLA-A, BRAF, GNA13, POU2AF1 HIST1H3B, KRAS, B2M, NOTCH1, PTEN, and MAP2K1, ZNF471.

27) The vector system of items 1-12, wherein the target sequences are genes selected from the group consisting of PTEN, PIK3CA, PIK3R1, TP53, KRAS, FBXW7, CTCF, ARID1A, ARHGAP35, ZFHX3, FGFR2, CCND1, PPP2R1A, ING1, ZNF471, ERBB3, CTNNB1, BCOR, CUX1, SPOP, NRAS, FAT1, ARID5B, CCDC6, ZNF620, AKT1, SLC1A3, EIF2S2, TCP11L2, SGK1, DIAPH1, KLHL8, SOX17, GNPTAB, SLC44A3, ADNP, POLE, TTLL9, SELP, CHD4, PPM1D, RSBN1L, MICALCL, SACS, ANK3, TBL1XR1, SOS1, DNER, MORC4, MYCN, TXNDC8, INPPL1, ZRANB3, TAP1, EP300, TPX2, CAP2, ALKBH6 CASP8, RASA1, MGA, PBRM1, ERBB2, MLL4, ATM, CDKN1B, RB1, NFE2L2, TP53BP1, COL5A1, FLT3, MTOR, and TDRD10.

28) The vector system of items 1-12, wherein the target sequences are genes selected from the group consisting of TP53, CDKN2A, FLG, SMARCA4, PIK3CA, SMARCB1, IL7R, COL5A1 SMAD4, KRAS, ARID1A, SETDB1, ERBB2, SACS, and PIK3R1.

29) The vector system of items 1-12, wherein the target sequences are genes selected from the group consisting of TP53, EGFR, PIK3R1, PIK3CA, IDH1, RB1, PTEN, NF1, STAG2, RPL5, SLC26A3, BRAF, MAP3K1, KEL, CD1D, CHD8, DDX5, MUC17, QKI, AZGP1, SETD2, NUP210L SMC1A, BCOR, PTPN11, and EZR.

30) The vector system of items 1-12, wherein the target sequences are genes selected from the group consisting of TP53, CDKN2A, CASP8, FAT1, NFE2L2, NOTCH1, MLL2, NSD1, HRAS, EPHA2, PIK3CA, AJUBA, RAC1, ZNF750, RHOA, TGFBR2, PTEN, HLA-A, EP300, B2M, OTUD7A, HLA-B, CTCF, IPO7, RASA1, MAP4K3 RB1, KDM6A, SMAD4, ARID2, ASXL1, ARHGAP35, MLL3, NCOR1, and PBRM1.

31) The vector system of items 1-12, wherein the target sequences are genes selected from the group consisting of SETD2, BAP1, VHL, PBRM1, PTEN, KDM5C, TP53, MTOR, ARID1A, GUSB, RHEB, ATM, TCEB1, MPO, CCDC120, PIK3CA, EGFR, ARHGAP35, BRCA1, FGFR3, and COL5A3.

32) The vector system of items 1-12, wherein the target sequences are genes selected from the group consisting of TP53, KEAP1, STK11, CDKN2A, KRAS, U2AF1, SMARCA4, EGFR, MET, NF1, RIT1, BRAF, PIK3CA, RBM10, ERBB2, ARID1A, ATM, SLC4A5, NBPF1, STX2, MAP2K1, RB1, FAT1, APC, MLL3, NRAS, SMAD4, CDKN1B, CTNNB1, ARHGAP35, ARID2, and CDK12.

33) The vector system of items 1-12, wherein the target sequences are genes selected from the group consisting of TP53, CDKN2A, MLL2, KEAP1, PIK3CA, NFE2L2, RB1, HRAS, HLA-A, ARID1A, FBXW7, PTEN, EP300, EGFR, ARHGAP35, RASA1, FGFR3, NF1, NSD1, ASXL1, STK11, FAT1, NOTCH1, and SERPINB13.

34) The vector system of items 1-12, wherein the target sequences are genes selected from the group consisting of DDX3X, CTNNB1, and TP53.

35) The vector system of items 1-12, wherein the target sequences are genes selected from the group consisting of BRAF, NRAS, CDKN2A, TP53, BCLAF1, RAC1, PTEN, PPP6C, FRMD7, CDK4, STK19, ACO1, XIRP2, LCTL, OR52N1, ALPK2, WASF3, OR4A16, MYOCD, CTNNB1, ZNF750, KIT, MXRA5, ARID2, ANK3, RXRA, PIK3CA, and NCOR1.

36) The vector system of items 1-12, wherein the target sequences are genes selected from the group consisting of KRAS, NRAS, TP53, DIS3, BRAF, FAM46C, INTS12, TRAF3, PRDM1, IRF4, IDH1, FGFR3, PTPN11, and EZR.

37) The vector system of items 1-12, wherein the target sequences are genes selected from the group consisting of TP53, BRCA1, RB1, CDK12, NF1, SMARCB1, KRAS, NRAS, CREBBP, and ERBB2.

38) The vector system of items 1-12, wherein the target sequences are genes selected from the group consisting of SPOP, TP53, ATM, MED12, FOXA1, and COL5A1.

39) The vector system of any one of items 1-38, wherein components I and II are located on the same vector of the vector system.

40) The vector system of any one of items 1-38, wherein components I and II are located on different vectors of the vector system.

41) The vector system of any one of items 1-38, wherein the vector system does not comprise component I.

42) The vector system of any one of items 1-38, wherein the vector system does not comprise component II.

43) The vector system of any one of items 1-38, wherein the guide sequences are located on different vectors of the vector system.

44) The vector system of any one of items 1-38, wherein the guide sequences are located on the same vector of the vector system.

45) A method for producing a genetically altered population of cells, wherein said method comprises transducing a population of cells from an organism with a vector system according to any one of items 1-44.

46) The method for producing a genetically altered population of cells of item 45, wherein the method is performed on non-humans.

47) The method for producing a genetically altered population of cells of items 45 or 46, wherein the population of cells is isolated from an organism.

48) The method for producing a genetically altered population of cells of item 45, wherein the method is performed *in vitro.*

49) A use of the vector system a vector system according to any one of items 1-44 for producing a genetically altered population of cells from an organism.

50) The use of the vector system according to item 49, wherein the use is performed in non-human animals.

51) The use of the vector system according to item 49, wherein the use is performed *in vitro.*

52) A genetically altered population of cells obtainable by a method according to any one of items 45-48.

53) A method for producing a non-human animal, wherein said method comprises the use of the vector system of any one of items 1-44.

54) A method for producing a non-human animal, wherein said method comprises the delivery of the vector system of any one of items 1-44 to a non-human animal.

55) A method for producing a non-human animal, wherein said method comprises the delivery of the genetically altered population of cells of item 52 to a non-human animal.

56) A method for producing a non-human animal, wherein said method comprises the delivery of the vector system of any one of items 1-44 to the pronucleus of a zygote from a pseudo pregnant female.

57) The method for producing a non-human animal of item 56, wherein the pseudo pregnant female is selected from the group consisting of mice, rats and rabbits.

58) The method for producing a non-human animal of item 56 or item 57, wherein the pseudo pregnant female is a mouse.

59) A non-human animal obtainable by a method according to any one of items 53-58.

60) The non-human animal according to item 59, wherein the non-human animal comprises a proliferative condition resulting from a method according to any one of items 53-58.

61) A non-human animal comprising the vector system of any one of items 1-44.

62) The non-human animal according to item 61, wherein the non-human animal comprises a proliferative condition resulting from the vector system of any one of items 1-44.

63) A non-human animal comprising a genetically altered population of cells of item 52.

64) The non-human animal according to item 63, wherein the non-human animal comprises a proliferative condition resulting from the genetically altered population of cells of item 52.

65) The non-human animal according to any one of items 60, 62, or 64, wherein the proliferative condition is a proliferative disorder.

66) The non-human animal according to any one of items 60, 62, or 64, wherein the proliferative condition is a cancer.

67) The non-human animal according to any one of items 60, 62, or 64, wherein the proliferative condition is a myeloid malignancy.

68 The non-human animal according to any one of items 60, 62, or 64, wherein the proliferative condition is selected from the group consisting of acute myeloid leukemia, bladder cancer, breast cancer, carcinoid cancer, chronic lymphocytic leukemia cancer, colorectal cancer, diffuse large B-cell lymphoma cancer, endometrial cancer, esophageal adenocarcinoma cancer, glioblastoma multiforme cancer, head and neck cancer, kidney clear cell cancer, lung adenocarcinoma cancer, lung squamous cell carcinoma cancer, medulloblastoma cancer, melanoma cancer, multiple myeloma cancer, neuroblastoma cancer, ovarian cancer, prostate cancer, and rhabdoid tumor/cancer

69) A method for inducing a proliferative condition, wherein said method comprises transducing a population of cells with a vector system according to any one of items 1-44.

70) The method for inducing a proliferative condition according to item 69, wherein said method comprises transducing a population of non-human cells with a vector system according to any one of items 1-44.

71) The method for inducing a proliferative condition according to item 69 or item 70, wherein said method is performed *in vitro.*

72) The method for inducing a proliferative condition according to any one of items 69-71, wherein said proliferative condition is proliferative disorder.

73) The method for inducing a proliferative condition according to any one of items 69-71, wherein said proliferative condition is a cancer.

74) The method for inducing a proliferative condition according to any one of items 69-71, wherein said proliferative condition is a myeloid malignancy.

75 The method for inducing a proliferative condition according to any one of items 69-71, wherein the proliferative condition is selected from the group consisting of acute myeloid leukemia, bladder cancer, breast cancer, carcinoid cancer, chronic lymphocytic leukemia cancer, colorectal cancer, diffuse large B-cell lymphoma cancer, endometrial cancer, esophageal adenocarcinoma cancer, glioblastoma multiforme cancer, head and neck cancer, kidney clear cell cancer, lung adenocarcinoma cancer, lung squamous cell carcinoma cancer, medulloblastoma cancer, melanoma cancer, multiple myeloma cancer, neuroblastoma cancer, ovarian cancer, prostate cancer, and rhabdoid tumor/cancer.

76) A vector system according to any one of items 1-44 for the use of inducing a proliferative condition in an organism.

77) The vector system according to item 76, wherein the organism is a non-human animal.

78) The vector system according to item 76 or item 77, wherein said proliferative condition is proliferative disorder.

79) The vector system according to item 76 or item 77, wherein said proliferative condition is a cancer.

80) The vector system according to to item 76 or item 77, wherein said proliferative condition is a myeloid malignancy.

81) The vector system according to item 76 or item 77, wherein the proliferative condition is selected from the group consisting of acute myeloid leukemia, bladder cancer, breast cancer, carcinoid cancer, chronic lymphocytic leukemia cancer, colorectal cancer, diffuse large B-cell lymphoma cancer, endometrial cancer, esophageal adenocarcinoma cancer, glioblastoma multiforme cancer, head and neck cancer, kidney clear cell cancer, lung adenocarcinoma cancer, lung squamous cell carcinoma cancer, medulloblastoma cancer, melanoma cancer, multiple myeloma cancer, neuroblastoma cancer, ovarian cancer, prostate cancer, and rhabdoid tumor/cancer

82) A genetically altered population of cells according to item 52, for the use of inducing a proliferative condition in an organism.

83) The genetically altered population of cells according to item 82, wherein the organism is a non-human animal.

84) The genetically altered population of cells according to item 82 or item 83, wherein said proliferative condition is proliferative disorder.

85) The genetically altered population of cells according to item 82 or item 83, wherein said proliferative condition is a cancer.

86) The genetically altered population of cells according to item 82 or item 83, wherein said proliferative condition is a myeloid malignancy.

87) The genetically altered population of cells according to item 82 or item 83, wherein said proliferative condition is selected from the group consisting of acute myeloid leukemia, bladder cancer, breast cancer, carcinoid cancer, chronic lymphocytic leukemia cancer, colorectal cancer, diffuse large B-cell lymphoma cancer, endometrial cancer, esophageal adenocarcinoma cancer, glioblastoma multiforme cancer, head and neck cancer, kidney clear cell cancer, lung adenocarcinoma cancer, lung squamous cell carcinoma cancer, medulloblastoma cancer, melanoma cancer, multiple myeloma cancer, neuroblastoma cancer, ovarian cancer, prostate cancer, and rhabdoid tumor/cancer.

88) A method of interrogating function of one or more genes of cells in a proliferative condition induced according to any one of items 69-75 comprising determining changes in expression of one or more genes of cells in the proliferative condition thereby interrogating the function of one or more genes.

89) A method of interrogating function of one or more genes of cells in a proliferative condition in a non-human animal according to any one of items 60, 62, 64, or 65-68 comprising determining changes in expression of one or more genes of cells in the proliferative condition thereby interrogating the function of one or more genes.

## EXAMPLES

*Example 1: CRISPR Complex Activity in the Nucleus of a Eukaryotic Cell*

An example type II CRISPR system is the type II CRISPR locus from *Streptococcus pyogenes* SF370, which contains a cluster of four genes Cas9, Cas1, Cas2, and Csn1, as well as two non-coding RNA elements, tracrRNA and a characteristic array of repetitive sequences (direct repeats) interspaced by short stretches of non-repetitive sequences (spacers, about 30bp each). In this system, targeted DNA double-strand break (DSB) is generated in four sequential steps (Fig. 2A). First, two non-coding RNAs, the pre-crRNA array and tracrRNA, are transcribed from the CRISPR locus. Second, tracrRNA hybridizes to the direct repeats of pre-crRNA, which is then processed into mature crRNAs containing individual spacer sequences. Third, the mature crRNA:tracrRNA complex directs Cas9 to the DNA target consisting of the protospacer and the corresponding PAM via heteroduplex formation between the spacer region of the crRNA and the protospacer DNA. Finally, Cas9 mediates cleavage of target DNA upstream of PAM to create a DSB within the protospacer (Fig. 2A). This example describes an example process for adapting this RNA-programmable nuclease system to direct CRISPR complex activity in the nuclei of eukaryotic cells.

*Cell culture and transfection*

Human embryonic kidney (HEK) cell line HEK 293FT (Life Technologies) was maintained in Dulbecco's modified Eagle's Medium (DMEM) supplemented with 10% fetal bovine serum (HyClone), 2mM GlutaMAX (Life Technologies), 100U/mL penicillin, and 100$\mu$g/mL streptomycin at 37°C with 5% $CO_2$ incubation. Mouse neuro2A (N2A) cell line (ATCC) was maintained with DMEM supplemented with 5% fetal bovine serum (HyClone), 2mM GlutaMAX (Life Technologies), 100U/mL penicillin, and 100$\mu$g/mL streptomycin at 37°C with 5% $CO_2$.

HEK 293FT or N2A cells were seeded into 24-well plates (Coming) one day prior to transfection at a density of 200,000 cells per well. Cells were transfected using Lipofectamine 2000 (Life Technologies) following the manufacturer's recommended protocol. For each well of a 24-well plate a total of 800ng of plasmids were used.

*Surveyor assay and sequencing analysis for genome modification*

HEK 293FT or N2A cells were transfected with plasmid DNA as described above. After transfection, the cells were incubated at 37°C for 72 hours before genomic DNA extraction. Genomic DNA was extracted using the QuickExtract DNA extraction kit (Epicentre) following the manufacturer's protocol. Briefly, cells were resuspended in QuickExtract solution and incubated at 65°C for 15 minutes and 98°C for 10 minutes. Extracted genomic DNA was immediately processed or stored at -20°C.

The genomic region surrounding a CRISPR target site for each gene was PCR amplified, and products were purified using QiaQuick Spin Column (Qiagen) following manufacturer's protocol. A total of 400ng of the purified PCR products were mixed with 2$\mu$l 10X Taq polymerase PCR buffer (Enzymatics) and ultrapure water to a final volume of 20$\mu$l, and subjected to a re-annealing process to enable heteroduplex formation: 95°C for 10min, 95°C to 85°C ramping at - 2°C/s, 85°C to 25°C at - 0.25°C/s, and 25°C hold for 1 minute. After re-annealing, products were treated with Surveyor nuclease and Surveyor enhancer S (Transgenomics) following the manufacturer's recommended protocol, and analyzed on 4-20% Novex TBE poly-acrylamide gels (Life Technologies). Gels were stained with SYBR Gold DNA stain (Life Technologies) for 30 minutes and imaged with a Gel Doc gel imaging system (Bio-rad). Quantification was based on relative band intensities, as a measure of the fraction of cleaved DNA. Fig. 7 provides a schematic illustration of this Surveyor assay.

Restriction fragment length polymorphism assay for detection of homologous recombination.

HEK 293FT and N2A cells were transfected with plasmid DNA, and incubated at 37°C for 72 hours before genomic DNA extraction as described above. The target genomic region was PCR amplified using primers outside the homology arms of the homologous recombination (HR) template. PCR products were separated on a 1% agarose gel and extracted with MinElute GelExtraction Kit (Qiagen). Purified products were digested with HindIII (Fermentas) and analyzed on a 6% Novex TBE poly-acrylamide gel (Life Technologies).

RNA secondary structure prediction and analysis

RNA secondary structure prediction was performed using the online webserver RNAfold developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62).

RNA purification

HEK 293FT cells were maintained and transfected as stated above. Cells were harvested by trypsinization followed by washing in phosphate buffered saline (PBS). Total cell RNA was extracted with TRI reagent (Sigma) following manufacturer's protocol. Extracted total RNA was quantified using Naonodrop (Thermo Scientific) and normalized to same concentration.

*Northern blot analysis of crRNA and tracrRNA expression in mammalian cells*

RNAs were mixed with equal volumes of 2X loading buffer (Ambion), heated to 95°C for 5 min, chilled on ice for 1 min, and then loaded onto 8% denaturing polyacrylamide gels (SequaGel, National Diagnostics) after pre-running the gel for at least 30 minutes. The samples were electrophoresed for 1.5 hours at 40W limit. Afterwards, the RNA was transferred to Hybond N+ membrane (GE Healthcare) at 300 mA in a semi-dry transfer apparatus (Bio-rad) at room temperature for 1.5 hours. The RNA was crosslinked to the membrane using autocrosslink button on Stratagene UV Crosslinker the Stratalinker (Stratagene). The membrane was pre-hybridized in ULTRAhyb-Oligo Hybridization Buffer (Ambion) for 30 min with rotation at 42°C, and probes were then added and hybridized overnight. Probes were ordered from IDT and labeled with [gamma-$^{32}$P] ATP (Perkin Elmer) with T4 polynucleotide kinase (New England Biolabs). The membrane was washed once with pre-warmed (42°C) 2xSSC, 0.5% SDS for 1 min followed by two 30 minute washes at 42°C. The membrane was exposed to a phosphor screen for one hour or overnight at room temperature and then scanned with a phosphorimager (Typhoon).

*Bacterial CRISPR system construction and evaluation*

CRISPR locus elements, including tracrRNA, *Cas9,* and leader were PCR amplified from *Streptococcus pyogenes* SF370 genomic DNA with flanking homology arms for Gibson Assembly. Two *Bsa*I type IIS sites were introduced in between two direct repeats to facilitate easy insertion of spacers (Fig. 8). PCR products were cloned into EcoRV-digested pACYC184 downstream of the tet promoter using Gibson Assembly Master Mix (NEB). Other endogenous CRISPR system elements were omitted, with the exception of the last 50bp of Csn2. Oligos (Integrated DNA Technology) encoding spacers with complimentary overhangs were cloned into the *Bsa*I-digested vector pDC000 (NEB) and then ligated with T7 ligase (Enzymatics) to generate pCRISPR plasmids. Challenge plasmids containing spacers with PAM

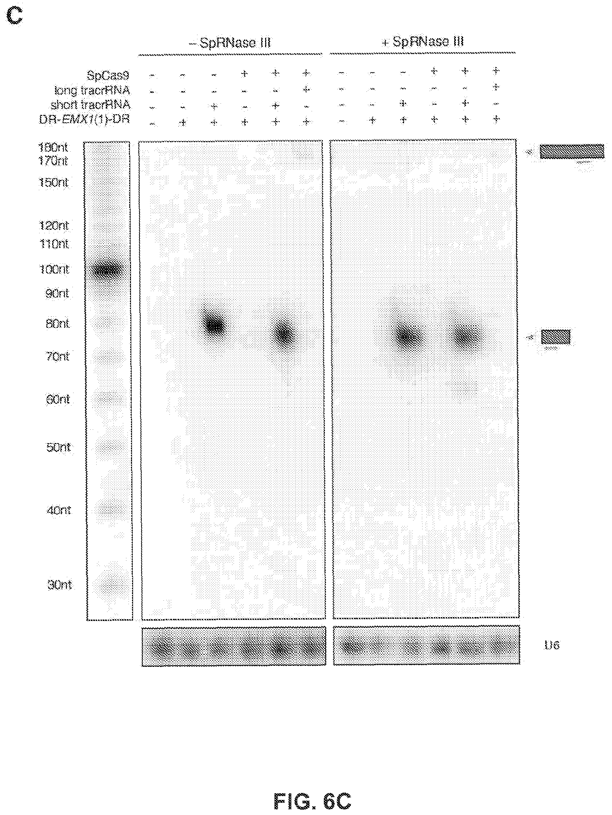

expression in mammalian cells (expression constructs illustrated in Fig. 6A, with functionality as determined by results of the Surveyor assay shown in Fig. 6B). Transcription start sites are marked as +1, and transcription terminator and the sequence probed by northern blot are also indicated. Expression of processed tracrRNA was also confirmed by Northern blot. Fig. 6C shows results of a Northern blot analysis of total RNA extracted from 293FT cells transfected with U6 expression constructs carrying long or short tracrRNA, as well as SpCas9 and DR-EMX1(1)-DR. Left and right panels are from 293FT cells transfected without or with SpRNase III, respectively. U6 indicate loading control blotted with a probe targeting human U6 snRNA. Transfection of the short tracrRNA expression construct led to abundant levels of the processed form of tracrRNA (~75bp). Very low amounts of long tracrRNA are detected on the Northern blot.

To promote precise transcriptional initiation, the RNA polymerase III-based U6 promoter was selected to drive the expression of tracrRNA (Fig. 2C). Similarly, a U6 promoter-based construct was developed to express a pre-crRNA array consisting of a single spacer flanked by two direct repeats (DRs, also encompassed by the term "tracr-mate sequences"; Fig. 2C). The initial spacer was designed to target a 33-base-pair (bp) target site (30-bp protospacer plus a 3-bp CRISPR motif (PAM) sequence satisfying the NGG recognition motif of Cas9) in the human *EMX1* locus (Fig. 2C), a key gene in the development of the cerebral cortex.

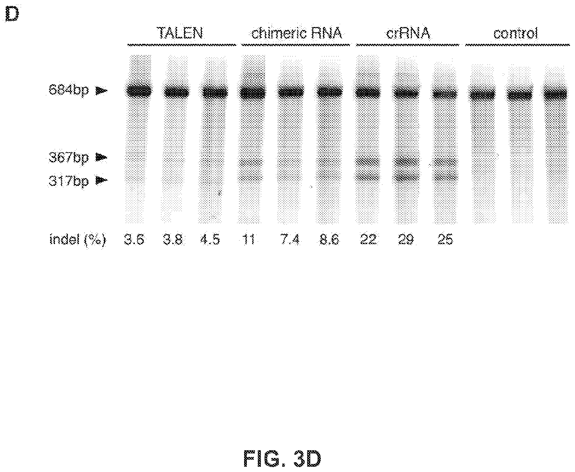

To test whether heterologous expression of the CRISPR system (SpCas9, SpRNase III, tracrRNA, and pre-crRNA) in mammalian cells can achieve targeted cleavage of mammalian chromosomes, HEK 293FT cells were transfected with combinations of CRISPR components. Since DSBs in mammalian nuclei are partially repaired by the non-homologous end joining (NHEJ) pathway, which leads to the formation of indels, the Surveyor assay was used to detect potential cleavage activity at the target *EMX1* locus (Fig. 7) (see e.g. Guschin et al., 2010, Methods Mol Biol 649: 247). Co-transfection of all four CRISPR components was able to induce up to 5.0% cleavage in the protospacer (see Fig. 2D). Co-transfection of all CRISPR components minus SpRNase III also induced up to 4.7% indel in the protospacer, suggesting that there may be endogenous mammalian RNases that are capable of assisting with crRNA maturation, such as for example the related Dicer and Drosha enzymes. Removing any of the remaining three components abolished the genome cleavage activity of the CRISPR system (Fig. 2D). Sanger sequencing of amplicons containing the target locus verified the cleavage activity: in 43 sequenced clones, 5 mutated alleles (11.6%) were found. Similar experiments using a variety of guide sequences produced indel percentages as high as 29% (see Figs. 3-6, 10, and 11). These results define a three-component system for efficient CRISPR-mediated genome modification in mammalian cells. To optimize the cleavage efficiency, Applicants also tested whether different isoforms of tracrRNA affected the cleavage efficiency and found that, in this example system, only the short (89-bp) transcript form was able to mediate cleavage of the human *EMX1* genomic locus (Fig. 6B).

Fig. 12 provides an additional Northern blot analysis of crRNA processing in mammalian cells. Fig. 12A illustrates a schematic showing the expression vector for a single spacer flanked by two direct repeats (DR-EMX1(1)-DR). The 30bp spacer targeting the human EMX1 locus protospacer 1 (see Fig. 6) and the direct repeat sequences are shown in the sequence beneath Fig. 12A. The line indicates the region whose reverse-complement sequence was used to generate Northern blot probes for EMX1(1) crRNA detection. Fig. 12B shows a Northern blot analysis of total RNA extracted from 293FT cells transfected with U6 expression constructs carrying DR-EMX1(1)-DR. Left and right panels are from 293FT cells transfected without or with SpRNase III respectively. DR-EMX1(1)-DR was processed into mature crRNAs only in the presence of SpCas9 and short tracrRNA and was not dependent on the presence of SpRNase III. The mature crRNA detected from transfected 293FT total RNA is ~33bp and is shorter than the 39-42bp mature crRNA from *S. pyogenes.* These results demonstrate that a CRISPR system can be transplanted into eukaryotic cells and reprogrammed to facilitate cleavage of endogenous mammalian target polynucleotides.

Fig. 2 illustrates the bacterial CRISPR system described in this example. Fig. 2A illustrates a schematic showing the CRISPR locus 1 from *Streptococcus pyogenes* SF370 and a proposed mechanism of CRISPR-mediated DNA cleavage by this system. Mature crRNA processed from the direct repeat-spacer array directs Cas9 to genomic targets consisting of complimentary protospacers and a protospacer-adjacent motif (PAM). Upon target-spacer base pairing, Cas9 mediates a double-strand break in the target DNA. Fig. 2B illustrates engineering of *S. pyogenes* Cas9 (SpCas9) and RNase III (SpRNase III) with nuclear localization signals (NLSs) to enable import into the mammalian nucleus. Fig. 2C illustrates mammalian expression of SpCas9 and SpRNase III driven by the constitutive EF1a promoter and tracrRNA and pre-crRNA array (DR-Spacer-DR) driven by the RNA Pol III promoter U6 to promote precise transcription initiation and termination. A protospacer from the human *EMX1* locus with a satisfactory PAM sequence is used as the spacer in the pre-crRNA array. Fig. 2D illustrates surveyor nuclease assay for SpCas9-mediated minor insertions and deletions. SpCas9 was expressed with and without SpRNase III, tracrRNA, and a pre-crRNA array carrying the *EMX1*-target spacer. Fig. 2E illustrates a schematic representation of base pairing between target locus and *EMX1*-targeting crRNA, as well as an example chromatogram showing a micro deletion adjacent to the SpCas9 cleavage site. Fig. 2F illustrates mutated alleles identified from sequencing analysis of 43 clonal amplicons showing a variety of micro insertions and deletions. Dashes indicate deleted bases, and non-aligned or mismatched bases indicate insertions or mutations. Scale bar = 10μm.

To further simplify the three-component system, a chimeric crRNA-tracrRNA hybrid design was adapted, where a mature crRNA (comprising a guide sequence) may be fused to a partial tracrRNA via a stem-loop to mimic the natural crRNA:tracrRNA duplex. To increase co-delivery efficiency, a bicistronic expression vector was created to drive co-expression of a chimeric RNA and SpCas9 in transfected cells. In parallel, the bicistronic vectors were used to express a pre-crRNA (DR-guide sequence-DR) with SpCas9, to induce processing into crRNA with a separately expressed tracrRNA (compare Fig. 11B top and bottom). Fig. 8 provides schematic illustrations of bicistronic expression vectors for pre-crRNA array (Fig. 8A) or chimeric crRNA (represented by the short line downstream of the guide sequence insertion site and upstream of the EF1a promoter in Fig. 8B) with hSpCas9, showing location of various elements and the point of guide sequence insertion. The expanded sequence around the location of the guide sequence insertion site in Fig. 8B also shows a partial DR sequence (GTTTAGAGCTA) and a partial tracrRNA sequence (TAGCAAGTTAAAATAAGGCTAGTCCGTTTTT). Guide sequences can be inserted between BbsI sites using annealed oligonucleotides. Sequence design for the oligonucleotides are shown below the schematic illustrations in Fig. 8, with appropriate ligation adapters indicated. WPRE represents the Woodchuck hepatitis virus post-transcriptional regulatory element. The efficiency of chimeric RNA-mediated cleavage was tested by targeting the same *EMX1* locus described above. Using both Surveyor assay and Sanger sequencing of amplicons, Applicants confirmed that the chimeric RNA design facilitates cleavage of human *EMX1* locus with approximately a 4.7% modification rate (Fig. 3).

Generalizability of CRISPR-mediated cleavage in eukaryotic cells was tested by targeting additional genomic loci in both human and mouse cells by designing chimeric RNA targeting multiple sites in the human *EMX1* and *PVALB,* as well as the mouse *Th* loci. Fig. 13 illustrates the selection of some additional targeted protospacers in human *PVALB* (Fig. 13A) and mouse *Th* (Fig. 13B) loci. Schematics of the gene loci and the location of three protospacers within the last exon of each are provided. The underlined sequences include 30bp of protospacer sequence and 3bp at the 3' end corresponding to the PAM sequences. Protospacers on the sense and anti-sense strands are indicated above and below the DNA sequences, respectively. A modification rate of 6.3% and 0.75% was achieved for the human *PVALB* and mouse *Th* loci respectively, demonstrating the broad applicability of the CRISPR system in modifying different loci across multiple organisms (Fig. 5). While cleavage was only detected with one out of three spacers for each locus using the chimeric constructs, all target sequences were cleaved with efficiency of indel production reaching 27% when using the co-expressed pre-crRNA arrangement (Figs. 6 and 13).

Fig. 11 provides a further illustration that SpCas9 can be reprogrammed to target multiple genomic loci in mammalian cells. Fig. 11A provides a schematic of the human *EMX1* locus showing the location of five protospacers, indicated by the underlined sequences. Fig. 11B provides a schematic of the pre-crRNA/trcrRNA complex showing hybridization between the direct repeat region of the pre-crRNA and tracrRNA (top), and a schematic of a chimeric RNA design comprising a 20bp guide sequence, and tracr mate and tracr sequences consisting of partial direct repeat and tracrRNA sequences hybridized in a hairpin structure (bottom). Results of a Surveyor assay comparing the efficacy of Cas9-mediated cleavage at five protospacers in the human *EMX1* locus is illustrated in Fig. 11C. Each protospacer is targeted using either processed pre-crRNA/tracrRNA complex (crRNA) or chimeric RNA (chiRNA).

Since the secondary structure of RNA can be crucial for intermolecular interactions, a structure prediction algorithm based on minimum free energy and Boltzmann-weighted structure ensemble was used to compare the putative secondary structure of all guide sequences used in the genome targeting experiment (see e.g. Gruber et al., 2008, Nucleic Acids Research, 36: W70). Analysis revealed that in most cases, the effective guide sequences in the chimeric crRNA context were substantially free of secondary structure motifs, whereas the ineffective guide sequences were more likely to form internal secondary structures that could prevent base pairing with the target protospacer DNA. It is thus possible that variability in the spacer secondary structure might impact the efficiency of CRISPR-mediated interference when using a chimeric crRNA.

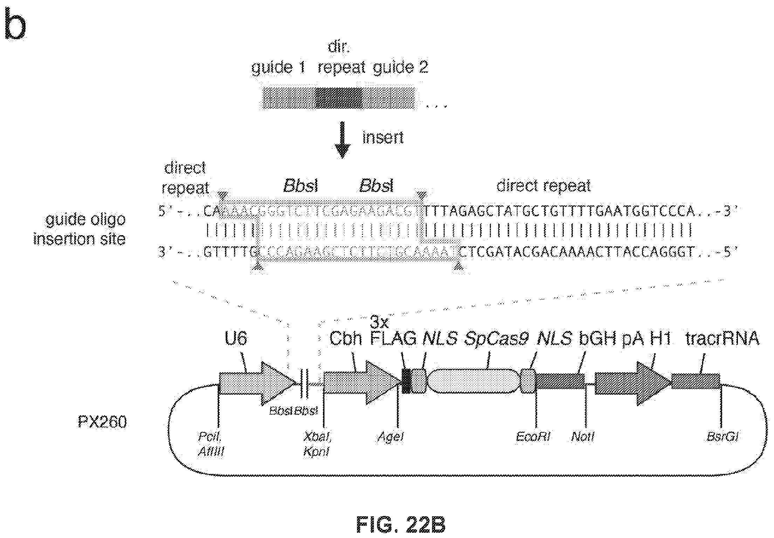

Further vector designs for SpCas9 are shown in Fig. 22, which illustrates single expression vectors incorporating a U6 promoter linked to an insertion site for a guide oligo, and a Cbh promoter linked to SpCas9 coding sequence. The vector shown in Fig. 22b includes a tracrRNA coding sequence linked to an H1 promoter.

In the bacterial assay, all spacers facilitated efficient CRISPR interference (Fig. 3C). These results suggest that there may be additional factors affecting the efficiency of CRISPR activity in mammalian cells.

To investigate the specificity of CRISPR-mediated cleavage, the effect of single-nucleotide mutations in the guide sequence on protospacer cleavage in the mammalian genome was analyzed using a series of *EMX1*-targeting chimeric crRNAs with single point mutations (Fig. 3A). Fig. 3B illustrates results of a Surveyor nuclease assay comparing the cleavage efficiency of Cas9 when paired with different mutant chimeric RNAs. Single-base mismatch up to 12-bp 5' of the PAM substantially abrogated genomic cleavage by SpCas9, whereas spacers with mutations at farther upstream positions retained activity against the original protospacer target (Fig. 3B). In addition to the PAM, SpCas9 has single-base specificity within the last 12-bp of the spacer. Furthermore, CRISPR is able to mediate genomic cleavage as efficiently as a pair of TALE nucleases (TALEN) targeting the same *EMX1* protospacer. Fig. 3C provides a schematic showing the design of TALENs targeting *EMX1*, and Fig. 3D shows a Surveyor gel comparing the efficiency of TALEN and Cas9 (n=3).

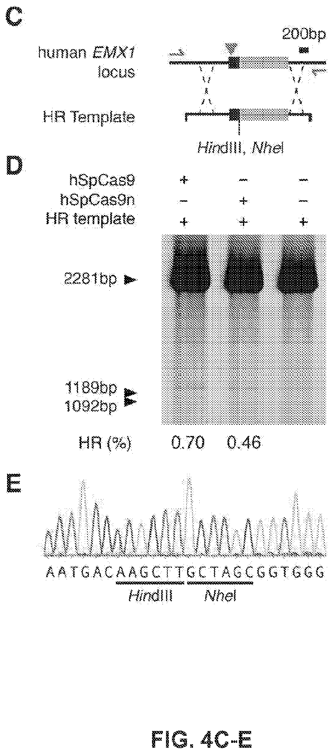

Having established a set of components for achieving CRISPR-mediated gene editing in mammalian cells through the error-prone NHEJ mechanism, the ability of CRISPR to stimulate homologous recombination (HR), a high fidelity gene repair pathway for making precise edits in the genome, was tested. The wild type SpCas9 is able to mediate site-specific DSBs, which can be repaired through both NHEJ and HR. In addition, an aspartate-to-alanine substitution (D10A) in the RuvC I catalytic domain of SpCas9 was engineered to convert the nuclease into a nickase (SpCas9n; illustrated in Fig. 4A) (see e.g. Sapranausaks et al., 2011, Nucleic Acids Research, 39: 9275; Gasiunas et al., 2012, Proc. Natl. Acad. Sci. USA, 109:E2579), such that nicked genomic DNA undergoes the high-fidelity homology-directed repair (HDR). Surveyor assay confirmed that SpCas9n does not generate indels at the *EMX1* protospacer target. As illustrated in Fig. 4B, co-expression of *EMX1*-targeting chimeric crRNA with SpCas9 produced indels in the target site, whereas co-expression with SpCas9n did not (n=3). Moreover, sequencing of 327 amplicons did not detect any indels induced by SpCas9n. The same locus was selected to test CRISPR-mediated HR by co-transfecting HEK 293FT cells with the chimeric RNA targeting *EMX1*, hSpCas9 or hSpCas9n, as well as a HR template to introduce a pair of restriction sites (*Hind*III and *Nhe*I) near the protospacer. Fig. 4C provides a schematic illustration of the HR strategy, with relative locations of recombination points and primer annealing sequences (arrows). SpCas9 and SpCas9n indeed catalyzed integration of the HR template into the *EMX1* locus. PCR amplification of the target region followed by restriction digest with *Hind*III revealed cleavage products corresponding to expected fragment sizes (arrows in restriction fragment length polymorphism gel analysis shown in Fig. 4D), with SpCas9 and SpCas9n mediating similar levels of HR efficiencies. Applicants further verified HR using Sanger sequencing of genomic amplicons (Fig. 4E). These results demonstrate the utility of CRISPR for facilitating targeted gene insertion in the mammalian genome. Given the 14-bp (12-bp from the spacer and 2-bp from the PAM) target specificity of the wild type SpCas9, the availability of a nickase can significantly reduce the likelihood of off-target modifications, since single strand breaks are not substrates for the error-prone NHEJ pathway.

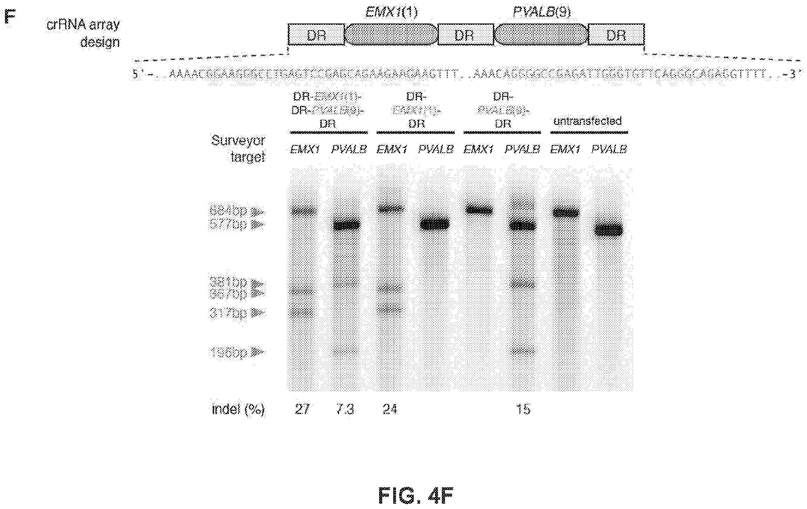

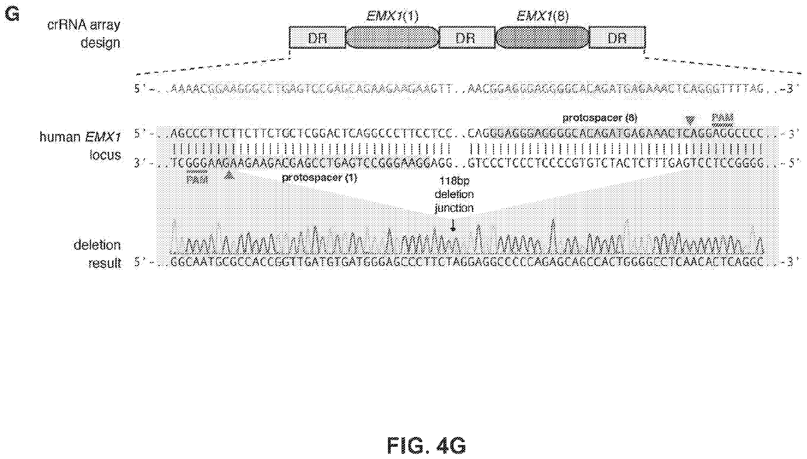

Expression constructs mimicking the natural architecture of CRISPR loci with arrayed spacers (Fig. 2A) were constructed to test the possibility of multiplexed sequence targeting. Using a single CRISPR array encoding a pair of *EMX1-* and *PVALB*-targeting spacers, efficient cleavage at both loci was detected (Fig. 4F, showing both a schematic design of the crRNA array and a Surveyor blot showing efficient mediation of cleavage). Targeted deletion of larger genomic regions through concurrent DSBs using spacers against two targets within *EMX1* spaced by 119bp was also tested, and a 1.6% deletion efficacy (3 out of 182 amplicons; Fig. 4G) was detected. This demonstrates that the CRISPR system can mediate multiplexed editing within a single genome.

*Example 2: CRISPR system modifications and alternatives*

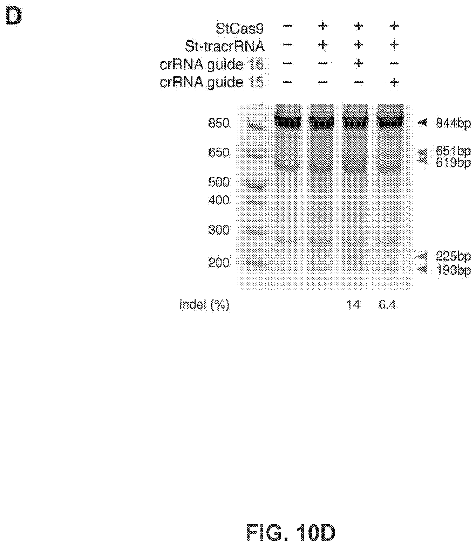

The ability to use RNA to program sequence-specific DNA cleavage defines a new class of genome engineering tools for a variety of research and industrial applications. Several aspects of the CRISPR system can be further improved to increase the efficiency and versatility of CRISPR targeting. Optimal Cas9 activity may depend on the availability of free $Mg^{2+}$ at levels higher than that present in the mammalian nucleus (see e.g. Jinek et al., 2012, Science, 337:816), and the preference for an NGG motif immediately downstream of the protospacer restricts the ability to target on average every 12-bp in the human genome (Fig. 9, evaluating both plus and minus strands of human chromosomal sequences). Some of these constraints can be overcome by exploring the diversity of CRISPR loci across the microbial metagenome (see e.g. Makarova et al., 2011, Nat Rev Microbiol, 9:467). Other CRISPR loci may be transplanted into the mammalian cellular milieu by a process similar to that described in Example 1. For example, Fig. 10 illustrates adaptation of the Type II CRISPR system from CRISPR 1 of *Streptococcus thermophilus* LMD-9 for heterologous expression in mammalian cells to achieve CRISPR-mediated genome editing. Fig. 10A provides a Schematic illustration of CRISPR 1 from *S. thermophilus* LMD-9. Figure 10B illustrates the design of an expression system for the *S. thermophilus* CRISPR system. Human codon-optimized *hStCas9* is expressed using a constitutive EF1α promoter. Mature versions of tracrRNA and crRNA are expressed using the U6 promoter to promote precise transcription initiation. Sequences from the mature crRNA and tracrRNA are illustrated. A single base indicated by the lower case "a" in the crRNA sequence is used to remove the polyU sequence, which serves as a RNA polIII transcriptional terminator. Fig. 10C provides a schematic showing guide sequences targeting the human *EMX1* locus. Fig. 10D shows the results of hStCas9-mediated cleavage in the target locus using the Surveyor assay. RNA guide spacers 1 and 2 induced 14% and 6.4%, respectively. Statistical analysis of cleavage activity across biological replica at these two protospacer sites is also provided in Fig. 5. Fig. 14 provides a schematic of additional protospacer and corresponding PAM sequence targets of the *S. thermophilus* CRISPR system in the human *EMX1* locus. Two protospacer sequences are highlighted and their corresponding PAM sequences satisfying NNAGAAW motif are indicated by underlining 3' with respect to the corresponding highlighted sequence. Both protospacers target the anti-sense strand.

*Example 3: Sample target sequence selection algorithm*

A software program is designed to identify candidate CRISPR target sequences on both strands of an input DNA sequence based on desired guide sequence length and a CRISPR motif sequence (PAM) for a specified CRISPR enzyme. For example, target sites for Cas9 from *S. pyogenes,* with PAM sequences NGG, may be identified by searching for 5'-$N_x$-NGG-3' both on the input sequence and on the reverse-complement of the input. Likewise, target sites for Cas9 of *S. thermophilus* CRISPR1, with PAM sequence NNAGAAW, may be identified by searching for 5'-$N_x$-NNAGAAW-3' both on the input sequence and on the reverse-complement of the input. Likewise, target sites for Cas9 of *S. thermophilus* CRISPR3, with PAM sequence NGGNG, may be identified by searching for 5'-$N_x$-NGGNG-3' both on the input sequence and on the reverse-complement of the input. The value "x" in $N_x$ may be fixed by the program or specified by the user, such as 20.

Since multiple occurrences in the genome of the DNA target site may lead to nonspecific genome editing, after identifying all potential sites, the program filters out sequences based on the number of times they appear in the relevant reference genome. For those CRISPR enzymes for which sequence specificity is determined by a 'seed' sequence, such as the 11-12bp 5' from the PAM sequence, including the PAM sequence itself, the filtering step may be based on the seed sequence. Thus, to avoid editing at additional genomic loci, results are filtered based on the number of occurrences of the seed:PAM sequence in the relevant genome. The user may be allowed to choose the length of the seed sequence. The user may also be allowed to specify the number of occurrences of the seed:PAM sequence in a genome for purposes of passing the filter. The default is to screen for unique sequences. Filtration level is altered by changing both the length of the seed sequence and the number of occurrences of the sequence in the genome. The program may in addition or alternatively provide the sequence of a guide sequence complementary to the reported target sequence(s) by providing the reverse complement of the identified target sequence(s). An example visualization of some target sites in the human genome is provided in Fig. 18.

Further details of methods and algorithms to optimize sequence selection can be found in U.S. application Serial No. 61/064,798 (Attorney docket 44790.11.2022; Broad Reference BI-2012/084).

*Example 4: Evaluation of multiple chimeric crRNA-tracrRNA hybrids*

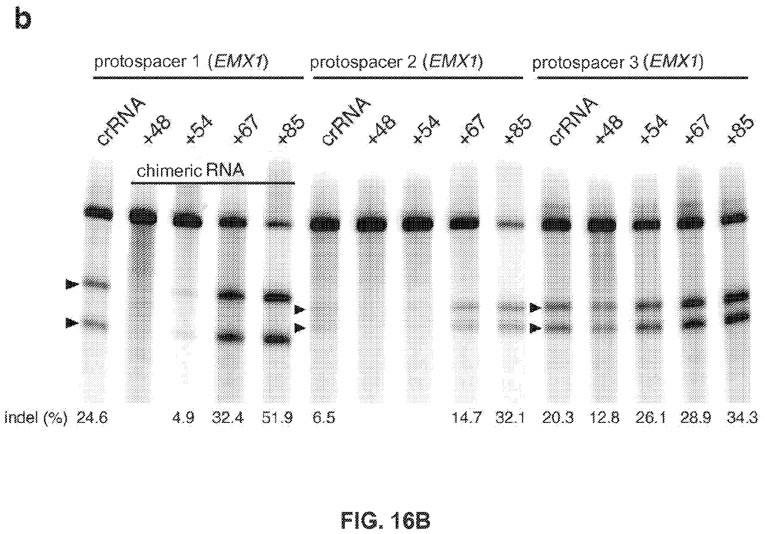

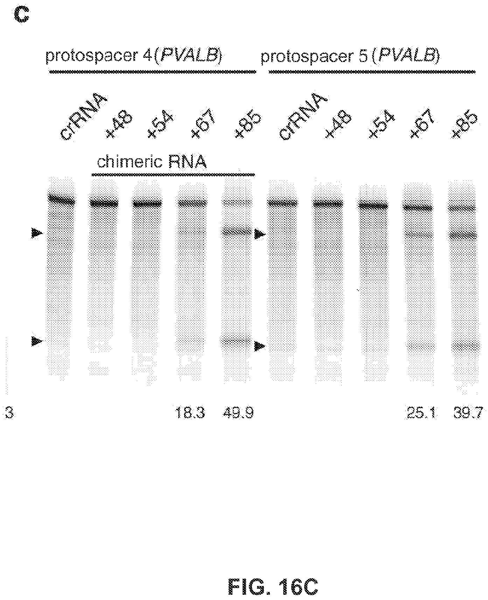

This example describes results obtained for chimeric RNAs (chiRNAs; comprising a guide sequence, a tracr mate sequence, and a tracr sequence in a single transcript) having tracr sequences that incorporate different lengths of wild-type tracrRNA sequence. Fig. 16a illustrates a schematic of a bicistronic expression vector for chimeric RNA and Cas9. Cas9 is driven by the CBh promoter and the chimeric RNA is driven by a U6 promoter. The chimeric guide RNA consists of a 20bp guide sequence (Ns) joined to the tracr sequence (running from the first "U" of the lower strand to the end of the transcript), which is truncated at various positions as indicated. The guide and tracr sequences are separated by the tracr-mate sequence GUUUUAGAGCUA followed by the loop sequence GAAA. Results of SURVEYOR assays for Cas9-mediated indels at the human *EMX1* and *PVALB* loci are illustrated in Figs. 16b and 16c, respectively. Arrows indicate the expected SURVEYOR fragments. ChiRNAs are indicated by their "+n" designation, and crRNA refers to a hybrid RNA where guide and tracr sequences are expressed as separate transcripts. Quantification of these results, performed in triplicate, are illustrated by histogram in Figs. 17a and 17b, corresponding to Figs. 16b and 16c, respectively ("N.D." indicates no indels detected). Protospacer IDs and their corresponding genomic target, protospacer sequence, PAM sequence, and strand location are provided in Table D. Guide sequences were designed to be complementary to the entire protospacer sequence in the case of separate transcripts in the hybrid system, or only to the underlined portion in the case of chimeric RNAs.

**Table D:**

| protospacer ID | genomic target | protospacer sequence (5' to 3') | PAM | strand |
|---|---|---|---|---|
| 1 | *EMX1* | GGACATCGATGTCACCTCCAATGACTAGGG | TGG | + |
| 2 | *EMX1* | CATTGGAGGTGACATCGATGTCCTCCCCAT | TGG | - |
| 3 | *EMX1* | GGAAGGGCCTGAGTCCGAGCAGAAGAAGAA | GGG | + |
| 4 | *PVALB* | GGTGGCGAGAGGGGCCGAGATTGGGTGTTC | AGG | + |
| 5 | *PVALB* | ATGCAGGAGGGTGGCGAGAGGGGCCGAGAT | TGG | + |

Further details to optimize guide sequences can be found in U.S. application Serial No. 61/836,127 (Attorney docket 44790.08.2022; Broad Reference BI-2013/004G).

Initially, three sites within the EMX1 locus in human HEK 293FT cells were targeted. Genome modification efficiency of each chiRNA was assessed using the SURVEYOR nuclease assay, which detects mutations resulting from DNA double-strand breaks (DSBs) and their subsequent repair by the non-homologous end joining (NHEJ) DNA damage repair pathway. Constructs designated chiRNA(+n) indicate that up to the +n nucleotide of wild-type tracrRNA is included in the chimeric RNA construct, with values of 48, 54, 67, and 85 used for n. Chimeric RNAs containing longer fragments of wild-type tracrRNA (chiRNA(+67) and chiRNA(+85)) mediated DNA cleavage at all three EMX1 target sites, with chiRNA(+85) in particular demonstrating significantly higher levels of DNA cleavage than the corresponding crRNA/tracrRNA hybrids that expressed guide and tracr sequences in separate transcripts (Figs. 16b and 17a). Two sites in the PVALB locus that yielded no detectable cleavage using the hybrid system (guide sequence and tracr sequence expressed as separate transcripts) were also targeted using chiRNAs. chiRNA(+67) and chiRNA(+85) were able to mediate significant cleavage at the two PVALB protospacers (Figs. 16c and 17b).

For all five targets in the EMX1 and PVALB loci, a consistent increase in genome modification efficiency with increasing tracr sequence length was observed. Without wishing to be bound by any theory, the secondary structure formed by the 3' end of the tracrRNA may play a role in enhancing the rate of CRISPR complex formation.

*Example 5: Cas9 diversity*

The CRISPR-Cas system is an adaptive immune mechanism against invading exogenous DNA employed by diverse species across bacteria and archaea. The type II CRISPR-Cas9 system consists of a set of genes encoding proteins responsible for the "acquisition" of foreign DNA into the CRISPR locus, as well as a set of genes encoding the "execution" of the DNA cleavage mechanism; these include the DNA nuclease (Cas9), a non-coding transactivating crRNA (tracrRNA), and an array of foreign DNA-derived spacers flanked by direct repeats (crRNAs). Upon maturation by Cas9, the tracrRNA and crRNA duplex guide the Cas9 nuclease to a target DNA sequence specified by the spacer guide sequences, and mediates double-stranded breaks in the DNA near a short sequence motif in the target DNA that is required for cleavage and specific to each CRISPR-Cas system. The type II CRISPR-Cas systems are found throughout the bacterial kingdom and highly diverse in in Cas9 protein sequence and size, tracrRNA and crRNA direct repeat sequence, genome organization of these elements, and the motif requirement for target cleavage. One species may have multiple distinct CRISPR-Cas systems.

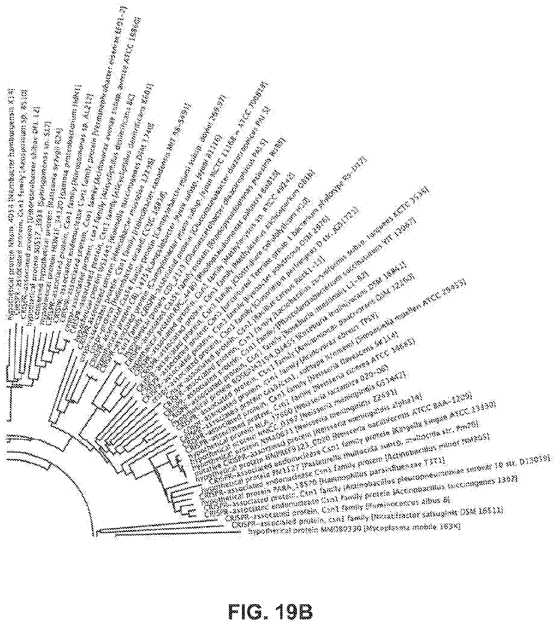

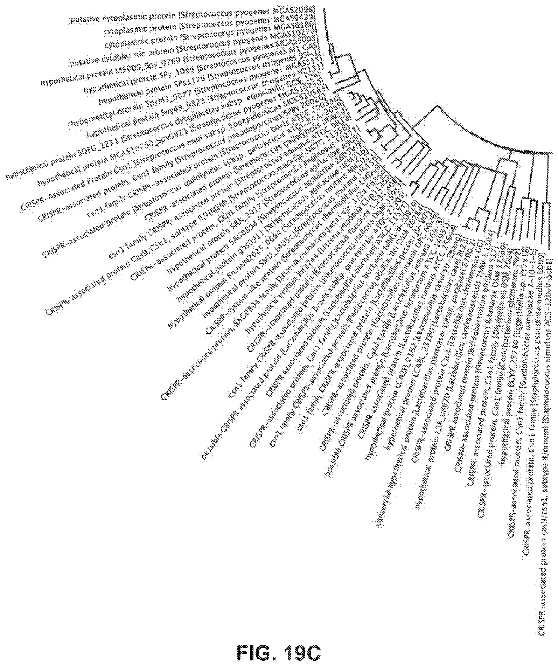

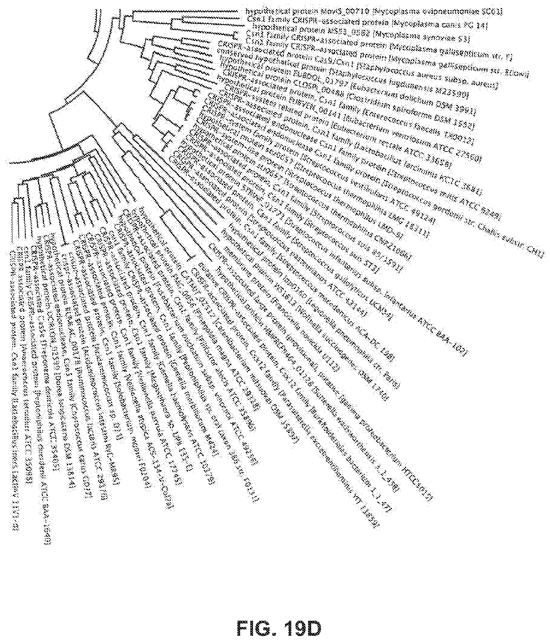

Applicants evaluated 207 putative Cas9s from bacterial species identified based on sequence homology to known Cas9s and structures orthologous to known subdomains, including the HNH endonuclease domain and the RuvC endonuclease domains [information from the Eugene Koonin and Kira Makarova]. Phylogenetic analysis based on the protein sequence conservation of this set revealed five families of Cas9s, including three groups of large Cas9s (~1400 amino acids) and two of small Cas9s (~1100 amino acids) (see Figs. 19 and 20A-F).

Further details of Cas9s and mutations of the Cas9 enzyme to convert into a nickase or DNA binding protein and use of same with altered functionality can be found in U.S. application Serial Nos 61/836,101 and 61/835,936 (Attorney docket 44790.09.2022 and 4790.07.2022 and Broad Reference BI-2013/004E and BI-2013/004F respectively).

*Example 6: Cas9 orthologs*

Applicants analyzed Cas9 orthologs to identify the relevant PAM sequences and the corresponding chimeric guide RNA. Having an expanded set of PAMs provides broader targeting across the genome and also significantly increases the number of unique target sites and provides potential for identifying novel Cas9s with increased levels of specificity in the genome.

The specificity of Cas9 orthologs can be evaluated by testing the ability of each Cas9 to tolerate mismatches between the guide RNA and its DNA target. For example, the specificity of SpCas9 has been characterized by testing the effect of mutations in the guide RNA on cleavage efficiency. Libraries of guide RNAs were made with single or multiple mismatches between the guide sequence and the target DNA. Based on these findings, target sites for SpCas9 can be selected based on the following guidelines:

To maximize SpCas9 specificity for editing a particular gene, one should choose a target site within the locus of interest such that potential 'off-target' genomic sequences abide by the following four constraints: First and foremost, they should not be followed by a PAM with either 5'-NGG or NAG sequences. Second, their global sequence similarity to the target sequence should be minimized. Third, a maximal number of mismatches should lie within the PAM-proximal region of the off-target site. Finally, a maximal number of mismatches should be consecutive or spaced less than four bases apart.

Similar methods can be used to evaluate the specificity of other Cas9 orthologs and to establish criteria for the selection of specific target sites within the genomes of target species. As mentioned previously phylogenetic analysis based on the protein sequence conservation of this set revealed five families of Cas9s, including three groups of large Cas9s (~1400 amino acids) and two of small Cas9s (~1100 amino acids) (see Figs. 19 and 20A-F). Further details on Cas orthologs can be found in U.S. application Serial Nos 61/836,101 and 61/835,936 (Attorney docket 44790.09.2022 and 4790.07.2022 and Broad Reference BI-2013/004E and BI-2013/004F respectively).

*Example 7: Methodological improvement to simplify cloning and delivery.*

Rather than encoding the U6-promoter and guide RNA on a plasmid, Applicants amplified the U6 promoter with a DNA oligo to add on the guide RNA. The resulting PCR product may be transfected into cells to drive expression of the guide RNA.

Example primer pair that allows the generation a PCR product consisting of U6-promoter::guideRNA targeting human Emx1 locus:

Forward Primer: AAACTCTAGAgagggcctatttcccatgattc

Reverse Primer (carrying the guide RNA, which is underlined):

acctctagAAAAAAAGCACCGACTCGGTGCCACTTTTTCAAGTTGATAACGGACTA

GC

CTTATTTTAACTTGCTATGCTGTTTTGTTTCCAAAACAGCATAGCTCTAAAACC

CC TAGTCATTGGAGGTGACGGTGTTTCGTCCTTTCCACaag

*Example 8: Methodological improvement to improve activity:*

Rather than use pol3 promoters, in particular RNA polymerase III (e.g. U6 or H1 promoters), to express guide RNAs in eukaryotic cells, Applicants express the T7 polymerase in eukaryotic cells to drive expression of guide RNAs using the T7 promoter.

One example of this system may involve introduction of three pieces of DNA:

1. expression vector for Cas9
2. expression vector for T7 polymerase
3. expression vector containing guideRNA fused to the T7 promoter

*Example 9: Methodological improvement to reduce toxicity of Cas9: Delivery of Cas9 in the form of mRNA.*

Delivery of Cas9 in the form of mRNA enables transient expression of Cas9 in cells, to reduce toxicity. For example, humanized SpCas9 may be amplified using the following primer pair:

Forward Primer (to add on T7 promoter for in vitro transcription):
TAATACGACTCACTATAGGAAGTGCGCCACCATGGCCCCAAAGAAGAAGCGG

Reverse Primer (to add on polyA tail):
GGTTTTTTTTTTTTTTTTTTTTTTTTTTTTTTTTTtttcttaCTTTTTCTTTTTTGCCTGGCCG

Applicants transfect the Cas9 mRNA into cells with either guide RNA in the form of RNA or DNA cassettes to drive guide RNA expression in eukaryotic cells.

*Example 10: Methodological improvement to reduce toxicity of Cas9: Use of an inducible promoter*

Applicants transiently turn on Cas9 expression only when it is needed for carrying out genome modification. Examples of inducible system include tetracycline inducible promoters (Tet-On or Tet-Off), small molecule two-hybrid transcription activations systems (FKBP, ABA, etc), or light inducible systems (Phytochrome, LOV domains, or cryptochrome).

*Example 11: Improvement of the Cas9 system for in vivo application*

Applicants conducted a Metagenomic search for a Cas9 with small molecular weight. Most Cas9 homologs are fairly large. For example the SpCas9 is around 1368aa long, which is too large to be easily packaged into viral vectors for delivery. A graph representing the length distribution of Cas9 homologs is generated from sequences deposited in GenBank (Fig. 23). Some of the sequences may have been mis-annotated and therefore the exact frequency for each length may not necessarily be accurate. Nevertheless it provides a glimpse at distribution of Cas9 proteins and suggest that there are shorter Cas9 homologs.

Through computational analysis, Applicants found that in the bacterial strain *Campylobacter,* there are two Cas9 proteins with less than 1000 amino acids. The sequence for one Cas9 from *Campylobacter jejuni* is presented below. At this length, CjCas9 can be easily packaged into AAV, lentiviruses, Adenoviruses, and other viral vectors for robust delivery into primary cells and *in vivo* in animal models. In a preferred embodiment the Cas9 protein from *S. aureus* is used.

>Campylobacter jejuni Cas9 (CjCas9)

MARILAFDIGISSIGWAFSENDELKDCGVRIFTKVENPKTGESLALPRRL
AR
SARKRLARRKARLNHLKHLIANEFKLNYEDYQSFDESLAKAYKGSLISPYELRFR
ALN
ELLSKQDFARVILHIAKRRGYDDIKNSDDKEKGAILKAIKQNEEKLANYQSVGEY
LYK
EYFQKFKENSKEFTNVRNKKESYERCIAQSFLKDELKLIFKKQREFGFSFSKKFEEE

VL

SVAFYKRALKDFSHLVGNCSFFTDEKRAPKNSPLAFMFVALTRIINLLNNLKNTEG

IL

YTKDDLNALLNEVLKNGTLTYKQTKKLLGLSDDYEFKGEKGTYFIEFKKYKEFIK

AL

GEHNLSQDDLNEIAKDITLIKDEIKLKKALAKYDLNQNQIDSLSKLEFKDHLNISFK

AL

KLVTPLMLEGKKYDEACNELNLKVAINEDKKDFLPAFNETYYKDEVTNPVVLRA

IKE

YRKVLNALLKKYGKVHKINIELAREVGKNHSQRAKIEKEQNENYKAKKDAELEC

EK

LGLKINSKNILKLRLFKEQKEFCAYSGEKIKISDLQDEKMLEIDHIYPYSRSFDDSY

MN

KVLVFTKQNQEKLNQTPFEAFGNDSAKWQKIEVLAKNLPTKKQKRILDKNYKDK

EQ

KNFKDRNLNDTRYIARLVLNYTKDYLDFLPLSDDENTKLNDTQKGSKVHVEAKS

GM

LTSALRHTWGFSAKDRNNHLHHAIDAVIIAYANNSIVKAFSDFKKEQESNSAELY

AK

KISELDYKNRKFFEPFSGFRQKVLDKIDEIFVSKPERKKPSGALHEETFRKEEEFY

QS

YGGKEGVLKALELGKIRKVNGKIVKNGDMFRVDIFKHKKTNKFYAVPIYTMDFA

LK

VLPNKAVARSKKGEIKDWILMDENYEFCFSLYKDSLILIQTKDMQEPEFVYYNAF

TSS

TVSLIVSKHDNKFETLSKNQKILFKNANEKEVIAKSIGIQNLKVFEKYIVSALGEVT

KA EFRQREDFKK.

The putative tracrRNA element for this CjCas9 is:

TATAATCTCATAAGAAATTTAAAAAGGGACTAAAATAAAGAGTTTG
CG GGACTCTGCGGGGTTACAATCCCCTAAAACCGCTTTTAAAATT

The Direct Repeat sequence is:
ATTTTACCATAAAGAAATTTAAAAAGGGACTAAAAC
An example of a chimeric guideRNA for CjCas9 is:

NNNNNNNNNNNNNNNNNNNNNNGUUUUAGUCCCGAAAGGGACUAAAAU

AAAGAGUUUGCGGGACUCUGCGGGGUUACAAUCCCCUAAAACCGCUUUU

*Example 12: Cas9 optimization*

For enhanced function or to develop new functions, Applicants generate chimeric Cas9 proteins by combining fragments from different Cas9 homologs. For example, two example chimeric Cas9 proteins:

For example, Applicants fused the N-term of St1Cas9 (fragment from this protein is in bold) with C-term of SpCas9 (fragment from this protein is underlined).

>Stl(N)Sp(C)Cas9

**MSDLVLGLDIGIGSVGVGILNKVTGEIIHKNSRIFPAAQAENNLVRRTN**

**RQGRRLARRKKHRRVRLNRLFEESGLITDFTKISINLNPYQLRVKGLTDELSNEE**

**LFIALKNMVKHRGISYLDDASDDGNSSVGDYAQIVKENSKQLETKTPGQIQLERY**

**QTYGQLRGDFTVEKDGKKHRLINVFPTSAYRSEALRILQTQQEFNPQITDEFINR**

**YLEILTGKRKYYHGPGNEKSRTDYGRYRTSGETLDNIFGILIGKCTFYPDEFRAAK**

**ASYTAQEFNLLNDLNNLTVPTETKKLSKEQKNQIINYVKNEKAMGPAKLFKYIAK**

**LLSCDVADIKGYRIDKSGKAEIHTFEAYRKMKTLETLDIEQMDRETLDKLAYVLT**

**LNTEREGIQEALEHEFADGSFSQKQVDELVQFRKANSSIFGKGWHNFSVKLMME**

**LIPELYETSEEQMTILTRLGKQKTTSSSNKTKYIDEKLLTEEIYNPVVAKSVRQAIK**

**IVNAAIKEYGDFDNIVIEMARE**NQTTQKGQKNSRERMKRIEEGIKELGSQILKEHPVE

NTQLQNEKLYLYYLQNGRDMYVDQELDINRLSDYDVDHIVPQSFLKDDSIDNKVLT

RSDKNRGKSDNVPSEEVVKKMKNYWRQLLNAKLITQRKFDNLTKAERGGLSEL

DK

AGFIKRQLVETRQITKHVAQILDSRMNTKYDENDKLIREVKVITLKSKLVSDFRKD
FQ

FYKVREINNYHHAHDAYLNAVVGTALIKKYPKLESEFVYGDYKVYDVRKMIAKS
EQ

EIGKATAKYFFYSNIMNFFKTEITLANGEIRKRPLIETNGETGEIVWDKGRDFATVR
KV

LSMPQVNIVKKTEVQTGGFSKESILPKRNSDKLIARKKDWDPKKYGGFDSPTVAY
SV

LVVAKVEKGKSKKLKSVKELLGITIMERSSFEKNPIDFLEAKGYKEVKKDLIIKLP
KYS

LFELENGRKRMLASAGELQKGNELALPSKYVNFLYLASHYEKLKGSPEDNEQKQ
LF

VEQHKHYLDEIIEQISEFSKRVILADANLDKVLSAYNKHRDKPIREQAENIIHLFTL
TNL GAPAAFKYFDTTIDRKRYTSTKEVLDATLIHQSITGLYETRIDLSQLGGD

>Sp(N)Stl(C)Cas9

MDKKYSIGLDIGTNSVGWAVITDEYKVPSKKFKVLGNTDRHSIKKNLI
GA

LLFDSGETAEATRLKRTARRRYTRRKNRICYLQEIFSNEMAKVDDSFFHRLEESFL
VE

EDKKHERHPIFGNIVDEVAYHEKYPTIYHLRKKLVDSTDKADLRLIYLALAHMIKF
RG

HFLIEGDLNPDNSDVDKLFIQLVQTYNQLFEENPINASGVDAKAILSARLSKSRRLE
NL

IAQLPGEKKNGLFGNLIALSLGLTPNFKSNFDLAEDAKLQLSKDTYDDDLDNLLA
QIG

DQYADLFLAAKNLSDAILLSDILRVNTEITKAPLSASMIKRYDEHHQDLTLLKALV
RQ

QLPEKYKEIFFDQSKNGYAGYIDGGASQEEFYKFIKPILEKMDGTEELLVKLNRED
LL

RKQRTFDNGSIPHQIHLGELHAILRRQEDFYPFLKDNREKIEKILTFRIPYYVGPLAR
GN

SRFAWMTRKSEETITPWNFEEVVDKGASAQSFIERMTNFDKNLPNEKVLPKHSLL
YE

92

YFTVYNELTKVKYVTEGMRKPAFLSGEQKKAIVDLLFKTNRKVTVKQLKEDYFKKIE

CFDSVEISGVEDRFNASLGTYHDLLKIIKDKDFLDNEENEDILEDIVLTLTLFEDREMIE

ERLKTYAHLFDDKVMKQLKRRRYTGWGRLSRKLINGIRDKQSGKTILDFLKSDGFAN

RNFMQLIHDDSLTFKEDIQKAQVSGQGDSLHEHIANLAGSPAIKKGILQTVKVVDELV

KVMGRHKPENIVIEMARETNEDDEKKAIQKIQKANKDEKDAAMLKAANQYNGKA

ELPHSVFHGHKQLATKIRLWHQQGERCLYTGKTISIHDLINNSNQFEVDHILPLSI

TFDDSLANKVLVYATANQEKGQRTPYQALDSMDDAWSFRELKAFVRESKTLSNK

KKEYLLTEEDISKFDVRKKFIERNLVDTRYASRVVLNALQEHFRAHKIDTKVSVVR

GQFTSQLRRHWGIEKTRDTYHHHAVDALIIAASSQLNLWKKQKNTLVSYSEDQL

LDIETGELISDDEYKESVFKAPYQHFVDTLKSKEFEDSILFSYQVDSKFNRKISDATI

YATRQAKVGKDKADETYVLGKIKDIYTQDGYDAFMKIYKKDKSKFLMYRHDPQT

FEKVIEPILENYPNKQINEKGKEVPCNPFLKYKEEHGYIRKYSKKGNGPEIKSLKYY

DSKLGNHIDITPKDSNNKVVLQSVSPWRADVYFNKTTGKYEILGLKYADLQFEKG

TGTYKISQEKYNDIKKKEGVDSDSEFKFTLYKNDLLLVKDTETKEQQLFRFLSRT

MPKQKHYVELKPYDKQKFEGGEALIKVLGNVANSGQCKKGLGKSNISIYKVRTD VLGNQHIIKNEGDKPKLDF

The benefit of making chimeric Cas9 include:

reduce toxicity
improve expression in eukaryotic cells
enhance specificity
reduce molecular weight of protein, make protein smaller by combining the smallest domains from different Cas9 homologs.

Altering the PAM sequence requirement

*Example 13: Utilization of Cas9 as a generic DNA binding protein*

Applicants used Cas9 as a generic DNA binding protein by mutating the two catalytic domains (D10 and H840) responsible for cleaving both strands of the DNA target. In order to upregulate gene transcription at a target locus Applicants fused the transcriptional activation domain (VP64) to Cas9. Applicants hypothesized that it would be important to see strong nuclear localization of the Cas9-VP64 fusion protein because transcription factor activation strength is a function of time spent at the target. Therefore, Applicants cloned a set of Cas9-VP64-GFP constructs, transfected them into 293 cells and assessed their localization under a fluorescent microscope 12 hours post-transfection.

The same constructs were cloned as a 2A-GFP rather than a direct fusion in order to functionally test the constructs without a bulky GFP present to interfere. Applicants elected to target the Sox2 locus with the Cas9 transactivator because it could be useful for cellular reprogram and the locus has already been validated as a target for TALE-TF mediated transcriptional activation. For the Sox2 locus Applicants chose eight targets near the transcriptional start site (TSS). Each target was 20bp long with a neighboring NGG protospacer adjacent motif (PAM). Each Cas9-VP64 construct was co-transfected with each PCR generated chimeric crispr RNA (chiRNA) in 293 cells. 72 hours post transfection the transcriptional activation was assessed using RT-qPCR.

To further optimize the transcriptional activator, Applicants titrated the ratio of chiRNA (Sox2.1 and Sox2.5) to Cas9 (NLS-VP64-NLS-hSpCas9-NLS-VP64-NLS), transfected into 293 cells, and quantified using RT-qPCR. These results indicate that Cas9 can be used as a generic DNA binding domain to upregulate gene transcription at a target locus.

Applicants designed a second generation of constructs. (Table below).

| pLenti-EF1a-GFP-2A-6xHis-NLS-VP64-NLS-hSpCsn1(D10A, H840A)-NLS |
| --- |
| pLenti-EF1a-GFP-2A-6xHis-NLS-VP64-NLS-hSpCsn1(D10A, H840A) |
| pLenti-EF1a-GFP-2A-6xHis-NLS-VP64-NLS-NLS-hSpCsn1(D10A, H840A) |
| pLenti-EF1a-GFP-2A-6xHis-NLS-hSpCsn1(D10A, H840A)-NLS |
| pLenti-EF1a-GFP-2A-6xHis-NLS-hSpCsn1(D10A, H840A) |
| pLenti-EF1a-GFP-2A-6xHis-NLS-NLS-hSpCsn1(D10A, H840A) |

Applicants use these constructs to assess transcriptional activation (VP64 fused constructs) and repression (Cas9 only) by RT-qPCR. Applicants assess the cellular localization of each construct using anti-His antibody, nuclease activity using a Surveyor nuclease assay, and DNA binding affinity using a gel shift assay. In a preferred embodiment the gel shift assay is an EMSA gel shift assay.

*Example 14: Cas9 transgenic and knock in mice*

To generate a mouse that expresses the Cas9 nuclease Applicants submit two general strategies, transgenic and knock in. These strategies may be applied to generate any other model organism of interest, for e.g. Rat. For each of the general strategies Applicants made a constitutively active Cas9 and a Cas9 that is conditionally expressed (Cre recombinase dependent). The constitutively active Cas9 nuclease is expressed in the following context: pCAG-NLS-Cas9-NLS-P2A-EGFP-WPRE-bGHpolyA. pCAG is the promoter, NLS is a nuclear localization signal, P2A is the peptide cleavage sequence, EGFP is enhanced green fluorescent protein, WPRE is the woodchuck hepatitis virus posttranscriptional regulatory element, and bGHpolyA is the bovine growth hormone poly-A signal sequence (Figs. 25A-B). The conditional version has one additional stop cassette element, loxP-SV40 polyA x3-loxP, after the promoter and before NLS-Cas9-NLS (i.e. pCAG-loxP-SV40polyAx3-loxP-NLS-Cas9-NLS-P2A-EGFP-WPRE-bGHpolyA). The important expression elements can be visualized as in Fig. 26. The constitutive construct should be expressed in all cell types throughout development, whereas, the conditional construct will only allow Cas9 expression when the same cell is expressing the Cre recombinase. This latter version will allow for tissue specific expression of Cas9 when Cre is under the expression of a tissue specific promoter. Moreover, Cas9 expression could be induced in adult mice by putting Cre under the expression of an inducible promoter such as the TET on or off system.

Validation of Cas9 constructs: Each plasmid was functionally validated in three ways: 1) transient transfection in 293 cells followed by confirmation of GFP expression; 2) transient transfection in 293 cells followed by immunofluorescence using an antibody recognizing the P2A sequence; and 3) transient transfection followed by Surveyor nuclease assay. The 293 cells may be 293FT or 293 T cells depending on the cells that are of interest. In a preferred embodiment the cells are 293FT cells. The results of the Surveyor were run out on the top and bottom row of the gel for the conditional and constitutive constructs, respectively. Each was tested in the presence and absence of chimeric RNA targeted to the hEMX1 locus (chimeric RNA hEMX1.1). The results indicate that the construct can successfully target the hEMX1 locus only in the presence of chimeric RNA (and Cre in the conditional case). The gel was quantified and the results are presented as average cutting efficiency and standard deviation for three samples.

Transgenic Cas9 mouse: To generate transgenic mice with constructs, Applicants inject pure, linear DNA into the pronucleus of a zygote from a pseudo pregnant CB56 female. Founders are identified, genotyped, and backcrossed to CB57 mice. The constructs were successfully cloned and verified by Sanger sequencing.

Knock in Cas9 mouse: To generate Cas9 knock in mice Applicants target the same constitutive and conditional constructs to the Rosa26 locus. Applicants did this by cloning each into a Rosa26 targeting vector with the following elements: Rosa26 short homology arm - constitutive/conditional Cas9 expression cassette - pPGK-Neo-Rosa26 long homology arm - pPGK-DTA. pPGK is the promoter for the positive selection marker Neo, which confers resistance to neomycin, a 1 kb short arm, a 4.3 kb long arm, and a negative selection diphtheria toxin (DTA) driven by PGK.

The two constructs were electroporated into R1 mESCs and allowed to grow for 2 days before neomycin selection was applied. Individual colonies that had survived by days 5-7 were picked and grown in individual wells. 5-7 days later the colonies were harvested, half were frozen and the other half were used for genotyping. Genotyping was done by genomic PCR, where one primer annealed within the donor plasmid (AttpF) and the other outside of the short homology arm (Rosa26-R) Of the 22 colonies harvested for the conditional case, 7 were positive (Left). Of the 27 colonies harvested for the constitutive case, zero were positive (Right). It is likely that Cas9 causes some level of toxicity in the mESC and for this reason there were no positive clones. To test this Applicants introduced a Cre expression plasmid into correctly targeted conditional Cas9 cells and found very low toxicity after many days in culture. The reduced copy number of Cas9 in correctly targeted conditional Cas9 cells (1-2 copies per cell) is enough to allow stable expression and relatively no cytotoxicity. Moreover, this data indicates that the Cas9 copy number determines toxicity. After electroporation each cell should get several copies of Cas9 and this is likely why no positive colonies were found in the case of the constitutive Cas9 construct. This provides strong evidence that utilizing a conditional, Cre-dependent strategy should show reduced toxicity. Applicants inject correctly targeted cells into a blastocyst and implant into a female mouse. Chimerics are identified and backcrossed. Founders are identified and genotyped.

Utility of the conditional Cas9 mouse: Applicants have shown in 293 cells that the Cas9 conditional expression construct can be activated by co-expression with Cre. Applicants also show that the correctly targeted R1 mESCs can have active Cas9 when Cre is expressed. Because Cas9 is followed by the P2A peptide cleavage sequence and then EGFP Applicants identify successful expression by observing EGFP. This same concept is what makes the conditional Cas9 mouse so useful. Applicants may cross their conditional Cas9 mouse with a mouse that ubiquitously expresses Cre (ACTB-Cre line) and may arrive at a mouse that expresses Cas9 in every cell. It should only take the delivery of chimeric RNA to induce genome editing in embryonic or adult mice. Interestingly, if the conditional Cas9 mouse is crossed with a mouse expressing Cre under a tissue specific promoter, there should only be Cas9 in the tissues that also express Cre. This approach may be used to edit the genome in only precise tissues by delivering chimeric RNA to the same tissue.

*Example 15: Cas9 diversity and chimeric RNAs*

The CRISPR-Cas system is an adaptive immune mechanism against invading exogenous DNA employed by diverse species across bacteria and archaea. The type II CRISPR-Cas system consists of a set of genes encoding proteins responsible for the "acquisition" of foreign DNA into the CRISPR locus, as well as a set of genes encoding the "execution" of the DNA cleavage mechanism; these include the DNA nuclease (Cas9), a non-coding transactivating cr-RNA (tracrRNA), and an array of foreign DNA-derived spacers flanked by direct repeats (crRNAs). Upon maturation by Cas9, the tracrRNA and crRNA duplex guide the Cas9 nuclease to a target DNA sequence specified by the spacer guide sequences, and mediates double-stranded breaks in the DNA near a short sequence motif in the target DNA that is required for cleavage and specific to each CRISPR-Cas system. The type II CRISPR-Cas systems are found throughout the bacterial kingdom and highly diverse in in Cas9 protein sequence and size, tracrRNA and crRNA direct repeat sequence, genome organization of these elements, and the motif requirement for target cleavage. One species may have multiple distinct CRISPR-Cas systems.

Applicants evaluated 207 putative Cas9s from bacterial species identified based on sequence homology to known Cas9s and structures orthologous to known subdomains, including the HNH endonuclease domain and the RuvC endonuclease domains [information from the Eugene Koonin and Kira Makarova]. Phylogenetic analysis based on the protein sequence conservation of this set revealed five families of Cas9s, including three groups of large Cas9s (~1400 amino acids) and two of small Cas9s (~1100 amino acids) (Figs. 19A-D and 20A-F).

Applicants have also optimized Cas9 guide RNA using *in vitro* methods.

*Example 16: Cas9 mutations*

In this example, Applicants show that the following mutations can convert SpCas9 into a nicking enzyme: D10A, E762A, H840A, N854A, N863A, D986A.

Applicants provide sequences showing where the mutation points are located within the SpCas9 gene (Fig. 24A-M). Applicants also show that the nickases are still able to mediate homologous recombination. Furthermore, Applicants show that SpCas9 with these mutations (individually) do not induce double strand break.

Cas9 orthologs all share the general organization of 3-4 RuvC domains and a HNH domain. The 5' most RuvC domain cleaves the non-complementary strand, and the HNH domain cleaves the complementary strand. All notations are in reference to the guide sequence.

The catalytic residue in the 5' RuvC domain is identified through homology comparison of the Cas9 of interest with other Cas9 orthologs (from S. pyogenes type II CRISPR locus, S. thermophilus CRISPR locus 1, S. thermophilus CRISPR locus 3, and Franciscilla novicida type II CRISPR locus), and the conserved Asp residue is mutated to alanine to convert Cas9 into a complementary-strand nicking enzyme. Similarly, the conserved His and Asn residues in the HNH domains are mutated to Alanine to convert Cas9 into a non-complementary-strand nicking enzyme.

*Example 17: Cas9 Transcriptional Activation and Cas9 Repressor*

Cas9 Transcriptional Activation

A second generation of constructs have been designed and are in the pipeline to be tested (Table 1). These constructs will be used to assess transcriptional activation (VP64 fused constructs) and repression (Cas9 only) by RT-qPCR. Applicants will also assess the cellular localization of each construct using anti-His antibody,, nuclease activity using a Surveyor nuclease assay, and DNA binding affinity using a gel shift assay.

Cas Repressor

It has been shown previously that dCas9 can be used as a generic DNA binding domain to repress gene expression. Applicants report an improved dCas9 design as well as dCas9 fusions to the repressor domains KRAB and SID4x. From the plasmid library created for modulating transcription using Cas9 in Table 1, the following repressor plasmids were functionally characterized by qPCR: pXRP27, pXRP28, pXRP29, pXRP48, pXRP49, pXRP50, pXRP51, pXRP52, pXRP53, pXRP56, pXRP58, pXRP59, pXRP61, and pXRP62.

Each dCas9 repressor plasmid was co-transfected with two guide RNAs targeted to the coding strand of the beta-catenin gene. RNA was isolated 72 hours after transfection and gene expression was quantified by RT-qPCR. The endogenous control gene was GAPDH. Two validated shRNAs were used as positive controls. Negative controls were certain plasmids transfected without gRNA, these are denoted as "pXRP## control". The plasmids pXRP28, pXRP29, pXRP48, and pXRP49 could repress the beta-catenin gene when using the specified targeting strategy. These plasmids correspond to dCas9 without a functional domain (pXRP28 and pXRP28) and dCas9 fused to SID4x (pXRP48 and pXRP49).

Further work investigates: repeating the above experiment, targeting different genes, utilizing other gRNAs to determine the optimal targeting position, and multiplexed repression.

Table 1

| |
|---|
| pXRP024-pLenti2-EF1a-VP64-NLS-FLAG-Linker-dCas9-NLS-gLuc-2A-GFP-WPRE |
| pXRP025-pLenti2-EF1a-VP64-NLS-GGGGS$_3$Linker-dCas9-NLS-gLuc-2A-GFP-WPRE |
| pXRP026-pLenti2-EF1a-VP64-NLS-EAAAK$_3$Linker-dCas9-NLS-gLuc-2A-GFP-WPRE |
| pXRP027-pLenti2-EF1a-NLS-FLAG-Linker-dCas9-NLS-gLuc-2A-GFP-WPRE |
| pXRP028-pLenti2-EF1a-NLS-GGGGS$_3$Linker-dCas9-NLS-gLuc-2A-GFP-WPRE |
| pXRP029-pLenti2-EF1a-NLS-EAAAK$_3$Linker-dCas9-NLS-gLuc-2A-GFP-WPRE |
| pXRP030-pLenti2-pSV40-VP64-NLS-FLAG-Linker-dCas9-NLS-gLuc-2A-GFP-WPRE |
| pXRP031-pLenti2-pPGK-VP64-NLS-FLAG-Linker-dCas9-NLS-gLuc-2A-GFP-WPRE |
| pXRP032-pLenti2-LTR-VP64-NLS-FLAG-Linker-dCas9-NLS-gLuc-2A-GFP-WPRE |
| pXRP033-pLenti2-pSV40-VP64-NLS-GGGGS$_3$Linker-dCas9-NLS-gLuc-2A-GFP-WPRE |

(continued)

| |
|---|
| pXRP034-pLenti2-pPGK-VP64-NLS-GGGGS$_3$Linker-dCas9-NLS-gLuc-2A-GFP-WPRE |
| pXRP035-pLenti2-LTR-VP64-NLS-GGGGS$_3$Linker-dCas9-NLS-gLuc-2A-GFP-WPRE |
| pXRP036-pLenti2-pSV40-VP64-NLS-EAAAK$_3$Linker-dCas9-NLS-gLuc-2A-GFP-WPRE |
| pXRP037-pLenti2-pPGK-VP64-NLS-EAAAK$_3$Linker-dCas9-NLS-gLuc-2A-GFP-WPRE |
| pXRP038-pLenti2-LTR-VP64-NLS-EAAAK$_3$Linker-dCas9-NLS-gLuc-2A-GFP-WPRE |
| pXRP048-pLenti2-EF1a-SID4x-NLS-FLAG-Linker-dCas9-NLS-gLuc-2A-GFP-WPRE |
| pXRP049-pLenti2-EF1a-SID4X-NLS-GGGGS$_3$Linker-dCas9-NLS-gLuc-2A-GFP-WPRE |
| pXRP050-pLenti2-EF1a-SID4X-NLS-EAAAK$_3$Linker-dCas9-NLS-gLuc-2A-GFP-WPRE |
| pXRP051-pLenti2-EF1a-KRAB-NLS-FLAG-Linker-dCas9-NLS-gLuc-2A-GFP-WPRE |
| pXRPOS2-pLenti2-EFla-KRAB-NLS-GGGGS$_3$Linker-dCas9-NLS-gLuc-2A-GFP-WPRE |
| pXRP053-pLenti2-EF1a-KRAB-NLS-EAAAK$_3$Linker-dCas9-NLS-gLuc-2A-GFP-WPRE |
| pXRP054-pLenti2-EF1a-dCas9-Linker-FLAG-NLS-VP64-gLuc-2A-GFP-WPRE |
| pXRP055-pLenti2-EF1a-dCas9-Linker-FLAG-NLS-SID4X-gLuc-2A-GFP-WPRE |
| pXRP056-pLenti2-EF1a-dCas9-Linker-FLAG-NLS-KRAB-gLuc-2A-GFP-WPRE |
| pXRP057-pLenti2-EF1a-dCas9-GGGGGS$_3$-NLS-VP64-gLuc-2A-GFP-WPRE |
| pXRP058-pLenti2-EF1a-dCas9-GGGGGS$_3$-NLS-SID4X-gLuc-2A-GFP-WPRE |
| pXRP059-pLenti2-EF1a-dCas9-GGGGGS$_3$-NLS-KRAB-gLuc-2A-GFP-WPRE |
| pXRP060-pLenti2-EF1a-dCas9-EAAAK$_3$-NLS-VP64-gLuc-2A-GFP-WPRE |
| pXRP061-pLenti2-EF1a-dCas9-EAAAK$_3$-NLS-SID4X-gLuc-2A-GFP-WPRE |
| pXRP062-pLenti2-EF1a-dCas9-EAAAK$_3$-NLS-KRAB-gLuc-2A-GFP-WPRE |
| pXRP024-pLenti2-EF la-VP64-NLS-FLAG-Linker-Cas9-NLS-gLuc-2A-GFP-WPRE |
| pXRP025-pLenti2-EF1a-VP64-NLS-GGGGS$_3$Linker-Cas9-NLS-gLuc-2A-GFP-WPRE |
| pXRP026-pLenti2-EF1a-VP64-NLS-EAAAK$_3$Linker-Cas9-NLS-gLuc-2A-GFP-WPRE |
| pXRP027-pLenti2-EF1a-NLS-FLAG-Linker-Cas9-NLS-gLuc-2A-GFP-WPRE |
| pXRP028-pLenti2-EF1a-NLS-GGGGS$_3$Linker-Cas9-NLS-gLuc-2A-GFP-WPRE |
| pXRP029-pLenti2-EF1a-NLS-EAAAK$_3$Linker-Cas9-NLS-gLuc-2A-GFP-WPRE |
| pXRP030-pLenti2-pSV40-VP64-NLS-FLAG-Linker-Cas9-NLS-gLuc-2A-GFP-WPRE |
| pXRP031-pLenti2-pPGK-VP64-NLS-FLAG-Linker-Cas9-NLS-gLuc-2A-GFP-WPRE |
| pXRP032-pLenti2-LTR-VP64-NLS-FLAG-Linker-Cas9-NLS-gLuc-2A-GFP-WPRE |
| pXRP033-pLenti2-pSV40-VP64-NLS-GGGGS$_3$Linker-Cas9-NLS-gLuc-2A-GFP-WPRE |
| pXRP034-pLenti2-pPGK-VP64-NLS-GGGGS$_3$Linker-Cas9-NLS-gLuc-2A-GFP-WPRE |
| pXRP035-pLenti2-LTR-VP64-NLS-GGGGS$_3$Linker-Cas9-NLS-gLuc-2A-GFP-WPRE |
| pXRP036-pLenti2-pSV40-VP64-NLS-EAAAK$_3$Linker-Cas9-NLS-gLuc-2A-GFP-WPRE |
| pXRP037-pLenti2-pPGK-VP64-NLS-EAAAK$_3$Linker-Cas9-NLS-gLuc-2A-GFP-WPRE |
| pXRP038-pLenti2-LTR-VP64-NLS-EAAAK$_3$Linker-Cas9-NLS-gLuc-2A-GFP-WPRE |
| pXRP048-pLenti2-EF1a-SID4x-NLS-FLAG-Linker-Cas9-NLS-gLuc-2A-GFP-WPRE |
| pXRP049-pLenti2-EF1a-SID4X-NLS-GGGGS$_3$Linker-Cas9-NLS-gLuc-2A-GFP-WPRE |
| pXRP050-pLenti2-EF1a-SID4X-NLS-EAAAK$_3$Linker-Cas9-NLS-gLuc-2A-GFP-WPRE |
| pXRP051-pLenti2-EF1a-KRAB-NLS-FLAG-Linker-Cas9-NLS-gLuc-2A-GFP-WPRE |

(continued)

| |
|---|
| pXRP052-pLenti2-EF1a-KRAB-NLS-GGGGS₃Linker-Cas9-NLS-gLuc-2A-GFP-WPRE |
| pXRP053-pLenti2-EF1a-KRAB-NLS-EAAAK₃Linker-Cas9-NLS-gLuc-2A-GFP-WPRE |
| pXRP054-pLenti2-EF1a-Cas9-Linker-FLAG-NLS-VP64-gLuc-2A-GFP-WPRE |
| pXRP055-pLenti2-EF1a-Cas9-Linker-FLAG-NLS-SID4X-gLuc-2A-GFP-WPRE |
| pXRP056-pLenti2-EF1a-Cas9-Linker-FLAG-NLS-KRAB-gLuc-2A-GFP-WPRE |
| pXRP057-pLenti2-EF1a-Cas9-GGGGGS₃-NLS-VP64-gLuc-2A-GFP-WPRE |
| pXRP058-pLenti2-EF1a-Cas9-GGGGGS₃-NLS-SID4X-gLuc-2A-GFP-WPRE |
| pXRP059-pLenti2-EF1a-Cas9-GGGGGS₃-NLS-KRAB-gLuc-2A-GFP-WPRE |
| pXRP060-pLenti2-EF1a-Cas9-EAAAK₃-NLS-VP64-gLuc-2A-GFP-WPRE |
| pXRP061-pLenti2-EF1a-Cas9-EAAAK₃-NLS-SID4X-gLuc-2A-GFP-WPRE |
| pXRP062-pLenti2-EF1a-Cas9-EAAAK₃-NLS-KRAB-gLuc-2A-GFP-WPRE |

*Example 18: Targeted deletion of genes involved in cholesterol biosynthesis, fatty acid biosynthesis, and other metabolic disorders, genes encoding mis-folded proteins involved in amyloid and other diseases, oncogenes leading to cellular transformation, latent viral genes, and genes leading to dominant-negative disorders, amongst other disorders.*

Applicants demonstrate gene delivery of a CRISPR-Cas system in the liver, brain, ocular, epithelial, hematopoetic, or another tissue of an organism, a subject or a patient in need thereof, suffering from metabolic disorders, amyloidosis and protein-aggregation related diseases, cellular transformation arising from genetic mutations and translocations, dominant negative effects of gene mutations, latent viral infections, and other related symptoms, using either viral or nanoparticle delivery system.

**Study Design:** Organisms or subjects or patients in need thereof suffering from metabolic disorders, amyloidosis and protein aggregation related disease which include but are not limited to human, non-primate human, canine, feline, bovine, equine, other domestic animals and related mammals. The CRISPR-Cas system is guided by a chimeric guide RNA and targets a specific site of the human genomic loci to be cleaved. After cleavage and non-homologous end-joining mediated repair, frame-shift mutation results in knock out of genes.

Applicants select guide-RNAs targeting genes involved in above-mentioned disorders to be specific to endogenous loci with minimal off-target activity. Two or more guide RNAs may be encoded into a single CRISPR array to induce simultaneous double-stranded breaks in DNA leading to micro-deletions of affected genes or chromosomal regions.

Identification and design of gene targets

For each candidate disease gene, Applicants select DNA sequences of interest include protein-coding exons, sequences including and flanking known dominant negative mutation sites, sequences including and flanking pathological repetitive sequences. For gene-knockout approaches, early coding exons closest to the start codon offer best options for achieving complete knockout and minimize possibility of truncated protein products retaining partial function.

Applicants analyze sequences of interest for all possible targetable 20-bp sequences immediately 5' to a NGG motif (for SpCas9 system) or a NNAGAAW (for StlCas9 system). Applicants choose sequences for unique, single RNA-guided Cas9 recognition in the genome to minimize off-target effects based on computational algorithm to determine specificity.

Cloning of guide sequences into a delivery system

Guide sequences are synthesized as double-stranded 20-24 bp oligonucleotides. After 5'-phosphorylation treatment of oligos and annealing to form duplexes, oligos are ligated into suitable vector depending on the delivery method:

Virus-based delivery methods
AAV-based vectors (PX260, 330, 334, 335) have been described elsewhere
Lentiviral-based vectors use a similar cloning strategy of directly ligating guide sequences into a single vector carrying a U6 promoter-driven chimeric RNA scaffold and a EF1a promoter-driven Cas9 or Cas9 nickase.

Virus production is described elsewhere.

Nanoparticle-based RNA delivery methods

1. Guide sequences are synthesized as an oligonucleotide duplex encoding T7 promoter-guide sequence-chimeric RNA. A T7 promoter is added 5' of Cas9 by PCR method.
2. T7-driven Cas9 and guide-chimeric RNAs are transcribed *in vitro,* and Cas9 mRNA is further capped and A-tailed using commercial kits. RNA products are purified per kit instructions.

Hydrodynamic tail vein delivery methods (for mouse)

Guide sequences are cloned into AAV plasmids as described above and elsewhere in this application.

*In vitro* validation on cell lines

Transfection

1. DNA plasmid transfection

Plasmids carrying guide sequences are transfected into human embryonic kidney (HEK293T) or human embryonic stem (hES) cells, other relevant cell types using lipid-, chemical-, or electroporation-based methods. For a 24-well transfection of HEK293T cells (~260,000 cells), 500ng of total DNA is transfected into each single well using Lipofectamine 2000. For a 12-well transfection of hES cells, lug of total DNA is transfected into a single well using Fugene HD.

2. RNA transfection

Purified RNA described above is used for transfection into HEK293T cells. 1-2ug of RNA may be transfected into ~260,000 using Lipofectamine 2000 per manufacturer's instruction. RNA delivery of Cas9 and chimeric RNA is shown in Fig. 28.

Assay of indel formation *in vitro*

Cells are harvested 72-hours post-transfection and assayed for indel formation as an indication of double-stranded breaks.

Briefly, genomic region around target sequence is PCR amplified (~400-600 bp amplicon size) using high-fidelity polymerase. Products are purified, normalized to equal concentration, and slowly annealed from 95°C to 4°C to allow formation of DNA heteroduplexes. Post annealing, the Cel-I enzyme is used to cleave heteroduplexes, and resulting products are separated on a polyacrylamide gel and indel efficiency calculated.

In vivo proof of principle in animal

Delivery mechanisms

AAV or Lentivirus production is described elsewhere.

Nanoparticle formulation: RNA mixed into nanoparticle formulation

Hydrodynamic tail vein injections with DNA plasmids in mice are conducted using a commercial kit

Cas9 and guide sequences are delivered as virus, nanoparticle-coated RNA mixture, or DNA plasmids, and injected into subject animals. A parallel set of control animals is injected with sterile saline, Cas9 and GFP, or guide sequence and GFP alone.

Three weeks after injection, animals are tested for amelioration of symptoms and sacrificed. Relevant organ systems analyzed for indel formation. Phenotypic assays include blood levels of HDL, LDL, lipids,

Assay for indel formation

DNA is extracted from tissue using commercial kits; indel assay will be performed as described for *in vitro* demonstration.

Therapeutic applications of the CRISPR-Cas system are amenable for achieving tissue-specific and temporally controlled targeted deletion of candidate disease genes. Examples include genes involved in cholesterol and fatty acid metabolism, amyloid diseases, dominant negative diseases, latent viral infections, among other disorders.

Examples of a single guide-RNA to introduce targeted indels at a gene locus

| Disease | GENE | SPACER | PAM | Mechanism | References |
|---|---|---|---|---|---|
| Hypercholesterolemia | HMG-CR | GCCAAATT GGACGACC CTCG | CGG | Knockout | Fluvastatin: a review of its pharmacology and use in the management of hypercholesterolaemia. ( Plosker GL et al. Drugs 1996, 51(3):433-459) |
| Hypercholesterolemia | SQLE | CGAGGAGA CCCCCGTTT CGG | TGG | Knockout | Potential role of nonstatin cholesterol lowering agents (Trapani et al. IUBMB Life, Volume 63, Issue 11, pages 964-971, November 2011) DGAT1 inhibitors as anti-obesity and anti-diabetic agents. (Birch AM et al. |
| Hyperlipidemia | DGAT 1 | CCCGCCGC CGCCGTGG CTCG | AGG | Knockout | Current Opinion in Drug Discovery & Development [2010,13(4):489-496] |
| Leukemia | BCR-ABL | TGAGCTCTA CGAGATCC ACA | AGG | Knockout | Killing of leukemic cells with a BCR/ABL fusion gene by RNA interference (RNAi).(Fuchs et al. Oncogene 2002, 21(37): 5716-5724) |

Examples of a pair of guide-RNA to introduce chromosomal microdeletion at a gene locus

| Disease | GENE | SPACER | PAM | Mechanism | References |
|---|---|---|---|---|---|
| Hyperlipidemia | PLIN2 guide1 | CTCAAAATT CATACCGGT TG | TGG | Microdeletion | Perilipin-2 Null Mice are Protected Against Diet-Induced Obesity, Adipose Inflammation and Fatty Liver Disease (McManaman JL et al. The Journal of Lipid Research, jlr.M035063. First Published on February 12, 2013) |
| Hyperlipidemia | PLIN2 guide2 | CGTTAAACA ACAACCGG ACT | TGG | Microdeletion | |
| Hyperlipidemia | SREBP guide1 | TTCACCCCG CGGCGCTG AAT | ggg | Microdeletion | Inhibition of SREBP by a Small Molecule, Betulin, Improves Hyperlipidemia and Insulin Resistance and Reduces Atherosclerotic Plaques (Tang J et al. Cell Metabolism, Volume 13, Issue 1, 44-56, 5 January 2011) |
| Hyperlipidemia | SREBP guide2 | ACCACTACC AGTCCGTCC AC | agg | Microdeletion | |

*Example 19: Targeted integration of repair for genes carrying disease-causing mutations; reconstitution of enzyme deficiencies and other related diseases.*

Study design

I. Identification and design of gene targets

- Described in Example 22

II. Cloning of guide sequences and repair templates into a delivery system

- Described above in Example 22

- Applicants clone DNA repair templates to include homology arms with diseased allele as well a wild-type repair template

III. *In vitro* validation on cell lines

a. Transfection is described above in Example 22; Cas9, guide RNAs, and repair template are co-transfected into relevant cell types.

b. Assay for repair *in vitro*

i. Applicants harvest cells 72-hours post-transfection and assay for repair

ii. Briefly, Applicants amplify genomic region around repair template PCR using high-fidelity polymerase. Applicants sequence products for decreased incidence of mutant allele.

IV. *In vivo* proof of principle in animal

a. Delivery mechanisms are described above Examples 22 and 34.

b. Assay for repair *in vivo*

i. Applicants perform the repair assay as described in the *in vitro* demonstration.

V. Therapeutic applications
The CRISPR-Cas system is amenable for achieving tissue-specific and temporally controlled targeted deletion of candidate disease genes. Examples include genes involved in cholesterol and fatty acid metabolism, amyloid diseases, dominant negative diseases, latent viral infections, among other disorders.

Example of one single missense mutation with repair template:

| Disease | GENE | SPACER | PAM |
|---|---|---|---|
| Familial amyloid polyneuropathy | TTR | AGCCTTTCTGAACACATGCA | CGG |

| Mechanism | References |
|---|---|
| V30M repair | Transthyretin mutations in health and disease (Joao et al. Human Mutation, Volume 5, Issue 3, pages 191-196, 1995) |
| V30M allele | CCTGCCATCAATGTGGCC**A**TGCATGTGTTCAGAAAGGCT |
| WT allele | CCTGCCATCAATGTGGCC**G̲**TGCATGTGTTCAGAAAGGCT |

*Example 20: Therapeutic application of the CRISPR-Cas system in Glaucoma, Amyloidosis, and Huntington's disease*

Glaucoma: Applicants design guide RNAs to target the first exon of the mycilin (MYOC) gene. Applicants use adenovirus vectors (Ad5) to package both Cas9 as well as a guide RNA targeting the MYOC gene. Applicants inject adenoviral vectors into the trabecular meshwork where cells have been implicated in the pathophysiology of glaucoma. Applicants initially test this out in mouse models carrying the mutated MYOC gene to see whether they improve visual acuity and decrease pressure in the eyes. Therapeutic application in humans employ a similar strategy.

Amyloidosis: Applicants design guide RNAs to target the first exon of the transthyretin (TTR) gene in the liver. Applicants use AAV8 to package Cas9 as well as guide RNA targeting the first exon of the TTR gene. AAV8 has been shown to have efficient targeting of the liver and will be administered intravenously. Cas9 can be driven either using liver specific promoters such as the albumin promoter, or using a constitutive promoter. A pol3 promoter drives the guide RNA.

Alternatively, Applicants utilize hydrodynamic delivery of plasmid DNA to knockout the TTR gene. Applicants deliver a plasmid encoding Cas9 and the guideRNA targeting Exon1 of TTR.

As a further alternative approach, Applicants administer a combination of RNA (mRNA for Cas9, and guide RNA). RNA can be packaged using liposomes such as Invivofectamine from Life Technologies and delivered intravenously. To reduce RNA-induced immunogenicity, increase the level of Cas9 expression and guide RNA stability, Applicants modify the Cas9 mRNA using 5' capping. Applicants also incorporate modified RNA nucleotides into Cas9 mRNA and guide RNA to increase their stability and reduce immunogenicity (e.g. activation of TLR). To increase efficiency, Applicants administer multiple doses of the virus, DNA, or RNA.

Huntington's Disease: Applicants design guide RNA based on allele specific mutations in the HTT gene of patients. For example, in a patient who is heterozygous for HTT with expanded CAG repeat, Applicants identify nucleotide sequences unique to the mutant HTT allele and use it to design guideRNA. Applicants ensure that the mutant base is located within the last 9 bp of the guide RNA (which Applicants have ascertained has the ability to discriminate between single DNA base mismatches between the target size and the guide RNA).

Applicants package the mutant HTT allele specific guide RNA and Cas9 into AAV9 and deliver into the striatum of Huntington's patients. Virus is injected into the striatum stereotactically via a craniotomy. AAV9 is known to transduce neurons efficiently. Applicants drive Cas9 using a neuron specific promoter such as human Synapsin I.

*Example 21: Therapeutic application of the CRISPR-Cas system in HIV*

Chronic viral infection is a source of significant morbidity and mortality. While there exists for many of these viruses conventional antiviral therapies that effectively target various aspects of viral replication, current therapeutic modalities are usually non-curative in nature due to "viral latency." By its nature, viral latency is characterized by a dormant phase in the viral life cycle without active viral production. During this period, the virus is largely able to evade both immune surveillance and conventional therapeutics allowing for it to establish long-standing viral reservoirs within the host from which subsequent re-activation can permit continued propagation and transmission of virus. Key to viral latency is the ability to stably maintain the viral genome, accomplished either through episomal or proviral latency, which stores the viral genome in the cytoplasm or integrates it into the host genome, respectively. In the absence of effective vaccinations which would prevent primary infection, chronic viral infections characterized by latent reservoirs and episodes of lytic activity can have significant consequences: human papilloma virus (HPV) can result in cervical cancer, hepatitis C virus (HCV) predisposes to hepatocellular carcinoma, and human immunodeficiency virus eventually destroys the host immune system resulting in susceptibility to opportunistic infections. As such, these infections require life-long use of currently available antiviral therapeutics. Further complicating matters is the high mutability of many of these viral genomes which lead to the evolution of resistant strains for which there exists no effective therapy.

The CRISPR-Cas system is a bacterial adaptive immune system able to induce double-stranded DNA breaks (DSB) in a multiplex-able, sequence-specific manner and has been recently re-constituted within mammalian cell systems. It has been shown that targeting DNA with one or numerous guide-RNAs can result in both indels and deletions of the intervening sequences, respectively. As such, this new technology represents a means by which targeted and multiplexed DNA mutagenesis can be accomplished within a single cell with high efficiency and specificity. Consequently, delivery of the CRISPR-Cas system directed against viral DNA sequences could allow for targeted disruption and deletion of latent viral genomes even in the absence of ongoing viral production.

As an example, chronic infection by HIV-1 represents a global health issue with 33 million individuals infected and an annual incidence of 2.6 million infections. The use of the multimodal highly active antiretroviral therapy (HAART), which simultaneously targets multiple aspects of viral replication, has allowed HIV infection to be largely managed as a chronic, not terminal, illness. Without treatment, progression of HIV to AIDS occurs usually within 9-10 years resulting in depletion of the host immune system and occurrence of opportunistic infections usually leading to death soon thereafter. Secondary to viral latency, discontinuation of HAART invariably leads to viral rebound. Moreover, even temporary disruptions in therapy can select for resistant strains of HIV uncontrollable by available means. Additionally, the costs of HAART therapy are significant: within the US $10,000-15,0000 per person per year. As such, treatment approaches directly targeting the HIV genome rather than the process of viral replication represents a means by which eradication of latent reservoirs could allow for a curative therapeutic option.

Development and delivery of an HIV-1 targeted CRISPR-Cas system represents a unique approach differentiable from existing means of targeted DNA mutagenesis, i.e. ZFN and TALENs, with numerous therapeutic implications. Targeted disruption and deletion of the HIV-1 genome by CRISPR-mediated DSB and indels in conjunction with HAART could allow for simultaneous prevention of active viral production as well as depletion of latent viral reservoirs within the host.

Once integrated within the host immune system, the CRISPR-Cas system allows for generation of a HIV-1 resistant sub-population that, even in the absence of complete viral eradication, could allow for maintenance and reconstitution of host immune activity. This could potentially prevent primary infection by disruption of the viral genome preventing viral production and integration, representing a means to "vaccination". Multiplexed nature of the CRISPR-Cas system allows targeting of multiple aspects of the genome simultaneously within individual cells.

As in HAART, viral escape by mutagenesis is minimized by requiring acquisition of multiple adaptive mutations concurrently. Multiple strains of HIV-1 can be targeted simultaneously which minimizes the chance of super-infection and prevents subsequent creation of new recombinants strains. Nucleotide, rather than protein, mediated sequence-specificity of the CRISPR-Cas system allows for rapid generation of therapeutics without need for significantly altering delivery mechanism.

In order to accomplish this, Applicants generate CRISPR-Cas guide RNAs that target the vast majority of the HIV-1 genome while taking into account HIV-1 strain variants for maximal coverage and effectiveness. Sequence analyses of genomic conservation between HIV-1 subtypes and variants should allow for targeting of flanking conserved regions of the genome with the aims of deleting intervening viral sequences or induction of frame-shift mutations which would disrupt viral gene functions.

Applicants accomplish delivery of the CRISPR-Cas system by conventional adenoviral or lentiviral-mediated infection of the host immune system. Depending on approach, host immune cells could be a) isolated, transduced with CRISPR-Cas, selected, and re-introduced in to the host or b) transduced in vivo by systemic delivery of the CRISPR-Cas system. The first approach allows for generation of a resistant immune population whereas the second is more likely to target latent viral reservoirs within the host.

| Examples of potential HIV-1 targeted spacers adapted from Mcintyre *et al*, which generated shRNAs against HIV-1 optimized for maximal coverage of HIV-1 variants. |
|---|
| CACTGCTTAAGCCTCGCTCGAGG<br>TCACCAGCAATATTCGCTCGAGG<br>CACCAGCAATATTCCGCTCGAGG<br>TAGCAACAGACATACGCTCGAGG<br>GGGCAGTAGTAATACGCTCGAGG<br>CCAATTCCCATACATTATTGTAC |

*Example 22: Targeted correction of deltaF508 or other mutations in cystic fibrosis*

An aspect provides for a pharmaceutical composition that may comprise an CRISPR-Cas gene therapy particle and a biocompatible pharmaceutical carrier. According to another aspect, a method of gene therapy for the treatment of a subject having a mutation in the CFTR gene comprises administering a therapeutically effective amount of a CRISPR-Cas gene therapy particle to the cells of a subject

This Example demonstrates gene transfer or gene delivery of a CRISPR-Cas system in airways of subject or a patient in need thereof, suffering from cystic fibrosis or from cystic fibrosis related symptoms, using adeno-associated virus (AAV) particles.

Study Design: Subjects or patients in need there of: Human, non-primate human, canine, feline, bovine, equine and other domestic animals, related. This study tests efficacy of gene transfer of a CRISPR-Cas system by a AAV vector. Applicants determine transgene levels sufficient for gene expression and utilize a CRISPR-Cas system comprising a Cas9 enzyme to target deltaF508 or other CFTR-inducing mutations.

The treated subjects receive pharmaceutically effective amount of aerosolized AAV vector system per lung endobronchially delivered while spontaneously breathing. The control subjects receive equivalent amount of a pseudotyped AAV vector system with an internal control gene. The vector system may be delivered along with a pharmaceutically acceptable or biocompatible pharmaceutical carrier. Three weeks or an appropriate time interval following vector administration, treated subjects are tested for amelioration of cystic fibrosis related symptoms.

Applicants use an adenovirus or an AAV particle.

Applicants clone the following gene constructs, each operably linked to one or more regulatory sequences (Cbh or EF1a promoter for Cas9, U6 or H1 promoter for chimeric guide RNA), into one or more adenovirus or AAV vectors or any other compatible vector: A CFTRdelta508 targeting chimeric guide RNA (Fig. 31B), a repair template for deltaF508 mutation (Fig. 31C) and a codon optimized Cas9 enzyme with optionally one or more nuclear localization signal or sequence(s) (NLS(s)), e.g., two (2) NLSs.

Identification of Cas9 target site

Applicants analyzed the human CFTR genomic locus and identified the Cas9 target site (Fig. 31A). (PAM may contain a NGG or a NNAGAAW motif).

Gene Repair Strategy

Applicants introduce an adenovirus/AAV vector system comprising a Cas9 (or Cas9 nickase) and the guide RNA along with a adenovirus/AAV vector system comprising the homology repair template containing the F508 residue into the subject via one of the methods of delivery discussed earlier. The CRISPR-Cas system is guided by the CFTRdelta 508 chimeric guide RNA and targets a specific site of the CFTR genomic locus to be nicked or cleaved. After cleavage, the repair template is inserted into the cleavage site via homologous recombination correcting the deletion that results in cystic fibrosis or causes cystic fibrosis related symptoms. This strategy to direct delivery and provide systemic introduction of CRISPR systems with appropriate guide RNAs can be employed to target genetic mutations to edit or otherwise manipulate genes that cause metabolic, liver, kidney and protein diseases and disorders such as those in Table B.

*Example 23: Generation of Gene Knockout Cell Library*

This example demonstrates how to generate a library of cells where each cell has a single gene knocked out:
Applicants make a library of ES cells where each cell has a single gene knocked out, and the entire library of ES cells will have every single gene knocked out. This library is useful for the screening of gene function in cellular processes as well as diseases.
To make this cell library, Applicants integrate Cas9 driven by an inducible promoter (e.g. doxycycline inducible promoter) into the ES cell. In addition, Applicants integrate a single guide RNA targeting a specific gene in the ES cell. To make the ES cell library, Applicants simply mix ES cells with a library of genes encoding guide RNAs targeting each gene in the human genome. Applicants first introduce a single BxB1 attB site into the AAVS1 locus of the human ES cell. Then Applicants use the BxB1 integrase to facilitate the integration of individual guide RNA genes into the BxB1 attB site in AAVS1 locus. To facilitate integration, each guide RNA gene is contained on a plasmid that carries of a single attP site. This way BxB1 will recombine the attB site in the genome with the attP site on the guide RNA containing plasmid.
To generate the cell library, Applicants take the library of cells that have single guide RNAs integrated and induce Cas9 expression. After induction, Cas9 mediates double strand break at sites specified by the guide RNA. To verify the diversity of this cell library, Applicants carry out whole exome sequencing to ensure that Applicants are able to observe mutations in every single targeted gene. This cell library can be used for a variety of applications, including who library-based screens, or can be sorted into individual cell clones to facilitate rapid generation of clonal cell lines with individual human genes knocked out

*Example 24: Engineering of Microalgae using Cas9*

Methods of delivering Cas9

Method 1: Applicants deliver Cas9 and guide RNA using a vector that expresses Cas9 under the control of a constitutive promoter such as Hsp70A-Rbc S2 or Beta2-tubulin.
Method 2: Applicants deliver Cas9 and T7 polymerase using vectors that expresses Cas9 and T7 polymerase under the control of a constitutive promoter such as Hsp70A-Rbc S2 or Beta2-tubulin. Guide RNA will be delivered using a vector containing T7 promoter driving the guide RNA.
Method 3: Applicants deliver Cas9 mRNA and *in vitro* transcribed guide RNA to algae cells. RNA can be *in vitro* transcribed. Cas9 mRNA will consist of the coding region for Cas9 as well as 3'UTR from Cop1 to ensure stabilization of the Cas9 mRNA.

For Homologous recombination, Applicants provide an additional homology directed repair template.
Sequence for a cassette driving the expression of Cas9 under the control of beta-2 tubulin promoter, followed by the 3' UTR of Cop1.

TCTTTCTTGCGCTATGACACTTCCAGCAAAAGGTAGGGCGGGCTGCGA

GACGGCTTCCCGGCGCTGCATGCAACACCGATGATGCTTCGACCCCCCGAAGC

TC

CTTCGGGGCTGCATGGGCGCTCCGATGCCGCTCCAGGGCGAGCGCTGTTTAAA

TA

GCCAGGCCCCGATTGCAAAGACATTATAGCGAGCTACCAAAGCCATATTCA

AAC

ACCTAGATCACTACCACTTCTACACAGGCCACTCGAGCTTGTGATCGCACTCC

GC

TAAGGGGGCGCCTCTTCCTCTTCGTTTCAGTCACAACCCGCAAACATGTACCC

ATA

CGATGTTCCAGATTACGCTTCGCCGAAGAAAAGCGCAAGGTCGAAGCGTCC

GA

CAAGAAGTACAGCATCGGCCTGGACATCGGCACCAACTCTGTGGGCTGGGCC

GTG

ATCACCGACGAGTACAAGGTGCCCAGCAAGAAATTCAAGGTGCTGGGCAACA

CC

GACCGGCACAGCATCAAGAAGAACCTGATCGGAGCCCTGCTGTTCGACAGCG

GC

GAAACAGCCGAGGCCACCCGGCTGAAGAGAACCGCCAGAAGAAGATACACC

AG

ACGGAAGAACCGGATCTGCTATCTGCAAGAGATCTTCAGCAACGAGATGGCC

AA

GGTGGACGACAGCTTCTTCCACAGACTGGAAGAGTCCTTCCTGGTGGAAGAGGAT

AAGAAGCACGAGCGGCACCCCATCTTCGGCAACATCGTGGACGAGGTGGCCTAC

CACGAGAAGTACCCCACCATCTACCACCTGAGAAAGAAACTGGTGGACAGCACC

GACAAGGCCGACCTGCGGCTGATCTATCTGGCCCTGGCCCACATGATCAAGTTCC

GGGGCCACTTCCTGATCGAGGGCGACCTGAACCCCGACAACAGCGACGTGGACA

AGCTGTTCATCCAGCTGGTGCAGACCTACAACCAGCTGTTCGAGGAAAACCCCAT

CAACGCCAGCGGCGTGGACGCCAAGGCCATCCTGTCTGCCAGACTGAGCAAGAG

CAGACGGCTGGAAAATCTGATCGCCCAGCTGCCCGGCGAGAAGAAGAATGGCCT

GTTCGGCAACCTGATTGCCCTGAGCCTGGGCCTGACCCCCAACTTCAAGAGCAAC

TTCGACCTGGCCGAGGATGCCAAACTGCAGCTGAGCAAGGACACCTACGACGAC

GACCTGGACAACCTGCTGGCCCAGATCGGCGACCAGTACGCCGACCTGTTTCTGG

CCGCCAAGAACCTGTCCGACGCCATCCTGCTGAGCGACATCCTGAGAGTGAACAC

CGAGATCACCAAGGCCCCCCTGAGCGCCTCTATGATCAAGAGATACGACGAGCA

CCACCAGGACCTGACCCTGCTGAAAGCTCTCGTGCGGCAGCAGCTGCCTGAGAAG

TACAAAGAGATTTTCTTCGACCAGAGCAAGAACGGCTACGCCGGCTACATTGACG

GCGGAGCCAGCCAGGAAGAGTTCTACAAGTTCATCAAGCCCATCCTGGAAAAGA

TGGACGGCACCGAGGAACTGCTCGTGAAGCTGAACAGAGAGGACCTGCTGCGGA

AGCAGCGGACCTTCGACAACGGCAGCATCCCCCACCAGATCCACCTGGGAGA
GC

TGCACGCCATTCTGCGGCGGCAGGAAGATTTTTACCCATTCCTGAAGGACAAC
CG

GGAAAAGATCGAGAAGATCCTGACCTTCCGCATCCCCTACTACGTGGGCCCTC
TG

GCCAGGGGAAACAGCAGATTCGCCTGGATGACCAGAAAGAGCGAGGAAACC
ATC

ACCCCCTGGAACTTCGAGGAAGTGGTGGACAAGGGCGCTTCCGCCCAGAGCT
TCA

TCGAGCGGATGACCAACTTCGATAAGAACCTGCCCAACGAGAAGGTGCTGCC
CA

AGCACAGCCTGCTGTACGAGTACTTCACCGTGTATAACGAGCTGACCAAAGTG
AA

ATACGTGACCGAGGGAATGAGAAAGCCCGCCTTCCTGAGCGGCGAGCAGAAA
AA

GGCCATCGTGGACCTGCTGTTCAAGACCAACCGGAAAGTGACCGTGAAGCAG
CT

GAAAGAGGACTACTTCAAGAAAATCGAGTGCTTCGACTCCGTGGAAATCTCC
GGC

GTGGAAGATCGGTTCAACGCCTCCCTGGGCACATACCACGATCTGCTGAAAAT
TA

TCAAGGACAAGGACTTCCTGGACAATGAGGAAACGAGGACATTCTGGAAGA
TA

TCGTGCTGACCCTGACACTGTTTGAGGACAGAGAGATGATCGAGGAACGGCT
GA

AAACCTATGCCCACCTGTTCGACGACAAAGTGATGAAGCAGCTGAAGCGGCG
GA

GATACACCGGCTGGGGCAGGCTGAGCCGGAAGCTGATCAACGGCATCCGGGA
CA

AGCAGTCCGGCAAGACAATCCTGGATTTCCTGAAGTCCGACGGCTTCGCCAAC
AG

AAACTTCATGCAGCTGATCCACGACGACAGCCTGACCTTTAAAGAGGACATCC
AG

AAAGCCCAGGTGTCCGGCCAGGGCGATAGCCTGCACGAGCACATTGCCAATC
TG

GCCGGCAGCCCCGCCATTAAGAAGGGCATCCTGCAGACAGTGAAGGTGGTGG
AC

GAGCTCGTGAAAGTGATGGGCCGGCACAAGCCCGAGAACATCGTGATCGAAA
TG

GCCAGAGAGAACCAGACCACCCAGAAGGGACAGAAGAACAGCCGCGAGAGA
AT

GAAGCGGATCGAAGAGGGCATCAAAGAGCTGGGCAGCCAGATCCTGAAAGA
ACA

CCCCGTGGAAAACACCCAGCTGCAGAACGAGAAGCTGTACCTGTACTACCTG
CAG

AATGGGCGGGATATGTACGTGGACCAGGAACTGGACATCAACCGGCTGTCCG
AC

TACGATGTGGACCATATCGTGCCTCAGAGCTTTCTGAAGGACGACTCCATCGA
CA

ACAAGGTGCTGACCAGAAGCGACAAGAACCGGGGCAAGAGCGACAACGTGC
CCT

CCGAAGAGGTCGTGAAGAAGATGAAGAACTACTGGCGGCAGCTGCTGAACGC
CA

AGCTGATTACCCAGAGAAAGTTCGACAATCTGACCAAGGCCGAGAGAGGCGG
CC

TGAGCGAACTGGATAAGGCCGGCTTCATCAAGAGACAGCTGGTGGAAACCCG
GC

AGATCACAAAGCACGTGGCACAGATCCTGGACTCCCGGATGAACACTAAGTA
CG

ACGAGAATGACAAGCTGATCCGGGAAGTGAAAGTGATCACCCTGAAGTCCAA
GC

TGGTGTCCGATTTCCGGAAGGATTTCCAGTTTTACAAAGTGCGCGAGATCAAC
AA

CTACCACCACGCCCACGACGCCTACCTGAACGCCGTCGTGGGAACCGCCCTGA
TC

AAAAAGTACCCTAAGCTGGAAAGCGAGTTCGTGTACGGCGACTACAAGGTGT
AC

GACGTGCGGAAGATGATCGCCAAGAGCGAGCAGGAAATCGGCAAGGCTACCG
CC

AAGTACTTCTTCTACAGCAACATCATGAACTTTTTCAAGACCGAGATTACCCT
GG

CCAACGGCGAGATCCGGAAGCGGCCTCTGATCGAGACAAACGGCGAAACCGG
GG

AGATCGTGTGGGATAAGGGCCGGGATTTTGCCACCGTGCGGAAAGTGCTGAG
CA

TGCCCCAAGTGAATATCGTGAAAAAGACCGAGGTGCAGACAGGCGGCTTCAG
CA

AAGAGTCTATCCTGCCCAAGAGGAACAGCGATAAGCTGATCGCCAGAAAGAA
GG

ACTGGGACCCTAAGAAGTACGGCGGCTTCGACAGCCCCACCGTGGCCTATTCT
GT

GCTGGTGGTGGCCAAAGTGGAAAAGGGCAAGTCCAAGAAACTGAAGAGTGTG
AA

AGAGCTGCTGGGGATCACCATCATGGAAGAAGCAGCTTCGAGAAGAATCCC
AT

CGACTTTCTGGAAGCCAAGGGCTACAAAGAAGTGAAAAAGGACCTGATCATC
AA

GCTGCCTAAGTACTCCCTGTTCGAGCTGGAAAACGGCCGGAAGAGAATGCTG
GC

CTCTGCCGGCGAACTGCAGAAGGGAAACGAACTGGCCCTGCCCTCCAAATAT
GT

GAACTTCCTGTACCTGGCCAGCCACTATGAGAAGCTGAAGGGCTCCCCCGAGG
AT

AATGAGCAGAAACAGCTGTTTGTGGAACAGCACAAGCACTACCTGGACGAGA
TC

ATCGAGCAGATCAGCGAGTTCTCCAAGAGAGTGATCCTGGCCGACGCTAATCT
GG

ACAAAGTGCTGTCCGCCTACAACAAGCACCGGGATAAGCCCATCAGAGAGCA
GG

CCGAGAATATCATCCACCTGTTTACCCTGACCAATCTGGGAGCCCCTGCCGCC
TT

CAAGTACTTTGACACCACCATCGACCGGAAGAGGTACACCAGCACCAAAGAGGT

GCTGGACGCCACCCTGATCCACCAGAGCATCACCGGCCTGTACGAGACACGGAT

CGACCTGTCTCAGCTGGGAGGCGACAGCCCCAAGAAGAAGAGAAAGGTGGAGGC

CAGCTAAGGATCCGGCAAGACTGGCCCCGCTTGGCAACGCAACAGTGAGCCCCTC

CCTAGTGTGTTTGGGGATGTGACTATGTATTCGTGTGTTGGCCAACGGGTCAACC

CGAACAGATTGATACCCGCCTTGGCATTTCCTGTCAGAATGTAACGTCAGTTGAT GGTACT

Sequence for a cassette driving the expression of T7 polymerase under the control of beta-2 tubulin promoter, followed by the 3' UTR of Cop1:

TCTTTCTTGCGCTATGACACTTCCAGCAAAAGGTAGGGCGGGCTGCGA

GACGGCTTCCCGGCGCTGCATGCAACACCGATGATGCTTCGACCCCCCGAAGCTC

CTTCGGGGCTGCATGGGCGCTCCGATGCCGCTCCAGGGCGAGCGCTGTTTAAATA

GCCAGGCCCCGATTGCAAAGACATTATAGCGAGCTACCAAAGCCATATTCAAAC

ACCTAGATCACTACCACTTCTACACAGGCCACTCGAGCTTGTGATCGCACTCCGC

TAAGGGGGCGCCTCTTCCTCTTCGTTTCAGTCACAACCCGCAAACatgcctaagaagaga

ggaaggttaacacgattaacatcgctaagaacgacttctctgacatcgaactggctgctatcccgttcaacactctggctgaccatt

acggtgagcgtttagctcgcgaacagttggcccttgagcatgagtcttacgagatggggtgaagcacgcttccgcaagatgtttga

gcgtcaacttaaagctggtgaggttgcggataacgctgccgccaagcctctcatcactaccctactccctaagatgattgcacgc

atcaacgactggtttgaggaagtgaaagctaagcgcggcaagcgcccgacagccttccagttcctgcaagaaatcaagccgga

agccgtagcgtacatcaccattaagaccactctggcttgcctaaccagtgctgacaatacaaccgttcaggctgtagcaagcgca

atcggtcgggccattgaggacgaggctcgcttcggtcgtatccgtgaccttgaagctaagcacttcaagaaaaacgttgaggaa

caactcaacaagcgcgtagggcacgtctacaagaaagcatttatgcaagttgtcgaggctgacatgctctctaagggtctactcg

gtggcgaggcgtggtcttcgtggcataaggaagactctattcatgtaggagtacgctgcatcgagatgctcattgagtcaaccgg

aatggttagcttacaccgccaaaatgctggcgtagtaggtcaagactctgagactatcgaactcgcacctgaatacgctgaggct

atcgcaacccgtgcaggtgcgctggctggcatctctccgatgttccaaccttgcgtagttcctcctaagccgtggactggcattac

tggtggtggctattgggctaacggtcgtcgtcctctggcgctggtgcgtactcacagtaagaaagcactgatgcgctacgaagac

gtttacatgcctgaggtgtacaaagcgattaacattgcgcaaaacaccgcatggaaaatcaacaagaaagtcctagcggtcgcc

aacgtaatcaccaagtggaagcattgtccggtcgaggacatccctgcgattgagcgtgaagaactcccgatgaaaccggaaga

catcgacatgaatcctgaggctctcaccgcgtggaaacgtgctgccgctgctgtgtaccgcaaggacaaggctcgcaagtctcg

ccgtatcagccttgagttcatgcttgagcaagccaataagtttgctaaccataaggccatctggttcccttacaacatggactggcg

cggtcgtgtttacgctgtgtcaatgttcaacccgcaaggtaacgatatgaccaaaggactgcttacgctggcgaaaggtaaacca

atcggtaaggaaggttactactggctgaaaatccacggtgcaaactgtgcgggtgtcgacaaggttccgttccctgagcgcatca

agttcattgaggaaaaccacgagaacatcatggcttgcgctaagtctccactggagaacacttggtgggctgagcaagattctcc

gttctgcttccttgcgttctgctttgagtacgctggggtacagcaccacggcctgagctataactgctcccttccgctggcgtttgac

gggtcttgctctggcatccagcacttctccgcgatgctccgagatgaggtaggtggtcgcgcggttaacttgcttcctagtgaaac

cgttcaggacatctacgggattgttgctaagaaagtcaacgagattctacaagcagacgcaatcaatgggaccgataacgaagta

gttaccgtgaccgatgagaacactggtgaaatctctgagaaagtcaagctgggcactaaggcactggctggtcaatggctggct

tacggtgttactcgcagtgtgactaagcgttcagtcatgacgctggcttacgggtccaaagagttcggcttccgtcaacaagtgct

ggaagataccattcagccagctattgattccggcaagggtctgatgttcactcagccgaatcaggctgctggatacatggctaag

ctgatttgggaatctgtgagcgtgacggtggtagctgcggttgaagcaatgaactggcttaagtctgctgctaagctgctggctgc

tgaggtcaaagataagaagactggagagattcttcgcaagcgttgcgctgtgcattgggtaactcctgatggtttccctgtgtggc

aggaatacaagaagcctattcagacgcgcttgaacctgatgttcctcggtcagttccgcttacagcctaccattaacaccaacaa

agatagcgagattgatgcacacaaacaggagtctggtatcgctcctaactttgtacacagccaagacggtagccaccttcgtaag

actgtagtgtgggcacacgagaagtacggaatcgaatcttttgcactgattcacgactccttcggtacgattccggctgacgctgc

gaacctgttcaaagcagtgcgcgaaactatggttgacacatatgagtcttgtgatgtactggctgatttctacgaccagttcgctga

ccagttgcacgagtctcaattggacaaaatgccagcacttccggctaaaggtaacttgaacctccgtgacatcttagagtcggact

tcgcgttcgcgtaaGGATCCGGCAAGACTGGCCCCGCTTGGCAACGCAACAGTGAGCC

C

CTCCCTAGTGTGTTTGGGGATGTGACTATGTATTCGTGTGTTGGCCAACGGGTC

A

ACCCGAACAGATTGATACCCGCCTTGGCATTTCCTGTCAGAATGTAACGTCAG

TT GATGGTACT

Sequence of guide RNA driven by the T7 promoter (T7 promoter, Ns represent targeting sequence):

gaaat**TAATACGACTCACTATA**<u>NNNNNNNNNNNNNNNNNNNNNNN</u>gttttagagc

ta**GAAA**tagcaagttaaaataaggctagtccgttatcaacttgaaaaagtggcaccgagtcggtgcttttttt

Gene delivery:

Chlamydomonas reinhardtii strain CC-124 and CC-125 from the Chlamydomonas Resource Center will be used for electroporation. Electroporation protocol follows standard recommended protocol from the GeneArt Chlamydomonas Engineering kit.

Also, Applicants generate a line of Chlamydomonas reinhardtii that expresses Cas9 constitutively. This can be done by using pChlamy1 (linearized using PvuI) and selecting for hygromycin resistant colonies. Sequence for pChlamy1 containing Cas9 is below. In this way to achieve gene knockout one simply needs to deliver RNA for the guideRNA. For homologous recombination Applicants deliver guideRNA as well as a linearized homologous recombination template.

pChlamy1-Cas9:

```
TGCGGTATTTCACACCGCATCAGGTGGCACTTTTCGGGGAAATGTGC
GCGGAACCCCTATTTGTTTATTTTTCTAAATACATTCAAATATGTATCCGCTCA
TGA
GATTATCAAAAAGGATCTTCACCTAGATCCTTTTAAATTAAAAATGAAGTTTT
AA
ATCAATCTAAAGTATATATGAGTAAACTTGGTCTGACAGTTACCAATGCTTAA
TC
AGTGAGGCACCTATCTCAGCGATCTGTCTATTTCGTTCATCCATAGTTGCCTGA
CT
CCCCGTCGTGTAGATAACTACGATACGGGAGGGCTTACCATCTGGCCCCAGTG
CT
GCAATGATACCGCGAGACCCACGCTCACCGGCTCCAGATTTATCAGCAATAAA
CC
AGCCAGCCGGAAGGGCCGAGCGCAGAAGTGGTCCTGCAACTTTATCCGCCTC
CAT
CCAGTCTATTAATTGTTGCCGGGAAGCTAGAGTAAGTAGTTCGCCAGTTAATA
GT
TTGCGCAACGTTGTTGCCATTGCTACAGGCATCGTGGTGTCACGCTCGTCGTTT
GG
TATGGCTTCATTCAGCTCCGGTTCCCAACGATCAAGGCGAGTTACATGATCCC
CC
ATGTTGTGCAAAAAAGCGGTTAGCTCCTTCGGTCCTCCGATCGTTGTCAGAAG
TA
```

AGTTGGCCGCAGTGTTATCACTCATGGTTATGGCAGCACTGCATAATTCTCTTACT

GTCATGCCATCCGTAAGATGCTTTTCTGTGACTGGTGAGTACTCAACCAAGTCATT

CTGAGAATAGTGTATGCGGCGACCGAGTTGCTCTTGCCCGGCGTCAATACGGGAT

AATACCGCGCCACATAGCAGAACTTTAAAAGTGCTCATCATTGGAAAACGTTCTT

CGGGGCGAAAACTCTCAAGGATCTTACCGCTGTTGAGATCCAGTTCGATGTAACC

CACTCGTGCACCCAACTGATCTTCAGCATCTTTTACTTTCACCAGCGTTTCTGGGT

GAGCAAAAACAGGAAGGCAAAATGCCGCAAAAAGGGAATAAGGGCGACACGG

AAATGTTGAATACTCATACTCTTCCTTTTTCAATATTATTGAAGCATTTATCAGGG

TTATTGTCTCATGACCAAAATCCCTTAACGTGAGTTTTCGTTCCACTGAGCGTCAG

ACCCCGTAGAAAAGATCAAAGGATCTTCTTGAGATCCTTTTTTTCTGCGCGTAATC

TGCTGCTTGCAAACAAAAAACCACCGCTACCAGCGGTGGTTTGTTTGCCGGATC

AAGAGCTACCAACTCTTTTTCCGAAGGTAACTGGCTTCAGCAGAGCGCAGATACC

AAATACTGTTCTTCTAGTGTAGCCGTAGTTAGGCCACCACTTCAAGAACTCTGTA

GCACCGCCTACATACCTCGCTCTGCTAATCCTGTTACCAGTGGCTGTTGCCAGTGG

CGATAAGTCGTGTCTTACCGGGTTGGACTCAAGACGATAGTTACCGGATAAGGCG

CAGCGGTCGGGCTGAACGGGGGGTTCGTGCACACAGCCCAGCTTGGAGCGAACG

ACCTACACCGAACTGAGATACCTACAGCGTGAGCTATGAGAAAGCGCCACGCTTC

CCGAAGGGAGAAAGGCGGACAGGTATCCGGTAAGCGGCAGGGTCGGAACAGGA

GAGCGCACGAGGGAGCTTCCAGGGGGAAACGCCTGGTATCTTTATAGTCCTGTCG

GGTTTCGCCACCTCTGACTTGAGCGTCGATTTTGTGATGCTCGTCAGGGGGGCGG

AGCCTATGGAAAAACGCCAGCAACGCGGCCTTTTACGGTTCCTGGCCTTTTGCTG

GCCTTTTGCTCACATGTTCTTTCCTGCGTTATCCCCTGATTCTGTGGATAACCGTA

TTACCGCCTTTGAGTGAGCTGATACCGCTCGCCGCAGCCGAACGACCGAGCGCAG

CGAGTCAGTGAGCGAGGAAGCGGTCGCTGAGGCTTGACATGATTGGTGCGTATGT

TTGTATGAAGCTACAGGACTGATTTGGCGGGCTATGAGGGCGGGGGAAGCTCTGG

AAGGGCCGCGATGGGGCGCGCGGCGTCCAGAAGGCGCCATACGGCCCGCTGGCG

GCACCCATCCGGTATAAAGCCCGCGACCCCGAACGGTGACCTCCACTTTCAGCG

ACAAACGAGCACTTATACATACGCGACTATTCTGCCGCTATACATAACCACTCAG

CTAGCTTAAGATCCCATCAAGCTTGCATGCCGGGCGCGCCAGAAGGAGCGCAGC

CAAACCAGGATGATGTTTGATGGGGTATTTGAGCACTTGCAACCCTTATCCGGAA

GCCCCCTGGCCCACAAAGGCTAGGCGCCAATGCAAGCAGTTCGCATGCAGCCCCT

GGAGCGGTGCCCTCCTGATAAACCGGCCAGGGGGCCTATGTTCTTTACTTTTTTAC

AAGAGAAGTCACTCAACATCTTAAAATGGCCAGGTGAGTCGACGAGCAAGCCCG

GCGGATCAGGCAGCGTGCTTGCAGATTTGACTTGCAACGCCCGCATTGTGTCGAC

GAAGGCTTTTGGCTCCTCTGTCGCTGTCTCAAGCAGCATCTAACCCTGCGTCGCCG

TTTCCATTTGCAGGAGATTCGAGGTACCATGTACCCATACGATGTTCCAGATTACG

CTTCGCCGAAGAAAAGCGCAAGGTCGAAGCGTCCGACAAGAAGTACAGCATCG

GCCTGGACATCGGCACCAACTCTGTGGGCTGGGCCGTGATCACCGACGAGTACAA

GGTGCCCAGCAAGAAATTCAAGGTGCTGGGCAACACCGACCGGCACAGCATCAA

GAAGAACCTGATCGGAGCCCTGCTGTTCGACAGCGGCGAAACAGCCGAGGCCAC

CCGGCTGAAGAGAACCGCCAGAAGAAGATACACCAGACGGAAGAACCGGATCTG

CTATCTGCAAGAGATCTTCAGCAACGAGATGGCCAAGGTGGACGACAGCTTCTTC

CACAGACTGGAAGAGTCCTTCCTGGTGGAAGAGGATAAGAAGCACGAGCGGCAC

CCCATCTTCGGCAACATCGTGGACGAGGTGGCCTACCACGAGAAGTACCCCACCA

TCTACCACCTGAGAAAGAAACTGGTGGACAGCACCGACAAGGCCGACCTGCGGC

TGATCTATCTGGCCCTGGCCCACATGATCAAGTTCCGGGGCCACTTCCTGATCGA

GGGCGACCTGAACCCCGACAACAGCGACGTGGACAAGCTGTTCATCCAGCTGGT

GCAGACCTACAACCAGCTGTTCGAGGAAAACCCCATCAACGCCAGCGGCGTGGA

CGCCAAGGCCATCCTGTCTGCCAGACTGAGCAAGAGCAGACGGCTGGAAAATCT

GATCGCCCAGCTGCCCGGCGAGAAGAAGAATGGCCTGTTCGGCAACCTGATTGC

CCTGAGCCTGGGCCTGACCCCCAACTTCAAGAGCAACTTCGACCTGGCCGAGGAT

GCCAAACTGCAGCTGAGCAAGGACACCTACGACGACGACCTGGACAACCTGCTG

GCCCAGATCGGCGACCAGTACGCCGACCTGTTTCTGGCCGCCAAGAACCTGTCCG

ACGCCATCCTGCTGAGCGACATCCTGAGAGTGAACACCGAGATCACCAAGGCCCC

CCTGAGCGCCTCTATGATCAAGAGATACGACGAGCACCACCAGGACCTGACCCTG

CTGAAAGCTCTCGTGCGGCAGCAGCTGCCTGAGAAGTACAAAGAGATTTTCTTCG

ACCAGAGCAAGAACGGCTACGCCGGCTACATTGACGGCGGAGCCAGCCAGGAAG

AGTTCTACAAGTTCATCAAGCCCATCCTGGAAAAGATGGACGGCACCGAGGAAC

TGCTCGTGAAGCTGAACAGAGAGGACCTGCTGCGGAAGCAGCGGACCTTCGACA

ACGGCAGCATCCCCCACCAGATCCACCTGGGAGAGCTGCACGCCATTCTGCGGCG

GCAGGAAGATTTTTACCCATTCCTGAAGGACAACCGGGAAAAGATCGAGAAGAT

CCTGACCTTCCGCATCCCCTACTACGTGGGCCCTCTGGCCAGGGGAAACAGCAGA

TTCGCCTGGATGACCAGAAAGAGCGAGGAAACCATCACCCCCTGGAACTTCGAG

GAAGTGGTGGACAAGGGCGCTTCCGCCCAGAGCTTCATCGAGCGGATGACCAAC

TTCGATAAGAACCTGCCCAACGAGAAGGTGCTGCCCAAGCACAGCCTGCTGTAC

GAGTACTTCACCGTGTATAACGAGCTGACCAAAGTGAAATACGTGACCGAGGGA

ATGAGAAAGCCCGCCTTCCTGAGCGGCGAGCAGAAAAAGGCCATCGTGGACCT

GCTGTTCAAGACCAACCGGAAAGTGACCGTGAAGCAGCTGAAAGAGGACTACTT

CAAGAAAATCGAGTGCTTCGACTCCGTGGAAATCTCCGGCGTGGAAGATCGGTTC

AACGCCTCCCTGGGCACATACCACGATCTGCTGAAAATTATCAAGGACAAGGACT

TCCTGGACAATGAGGAAAACGAGGACATTCTGGAAGATATCGTGCTGACCCTGA

CACTGTTTGAGGACAGAGAGATGATCGAGGAACGGCTGAAAACCTATGCCCACC

TGTTCGACGACAAAGTGATGAAGCAGCTGAAGCGGCGGAGATACACCGGCTGGG

GCAGGCTGAGCCGGAAGCTGATCAACGGCATCCGGGACAAGCAGTCCGGCAAGA

CAATCCTGGATTTCCTGAAGTCCGACGGCTTCGCCAACAGAAACTTCATGCAGCT

GATCCACGACGACAGCCTGACCTTTAAAGAGGACATCCAGAAAGCCCAGGTGTC

CGGCCAGGGCGATAGCCTGCACGAGCACATTGCCAATCTGGCCGGCAGCCCCGC

CATTAAGAAGGGCATCCTGCAGACAGTGAAGGTGGTGGACGAGCTCGTGAAAGT

GATGGGCCGGCACAAGCCCGAGAACATCGTGATCGAAATGGCCAGAGAGAACCA

GACCACCCAGAAGGGACAGAAGAACAGCCGCGAGAATGAAGCGGATCGAAG

AGGGCATCAAAGAGCTGGGCAGCCAGATCCTGAAGAACACCCCGTGGAAAACA

CCCAGCTGCAGAACGAGAAGCTGTACCTGTACTACCTGCAGAATGGGCGGGATA

TGTACGTGGACCAGGAACTGGACATCAACCGGCTGTCCGACTACGATGTGGACC

ATATCGTGCCTCAGAGCTTTCTGAAGGACGACTCCATCGACAACAAGGTGCTGAC

CAGAAGCGACAAGAACCGGGGCAAGAGCGACAACGTGCCCTCCGAAGAGGTCG

TGAAGAAGATGAAGAACTACTGGCGGCAGCTGCTGAACGCCAAGCTGATTAC
CC

AGAGAAAGTTCGACAATCTGACCAAGGCCGAGAGAGGCGGCCTGAGCGAACT
GG

ATAAGGCCGGCTTCATCAAGAGACAGCTGGTGGAAACCCGGCAGATCACAAA
GC

ACGTGGCACAGATCCTGGACTCCCGGATGAACACTAAGTACGACGAGAATGA
CA

AGCTGATCCGGGAAGTGAAAGTGATCACCCTGAAGTCCAAGCTGGTGTCCGA
TTT

CCGGAAGGATTTCCAGTTTTACAAAGTGCGCGAGATCAACAACTACCACCACG
CC

CACGACGCCTACCTGAACGCCGTCGTGGGAACCGCCCTGATCAAAAAGTACC
CTA

AGCTGGAAAGCGAGTTCGTGTACGGCGACTACAAGGTGTACGACGTGCGGAA
GA

TGATCGCCAAGAGCGAGCAGGAAATCGGCAAGGCTACCGCCAAGTACTTCTT
CTA

CAGCAACATCATGAACTTTTTCAAGACCGAGATTACCCTGGCCAACGGCGAGA
TC

CGGAAGCGGCCTCTGATCGAGACAAACGGCGAAACCGGGGAGATCGTGTGGG
AT

AAGGGCCGGGATTTTGCCACCGTGCGGAAAGTGCTGAGCATGCCCCAAGTGA
AT

ATCGTGAAAAAGACCGAGGTGCAGACAGGCGGCTTCAGCAAAGAGTCTATCC
TG

CCCAAGAGGAACAGCGATAAGCTGATCGCCAGAAAGAAGGACTGGGACCCTA
AG

AAGTACGGCGGCTTCGACAGCCCCACCGTGGCCTATTCTGTGCTGGTGGTGGC
CA

AAGTGGAAAAGGGCAAGTCCAAGAAACTGAAGAGTGTGAAAGAGCTGCTGG
GG

ATCACCATCATGGAAAGAAGCAGCTTCGAGAAGAATCCCATCGACTTTCTGGA
AG

CCAAGGGCTACAAAGAAGTGAAAAAGGACCTGATCATCAAGCTGCCTAAGTA
CT

CCCTGTTCGAGCTGGAAACGGCCGGAAGAGAATGCTGGCCTCTGCCGGCGA
AC

TGCAGAAGGGAAACGAACTGGCCCTGCCCTCCAAATATGTGAACTTCCTGTAC
C

TGGCCAGCCACTATGAGAAGCTGAAGGGCTCCCCCGAGGATAATGAGCAGAA
AC

AGCTGTTTGTGGAACAGCACAAGCACTACCTGGACGAGATCATCGAGCAGAT
CA

GCGAGTTCTCCAAGAGAGTGATCCTGGCCGACGCTAATCTGGACAAAGTGCTG
TC

CGCCTACAACAAGCACCGGGATAAGCCCATCAGAGAGCAGGCCGAGAATATC
AT

CCACCTGTTTACCCTGACCAATCTGGGAGCCCCTGCCGCCTTCAAGTACTTTGA
CA

CCACCATCGACCGGAAGAGGTACACCAGCACCAAAGAGGTGCTGGACGCCAC
CC

TGATCCACCAGAGCATCACCGGCCTGTACGAGACACGGATCGACCTGTCTCAG
CT

GGGAGGCGACAGCCCCAAGAAGAAGAGAAAGGTGGAGGCCAGCTAACATAT
GATTCGAATGTCTTTCTTGCGCTATGACACTTCCAGCAAAAGGTAGGGCG
GGCTGCGAGACGGCTTCCCGGCGCTGCATGCAACACCGATGATGCTTCGACCC
CC

CGAAGCTCCTTCGGGGCTGCATGGGCGCTCCGATGCCGCTCCAGGGCGAGCGC
TG

TTTAAATAGCCAGGCCCCCGATTGCAAAGACATTATAGCGAGCTACCAAAGCC
AT

ATTCAAACACCTAGATCACTACCACTTCTACACAGGCCACTCGAGCTTGTGAT
CG

CACTCCGCTAAGGGGGCGCCTCTTCCTCTTCGTTTCAGTCACAACCCGCAAAC
AT

GACACAAGAATCCCTGTTACTTCTCGACCGTATTGATTCGGATGATTCCTACG
CG

AGCCTGCGGAACGACCAGGAATTCTGGGAGGTGAGTCGACGAGCAAGCCCGG
CG

GATCAGGCAGCGTGCTTGCAGATTTGACTTGCAACGCCCGCATTGTGTCGACG
AA

GGCTTTTGGCTCCTCTGTCGCTGTCTCAAGCAGCATCTAACCCTGCGTCGCCGT
TT

CCATTTGCAGCCGCTGGCCCGCCGAGCCCTGGAGGAGCTCGGGCTGCCGGTGC
CG

CCGGTGCTGCGGGTGCCCGGCGAGAGCACCAACCCCGTACTGGTCGGCGAGC
CC

GGCCCGGTGATCAAGCTGTTCGGCGAGCACTGGTGCGGTCCGGAGAGCCTCG
CG

TCGGAGTCGGAGGCGTACGCGGTCCTGGCGGACGCCCCGGTGCCGGTGCCCC
GC

CTCCTCGGCCGCGGCGAGCTGCGGCCCGGCACCGGAGCCTGGCCGTGGCCCTA
CC

TGGTGATGAGCCGGATGACCGGCACCACCTGGCGGTCCGCGATGGACGGCAC
GA

CCGACCGGAACGCGCTGCTCGCCCTGGCCCGCGAACTCGGCCGGGTGCTCGGC
CG

GCTGCACAGGGTGCCGCTGACCGGGAACACCGTGCTCACCCCCCATTCCGAGG
TC

TTCCCGGAACTGCTGCGGGAACGCCGCGCGGCGACCGTCGAGGACCACCGCG
GG

TGGGGCTACCTCTCGCCCCGGCTGCTGGACCGCCTGGAGGACTGGCTGCCGGA
CG

TGGACACGCTGCTGGCCGGCCGCGAACCCCGGTTCGTCCACGGCGACCTGCAC
GG

GACCAACATCTTCGTGGACCTGGCCGCGACCGAGGTCACCGGGATCGTCGACT
TC

ACCGACGTCTATGCGGGAGACTCCCGCTACAGCCTGGTGCAACTGCATCTCAA
CG

CCTTCCGGGGCGACCGCGAGATCCTGGCCGCGCTGCTCGACGGGGCGCAGTG
GA

AGCGGACCGAGGACTTCGCCCGCGAACTGCTCGCCTTCACCTTCCTGCACGAC
TT

CGAGGTGTTCGAGGAGACCCCGCTGGATCTCTCCGGCTTCACCGATCCGGAGG
AA

CTGGCGCAGTTCCTCTGGGGGCCGCCGGACACCGCCCCCGGCGCCTGATAAGG
AT

CCGGCAAGACTGGCCCCGCTTGGCAACGCAACAGTGAGCCCCTCCCTAGTGTG
TT

TGGGGATGTGACTATGTATTCGTGTGTTGGCCAACGGGTCAACCCGAACAGAT
TG ATACCCGCCTTGGCATTCCTGTCAGAATGTAACGTCAGTTGATGGTACT

For all modified Chlamydomonas reinhardtii cells, Applicants use PCR, SURVEYOR nuclease assay, and DNA sequencing to verify successful modification.

*Example 25: Use of Cas9 to target a variety of disease types*

Diseases that involve mutations in protein coding sequence:
Dominant disorders may be targeted by inactivating the dominant negative allele. Applicants use Cas9 to target a unique sequence in the dominant negative allele and introduce a mutation via NHEJ. The NHEJ-induced indel may be able to introduce a frame-shift mutation in the dominant negative allele and eliminate the dominant negative protein. This may work if the gene is haplo-sufficient (e.g. MYOC mutation induced glaucoma and Huntington's disease).
Recessive disorders may be targeted by repairing the disease mutation in both alleles. For dividing cells, Applicants use Cas9 to introduce double strand breaks near the mutation site and increase the rate of homologous recombination using an exogenous recombination template. For dividing cells, this may be achieved using multiplexed nickase activity to catalyze the replacement of the mutant sequence in both alleles via NHEJ-mediated ligation of an exogenous DNA fragment carrying complementary overhangs.
Applicants also use Cas9 to introduce protective mutations (e.g. inactivation of CCR5 to prevent HIV infection, inactivation of PCSK9 for cholesterol reduction, or introduction of the A673T into APP to reduce the likelihood of Alzheimer's disease).

**Diseases that involve non-coding sequences**

Applicants use Cas9 to disrupt non-coding sequences in the promoter region, to alter transcription factor binding sites and alter enhancer or repressor elements. For example, Cas9 may be used to excise out the Klf1 enhancer EHS1 in hematopoietic stem cells to reduce BCL11a levels and reactivate fetal globin gene expression in differentiated erythrocytes
Applicants also use Cas9 to disrupt functional motifs in the 5' or 3' untranslated regions. For example, for the treatment of myotonic dystrophy, Cas9 may be used to remove CTG repeat expansions in the DMPK gene.

*Example 26: Multiplexed Nickase*

Aspects of optimization and the teachings of Cas9 detailed in this application may also be used to generate Cas9 nickases. Applicants use Cas9 nickases in combination with pairs of guide RNAs to generate DNA double strand breaks with defined overhangs. When two pairs of guide RNAs are used, it is possible to excise an intervening DNA fragment. If an exogenous piece of DNA is cleaved by the two pairs of guide RNAs to generate compatible overhangs with the genomic DNA, then the exogenous DNA fragment may be ligated into the genomic DNA to replace the excised fragment. For example, this may be used to remove trinucleotide repeat expansion in the huntintin (HTT) gene to treat Huntington's Disease.
If an exogenous DNA that bears fewer number of CAG repeats is provided, then it may be able to generate a fragment of DNA that bears the same overhangs and can be ligated into the HTT genomic locus and replace the excised fragment.

| HTT locus with fragment excised by Cas9 nickase and two pairs of guide RNAs | ...CCGTGCCGGGCGGGAGACCGCCATGG ...GGCACGGCCCGCCCTCTGGG | GGCCCGGCTGTGGCTGAGGAGC... TGGGCCGGGCCGACACCGACTCCTGC.. |
|---|---|---|

+

CGACCCTGGAAA....reduced number of CAG repeats.....CCCCGCCGCCACCC

GGTACCGCTGGGACCTTT....  .....GGGGCGGCGG

exogenous DNA fragment with fewer number of CAG repeats also cleaved by Cas9 nicakse and the two pairs of guide RNAs

The ligation of the exogenous DNA fragment into the genome does not require homologous recombination machineries and therefore this method may be used in post-mitotic cells such as neurons.

*Example 26: Delivery of CRISPR System*

Cas9 and its chimeric guide RNA, or combination of tracrRNA and crRNA, can be delivered either as DNA or RNA. Delivery of Cas9 and guide RNA both as RNA (normal or containing base or backbone modifications) molecules can be used to reduce the amount of time that Cas9 protein persist in the cell. This may reduce the level of off-target cleavage activity in the target cell. Since delivery of Cas9 as mRNA takes time to be translated into protein, it might be advantageous to deliver the guide RNA several hours following the delivery of Cas9 mRNA, to maximize the level of guide RNA available for interaction with Cas9 protein.

In situations where guide RNA amount is limiting, it may be desirable to introduce Cas9 as mRNA and guide RNA in the form of a DNA expression cassette with a promoter driving the expression of the guide RNA. This way the amount of guide RNA available will be amplified via transcription.

A variety of delivery systems can be introduced to introduce Cas9 (DNA or RNA) and guide RNA (DNA or RNA) into the host cell. These include the use of liposomes, viral vectors, electroporation, nanoparticles , nanowires (Shalek et al., Nano Letters, 2012), exosomes. Molecular trojan horses liposomes (Pardridge et al., Cold Spring Harb Protoc; 2010; doi:10.1101/pdb.prot5407) may be used to deliver Cas9 and guide RNA across the blood brain barrier.

*Example 28: Therapeutic strategies for Trinucleotide repeat disorders*

As previously mentioned in the application, the target polynucleotide of a CRISPR complex may include a number of disease-associated genes and polynucleotides and some of these disease associated gene may belong to a set of genetic disorders referred to as Trinucleotide repeat disorders (referred to as also trinucleotide repeat expansion disorders, triplet repeat expansion disorders or codon reiteration disorders).

These diseases are caused by mutations in which the trinucleotide repeats of certain genes exceed the normal, stable threshold which may usually differ in a gene. The discovery of more repeat expansion disorders has allowed for the classification of these disorders into a number of categories based on underlying similar characteristics. Huntington's disease (HD) and the spinocerebellar ataxias that are caused by a CAG repeat expansion in protein-coding portions of specific genes are included in Category I. Diseases or disorders with expansions that tend to make them phenotypically diverse and include expansions are usually small in magnitude and also found in exons of genes are included in Category II. Category III includes disorders or diseases which are characterized by much larger repeat expansions than either Category I or II and are generally located outside protein coding regions. Examples of Category III diseases or disorders include but are not limited to Fragile X syndrome, myotonic dystrophy, two of the spinocerebellar ataxias, juvenile myoclonic epilepsy, and Friedreich's ataxia.

Similar therapeutic strategies, like the one mentioned for Friedreich's ataxia below may be adopted to address other trinucleotide repeat or expansion disorders as well. For example, another triple repeat disease that can be treated using almost identical strategy is dystrophia myotonica. 1 (DM1), where there is an expanded CTG motif in the 3' UTR. In Friedreich's ataxia, the disease results from expansion of GAA trinucleotides in the first intron of frataxin (FXN). One therapeutic strategy using CRISPR is to excise the GAA repeat from the first intron. The expanded GAA repeat is thought to affect the DNA structure and leads to recruit the formation of heterochromatin which turn off the frataxin gene (Fig. 32A).

Competitive Advantage over other therapeutic strategies are listed below:

siRNA knockdown is not applicable in this case, as disease is due to reduced expression of frataxin. Viral gene therapy is currently being explored. HSV-1 based vectors were used to deliver the frataxin gene in animal models and have shown therapeutic effect. However, long term efficacy of virus-based frataxin delivery suffer from several problems: First, it is difficult to regulate the expression of frataxin to match natural levels in health individuals, and second, long term over expression of frataxin leads to cell death.

Nucleases may be used to excise the GAA repeat to restore healthy genotype, but Zinc Finger Nuclease and TALEN strategies require delivery of two pairs of high efficacy nucleases, which is difficult for both delivery as well as nuclease engineering (efficient excision of genomic DNA by ZFN or TALEN is difficult to achieve).

In contrast to above strategies, the CRISPR-Cas system has clear advantages. The Cas9 enzyme is more efficient and more multiplexible, by which it is meant that one or more targets can be set at the same time. So far, efficient excision of genomic DNA > 30% by Cas9 in human cells and may be as high as 30%, and may be improved in the future. Furthermore, with regard to certain trinucleotide repeat disorders like Huntington's disease (HD), trinucleotide repeats in the coding region may be addressed if there are differences between the two alleles. Specifically, if a HD patient is heterozygous for mutant HTT and there are nucleotide differences such as SNPs between the wt and mutant HTT alleles, then Cas9 may be used to specifically target the mutant HTT allele. ZFN or TALENs will not have the ability to distinguish two alleles based on single base differences.

In adopting a strategy using the CRISPR-Cas 9 enzyme to address Friedreich's ataxia, Applicants design a number of guide RNAs targeting sites flanking the GAA expansion and the most efficient and specific ones are chosen (Fig. 32B).

Applicants deliver a combination of guide RNAs targeting the intron 1 of FXN along with Cas9 to mediate excision of the GAA expansion region. AAV9 may be used to mediate efficient delivery of Cas9 and in the spinal cord.

If the Alu element adjacent to the GAA expansion is considered important, there may be constraints to the number of sites that can be targeted but Applicants may adopt strategies to avoid disrupting it.

Alternative Strategies:

Rather than modifying the genome using Cas9, Applicants may also directly activate the FXN gene using Cas9 (nuclease activity deficient)-based DNA binding domain to target a transcription activation domain to the FXN gene. Applicants may have to address the robustness of the Cas9-mediated artificial transcription activation to ensure that it is robust enough as compared to other methods (Tremblay et al., Transcription Activator-Like Effector Proteins Induce the Expression of the Frataxin Gene; Human Gene Therapy. August 2012, 23(8): 883-890.)

*Example 29: Strategies for minimizing off-target cleavage using Cas9 nickase*

As previously mentioned in the application, Cas9 may be mutated to mediate single strand cleavage via one or more of the following mutations: D10A, E762A, and H840A.

To mediate gene knockout via NHEJ, Applicants use a nickase version of Cas9 along with two guide RNAs. Off-target nicking by each individual guide RNA may be primarily repaired without mutation, double strand breaks (which can lead to mutations via NHEJ) only occur when the target sites are adjacent to each other. Since double strand breaks introduced by double nicking are not blunt, co-expression of end-processing enzymes such as TREX1 will increase the level of NHEJ activity.

The following list of targets in tabular form are for genes involved in the following diseases:

Lafora's Disease - target GSY1 or PPP1R3C (PTG) to reduce glycogen in neurons.

Hypercholesterolemia - target PCSK9

Target sequences are listed in pairs (L and R) with different number of nucleotides in the spacer (0 to 3bp). Each spacer may also be used by itself with the wild type Cas9 to introduce double strand break at the target locus.

| Gene | Primer | Sequence |
|---|---|---|
| GYS1 (human) | GGCC-L | ACCCTTGTTAGCCACCTCCC |
| | GGCC-R | GAACGCAGTGCTCTTCGAAG |
| | GGNCC-L | CTCACGCCCTGCTCCGTGTA |
| | GGNCC-R | GGCGACAACTACTTCCTGGT |
| | GGNNCC-L | CTCACGCCCTGCTCCGTGTA |
| | GGNNCC-R | GGGCGACAACTACTTCCTGG |
| | GGNNNCC-L | CCTCTTCAGGGCCGGGGTGG |
| | GGNNNCC-R | GAGGACCCAGGTGGAACTGC |
| PCSK9 (human) | GGCC-L | TCAGCTCCAGGCGGTCCTGG |
| | GGCC-R | AGCAGCAGCAGCAGTGGCAG |
| | GGNCC-L | TGGGCACCGTCAGCTCCAGG |
| | GGNCC-R | CAGCAGTGGCAGCGGCCACC |
| | GGNNCC-L | ACCTCTCCCCTGGCCCTCAT |
| | GGNNCC-R | CCAGGACCGCCTGGAGCTGA |
| | GGNNNCC-L | CCGTCAGCTCCAGGCGGTCC |
| | GGNNNCC-R | AGCAGCAGCAGCAGTGGCAG |
| PPP1R3C (PTG) (human) | GGCC-L | ATGTGCCAAGCAAAGCCTCA |
| | GGCC-R | TTCGGTCATGCCCGTGGATG |
| | GGNCC-L | GTCGTTGAAATTCATCGTAC |
| | GGNCC-R | ACCACCTGTGAAGAGTTTCC |
| | GGNNCC-L | CGTCGTTGAAATTCATCGTA |
| | GGNNCC-R | ACCACCTGTGAAGAGTTTCC |
| Gys1 (mouse) | GGCC-L | GAACGCAGTGCTTTTCGAGG |
| | GGCC-R | ACCCTTGTTGGCCACCTCCC |
| | GGNCC-L | GGTGACAACTACTATCTGGT |
| | GGNCC-R | CTCACACCCTGCTCCGTGTA |
| | GGNNCC-L | GGGTGACAACTACTATCTGG |
| | GGNNCC-R | CTCACACCCTGCTCCGTGTA |
| | GGNNNCC-L | CGAGAACGCAGTGCTTTTCG |
| | GGNNNCC-R | ACCCTTGTTGGCCACCTCCC |
| PPP1R3C (PTG) (mouse) | GGCC-L | ATGAGCCAAGCAAATCCTCA |
| | GGCC-R | TTCCGTCATGCCCGTGGACA |
| | GGNCC-L | CTTCGTTGAAAACCATTGTA |
| | GGNCC-R | CCACCTCTGAAGAGTTTCCT |
| | GGNNCC-L | CTTCGTTGAAAACCATTGTA |
| | GGNNCC-R | ACCACCTCTGAAGAGTTTCC |
| | GGNNNCC-L | CTTCCACTCACTCTGCGATT |

(continued)

| | | |
|---|---|---|
| PCSK9 (mouse) | L GGNNCC-R | ACCATGTCTCAGTGTCAAGC GGCGGCAACAGCGGCAACAG ACTGCTCTGCGTGGCTGCGG |
| | GGCC-L GGCC-R GGNNCC-L GGNNCC-R | CCGCAGCCACGCAGAGCAGT GCACCTCTCCTCGCCCCGAT |

Alternative strategies for improving stability of guide RNA and increasing specificity

1. Nucleotides in the 5' of the guide RNA may be linked via thiolester linkages rather than phosphoester linkage like in natural RNA. Thiolester linkage may prevent the guide RNA from being digested by endogenous RNA degradation machinery.
2. Nucleotides in the guide sequence (5' 20bp) of the guide RNA can use bridged nucleic acids (BNA) as the bases to improve the binding specificity.

*Example 30: CRISPR-Cas for rapid, multiplex genome editing*

Aspects of the disclosure relate to protocols and methods by which efficiency and specificity of gene modification may be tested within 3-4 days after target design, and modified clonal cell lines may be derived within 2-3 weeks.

Programmable nucleases are powerful technologies for mediating genome alteration with high precision. The RNA-guided Cas9 nuclease from the microbial CRISPR adaptive immune system can be used to facilitate efficient genome editing in eukaryotic cells by simply specifying a 20-nt targeting sequence in its guide RNA. Applicants describe a set of protocols for applying Cas9 to facilitate efficient genome editing in mammalian cells and generate cell lines for downstream functional studies. Beginning with target design, efficient and specific gene modification can be achieved within 3-4 days, and modified clonal cell lines can be derived within 2-3 weeks.

The ability to engineer biological systems and organisms holds enormous potential for applications across basic science, medicine, and biotechnology. Programmable sequence-specific endonucleases that facilitate precise editing of endogenous genomic loci are now enabling systematic interrogation of genetic elements and causal genetic variations in a broad range of species, including those that have not been genetically tractable previously. A number of genome editing technologies have emerged in recent years, including zinc finger nucleases (ZFNs), transcription activator-like effector nucleases (TALENs), and the RNA-guided CRISPR-Cas nuclease system. The first two technologies use a common strategy of tethering endonuclease catalytic domains to modular DNA-binding proteins for inducing targeted DNA double stranded breaks (DSB) at specific genomic loci. By contrast, Cas9 is a nuclease guided by small RNAs through Watson-Crick base-pairing with target DNA, presenting a system that is easy to design, efficient, and well-suited for high-throughput and multiplexed gene editing for a variety of cell types and organisms. Here Applicants describe a set of protocols for applying the recently developed Cas9 nuclease to facilitate efficient genome editing in mammalian cells and generate cell lines for downstream functional studies.

Like ZFNs and TALENs, Cas9 promotes genome editing by stimulating DSB at the target genomic loci. Upon cleavage by Cas9, the target locus undergoes one of two major pathways for DNA damage repair, the error-prone non-homologous end joining (NHEJ) or the high-fidelity homology directed repair (HDR) pathway. Both pathways may be utilized to achieve the desired editing outcome.

NHEJ: In the absence of a repair template, the NHEJ process re-ligates DSBs, which may leave a scar in the form of indel mutations. This process can be harnessed to achieve gene knockouts, as indels occurring within a coding exon may lead to frameshift mutations and a premature stop codon. Multiple DSBs may also be exploited to mediate larger deletions in the genome.

HDR: Homology directed repair is an alternate major DNA repair pathway to NHEJ. Although HDR typically occurs at lower frequencies than NHEJ, it may be harnessed to generate precise, defined modifications at a target locus in the presence of an exogenously introduced repair template. The repair template may be either in the form of double stranded DNA, designed similarly to conventional DNA targeting constructs with homology arms flanking the insertion sequence, or single-stranded DNA oligonucleotides (ssODNs). The latter provides an effective and simple method for making small edits in the genome, such as the introduction of single nucleotide mutations for probing causal genetic variations. Unlike NHEJ, HDR is generally active only in dividing cells and its efficiency varies depending on the cell type and state.

Overview of CRISPR: The CRISPR-Cas system, by contrast, is at minimum a two-component system consisting of the Cas9 nuclease and a short guide RNA. Re-targeting of Cas9 to different loci or simultaneous editing of multiple genes simply requires cloning a different 20-bp oligonucleotide. Although specificity of the Cas9 nuclease has yet to be thoroughly elucidated, the simple Watson-Crick base-pairing of the CRISPR-Cas system is likely more predictable than that of ZFN or TALEN domains.

The type II CRISPR-Cas (clustered regularly interspaced short palindromic repeats) is a bacterial adaptive immune system that uses Cas9, to cleave foreign genetic elements. Cas9 is guided by a pair of non-coding RNAs, a variable crRNA and a required auxiliary tracrRNA. The crRNA contains a 20-nt guide sequence determines specificity by locating the target DNA via Watson-Crick base-pairing. In the native bacterial system, multiple crRNAs are co-transcribed to direct Cas9 against various targets. In the CRISPR-Cas system derived from *Streptococcus pyogenes,* the target DNA must immediately precede a 5'-NGG/NRG protospacer adjacent motif (PAM), which can vary for other CRISPR systems.

CRISPR-Cas is reconstituted in mammalian cells through the heterologous expression of human codon-optimized Cas9 and the requisite RNA components. Furthermore, the crRNA and tracrRNA can be fused to create a chimeric, synthetic guide RNA (sgRNA). Cas9 can thus be re-directed toward any target of interest by altering the 20-nt guide sequence within the sgRNA.

Given its ease of implementation and multiplex capability, Cas9 has been used to generate engineered eukaryotic cells carrying specific mutations via both NHEJ and HDR. In addition, direct injection of sgRNA and mRNA encoding Cas9 into embryos has enabled the rapid generation of transgenic mice with multiple modified alleles; these results hold promise for editing organisms that are otherwise genetically intractable.

A mutant Cas9 carrying a disruption in one of its catalytic domains has been engineered to nick rather than cleave DNA, allowing for single-stranded breaks and preferential repair through HDR, potentially ameliorating unwanted indel mutations from off-target DSBs. Additionally, a Cas9 mutant with both DNA-cleaving catalytic residues mutated has been adapted to enable transcriptional regulation in *E. coli,* demonstrating the potential of functionalizing Cas9 for diverse applications. Certain aspects of the disclosure relate to the construction and application of Cas9 for multiplexed editing of human cells.

Applicants have provided a human codon-optimized, nuclear localization sequence-flanked Cas9 to facilitate eukaryotic gene editing. Applicants describe considerations for designing the 20-nt guide sequence, protocols for rapid construction and functional validation of sgRNAs, and finally use of the Cas9 nuclease to mediate both NHEJ- and HDR-based genome modifications in human embryonic kidney (HEK-293FT) and human stem cell (HUES9) lines. This protocol can likewise be applied to other cell types and organisms.

Target selection for sgRNA: There are two main considerations in the selection of the 20-nt guide sequence for gene targeting: 1) the target sequence should precede the 5'-NGG PAM for S. pyogenes Cas9, and 2) guide sequences should be chosen to minimize off-target activity. Applicants provided an online Cas9 targeting design tool that takes an input sequence of interest and identifies suitable target sites. To experimentally assess off-target modifications for each sgRNA, Applicants also provide computationally predicted off-target sites for each intended target, ranked according to Applicants' quantitative specificity analysis on the effects of base-pairing mismatch identity, position, and distribution.

The detailed information on computationally predicted off-target sites is as follows:
Considerations for Off-target Cleavage Activities: Similar to other nucleases, Cas9 can cleave off-target DNA targets in the genome at reduced frequencies. The extent to which a given guide sequence exhibit off-target activity depends on a combination of factors including enzyme concentration, thermodynamics of the specific guide sequence employed, and the abundance of similar sequences in the target genome. For routine application of Cas9, it is important to consider ways to minimize the degree of off-target cleavage and also to be able to detect the presence of off-target cleavage.

Minimizing off-target activity: For application in cell lines, Applicants recommend following two steps to reduce the degree of off-target genome modification. First, using Applicants' online CRISPR target selection tool, it is possible to computationally assess the likelihood of a given guide sequence to have off-target sites. These analyses are performed through an exhaustive search in the genome for off-target sequences that are similar sequences as the guide sequence. Comprehensive experimental investigation of the effect of mismatching bases between the sgRNA and its target DNA revealed that mismatch tolerance is 1) position dependent - the 8-14 bp on the 3' end of the guide sequence are less tolerant of mismatches than the 5' bases, 2) quantity dependent - in general more than 3 mismatches are not tolerated, 3) guide sequence dependent - some guide sequences are less tolerant of mismatches than others, and 4) concentration dependent - off-target cleavage is highly sensitive to the amount of transfected DNA. The Applicants' target site analysis web tool (available at the website genome-engineering.org/tools) integrates these criteria to provide predictions for likely off-target sites in the target genome. Second, Applicants recommend titrating the amount of Cas9 and sgRNA expression plasmid to minimize off-target activity.

Detection of off-target activities: Using Applicants' CRISPR targeting web tool, it is possible to generate a list of most likely off-target sites as well as primers performing SURVEYOR or sequencing analysis of those sites. For isogenic clones generated using Cas9, Applicants strongly recommend sequencing these candidate off-target sites to check for any undesired mutations. It is worth noting that there may be off target modifications in sites that are not included in the predicted candidate list and full genome sequence should be performed to completely verify the absence of off-target sites. Furthermore, in multiplex assays where several DSBs are induced within the same genome, there may be low rates of translocation events and can be evaluated using a variety of techniques such as deep sequencing.

The online tool provides the sequences for all oligos and primers necessary for 1) preparing the sgRNA constructs, 2) assaying target modification efficiency, and 3) assessing cleavage at potential off-target sites. It is worth noting that because the U6 RNA polymerase III promoter used to express the sgRNA prefers a guanine (G) nucleotide as the first base of its transcript, an extra G is appended at the 5' of the sgRNA where the 20-nt guide sequence does not begin with G.

Approaches for sgRNA construction and delivery: Depending on the desired application, sgRNAs may be delivered as either 1) PCR amplicons containing an expression cassette or 2) sgRNA-expressing plasmids. PCR-based sgRNA delivery appends the custom sgRNA sequence onto the reverse PCR primer used to amplify a U6 promoter template. The resulting amplicon may be co-transfected with a plasmid containing Cas9 (PX165). This method is optimal for rapid screening of multiple candidate sgRNAs, as cell transfections for functional testing can be performed mere hours after obtaining the sgRNA-encoding primers. Because this simple method obviates the need for plasmid-based cloning and sequence verification, it is well suited for testing or co-transfecting a large number of sgRNAs for generating large knockout libraries or other scale-sensitive applications. Note that the sgRNA-encoding primers are over 100-bp, compared to the ~20-bp oligos required for plasmid-based sgRNA delivery.

Construction of an expression plasmid for sgRNA is also simple and rapid, involving a single cloning step with a pair of partially complementary oligonucleotides. After annealing the oligo pairs, the resulting guide sequences may be inserted into a plasmid bearing both Cas9 and an invariant scaffold bearing the remainder of the sgRNA sequence (PX330). The transfection plasmids may also be modified to enable virus production for *in vivo* delivery.

In addition to PCR and plasmid-based delivery methods, both Cas9 and sgRNA can be introduced into cells as RNA.

Design of repair template: Traditionally, targeted DNA modifications have required use of plasmid-based donor repair templates that contain homology arms flanking the site of alteration. The homology arms on each side can vary in length, but are typically longer than 500 bp. This method can be used to generate large modifications, including insertion of reporter genes such as fluorescent proteins or antibiotic resistance markers. The design and construction of targeting plasmids has been described elsewhere.

More recently, single-stranded DNA oligonucleotides (ssODNs) have been used in place of targeting plasmids for short modifications within a defined locus without cloning. To achieve high HDR efficiencies, ssODNs contain flanking sequences of at least 40 bp on each side that are homologous to the target region, and can be oriented in either the sense or antisense direction relative to the target locus.

Functional testing

SURVEYOR nuclease assay: Applicants detected indel mutations either by the SURVEYOR nuclease assay (or PCR amplicon sequencing. Applicants online CRISPR target design tool provides recommended primers for both approaches. However, SURVEYOR or sequencing primers may also be designed manually to amplify the region of interest from genomic DNA and to avoid non-specific amplicons using NCBI Primer-BLAST. SURVEYOR primers should be designed to amplify 300-400 bp (for a 600-800 bp total amplicon) on either side of the Cas9 target for allowing clear visualization of cleavage bands by gel electrophoresis. To prevent excessive primer dimer formation, SURVEYOR primers should be designed to be typically under 25-nt long with melting temperatures of ~60°C. Applicants recommend testing each pair of candidate primers for specific PCR amplicons as well as for the absence of non-specific cleavage during the SURVEYOR nuclease digestion process.

Plasmid- or ssODN-mediated HDR: HDR can be detected via PCR-amplification and sequencing of the modified region. PCR primers for this purpose should anneal outside the region spanned by the homology arms to avoid false detection of residual repair template (HDR Fwd and Rev, Fig. 30). For ssODN-mediated HDR, SURVEYOR PCR primers can be used.

Detection of indels or HDR by sequencing: Applicants detected targeted genome modifications by either Sanger or next-generation deep sequencing (NGS). For the former, genomic DNA from modified region can be amplified using either SURVEYOR or HDR primers. Amplicons should be subcloned into a plasmid such as pUC19 for transformation; individual colonies can be sequenced to reveal clonal genotype.

Applicants designed next-generation sequencing (NGS) primers for shorter amplicons, typically in the 100-200 bp size range. For detecting NHEJ mutations, it is important to design primers with at least 10-20 bp between the priming regions and the Cas9 target site to allow detection of longer indels. Applicants provide guidelines for a two-step PCR method to attach barcoded adapters for multiplex deep sequencing. Applicants recommend the Illumina platform, due to its generally low levels of false positive indels. Off-target analysis (described previously) can then be performed through read alignment programs such as ClustalW, Geneious, or simple sequence analysis scripts.

Materials and Reagents

**sgRNA preparation:**

UltraPure DNaseRNase-free distilled water (Life Technologies, cat. no. 10977-023)
Herculase II fusion polymerase (Agilent Technologies, cat. no. 600679)
CRITICAL. Standard Taq polymerase, which lacks 3'-5' exonuclease proofreading activity, has lower fidelity and can lead to amplification errors. Herculase II is a high-fidelity polymerase (equivalent fidelity to Pfu) that produces high yields of PCR product with minimal optimization. Other high-fidelity polymerases may be substituted.
Herculase II reaction buffer (5x; Agilent Technologies, included with polymerase)
dNTP solution mix (25 mM each; Enzymatics, cat. no. N205L)
MgCl2 (25mM; ThermoScientific, cat no. R0971)
QIAquick gel extraction kit (Qiagen, cat. no. 28704)
QIAprep spin miniprep kit (Qiagen, cat. no. 27106)
UltraPure TBE buffer (10X; Life Technologies, cat. no. 15581-028)
SeaKem LE agarose (Lonza, cat. no. 50004)
SYBR Safe DNA stain (10,000x; Life Technologies, cat. no. S33102)
1-kb Plus DNA ladder (Life Technologies, cat. no. 10787-018)
TrackIt CyanOrange loading buffer (Life Technologies, cat no. 10482-028)
FastDigest BbsI (BpiI) (Fermentas/ThermoScientific, cat. no. FD1014)
Fermentas Tango Buffer (Fermentas/ThermoScientific, cat no. BY5)
DL-dithiothreitol (DTT; Fermentas/ThermoScientific, cat. no. R0862)
T7 DNA ligase (Enzymatics, cat. no. L602L)
Critical:Do not substitute the more commonly used T4 ligase. T7 ligase has 1,000-fold higher activity on the sticky ends than on the blunt ends and higher overall activity than commercially available concentrated T4 ligases.
T7 2X Rapid Ligation Buffer (included with T7 DNA ligase, Enzymatics, cat no. L602L)
T4 Polynucleotide Kinase (New England Biolabs, cat. no M0201S)
T4 DNA Ligase Reaction Buffer (10X; New England Biolabs, cat no B0202S)
Adenosine 5'-triphosphate (10 mM; New England Biolabs, cat no. P0756S)
PlasmidSafe ATP-dependent DNase (Epicentre, cat. no. E3101K)
One Shot Stbl3 chemically competent Escherichia coli (E. coli) (Life Technologies, cat. no. C7373-03)
SOC medium (New England Biolabs, cat. no. B9020S)
LB medium (Sigma, cat no. L3022)
LB agar medium (Sigma, cat. no. L2897)
Ampicillin, sterile filtered (100 mg ml-1; Sigma, cat. no. A5354)

**Mammalian cell culture:**

HEK293FT cells (Life Technologies, cat no. R700-07)
Dulbecco's minimum Eagle's medium (DMEM, IX, high glucose; Life Technologies, cat. no. 10313-039)
Dulbecco's minimum Eagle's medium (DMEM, 1X, high glucose, no phenol red; Life Technologies, cat. no. 31053-028)
Dulbecco's phosphate-buffered saline (DPBS, IX; Life Technologies, cat. no. 14190-250)
Fetal bovine serum, qualified and heat inactivated (Life Technologies, cat. no. 10438-034)
Opti-MEM I reduced-serum medium (FBS; Life Technologies, cat. no. 11058-021)
Penicillin-streptomycin (100x; Life Technologies, cat. no. 15140-163)
TrypLE™ Express (1X, no Phenol Red; Life Technologies, cat no. 12604-013)
Lipofectamine 2000 transfection reagent (Life Technologies, cat. no. 11668027)
Amaxa SF Cell Line 4D-Nucleofector® X Kit S (32 RCT; Lonza, cat no V4XC-2032)
HUES 9 cell line (HARVARD STEM CELL SCIENCE)
Geltrex LDEV-Free Reduced Growth Factor Basement Membrane Matrix (Life Technologies, cat. no. A1413201)
mTeSR1 medium (Stemcell Technologies, cat no. 05850)
Accutase cell detachment solution (Stemcell Technologies, cat. no. 07920)
ROCK Inhibitor (Y-27632; Millipore, cat. no. SCM075)
Amaxa P3 Primary Cell 4D-Nucleofector® X Kit S (32 RCT; Lonza cat. no. V4XP-3032)

**Genotyping analysis:**

QuickExtract DNA extraction solution (Epicentre, cat no. QE09050)

PCR primers for SURVEYOR, RFLP analysis, or sequencing (see Primer table)

Herculase II fusion polymerase (Agilent Technologies, cat. no. 600679)

CRITICAL. As Surveyor assay is sensitive to single-base mismatches, it is particularly important to use a high-fidelity polymerase. Other high-fidelity polymerases may be substituted.

Herculase II reaction buffer (5x; Agilent Technologies, included with polymerase)

dNTP solution mix (25 mM each; Enzymatics, cat. no. N205L)

QIAquick gel extraction kit (Qiagen, cat. no. 28704)

Taq Buffer (10x; Genscript, cat. no. B0005)

SURVEYOR mutation detection kit for standard gel electrophoresis (Transgenomic, cat. no. 706025)

UltraPure TBE buffer (10x; Life Technologies, cat. no. 15581-028)

SeaKem LE agarose (Lonza, cat. no. 50004)

4-20% TBE Gels 1.0 mm, 15 Well (Life Technologies, cat no. EC62255BOX)

Novex® Hi-Density TBE Sample Buffer (5X; Life Technologies, cat no. LC6678)

SYBR Gold Nucleic Acid Gel Stain (10,000X; Life Technologies, cat. no. S-11494)

1-kb Plus DNA ladder (Life Technologies, cat. no. 10787-018)

TrackIt CyanOrange loading buffer (Life Technologies, cat. no. 10482-028)

FastDigest HindIII (Fermentas/ThermoScientific, cat. no. FD0504)

**Equipment**

Filtered sterile pipette tips (Corning)

Standard 1.5ml microcentrifuge tubes (Eppendorf, cat. no. 0030 125.150)

Axygen 96-well PCR plates (VWR, cat. no. PCR-96M2-HSC)

Axygen 8-Strip PCR tubes (Fischer Scientific, cat. no. 14-222-250)

Falcon tubes, polypropylene, 15 ml (BD Falcon, cat. no. 352097)

Falcon tubes, polypropylene, 50 ml (BD Falcon, cat. no. 352070)

Round-bottom Tube with cell strainer cap, 5ml (BD Falcon, cat. no. 352235)

Petri dishes (60 mm $\times$ 15 mm; BD Biosciences, cat. no. 351007)

Tissue culture plate (24 well; BD Falcon, cat no. 353047)

Tissue culture plate (96 well, flat bottom; BD Falcon, cat. no. 353075)

Tissue culture dish (100 mm; BD Falcon, 353003)

96-well thermocycler with programmable temperature stepping functionality (Applied Biosystems Veriti, cat. no. 4375786).

Desktop microcentrifuges 5424, 5804 (Eppendorf)

Gel electrophoresis system (PowerPac basic power supply, Bio-Rad, cat. no. 164-5050, and Sub-Cell GT System gel tray, Bio-Rad, cat. no. 170-4401)

Novex XCell SureLock Mini-Cell (Life Technologies, cat. no. EI0001)

Digital gel imaging system (GelDoc EZ, Bio-Rad, cat. no. 170-8270, and blue sample tray, Bio-Rad, cat. no. 170-8273)

Blue light transilluminator and orange filter goggles (SafeImager 2.0; Invitrogen, cat. no. G6600)

Gel quantification software (Bio-Rad, ImageLab, included with GelDoc EZ,or open-source ImageJ from the National Institutes of Health, available at the website rsbweb.nih.gov/ij/) UV spectrophotometer (NanoDrop 2000c, Thermo Scientific)

**Reagent Setup**

Tris-borate EDTA (TBE) electrophoresis solution Dilute TBE buffer in distilled water to 1X working solution for casting agarose gels and for use as a buffer for gel electrophoresis. Buffer may be stored at room temperature (18 - 22 °C) for at least 1 year.

• ATP, 10 mM Divide 10 mM ATP into 50-$\mu$l aliquots and store at - 20 °C for up to 1 year; avoid repeated freeze-thaw cycles.

• DTT, 10 mM Prepare 10 mM DTT solution in distilled water and store in 20-$\mu$l aliquots at - 70 °C for up to 2 years; for each reaction, use a new aliquot, as DTT is easily oxidized.

• D10 culture medium For culture of HEK293FT cells, prepare D10 culture medium by supplementing DMEM with 1X GlutaMAX and 10% (vol/vol) fetal bovine serum. As indicated in the protocol, this medium can also be supplemented with 1X penicillin-streptomycin . D 10 medium can be made in advance and stored at 4 °C for up to 1 month.

• mTeSR1 culture medium For culture of human embryonic stem cells, prepare mTeSR1 medium by supplementing the 5X supplement (included with mTeSR1 basal medium), and 100 ug/ml Normocin.

**Procedure**

Design of targeting components and use of the online tool • Timing 1 d

1| Input target genomic DNA sequence. Applicants provide an online Cas9 targeting design tool that takes an input sequence of interest, identifies and ranks suitable target sites, and computationally predicts off-target sites for each intended target. Alternatively, one can manually select guide sequence by identifying the 20-bp sequence directly upstream of any 5'-NGG.

2| Order necessary oligos and primers as specified by the online tool. If the site is chosen manually, the oligos and primers should be designed.

Preparation of sgRNA expression construct

3| To generate the sgRNA expression construct, either the PCR- or plasmid-based protocol can be used.

(A) via PCR amplification • Timing 2 h

(i) Applicants prepare diluted U6 PCR template. Applicants recommend using PX330 as a PCR template, but any U6-containing plasmid may likewise be used as the PCR template. Applicants diluted template with ddH$_2$O to a concentration of 10 ng/ul. Note that if a plasmid or cassette already containing an U6-driven sgRNA is used as a template, a gel extraction needs to be performed to ensure that the product contains only the intended sgRNA and no trace sgRNA carryover from template.

(ii) Applicants prepared diluted PCR oligos. U6-Fwd and U6-sgRNA-Rev primers are diluted to a final concentration of 10 uM in ddH$_2$O (add 10 ul of 100 uM primer to 90 ul ddH$_2$O).

(iii) U6-sgRNA PCR reaction. Applicants set up the following reaction for each U6-sgRNA-Rev primer and mastermix as needed:

| Component: | Amount (ul) | Final concentration |
|---|---|---|
| Herculase II PCR buffer, 5X | 10 | 1X |
| dNTP, 100mM (25mM each) | 0.5 | 1 mM |
| U6 template (PX330) | 1 | 0.2 ng/ul |
| U6-Fwd primer | 1 | 0.2 uM |
| U6-sgRNA-Rev primer (variable) | 1 | 0.2 uM |
| Herculase II Fusion polymerase | 0.5 | |
| Distilled water | 36 | |
| Total | 50 | |

(iv) Applicants performed PCR reaction on the reactions from step (iii) using the following cycling conditions:

| Cycle number | Denature | Anneal | Extend |
|---|---|---|---|
| 1 | 95°C, 2 m | | |
| 2-31 | 95°C, 20 s | 60°C, 20 s | 72°C, 20 s |
| 32 | | | 72°C, 3 m |

(v) After the reaction is completed, Applicants ran the product on a gel to verify successful, single-band amplification. Cast a 2% (wt/vol) agarose gel in 1X TBE buffer with 1X SYBR Safe dye. Run 5 ul of the PCR product in the gel at 15 V cm-1 for 20-30 min. Successful amplicons should yield one single 370-bp product and the template should be invisible. It should not be necessary to gel extract the PCR amplicon.

(vi) Applicants purified the PCR product using the QIAquick PCR purification kit according to the manufacturer's directions. Elute the DNA in 35 ul of Buffer EB or water. Purified PCR products may be stored at 4°C or -20°C.

**(B) Cloning sgRNA into Cas9-containing bicistronic expression vector • Timing 3 d**

(i) *Prepare the sgRNA oligo inserts.* Applicants resuspended the top and bottom strands of oligos for each sgRNA design to a final concentration of 100 uM. Phosphorylate and anneal the oligo as follows:

| | |
|---|---|
| Oligo 1 (100 uM) | 1 ul |
| Oligo 2 (100 uM) | 1 ul |
| T4 Ligation Buffer, 10X | 1 ul |
| T4 PNK | 1 ul |
| ddH$_2$O | 6 ul |
| Total | 10 ul |

(ii) Anneal in a thermocycler using the following parameters:

37°C for 30 m

95°C for 5 m

Ramp down to 25°C at 5°C per m

(iii) Applicants diluted phosphorylated and annealed oligos 1:200 by add lul of oligo to 199 ul room temperature ddH$_2$O.

(iv) *Clone sgRNA oligo into PX330.* Applicants set up Golden Gate reaction for each sgRNA. Applicants recommend also setting up a no-insert, PX330 only negative control.

| | |
|---|---|
| PX330 (100 ng) | x ul |
| Diluted oligo duplex from step (iii) | 2 ul |
| Tango Buffer, 10X | 2 ul |
| DTT, 10mM | 1 ul |
| ATP, 10mM | 1 ul |
| FastDigest BbsI | 1 ul |
| T7 Ligase | 0.5 ul |
| ddH$_2$O | x ul |
| Total | 20 ul |

(v) Incubate the Golden Gate reaction for a total of 1 h:

| Cycle number | Condition |
|---|---|
| 1-6 | 37°C for 5 m, 21°C for 5 m |

(vi) Applicants treated Golden Gate reaction with PlasmidSafe exonuclease to digest any residual linearized DNA. This step is optional but highly recommended.

| | |
|---|---|
| Golden Gate reaction from step 4 | 11 ul |
| 10X PlasmidSafe Buffer | 1.5 ul |
| ATP, 10 mM | 1.5 ul |
| PlasmidSafe exonuclease | 1 ul |
| Total | 15 ul |

(vii) Applicants incubated the PlasmidSafe reaction at 37°C for 30 min, followed by inactivation at 70°C for 30 min. **Pause point:** after completion, the reaction may be frozen and continued later. The circular DNA should be stable for at least 1 week.

(viii) *Transformation.* Applicants transformed the PlasmidSafe-treated plasmid into a competent *E. coli* strain, according to the protocol supplied with the cells. Applicants recommend Stbl3 for quick transformation. Briefly, Applicants added 5ul of the product from step (vii) into 20ul of ice-cold chemically competent Stbl3 cells. This is then incubated on ice for 10 m, heat shocked at 42°C for 30 s, returned immediately to ice for 2 m, 100 ul of SOC medium is added, and this is plated onto an LB plate containing 100 ug/ml ampicillin with incubation overnight at 37°C.

(ix) Day 2: Applicants inspected plates for colony growth. Typically, there are no colonies on the negative control plates (ligation of BbsI-digested PX330 only, no annealed sgRNA oligo), and tens to hundreds of colonies on the PX330-sgRNA cloning plates.

(x) From each plate, Applicants picked 2-3 colonies to check correct insertion of sgRNA. Applicants used a sterile pipette tip to inoculate a single colony into a 3 ml culture of LB medium with 100 ug/ml ampicillin. Incubate and shake at 37°C overnight.

(xi) Day 3: Applicants isolated plasmid DNA from overnight cultures using a QiAprep Spin miniprep kit according to the manufacturer's instructions.

(xii) *Sequence validate CRISPR plasmid.* Applicants verified the sequence of each colony by sequencing from the U6 promoter using the U6-Fwd primer. Optional: sequence the Cas9 gene using primers listed in the following Primer table.

| Primer | Sequence (5' to 3') | Purpose |
|---|---|---|
| U6-For | GAGGGCCTATTTCCCATGATTCC | Amplify U6-sgRNA |
| U6-Rev | AAAAAAAGCACCGACTCGGTGCCACTTTTTCAAGTTGATAACGGACTAGCCTTATTTTAACTTGCTATTTCTAG CTCTAAAACNNNNNNNNNNNNNNNNNNNNCCGGTGTTTCGTCCTTTCCACAAG | Amplify U6-sgRNA; N is reverse complement of target |
| sgRNA-top | CACCGNNNNNNNNNNNNNNNNNNNN | Clone sgRNA into PX330 |
| sgRNA-bottom | AAACNNNNNNNNNNNNNNNNNNNNC | Clone sgRNA into PX330 |
| U6-*EMX1*-Rev | AAAAAAAGCACCGACTCGGTGCCACTTTTTCAAGTTGATAACGGACTAGCCTTATTTTAACTTGCTATTTCTAG CTCTAAAACCCCTAGTCATTGGAGGTGACCGGTGTTTCGTCCTTTCCACAAG | Amplify U6-EMX1 sgRNA |
| *EMX1*-top | CACCGTCACCTCCAATGACTAGGG | Clone EMX1 sgRNA into PX330 |
| *EMX1*-bottom | AAACCCCTAGTCATTGGAGGTGAC | Clone EMX1 sgRNA into PX330 |
| ssODN-sense | CAGAAGAAGAAGGGCTCCCATCACATCAACCGGTGGCGCATTGCCACGAAGCAGGCCAATGGGGAGGACATC GATGTCACCTCCAATGAC<u>AAGCTTGCTAGC</u>GGTGGGCAACCACAAACCCACGAGGGCAGAGTGCTGCTTGCTG CTGGCCAGGCCCCTGCGTGGGCCCAAGCTGGACTCTGGCCACTCCCT | EMX1 HDR (sense; insertion underlined) |
| ssODN-antisense | AGGGAGTGGCCAGAGTCCAGCTTGGGCCCACGCAGGGGCCTGGCCAGCAGCAAGCAGCACTCTGCCCTCGTG GGTTTGTGGTTGCCCACC<u>GCTAGCAAGCTT</u>GTCATTGGAGGTGACATCGATGTCCTCCCCATTGGCCTGCTTCG TGGCAATGCGCCACCGGTTGATGTGATGGGAGCCCTTCTTCTTCTG | EMX1 HDR (antisense; insertion underlined) |

(continued)

| Primer | Sequence (5' to 3') | Purpose |
|---|---|---|
| EMX1-SURV-F | CCATCCCCTTCTGTGAATGT | EMX1 SURVEYOR assay PCR, sequencing |
| EMX1-SURV-R | GGAGATTGGAGACACGGAGA | EMX1 SURVEYOR assay PCR, sequencing |
| EMX1-HDR-F | GGCTCCCTGGGTTCAAAGTA | EMX1 RFLP analysis PCR, sequencing |
| EMX1-HDR-R | AGAGGGGTCTGGATGTCGTAA | EMX1 RFLP analysis PCR, sequencing |
| pUC19-F | CGCCAGGGTTTTCCCAGTCACGAC | pUC19 multiple cloning site F primer, for Sanger sequencing |

Applicants referenced the sequencing results against the PX330 cloning vector sequence to check that the 20 bp guide sequence was inserted between the U6 promoter and the remainder of the sgRNA scaffold. Details and sequence of the PX330 map in GenBank vector map format (*.gb file) can be found at the website crispr.genome-engineering.org.

**(Optional) Design of ssODN template • Timing 3 d planning ahead**

3| *Design and order ssODN.* Either the sense or antisense ssODN can be purchased directly from supplier. Applicants recommend designing homology arms of at least 40 bp on either side and 90 bp for optimal HDR efficiency. In Applicants' experience, antisense oligos have slightly higher modification efficiencies.

4| Applicants resuspended and diluted ssODN ultramers to a final concentration of 10 uM. Do not combine or anneal the sense and antisense ssODNs. Store at -20°C.

5| Note for HDR applications, Applicants recommend cloning sgRNA into the PX330 plasmid.

**Functional validation of sgRNAs: cell culture and transfections • Timing 3-4 d**

The CRISPR-Cas system has been used in a number of mammalian cell lines. Conditions may vary for each cell line. The protocols below details transfection conditions for HEK239FT cells. Note for ssODN-mediated HDR transfections, the Amaxa SF Cell Line Nucleofector Kit is used for optimal delivery of ssODNs. This is described in the next section.

7| *HEK293FT maintenance.* Cells are maintained according to the manufacturer's recommendations. Briefly, Applicants cultured cells in D10 medium (GlutaMax DMEM supplemented with 10% Fetal Bovine Serum), at 37°C and 5% CO2.

8| To passage, Applicants removed medium and rinsed once by gently adding DPBS to side of vessel, so as not to dislodge cells. Applicants added 2 ml of TrypLE to a T75 flask and incubated for 5 m at 37°C. 10 ml of warm D10 medium is added to inactivate and transferred to a 50 ml Falcon tube. Applicants dissociated cells by triturating gently, and re-seeded new flasks as necessary. Applicants typically passage cells every 2-3 d at a split ratio of 1:4 or 1:8, never allowing cells to reach more than 70% confluency. Cell lines are restarted upon reaching passage number 15.

9| *Prepare cells for transfection.* Applicants plated well-dissociated cells onto 24-well plates in D10 medium without antibiotics 16-24 h before transfection at a seeding density of 1.3 x $10^5$ cells per well and a seeding volume of 500 ul. Scale up or down according to the manufacturer's manual as needed. It is suggested to not plate more cells than recommended density as doing so may reduce transfection efficiency.

10| On the day of transfection, cells are optimal at 70-90% confluency. Cells may be transfected with Lipofectamine 2000 or Amaxa SF Cell Line Nucleofector Kit according to the manufacturers' protocols.

(A) For sgRNAs cloned into PX330, Applicants transfected 500 ng of sequence-verified CRISPR plasmid; if transfecting more than one plasmid, mix at equimolar ratio and no more than 500 ng total.

(B) For sgRNA amplified by PCR, Applicants mixed the following:

| | |
|---|---|
| PX165 (Cas9 only) | 200 ng |
| sgRNA amplicon (each) | 40 ng |
| pUC19 | fill up total DNA to 500 ng |

Applicants recommend transfecting in technical triplicates for reliable quantification and including transfection controls (e.g. GFP plasmid) to monitor transfection efficiency. In addition, PX330 cloning plasmid and/or sgRNA amplicon may be transfected alone as a negative control for downstream functional assays.

11| Applicants added Lipofectamine complex to cells gently as HEK293FT cells may detach easily from plate easily and result in lower transfection efficiency.

12| Applicants checked cells 24 h after transfection for efficiency by estimating the fraction of fluorescent cells in the control (e.g., GFP) transfection using a fluorescence microscope. Typically cells are more than 70% transfected.

13| Applicants supplemented the culture medium with an additional 500 ul of warm D10 medium. Add D10 very slowly to the side of the well and do not use cold medium, as cells can detach easily.

14| Cells are incubated for a total of 48-72 h post-transfection before harvested for indel analysis. Indel efficiency does not increase noticeably after 48 h.

**(Optional) Co-transfection of CRISPR plasmids and ssODNs or targeting plasmids for HR • Timing 3-4 d**

15| *Linearize targeting plasmid.* Targeting vector is linearized if possible by cutting once at a restriction site in the vector backbone near one of the homology arms or at the distal end of either homology arm.

16| Applicants ran a small amount of the linearized plasmid alongside uncut plasmid on a 0.8-1% agarose gel to check successful linearization. Linearized plasmid should run above the supercoiled plasmid.

17| Applicants purified linearized plasmid with the QIAQuick PCR Purification kit.

18| Prepare cells for transfection. Applicants cultured HEK293FT in T75 or T225 flasks. Sufficient cell count before day of transfection is planned for. For the Amaxa strip-cuvette format, $2 \times 10^6$ cells are used per transfection.

19| Prepare plates for transfection. Applicants added 1 ml of warm D10 medium into each well of a 12 well plate. Plates are placed into the incubator to keep medium warm.

20| Nucleofection. Applicants transfected HEK293FT cells according to the Amaxa SF Cell Line Nucleofector 4D Kit manufacturer's instructions, adapted in the steps below.

a. For ssODN and CRISPR cotransfection, pre-mix the following DNA in PCR tubes:

| | |
|---|---|
| pCRISPR plasmid (Cas9 + sgRNA) | 500 ng |
| ssODN template (10uM) | 1 ul |

b. For HDR targeting plasmid and CRISPR cotransfection, pre-mix the following DNA in PCR tubes:

| | |
|---|---|
| CRISPR plasmid (Cas9 + sgRNA) | 500 ng |
| Linearized targeting plasmid | 500 ng |

For transfection controls, see previous section. In addition, Applicants recommend transfecting ssODN or targeting plasmid alone as a negative control.

21| Dissociate to single cells. Applicants removed medium and rinsed once gently with DPBS, taking care not to dislodge cells. 2 ml of TrypLE is added to a T75 flask and incubated for 5 m at 37°C. 10 ml of warm D10 medium is added to inactivate and triturated gently in a 50 ml Falcon tube. It is recommended that cells are triturated gently and dissociated to single cells. Large clumps will reduce transfection efficiency. Applicants took a 10 ul aliquot from the suspension and diluted into 90 ul of D10 medium for counting. Applicants counted cells and calculated the number of cells and volume of suspension needed for transfection. Applicants typically transfected $2 \times 10^5$ cells per condition using the Amaxa Nucleocuvette strips, and recommend calculating for 20% more cells than required to adjust for volume loss in subsequent pipetting steps. The volume needed is transferred into a new Falcon tube.

231 Applicants spun down the new tube at 200 x g for 5 m.

Applicants prepared the transfection solution by mixing the SF solution and S1 supplement as recommended by Amaxa. For Amaxa strip-cuvettes, a total of 20 ul of supplemented SF solution is needed per transfection. Likewise, Applicants recommend calculating for 20% more volume than required.

251 Applicants removed medium completely from pelleted cells from step 23 and gently resuspended in appropriate volume (20 ul per 2 x 10$^5$ cells) of S1-supplemented SF solution. Do not leave cells in SF solution for extended period of time.

26| 20 ul of resuspended cells is pipetted into each DNA pre-mix from step 20. Pipette gently to mix and transfer to Nucleocuvette strip chamber. This is repeated for each transfection condition.

Electroporate cells using the Nucleofector 4D program recommended by Amaxa, CM-130.

28| Applicants gently and slowly pipetted 100 ul of warm D10 medium into each Nucleocuvette strip chamber, and transferred all volume into the pre-warmed plate from step 19. CRITICAL. Cells are very fragile at this stage and harsh pipetting can cause cell death. Incubate for 24 h. At this point, transfection efficiency can be estimated from fraction of fluorescent cells in positive transfection control. Nucleofection typically results in greater than 70-80% transfection efficiency. Applicants slowly added 1 ml warm D10 medium to each well without dislodging the cells. Incubate cells for a total of 72 h.

**Human embryonic stem cell (HUES 9) culture and transfection • Timing 3-4 d**

Maintaining hESC (HUES9) line. Applicants routinely maintain HUES9 cell line in feeder-free conditions with mTesR1 medium. Applicants prepared mTeSR1 medium by adding the 5X supplement included with basal medium and 100 ug/ml Normocin. Applicants prepared a 10 ml aliquot of mTeSR1 medium supplemented further with 10 uM Rock Inhibitor. Coat tissue culture plate. Dilute cold GelTrex 1:100 in cold DMEM and coat the entire surface of a 100 mm tissue culture plate.

Place plate in incubator for at least 30 m at 37°C. Thaw out a vial of cells at 37°C in a 15 ml Falcon tube, add 5 ml of mTeSR1 medium, and pellet at 200 x g for 5 m. Aspirate off GelTrex coating and seed ~1 x 106 cells with 10 ml mTeSR1 medium containing Rock Inhibitor. Change to normal mTeSR1 medium 24 h after transfection and re-feed daily. Passaging cells. Re-feed cells with fresh mTeSR1 medium daily and passage before reaching 70% confluency. Aspirate off mTeSR1 medium and wash cells once with DPBS. Dissociate cells by adding 2 ml Accutase and incubating at 37°C for 3 - 5 m. Add 10 ml mTeSR1 medium to detached cells, transfer to 15 ml Falcon tube and resuspend gently. Re-plate onto GelTrex-coated plates in mTeSR1 medium with 10 uM Rock Inhibitor. Change to normal mTeSR1 medium 24 h after plating.

Transfection. Applicants recommend culturing cells for at least 1 week post-thaw before transfecting using the Amaxa P3 Primary Cell 4-D Nucleofector Kit (Lonza). Re-feed log-phase growing cells with fresh medium 2 h before transfection. Dissociate to single cells or small clusters of no more than 10 cells with accutase and gentle resuspension. Count the number of cells needed for nucleofection and spin down at 200 x g for 5 m. Remove medium completely and resuspend in recommended volume of S1-supplemented P3 nucleofection solution. Gently plate electroporated cells into coated plates in presence of 1X Rock Inhibitor.

Check transfection success and re-feed daily with regular mTeSR1 medium beginning 24 h after nucleofection. Typically, Applicants observe greater than 70% transfection efficiency with Amaxa Nucleofection. Harvest DNA. 48-72 h post transfection, dissociate cells using accutase and inactivate by adding 5 x volume of mTeSR1. Spin cells down at 200 x g for 5 m. Pelleted cells can be directed processed for DNA extraction with QuickExtract solution. It is recommended to not mechanically dissociate cells without accutase. It is recommended to not spin cells down without inactivating accutase or above the recommended speed; doing so may cause cells to lyse.

**Isolation of clonal cell lines by FACS. Timing • 2-3 h hands-on; 2-3 weeks expansion**

Clonal isolation may be performed 24 h post-transfection by FACS or by serial dilution.

54| *Prepare FACS buffer.* Cells that do not need sorting using colored fluorescence may be sorted in regular D10 medium supplemented with 1X penicillin/streptinomycin. If colored fluorescence sorting is also required, a phenol-free DMEM or DPBS is substituted for normal DMEM. Supplement with 1X penicillin/streptinomycin and filter through a .22 um Steriflip filter.

55| *Prepare 96 well plates.* Applicants added 100 ul of D10 media supplemented with 1X penicillin/streptinomycin per well and prepared the number of plates as needed for the desired number of clones.

56| *Prepare cells for FACS.* Applicants dissociated cells by aspirating the medium completely and adding 100 ul TrypLE per well of a 24-well plate. Incubate for 5 m and add 400 ul warm D10 media.

57| Resuspended cells are transferred into a 15 ml Falcon tube and gently triturated 20 times. Recommended to check under the microscope to ensure dissociation to single cells.

58| Spin down cells at 200 x g for 5 minutes.

59| Applicants aspirated the media, and resuspended the cells in 200 ul of FACS media.

60| Cells are filtered through a 35 um mesh filter into labeled FACS tubes. Applicants recommend using the BD Falcon 12 x 75 mm Tube with Cell Strainer cap. Place cells on ice until sorting.

61| Applicants sorted single cells into 96-well plates prepared from step 55. Applicants recommend that in one single designated well on each plate, sort 100 cells as a positive control.

NOTE. The remainder of the cells may be kept and used for genotyping at the population level to gauge overall modification efficiency.

62| Applicants returned cells into the incubator and allowed them to expand for 2-3 weeks. 100 ul of warm D10 medium is added 5 d post sorting. Change 100 ul of medium every 3-5 d as necessary.

63| Colonies are inspected for "clonal" appearance 1 week post sorting: rounded colonies radiating from a central point. Mark off wells that are empty or may have been seeded with doublets or multiplets.

64| When cells are more than 60% confluent, Applicants prepared a set of replica plates for passaging. 100 ul of D10 medium is added to each well in the replica plates. Applicants dissociated cells directly by pipetting up and down vigorously 20 times. 20% of the resuspended volume was plated into the prepared replica plates to keep the clonal lines. Change the medium every 2-3 d thereafter and passage accordingly.

65| Use the remainder 80% of cells for DNA isolation and genotyping.

**Optional: Isolation of clonal cell lines by dilution. Timing • 2-3 h hands-on; 2-3 weeks expansion**

66| Applicants dissociated cells from 24-well plates as described above. Make sure to dissociate to single cells. A cell strainer can be used to prevent clumping of cells.

67| The number of cells are counted in each condition. Serially dilute each condition in D10 medium to a final concentration of 0.5 cells per 100 ul. For each 96 well plate, Applicants recommend diluting to a final count of 60 cells in 12 ml of D10. Accurate count of cell number is recommended for appropriate clonal dilution. Cells may be recounted at an intermediate serial dilution stage to ensure accuracy.

68| Multichannel pipette was used to pipette 100 ul of diluted cells to each well of a 96 well plate.

NOTE. The remainder of the cells may be kept and used for genotyping at the population level to gauge overall modification efficiency.

69| Applicants inspected colonies for "clonal" appearance ~1 week post plating: rounded colonies radiating from a central point Mark off wells that may have seeded with doublets or multiplets.

70| Applicants returned cells to the incubator and allowed them to expand for 2-3 weeks. Re-feed cells as needed as detailed in previous section.

**SURVEYOR assay for CRISPR cleavage efficiency. Timing • 5-6 h**

Before assaying cleavage efficiency of transfected cells, Applicants recommend testing each new SURVEYOR primer on negative (untransfected) control samples through the step of SURVEYOR nuclease digestion using the protocol described below. Occasionally, even single-band clean SURVEYOR PCR products can yield non-specific SURVEYOR nuclease cleavage bands and potentially interfere with accurate indel analysis.

71| *Harvest cells for DNA.* Dissociate cells and spin down at 200 x g for 5 m. NOTE. Replica plate at this stage as needed to keep transfected cell lines.

72| Aspirate the supernatant completely.

73| Applicants used QuickExtract DNA extraction solution according to the manufacturer's instructions. Applicants typically used 50 ul of the solution for each well of a 24 well plate and 10 ul for a 96 well plate.

74| Applicants normalized extracted DNA to a final concentration of 100-200 ng/ul with ddH2O. **Pause point:** Extracted DNA may be stored at -20°C for several months.

75| *Set up the SURVEYOR PCR.* Master mix the following using SURVEYOR primers provided by Applicants online/computer algorithm tool:

| Component: | Amount (ul) | Final concentration |
|---|---|---|
| Herculase II PCR buffer, 5X | 10 | 1X |
| dNTP, 100mM (25mM each) | 1 | 1 mM |
| SURVEYOR Fwd primer (10uM) | 1 | 0.2 uM |
| SURVEYOR Rev primer (10uM) | 1 | 0.2 uM |
| Herculase II Fusion polymerase | 1 | |
| $MgCl_2$ (25mM) | 2 | 1 mM |
| Distilled water | 33 | |

(continued)

| Component: | Amount (ul) | Final concentration |
|---|---|---|
| Total | 49 (for each reaction) | |

76| Applicants added 100-200 ng of normalized genomic DNA template from step 74 for each reaction.

77| PCR reaction was performed using the following cycling conditions, for no more than 30 amplification cycles:

| Cycle number | Denature | Anneal | Extend |
|---|---|---|---|
| 1 | 95°C, 2 min | | |
| 2-31 | 95°C, 20 s | 60°C, 20 s | 72°C, 30 s |
| 32 | | | 72°C, 3 min |

78| Applicants ran 2-5 ul of PCR product on a 1% gel to check for single-band product Although these PCR conditions are designed to work with most pairs of SURVEYOR primers, some primers may need additional optimization by adjusting the template concentration, MgCl$_2$ concentration, and/or the annealing temperature.

79| Applicants purified the PCR reactions using the QIAQuick PCR purification kit and normalized eluant to 20 ng/ul.

**Pause point:** Purified PCR product may be stored at -20°C.

80| *DNA heteroduplex formation.* The annealing reaction was set up as follows:

| | |
|---|---|
| Taq PCR buffer, 10X | 2 ul |
| Normalized DNA (20 ng/ul) | 18 ul |
| Total volume | 20 ul |

81| Anneal the reaction using the following conditions:

| Cycle number | Condition |
|---|---|
| 1 | 95°C, 10 mn |
| 2 | 95°C-85°C, -2°C/s |
| 3 | 85°C, 1 min |
| 4 | 85°C-75°C, -0.3°C/s |
| 5 | 75°C, 1 min |
| 6 | 75°C-65°C, -0.3°C/s |
| 7 | 65°C, 1 min |
| 8 | 65°C-55°C, -0.3°C/s |
| 9 | 55°C, 1 min |
| 10 | 55°C-45°C, -0.3°C/s |
| 11 | 45°C, 1 min |
| 12 | 45°C-35°C, -0.3°C/s |
| 13 | 35°C, 1 min |
| 14 | 35°C-25°C, -0.3°C/s |
| 15 | 25°C, 1 min |

82| *SURVEYOR nuclease S digestion.* Applicants prepared master-mix and added the following components on ice to annealed heteroduplexes from step 81 for a total final volume of 25 ul:

| Component | Amount (ul) | Final Concentration |
|---|---|---|
| MgCl$_2$ solution, 0.15M | 2.5 | 15mM |
| ddH$_2$O | 0.5 | |
| SURVEYOR nuclease S | 1 | 1X |
| SURVEYOR enhancer S | 1 | 1X |

(continued)

| Component | Amount (ul) | Final Concentration |
|---|---|---|
| Total | 5 | |

83| Vortex well and spin down. Incubate the reaction at 42°C for 1 h.

84| Optional: 2 ul of the Stop Solution from the SURVEYOR kit may be added. **Pause point.** The digested product may be stored at -20°C for analysis at a later time.

85| *Visualize the SURVEYOR reaction.* SURVEYOR nuclease digestion products may be visualized on a 2% agarose gel. For better resolution, products may be run on a 4-20% gradient Polyacrylamide TBE gel. Applicants loaded 10 ul of product with the recommended loading buffer and ran the gel according to manufacturer's instructions. Typically, Applicants run until the bromophenol blue dye has migrated to the bottom of the gel. Include DNA ladder and negative controls on the same gel.

86| Applicants stained the gel with 1X SYBR Gold dye diluted in TBE. The gel was gently rocked for 15 m.

87| Applicants imaged the gel using a quantitative imaging system without overexposing the bands. The negative controls should have only one band corresponding to the size of the PCR product, but may have occasionally non-specific cleavage bands of other sizes. These will not interfere with analysis if they are different in size from target cleavage bands. The sum of target cleavage band sizes, provided by Applicants online/computer algorithm tool, should be equal to the size of the PCR product

88| *Estimate the cleavage intensity.* Applicants quantified the integrated intensity of each band using ImageJ or other gel quantification software.

89| For each lane, Applicants calculated the fraction of the PCR product cleaved ($f_{cut}$) using the following formula: $f_{cut}$ = *(b + c) / (a + b + c)*, where a is the integrated intensity of the undigested PCR product and b and c are the integrated intensities of each cleavage product. 90| Cleavage efficiency may be estimated using the following formula, based on the binomial probability distribution of duplex formation:

$$91| \; indel\,(\%) = 100 \times (1 - \sqrt{(1 - f_{cut})})$$

**Sanger sequencing for assessing CRISPR cleavage efficiency. Timing • 3 d**

Initial steps are identical to Steps 71-79 of the SURVEYOR assay. Note: SURVEYOR primers may be used for Sanger sequencing if appropriate restriction sites are appended to the Forward and Reverse primers. For cloning into the recommended pUC19 backbone, EcoRI may be used for the Fwd primer and HindIII for the Rev primer.

92| *Amplicon digestion.* Set up the digestion reaction as follows:

| Component | Amount (ul) |
|---|---|
| Fast Digest buffer, 10X | 3 |
| FastDigest EcoRI | 1 |
| FastDigest HindIII | 1 |
| Normalized DNA (20 ng/ul) | 10 |
| ddH$_2$O | 15 |
| Total volume | 30 |

93| pUC19 backbone digestion. Set up the digestion reaction as follows:

| Component | Amount (ul) |
|---|---|
| Fast Digest buffer, 10X | 3 |
| FastDigest EcoRI | 1 |
| FastDigest HindIII | 1 |
| FastAP Alkaline Phosphatase | 1 |
| pUC19 vector (200 ng/ul) | 5 |
| ddH$_2$O | 20 |
| Total volume | 30 |

94| Applicants purified the digestion reactions using the QIAQuick PCR purification kit **Pause point:** Purified PCR product may be stored at -20°C.

95| Applicants ligated the digested pUC19 backbone and Sanger amplicons at a 1:3 vector:insert ratio as follows:

| Component | Amount (ul) |
|---|---|
| Digested pUC 19 | x (50 ng) |
| Digested insert | x (1:3 vector:insert molar ratio) |
| T7 ligase | 1 |
| 2X Rapid Ligation Buffer | 10 |
| ddH$_2$O | x |
| Total volume | 20 |

96| *Transformation.* Applicants transformed the PlasmidSafe-treated plasmid into a competent *E. coli* strain, according to the protocol supplied with the cells. Applicants recommend Stbl3 for quick transformation. Briefly, 5ul of the product from step 95 is added into 20ul of ice-cold chemically competent Stbl3 cells, incubated on ice for 10 m, heat shocked at 42°C for 30 s, returned immediately to ice for 2 m, 100 ul of SOC medium is added, and plated onto an LB plate containing 100 ug/ml ampicillin. This is incubated overnight at 37°C.

97| Day 2: Applicants inspected plates for colony growth. Typically, there are no colonies on the negative control plates (ligation of EcoRI-HindIII digested pUC19 only, no Sanger amplicon insert), and tens to hundreds of colonies on the pUC19-Sanger amplicon cloning plates.

98| Day 3: Applicants isolated plasmid DNA from overnight cultures using a QIAprep Spin miniprep kit according to the manufacturer's instructions.

99| *Sanger sequencing.* Applicants verified the sequence of each colony by sequencing from the pUC19 backbone using the pUC19-For primer. Applicants referenced the sequencing results against the expected genomic DNA sequence to check for the presence of Cas9-induced NHEJ mutations. % editing efficiency = (# modified clones)/(# total clones). It is important to pick a reasonable number of clones (>24) to generate accurate modification efficiencies.

**Genotyping for microdeletion. Timing • 2-3 d hands on; 2-3 weeks expansion**

100| Cells were transfected as described above with a pair of sgRNAs targeting the region to be deleted.

101| 24 h post-transfection, clonal lines are isolated by FACS or serial dilution as described above.

102| Cells are expanded for 2-3 weeks.

103| Applicants harvested DNA from clonal lines as described above using 10 ul QuickExtract solution and normalized genomic DNA with ddH$_2$O to a final concentration of 50-100 ng/ul.

104| *PCR Amplify the modified region.* The PCR reaction is set up as follows:

| Component: | Amount (ul) | Final concentration |
|---|---|---|
| Herculase II PCR buffer, 5X | 10 | 1X |
| dNTP, 100mM (25mM each) | 1 | 1 mM |
| Out Fwd primer (10uM) | 1 | 0.2 uM |
| Out Rev primer (10uM) | 1 | 0.2 uM |
| Herculase II Fusion polymerase | 1 | |
| MgCl2 (25mM) | 2 | 1 mM |
| ddH$_2$O | 32 | |
| Total | 48 (for each reaction) | |

Note: if deletion size is more than 1 kb, set up a parallel set of PCR reactions with In-Fwd and In-Rev primers to screen for the presence of the wt allele.

105| To screen for inversions, a PCR reaction is set up as follows:

| Component: | Amount (ul) | Final concentration |
|---|---|---|
| Herculase II PCR buffer, 5X | 10 | 1X |
| dNTP, 100mM (25mM each) | 1 | 1 mM |

(continued)

| Component: | Amount (ul) | Final concentration |
|---|---|---|
| Out Fwd *or* Out-Rev primer (10uM) | 1 | 0.2 uM |
| In Fwd *or* In-Rev primer (10uM) | 1 | 0.2 uM |
| Herculase II Fusion polymerase | 1 | |
| MgCl$_2$ (25mM) | 2 | 1 mM |
| ddH$_2$O | 32 | |
| Total | 48 (for each reaction) | |

Note: primers are paired either as Out-Fwd + In Fwd, or Out-Rev + In-Rev.

106| Applicants added 100-200 ng of normalized genomic DNA template from step 103 for each reaction.

107| PCR reaction was performed using the following cycling conditions:

| Cycle number | Denature | Anneal | Extend |
|---|---|---|---|
| 1 | 95°C, 2 min | | |
| 2-31 | 95°C, 20 s | 60°C, 20 s | 72°C, 30 s |
| 32 | | | 72°C, 3 m |

108| Applicants run 2-5 ul of PCR product on a 1-2% gel to check for product. Although these PCR conditions are designed to work with most primers, some primers may need additional optimization by adjusting the template concentration, MgCl$_2$ concentration, and/or the annealing temperature.

**Genotyping for targeted modifications via HDR. Timing • 2-3 d, 2-3 h hands on**

109| Applicants harvested DNA as described above using QuickExtract solution and normalized genomic DNA with TE to a final concentration of 100-200 ng/ul.

110| *PCR Amplify the modified region.* The PCR reaction is set up as follows:

| Component: | Amount (ul) | Final concentration |
|---|---|---|
| Herculase II PCR buffer, 5X | 10 | 1X |
| dNTP, 100 mM (25 mM each) | 1 | 1 mM |
| HDR Fwd primer (10 uM) | 1 | 0.2 uM |
| HDR Rev primer (10 uM) | 1 | 0.2 uM |
| Herculase II Fusion polymerase | 1 | |
| MgCl$_2$ (25mM) | 2 | 1 mM |
| ddH$_2$O | 33 | |
| Total | 49 (for each reaction) | |

111| Applicants added 100-200 ng of genomic DNA template from step 109 for each reaction and run the following program.

| Cycle number | Denature | Anneal | Extend |
|---|---|---|---|
| 1 | 95°C, 2 min | | |
| 2-31 | 95°C, 20 s | 60°C, 20 s | 72°C, *30-60 s per kb* |
| 32 | | | 72°C, 3 min |

112| Applicants ran 5 ul of PCR product on a 0.8-1% gel to check for single-band product. Primers may need additional optimization by adjusting the template concentration, MgCl$_2$ concentration, and/or the annealing temperature.

113| Applicants purified the PCR reactions using the QIAQuick PCR purification kit

114| In the HDR example, a *Hind*III restriction site is inserted into the EMX1 gene. These are detected by a restriction digest of the PCR amplicon:

| Component | Amount (ul) |
|---|---|
| Purified PCR amplicon (200-300ng) | x |
| F.D. buffer, Green | 1 |
| HindIII | 0.5 |
| ddH2O | x |
| Total | 10 |

i. The DNA is digested for 10 m at 37°C:

ii. Applicants ran 10 ul of the digested product with loading dye on a 4-20% gradient polyacrylamide TBE gel until the xylene cyanol band had migrated to the bottom of the gel.

iii. Applicants stained the gel with 1X SYBR Gold dye while rocking for 15 m.

iv. The cleavage products are imaged and quantified as described above in the SURVEYOR assay section. HDR efficiency is estimated by the formula: (b + c)/(a + b + c), where a is the integrated intensity for the undigested HDR PCR product, and b and c are the integrated intensities for the HindIII-cut fragments.

115| Alternatively, purified PCR amplicons from step 113 may be cloned and genotyped using Sanger sequencing or NGS.

**Deep sequencing and off-target analysis • Timing 1 - 2 d**

The online CRISPR target design tool generates candidate genomic off-target sites for each identified target site. Off-target analysis at these sites can be performed by SURVEYOR nuclease assay, Sanger sequencing, or next-generation deep sequencing. Given the likelihood of low or undetectable modification rates at many of these sites, Applicants recommend deep sequencing with the Illumina Miseq platform for high sensitivity and accuracy. Protocols will vary with sequencing platform; here, Applicants briefly describe a fusion PCR method for attaching sequencing adapters.

116| *Design deep sequencing primers.* Next-generation sequencing (NGS) primers are designed for shorter amplicons, typically in the 100-200 bp size range. Primers may be manually designed using NCBI Primer-Blast or generated with online CRISPR target design tools (website at genome-engineering.org/tools).

117| Harvest genomic DNA from Cas9-targeted cells. Normalize QuickExtract genomic DNA to 100-200 ng/ul with ddH2O.

118| *Initial library preparation PCR.* Using the NGS primers from step 116, prepare the initial library preparation PCR

| Component: | Amount (ul) | Final concentration |
|---|---|---|
| Herculase II PCR buffer, 5X | 10 | 1X |
| dNTP, 100mM (25mM each) | 1 | 1 mM |
| NGS Fwd primer (10uM) | 1 | 0.2 uM |
| NGS Rev primer (10uM) | 1 | 0.2 uM |
| Herculase II Fusion polymerase | 1 | |
| MgCl2 (25mM) | 2 | 1 mM |
| ddH2O | 33 | |
| Total | 49 (for each reaction) | |

119| Add 100-200 ng of normalized genomic DNA template for each reaction.

120| Perform PCR reaction using the following cycling conditions, for no more than 20 amplification cycles:

| Cycle number | Denature | Anneal | Extend |
|---|---|---|---|
| 1 | 95°C, 2 min | | |
| 2-21 | 95°C, 20 s | 60°C, 20 s | 72°C, 15 s |
| 22 | | | 72°C, 3 min |

121| Run 2-5 ul of PCR product on a 1% gel to check for single-band product As with all genomic DNA PCRs, NGS primers may require additional optimization by adjusting the template concentration, $MgCl_2$ concentration, and/or the annealing temperature.

122| Purify the PCR reactions using the QIAQuick PCR purification kit and normalize eluant to 20 ng/ul. Pause point: Purified PCR product may be stored at -20°C.

123| *Nextera XT DNA Sample Preparation Kit.* Following the manufacturer's protocol, generate Miseq sequencing-ready libraries with unique barcodes for each sample.

124| *Analyze sequencing data.* Off-target analysis may be performed through read alignment programs such as ClustalW, Geneious, or simple sequence analysis scripts.

**Timing**

Steps 1 - 2 Design and synthesis of sgRNA oligos and ssODNs: 1-5 d, variable depending on supplier
Steps 3-5 Construction of CRISPR plasmid or PCR expression cassette: 2 h to 3d
Steps 6-53 Transfection into cell lines: 3 d (1 h hands-on time)
Steps 54 - 70 Optional derivation of clonal lines: 1-3 weeks, variable depending on cell type
Steps 71 - 91 Functional validation of NHEJ via SURVEYOR: 5-6 h
Steps 92 - 124 Genotyping via Sanger or next-gen deep sequencing: 2-3 d (3-4 h hands on time)

**Addressing Situations Concerning Herein Examples**

| Situation | Solution |
|---|---|
| No amplification of sgRNA | Titrate U6-template concentration |
| SURVEYOR or HDR PCR dirty or no amplification | Titrate MgCl2; normalize and titrate template concentration; annealing temp gradient; redesign primers |
| Unequal amplification of alleles in microdeletion PCRs | Set up separate PCRs to detect wildtype and deletion alleles; Redesign primers with similar sized amplicons |
| Colonies on negative control plate | Increase *BbsI;* increase Golden Gate reaction cycle number, cut PX330 separately with Antarctic Phosphate treatment |
| No sgRNA sequences or wrong sequences | Screen additional colonies |
| Low lipofectamine transfection efficiency | Check cell health and density; titrate DNA; add GFP transfection control |
| Low nucleofection transfection efficiency | Check cell health and density; titrate DNA; suspend to single cell |
| Clumps or no cells after FACS | Filter cells before FACS; dissociate to single cells; resuspend in appropriate density |
| Clumps or no cells in serial dilution | Recount cells; dissociate to single cells and filter through strainer; check serial dilution |
| High SURVEYOR background on negative sample | Redesign primers to prime from different locations |
| Dirty SURVEYOR result on gel | Purify PCR product; reduce input DNA; reduce 42°C incubation to 30 m |
| No SURVEYOR cleavage | Purify and normalize PCR product; re-anneal with TaqB buffer; Redesign sgRNAs; sequence verify Cas9 on px330 backbone |
| Samples do not sink in TBE acrylamide gel | Supplement with MgCl2 to a final concentration of 15mM or add loading buffer containing glycerol |

**Discussion**

CRISPR-Cas may be easily multiplexed to facilitate simultaneous modification of several genes and mediate chromosomal microdeletions at high efficiencies. Applicants used two sgRNAs to demonstrate simultaneous targeting of the human *GRIN2B* and *DYRK1A* loci at efficiencies of up to 68% in HEK293FT cells. Likewise, a pair of sgRNAs may be used to mediate microdeletions, such as excision of an exon, which can be genotyped by PCR on a clonal level. Note that the precise location of exon junctions can vary. Applicants also demonstrated the use of ssODNs and targeting vector to mediate HDR with both wildtype and nickase mutant of Cas9 in HEK 293FT and HUES9 cells (Fig. 30). Note that Applicants have not been able to detect HDR in HUES9 cells using the Cas9 nickase, which may be due to low efficiency or a potential difference in repair activities in HUES9 cells. Although these values are typical, there is some variability in the cleavage efficiency of a given sgRNA, and on rare occasions certain sgRNAs may not work for reasons yet unknown. Applicants recommend designing two sgRNAs for each locus, and testing their efficiencies in the intended cell type.

*Example 31: NLSs*

Cas9 Transcriptional Modulator: Applicants set out to turn the Cas9/gRNA CRISPR system into a generalized DNA binding system in which functions beyond DNA cleavage can be executed. For instance, by fusing functional domain(s) onto a catalytically inactive Cas9 Applicants have imparted novel functions, such as transcriptional activation/repression, methylation/demethylation, or chromatin modifications. To accomplish this goal Applicants made a catalytically inactive Cas9 mutant by changing two residues essential for nuclease activity, D10 and H840, to alanine. By mutating these two residues the nuclease activity of Cas9 is abolished while maintaining the ability to bind target DNA. The functional domains Applicants decided to focus on to test Applicants' hypothesis are the transcriptional activator VP64 and the transcriptional repressors SID and KRAB.

Cas9 Nuclear localization: Applicants hypothesized that the most effective Cas9 transcriptional modulator would be strongly localized to the nucleus where it would have its greatest influence on transcription. Moreover, any residual Cas9 in the cytoplasm could have unwanted effects. Applicants determined that wild-type Cas9 does not localize into the nucleus without including multiple nuclear localization signals (NLSs) (although a CRISPR system need not have one or more NLSs but advantageously has at least one or more NLS(s)). Because multiple NLS sequences were required it was reasoned that it is difficult to get Cas9 into the nucleus and any additional domain that is fused to Cas9 could disrupt the nuclear localization. Therefore, Applicants made four Cas9-VP64-GFP fusion constructs with different NLS sequences (pXRP02- pLenti2-EFIa-NLS-hSpCsn1(10A,840A)-NLS-VP64-EGFP, pXRP04- pLenti2-EF1a-NLS-hSpCsn1(10A,840A)-NLS-VP64-2A-EGFP-NLS, pXRP06- pLenti2-EF1a-NLS-EGFP-VP64-NLS-hSpCsnl(10A,840A)-NLS, pXRP08- pLenti2-EFIa-NLS-VP64-NLS-hSpCsn1(10A,840A)-NLS-VP64-EGFP-NLS). These constructs were cloned into a lenti backbone under the expression of the human EF1a promoter. The WPRE element was also added for more robust protein expression. Each construct was transfected into HEK 293FT cells using Lipofectame 2000 and imaged 24 hours post-transfection. The best nuclear localization is obtained when the fusion proteins have NLS sequences on both the N- and C-term of the fusion protein. The highest observed nuclear localization occurred in the construct with four NLS elements.

To more robustly understand the influence of NLS elements on Cas9 Applicants made 16 Cas9-GFP fusions by adding the same alpha importin NLS sequence on either the N- or C-term looking at zero to three tandem repeats. Each construct was transfected into HEK 293FT cells using Lipofectame 2000 and imaged 24 hours post-transfection. Notably, the number of NLS elements does not directly correlate with the extent of nuclear localization. Adding an NLS on the C-term has a greater influence on nuclear localization than adding on the N-term.

Cas9 Transcriptional Activator: Applicants functionally tested the Cas9-VP64 protein by targeting the Sox2 locus and quantifying transcriptional activation by RT-qPCR. Eight DNA target sites were chosen to span the promoter of Sox2. Each construct was transfected into HEK 293FT cells using Lipofectame 2000 and 72 hours post-transfection total RNA was extracted from the cells. 1 ug of RNA was reverse transcribed into cDNA (qScript Supermix) in a 40 ul reaction. 2 ul of reaction product was added into a single 20 ul TaqMan assay qPCR reaction. Each experiment was performed in biological and technical triplicates. No RT control and no template control reactions showed no amplification. Constructs that do not show strong nuclear localization, pXRP02 and pXRP04, result in no activation. For the construct that did show strong nuclear localization, pXRP08, moderate activation was observed. Statistically significant activation was observed in the case of guide RNAs Sox2.4 and Sox2.5.

*Example 32: In Vivo Mouse Data*

**Material and reagents**

Herculase II fusion polymerase (Agilent Technologies, cat. no. 600679)
10x NEBuffer 4 (NEB, cat. No. B7004S)
BsaIHF (NEB, cat No. R3535S)
T7 DNA ligase (Enzymatics, cat no. L602L)
Fast Digest buffer, 10X (ThermoScientific, cat. No. B64)

FastDigest NotI (ThermoScientific, cat. No. FD0594)
FastAP Alkaline Phosphatase (ThermoScientific, cat. No. EF0651)
Lipofectamine2000 (Life Technologies, cat. No. 11668-019)
Trypsin (Life Technologies, cat. No. 15400054)
Forceps #4 (Sigma, cat. No. Z168777-1EA)
Forceps #5 (Sigma, cat No. F6521-1EA)
10x Hank's Balanced Salt Solution (Sigma, cat. No. H4641-500ML)
Penicillin/Streptomycin solution (Life Technologies, cat. No. P4333)
Neurobasal (Life Technologies, cat. No. 21103049)
B27 Supplement (Life Technologies, cat. No. 17504044)
L-glutamine (Life Technologies, cat. No. 25030081)
Glutamate (Sigma, cat. No. RES5063G-A7)
β-mercaptoethanol (Sigma, cat. No. M6250-100ML)
HA rabbit antibody (Cell Signaling, cat No. 3724S)
LIVE/DEAD® Cell Imaging Kit (Life Technologies, cat. No. R37601)
30G World Precision Instrument syringe (World Precision Instruments, cat. No. NANOFIL)
Stereotaxic apparatus (Kopf Instruments)
UltraMicroPump3 (World Precision Instruments, cat. No. UMP3-4)
Sucrose (Sigma, cat. No. S7903)
Calcium chloride (Sigma, cat. No. C1016)
Magnesium acetate (Sigma, cat. No. M0631)
Tris-HCl (Sigma, cat. no T5941)
EDTA (Sigma, cat No. E6758)
NP-40 (Sigma, cat. No. NP40)
Phenylmethanesulfonyl fluoride (Sigma, cat. No. 78830)
Magnesium chloride (Sigma, cat. No. M8266)
Potassium chloride (Sigma, cat. No. P9333)
β-glycerophosphate (Sigma, cat. No. G9422)
Glycerol (Sigma, cat. No. G9012)
Vybrant® DyeCycle™ Ruby Stain (Life technologies, cat. No. S4942)
FACS Aria Flu-act-cell sorter (Koch Institute of MIT, Cambridge US)
DNAeasy Blood & Tissue Kit (Qiagen, cat. No. 69504)

**Procedure**

***Constructing gRNA multiplexes for using in vivo in the brain***

Applicants designed and PCR amplified single gRNAs targeting mouse TET and DNMT family members (as described herein) Targeting efficiency was assessed in N2a cell line (Fig. 33). To obtain simultaneous modification of several genes in vivo, efficient gRNA was multiplexed in AAV-packaging vector (Fig. 34). To facilitate further analysis of system efficiency applicants added to the system expression cassette consistent of GFP-KASH domain fusion protein under control of human Synapsin I promoter (Fig. 34). This modification allows for further analysis of system efficiency in neuronal population (more detail procedure in section ***Sorting nuclei and in vivo results***).

All 4 parts of the system were PCR amplified using Herculase II Fusion polymerase using following primers:

1 st U6 Fw:
gagggtctcgtccttgcggccgcgctagcgagggcctatttcccatgattc

```
1st gRNA Rv:

ctcggtctcggtAAAAAgcaccgactcggtgccactttttcaagttgataacggactag

c

cttattttaacttgctaTTTCtagctctaaaacNNNNNNNNNNNNNNNNNNNNNNGGTGTTT

C GTCCTTTCCAC
```

2nd U6 Fw:
gagggtctcTTTaccggtgagggcctatttcccatgattcc

2nd gRNA Rv:

ctcggtctcctcAAAAAgcaccgactcggtgccacttttttcaagttgataacgg actagc

cttattttaacttgctaTTTCtagctctaaaacNNNNNNNNNNNNNNNNNNNNNGGTGTTT C GTCCTTTCCAC

3rd U6 Fw:
gagggtctcTTTgagctcgagggcctatttcccatgattc

3rd gRNA Rv:

ctcggtctcgcgtAAAAAgcaccgactcggtgccacttttttcaagttgataacg gactag

ccttattttaacttgctaTTTCtagctctaaaacNNNNNNNNNNNNNNNNNNNNNGGTGTT T CGTCCTTTCCA

hSyn_GFP-kash Fw: gagggtctcTTacgcgtgtgtctagac

hSyn_GFP-kash          Rv:

ctcggtctcAaggaCAGGGAAGGGAGCAGTGGTTCACGCCTGTAATCCCAGCAAT TTGGGA GGCCAAGGTGGGTAGATCACCTGAGATTAGGAGTTGC

(NNNNNNNNNNNNNNNNNNNNN is a reverse compliment targeted genomic sequence)

Applicants used Golden Gate strategy to assemble all parts (1:1 molecular ratio) of the system in a single step reaction:

| | |
|---|---|
| 1st U6_gRNA | 18 ng |
| 2nd U6_gRNA | 18 ng |
| 3rd U6_gRNA | 18 ng |
| Syn_GFP-kash | 100 ng |
| 10x NEBuffer 4 | 1.0 $\mu$l |
| 10x BSA | 1.0 $\mu$l |
| 10 mM ATP | 1.0 $\mu$l |
| BsaI HF | 0.75 $\mu$l |
| T7 ligase | 0.25 $\mu$l |
| ddH$_2$O | 10 $\mu$l |

| Cycle number | Condition |
|---|---|
| 1-50 | 37°C for 5 m, 21°C for 5 m |

Golden Gate reaction product was PCR amplified using Herculase II fusion polymerase and following primers:

Fw 5'cctgtccttgcggccgcgctagcgagggcc

Rv 5'cacgcggccgcaaggacagggaagggagcag

PCR product was cloned into AAV backbone, between ITR sequences using NotI restriction sites:

PCR product digestion:

| | |
|---|---|
| Fast Digest buffer, 10X | 3 μl |
| FastDigest NotI | 1 μl |
| DNA | 1 μg |
| ddH$_2$O | up to 30 μl |

AAV backbone digestion:

| | |
|---|---|
| Fast Digest buffer, 10X | 3 μl |
| FastDigest NotI | 1 μl |
| FastAP Alkaline Phosphatase | 1 μl |
| AAV backbone | 1 μg |
| ddH$_2$O | up to 30 μl |

After 20 min incubation in 37°C samples were purified using QIAQuick PCR purification kit. Standardized samples were ligated at a 1:3 vector:insert ratio as follows:

| | |
|---|---|
| Digested pUC19 | 50 ng |
| Digested insert | 1:3 vector:insert molar ratio |
| T7 ligase | 1 μl |
| 2X Rapid Ligation Buffer | 5 μl |
| ddH$_2$O | up to 10 μl |

After transformation of bacteria with ligation reaction product, applicants confirmed obtained clones with Sanger sequencing.

Positive DNA clones were tested in N2a cells after co-transfection with Cas9 construct (Figs. 35 and 36).

### Design of new Cas9 constructs for AAV delivery

AAV delivery system despite its unique features has packing limitation - to successfully deliver expressing cassette in vivo it has to be in size < then 4.7 kb. To decrease the size of SpCas9 expressing cassette and facilitate delivery applicants tested several alteration: different promoters, shorter polyA signal and finally a smaller version of Cas9 from *Staphylococcus aureus* (SaCas9) (Figs. 37 and 38). All tested promoters were previously tested and published to be active in neurons, including mouse *Mecp2* (Gray et al., 2011), rat *Map1b* and truncated rat *Map1b* (Liu and Fischer, 1996). Alternative synthetic polyA sequence was previously shown to be functional as well (Levitt et al., 1989; Gray et al., 2011). All cloned constructs were expressed in N2a cells after transfection with Lipofectamine 2000, and tested with Western blotting method (Fig. 39).

### Testing AAV multiplex system in primary neurons

To confirm functionality of developed system in neurons, Applicants use primary neuronal cultures *in vitro*. Mouse cortical neurons was prepared according to the protocol published previously by Banker and Goslin (Banker and Goslin, 1988).

Neuronal cells are obtained from embryonic day 16. Embryos are extracted from the euthanized pregnant female and decapitated, and the heads are placed in ice-cold HBSS. The brains are then extracted from the skulls with forceps (#4 and #5) and transferred to another change of ice-cold HBSS. Further steps are performed with the aid of a stereoscopic microscope in a Petri dish filled with ice-cold HBSS and #5 forceps. The hemispheres are separated from each other and the brainstem and cleared of meninges. The hippocampi are then very carefully dissected and placed in a 15 ml conical tube filled with ice-cold HBSS. Cortices that remain after hippocampal dissection can be used for further cell isolation using an analogous protocol after removing the brain steam residuals and olfactory bulbs. Isolated hippocampi are washed three times with 10 ml ice-cold HBSS and dissociated by 15 min incubation with trypsin in HBSS (4 ml HBSS with the addition of 10 μl 2.5% trypsin per hippocampus) at 37°C. After trypsinization, the hippocampi are very carefully washed three times to remove any traces of trypsin with HBSS preheated to 37°C and dissociated in warm HBSS. Applicants usually dissociate cells obtained from 10-12 embryos in 1 ml HBSS using 1 ml pipette tips and dilute dissociated cells up to 4 ml. Cells are plated at a density of 250 cells/mm2 and cultured at 37°C and 5% CO2 for up to 3 week

**HBSS**

435 ml H2O
50 ml 10x Hank's Balanced Salt Solution
16.5 ml 0.3M HEPES pH 7.3
5 ml penicillin-streptomycin solution
Filter (0.2 μm) and store 4°C

**Neuron Plating Medium** (100 ml)

97 ml Neurobasal
2 ml B27 Supplement
1 ml penicillin-streptomycin solution
250 μl glutamine
125 μl glutamate
Neurons are transduced with concentrated AAV1/2 virus or AAV1 virus from filtered medium of HEK293FT cells, between 4-7 days in culture and keep for at least one week in culture after transduction to allow for delivered gene expression.

*AAV-driven expression of the system*

Applicants confirmed expression of SpCas9 and SaCas9 in neuronal cultures after AAV delivery using Western blot method (Fig. 42). One week after transduction neurons were collected in NuPage SDS loading buffer with β-mercaptoethanol to denaturate proteins in 95°C for 5 min. Samples were separated on SDS PAGE gel and transferred on PVDF membrane for WB protein detection. Cas9 proteins were detected with HA antibody.
Expression of *Syn*-GFP-kash from gRNA multiplex AAV was confirmed with fluorescent microscopy (Fig. 50).

*Toxicity*

To assess the toxicity of AAV with CRISPR system Applicants tested overall morphology of neurons one week after virus transduction (Fig. 45). Additionally, Applicants tested potential toxicity of designed system with the LIVE/DEAD® Cell Imaging Kit, which allows to distinguish live and dead cells in culture. It is based on the presence of intracellular esterase activity (as determined by the enzymatic conversion of the non-fluorescent calcein AM to the intensely green fluorescent calcein). On the other hand, the red, cell-impermeant component of the Kit enters cells with damaged membranes only and bind to DNA generating fluorescence in dead cells. Both flourophores can be easily visualized in living cells with fluorescent microscopy. AAV-driven expression of Cas9 proteins and multiplex gRNA constructs in the primary cortical neurons was well tolerated and not toxic (Figs. 43 and 44), what indicates that designed AAV system is suitable for in vivo tests.

**Virus production**

Concentrated virus was produced according to the methods described in McClure et al., 2011. Supernatant virus production occurred in HEK293FT cells.

**Brain surgeries**

For viral vector injections 10-15 week old male C57BL/6N mice were anesthetized with a Ketamine/Xylazine cocktail (Ketamine dose of 100 mg/kg and Xylazine dose of 10 mg/kg) by intraperitoneal injection. Intraperitonial administration of Buprenex was used as a pre-emptive analgesic (1 mg/kg). Animals were immobilized in a Kopf stereotaxic apparatus using intra-aural positioning studs and tooth bar to maintain an immobile skull. Using a hand-held drill, a hole (1-2mm) at -3.0 mm posterior to Bregma and 3.5 mm lateral for injection in the CA1 region of the hippocampus was made. Using 30G World Precision Instrument syringe at a depth of 2.5 mm, the solution of AAV viral particles in a total volume of 1 ul was injected. The injection was monitored by a 'World Precision Instruments UltraMicroPump3' injection pump at a flow rate of 0.5 ul/min to prevent tissue damage. When the injection was complete, the injection needle was removed slowly, at a rate of 0.5 mm/min. After injection, the skin was sealed with 6-0 Ethilon sutures. Animals were postoperatively hydrated with 1 mL lactated Ringer's (subcutaneous) and housed in a temperature controlled (37°C) environment until achieving an ambulatory recovery. 3 weeks after surgery animals were euthanized by deep anesthesia followed by tissue removal for nuclei sorting or with 4% paraformaldehyde perfusion for immunochemistry.

*Sorting nuclei and in vivo results*

Applicants designed a method to specifically genetically tag the gRNA targeted neuronal cell nuclei with GFP for Fluorescent Activated Cell Sorting (FACS) of the labeled cell nuclei and downstream processing of DNA, RNA and nuclear proteins. To that purpose the applicants' multiplex targeting vector was designed to express both a fusion protein between GFP and the mouse nuclear membrane protein domain KASH (Starr DA, 2011, Current biology) and the 3 gRNAs to target specific gene loci of interest (Fig. 34). GFP-KASH was expressed under the control of the human Synapsin promoter to specifically label neurons. The amino acid of the fusion protein GFP-KASH was:

MVSKGEELFTGVVPILVELDGDVNGHKFSVSGEGEGDATYGKLTLKFICT

TGKLPVPWPTLVTTLTYGVQCFSRYPDHMKQHDFFKSAMPEGYVQERTIFFKDD

GN

YKTRAEVKFEGDTLVNRIELKGIDFKEDGNILGHKLEYNYNSHNVYIMADKQKN

GIK

VNFKIRHNIEDGSVQLADHYQQNTPIGDGPVLLPDNHYLSTQSALSKDPNEKRDH

MV

LLEFVTAAGITLGMDELYKSGLRSREEEEETDSRMPHLDSPGSSQPRRSFLSRVIRA

AL PLQLLLLLLLLLLACLLPASEDDYSCTQANNFARSFYPMLRYTNGPPPT

One week after AAV1/2 mediated delivery into the brain a robust expression of GFP-KASH was observed. For FACS and downstream processing of labeled nuclei, the hippocampi were dissected 3 weeks after surgery and processed for cell nuclei purification using a gradient centrifugation step. For that purpose the tissue was homogenized in 320 mM Sucrose, 5 mM CaCl, 3 mM Mg(Ac)2, 10 mM Tris pH 7.8, 0.1 mM EDTA, 0.1% NP40, 0.1 mM Phenylmethanesulfonyl fluoride (PMSF), 1 mM β-mercaptoethanol using 2ml Dounce homogenizer (Sigma) The homogenisate was centrifuged on a 25% to 29% Optiprep® gradient according to the manufacture's protocol for 30 min at 3.500 rpm at 4 °C. The nuclear pellet was resuspended in 340 mM Sucrose, 2 mM MgCl2, 25 mM KCl, 65 mM glycerophosphate, 5% glycerol, 0.1 mM PMSF, 1 mM β-mercaptoethanol and Vybrant® DyeCycle™ Ruby Stain (Life technologies) was added to label cell nuclei (offers near-infrared emission for DNA). The labeled and purified nuclei were sorted by FACS using an Aria Flu-act-cell sorter and BDFACS Diva software. The sorted GFP+ and GFP- nuclei were finally used to purify genomic DNA using DNAeasy Blood & Tissue Kit (Qiagen) for Surveyor assay analysis of the targeted genomic regions. The same approach can be easily used to purify nuclear RNA or protein from targeted cells for downstream processing. Due to the 2-vector system (Fig. 34) the applicants using in this approach efficient Cas9 mediated DNA cleavage was expected to occur only in a small subset of cells in the brain (cells which were co-infected with both the multiplex targeting vector and the Cas9 encoding vector). The method described here enables the applicants to specifically purify DNA, RNA and nuclear proteins from the cell population expressing the 3 gRNAs of interest and therefore are supposed to undergo Cas9 mediated DNA cleavage. By using this method the applicants were able to visualize efficient DNA cleavage *in vivo* occurring only in a small subset of cells.

Essentially, what Applicants have shown here is targeted *in vivo* cleavage. Furthermore, Applicants used a multiple approach, with several different sequences targeted at the same time, but independently. Presented system can be applied for studying brain pathologic conditions (gene knock out, e.g. Parkinson disease) and also open a field for further development of genome editing tools in the brain. By replacing nuclease activity with gene transcription regulators or epigenetic regulators it will be possible to answer whole spectrum of scientific question about role of gene regulation and epigenetic changes in the brain in not only in the pathologic conditions but also in physiological process as learning and memory formation. Finally, presented technology can be applied in more complex mammalian system as primates, what allows to overcome current technology limitations.

*Example 33: Model Data*

Several disease models have been specifically investigated. These include *de novo* autism risk genes CHD8, KATNAL2, and SCN2A; and the syndromic autism (Angelman Syndrome) gene UBE3A. These genes and resulting autism models are of course preferred, but show that the disclosure may be applied to any gene and therefore any model is possible.

Applicants have made these cells lines using Cas9 nuclease in human embryonic stem cells (hESCs). The lines were created by transient transfection of hESCs with Cbh-Cas9-2A-EGFP and pU6-sgRNA. Two sgRNAs are designed for each gene targeting most often the same exons in which patient nonsense (knock-out) mutations have been recently described from whole exome sequencing studies of autistic patients. The Cas9-2A-EGFP and pU6 plasmids were created specifically for this project.

*Example 34: AAV production system or protocol*

An AAV production system or protocol that was developed for, and works particularly well with, high through put screening uses is provided herein, but it has broader applicability in the present disclosure as well. Manipulating endogenous gene expression presents various challenges, as the rate of expression depends on many factors, including regulatory elements, mRNA processing, and transcript stability. To overcome this challenge, Applicants developed an adeno-associated virus (AAV)-based vector for the delivery. AAV has an ssDNA-based genome and is therefore less susceptible to recombination.

AAV1/2 (serotype AAV1/2, i.e., hybrid or mosaic AAV1 / AAV2 capsid AAV) heparin purified concentrated virus protocol

**Media:** D10 + HEPES

500ml bottle DMEM high glucose + Glutamax (GIBCO)
50ml Hyclone FBS (heat-inactivated) (Thermo Fischer)
5.5ml HEPES solution (1M, GIBCO)
Cells: low passage HEK293FT (passage <10 at time of virus production, thaw new cells of passage 2-4 for virus production, grow up for 3-5 passages)

**Transfection reagent: Polyethylenimine (PEI) "Max"**

Dissolve 50mg PEI "Max" in 50ml sterile Ultrapure H20
Adjust pH to 7.1
Filter with 0.22um fliptop filter
Seal tube and wrap with parafilm
Freeze aliquots at -20°C (for storage, can also be used immediately)

**Cell Culture**

Culture low passage HEK293FT in D10 + HEPES
Passage everyday between 1:2 and 1:2.5
Advantageously do not allow cells to reach more than 85% confluency

For T75

- Warm 10ml HBSS (-Mg2+, -Ca2+, GIBCO) + 1ml TrypLE Express (GIBCO) per flask to 37°C (Waterbath)
  Aspirate media fully
- Add 10ml warm HBSS gently (to wash out media completely)
- Add 1ml TrypLE per Flask
- Place flask in incubator (37°C) for 1min
- Rock flask to detach cells
- Add 9ml D10 + HEPES media (37°C)
- Pipette up and down 5 times to generate single cell suspension
- Split at 1:2 - 1:2.5 (12ml media for T75) ratio (if cells are growing more slowly, discard and thaw a new batch, they are not in optimal growth)
- transfer to T225 as soon as enough cells are present (for ease of handling large amounts of cells)

**AAV production (5*15cm dish scale per construct):**

Plate 10 million cells in 21.5 ml media into a 15cm dish

Incubate for 18-22 hours at 37°C

Transfection is ideal at 80% confluence

**Per plate**

Prewarm 22ml media (D10 + HEPES)

Prepare tube with DNA mixture (use endofree maxiprep DNA):

5.2 ug vector of interest plasmid

4.35 ug AAV 1 serotype plasmid

4.35 ug AAV 2 serotype plasmid

10.4 ug pDF6 plasmid (adenovirus helper genes) ☐ Vortex to mix

Add 434 uL DMEM (no serum!)

Add 130 ul PEI solution

Vortex 5-10 seconds

Add DNA/DMEM/PEI mixture to prewarmed media

Vortex briefly to mix

Replace media in 15cm dish with DNA/DMEM/PEI mixture

Return to 37°C incubator

Incubate 48h before harvesting (make sure medium isn't turning too acidic)

**Virus harvest:**

1. aspirate media carefully from 15cm dish dishes (advantageously do not dislodge cells)

2. Add 25 ml RT DPBS (Invitrogen) to each plate and gently remove cells with a cell scraper. Collect suspension in 50 ml tubes.

3. Pellet cells at 800x g for 10 minutes.

4. Discard supernatant

**pause point: freeze cell pellet at -80C if desired**

5. resuspend pellet in 150 mM NaCl, 20 mM Tris pH 8.0, use 10 ml per tissue culture plate.

6. Prepare a fresh solution of 10% sodium deoxycholate in dH2O. Add 1.25 ml of this per tissue culture plate for a final concentration of 0.5%. Add benzonase nuclease to a final concentration of 50 units per ml. Mix tube thoroughly.

7. Incubate at 37°C for 1 hour (Waterbath).

8. Remove cellular debris by centrifuging at 3000 x g for 15 mins. Transfer to fresh 50 ml tube and ensure all cell debris has been removed to prevent blocking of heparin columns.

**Heparin column purification of AAV1/2:**

1. **Set up** HiTrap heparin columns using a peristaltic pump so that solutions flow through the column at 1 ml per minute. It is important to ensure no air bubbles are introduced into the heparin column.

2. **Equilibrate** the column with 10 ml 150 mM NaCl, 20 mM Tris, pH 8.0 using the peristaltic pump.

3. **Binding of virus:** Apply 50 ml virus solution to column and allow to flow through.

4. **Wash step 1:** column with 20 ml 100 mM NaCl, 20 mM Tris, pH 8.0. (using the peristaltic pump)

5. **Wash step 2:** Using a 3 ml or 5 ml syringe continue to wash the column with 1 ml 200 mM NaCl, 20 mM Tris, pH 8.0, followed by 1 ml 300 mM NaCl, 20 mM Tris, pH 8.0. Discard the flow-through.

(prepare the syringes with different buffers during the 50min flow through of virus solution above)

6. **Elution** Using 5 ml syringes and gentle pressure (flow rate of < 1ml/min) elute the virus from the column by applying:

1.5 ml 400 mM NaCl, 20 mM Tris, pH 8.0

3.0 ml 450 mM NaCl, 20 mM Tris, pH 8.0
1.5 ml 500 mM NaCl, 20 mM Tris, pH 8.0
Collect these in a 15 ml centrifuge tube.

**Concentration of AAV1/2:**

**1. Concentration step 1:** Concentrate the eluted virus using Amicon ultra 15ml centrifugal filter units with a 100,000 molecular weight cutoff. Load column eluate into the concentrator and centrifuge at 2000x g for 2 minutes (at room temperature. Check concentrated volume - it should be approximately 500 μl. If necessary, centrifuge in 1min intervals until correct volume is reached.
2. **buffer exchange:** Add 1ml sterile DPBS to filter unit, centrifuge in 1min intervals until correct volume (500ul) is reached.
3. **Concentration step 2:** Add 500ul concentrate to an Amicon Ultra 0.5ml 100K filter unit. Centrifuge at 6000g for 2min. Check concentrated volume - it should be approximately 100 μl. If necessary, centrifuge in 1min intervals until correct volume is reached.
4. **Recovery:** Invert filter insert and insert into fresh collection tube. Centrifuge at 1000g for 2min.

Aliquot and freeze at -80°C
lul is typically required per injection site, small aliquots (e.g. 5ul) are therefore recommended (avoid freeze-thaw of virus).
determine DNaseI-resistant GC particle titer using qPCR (see separate protocol)

**Materials**

Amicon Ultra, 0.5ml, 100K; MILLIPORE; UFC510024
Amicon Ultra, 15ml, 100K; MILLIPORE; UFC910024
Benzonase nuclease; Sigma-Aldrich, E1014
HiTrap Heparin cartridge; Sigma-Aldrich; 54836
Sodium deoxycholate; Sigma-Aldrich; D5670

AAV1 supernatant production protocol

Media: D10 + HEPES
500ml bottle DMEM high glucose + Glutamax (Invitrogen)
50ml Hyclone FBS (heat-inactivated) (Thermo Fischer)
5.5ml HEPES solution (1M, GIBCO)
Cells: low passage HEK293FT (passage <10 at time of virus production)
Thaw new cells of passage 2-4 for virus production, grow up for 2-5 passages
Transfection reagent: Polyethylenimine (PEI) "Max"
Dissolve 50mg PEI "Max" in 50ml sterile Ultrapure H20
Adjust pH to 7.1
Filter with 0.22um fliptop filter
Seal tube and wrap with parafilm
Freeze aliquots at -20°C (for storage, can also be used immediately)
Cell Culture
Culture low passage HEK293FT in D10 + HEPES Passage everyday between 1:2 and 1:2.5
Advantageously do let cells reach more than 85% confluency
For T75

- Warm 10ml HBSS (-Mg2+, -Ca2+, GIBCO) + 1ml TrypLE Express (GIBCO) per flask to 37°C (Waterbath)
- Aspirate media fully
- Add 10ml warm HBSS gently (to wash out media completely)
- Add 1 ml TrypLE per Flask
- Place flask in incubator (37°C) for 1min
- Rock flask to detach cells
- Add 9ml D10 + HEPES media (37°C)
- Pipette up and down 5 times to generate single cell suspension

- Split at 1:2 - 1:2.5 (12ml media for T75) ratio (if cells are growing more slowly, discard and thaw a new batch, they are not in optimal growth)
- transfer to T225 as soon as enough cells are present (for ease of handling large amounts of cells)

AAV production (single 15cm dish scale)

Plate 10 million cells in 21.5 ml media into a 15cm dish

Incubate for 18-22 hours at 37°C

Transfection is ideal at 80% confluence per plate

Prewarm 22ml media (D10 + HEPES)

Prepare tube with DNA mixture (use endofree maxiprep DNA):

5.2 ug vector of interest plasmid

8.7 ug AAV 1 serotype plasmid

10.4 ug DF6 plasmid (adenovirus helper genes)

Vortex to mix

Add 434 uL DMEM (no serum!) Add 130 ul PEI solution

Vortex 5-10 seconds

Add DNA/DMEM/PEI mixture to prewarmed media

Vortex briefly to mix

Replace media in 15cm dish with DNA/DMEM/PEI mixture

Return to 37°C incubator

Incubate 48h before harvesting (advantageously monitor to ensure medium is not turning too acidic)

Virus harvest:

Remove supernatant from 15cm dish

Filter with 0.45 um filter (low protein binding) Aliquot and freeze at -80°C

Transduction (primary neuron cultures in 24-well format, 5DIV)

Replace complete neurobasal media in each well of neurons to be transduced with fresh neurobasal (usually 400ul out of 500ul per well is replaced)

Thaw AAV supernatant in 37°C waterbath

Let equilibrate in incubator for 30min

Add 250 ul AAV supernatant to each well

Incubate 24h at 37°C

Remove media/supernatant and replace with fresh complete neurobasal

Expression starts to be visible after 48h, saturates around 6-7 Days Post Infection

Constructs for pAAV plasmid with GOI should not exceed 4.8kb including both ITRS.

Example of a human codon optimized sequence (i.e. being optimized for expression in humans) sequence : SaCas9 is provided below:

ACCGGTGCCACCATGTACCCATACGATGTTCCAGATTACGC

TTCGCCGAAGAAAAGCGCAAGGTCGAAGCGTCCATGAAAAGGAACTACATT
CT

GGGGCTGGACATCGGGATTACAAGCGTGGGGTATGGGATTATTGACTATGAA
AC

AAGGGACGTGATCGACGCAGGCGTCAGACTGTTCAAGGAGGCCAACGTGGAA
AA

CAATGAGGGACGGAGAAGCAAGAGGGGAGCCAGGCGCCTGAAACGACGGAG
AA

GGCACAGAATCCAGAGGGTGAAGAACTGCTGTTCGATTACAACCTGCTGAC
CG

ACCATTCTGAGCTGAGTGGAATTAATCCTTATGAAGCCAGGGTGAAAGGCCTG
A

GTCAGAAGCTGTCAGAGGAAGAGTTTTCCGCAGCTCTGCTGCACCTGGCTAAG
C

GCCGAGGAGTGCATAACGTCAATGAGGTGGAAGAGGACACCGGCAACGAGCT
G

TCTACAAAGGAACAGATCTCACGCAATAGCAAAGCTCTGGAAGAGAAGTATG
TC

GCAGAGCTGCAGCTGGAACGGCTGAAGAAAGATGGCGAGGTGAGAGGGTCA
ATT

AATAGGTTCAAGACAAGCGACTACGTCAAAGAAGCCAAGCAGCTGCTGAAAG
TG

CAGAAGGCTTACCACCAGCTGGATCAGAGCTTCATCGATACTTATATCGACCT
GC

TGGAGACTCGGAGAACCTACTATGAGGGACCAGGAGAAGGGAGCCCCTTCGG
AT

GGAAAGACATCAAGGAATGGTACGAGATGCTGATGGGACATTGCACCTATTT

TCC
AGAAGAGCTGAGAAGCGTCAAGTACGCTTATAACGCAGATCTGTACAACGCC
CT
GAATGACCTGAACAACCTGGTCATCACCAGGGATGAAAACGAGAAACTGGAA
TA
CTATGAGAAGTTCCAGATCATCGAAACGTGTTTAAGCAGAAGAAAAAGCCT
AC
ACTGAAACAGATTGCTAAGGAGATCCTGGTCAACGAAGAGGACATCAAGGGC
TA
CCGGGTGACAAGCACTGGAAACCAGAGTTCACCAATCTGAAAGTGTATCAC
GA
TATTAAGGACATCACAGCACGGAAAGAAATCATTGAGAACGCCGAACTGCTG
GA
TCAGATTGCTAAGATCCTGACTATCTACCAGAGCTCCGAGGACATCCAGGAAG
AG
CTGACTAACCTGAACAGCGAGCTGACCCAGGAAGAGATCGAACAGATTAGTA
AT
CTGAAGGGGTACACCGGAACACACAACCTGTCCCTGAAAGCTATCAATCTGAT
TC
TGGATGAGCTGTGGCATACAAACGACAATCAGATTGCAATCTTTAACCGGCTG
AA
GCTGGTCCCAAAAAAGGTGGACCTGAGTCAGCAGAAAGAGATCCCAACCACA
CT
GGTGGACGATTTCATTCTGTCACCCGTGGTCAAGCGGAGCTTCATCCAGAGCA
TCAAAGTGATCAACGCCATCATCAAGAAGTACGGCCTGCCCAATGATATCATT
ATCGAGCTGGCTAGGGAGAAGAACAGCAAGGACGCACAGAAGATGATCAAT
GAGATGCAGAAACGAAACCGGCAGACCAATGAACGCATTGAAGAGATTATCC
GAACTACCGGGAAAGAGAACGCAAAGTACCTGATTGAAAAAATCAAGCTGCA
CGATATGCAGGAGGGAAAGTGTCTGTATTCTCTGGAGGCCATCCCCCTGGAGG
ACCTGCTGAACAATCCATTCAACTACGAGGTCGATCATATTATCCCCAGAAGC
GTGTCCTTCGACAATTCCTTTAACAACAAGGTGCTGGTCAAGCAGGAAGAGAA
CTCTAAAAAGGGCAATAGGACTCCTTTCCAGTACCTGTCTAGTTCAGATTCCA
AGATCTCTTACGAAACCTTTAAAAAGCACATTCTGAATCTGGCCAAAGGAAAG
GGCCGCATCAGCAAGACCAAAAAGGAGTACCTGCTGGAAGAGCGGGACATCA

ACAGATTCTCCGTCCAGAAGGATTTTATTAACCGGAATCTGGTGGACACAAGA
TACGCTACTCGCGGCCTGATGAATCTGCTGCGATCCTATTTCCGGGTGAACAA
TCTGGATGTGAAAGTCAAGTCCATCAACGGCGGGTTCACATCTTTTCTGAGGC
GCAAATGGAAGTTTAAAAAGGAGCGCAACAAAGGGTACAAGCACCATGCCGA
AGATGCTCTGATTATCGCAAATGCCGACTTCATCTTTAAGGAGTGGAAAAAGC
TGGACAAAGCCAAGAAAGTGATGGAGAACCAGATGTTCGAAGAGAAGCAGG
CCGAATCTATGCCCGAAATCGAGACAGAACAGGAGTACAAGGAGATTTTCAT
CACTCCTCACCAGATCAAGCATATCAAGGATTTCAAGGACTACAAGTACTCTC
ACCGGGTGGATAAAAAGCCCAACAGAGAGCTGATCAATGACACCCTGTATAG
TACAAGAAAGACGATAAGGGGAATACCCTGATTGTGAACAATCTGAACGGA
CTGTACGACAAAGATAATGACAAGCTGAAAAAGCTGATCAACAAAGTCCCG
AGAAGCTGCTGATGTACCACCATGATCCTCAGACATATCAGAAACTGAAGCTG
ATTATGGAGCAGTACGGCGACGAGAAGAACCCACTGTATAAGTACTATGAAG
AGACTGGGAACTACCTGACCAAGTATAGCAAAAAGGATAATGGCCCCGTGAT
CAAGAAGATCAAGTACTATGGGAACAAGCTGAATGCCCATCTGGACATCACA
GACGATTACCCTAACAGTCGCAACAAGGTGGTCAAGCTGTCACTGAAGCCAT
ACAGATTCGATGTCTATCTGGACAACGGCGTGTATAAATTTGTGACTGTCAAG
AATCTGGATGTCATCAAAAAGGAGAACTACTATGAAGTGAATAGCAAGTGCT
ACGAAGAGGCTAAAAAGCTGAAAAAGATTAGCAACCAGGCAGAGTTCATCGC
CTCCTTTTACAACAACGACCTGATTAAGATCAATGGCGAACTGTATAGGGTCA
TCGGGGTGAACAATGATCTGCTGAACCGCATTGAAGTGAATATGATTGACATC
ACTTACCGAGAGTATCTGGAAAACATGAATGATAAGCGCCCCCCTCGAATTAT
CAAAACAATTGCCTCTAAGACTCAGAGTATCAAAAAGTACTCAACCGACATTC
TGGGAAACCTGTATGAGGTGAAGAGCAAAAAGCACCCTCAGATTATCAAAAA
GGGCTAAGAATTC

*Example 35: Minimizing off-target cleavage using Cas9 nickase and two guide RNAs*

Cas9 is a RNA-guided DNA nuclease that may be targeted to specific locations in the genome with the help of a 20bp RNA guide. However the guide sequence may tolerate some mismatches between the guide sequence and the DNA-target sequence. The flexibility is undesirable due to the potential for off-target cleavage, when the guide RNA targets Cas9 to a an off-target sequence that has a few bases different from the guide sequence. For all experimental applications (gene targeting, crop engineering, therapeutic applications, etc) it is important to be able to improve the specificity of Cas9 mediated gene targeting and reduce the likelihood of off-target modification by Cas9.

Applicants developed a method of using a Cas9 nickase mutant in combination with two guide RNAs to facilitate targeted double strand breaks in the genome without off-target modifications. The Cas9 nickase mutant may be generated from a Cas9 nuclease by disabling its cleavage activity so that instead of both strands of the DNA duplex being cleaved only one strand is cleaved. The Cas9 nickase may be generated by inducing mutations in one ore more domains of the Cas9 nuclease, e.g. Ruvcl or HNH. These mutations may include but are not limited to mutations in a Cas9 catalytic domain, e.g in SpCas9 these mutations may be at positions D10 or H840. These mutations may include but are not limited to D10A, E762A, H840A, N854A, N863A or D986A in SpCas9 but nickases may be generated by inducing mutations at corresponding positions in other CRISPR enzymes or Cas9 orthologs. In a most preferred embodiment the Cas9 nickase mutant is a SpCas9 nickase with a D10A mutation.

The way this works is that each guide RNA in combination with Cas9 nickase would induce the targeted single strand break of a duplex DNA target. Since each guide RNA nicks one strand, the net result is a double strand break. The reason this method eliminates off-target mutations is because it is very unlikely to have an off-target site that has high degrees of similarity for both guide sequences (20bp+2bp(PAM) = 22bp specificity for each guide, and two guides means any off-target site will have to have close to 44bp of homologous sequence). Although it is still likely that individual guides may have off-targets, but those off-targets will only be nicked, which is unlikely to be repaired by the mutagenic NHEJ process. Therefore the multiplexing of DNA double strand nicking provides a powerful way of introducing targeted DNA double strand breaks without off-target mutagenic effects.

Applicants carried out experiments involving the co-transfection of HEK293FT cells with a plasmid encoding Cas9(D10A) nickase as well as DNA expression cassettes for one or more guides. Applicants transfected cells using Lipofectamine 2000, and transfected cells were harvested 48 or 72 hours after transfections. Double nicking-induced NHEJ were detected using the SURVEYOR nuclease assay as described previously herein (Figs. 51, 52 and 53).

Applicants have further identified parameters that relate to efficient cleavage by the Cas9 nickase mutant when combined with two guide RNAs and these parameters include but are not limited to the length of the 5' overhang. Efficient cleavage is reported for 5' overhang of at least 26 base pairs. In a preferred embodiment the 5' overhang is at least 30 base pairs and more preferably at least 34 base pairs. Overhangs of up to 200 base pairs may be acceptable for cleavage, while 5' overhangs less than 100 base pairs are preferred and 5' overhangs less than 50 base pairs are most preferred (Figs. 54 and 55).

*Example 36: Generation of in vivo murine models of myeloid malignancy with combinatorial genetic lesions using CRISPR/Cas genome editing (I)*

Recent genome sequencing efforts have demonstrated that human malignancies commonly bear mutations in four or more driver genes. Murine models of these recurrent mutations have yielded insights into the biology and treatment of cancer, but it has not yet been possible to engineer primary cells that recapitulate the genetic complexity of human disease. Mice bearing combinations of three or more genetic lesions are exponentially more difficult to generate through breeding. Recent advances in genome editing technologies now offer the potential to overcome these limitations, but are restricted to cell types and organisms amendable to direct nucleic acid transfer. Applicants generated a lentiviral Clustered-Regularly-Interspaced-Short-Palindromatic-Repeats (CRISPR)-CRISPR-associated-system (Cas) delivery system and utilized it to modify murine hematopoietic stem cells (HSCs) *in vivo* at up to five genomic loci, leading to clonal outgrowth and myeloid malignancy. Lentivirus-delivered CRISPR-Cas genome editing is a powerful tool for the engineering of *in vivo* cancer models.

Transcriptional Activator Like Effector Nucleases (TALENs), and more recently the CRISPR-Cas system have been developed for the targeted editing of genomic loci. Both systems have been successfully used to engineer the genomes of multiple model organisms. A limitation of these technologies, however, is the efficient delivery of the required components into specific cell types. In particular, primary hematopoietic stem and progenitor cells (HSPC), the disease-initiating cell populations for myeloid malignancies, are difficult to target with commonly used gene transfer methods. Viral vectors derived from HIV-1 are currently the most efficient gene delivery tools, but their application for the delivery of TALENs has been restricted due to the highly repetitive nature of the introduced proteins, leading to recombination of the vectors. Type II CRISPR-Cas, which does not contain such highly repetitive sequences, has recently been adapted for specific DNA targeting in mammalian cells, and is therefore amenable to expression from lentiviral vectors.

To perform genome editing with high efficiency in primary HSPCs, and to track the engineered cells in vivo, Applicants generated a modular lentiviral CRISPR-Cas vector (Figs. 56A and 59). This lentiviral vector simultaneously delivers both the *Streptococcus pyogenes* Cas9 gene and a chimeric small guide RNA (sgRNA) or a chimeric RNA polynucleotide sequence (chiRNA). This single vector enables the targeting of any genomic locus in a broad range of cell types by a one-step exchange of the target site (spacer). To allow tracking or selection of transduced cells, a fluorescent protein or selectable marker was linked to the Cas9 gene with a picorna virus derived 2A protein cleavage site.

Since efficacy of the CRISPR-Cas system may depend on the spacer sequence, Applicants developed an assay for rapid and quantitative evaluation of target site cleavage. Standard assays such as those utilizing T7 endonuclease are time consuming, require optimization for individual target sites, and are challenging to use quantitatively. Fluorescent reporters are an alternative approach to quantify the efficacy of genome engineering tools or RNA interference. Applicants generated a lentiviral reporter system in which Applicants introduced up to 20 target sequences into the coding sequence of a fluorescent protein (Fig. 56A, bottom). The vector confers puromycin resistance, enabling the rapid generation of stable cell lines for genomic testing of spacer efficacy. Transduction of these reporter cell lines with targeting CRISPR-Cas vectors leads to quantifiable loss of reporter fluorescence (Fig. 56B).

To model myeloid malignancies *in vivo,* Applicants first developed lentiviral CRISPR-Cas vectors targeting 8 genes that are recurrently mutated in hematopoietic malignancies. Applicants tested an average of 3 spacers per gene and found that approximately 60% of the vectors achieved cleavage (>20% reduction of mean fluorescent intensity (MFI)) of their respective target genes (Fig. 56C). Results for two spacers were confirmed by the standard T7 endonuclease assay, demonstrating cutting at the genomic locus (Fig. 60). These findings demonstrate that the Applicants' lentiviral system achieves efficient cleavage of target sites, and that engineered fluorescent reporter cell lines can provide rapid, quantitative evaluation of spacer efficacy.

Having demonstrated the efficiency of the lentiviral delivery system *in vitro,* Applicants examined whether the genomes of primary murine HSPCs could be engineered *in vivo.* Applicants isolated Lineage-/Scal+/cKit+ (LSK) cells, which are enriched for HSPC activity, from mice with knock-in of the Flt3 internal tandem duplication (Flt3-ITD) mutation. While Flt3-ITD mutations frequently occur in acute myeloid leukemia (AML), the Flt3-ITD knock-in mice have only a mild hematopoietic phenotype, and Applicants hypothesized that additional co-operating oncogenic lesions would induce a more aggressive disease. Heterozygous Flt3-ITD expressing LSK cells were transduced with an eGFP-expressing CRISPR-Cas lentiviral vector with an sgRNA targeting the Runx1 gene or a non-targeting sgRNA (Fig. 56A), and were then transplanted into lethally irradiated wild-type (WT) congenic recipient mice. In serial analysis of the peripheral blood of recipient mice, Applicants found that the population of cells expressing Cas9 and the *Runx1* sgRNA expanded significantly over a period of 19 weeks in comparison to the population of cells expressing Cas9 and the non-targeting sgRNA (p=0.03; t-test, two-sided, unpaired), thus indicating that loss of *Runx1* may induce a growth advantage in heterozygous Flt3-ITD expressing cells in vivo (Figs. 56D, 61A). Detection of eGFP-positive cells at 19 weeks post-transplant indicated that Applicants transduced long-term repopulating HSCs (Figs. 56E, 61B and 61C).

To demonstrate genome modification of HSCs, Applicants performed deep sequencing of the genomic sequence targeted in *Runx1* in peripheral blood at serial time points. At 19 weeks post-transplant, only long-term repopulating HSCs provide mature cells to hematopoiesis, and any unique mutation found at this time point is therefore expected to be in an HSC. Applicants identified at least 16 distinct insertions or deletions at the targeted *Runx1* site in peripheral blood harvested 5 weeks post-transplant that were also present in blood 19 weeks post-transplant (Fig. 62). These findings demonstrate that multiple distinct HSCs were genetically modified. Overall, these results demonstrate that lentiviral delivery of the CRISPR-Cas system in HSCs is feasible and well tolerated, and that this system can be employed to edit the genomes of HSCs to interrogate gene function in hematopoiesis.

Since myeloid malignancies, and cancer more broadly, are caused by multiple cooperating genetic lesions, Applicants next sought to target multiple cancer-associated genes simultaneously. To facilitate multiplex genome editing, Applicants generated a two-vector system in which Cas9, and an eGFP fluorescent marker, are delivered from one lentiviral vector, while additional sgRNAs and an RFP657 fluorescent marker are delivered by a second lentiviral vector (Fig. 57A). Multiple sgRNAs can thus be introduced into a single cell.

Applicants sought to perform multiplex gene editing by transducing LSK cells from C57Bl/6 wild-type mice with a pool of vectors providing sgRNAs, thereby enabling the simultaneous targeting of the *Tet2, Runx1, Dnmt3a, Nf1, Ezh2, Smc3* genes. Applicants found that all mice transplanted with LSK cells transduced with this pool developed significant myeloid skewing of hematopoiesis (p=0.007; t-test; Welch corrected; two-sided; unpaired) with reduction of B-cells (p=0.001; t-test, two-sided; unpaired), and some developed leukocytosis, as compared to the control group that received LSK cells transduced with control vectors expressing Cas9 and a non-targeting sgRNA (Fig. 57B).

To determine the efficacy of multiplex genome editing, Applicants examined the genetics of putative clones in the peripheral blood of mice. Two-dimensional flow cytometry analysis of eGFP and RFP657 allowed us to detect clonal hematopoietic expansion *in vivo* (Figs. 57C, 57D, 63), though some clonal, mutated populations were only GFP dim (Figs. 63 and 64). For two mice, Applicants purified clonal populations from peripheral blood based on their eGFP/RFP657 profile (red gated), by fluorescent activated cell sorting (FACS) (Figs. 57C and 57D). Applicants examined the mutated genes by PCR amplification, sub-cloning, and sequencing of targeted gene regions. In one mouse, a clone expanded gradually over 12 weeks that had mutations in *Tet2, Dnmt3a, Runx1,* and *Nf1* (Figs. 57C, 66A), while no mutations in *Ezh2* and *Smc3* were detected. In a second mouse, a clone expanded rapidly after transplantation with mutations in *Tet2, Runx1, Nf1* and *Ezh2* (Figs. 57D, 66B), and no mutations in *Dnmt3a* and *Smc3.* In both cases, the detected mutational pattern strongly suggests that a single cell was transduced with four separate sgRNAs, resulting in modification of one or both alleles of four tumor suppressor genes. Despite expansion of these clonal populations, no transformation to acute leukemia was observed in these mice during the observation period of up to 14 weeks.

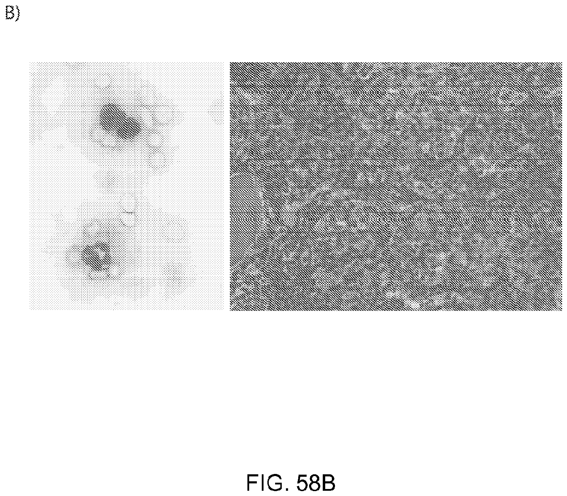

Transformation to acute leukemia was observed in a different mouse transduced with the same pool of sgRNAs. A clonal population was detected at 4 weeks post-transplant, and the mouse was euthanized at 6 ½ weeks post-transplant with mild leukocytosis (white blood cell (WBC) count: 15.3x10^6/ml; normal: 5-10x10^6/ml) and splenomegaly (436 mg; normal: 80-120mg). The bone marrow had a high disease burden, with 85% of cells expressing the CRISPR-Cas vectors (Fig. 58A). The cells were skewed to the myeloid lineage (Gr1+/CD11b+). Analysis of the targeted genes revealed deletions in *Dnmt3a, Ezh2, Smc3* and *Nf1,* confirming a clonal origin of the disease (Figs. 58A right panel, 67). The peripheral blood from these mice had increased granulocytes and circulating blasts, which are signs of leukemic transformation (Fig. 58B, left panel). Bone marrow histology showed an excess of myeloid lineage cells (>90%) with 20-30% blast cells, while other lineages were suppressed (Fig. 58B, right panel), consistent with acute myeloid leukemia (AML).

157

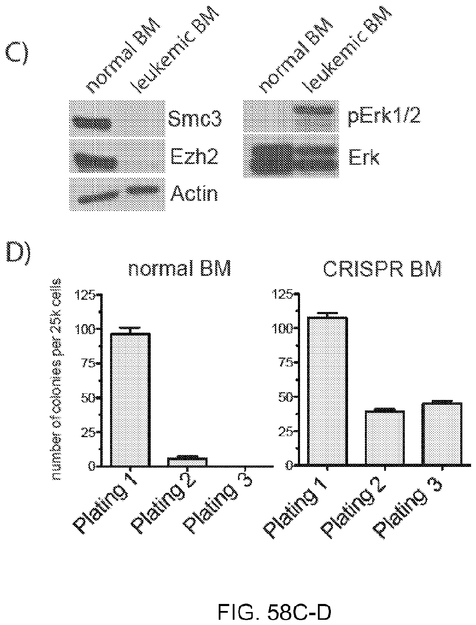

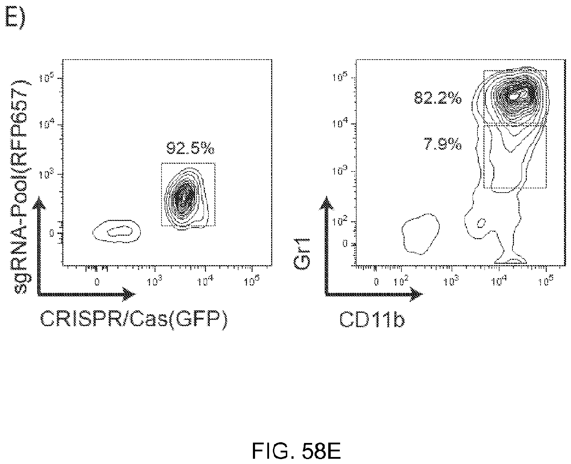

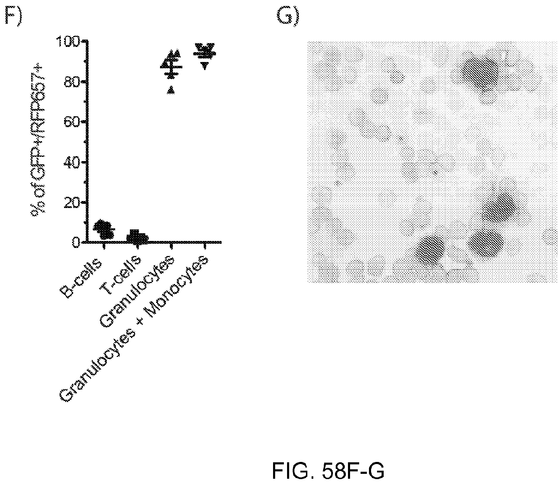

Applicants next confirmed that the detected mutations had an impact on protein expression and cellular phenotype. Both Ezh2 and Smc3 protein expression were markedly reduced. Nf1 is a negative regulator of the Ras pathway, and Applicants observed de-repression of the downstream targets, Erk1 and Erk2, as expected for cells with inactivation of Nf1 (Fig. 58C). Serial replating of FACS-purified, double positive eGFP/RFP657-expressing cells indicates increased self-renewal capacity compared to wild-type C57Bl/6 BM cells (Fig. 58D). In transplant experiments, the GFP/RFP657 double positive mutated cells robustly engrafted in secondary recipient mice (Fig. 68A). At 8 weeks post-transplant two out of five recipient mice developed leukocytosis (Fig. 68B). Mutated cells in all mice had highly biased differentiation with >90% of the cells belonging to the myeloid lineage (Figs. 58E and F) similar to the phenotype of the primary disease (Fig. 58G). At 14 weeks post transplant a second primary mouse succumbed to myeloid leukemia characterized by splenomegaly (412mg), leukocytosis (WBC: $33.6x10^6$/ml), excess myeloid cells in the BM (Fig. 69A), and circulating blasts in peripheral blood (Fig. 69B). Clonal mutations were identified in *Tet2, Dnmt3a, Runx1, Nf1,* and *Ezh2.* None of the mice transplanted with non-targeting sgRNA expressing cells developed disease during the course of the experiment These studies demonstrate that, using lentiviral delivered CRISPR-Cas components, Applicants have engineered novel models of myeloid malignancy bearing mutations in 4-5 different genes that can be propagated and studied through serial transplantation.

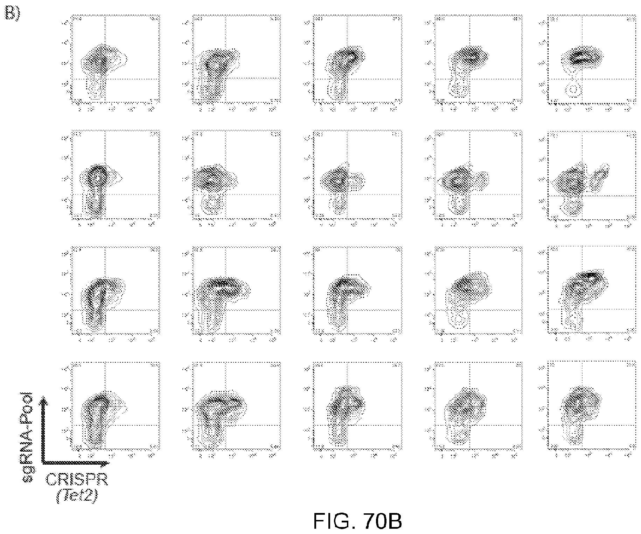

To examine an alternative set of co-operative mutations, the same pool of sgRNAs targeting leukemia tumor suppressor genes was used to transduce LSK cells from mice with heterozygous knock-in of the Flt3-ITD oncogene allele. Targeting of Runx1 alone did not induce transformation of *Flt3-ITD* heterozygous expressing cells (Fig. 56D). Consistent with the results of pooled tumor suppressor gene targeting in wild-type C57Bl/6 mice, expansion of CRISPR/Cas-transduced, Flt3-ITD-expressing cells was observed over time. In some mice, expression of the CRISPR/Cas vector was GFP-dim at 8-10 weeks (Fig. 70). A total of three out of nine (33%) mice developed disease at 10-12 weeks with leukocytosis (WBC: $15.4x10^6$/ml, $40.4x10^6$/ml, and $78.1x10^6$/ml) and splenomegaly (326mg, 533mg, and 1036mg). Flow cytometry of the BM demonstrated that cells were myeloid biased (Figs. 71A, 72A, 73A). Myeloid differentiation bias and presence of blast cells were confirmed by histological analysis of blood smears (Figs. 71B, 72B, 73B), consistent with AML in these mice. Clonal mutations were detected in 3-5 genes in the three mice (Figs. 71C, 72C, 73C). Since the mutations were engineered in the Flt3-ITD heterozygous background, AML was generated from primary cells with mutations in 4 to 6 leukemia driver genes.

Taken together, these studies of lentivirus-delivered CRISPR/Cas with multiplex sgRNAs demonstrated highly efficient genome editing in murine HSPCs. Applicants simultaneously inactivated multiple tumor suppressor genes causing outgrowth of a myeloid clone and transformation to leukemia. Applicants described models with 4 and more genes modified, with editing of up to 10 individual alleles, when a pool of sgRNAs targeting 6 genes was introduced in primary HSPCs. While genetic models have recently been generated using CRISPR-Cas in whole organisms, Applicants' system enables tissue specific interrogation of gene function in adult organisms while avoiding potential developmental defects. The genetic complexity thus generated is beyond the capacities of current approaches for engineering murine malignancies and will allow rapid generation of novel disease models. Such models are critical for the functional interrogation of novel disease alleles and for the development and pre-clinical testing of targeted therapies.

The novel leukemia models generated reflect both the number and functional classes of co-occurring mutations that occur during the pathogenesis of human myeloid malignancies. All of the clones characterized had inactivating mutations in *Nf1,* a lesion that activates Ras signaling, and the only mutation in the screen that causes activated kinase signaling, a common feature of leukemias and other cancers. Applicants also identified mutations in *Ezh2,* a gene recently shown to be involved in the differentiation block in AML, in all mice that presented with myeloid transformation. Inhibition of myeloid differentiation has also been reported *Dnmt3a, Tet2,* and *Runx1* each of which were successfully mutated in Applicants' models.

Overall, Applicants studies demonstrated a robust methodology to generate multiple genetic lesions via lentivirus-delivered CRISPR-Cas system in a broad range of cells, including the primary adult stem cells that are the disease-initiating population for leukemia. The use of viral-based CRISPR-Cas will enable the rapid and efficient generation of cancer models that reflect the complex genetics of human disease.

Vector construction and viral particle production: Lentiviral vectors were based on the pLKO_TRC005 lentiviral backbone. The codon optimized Streptococcus pyogenes Cas9 (SpCas9) cDNA and small guide RNA (sgRNA) have been described by Applicants. The sgRNA was amplified from the pLX330 backbone and placed behind the human U6 promoter of the pLKO_TRC005. Vectors expressing only Cas9 and no sgRNA were generated by deleting the hU6 promoter. BbsI sites were replaced by BsmBI sites for insertion of the spacer sequence. The hPGK promoter of the pLKO_TRC005 was replaced by a minimal size hEFla promoter (EFS). To generate vectors expressing Cas9 only, the hU6 promoter was deleted from the pLKO_TRC005 backbone by cutting PpuMI/EcoRI followed by Klenow fragment fill-in reaction and ligation. SpCas9 cDNA was PCR amplified (Phusion DNA Polymerase, NEB) and placed behind the EFS promoter followed by addition of P2A-eGFP, P2A-tagRFP, P2A-PAC or P2A-BSD. Optimized picorna virus 2A sites were fused to respective cDNAs via PCR. The sgRNA-only vectors were generated by replacing the SpCas9 and respective marker gene with a fluorescent protein marker, or selection marker only. The RFP657 fluorescent protein was described previously in Morozova et. al (2010). Suitable protospacer sequences were identified with unique 13bp seed sequence and a minimum of 3 mismatches to the mouse genome. Lentiviral reporter vectors were generated by replacing the eGFP fluorescent reporter gene in the RRL.PPT.SFFV.IRES.eGFP.pre with PAC, followed by insertion of the RFP657 fluorescent reporter gene 5' of the IRES. CRISPR/Cas target sites were introduced behind the start codon of the RFP657.

Lentiviral particles were produced by transient transfection of 293T cells using the calcium-phosphate transfection method. Viral constructs were co-transfected with pMD2.G and psPAX2 . Lentiviral particles were concentrated using ultracentrifugation. Oligonucleotide sequences used for the cloning of CRISPR/Cas protospacers used in this study are shown in Table 1 below.

| Targeted Gene | # | Oligo | Sequence (5'>3') |
|---|---|---|---|
| Runx1 | 1 | s | CACC GGCACTGGGGGACGTCCCGGA |
| | | as | AAAC TCCGGGACGTCCCCCAGTGCC |
| Runx1 | 2 | s | CACC GCCGGGTGAAATGGGTGTCGC |
| | | as | AAAC GCGACACCCATTTCACCCGGC |
| Runx1 | 3 | s | CACC GGGCTGGTCATGCCGCTGGCA |
| | | as | AAAC TGCCAGCGGCATGACCAGCCC |
| Tet2 | 1 | s | CACC GAATACTATCCTAGTTCCGAC |
| | | as | AAAC GTCGGAACTAGGATAGTATTC |
| Tet2 | 2 | s | CACC GGAACAAGCTCTACATCCCGT |
| | | as | AAAC ACGGGATGTAGAGCTTGTTCC |
| Tet2 | 3 | s | CACC GATGAGATGCGGTACTCTGCA |
| | | as | AAAC TGCAGAGTACCGCATCTCATC |
| Dnmt3a | 1 | s | CACC GTGGGCATGGTGCGGCACCA |
| | | as | AAAC TGGTGCCGCACCATGCCCAC |
| Dnmt3a | 2 | s | CACC GCATGATGCGCGGCCCAAGG |
| | | as | AAAC CCTTGGGCCGCGCATCATGC |
| Dnmt3a | 3 | s | CACC GCTTACCAGTATGACGACGA |
| | | as | AAAC TCGTCGTCATACTGGTAAGC |
| Nf1 | 1 | s | CACC GAAGGAGCGGTCCCGGTAAC |
| | | as | AAAC GTTACCGGGACCGCTCCTTC |
| Nf1 | 2 | s | CACC GCAGATGAGCCGCCACATCGA |
| | | as | AAAC TCGATGTGGCGGCTCATCTGC |
| Nf1 | 3 | s | CACC GCACTGACAGCGCCCATAGGT |
| | | as | AAAC ACCTATGGGCGCTGTCAGTGC |
| Ezh2 | 1 | s | CACC GCTGAGAAGGGACCGGTTTGT |
| | | as | AAAC ACAAACCGGTCCCTTCTCAGC |
| Ezh2 | 2 | s | CACC GACACGCTTCCGCCAACAAAC |
| | | as | AAAC GTTTGTTGGCGGAAGCGTGTC |
| Ezh2 | 3 | s | CACC GAAGACACCACCTAAACGCCC |
| | | as | AAAC GGGCGTTTAGGTGGTGTCTTC |
| Smc3 | 1 | s | CACC GCTCCAGTCAGAGCACCTCGA |
| | | as | AAAC TCGAGGTGCTCTGACTGGAGC |
| Smc3 | 2 | s | CACC GAAAAGCTGATAAAGCGGCAAG |
| | | as | AAAC CTTGCCGCTTTATCAGCTTTTC |
| Asxl1 | 1 | s | CACC GCAACAGTGCCATTCGAGGCC |
| | | as | AAAC GGCCTCGAATGGCACTGTTGC |
| Asxl1 | 2 | s | CACC GATACTAAAACCGACTTAGC |
| | | as | AAAC GCTAAGTCGGTTTTAGTATC |
| Asxl1 | 3 | s | CACC GAGCCCAAAGTCCCGCCCATC |
| | | as | AAAC GATGGGCGGGACTTTGGGCTC |
| p53 | 1 | s | CACC GAGAAGAAAATTTCCGCAAAA |
| | | as | AAAC TTTTGCGGAAATTTTCTTCTC |
| p53 | 2 | s | CACC GCGCTCCCTGGGGGCAGTTCA |
| | | as | AAAC TGAACTGCCCCCAGGGAGCGC |
| p53 | 3 | s | CACC GCTATTACACATGTACTTGTAG |
| | | as | AAAC CTACAAGTACATGTGTAATAGC |

#: Spacer number; s: sense; as: antisense;

Reporter based efficacy assessment of CRISPR-Cas spacer sequences: Fluorescent reporter vectors were constructed and produced as described above. HEL cells (ATCC) were cultured in RPMI with 10% FBS and 100 U/ml Penicillin/Streptomycin. HEL cells were transduced with CRISPR/Cas reporter vectors in the presence of 4ug/ml hexadimethrine bromide (Polybrene (Life Technologies)) and transduced cells were selected with 2ug/ml Puromycin (Life Technologies) for 72h, starting 48h post transduction. Selection was verified by flow cytometry. Selected reporter cell lines were transduced with CRISPR-Cas vectors targeting the respective target sites and non-targeting controls. RFP657 reporter fluorescence was assessed by flow cytometry at 6 days post transduction in comparison to non-targeting controls and untransduced cells.

T7-endonuclease assay: Ba/F3 cells (DSMZ) were cultured in RPMI with 10% FBS, 100 U/ml Penicillin/Streptomycin and 5 ng/ml 113 (Peprotech). Ba/F3 cells were transduced with CRISPR/Cas vectors, and transduced cells were purified by FACS 7 days post-infection. Genomic DNA was isolated according to manufacturers instruction with a DNA Micro Kit (Quiagen). Targeted loci were amplified by PCR (Phusion DNA Polymerase, NEB). CRISPR/Cas efficacy at the endogenous locus was assessed using the Surveyor Mutation Detection Kit (Transgenomic).

Mouse bone marrow transplant and analysis: Total BM was isolated from femurs and tibias from 6-10 week old female C57B1/6 mice or heterozygous 6-12 week old Flt3-ITD knock-in mice. BM was subjected to erythrocyte lysis (BD PharmLyse, BD Bioscience), followed by magnetic bead selection of cKit (CD117) positive cells using CD117 MicroBeads (Milteny Biotech). CD117 selected cells were stained with APC labeled cKit (eBioscience), PE labeled Sca1 (BioLegend) PacificBlue labeled Gr1, CD11b, B220(CD45R), Ter119, and CD3 (all BioLegend) and then flow sorted on a BD FACS Aria II. LSK cells were cultured in StemSpan SFEM (StemCell Technologies) supplemented with 50ng/ml murine Thpo and 50ng murine Scf (both Peprotech) for 48h and then transduced with concentrated lentiviral supernatant in presence of 2ug/ml Polybrene. 24h post transduction cells were collected and intravenously injected into lethally irradiated (950 cGy) 6-10 week old female C57B1/6 mice. C57B1/6 mice were obtained from Taconic. Flt3-ITD mice were bred in local facilities.

All transplant experiments were performed with 4-5 mice per group. Statistical significance between groups was assessed using an unpaired t-test. Peripheral blood was sampled by retro-orbital bleeding. Bone marrow was harvested from femurs and tibias. Nucleated cells were obtained by erythrocyte lysis. To detect mature lineage markers, cells were stained with Gr1-APC-eFluor780 (eBioscience), CD11b-APC (BioLegend), B220(CD45R)-PE-Cy7 (BioLegend) and CD3e-PE (eBioscience). HSPCs were stained with PacificBlue labeled Grl, CD11b, B220(CD45R), Ter119, and CD3 (all BioLegend), Sca1-PE (BioLegend), CD150-PE-Cy7 (BioLegend), cKit-APC (eBioscience), CD48-PerCP-Cy5.5 (eBioscience), and CD34-AlexaFluor700 (eBioscience). Flow cytometry was performed on a BD LSR II flow cytometer. Cells expressing both the eGFP and RFP657 reporter gene were stained with Gr1-PE-Cy7 (eBioscience) and CD11b-PE (BioLegend) or B220(CD45R)-PE-Cy7 and CD3e-PE for mature lineage detection.

To assess mutations at targeted loci, peripheral blood nucleated cells were purified by FACS. Genomic DNA was isolated according to manufacturers instruction with a DNA Micro Kit (Quiagen). Targeted loci were amplified by PCR (Phusion DNA Polymerase, NEB). PCR products were cloned into pJetl.2 PCR cloning vector (Life Technologies). Plasmid DNA was isolated from individual bacterial clones with a DNA Plasmid Mini Kit (Quiagen) and sequences were assessed by Sanger sequencing.

Secondary transplant of leukemic mice was performed by transplanting 5x10^6 primary BM cells into sub-lethally (650cGy) irradiated C57B1/6 mice. Peripheral blood semars were stained with May-Gruenwald/Giemsa stain. BM histology was assessed from paraffin embedded tissue sections stained with Hematoxilin and Eosin. Clonogenic colony assays were performed in cytokine-supplemented methylcellulose (StemCell Technologies ). 25,000 C57B1/6 BM wildtype BM cells or 25,000 cells from leukemic mice were plated. Colony numbers were assessed 7 days post seeding. For re-plating experiments, cells were washed with PBS, counted, and plated as described above.

Immunoblots: Nucleated C57B1/6 wildtype BM cells or leukemic BM cells were lysed with RIPA buffer (Pierce) supplemented with Halt Protease Inhibitor (Pierce). Protein concentrations were determined using Bradford protein assay (Bio-Rad). Equal protein amounts were separated by SDS-PAGE and blotted on nitrocellulose membrane, blocked with fat free milk and incubated with primary antibodies (Cell Signaling; Ezh2 clone D2C9; Smc3 clone D47B5; phospho-Erk1/2 (phospho-p44/42) clone D13.14.4E; Erkl/2 (p44/42) clone 137F5). Mouse beta -actin was obtained from Satnta Cruz (clone C4). After washing blots were incubated with HRP-conjugated secondary antibody and developed using SuperSignal West Pico Chemiluminescent Substrate (Pierce).

Statistical analysis: Statistical analysis was performed with GraphPad Prism software (Graphpad Software). Data is are shown as the mean with standard error of the mean. Groups were compared using an unpaired t-test. Normal distribution was assumed. Differences in variance were analyzed using an F-test Welch correction for t-test was applied for unequal variances and is indicated.

Next Generation Sequencing to Assess Mutational Repertoire: gDNA was isolated using a DNA Blood Mini kit (Quiagen) according to manufacturers instructions. The genomic region flanking the CRISPR target site for each region was amplified by a nested PCR to attach the Illumina adapters in the first round and to add barcodes unique for each individual mouse in the second PCR. All libraries were pooled and purified using Agencourt AMPure XP system (Beckman Coulter). Single end, 300 cycle sequencing was performed using MiSeq (Illumina). Reads were adapter trimmed and aligned using BLAT to reference amplicon sequences. Indels were surveyed using Integrative Genomics Viewer.

Generation of human isogenic cancer cell lines is of particular interest for pharmaceutic industry and Applicants provide further data on the transformation of human cancer cell lines (Fig. 74). The lentiviral vectors for delivery of Cas9, sgRNA and marker gene have been described by applicants previously in this application. The GM-CSF dependent human erythroleukemia cell line was obtained from ATCC and grown in RPMI supplemented with 10% FBS and 2ng/ml human GM-CSF. TF1 cells were transduced with lentiviral CRISPR-Cas constructs in the presence of 4ug Polybrene. Seven days post transduction cells were washed three times with DMEM to remove the GM-CSF. Washed cells were counted using a Beckman/Coulter Vi-Cell and seeded at a density of 2x10^5 cells per ml in 12-well plates (2.5ml per well) in RPMI with 10% FBS. Control vector transduced cells were seeded in RPMI with 10% FBS and in RPMI with 10% FBS and 2ng/ml GM-CSF as positive control. Cells were counted over time using a Beckman/Coulter Vi-Cell and cumulative cell count was assessed

A listing of targeted genes and the spacer ot guide sequences used are as follows:

| Targeted Gene | Spacer |
| --- | --- |
| TET2 | GTGGAGAAAGACGTAACTTCG |
| TET2 | GTCTGCCCTGAGGTATGCGAT |
| TET2 | GAGGAGAACTTGCGCCTGTCA |
| DNMT3a | GCATGGGCTGCTTGTTGTACG |
| DNMT3a | GTGTCACTCTCATCGCTGTCG |
| DNMT3a | GCAGAAGTGCCGGAACATTG |
| BCOR | GACTGGAGAATACAGCGGCT |
| BCOR | GTGGGGACCGACGTAGTGAGG |
| BCOR | GTGCTGAATAACGGATGGTG |
| EZH2 | GAGCTCATTGCGCGGGACTA |
| EZH2 | GATTCCAGCACATTAATGGT |
| EZH2 | GGAAGCAGGGACTGAAACGG |
| TP53 | GCCATTGTTCAATATCGTCCG |
| TP53 | GCACCAGCAGCTCCTACACCGG |
| TP53 | GACGGAAACCGTAGCTGCCC |
| ASXL1 | GTTCTCGGGCTGCCACGCCGA |
| ASXL1 | GCTGGGCAGCGCTGCTATTCG |
| ASXL1 | GGAAGGTCCGAGAGTTGATC |
| NF1 | GAATTACAGGGCTCGTCCAAC |
| NF1 | GTCTGGGCTTGTCGGCAAATCG |
| NF1 | GAACCCTTACGTTCACTGAC |
| WT1 | GTAGCTGGGCGTCCCGTCGA |
| WT1 | GGTTGGGGAACTGCGCCGCA |
| WT1 | GATGCCGACCGTACAAGAGTC |
| PTEN | GAACTTGTCTTCCCGTCGTGT |
| RUNX1 | GACTGATCGTAGGACCACGGT |
| RUNX1 | GGTAGGTGGCGACTTGCGGT |
| RUNX1 | GCTCAGGTTTGTCGGTCGAAG |
| SMC3 | GTCATCTTCGTCCAGAACAG |
| SMC3 | GTAGAGTGTATGACGAACGAA |
| SMC3 | GGTGACCAAGTCAGCCATCG |

References:

1. Genomic and epigenomic landscapes of adult de novo acute myeloid leukemia. The New England journal of medicine 368, 2059-2074 (2013).

2. Jinek, M. et al. A programmable dual-RNA-guided DNA endonuclease in adaptive bacterial immunity. Science 337, 816-821 (2012).

3. Cong, L. et al. Multiplex genome engineering using CRISPR/Cas systems. Science 339, 819-823 (2013).

4. Mali, P. et al. RNA-guided human genome engineering via Cas9. Science 339, 823-826 (2013).

5. Bogdanove, A.J. & Voytas, D.F. TAL effectors: customizable proteins for DNA targeting. Science 333, 1843-1846 (2011).

6. Reyon, D. et al. FLASH assembly of TALENs for high-throughput genome editing. Nature biotechnology 30, 460-465 (2012).

7. Hwang, W.Y. et al. Efficient genome editing in zebrafish using a CRISPR-Cas system. Nature biotechnology 31, 227-229 (2013).

8. Wang, H. et al. One-step generation of mice carrying mutations in multiple genes by CRISPR/Cas-mediated genome engineering. Cell 153, 910-918 (2013).

9. Friedland, A.E. et al. Heritable genome editing in C. elegans via a CRISPR-Cas9 system. Nature methods 10, 741-743 (2013).

10. Shan, Q. et al. Targeted genome modification of crop plants using a CRISPR-Cas system. Nature biotechnology 31, 686-688 (2013).

11. Wood, A.J. et al. Targeted genome editing across species using ZFNs and TALENs. Science 333, 307 (2011).

12. Sander, J.D. et al. Targeted gene disruption in somatic zebrafish cells using engineered TALENs. Nature biotechnology 29, 697-698 (2011).

13. Holkers, M. et al. Differential integrity of TALE nuclease genes following adenoviral and lentiviral vector gene transfer into human cells. Nucleic acids research 41, e63 (2013).

14. Hsu, P.D. et al. DNA targeting specificity of RNA-guided Cas9 nucleases. Nature biotechnology 31, 827-832 (2013).

15. Fellmann, C. et al. Functional identification of optimized RNAi triggers using a massively parallel sensor assay. Molecular cell 41, 733-746 (2011).

16. Lee, B.H. et al. FLT3 mutations confer enhanced proliferation and survival properties to multipotent progenitors in a murine model of chronic myelomonocytic leukemia. Cancer cell 12, 367-380 (2007).

17. Kelly, L.M. & Gilliland, D.G. Genetics of myeloid leukemias. Annual review of genomics and human genetics 3, 179-198 (2002).

18. Tanaka, S. et al. Ezh2 augments leukemogenicity by reinforcing differentiation blockage in acute myeloid leukemia. Blood 120, 1107-1117 (2012).

19. Challen, G.A. et al. Dnmt3a is essential for hematopoietic stem cell differentiation. Nature genetics 44, 23-31 (2012).

20. Moran-Crusio, K. et al. Tet2 loss leads to increased hematopoietic stem cell self-renewal and myeloid transformation. Cancer cell 20, 11-24 (2011).

21. Quivoron, C. et al. TET2 inactivation results in pleiotropic hematopoietic abnormalities in mouse and is a recurrent event during human lymphomagenesis. Cancer cell 20, 25-38 (2011).

22. Mead, A.J. et al. FLT3-ITDs instruct a myeloid differentiation and transformation bias in lymphomyeloid multipotent progenitors. Cell reports 3, 1766-1776 (2013).

23. Cong, L. et al. Multiplex genome engineering using CRISPR/Cas systems. Science 339, 819-823 (2013).

24. Hsu, P.D. et al. DNA targeting specificity of RNA-guided Cas9 nucleases. Nature biotechnology 31, 827-832 (2013).

25. Schambach, A. et al. Equal potency of gammaretroviral and lentiviral SIN vectors for expression of O6-methylguanine-DNA methyltransferase in hematopoietic cells. Molecular therapy : the journal of the American Society of Gene Therapy 13, 391-400 (2006).

26. Kim, J.H. et al. High cleavage efficiency of a 2A peptide derived from porcine teschovirus-1 in human cell lines, zebrafish and mice. PloS one 6, e18556 (2011).

27. Morozova, K.S. et al. Far-red fluorescent protein excitable with red lasers for flow cytometry and superresolution STED nanoscopy. Biophysical journal 99, L13-15 (2010).

28. Lee, B.H. et al. FLT3 mutations confer enhanced proliferation and survival properties to multipotent progenitors in a murine model of chronic myelomonocytic leukemia. Cancer cell 12, 367-380 (2007).

*Example 37: Generation of in vivo murine models of myeloid malignancy with combinatorial genetic lesions using CRISPR/Cas genome editing (II)*

Recent genome sequencing efforts have demonstrated that human malignancies commonly bear mutations in four or more driver genes.(Ref. 1) Murine models of these recurrent mutations have yielded insights into the biology and treatment of cancer, but it has not yet been possible to engineer primary cells that recapitulate the genetic complexity of human disease. Mice bearing combinations of three or more genetic lesions are exponentially more difficult to generate through breeding. Recent advances in genome editing technologies now offer the potential to overcome these limitations. (Ref. 2-4) Using a lentiviral small guide RNA (sgRNA):Cas9 delivery system Applicants modified up to five genes in a single murine hematopoietic stem cell (HSC) *in vivo,* leading to clonal outgrowth and myeloid malignancy. sgRNA:Cas9 genome editing, e.g., vector-delivered, such as Lentivirus-delivered sgRNA:Cas9 is a powerful tool for the engineering of *in vivo* cancer models reflecting the complexity of human disease.

Transcriptional Activator Like Effector Nucleases (TALENs), and more recently CRISPR-Cas have been developed for the targeted editing of genomic loci (Ref. 2-6)._ENREF_2 Both systems have been successfully used to engineer the genomes of multiple model organisms. (Ref. 7-12) Primary hematopoietic stem and progenitor cells (HSPC), the disease-initiating cell populations for myeloid malignancies, are difficult to target with commonly used gene transfer methods. Viral vectors derived from HIV-1 are currently the most efficient gene delivery tools, but their application for the delivery of TALENs has been restricted due to the highly repetitive nature of the introduced proteins, leading to recombination of the vectors. (Ref. 13) The *Streptococcus pyogenes cas9* gene (Cas9), which does not contain such highly repetitive sequences, has recently been adapted for specific DNA targeting in mammalian cells,(Ref. 2-4) and is therefore amenable to expression from lentiviral vectors.

Towards the goal of modeling myeloid malignancies using genome editing of primary HSPCs *in vivo,* Applicants generated a modular lentiviral sgRNA:Cas9 vector (Figure 75A and Figure 78). This lentiviral vector simultaneously delivers the *Streptococcus pyogenes cas9* gene, a previously described chimeric sgRNA, and a fluorescent marker.(Ref. 2, 3, 14) This enables the targeting of any genomic locus in a broad range of cell types, and consequent NHEJ-mediated gene disruption, by a one-step exchange of the target site (spacer).

Applicants sought to model loss-of-function mutations in 8 genes that are recurrently inactivated in myeloid malignancies (Tet2, Runx1, Dnmt3a, Ezh2, Nf1, Smc3, p53 and Asx11) using lentiviral sgRNA:Cas9 vectors. Applicants validated the targeting efficacy using a fluorescent reporter harboring up to 20 sgRNA:Cas9 target sites as an alternative approach to commonly used endonuclease assays (Figure 75A/75B, and herein Data & Methods). At least one effective sgRNA was identified for 6 genes (Figure 75C). Results for two spacers were confirmed by the standard T7 endonuclease assay, validating efficient cutting at genomic loci (Figure 75D). These findings demonstrate that Applicants lentiviral system achieves efficient cleavage of target sites, and that engineered fluorescent reporter cell lines can provide rapid, quantitative evaluation of spacer efficacy.

Having demonstrated the efficiency of the lentiviral delivery system *in vitro,* Applicants examined whether Applicants could engineer the genomes of primary murine HSPCs *in vivo.* Applicants isolated Lineage-/Scal+/cKit+ (LSK) cells, which are enriched for HSPC activity, from mice with knock-in of the Flt3 internal tandem duplication (Flt3-ITD) mutation.(Ref. 15) While F1t3-ITD mutations frequently occur in acute myeloid leukemia (AML), Flt3-ITD knock-in is insufficient to cause AML in mice, and Applicants hypothesized that additional co-operating oncogenic lesions would induce transformation. Heterozygous Flt3-ITD expressing LSK cells were transduced with eGFP-expressing sgRNA:Cas9 lentiviral vectors targeting the *Runx1* gene or a non-targeting sgRNA (Figure 75A), and were then transplanted into lethally irradiated wild-type (WT) congenic recipient mice. In serial analysis of the peripheral blood of recipient mice, Applicants found that the population of cells expressing Cas9 and the *Runx1* sgRNA expanded significantly over a period of 19 weeks in comparison to the population of cells expressing Cas9 and the non-targeting sgRNA (p=0.03), thus indicating that loss of *Runx1* may induce a growth advantage in heterozygous F1t3-ITD expressing cells *in vivo* (Figure 75E, Figure 79A).

Detection of eGFP-positive cells at 19 weeks post-transplant in all HSPC compartments indicated that Applicants transduced long-term repopulating HSCs (Figure 75E and Figure 79B and 79C). Stable expression of Cas9-eGFP over the 19 weeks in HSPCs and normal differentiation capacity indicates that continuous Cas9 expression at the levels provided does not cause significant toxicity in hematopoietic cells (Figure 80). As further validation of genome modification of HSCs, Applicants performed deep sequencing of the genomic sequence targeted in *Runx1* in peripheral blood at serial time points. Applicants identified at least 16 distinct insertions or deletions at the targeted *Runx1* site in peripheral blood harvested 5 weeks post-transplant that were also present in blood 19 weeks post-transplant (Figure 81). The majority of genome modifications were deletions ranging from 1-251bp. Overall, these results demonstrate that lentiviral delivery of the sgRNA:Cas9 system in HSCs is feasible and well tolerated, and that this system can be employed to edit the genomes of HSCs to interrogate gene function in hematopoiesis.

Since myeloid malignancies, and cancer more broadly, are caused by multiple cooperating genetic lesions (at least 3-4 oncogenic lesions in AML(Ref. 1)), Applicants next sought to perform multiplex targeting of cancer-associated genes to induce myeloid malignancy. To facilitate multiplex genome editing, Applicants generated a two-vector system in which Cas9 and an eGFP are delivered from one lentiviral vector, while additional sgRNAs and a RFP657 fluorescent marker are delivered by a second lentiviral vector (Figure 76A). The small size of the sgRNA-only vector enables high viral titers and introduction of multiple sgRNAs into a single LSK cell.

Applicants generated a pool of vectors with the single most effective sgRNA for the *Tet2, Runx1, Dnmt3a, Nf1, Ezh2,* and *Smc3* genes. Transduction of LSK cells from C57B1/6 wild-type mice with the pooled virus, following transplantation into lethally irradiated recipient mice, caused significant myeloid skewing of hematopoiesis (p=0.007) with reduction of B-cells (p=0.001) within the transduced cell population, and leukocytosis in some mice, as compared to the control group that received LSK cells transduced with control vectors expressing Cas9 and a non-targeting sgRNA (Figure 76B).

To determine the efficacy of multiplex genome editing and to assess the selective pressure for combinations of mutations Applicants examined the genetics of putative clones in the peripheral blood of mice. Since Applicants used pooled virus expressing different sgRNAs, transduced cells bear different combinations of mutations, and cells with a genotype that drives a competitive advantage would gain a clonal dominance as occurs naturally during leukemogenesis. Two-dimensional flow cytometry analysis of eGFP and RFP657 allowed Applicants to detect putatively clonal hematopoietic expansion *in vivo* (Figures 76C and 76D, Figure 82), though some clonal, mutated populations were only GFP dim (Figure 83 and 84).

For two mice, Applicants purified populations from peripheral blood based on their eGFP/RFP657 density profile (red gated), by fluorescence-activated cell sorting (FACS) (Figure 76C and 76D). Applicants examined the targeted genes by PCR amplification, sub-cloning and sequencing of targeted gene regions. In one mouse, a population expanded gradually over 12 weeks that had mutations in *Tet2, Dnmt3a, Runx1,* and *Nf1* (Figure 76C, Figure 85A), while no mutations in *Ezh2* and *Smc3* were detected. In a second mouse, a population expanded rapidly after transplantation with mutations in *Tet2, Runx1, Nf1* and *Ezh2* (Figure 76D, Figure 85B), and no mutations in *Dnmt3a* and *Smc3*. In both cases, the detected mutational pattern (detection of two allele variants) strongly suggests that a single cell was transduced with four separate sgRNAs, resulting in modification of one or both alleles of four tumor suppressor genes. Despite expansion of these clonal populations, no transformation to acute leukemia was observed in these mice during the observation period of up to 30 weeks.

Transformation to acute leukemia was observed in a different mouse transduced with the same pool of sgRNAs. A clonal population was detected at 4 weeks post-transplant, and the mouse was euthanized at 6.5 weeks post-transplant with mild leukocytosis (white blood cell (WBC) count: $15.3 \times 10^6$/ml; normal: $5\text{-}10 \times 10^6$/ml) and splenomegaly (436 mg; normal: 80-120mg). The cells were skewed to the myeloid lineage (Grl+/CDllb+) (Figure 77A left panel) with 85% of cells expressing the sgRNA:Cas9 vectors (Figure 77A right panel). Analysis of the targeted genes revealed deletions in *Dnmt3a, Ezh2, Smc3* and *Nf1* (Figure 86). Leukemic cells showed enhanced re-plating capacity and analysis of methylcellulose colonies from the leukemia confirmed that all mutations occurred within a single clone (Figure 87, Example 37 Table 2). The peripheral blood from these mice revealed increased granulocytes and circulating blasts, indicative of leukemic transformation (Figure 77B, left panel). Bone marrow histology revealed greater than 20% blast cells and suppression of normal differentiation (Figure 77B, right panel, Figure 88A, Figure 92), consistent with acute myeloid leukemia (AML).

Applicants next confirmed that the detected mutations had an impact on protein expression and cellular phenotype. Both Ezh2 and Smc3 protein expression were markedly reduced, consistent with the bi-allelic mutations detected in both genes. Nf1 is a negative regulator of the Ras pathway, and Applicants observed de-repression of the downstream targets, Erkl and Erk2, as expected for cells with inactivation of Nf1 (Figure 77C). Serial re-plating of FACS-purified, double positive eGFP/RFP657-expressing cells indicates increased self-renewal capacity compared to wild-type C57B1/6 BM cells (Figure 87). Following transplantation into sub-lethally irradiated secondary recipient mice Applicants observed robust engraftment of the GFP/RFP657 double positive mutated cells (Figure 88B). At 8 weeks post-transplant two out of five recipient mice developed leukocytosis and succumbed to leukemia at 11-12 weeks post transplant (Figure 88C/D). Mutated cells in all mice showed a strong differentiation bias, with >90% of the cells belonging to the myeloid lineage (Figure 77D and E). Bone marrow pathology confirmed AML in the secondary recipients (Figure 77F, Figure 88E).

At 14 weeks following transplantation with the pool of tumor suppressor-targeting sgRNA pooled lentivirus, another primary mouse succumbed to a distinct myeloid leukemia characterized by splenomegaly (412mg), leukocytosis (WBC: $33.6 \times 10^6$/ml), excess myeloid cells in the BM (Figure 89A), circulating blasts in peripheral blood, and diagnostic features of AML in the bone marrow (>20% blasts) (Figure 89B). Clonal mutations were identified in *Tet2, Dnmt3a, Runx1, Nf1,* and *Ezh2.* None of the mice transplanted with non-targeting sgRNA expressing cells developed disease during the course of the experiment (Figure 90). These studies demonstrate that by using lentiviral delivered sgRNA:Cas9 components, Applicants have engineered novel models of myeloid malignancy bearing mutations in 4-5 different genes that can be propagated and studied through serial transplantation.

To examine an alternative set of co-operative mutations, the same pool of sgRNAs targeting leukemia tumor suppressor genes was used to transduce LSK cells from mice with heterozygous knock-in of the F1t3-ITD oncogene allele. Targeting of *Runx1* alone did not induce transformation of *Flt3-ITD* heterozygous expressing cells (Figure 75D). Consistent with the results of pooled tumor suppressor gene targeting in wild-type C57B1/6 mice, expansion of sgRNA:Cas9-transduced, Flt3-ITD-expressing cells was observed over time. In some mice, expression of the sgRNA:Cas9 vector was GFP-dim at 8-10 weeks (Figure 91). A total of five out of nine (55%) mice developed disease at 10-16 weeks with leukocytosis and splenomegaly (Figure 92). Three of the mice were analyzed further. Bone marrow flow cytometry demonstrated that cells were myeloid biased in each of these mice (Figures 93A, 94A, 95A). Diagnostic features of AML were evident with an excess of myeloblasts (>20%) in the bone marrow and circulating blast cells in the peripheral blood (Figures 93B, 94B, 95B). Clonal mutations were detected in 3-5 genes in the three mice (Figures 93C, 94C, 95C). Since the mutations were engineered in the *Flt3-ITD* heterozygous background, AML was generated from primary cells with mutations in 4 to 6 leukemia driver genes.

As off-target cleavage by Cas9:sgRNA could potentially contribute to leukemogenesis, Applicants examined leukemias Applicants generated for off-target genome editing. (Ref. 14, 16) Applicants performed targeted sequencing of the top five off-target sites for each of the sgRNAs in each of 4 leukemias. None of the off-target sequences had any mutations in any of the leukemias examined, indicating that off-target cleavages are unlikely to be a significant contributor to leukemogenesis in Applicants' studies (Example 37 Tables 3 and 4).

Taken together, these studies of lentivirus-delivered Cas9:sgRNA with multiplex sgRNAs demonstrate highly efficient genome editing in murine HSPCs. Applicants simultaneously inactivated multiple tumor suppressor genes causing outgrowth of a myeloid clone and transformation to acute leukemia. Applicants describe models with up to 5 genes modified, with editing of up to 10 individual alleles, when a pool of sgRNAs targeting 6 genes was introduced in primary HSPCs (Figure 77G). While genetic models have recently been generated using Cas9:sgRNA in whole organisms (Ref. 7-9), Applicants' system enables tissue specific interrogation of gene function in adult organisms while avoiding potential developmental defects. The genetic complexity thus generated is beyond the capacities of current approaches for engineering murine malignancies and will allow rapid generation of novel disease models beyond hematopoietic malignancies. Such models are critical for the functional interrogation of novel disease alleles and for the development and pre-clinical testing of targeted therapies.

The novel leukemia models generated reflect both the number and functional classes of co-occurring mutations that occur during the pathogenesis of human myeloid malignancies.(Ref. 1, 17) All of the clones characterized had inactivating mutations in *Nf1*, a lesion that activates Ras signaling, and the only mutation in the screen that causes activated kinase signaling, a common feature of leukemias and other cancers.(Ref. 17) Although NF1 mutations are not frequent in AML patients, it was chosen as an amendable target for NHEJ mediated gene editing and representative for cytokine activation in AML. *Nf1* inactivation in mice, however, is insufficient to cause AML and induces a long-latency myeloproliferativen disease.(Ref. 18) Applicants also identified mutations in *Ezh2*, a gene recently shown to be involved in the differentiation block in AML (Ref. 19), in all mice that presented with myeloid transformation. Inhibition of myeloid differentiation has also been reported after deletion of *Dnmt3a*(*Ref.* 20), *Tet2*(Ref. 21, 22) and *Runx1*,(Ref. 23) each of which were successfully mutated in Applicants models.

Overall, Applicants studies demonstrate a robust methodology to generate multiple genetic lesions via lentivirus-delivered Cas9:sgRNA in a broad range of cells, including the primary adult stem cells that are the disease-initiating population for leukemia. The use of viral-based Cas9:sgRNA will enable the rapid and efficient generation of cancer models that reflect the complex genetics of human disease.

Methods and Data

For evaluation of sgRNA-Cas9 efficacy Applicants developed a modular reporter system based on disruption of the reading frame of a fluorescent protein (RFP657) (Figure 75A). Reporter fluorescence is lost upon out-of-frame deletion/insertion after NHEJ thus not detecting all cleavage events, however, for studies based on NHEJ mediated gene disruption these out of frame deletions/insertions are relevant and the reporter therefore provides a precise measurement of gene disruption events.

Off note, similar approaches using disruption of a fluorescent protein coding sequence have been used before for evaluation of genome editing or RNA interference tools and allow more rapid and quantitative testing of cleavage efficiency than standard T7-endonuclease assays.(Ref. 24) In contrast, others have used homologous recombination to evaluate the cleavage efficacy of CRISPR-Cas9 or sgRNA-Cas9 systems, which is also limited in detection of cleavage efficacy, in particular when transfection rates are not provided.(Ref. 25)

In Applicants' approach to model myeloid malignancy, Applicants used this reporter assay to establish high efficacy sgRNAs for 8 genes that are recurrently mutated in myeloid malignancies (Tet2, Runx1, Dnmt3a, Ezh2, Nf1, Smc3, p53 and Asxll). The chosen genes have been described as leukemia promoting loss-of-function mutations and have been found co-mutated in various combinations.

Off note, Wang and colleagues used an efficient Tet2 target site to generate sgRNA:Cas9 based knock-out mice, which was located in the same exon as the one used in Applicants' study.(Ref. 26) As its efficacy was proven, future studies may use sites described here or by Wang and colleagues.

Applicants tested an average of 3 spacers per gene and found that about 60% of the spacers could reduce the Mean Fluorescence Intensity (MFI) of the reporter by more than 20%. About 30% of the sgRNAs reduced the MFI by more than 50%, thereby exceeding formerly published cleavage efficacies of TALENs (Figure 75C). Comparison of Applicants' results for Cas9 cleavage efficacy is hampered by differences in applied methods. Former studies mostly employed transient transfection with limited information about transfection efficacies. The use of lentiviral delivery may increase the efficacy of sgRNA:Cas9 genome editing due to prolonged expression. At the transduction rates achieved in Applicants' study (<30%) one can assume to be in a 1-2 vector copy number per cell. The employed EFS promoter delivers relatively low expression of the Cas9-eGFP cassette. This is in contrast to studies using transfection (which results in a high copy number per cell) and strong viral promoters.

Variance of efficacy between spacers as seen in Applicants' study has been observed before and may be attributed to recently published criteria on optimal spacer design.(Ref. 27,28)

Beside variances detected, efficient cleavage for all pre-tested spacers was detected in-vivo (Figure 83,85,86,89,93,94,95). Analysis of mutations from mouse samples showed that NHEJ in murine hematopoietic cells primarily causes deletions. Deletion size was variable ranging from 1 bp to 251 bp. The majority of mutations were between 1-40 bp. The cell type used and its particular DNA repair preferences may however influence the spectrum of mutations that occur.

Larger deletions than the ones detected may occur but detection is limited with PCR based approaches.

Subsequent analysis of cleavage at off-target sites did not detect mutations at these sites in line with former publications showing abrogation of Cas9 cleavage when spacers have three or more mismatches to the genomic site.

One concern when using integrating viral vectors is the accidental activation of nearby oncogens, which may cause leukemia or contribute to leukemia development (Ref. 29) In Applicants' study a self-inactivating lentiviral vector with a eukaryotic EFS promoter was used. Both the use of self-inactivating vectors with internal promoters and the use of physiological promoters strongly reduces the risk of insertional mutagenesis. (Ref. 30) It is therefore unlikely that vector integration has contributed to leukemia development in Applicants' study.

Materials & Methods

Vector construction and viral particle production

Lentiviral vectors were based on the pLKO_TRC005 lentiviral backbone. The codon optimized Streptococcus pyogenes Cas9 (SpCas9) cDNA and small guide RNA (sgRNA) have been described before.(Ref. 28) The sgRNA was amplified from the pLX330 backbone and placed behind the human U6 promoter of the pLKO_TRC005. Vectors expressing only Cas9 and no sgRNA were generated by deleting the hU6 promoter. BbsI sites were replaced by BsmBI sites for insertion of the spacer sequence. The hPGK promoter of the pLKO_TRC005 was replaced by a minimal size hEF1a promoter (EFS).(Ref. 32) To generate vectors expressing Cas9 only, the hU6 promoter was deleted from the pLKO_TRC005 backbone by cutting PpuMI/EcoRI followed by Klenow fragment fill-in reaction and ligation. SpCas9 cDNA was PCR amplified (Phusion DNA Polymerase, NEB) and placed behind the EFS promoter followed by addition of P2A-eGFP, P2A-tagRFP, P2A-PAC or P2A-BSD. Optimized picorna virus 2A sites have been described before (Ref. 33) and were fused to respective cDNAs via PCR The sgRNA-only vectors were generated by replacing the SpCas9 and respective marker gene with a fluorescent protein marker, or selection marker only. The RFP657 fluorescent protein was described before.(Ref. 34)

Suitable protospacer sequences were identified as described before with unique 13bp seed sequence and a minimum of 3 mismatches to the mouse genome.(Ref. 23)

Lentiviral reporter vectors were generated by replacing the eGFP fluorescent reporter gene in the RRL.PPT.SFFV.IRES.eGFP.pre* (kindly provided by Christopher Baum and Axel Schambach, Hannover Medical School, Hannover, Germany) with PAC, followed by insertion of the RFP657 fluorescent reporter gene 5' of the IRES. CRISPR/Cas target sites were introduced behind the start codon of the RFP657.

Lentiviral particles were produced by transient transfection of 293T cells using the calcium-phosphate transfection method. Viral constructs were co-transfected with pMD2.G (Addgene plasmid 12259) and psPAX2 (Addgene plasmid 12260) (both kindly provided by Didier Trono., EPFL, Lausanne, Switzerland). Lentiviral particles were concentrated using ultracentrifugation.

Oligonucleotide sequences used for the cloning of CRISPR/Cas protospacers used in this study are shown in Example 37 Table 1.

Reporter based efficacy assessment of CRISPR/Cas spacer sequences

Fluorescent reporter vectors were constructed and produced as described above. HEL cells (ATCC) were cultured in RPMI with 10% FBS and 100 U/ml Penicillin/Streptomycin. HEL cells were transduced with CRISPR/Cas reporter vectors in the presence of 4ug/ml hexadimethrine bromide (Polybrene (Life Technologies)) and transduced cells were selected with 2ug/ml Puromycin (Life Technologies) for 72h, starting 48h post transduction. Selection was verified by flow cytometry. Selected reporter cell lines were transduced with CRISPR/Cas vectors targeting the respective target sites and non-targeting controls. RFP657 reporter fluorescence was assessed by flow cytometry at 6 days post transduction in comparison to non-targeting controls and untransduced cells.

T7-endonuclease assay

Ba/F3 cells (DSMZ) were cultured in RPMI with 10% FBS, 100 U/ml Penicillin/Streptomycin and 5 ng/ml 113 (Peprotech). Ba/F3 cells were transduced with CRISPR/Cas vectors, and transduced cells were purified by FACS 7 days post-infection. Genomic DNA was isolated according to manufacturers instruction with a DNA Micro Kit (Quiagen). Targeted loci were amplified by PCR (Phusion DNA Polymerase, NEB). CRISPR/Cas efficacy at the endogenous locus was assessed using the Surveyor Mutation Detection Kit (Transgenomic).

Mouse bone marrow transplant and analysis

Total BM was isolated from femurs and tibias from 6-10 week old female C57B1/6 mice or heterozygous 6-12 week old Flt3-ITD knock-in mice.35 BM was subjected to erythrocyte lysis (BD PharmLyse, BD Bioscience), followed by magnetic bead selection of cKit (CD117) positive cells using CD117 MicroBeads (Milteny Biotech). CD 117 selected cells were stained with APC labeled cKit (eBioscience), PE labeled Sca1 (BioLegend) PacificBlue labeled Gr1, CD11b, B220(CD45R), Ter119, and CD3 (all BioLegend) and then flow sorted on a BD FACS Aria II. LSK cells were cultured in StemSpan SFEM (StemCell Technologies) supplemented with 50ng/ml murine Thpo and 50ng murine Scf (both Peprotech) for 48h and then transduced with concentrated lentiviral supernatant in presence of 2ug/ml Polybrene. 24h post transduction cells were collected and intravenously injected into lethally irradiated (950 cGy) 6-10 week old female C57B1/6 mice. C57B1/6 mice were obtained from Taconic. Flt3-ITD mice were bred in local facilities.

All transplant experiments were performed with 4-5 mice per group. Statistical significance between groups was assessed using an unpaired t-test.

No blinding and no randomization were carried out.

Experiments and procedures were performed in the Children's Hospital Boston animal facility and were approved by the Children's Hospital Boston Institutional Animal Care and Use Committee.

Peripheral blood was sampled by retro-orbital bleeding. Bone marrow was harvested from femurs and tibias. Nucleated cells were obtained by erythrocyte lysis.

To detect mature lineage markers, cells were stained with Grl-APC-eFluor780 (eBioscience), CD11b-APC (BioLegend), B220(CD45R)-PE-Cy7 (BioLegend) and CD3e-PE (eBioscience). HSPCs were stained with PacificBlue labeled Grl, CD11b, B220(CD45R), Ter119, and CD3 (all BioLegend), Scal-PE (BioLegend), CD150-PE-Cy7 (BioLegend), cKit-APC (eBioscience), CD48-PerCP-Cy5.5 (eBioscience), and CD34-AlexaFluor700 (eBioscience). Flow cytometry was performed on a BD LSR II flow cytometer. Cells expressing both the eGFP and RFP657 reporter gene were stained with Grl-PE-Cy7 (eBioscience) and CDllb-PE (BioLegend) or B220(CD45R)-PE-Cy7 and CD3e-PE for mature lineage detection.

To assess mutations at targeted loci, peripheral blood nucleated cells were purified by FACS. Genomic DNA was isolated according to manufacturers instruction with a DNA Micro Kit (Quiagen). Targeted loci were amplified by PCR (Phusion DNA Polymerase, NEB). PCR products were cloned into pJet1.2 PCR cloning vector (Life Technologies). Plasmid DNA was isolated from individual bacterial clones with a DNA Plasmid Mini Kit (Quiagen) and sequences were assessed by Sanger sequencing.

Secondary transplant of leukemic mice was performed by transplanting 5x10^6 primary BM cells into sub-lethally (650cGy) irradiated C57B1/6 mice.

Peripheral blood semars were stained with May-Gruenwald/Giemsa stain. BM histology was assessed from paraffin embedded tissue sections stained with Hematoxilin and Eosin.

Clonogenic colony assays were performed in cytokine-supplemented methylcellulose (M3434; StemCell Technologies). 25,000 C57B1/6 BM wildtype BM cells or 25,000 cells from leukemic mice were plated. Colony numbers were assessed 7 days post seeding. For re-plating experiments, cells were washed with PBS, counted, and plated as described above.

Immunoblots

Nucleated C57B1/6 wildtype BM cells or leukemic BM cells were lysed with RIPA buffer (Pierce) supplemented with Halt Protease Inhibitor (Pierce). Protein concentrations were determined using Bradford protein assay (Bio-Rad).

Equal protein amounts were separated by SDS-PAGE and blotted on nitrocellulose membrane, blocked with fat free milk and incubated with primary antibodies (Cell Signaling; Ezh2 clone D2C9; Smc3 clone D47B5; phospho-Erk1/2 (phospho-p44/42) clone D13.14.4E; Erkl/2 (p44/42) clone 137F5). Mouse beta actin was obtained from Santa Cruz (clone C4). After washing blots were incubated with HRP-conjugated secondary antibody and developed using Super-Signal West Pico Chemiluminescent Substrate (Pierce).

Statistical analysis

Statistical analysis was performed with GraphPad Prism software (Graphpad Software). Data are shown as the mean with standard error of the mean. Groups were compared using an unpaired, two sided t-test. Normal distribution was assumed. Differences in variance were analyzed using an F-test Welch correction for t-test was applied for unequal variances as indicated.

Next Generation Sequencing to Assess Mutational Repertoire

gDNA was isolated using a DNA Blood Mini kit (Quiagen) according to manufacturers instructions. The genomic region flanking the CRISPR target site for each region was amplified by a nested PCR to attach the Illumina adapters in the first round and to add barcodes unique for each individual mouse in the second PCR All libraries were pooled and purified using Agencourt AMPure XP system (Beckman Coulter). Single end, 300 cycle sequencing was performed using MiSeq (Illumina). Reads were adapter trimmed and aligned using BLAT to reference amplicon sequences. Indels were surveyed using Integrative Genomics Viewer.

Example 37 Table 1 - Target sites and efficacies

| Targeted Gene | Targeted Sequence | Efficacy (Reporter) |
|---|---|---|
| Runx1 | GCACTGGGGGACGTCCCGGATGG | 0.17 |
| Runx1 | CCAGCGACACCCATTTCACCCGG | 0.20 |
| Runx1 | CCGTGCCAGCGGCATGACCAGCC | 0.81 |
| Tet2 | CCGTGCAGAGTACCGCATCTCAT | 0.66 |
| Tet2 | AATACTATCCTAGTTCCGACCGG | 0.10 |
| Tet2 | GAACAAGCTCTACATCCCGTAGG | 0.11 |
| Dnmt3a | GTGGGCATGGTGCGGCACCAGGG | 0.44 |
| Dnmt3a | CACC GCATGATGCGCGGCCCAAGG | 0.50 |
| Dnmt3a | CACC GCTTACCAGTATGACGACGA | 0.86 |
| Nf1 | CCAGTTACCGGGACCGCTCCTTC | 1.03 |

| Nf1 | CCCTCGATGTGGCGGCTCATCTG | 0.55 |
|---|---|---|
| Nf1 | CCTCGATGTGGCGGCTCATCTGC | 0.66 |
| Ezh2 | CTGAGAAGGGACCGGTTTGTTGG | 0.77 |
| Ezh2 | CCGGTTTGTTGGCGGAAGCGTGT | 1.10 |
| Ezh2 | AAGACACCACCTAAACGCCCAGG | 0.61 |
| Smc3 | AAAGCTGATAAAGCGGCAAGAGG | 0.34 |
| Smc3 | CCATCGAGGTGCTCTGACTGGAG | 0.39 |
| Asxl1 | CAACAGTGCCATTCGAGGCCAGG | 0.88 |
| Asxl1 | GATACTAAAACCGACTTAGCAGG | 1.01 |
| Asxl1 | AGCCCAAAGTCCCGCCCATCCGG | 1.07 |
| p53 | AGAAGAAAATTTCCGCAAAAAGG | 1.01 |
| p53 | CCCTGAACTGCCCCCAGGGAGCG | 1.10 |
| p53 | CCACTACAAGTACATGTGTAATA | 1.11 |
| PAM sequence underlined | | |
| bold: target sites used for in-vivo experiments | | |
| Efficacy describes the relative fluorescence compared to control target site | | |

Example 37 Table 2 - Genotyping of methylcellulose colonies

| | | | | Primary genotype | | | | | |
|---|---|---|---|---|---|---|---|---|---|
| | Tet2 | Runx1 | Dnmt3a | Nf1 | Ezh2 | Smc3 | Colonies with primary genotype | Colonies wt | Colonies with divergent genotype |
| Figure3 | wt | wt | 181bp del | 36bp del / 1bp del | 5bp del / 9 bp del | 33bp del | 5/5 | 0/5 | 0/5 |
| Suppl. Figure 15 | wt | 1bp ins | wt | 1bp del / 25 bp del | 53 bp del / wt | wt | 4/5 | 1/5 | 0/5 |

Example 37 Table 3 - Off-target sites of sgRNA target sites used for in-vivo studies

| target | guide sequence | on-target locus | off target name | off target sequence | mismatches | UCSC gene | locus | genic location |
|---|---|---|---|---|---|---|---|---|
| Runx1.1 | GCACT-GGGGGACGT CCCGGATGG | chr16: +92689348 | Runx1_OT1 | GCCCT-GGGGGATGT-GCCGGATGG | 3MMs [3:12:15] | NM_019732 | chr4:+ 134711201 | Runx3 exon |
| | | | Runx1_OT2 | GCCCT-GGGGAC-CGTCCGGGA-GAG | 4MMs [3:10:11: 17] | NM_011801 | chr8: +114377958 | Cfdp1 intron,CpG island |
| | | | Runx1_OT3 | GGACTGGT-GGAGGTTC-CGGATGG | 4MMs [2:8:12:15] | NM_ 001136058 | chr5:-37680166 | Crmp1 exon |
| | | | Runx1_OT4 | GGACGGCG-GGACG-GCCCGGA-GAG | 4MMs [2:5:7:14] | NM_145837 | chr14: +58143823 | ||17d intron, CpG island |
| | | | Runx1_0T5 | GCTCT-GGGGGCCCT CCCGGGGAG | 4MMs [3:11:13: 20] | NM_027711 | chr13: +96661671 | Iqgap2 exon, CpG island |
| Nf1.2 | CAGAT-GAGCCGCCA-CATCGAGGG | chr11:-79360625 | Nf1_OT1 | CCGAG-GAGCCGCAA-CATCGTAAG | 4MMs [2:5:13:20] | NM_177213 | chr7:-127505021 | Abca15 exon |
| | | | Nf1_OT2 | GAGATGAG-CAGCGACAT-CAATGG | 4MMs [1:10:13: 19] | NM_178017 | chr8:-77547985 | Hmgxb4 exon |
| | | | Nf1_OT3 | CAGGT-GAGCTGCAA-CATCTACGG | 4MMs [4:10:13: 19] | NM 001201378 | chr6:+29355866 | Ccdc136 exon |
| | | | Nf1_OT4 | CAGAT-GATCGGGCA-GATCGATGG | 4MMs [8:10:12: 15] | NM_019694 | chr5:-34087729 | Letm1 exon |
| | | | Nf1_OT5 | CAGCT-GGGCCCCCA-CATTGACGG | 4MMs [4:7:11:18] | NM_023633 | chr12:-85293126 | R2410016006Rik exon |

| target | guide se-quence | on-target locus | off target name | off target sequence | mismatches | UCSC gene | locus | genic location |
|---|---|---|---|---|---|---|---|---|
| Tet2.4 | GAACAAGCTC TACATCCCG-TAGG | chr3: +133149224 | Tet2_OT1 | GGACAAGT-TCCACATC-CCGCAAG | 4MMs [2:8:11:20] | NM_146086 | chr18: +61416955 | Pde6a exon |
| | | | Tet2_OT2 | GAAG-CAGCTCTC-CATCCCT-TCGG | 4MMs [4:5:12:19] | NM_145741 | chr14:-34745345 | GdflO exon |
| | | | Tet2_OT3 | GAAGAAGCT-CAG-CATCTCGT-CAG | 4MMs [4:11:12: 17] | NM_028816 | chr7: +133252384 | Xpo6 exon |
| | | | Tet2_OT4 | GAG-GAAGCTCTA-CATCCT-GGAGG | 4MMs [3:4:18:20] | NM_031257 | chr8:-26182382 | Plekha2 intron |
| | | | Tet2_OT5 | TACCAAGCTC-TACATC-CTCTTGG | 4MMs [1:3:18:19] | NM_199252 | chr17:-13302577 | Unc93a exon |

| target | guide se-quence | on-target locus | off target name | off target sequence | mismatches | UCSC gene | locus | genic location |
|---|---|---|---|---|---|---|---|---|
| Smc3.3 | CTCCAGTCA-GAGCACCTC-GATGG | chr19:-53708484 | Smc3_OT1 | CTCAAGTCA-GAGAAC-CTCGCGGG | 3MMs [4:13:20] | NM_198642 | chr14:-75415982 | 5031414D18Rik gene, CpG island |
| | | | Smc3_OT2 | CCCCACACAC AGCACCTC-GACAG | 4MMs [2:6:7:10] | NM_144803 | chr14: +66771517 | Chma2 exon |
| | | | Smc3_OT3 | CTTCAGGCT-GAGCAC-CTCGCAGG | 4MMs [3:7:9:20] | NM-0119D4 | chr19:-41163120 | Tll2 intron, CpG island |
| | | | Smc3_OT4 | CTCCAGAAA-GAACAC-CTCGCTAG | 4MMs [7:8:12:20] | NM_023878 | chrl4:-119260844 | Cldn10 intron |
| | | | Smc3_OT5 | ACCCAGTCT-GAGCACCT-GGAAGG | 4MMs [1:2:9:18] | NM_001080815 | chr7:+19744847 | Gipr intron-exon |
| Dnmt3a.1 | GTGGGCAT-GGTGCG-GCACCAGGG | chr12:+3901625 | Dnmt3_OT1 | GTGAGCCAG-GTGGGGCAC-CACAG | 4MMs [4:7:8:13] | NM_013908 | chr2:+25359855 | Fbxw5 intron |
| | | | Dnmt3_OT2 | GTGGGCAAG-GGGCG-GCAACACAG | 3MMs [8:11:18] | NM-001220499 | chr4:-155506697 | Rnf223 gene |
| | | | Dnmt3_OT3 | ATGGGCAT-GAAGT-GGCACCATGG | 4MMs [1:10:11:13] | NM_178627 | chr15: +82967944 | Poldip3 exon, unbal-anced |
| | | | Dnmt3_OT4 | ATGGACAT-GGGGCG-GCACAAAGG | 4MMs [1:5:11:19] | NM_201407 | chr3:+90072469 | Dennd4b, unbalanced |
| | | | Dnmt3_OT5 | GTGGCCTT-GTTGCGT-CACCATGG | 4MMs [5:7:10:15] | NM-001081302 | chr15:-27781515 | Trio intron |

173

EP 3 011 035 B1

| target | guide se-quence | on-target locus | off target name | off target sequence | mismatches | UCSC gene | locus | genic location |
|---|---|---|---|---|---|---|---|---|
| Ezh2.3 | AAGACAC-CAC-CTAAACGCCCAGG | chr6:+47495847 | Ezh2_OT1 | CAGCTAC-CAC-CCAAACGCCCCAG | 4MMs [1:4:5:12] | NM_172434 | chr3:+94282696 | Celf3 intron |
| | | | Ezh2_OT2 | GTGACACCCCCTAAACTCCCTAG | 4MMs [1:2:9:17] | NM_027627 | chr16:+90831218 | C21orf63 exon |
| | | | Ezh2_OT3 | AACTCAGCACCTAAAAGCCCAAG | 4MMs [3:4:7:16] | NM_133762 | chr12:-117663077 | Ncapg2 intro-exon |
| | | | Ezh2_OT4 | GAGAGACCA-CAT-AAAAGCCCAAG | 4MMs [1:5:11:16] | NM_001081467 | chr5:+134868172 | Gtf2ird1 intron |
| | | | Ezh2_OT5 | AAGACACAGGCTAAAAGCCCGGG | 4MMs [8:9:10:16] | NM_027667 | chr19:+41847484 | Arhgap19 intron |

Example 37 Table 4 - Percentages of mutated sequencereads at off-target sites

| | WT | Figure 3 | Supplementary Figure 12 | Supplementary Figure 15 | Supplementary Figure 16 |
|---|---|---|---|---|---|
| Runx1_OT1 | 0% | 0% | 0% | 0% | 0% |
| Runx1_OT2 | 0% | 0% | 0% | 0% | 0% |
| Runx1_OT3 | 0% | 0% | 0% | 0% | 0% |
| Runx1_OT4 | 0% | 0% | 0% | 0% | 0% |
| Runx1_OT5 | 0% | 0% | 0% | 0% | 0% |
| Nf1_OT1 | 0% | 0% | 0% | 0% | 0% |
| Nf1_OT2 | 0% | 0% | 0% | 0% | 0% |
| Nf1_OT3 | 0% | 0% | 0% | 0% | 0% |
| Nf1_OT4 | 0% | 0% | 0% | 0% | 0% |
| Nf1_OT5 | 0% | 0% | 0% | 0% | 0% |
| Tet2_OT1 | 0% | 0% | 0% | 0% | 0% |
| Tet2_OT2 | 0% | 0% | 0% | 0% | 0% |
| Tet2_OT3 | 0% | 0% | 0% | 0% | 0% |
| Tet2_OT4 | 0% | 0% | 0% | 0% | 0% |
| Tet2_OT5 | 0% | 0% | 0% | 0% | 0% |
| Smc3_OT1 | 0% | 0% | 0% | 0% | 0% |
| Smc3_OT2 | 0% | 0% | 0% | 0% | 0% |
| Smc3_OT3 | 0% | 0% | 0% | 0% | 0% |
| Smc3_OT4 | 0% | 0% | 0% | 0% | 0% |
| Smc3_OT5 | 0% | 0% | 0% | 0% | 0% |
| Dnmt3_OT1 | 0% | 0% | 0% | 0% | 0% |
| Dnmt3_OT2 | 0% | 0% | 0% | 0% | 0% |
| Dnmt3_OT3 | 0% | 0% | 0% | 0% | 0% |
| Dnmt3_OT4 | 0% | 0% | 0% | 0% | 0% |
| Dnmt3_OT5 | 0% | 0% | 0% | 0% | 0% |
| Ezh2_OT1 | 0% | 0% | 0% | 0% | 0% |
| Ezh2_OT2 | 0% | 0% | 0% | 0% | 0% |
| Ezh2_OT3 | 0% | 0% | 0% | 0% | 0% |
| Ezh2_OT4 | 0% | 0% | 0% | 0% | 0% |
| Ezh2_OT5 | 0% | 0% | 0% | 0% | 0% |

Off-target sites (column 1) are listed in Example 37 Table 3. Top row indicates where respective leukemias are presented. A C57B1/6 (WT) control was included.

References Ref 1. Genomic and epigenomic landscapes of adult de novo acute myeloid leukemia. The New England journal of medicine 368, 2059-2074 (2013); Ref 2. Jinek, M. et al. A programmable dual-RNA-guided DNA endonuclease in adaptive bacterial immunity. Science 337, 816-821 (2012). Ref 3. Cong, L. et al. Multiplex genome engineering using CRISPR/Cas systems. Science 339, 819-823 (2013). Ref. 4. Mali, P. et al. RNA-guided human genome engineering via Cas9. Science 339, 823-826 (2013). Ref 5. Bogdanove, A.J. & Voytas, D.F. TAL effectors: customizable proteins for DNA targeting. Science 333, 1843-1846 (2011). Ref 6. Reyon, D. et al. FLASH assembly of TALENs for high-throughput genome editing. Nature biotechnology 30, 460-465 (2012). Ref 7. Hwang, W.Y. et al. Efficient genome editing in zebrafish using a CRISPR-Cas system. Nature biotechnology 31, 227-229 (2013). Ref 8. Wang, H. et al. One-step generation of mice carrying mutations in multiple genes by CRISPR/Cas-mediated genome engineering. Cell 153, 910-918 (2013). Ref 9. Friedland, A.E. et al. Heritable genome editing in C. elegans via a CRISPR-Cas9 system. Nature methods 10, 741-743 (2013). Ref 10. Shan, Q. et al. Targeted genome modification of crop plants using a CRISPR-Cas system. Nature biotechnology 31, 686-688 (2013). Ref 11. Wood, A.J. et al. Targeted genome editing across species using ZFNs and TALENS. Science 333, 307 (2011). Ref. 12. Sander, J.D. et al. Targeted gene disruption in somatic zebrafish cells using engineered TALENS. Nature biotechnology 29, 697-698 (2011). Ref. 13. Holkers, M. et al. Differential integrity of TALE nuclease genes following adenoviral and lentiviral vector gene transfer into human cells. Nucleic acids research 41, e63 (2013). Ref. 14. Hsu, P.D. et al. DNA targeting specificity of RNA-guided Cas9 nucleases. Nature biotechnology 31, 827-832 (2013). Ref. 15. Lee, B.H. et al. FLT3 mutations confer enhanced proliferation and survival properties to multipotent progenitors in a murine model of chronic myelomonocytic leukemia. Cancer cell 12, 367-380 (2007). Ref. 16.Fu, Y. et al. High-frequency off-target mutagenesis induced by CRISPR-Cas nucleases in human cells. Nature biotechnology 31, 822-826 (2013). Ref. 17. Kelly, L.M. & Gilliland, D.G. Genetics of myeloid leukemias. Annual review of genomics and human genetics 3, 179-198 (2002). Ref. 18. Zhang, Y., Taylor, B.R., Shannon, K. & Clapp, D.W. Quantitative effects of Nfl inactivation on in vivo hematopoiesis. The Journal of clinical investigation 108, 709-715 (2001). Ref. 19. Tanaka, S. et al. Ezh2 augments leukemogenicity by reinforcing differentiation blockage in acute myeloid leukemia. Blood 120, 1107-1117 (2012). Ref. 20. Challen, G.A. et al. Dnmt3a is essential for hematopoietic stem cell differentiation. Nature genetics 44, 23-31 (2012). Ref. 21. Moran-Crusio, K. et al. Tet2 loss leads to increased hematopoietic stem cell self-renewal and myeloid transformation. Cancer cell 20, 11-24 (2011). Ref. 22. Quivoron, C. et al. TET2 inactivation results in pleiotropic hematopoietic abnormalities in mouse and is a recurrent event during human lymphomagenesis. Cancer cell 20, 25-38 (2011). Ref. 23. Mead, A.J. et al. FLT3-ITDs instruct a myeloid differentiation and transformation bias in lymphomyeloid multipotent progenitors. Cell reports 3, 1766-1776 (2013). Ref 24. Reyon, D. et al. FLASH assembly of TALENs for high-throughput genome editing. Nature biotechnology 30, 460-465 (2012); Ref 25. Mali, P. et al. RNA-guided human genome engineering via Cas9. Science 339, 823-826 (2013); Ref 26. Wang, H. et al. One-step generation of mice carrying mutations in multiple genes by CRISPR/Cas-mediated genome engineering. Cell 153, 910-918 (2013); Ref 27. Wang, T., Wei, J.J., Sabatini, D.M. & Lander, E.S. Genetic screens in human cells using the CRISPR-Cas9 system. Science 343, 80-84 (2014); Ref 28. Cong, L. et al. Multiplex genome engineering using CRISPR/Cas systems. Science 339, 819-823 (2013); Ref 29. Li, Z. et al. Murine leukemia induced by retroviral gene marking. Science 296, 497 (2002); Ref 30. Zychlinski, D. et al. Physiological promoters reduce the genotoxic risk of integrating gene vectors. Molecular therapy : the journal of the American Society of Gene Therapy 16, 718-725 (2008); Ref 31. Hsu, P.D. et al. DNA targeting specificity of RNA-guided Cas9 nucleases. Nature biotechnology 31, 827-832 (2013); Ref 32. Schambach, A. et al. Equal potency of gammaretroviral and lentiviral SIN vectors for expression of 06-methylguanine-DNA methyltransferase in hematopoietic cells. Molecular therapy : the journal of the American Society of Gene Therapy 13, 391-400 (2006); Ref 33. Kim, J.H. et al. High cleavage efficiency of a 2A peptide derived from porcine teschovirus-1 in human cell lines, zebrafish and mice. PloS one 6, e18556 (2011); Ref 34. Morozova, K.S. et al. Far-red fluorescent protein excitable with red lasers for flow cytometry and superresolution STED nanoscopy. Biophysical journal 99, L13-15 (2010); Ref 35. Lee, B.H. et al. FLT3 mutations confer enhanced proliferation and survival properties to multipotent progenitors in a murine model of chronic myelomonocytic leukemia. Cancer cell 12, 367-380 (2007).

**References**

1. Ding, Q. et al. A TALEN genome-editing system for generating human stem cell-based disease models. Cell Stem Cell 12, 238-251 (2013).

2. Soldner, F. et al. Generation of isogenic pluripotent stem cells differing exclusively at two early onset Parkinson point mutations. Cell 146, 318-331 (2011).

3. Carlson, D.F. et al. Efficient TALEN-mediated gene knockout in livestock. Proc Natl Acad Sci U S A 109, 17382-17387 (2012).

4. Geurts, A.M. et al. Knockout Rats via Embryo Microinjection of Zinc-Finger Nucleases. Science 325,433-433 (2009).

5. Takasu, Y. et al. Targeted mutagenesis in the silkworm Bombyx mori using zinc finger nuclease mRNA injection. Insect Biochem Molec 40, 759-765 (2010).

6. Watanabe, T. et al. Non-transgenic genome modifications in a hemimetabolous insect using zinc-finger and TAL effector nucleases. Nat Commun 3 (2012).

7. Porteus, M.H. & Baltimore, D. Chimeric nucleases stimulate gene targeting in human cells. Science 300, 763 (2003).

8. Miller, J.C. et al. An improved zinc-finger nuclease architecture for highly specific genome editing. Nat Biotechnol 25, 778-785 (2007).

9. Sander, J.D. et al. Selection-free zinc-finger-nuclease engineering by context-dependent assembly (CoDA). Nat Methods 8, 67-69 (2011).

10. Wood, A.J. et al. Targeted genome editing across species using ZFNs and TALENs. Science 333, 307 (2011).

11. Christian, M. et al. Targeting DNA double-strand breaks with TAL effector nucleases. Genetics 186, 757-761 (2010).

12. Zhang, F. et al. Efficient construction of sequence-specific TAL effectors for modulating mammalian transcription. Nat Biotechnol 29, 149-153 (2011).

13. Miller, J.C. et al. A TALE nuclease architecture for efficient genome editing. Nat Biotechnol 29, 143-148 (2011).

14. Reyon, D. et al. FLASH assembly of TALENs for high-throughput genome editing. Nat Biotechnol 30, 460-465 (2012).

15. Boch, J. et al. Breaking the code of DNA binding specificity of TAL-type III effectors. Science 326, 1509-1512 (2009).

16. Moscou, M.J. & Bogdanove, A.J. A simple cipher governs DNA recognition by TAL effectors. Science 326, 1501 (2009).

17. Sanjana, N.E. et al. A transcription activator-like effector toolbox for genome engineering. Nat Protoc 7, 171-192 (2012).

18. Deveau, H., Garneau, J.E. & Moineau, S. CRISPR-Cas system and its role in phage-bacteria interactions. Annu Rev Microbiol 64, 475-493 (2010).

19. Horvath, P. & Barrangou, R. CRISPR-Cas, the immune system of bacteria and archaea. Science 327, 167-170 (2010).

20. Makarova, K.S. et al. Evolution and classification of the CRISPR-Cas systems. Nat Rev Microbiol 9, 467-477 (2011).

21. Bhaya, D., Davison, M. & Barrangou, R. CRISPR-Cas systems in bacteria and archaea: versatile small RNAs for adaptive defense and regulation. Annu Rev Genet 45, 273-297 (2011).

22. Cong, L. et al. Multiplex genome engineering using CRISPR-Cas systems. Science 339, 819-823 (2013).

23. Mali, P. et al. RNA-guided human genome engineering via Cas9. Science 339, 823-826 (2013).

24. Jinek, M. et al. RNA-programmed genome editing in human cells. eLife 2, e00471 (2013).

25. Cho, S.W., Kim, S., Kim, J.M. & Kim, J.S. Targeted genome engineering in human cells with the Cas9 RNA-guided endonuclease. Nat Biotechnol 31, 230-232 (2013).

26. Garneau, J.E. et al. The CRISPR-Cas bacterial immune system cleaves bacteriophage and plasmid DNA. Nature 468, 67-71 (2010).

27. Jinek, M. et al. A programmable dual-RNA-guided DNA endonuclease in adaptive bacterial immunity. Science 337, 816-821 (2012).

28. Gasiunas, G., Barrangou, R., Horvath, P. & Siksnys, V. Cas9-crRNA ribonucleoprotein complex mediates specific DNA cleavage for adaptive immunity in bacteria. Proc Natl Acad Sci U S A 109, E2579-2586 (2012).

29. Urnov, F.D., Rebar, E.J., Holmes, M.C., Zhang, H.S. & Gregory, P.D. Genome editing with engineered zinc finger nucleases. Nat Rev Genet 11, 636-646 (2010).

30. Hsu, P.D. & Zhang, F. Dissecting neural function using targeted genome engineering technologies. ACS Chem Neurosci 3, 603-610 (2012).

31. Perez, E.E. et al. Establishment of HIV-1 resistance in CD4(+) T cells by genome editing using zinc-finger nucleases. Nat Biotechnol 26, 808-816 (2008).

32. Cong, L. et al. Multiplex Genome Engineering Using CRISPR-Cas Systems. Science 339, 819-823 (2013).

33. Chen, F.Q. et al. High-frequency genome editing using ssDNA oligonucleotides with zinc-finger nucleases. Nat Methods 8, 753-U796 (2011).

34. Bedell, V.M. et al. In vivo genome editing using a high-efficiency TALEN system. Nature 491, 114-U133 (2012).

35. Saleh-Gohari, N. & Helleday, T. Conservative homologous recombination preferentially repairs DNA double-strand breaks in the S phase of the cell cycle in human cells. Nucleic Acids Res 32, 3683-3688 (2004).

36. Deltcheva, E. et al. CRISPR RNA maturation by trans-encoded small RNA and host factor RNase III. Nature 471, 602-607 (2011).

37. Sapranauskas, R. et al. The Streptococcus thermophilus CRISPR-Cas system provides immunity in Escherichia coli. Nucleic Acids Res 39, 9275-9282 (2011).

38. Hwang, W.Y. et al. Efficient genome editing in zebrafish using a CRISPR-Cas system. Nat Biotechnol 31, 227-229 (2013).

39. Wang, H. et al. One-Step Generation of Mice Carrying Mutations in Multiple Genes by CRISPR-Cas-Mediated Genome Engineering. Cell 153, 910-918 (2013).

40. Shen, B. et al. Generation of gene-modified mice via Cas9/RNA-mediated gene targeting. Cell Res 23, 720-723 (2013).

41. Qi, L.S. et al. Repurposing CRISPR as an RNA-guided platform for sequence-specific control of gene expression. Cell 152, 1173-1183 (2013).

42. Tuschl, T. Expanding small RNA interference. Nat Biotechnol 20, 446-448 (2002).

43. Smithies, O., Gregg, R.G., Boggs, S.S., Koralewski, M.A. & Kucherlapati, R.S. Insertion of DNA sequences into the human chromosomal beta-globin locus by homologous recombination. Nature 317, 230-234 (1985).

44. Thomas, K.R., Folger, K.R. & Capecchi, M.R. High frequency targeting of genes to specific sites in the mammalian genome. Cell 44, 419-428 (1986).

45. Hasty, P., Rivera-Perez, J. & Bradley, A. The length of homology required for gene targeting in embryonic stem cells. Mol Cell Biol 11, 5586-5591 (1991).

46. Wu, S., Ying, G.X., Wu, Q. & Capecchi, M.R. A protocol for constructing gene targeting vectors: generating knockout mice for the cadherin family and beyond. Nat Protoc 3, 1056-1076 (2008).

47. Guschin, D.Y. et al. A rapid and general assay for monitoring endogenous gene modification. Methods Mol Biol 649, 247-256 (2010).

48. Oliveira, T.Y. et al. Translocation capture sequencing: a method for high throughput mapping of chromosomal rearrangements. J Immunol Methods 375, 176-181 (2012).

49. Deltcheva, E. et al. CRISPR RNA maturation by trans-encoded small RNA and host factor RNase III. Nature 471, 602-607 (2011).

50. Bogenhagen, D.F. & Brown, D.D. Nucleotide sequences in Xenopus 5S DNA required for transcription termination. Cell 24, 261-270 (1981).

51. Bultmann, S. et al. Targeted transcriptional activation of silent oct4 pluripotency gene by combining designer TALEs and inhibition of epigenetic modifiers. Nucleic Acids Res 40, 5368-5377 (2012).

52. Valton, J. et al. Overcoming transcription activator-like effector (TALE) DNA binding domain sensitivity to cytosine methylation. J Biol Chem 287, 38427-38432 (2012).

53. Christian, M. et al. Targeting DNA double-strand breaks with TAL effector nucleases. Genetics 186, 757-761 (2010).

54. Mussolino, C. et al. A novel TALE nuclease scaffold enables high genome editing activity in combination with low toxicity. Nucleic acids research 39, 9283-9293 (2011).

55. Bobis-Wozowicz, S., Osiak, A., Rahman, S.H. & Cathomen, T. Targeted genome editing in pluripotent stem cells using zinc-finger nucleases. Methods 53, 339-346 (2011).

56. Jiang, W., Bikard, D., Cox, D., Zhang, F. & Marraffini, L.A. RNA-guided editing of bacterial genomes using CRISPR-Cas systems. Nat Biotechnol 31, 233-239 (2013).

57. Michaelis, L.M., Maud "Die kinetik der invertinwirkung.". Biochem. z (1913).

58. Mahfouz, M.M. et al. De novo-engineered transcription activator-like effector (TALE) hybrid nuclease with novel DNA binding specificity creates double-strand breaks. Proc Natl Acad Sci U S A 108, 2623-2628 (2011).

59. Wilson, E.B. Probable inference, the law of succession, and statistical inference. J Am Stat Assoc 22, 209-212 (1927).

60. Tangri S, et al., Rationally engineered therapeutic proteins with reduced immunogenicity, J Immunol. 2005 Mar 15;174(6):3187-96.

61. REMINGTON'S PHARMACEUTICAL SCIENCES (Mack Pub. Co., N.J. 1991)

62. Lopes, V.S., etc al., Retinal gene therapy with a large MYO7A cDNA using adeno-assocaited virus. Gene Ther, 2013 Jan 24. doi: 10.1038/gt 2013.3.[Epub ahead of print]

63. Kaplitt, M.G., et al., Safety and tolerability of gene therapy with an adeno-associated virus (AAV) borne GAD gene for Parkinson's disease: an open label, phase I trial. Lancet. 2007 Jun 23;369(9579):2097-105.

64. Nathwani, A.C., et al., Adenovirus-associated virus vector-mediated gene transfer in hemophilia B. N Engl J Med. 2011 Dec 22;365(25):2357-65. doi: 10.1056/NEJMoal108046. Epub 2011 Dec 10.

65. Gray SJ, Foti SB, Schwartz JW, Bachaboina L, Taylor-Blake B, Coleman J, Ehlers MD, Zylka MJ, McCown TJ, Samulski RJ. Optimizing promoters for recombinant adeno-associated virus-mediated gene expression in the peripheral and central nervous system using self-complementary vectors. Hum Gene Ther. 2011 Sep;22(9):1143-53. doi: 10.1089/hum.2010.245.

66. Liu D, Fischer I. Two alternative promoters direct neuron-specific expression of the rat microtubule-associated protein 1B gene. J Neurosci. 1996 Aug 15;16(16):5026-36.

67. Levitt N. Briggs D. Gil A. Proudfoot N.J. Definition of an efficient synthetic poly(A) site. Genes Dev. 1989;3:1019-1025.

68. McClure C, Cole KL, Wulff P, Klugmann M, Murray AJ. Production and titering of recombinant adeno-associated viral vectors. J Vis Exp. 2011 Nov 27;(57):e3348. doi: 10.3791/3348.

69. Banker G, Goslin K. Developments in neuronal cell culture. Nature. 1988 Nov 10;336(6195):185-6.

## Claims

1. An assay for quantitative evaluation of target site cleavage by one or more CRISPR guide sequences, the assay comprising:

   introducing up to 20 targets for one or more CRISPR guide sequences into the coding sequence of a fluorescent protein;
   generating a reporter cell line comprising and expressing a lentiviral reporter system comprising said fluorescent protein;
   transducing the reporter cell line with CRISPR-Cas vectors targeting said up to 20 targets; and
   quantifying loss of reporter fluorescence to determine the efficiency with which the one or more guides directed cleavage at said target site in the coding sequence for the fluorescent protein.

**Patentansprüche**

1. Test zur quantitativen Evaluierung der Zielstellenspaltung durch eine oder mehrere CRISPR Führungssequenzen, wobei der Test umfasst:

Einführen von bis zu 20 Zielen für eine oder mehrere CRISPR Führungssequenzen in die Codiersequenz eines fluoreszierenden Proteins;
Generieren einer Reporterzelllinie, die ein lentivirales Reportersystem umfasst und exprimiert, welches das fluoreszierende Protein umfasst;
Transduzieren der Reporterzelllinie mit CRISPR-Cas Vektoren, die auf die bis zu 20 Ziele abzielen; und
Quantifizieren des Verlusts der Reporterfluoreszenz, um die Effizienz zu bestimmen, mit der die eine oder mehreren Führungen die Spaltung an der Zielstelle in der Codiersequenz für das fluoreszierende Protein geführt haben.

**Revendications**

1. Dosage pour l'évaluation quantitative d'un clivage de site cible par une ou plusieurs séquences de guidage de CRISPR, le dosage comprenant :

l'introduction de jusqu'à 20 cibles pour une ou plusieurs séquences de guidage de CRISPR dans la séquence codante d'une protéine fluorescente ;
la génération d'une lignée cellulaire rapporteur comprenant et exprimant un système rapporteur lentiviral comprenant ladite protéine fluorescente ;
la transduction de la lignée cellulaire rapporteur avec des vecteurs CRISPR-Cas ciblant lesdites jusqu'à 20 cibles ; et
la quantification de la perte de fluorescence du rapporteur pour déterminer l'efficacité avec laquelle les un ou plusieurs guides ont dirigé un clivage au niveau dudit site cible dans la séquence codante pour la protéine fluorescente.

FIG. 1

FIG. 2A

FIG. 2B

C

FIG. 2C

D

|  | | | | | |
|---|---|---|---|---|---|
| 2xNLS-SpCas9 | + | + | + | + | + |
| SpRNAse III | − | + | + | − | + |
| short tracrRNA | − | + | − | + | + |
| DR-EMX1-DR | + | − | + | + | + |

684bp ►

367bp ►
317bp ►

indel (%):  4.7  5.0

FIG. 2D

E

Target locus  5'-..AGCTGGAGGAGGAAGGGCCTGAGTCCGAGCAGAAGAAGAAGGGCTCCCAC..-3'
              |||||||||||||||||||||                        |||||||||||
              3'-..TCGACCTCCTCCTTCCCGGACTCAGGCTCGTCTTCTTCTTCCCGAGGGTG..-5'
                                     |||||||||||||||||||||||||||
crRNA                         5'-  GAGUCCGAGCAGAAGAAGAAGUUUUAGAGC...-3'

indel          AGCTGGAGGAGGAAGGGCCTGAGTCCGAGCAGAAGA--GAAGGGCTCCCAT

F

human *EMX1* protospacer target (mutation in 5 of 43 sequenced clones = 11.6%)

| | |
|---|---|
| WT | 5'-..CTGGAGGAGGAAGGGCCTGAGTCCGAGCAGAAG AAGAAGGGCTCCCATCACAT..-3' |
| Δ1 | CTGGAGGAGGAAGGGCCTGAGTCCGAGCAGAAG---AGAAGGGCTCCCATCACAT |
| +1 | CTGGAGGAGGAAGGGCCTGAGTCCGAGCAGAAGAAAGAAGGGCTCCCATCACAT |
| Δ3 | CTGGAGGAGGAAGGGCCTGAGTCCGAGCAGAAG-----AAGGGCTCCCATCACAT |
| m1, Δ6 | CTGGAGGAGGAAGGGCCTGAGCCCGAGCAGAAG--------GGCTCCCATCACAT |

FIG. 2E-F

**A**

**B**

**C**

FIG. 3A-C

FIG. 3D

FIG. 4A-B

**C**

human *EMX1* locus

HR Template

*Hind*III, *Nhe*I

200bp

**D**

| | | | |
|---|---|---|---|
| hSpCas9 | + | − | − |
| hSpCas9n | − | + | − |
| HR template | + | + | + |

2281bp ►

1189bp ►
1092bp ►

HR (%)  0.70  0.46

**E**

AATGACAAGCTTGCTAGCGGTGGG

*Hind*III  *Nhe*I

FIG. 4C-E

FIG. 4F

G

crRNA array
design

EMX1(1)          EMX1(8)

DR    [         ]    DR    [         ]    DR

5'-..AAAACGGAAGGGCCTGAGTCCGAGCAGAAGAAGAAGTT...AACGGAGGGAGGGGCACAGATGAGAAACTCAGGGTTTTAG..-3'

                                                protospacer (8)          ▼▼  PAM

human EMX1   5'-..AGCCCTTCTTCTTCTGCTCGGACTCAGGCCCTTCCTCC..CAGGGAGGGAGGGGCACAGATGAGAAACTCAGGAGGCCCC..-3'
locus           | | | | | | | | | | | | | | | | | | | | | | | | |  | | | | | | | | | | | | | | | | | | | | | | | | | |
             3'-..TCGGGAAGAAGAAGACGAGCCTGAGTCCGGGAAGGAGG..GTCCCTCCCTCCCCGTGTCTACTCTTTGAGTCCTCCGGGG..-5'

                PAM  ▲              protospacer (1)          118bp
                                                             deletion
                                                             junction
                                                                ↓

deletion
result   5'-..GGCAATGCGCCACCGGTTGATGTGATGGGAGCCCTTCTAGGAGGCCCCCAGAGCAGCCACTGGGGCCTCAACACTCAGGC..-3'

FIG. 4G

| Cas9 | target species | gene | protospacer ID | protospacer sequence (5' to 3') | PAM | strand | cell line tested | % indel (pre-crRNA + tracrRNA) | % indel (chimeric RNA) |
|---|---|---|---|---|---|---|---|---|---|
| S. pyogenes SF370 type II CRISPR | Homo sapiens | EMX1 | 1 | GGAAGGGCCTGAGTCCGAGCAGAAGAAGAA | GGG | + | 293FT | 28 ± 1.6 | 6.7 ± 0.62 |
| | | EMX1 | 2 | CATTGGAGGTGACATCGATGTCCTCCCCAT | TGG | – | 293FT | 2.1 ± 0.31 | N.D. |
| | | EMX1 | 3 | GGACATCGATGTCACCTCCAATGACTAGGG | TGG | + | 293FT | 14 ± 1.1 | N.D. |
| | | EMX1 | 4 | CATCGATGTCCTCCCCATTGGCCTGCTTCG | TGG | – | 293FT | 11 ± 1.7 | N.D. |
| | | EMX1 | 5 | TTCGTGGCAATGCGCCACCGGTTGATGTGA | TGG | – | 293FT | 4.3 ± 0.46 | 2.1 ± 0.51 |
| | | EMX1 | 6 | TCGTGGCAATGCGCCACCGGTTGATGTGAT | GGG | – | 293FT | 4.0 ± 0.86 | 0.41 ± 0.25 |
| | | EMX1 | 7 | TCCAGCTTCTGCCGTTTGTACTTTGTCCTC | CGG | – | 293FT | 1.5 ± 0.12 | N.D. |
| | | EMX1 | 8 | GGAGGGAGGGGCACAGATGAGAAACTCAGG | AGG | – | 293FT | 7.8 ± 0.83 | 2.3 ± 1.2 |
| | Homo sapiens | PVALB | 9 | AGGGCCCGAGATTGGGTGTTCAGGGCAGAG | AGG | + | 293FT | 21 ± 2.6 | 6.5 ± 0.32 |
| | | PVALB | 10 | ATGCAGGAGGGTGGCGAGAGGGGCCGAGAT | TGG | + | 293FT | N.D. | N.D. |
| | | PVALB | 11 | GGTGGCGAGAGGGGCCGAGATTGGGTGTTC | AGG | + | 293FT | N.D. | N.D. |
| | Mus musculus | Th | 12 | CAAGCACTGAGTGCCATTAGCTAAATGCAT | AGG | – | Neuro2A | 27 ± 4.3 | 4.1 ± 2.2 |
| | | Th | 13 | AATGCATAGGGTACCACCCACAGGTGCCAG | GGG | – | Neuro2A | 4.8 ± 1.2 | N.D. |
| | | Th | 14 | ACACACATGGAAAGCCTCTGGGCCAGGAA | AGG | + | Neuro2A | 11.3 ± 1.3 | N.D. |
| S. thermophilus LMD-9 CRISPR1 | Homo sapiens | EMX1 | 15 | GGAGGAGGTAGTATACAGAAACACAGAGAA | GTAGAAT | – | 293FT | 14 ± 0.88 | N.T. |
| | | EMX1 | 16 | AGAATGTAGAGGAGTCACAGAAACTCAGCA | CTAGAAA | – | 293FT | 7.8 ± 0.77 | N.T. |

**FIG. 5**

**A**

**B**

FIG. 6A-B

C

FIG. 6C

indel

rehybridize

surveyor nuclease

$a$ $b$

$c$

gel quantification

% indel = $\left(1 - \sqrt{1 - (a + b)/(a + b + c)}\right) * 100$

FIG. 7

FIG. 8A-B

A

*Streptococcus pyogenes* SF370 type II CRISPR
PAM occurence in human genome (NGG)

Median: 8bp
Mean: 12.7bp

B

*Streptococcus thermophilus* LMD-9 CRISPR1
PAM occurence in human genome (NNAGAAW)

Median: 65bp
Mean: 106.6bp

C

| | NGG | | NNAGAAW | |
| Chr | median | mean | median | mean |
|---|---|---|---|---|
| 1 | 7 | 12.8 | 67 | 115.8 |
| 2 | 8 | 12.7 | 64 | 100.8 |
| 3 | 8 | 13.0 | 63 | 98.5 |
| 4 | 9 | 14.0 | 61 | 94.5 |
| 5 | 8 | 13.1 | 63 | 97.9 |
| 6 | 8 | 13.1 | 63 | 98.5 |
| 7 | 8 | 12.4 | 64 | 102.9 |
| 8 | 8 | 12.8 | 64 | 100.9 |
| 9 | 7 | 13.9 | 65 | 120.5 |
| 10 | 7 | 12.1 | 66 | 107.0 |
| 11 | 7 | 12.0 | 65 | 105.8 |
| 12 | 8 | 12.4 | 65 | 103.5 |
| 13 | 8 | 13.6 | 62 | 94.6 |
| 14 | 8 | 12.0 | 65 | 101.5 |
| 15 | 7 | 11.5 | 68 | 107.7 |
| 16 | 7 | 11.7 | 74 | 136.8 |
| 17 | 6 | 10.3 | 76 | 127.9 |
| 18 | 8 | 13.4 | 63 | 101.8 |
| 19 | 6 | 9.4 | 82 | 145.4 |
| 20 | 7 | 11.1 | 72 | 121.8 |
| 21 | 7 | 13.4 | 64 | 111.4 |
| 22 | 6 | 9.2 | 85 | 140.3 |
| X | 8 | 13.2 | 63 | 99.0 |
| Y | 8 | 29.2 | 62 | 223.7 |

FIG. 9A-C

EP 3 011 035 B1

**FIG. 10A-C**

**D**

FIG. 10D

FIG. 11A-C

FIG. 12A-B

FIG. 13A-B

FIG. 14

| Primer name | Assay | Genomic Target | Primer sequence |
|---|---|---|---|
| Sp-EMX1-F | SURVEYOR assay, sequencing | EMX1 | AAAACCACCCTTCTCTCTGGC |
| Sp-EMX1-R | SURVEYOR assay, sequencing | EMX1 | GGAGATTGGAGACACGGAGAG |
| Sp-PVALB-F | SURVEYOR assay, sequencing | PVALB | CTGGAAAGCCAATGCCTGAC |
| Sp-PVALB-R | SURVEYOR assay, sequencing | PVALB | GGCAGCAAACTCCTTGTCCT |
| Sp-Th-F | SURVEYOR assay, sequencing | Th | GTGCTTTGCAGAGGCCTACC |
| Sp-Th-R | SURVEYOR assay, sequencing | Th | CCTGGAGCGCATGCAGTAGT |
| St-EMX1-F | SURVEYOR assay, sequencing | EMX1 | ACCTTCTGTGTTCCACCATTC |
| St-EMX1-R | SURVEYOR assay, sequencing | EMX1 | TTGGGGAGTGCACAGACTTC |
| Sp-EMX1-RFLP-F | RFLP, sequencing | EMX1 | GGCTCCCTGGGTTCAAAGTA |
| Sp-EMX1-RFLP-R | RFLP, sequencing | EMX1 | AGAGGGGTCTGGATGTCGTAA |
| Pb_EMX1_sp1 | Northern Blot Probe | Not applicable | TAGCTCTAAAACTTCTTCTTCTGCTCGGAC |
| Pb_tracrRNA | Northern Blot Probe | Not applicable | CTAGCCTTATTTTAACTTGCTATGCTGTTT |

FIG. 15

FIG. 16A

FIG. 16B

FIG. 16C

FIG. 17A-B

FIG. 18

EP 3 011 035 B1

FIG. 19A

210

FIG. 19B

FIG. 19C

FIG. 19D

FIG. 20A

**FIG. 20B**

FIG. 20C

**FIG. 20D**

FIG. 20E

**FIG. 20F**

FIG. 21A

FIG. 21B

FIG. 21C

d

FIG. 21D

FIG. 22A

b

FIG. 22B

FIG. 23

SpCas9 mutation positions

hSpCas9

FIG. 24A

hSpCas9

5'  CTGGAAGAGTCCTTCCTGGTGGAAGAGGATAAGAAGCACGAGCGGCACCCCATCTTCGGC  360

hSpCas9

L  E  E  S  F  L  V  E  E  D  K  K  H  E  R  H  P  I  F  G

101 102 103 104 105 106 107 108 109 110 111 112 113 114 115 116 117 118 119 120

5'  AACATCGTGGACGAGGTGGCCTACCACGAGAAGTACCCCACCATCTACCACCTGAGAAAG  420

hSpCas9

N  I  V  D  E  V  A  Y  H  E  K  Y  P  T  I  Y  H  L  R  K

121 122 123 124 125 126 127 128 129 130 131 132 133 134 135 136 137 138 139 140

5'  AAACTGGTGGACAGCACCGACAAGGCCGACCTGCGGCTGATCTATCTGGCCCTGGCCCAC  480

hSpCas9

K  L  V  D  S  T  D  K  A  D  L  R  L  I  Y  L  A  L  A  H

141 142 143 144 145 146 147 148 149 150 151 152 153 154 155 156 157 158 159 160

5'  ATGATCAAGTTCCGGGGCCACTTCCTGATCGAGGGCGACCTGAACCCCGACAACAGCGAC  540

hSpCas9

M  I  K  F  R  G  H  F  L  I  E  G  D  L  N  P  D  N  S  D

161 162 163 164 165 166 167 168 169 170 171 172 173 174 175 176 177 178 179 180

5'  GTGGACAAGCTGTTCATCCAGCTGGTGCAGACCTACAACCAGCTGTTCGAGGAAAACCCC  600

hSpCas9

V  D  K  L  F  I  Q  L  V  Q  T  Y  N  Q  L  F  E  E  N  P

181 182 183 184 185 186 187 188 189 190 191 192 193 194 195 196 197 198 199 200

5'  ATCAACGCCAGCGGCGTGGACGCCAAGGCCATCCTGTCTGCCAGACTGAGCAAGAGCAGA  660

hSpCas9

I  N  A  S  G  V  D  A  K  A  I  L  S  A  R  L  S  K  S  R

201 202 203 204 205 206 207 208 209 210 211 212 213 214 215 216 217 218 219 220

FIG. 24B

hSpCas9

5'  CGGCTGGAAAATCTGATCGCCCAGCTGCCCGGCGAGAAGAAGAATGGCCTGTTCGGCAAC
                                                                              720
hSpCas9

R   L   E   N   L   I   A   Q   L   P   G   E   K   K   N   G   L   F   G   N

5'  CTGATTGCCCTGAGCCTGGGCCTGACCCCCAACTTCAAGAGCAACTTCGACCTGGCCGAG
                                                                              780
hSpCas9

L   I   A   L   S   L   G   L   T   P   N   F   K   S   N   F   D   L   A   E

5'  GATGCCAAACTGCAGCTGAGCAAGGACACCTACGACGACGACCTGGACAACCTGCTGGCC
                                                                              840
hSpCas9

D   A   K   L   Q   L   S   K   D   T   Y   D   D   D   L   D   N   L   L   A

5'  CAGATCGGCGACCAGTACGCCGACCTGTTCCTGGCCGCCAAGAACCTGTCCGACGCCATC
                                                                              900
hSpCas9

Q   I   G   D   Q   Y   A   D   L   F   L   A   A   K   N   L   S   D   A   I

5'  CTGCTGAGCGACATCCTGAGAGTGAACACCGAGATCACCAAGGCCCCCCTGAGCGCCTCT
                                                                              960
hSpCas9

L   L   S   D   I   L   R   V   N   T   E   I   T   K   A   P   L   S   A   S

5'  ATGATCAAGAGATACGACGAGCACCACCAGGACCTGACCCTGCTGAAAGCTCTCGTGCGG
                                                                              1020
hSpCas9

M   I   K   R   Y   D   E   H   H   Q   D   L   T   L   L   K   A   L   V   R

FIG. 24C

229

hSpCas9

5' CAGCAGCTGCCTGAGAAGTACAAAGAGATTTTCTTCGACTAGAGCAAGAACGGCTACGCC

hSpCas9

Q Q L P E K Y K E I F F D Q S K N G Y A

341 342 343 344 345 346 347 348 349 350 351 352 353 354 355 356 357 358 359 360

1080

5' GGCTACATTGACGGCGGAGCCAGCCAGGAAGAGTTCTACAAGTTCATCAAGCCCATCCTG

hSpCas9

G Y I D G G A S Q E E F Y K F I K P I L

361 362 363 364 365 366 367 368 369 370 371 372 373 374 375 376 377 378 379 380

1140

5' GAAAAGATGGACGGCACCGAGGAACTGCTCGTGAAGCTGAACAGAGAGGACCTGCTGCGG

hSpCas9

E K M D G T E E L L V K L N R E D L L R

381 382 383 384 385 386 387 388 389 390 391 392 393 394 395 396 397 398 399 400

1200

5' AAGCAGCGGACCTTCGACAACGGCAGCATCCCCCACCAGATCCACCTGGGAGAGCTGCAC

hSpCas9

K Q R T F D N G S I P H Q I H L G E L H

401 402 403 404 405 406 407 408 409 410 411 412 413 414 415 416 417 418 419 420

1260

5' GCCATTCTGCGGCGGCAGGAAGATTTTTACCCATTCCTGAAGGACAACCGGGAAAAGATC

hSpCas9

A I L R R Q E D F Y P F L K D N R E K I

421 422 423 424 425 426 427 428 429 430 431 432 433 434 435 436 437 438 439 440

1320

5' GAGAAGATCCTGACCTTCCGCATCCCCTACTACGTGGGCCCTCTGGCCAGGGGAAACAGC

hSpCas9

E K I L T F R I P Y Y V G P L A R G N S

441 442 443 444 445 446 447 448 449 450 451 452 453 454 455 456 457 458 459 460

1380

FIG. 24D

hSpCas9

5' AGATTCGCCTGGATGACCAGAAAGAGCGAGGAAACCATCACCCCCTGGAACTTCGAGGAA

＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜ 1440

hSpCas9

R   F   A   W   M   T   R   K   S   E   E   T   I   T   P   W   N   F   E   E

461 462 463 464 465 466 467 468 469 470 471 472 473 474 475 476 477 478 479 480

5' GTGGTGGACAAGGGCGCTTCCGCCCAGAGCTTCATCGAGCGGATGACCAACTTCGATAAG

＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜ 1500

hSpCas9

V   V   D   K   G   A   S   A   Q   S   F   I   E   R   M   T   N   F   D   K

481 482 483 484 485 486 487 488 489 490 491 492 493 494 495 496 497 498 499 500

5' AACCTGCCCAACGAGAAGGTGCTGCCCAAGCACAGCCTGCTGTACGAGTACTTCACCGTG

＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜ 1560

hSpCas9

N   L   P   N   E   K   V   L   P   K   H   S   L   L   Y   E   Y   F   T   V

501 502 503 504 505 506 507 508 509 510 511 512 513 514 515 516 517 518 519 520

5' TATAACGAGCTGACCAAAGTGAAATACGTGACCGAGGGAATGAGAAAGCCCGCCTTCCTG

＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜ 1620

hSpCas9

Y   N   E   L   T   K   V   K   Y   V   T   E   G   M   R   K   P   A   F   L

521 522 523 524 525 526 527 528 529 530 531 532 533 534 535 536 537 538 539 540

5' AGCGGCGAGCAGAAAAAGGCCATCGTGGACCTGCTGTTCAAGACCAACCGGAAAGTGACC

＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜ 1680

hSpCas9

S   G   E   Q   K   K   A   I   V   D   L   L   F   K   T   N   R   K   V   T

541 542 543 544 545 546 547 548 549 550 551 552 553 554 555 556 557 558 559 560

5' GTGAAGCAGCTGAAAGAGGACTACTTCAAGAAAATCGAGTGCTTCGACTCCGTGGAAATC

＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜＋＋＋＋｜ 1740

hSpCas9

V   K   Q   L   K   E   D   Y   F   K   K   I   E   C   F   D   S   V   E   I

561 562 563 564 565 566 567 568 569 570 571 572 573 574 575 576 577 578 579 580

FIG. 24E

hSpCas9

```
5'   TCCGGCGTGGAAGATCGGTTCAACGCCTCCCTGGGCACATACCACGATCTGCTGAAAATT
                                                                    1800
                         hSpCas9
     S   G   V   E   D   R   F   N   A   S   L   G   T   Y   H   D   L   L   K   I
    581 582 583 584 585 586 587 588 589 590 591 592 593 594 595 596 597 598 599 600
```

```
5'   ATCAAGGACAAGGACTTCCTGGACAATGAGGAAACGAGGACATTCTGGAAGATATCGTG
                                                                    1860
                         hSpCas9
     I   K   D   K   D   F   L   D   N   E   E   N   E   D   I   L   E   D   I   V
    601 602 603 604 605 606 607 608 609 610 611 612 613 614 615 616 617 618 619 620
```

```
5'   CTGACCCTGACACTGTTTGAGGACAGAGAGATGATCGAGGAACGGCTGAAAACCTATGCC
                                                                    1920
                         hSpCas9
     L   T   L   T   L   F   E   D   R   E   M   I   E   E   R   L   K   T   Y   A
    621 622 623 624 625 626 627 628 629 630 631 632 633 634 635 636 637 638 639 640
```

```
5'   CACCTGTTCGACGACAAAGTGATGAAGCAGCTGAAGCGGCGGAGATACACCGGCTGGGGC
                                                                    1980
                         hSpCas9
     H   L   F   D   D   K   V   M   K   Q   L   K   R   R   R   Y   T   G   W   G
    641 642 643 644 645 646 647 648 649 650 651 652 653 654 655 656 657 658 659 660
```

```
5'   AGGCTGAGCCGGAAGCTGATCAACGGCATCCGGGACAAGCAGTCCGGCAAGACAATCCTG
                                                                    2040
                         hSpCas9
     R   L   S   R   K   L   I   N   G   I   R   D   K   Q   S   G   K   T   I   L
    661 662 663 664 665 666 667 668 669 670 671 672 673 674 675 676 677 678 679 680
```

```
5'   GATTTCCTGAAGTCCGACGGCTTCGCCAACAGAAACTTCATGCAGCTGATCCACGACGAC
                                                                    2100
                         hSpCas9
     D   F   L   K   S   D   G   F   A   N   R   N   F   M   Q   L   I   H   D   D
    681 682 683 684 685 686 687 688 689 690 691 692 693 694 695 696 697 698 699 700
```

FIG. 24F

hSpCas9

5' AGCCTGACCTTTAAAGAGGACATCCAGAAAGCCCAGGTGTCTGGCCAGGGCGATAGCCTG

hSpCas9

S  L  T  F  K  E  D  I  Q  K  A  Q  V  S  G  Q  G  D  S  L
701 702 703 704 705 706 707 708 709 710 711 712 713 714 715 716 717 718 719 720

5' CACGAGCACATTGCCAATCTGGCCGGCAGCCCCGCCATTAAGAAGGGCATCCTGCAGACA

hSpCas9

H  E  H  I  A  N  L  A  G  S  P  A  I  K  K  G  I  L  Q  T
721 722 723 724 725 726 727 728 729 730 731 732 733 734 735 736 737 738 739 740

5' GTGAAGGTGGTGGACGAGCTCGTGAAAGTGATGGGCCGGCACAAGCCCGAGAACATCGTG

hSpCas9

SacC I

Y  K  V  V  D  E  L  V  K  Y  M  G  R  H  K  P  E  N  I  V
741 742 743 744 745 746 747 748 749 750 751 752 753 754 755 756 757 758 759 760

5' ATCGCCATGGCCAGAGAGAACCAGACCACCCAGAAGGGGACAGAAGAACAGCCGCGAGAGA

hSpCas9

RuvCiii

E

I  A  M  A  R  E  N  Q  T  T  Q  K  G  Q  K  N  S  R  E  R
761 762 763 764 765 766 767 768 769 770 771 772 773 774 775 776 777 778 779 780

5' ATGAAGCGGATCGAAGAGGGCATCAAAGAGCTGGGCAGCCAGATCCTGAAAGAACACCCC

hSpCas9

M  K  R  I  E  E  G  I  K  E  L  G  S  Q  I  L  K  E  H  P
781 782 783 784 785 786 787 788 789 790 791 792 793 794 795 796 797 798 799 800

FIG. 24G

hSpCas9

5'  GTGGAAAACACCCAGCTGCAGAACGAGAAGCTGTACCTGTACTACCTGCAGAATGGGCGG

+++++|+++++|+++++|+++++|+++++|+++++|+++++|+++++|+++++|+++++|  2460

hSpCas9

V   E   N   T   Q   L   Q   N   E   K   L   Y   L   Y   Y   L   Q   N   G   R

801 802 803 804 805 806 807 808 809 810 811 812 813 814 815 816 817 818 819 820

5'  GATATGTACGTGGACCAGGAACTGGACATCAACCGGCTGTCCGACTACGATGTGGACGCC

+++++|+++++|+++++|+++++|+++++|+++++|+++++|+++++|+++++|+++++|  2520

hSpCas9

HNH

H...

D   M   Y   V   D   Q   E   L   D   I   N   R   L   S   D   Y   D   V   D   A

821 822 823 824 825 826 827 828 829 830 831 832 833 834 835 836 837 838 839 840

5'  ATCGTGCCTCAGAGCTTTCTGAAGGACGACTCCATCGACGCCAAGGTGCTGACCAGAAGC

+++++|+++++|+++++|+++++|+++++|+++++|+++++|+++++|+++++|+++++|  2580

hSpCas9

HNH

H...

I   V   P   Q   S   F   L   K   D   D   S   I   D   A   K   V   L   T   R   S

841 842 843 844 845 846 847 848 849 850 851 852 853 854 855 856 857 858 859 860

5'  GACAAGGCCCGGGGCAAGAGCGACAACGTGCCCTCCGAAGAGGTCGTGAAGAAGATGAAG

+++++|+++++|+++++|+++++|+++++|+++++|+++++|+++++|+++++|+++++|  2640

hSpCas9

HNH

H...

D   K   A   R   G   K   S   D   N   V   P   S   E   E   V   Y   K   K   M   K

861 862 863 864 865 866 867 868 869 870 871 872 873 874 875 876 877 878 879 880

5'  AACTACTGGCGGCAGCTGCTGAACGCCAAGCTGATTACCCAGAGAAAGTTCGACAATCTG

+++++|+++++|+++++|+++++|+++++|+++++|+++++|+++++|+++++|+++++|  2700

hSpCas9

N   Y   W   R   Q   L   L   N   A   K   L   I   T   Q   R   K   F   D   N   L

881 882 883 884 885 886 887 888 889 890 891 892 893 894 895 896 897 898 899 900

FIG. 24H

hSpCas9

FIG. 24I

EP 3 011 035 B1

hSpCas9

5' TACCCTAAGCTGGAAAGCGAGTTCGTGTACGGCGACTACAAGGTGTACGACGTGCGGAAG
+++++|++++|++++|++++|++++|++++|++++|++++|++++|++++|++++|++++| 3060

hSpCas9

Y  P  K  L  E  S  E  F  V  Y  G  D  Y  K  V  Y  D  V  R  K

5' ATGATCGCCAAGAGCGAGCAGGAAATCGGCAAGGCTACCGCCAAGTACTTCTTCTACAGC
+++++|++++|++++|++++|++++|++++|++++|++++|++++|++++|++++|++++| 3120

hSpCas9

M  I  A  K  S  E  Q  E  I  G  K  A  T  A  K  Y  F  F  Y  S

5' AACATCATGAACTTTTTCAAGACCGAGATTACCCTGGCCAACGGCGAGATCCGGAAGCGG
+++++|++++|++++|++++|++++|++++|++++|++++|++++|++++|++++|++++| 3180

hSpCas9

N  I  M  N  F  F  K  T  E  I  T  L  A  N  G  E  I  R  K  R

5' CCTCTGATCGAGACAAACGGCGAAACCGGGGAGATCGTGTGGGATAAGGGCCGGGATTTT
+++++|++++|++++|++++|++++|++++|++++|++++|++++|++++|++++|++++| 3240

hSpCas9

P  L  I  E  T  N  G  E  T  G  E  I  V  W  D  K  G  R  D  F

5' GCCACCGTGCGGAAAGTGCTGAGCATGCCCCAAGTGAATATCGTGAAAAAGACCGAGGTG
+++++|++++|++++|++++|++++|++++|++++|++++|++++|++++|++++|++++| 3300

hSpCas9

A  T  V  R  K  V  L  S  M  P  Q  V  N  I  V  K  K  T  E  V

5' CAGACAGGCGGCTTCAGCAAAGAGTCTATCCTGCCCAAGAGGAACAGCGATAAGCTGATC
+++++|++++|++++|++++|++++|++++|++++|++++|++++|++++|++++|++++| 3360

hSpCas9

Q  T  G  G  F  S  K  E  S  I  L  P  K  R  N  S  D  K  L  I

FIG. 24J

236

hSpCas9

5' GCCAGAAAGAAGGACTGGGACCCCTAAGAAGTACGGCGGCTTCGACAGCCCCACCGTGGCC

3420

hSpCas9

A R K K D W D P K K Y G G F D S P T V A

5' TATTCTGTGCTGGTGGTGGCCAAAGTGGAAAAGGGCAAGTCCAAGAAACTGAAGAGTGTG

3480

hSpCas9

Y S V L V V A K V E K G K S K K L K S V

5' AAAGAGCTGCTGGGGATCACCATCATGGAAAGAAGCAGCTTCGAGAAGAATCCCATCGAC

3540

hSpCas9

K E L L G I T I M E R S S F E K N P I D

5' TTTCTGGAAGCCAAGGGCTACAAAGAAGTGAAAAAGGACCTGATCATCAAGCTGCCTAAG

3600

hSpCas9

F L E A K G Y K E V K K D L I I K L P K

5' TACTCCCTGTTCGAGCTGGAAAACGGCCGGAAGAGAATGCTGGCCTCTGCCGGCGAACTG

3660

hSpCas9

Y S L F E L E N G R K R M L A S A G E L

5' CAGAAGGGAAACGAACTGGCCCTGCCCTCCAAATATGTGAACTTCCTGTACCTGGCCAGC

3720

hSpCas9

Q K G N E L A L P S K Y V N F L Y L A S

FIG. 24K

hSpCas9

5' CACTATCAGAAGCTCAAGGGCTCCCCCCCAGGATAATGAGCAGAAACAGCTGTTTGTGGAA
  +++++|+++++|+++++|+++++|+++++|+++++|+++++|+++++|+++++|+++++|  3780
  hSpCas9
  H Y E K L K G S P E D N E Q K Q L F V E
  1241 1242 1243 1244 1245 1246 1247 1248 1249 1250 1251 1252 1253 1254 1255 1256 1257 1258 1259 1260

5' CAGCACAAGCACTACCTGGACGAGATCATCGAGCAGATCAGCGAGTTCTCCAAGAGAGTG
  +++++|+++++|+++++|+++++|+++++|+++++|+++++|+++++|+++++|+++++|  3840
  hSpCas9
  Q H K H Y L D E I I E Q I S E F S K R V
  1261 1262 1263 1264 1265 1266 1267 1268 1269 1270 1271 1272 1273 1274 1275 1276 1277 1278 1279 1280

5' ATCCTGGCCGACGCTAATCTGGACAAAGTGCTGTCCGCCTACAACAAGCACCGGGATAAG
  +++++|+++++|+++++|+++++|+++++|+++++|+++++|+++++|+++++|+++++|  3900
  hSpCas9
  I L A D A N L D K V L S A Y N K H R D K
  1281 1282 1283 1284 1285 1286 1287 1288 1289 1290 1291 1292 1293 1294 1295 1296 1297 1298 1299 1300

5' CCCATCAGAGAGCAGGCCGAGAATATCATCCACCTGTTTACCCTGACCAATCTGGGAGCC
  +++++|+++++|+++++|+++++|+++++|+++++|+++++|+++++|+++++|+++++|  3960
  hSpCas9
  P I R E Q A E N I I H L F T L T N L G A
  1301 1302 1303 1304 1305 1306 1307 1308 1309 1310 1311 1312 1313 1314 1315 1316 1317 1318 1319 1320

5' CCTGCCGCCTTCAAGTACTTTGACACCACCATCGACCGGAAGAGGTACACCAGCACCAAA
  +++++|+++++|+++++|+++++|+++++|+++++|+++++|+++++|+++++|+++++|  4020
  hSpCas9
  P A A F K Y F D T T I D R K R Y T S T K
  1321 1322 1323 1324 1325 1326 1327 1328 1329 1330 1331 1332 1333 1334 1335 1336 1337 1338 1339 1340

5' GAGGTGCTGGACGCCACCCTGATCCACCAGAGCATCACCGGCCTGTACGAGACACGGATC
  +++++|+++++|+++++|+++++|+++++|+++++|+++++|+++++|+++++|+++++|  4080
  hSpCas9
  E V L D A T L I H Q S I T G L Y E T R I
  1341 1342 1343 1344 1345 1346 1347 1348 1349 1350 1351 1352 1353 1354 1355 1356 1357 1358 1359 1360

FIG. 24L

hSpCas9

5'    GACCTGTCTCAGCTGGGAGGCGAC

hSpCas9

D    L    S    Q    L    G    G    D

1361 1362 1363 1364 1365 1366 1367 1368

4104

FIG. 24M

Conditional Cas9, Rosa26 targeting vector map

FIG. 25A

Constitutive Cas9, Rosa26 targeting vector map

FIG. 25B

FIG. 26

Cas9 Expression in Mouse Hippocampus (AAV)

Cas9 Expression in Mouse Cortex (AAV)

FIG. 27

FIG. 28A-C

FIG. 29

FIG. 30A-C

## Repair Strategy for Cystic Fibrosis deltaF508 Mutation

FIG. 31A-C

A

GAA repeat expansion in *FXN* intron 1

Transcription repression likely due to aberrant DNA structure or recruitment of heterchromatin binding proteins to long GAA repeats

B

FIG. 32A-B

FIG. 33

FIG. 34

FIG. 35

FIG. 36

FIG. 37

FIG. 38

# Expression of SpCas9 & SaCas9 in N2a cells

FIG. 39

FIG. 40

CA1region of hippocampus

HA DAPI

no primary Ab

FIG. 41

FIG. 42

FIG. 43

FIG. 44

FIG. 45

FIG. 46

# SpCas9 *in vivo: Mecp2*

FIG. 47

## Purification of cell nuclei from brain

FIG. 48

FIG. 49

FIG. 50

FIG. 51

Reverse PCR primers for generating U6-guide RNA expression Cassette (pair with U6 forward primer)

| Primer | Sequence | Label |
|---|---|---|
| FR0260 | AAAAAAGCACCGACTCGGTGCCACTTTTTCAAGTTGATAAGGGACTAGCCTTATTTTAACTTGCTATTTCTAGCTCTAAAACA... | Left guide RNA 1 |
| FR0261 | AAAAAAGCACCGACTCGGTGCCACTTTTTCAAGTTGATAAGGGACTAGCCTTATTTTAACTTGCTATTTCTAGCTCTAAAAC... | Left guide RNA 2 |
| FR0262 | AAAAAAGCACCGACTCGGTGCCACTTTTTCAAGTTGATAAGGGACTAGCCTTATTTTAACTTGCTATTTCTAGCTCTAAAAC... | Left guide RNA 3 |
| FR0263 | AAAAAAGCACCGACTCGGTGCCACTTTTTCAAGTTGATAAGGGACTAGCCTTATTTTAACTTGCTATTTCTAGCTCTAAAAC... | Left guide RNA 4 |
| FR0264 | AAAAAAGCACCGACTCGGTGCCACTTTTTCAAGTTGATAAGGGACTAGCCTTATTTTAACTTGCTATTTCTAGCTCTAAAAC... | Left guide RNA 5 |
| FR0265 | AAAAAAGCACCGACTCGGTGCCACTTTTTCAAGTTGATAAGGGACTAGCCTTATTTTAACTTGCTATTTCTAGCTCTAAAAC... | Left guide RNA 6 |
| FR0266 | AAAAAAGCACCGACTCGGTGCCACTTTTTCAAGTTGATAAGGGACTAGCCTTATTTTAACTTGCTATTTCTAGCTCTAAAAC... | Left guide RNA 7 |
| FR0267 | AAAAAAGCACCGACTCGGTGCCACTTTTTCAAGTTGATAAGGGACTAGCCTTATTTTAACTTGCTATTTCTAGCTCTAAAAC... | Left guide RNA 8 |
| FR0268 | AAAAAAGCACCGACTCGGTGCCACTTTTTCAAGTTGATAAGGGACTAGCCTTATTTTAACTTGCTATTTCTAGCTCTAAAAC... | Left guide RNA 9 |
| FR0269 | AAAAAAGCACCGACTCGGTGCCACTTTTTCAAGTTGATAAGGGACTAGCCTTATTTTAACTTGCTATTTCTAGCTCTAAAAC... | Left guide RNA 10 |
| FR0270 | AAAAAAGCACCGACTCGGTGCCACTTTTTCAAGTTGATAAGGGACTAGCCTTATTTTAACTTGCTATTTCTAGCTCTAAAAC... | Left guide RNA 11 |
| FR0271 | AAAAAAGCACCGACTCGGTGCCACTTTTTCAAGTTGATAAGGGACTAGCCTTATTTTAACTTGCTATTTCTAGCTCTAAAAC... | Left guide RNA 12 |
| FR0272 | AAAAAAGCACCGACTCGGTGCCACTTTTTCAAGTTGATAAGGGACTAGCCTTATTTTAACTTGCTATTTCTAGCTCTAAAAC... | Left guide RNA 13 |
| FR0273 | AAAAAAGCACCGACTCGGTGCCACTTTTTCAAGTTGATAAGGGACTAGCCTTATTTTAACTTGCTATTTCTAGCTCTAAAAC... | Left guide RNA 14 |
| FR0274 | AAAAAAGCACCGACTCGGTGCCACTTTTTCAAGTTGATAAGGGACTAGCCTTATTTTAACTTGCTATTTCTAGCTCTAAAAC... | Left guide RNA 15 |
| FR0275 | AAAAAAGCACCGACTCGGTGCCACTTTTTCAAGTTGATAAGGGACTAGCCTTATTTTAACTTGCTATTTCTAGCTCTAAAAC... | Left guide RNA 16 |
| FR0276 | AAAAAAGCACCGACTCGGTGCCACTTTTTCAAGTTGATAAGGGACTAGCCTTATTTTAACTTGCTATTTCTAGCTCTAAAAC... | Left guide RNA 17 |
| FR0277 | AAAAAAGCACCGACTCGGTGCCACTTTTTCAAGTTGATAAGGGACTAGCCTTATTTTAACTTGCTATTTCTAGCTCTAAAAC... | Left guide RNA 18 |
| FR0278 | AAAAAAGCACCGACTCGGTGCCACTTTTTCAAGTTGATAAGGGACTAGCCTTATTTTAACTTGCTATTTCTAGCTCTAAAAC... | Left guide RNA 19 |
| FR0279 | AAAAAAGCACCGACTCGGTGCCACTTTTTCAAGTTGATAAGGGACTAGCCTTATTTTAACTTGCTATTTCTAGCTCTAAAAC... | Left guide RNA 20 |
| FR0280 | AAAAAAGCACCGACTCGGTGCCACTTTTTCAAGTTGATAAGGGACTAGCCTTATTTTAACTTGCTATTTCTAGCTCTAAAAC... | Left guide RNA 21 |
| FR0281 | AAAAAAGCACCGACTCGGTGCCACTTTTTCAAGTTGATAAGGGACTAGCCTTATTTTAACTTGCTATTTCTAGCTCTAAAAC... | Left guide RNA 22 |
| FR0282 | AAAAAAGCACCGACTCGGTGCCACTTTTTCAAGTTGATAAGGGACTAGCCTTATTTTAACTTGCTATTTCTAGCTCTAAAAC... | Left guide RNA 23 |
| FR0283 | AAAAAAGCACCGACTCGGTGCCACTTTTTCAAGTTGATAAGGGACTAGCCTTATTTTAACTTGCTATTTCTAGCTCTAAAAC... | Left guide RNA 24 |
| FR0284 | AAAAAAGCACCGACTCGGTGCCACTTTTTCAAGTTGATAAGGGACTAGCCTTATTTTAACTTGCTATTTCTAGCTCTAAAAC... | Right guide RNA 1 |
| FR0285 | AAAAAAGCACCGACTCGGTGCCACTTTTTCAAGTTGATAAGGGACTAGCCTTATTTTAACTTGCTATTTCTAGCTCTAAAAC... | Right guide RNA 2 |
| FR0286 | AAAAAAGCACCGACTCGGTGCCACTTTTTCAAGTTGATAAGGGACTAGCCTTATTTTAACTTGCTATTTCTAGCTCTAAAAC... | Right guide RNA 3 |
| FR0287 | AAAAAAGCACCGACTCGGTGCCACTTTTTCAAGTTGATAAGGGACTAGCCTTATTTTAACTTGCTATTTCTAGCTCTAAAAC... | Right guide RNA 4 |
| FR0288 | AAAAAAGCACCGACTCGGTGCCACTTTTTCAAGTTGATAAGGGACTAGCCTTATTTTAACTTGCTATTTCTAGCTCTAAAAC... | Right guide RNA 5 |
| FR0289 | AAAAAAGCACCGACTCGGTGCCACTTTTTCAAGTTGATAAGGGACTAGCCTTATTTTAACTTGCTATTTCTAGCTCTAAAAC... | Right guide RNA 6 |
| FR0290 | AAAAAAGCACCGACTCGGTGCCACTTTTTCAAGTTGATAAGGGACTAGCCTTATTTTAACTTGCTATTTCTAGCTCTAAAAC... | Right guide RNA 7 |
| FR0291 | AAAAAAGCACCGACTCGGTGCCACTTTTTCAAGTTGATAAGGGACTAGCCTTATTTTAACTTGCTATTTCTAGCTCTAAAAC... | Right guide RNA 8 |
| FR0292 | AAAAAAGCACCGACTCGGTGCCACTTTTTCAAGTTGATAAGGGACTAGCCTTATTTTAACTTGCTATTTCTAGCTCTAAAAC... | Right guide RNA 9 |
| FR0293 | AAAAAAGCACCGACTCGGTGCCACTTTTTCAAGTTGATAAGGGACTAGCCTTATTTTAACTTGCTATTTCTAGCTCTAAAAC... | Right guide RNA 10 |
| FR0294 | AAAAAAGCACCGACTCGGTGCCACTTTTTCAAGTTGATAAGGGACTAGCCTTATTTTAACTTGCTATTTCTAGCTCTAAAAC... | Right guide RNA 11 |
| FR0295 | AAAAAAGCACCGACTCGGTGCCACTTTTTCAAGTTGATAAGGGACTAGCCTTATTTTAACTTGCTATTTCTAGCTCTAAAAC... | Right guide RNA 12 |
| FR0296 | AAAAAAGCACCGACTCGGTGCCACTTTTTCAAGTTGATAAGGGACTAGCCTTATTTTAACTTGCTATTTCTAGCTCTAAAAC... | Right guide RNA 13 |
| FR0297 | AAAAAAGCACCGACTCGGTGCCACTTTTTCAAGTTGATAAGGGACTAGCCTTATTTTAACTTGCTATTTCTAGCTCTAAAAC... | Right guide RNA 14 |
| FR0298 | AAAAAAGCACCGACTCGGTGCCACTTTTTCAAGTTGATAAGGGACTAGCCTTATTTTAACTTGCTATTTCTAGCTCTAAAAC... | Right guide RNA 15 |
| FR0299 | AAAAAAGCACCGACTCGGTGCCACTTTTTCAAGTTGATAAGGGACTAGCCTTATTTTAACTTGCTATTTCTAGCTCTAAAAC... | Right guide RNA 16 |
| FR0300 | AAAAAAGCACCGACTCGGTGCCACTTTTTCAAGTTGATAAGGGACTAGCCTTATTTTAACTTGCTATTTCTAGCTCTAAAAC... | Right guide RNA 17 |
| FR0301 | AAAAAAGCACCGACTCGGTGCCACTTTTTCAAGTTGATAAGGGACTAGCCTTATTTTAACTTGCTATTTCTAGCTCTAAAAC... | Right guide RNA 18 |
| FR0302 | AAAAAAGCACCGACTCGGTGCCACTTTTTCAAGTTGATAAGGGACTAGCCTTATTTTAACTTGCTATTTCTAGCTCTAAAAC... | Right guide RNA 19 |
| FR0303 | AAAAAAGCACCGACTCGGTGCCACTTTTTCAAGTTGATAAGGGACTAGCCTTATTTTAACTTGCTATTTCTAGCTCTAAAAC... | Right guide RNA 20 |
| FR0304 | AAAAAAGCACCGACTCGGTGCCACTTTTTCAAGTTGATAAGGGACTAGCCTTATTTTAACTTGCTATTTCTAGCTCTAAAAC... | Right guide RNA 21 |
| FR0305 | AAAAAAGCACCGACTCGGTGCCACTTTTTCAAGTTGATAAGGGACTAGCCTTATTTTAACTTGCTATTTCTAGCTCTAAAAC... | Right guide RNA 22 |
| FR0306 | AAAAAAGCACCGACTCGGTGCCACTTTTTCAAGTTGATAAGGGACTAGCCTTATTTTAACTTGCTATTTCTAGCTCTAAAAC... | Right guide RNA 23 |
| FR0307 | AAAAAAGCACCGACTCGGTGCCACTTTTTCAAGTTGATAAGGGACTAGCCTTATTTTAACTTGCTATTTCTAGCTCTAAAAC... | Right guide RNA 24 |

FIG. 52

FIG. 53

| lane # | Cas9 to use | Left gRNA | Right gRNA | Overhang (D10A) - number of bases protruding from 5' |
|---|---|---|---|---|
| 1 | D10A | left 23 | left 12 | -36 |
| 2 | D10A | right 4 | left 9 | -25 |
| 3 | D10A | left 23 | right 23 | -16 |
| 4 | D10A | right 7 | left 10 | -15 |
| 5 | D10A | right 16 | left 3 | -8 |
| 6 | D10A | right 22 | right 6 | 26 |
| 7 | D10A | left 12 | right 16 | 31 |
| 8 | D10A | left 12 | right 13 | 34 |
| 9 | D10A | left 10 | right 1 | 38 |
| 10 | D10A | right 23 | right 16 | 51 |
| 11 | D10A | right 23 | right 13 | 54 |
| 12 | D10A | left 3 | right 7 | 57 |
| 13 | D10A | left 12 | right 4 | 65 |
| 14 | D10A | left 12 | right 3 | 69 |
| 15 | D10A | left 3 | right 10 | 76 |
| 16 | D10A | right 23 | right 4 | 85 |
| 17 | D10A | left 12 | right 9 | 95 |
| 18 | D10A | left 12 | right 10 | 115 |
| 19 | D10A | right 23 | right 10 | 135 |
| 20 | D10A | left 12 | right 2 | 145 |
| 21 | D10A | left 12 | left 22 | 181 |
| 22 | D10A | right 23 | left 22 | 201 |
| 23 | D10A | left 12 | right 6 | 222 |
| 24 | D10A | right 23 | right 6 | 242 |

FIG. 54

FIG. 55

A)

B)

FIG. 56A-B

C)

D)

FIG. 56C-D

MPP ST-HSC LT-HSC LT-HSC CD34-

32% 47% 51% 44%

SSC

CRISPR/Cas(GFP)

FIG. 56E

FIG. 57A-B

FIG. 57C

FIG. 57D

FIG. 58A

B)

FIG. 58B

FIG. 58C-D

FIG. 58E

F)

G)

FIG. 58F-G

A)

B)

C)

FIG. 59A-C

FIG. 60

FIG. 61A-C

A)

B)

FIG. 62A-B

FIG. 63A

B)

FIG. 63B

Ezh2
AGACAC----------------------------------------------TTCCGA 36bp del(4/8)
AGACACCACCTAAACGCC-AGGGGCCGCAGAAGAGGAAGACTTCCGA 1bp del (1/8)
AGACACCACCTAAACGCCCAGGGGCCGCAGAAGAGGAAGACTTCCGA wt (3/8)

Nf1
GATGAGCCGCCA--TCGAGGGAGTTGTTGAAGG 2bp del(2/7)
GATGAGGGCAGA-------------------TGAAGG 21bp del / 6bp ins (2/7)
GATGAGCCGCCACATCGAGGGAGTTGTTGAAGG wt (3/7)

Tet2
TGCAGG------------------------------------------CTCACA 36bp del(1/7)
TGCAGGTTTGAGGTCTTACTTCCTAC-GGATGTAGAGCTTGTTCCCGCTCACA 1bp del (2/7)
TGCAGGTTTGAGGTCTTACTTCCTACGGATGTAGAGCTTGTTCCCGCTCACA wt (4/7)

Runx1
CATCCGG--ACGTCC 1bp del(2/8)
CATCCG---ACGTCC 2bp del(2/8)
CATCCGGGGACGTCC 1bp ins (3/8)
CATCCGGG-ACGTCC wt (1/8)

Smc3
CATCGA--GTGCTC 1bp del(1/7)
CATCGAAGGTGCTC 1bp ins (1/7)
CATCGA-GGTGCTC wt (5/7)

FIG. 64

FIG. 65

A)

*Tet2*
```
GCGGG-----------------------AGTAAG 23bp del (5/8)
GCGGGAACAAGCTCTACA------TAGGAAGTAAG 8bp del (2/8)
GCGGGAACAAGCTCTACATCCCGTAGGAAGTAAG wt (1/8)
```

*Dnmt3a*
```
TCTTTCCCTGGTTGCCGCACCATGCC 1bp ins (6/12)
TCTTTC--------CCGCACCATGCC 8bp del (4/12)
TCTTTCCCTGG---------CATGCC 9bp del (1/12)
TCTTTCCCTGG-TGCCGCACCATGCC wt (1/12)
```

*Nf1*
```
CAACTC------ATGTGG 5bp del (6/8)
CAACTCCCTCGATGTGG wt (2/8)
```

*Runx1*
```
GGACGT--CCGGAT 1bp del (5/10)
GGACGTCCCCGGAT 1bp ins (3/8)
GGACGT-CCCGGAT wt (2/8)
```

B)

*Tet2*
```
CTACAT-CCGTAG 1bp del (8/8)
CTACATCCCGTAG wt (0/8)
```

*Ezh2*
```
ACCACC-----------CCAGGG 7bp del (3/7)
ACCACCTAAAC----CCAGGG 2bp del (4/7)
ACCACCTAAACGCCCAGGG wt (0/7)
```

*Nf1*
```
TGTCCT--------------------------ATCTCT 36bp del (4/8)
TGTCCTTCAACAACTCCCTCGATGTGGCGGCTCATCTGCCCTATCTCT wt (4/8)
```

*Runx1*
```
CCATCCGGGACG 1bp ins (8/8)
CCATCC-GGGACG wt (0/8)
```

# FIG. 66A-B

```
Dnmt3a    C C A A G T ~ ~ ~ ~ ~ ~ ~ ~ ~ ~ ~ ~ ~ // ~ ~ ~ ~ ~ ~ ~ ~ ~ ~ ~ ~ C C T T C C  81bp del (10/10)
          C C A A G T G G A C C G C T A C A T // T T G C C A T C C A C T C C T T C C  wt (0/10)

Ezh2      G A C A C C ~ ~ ~ ~ ~ ~ ~ ~ ~ ~ C C C A G G  9bp del (6/11)
          G A C A C C A C C T ~ ~ ~ ~ ~ C C C A G G  5bp del (5/11)
          G A C A C C A C C T A A A C G C C C A G G  wt (0/11)

Smc3      G G T G A T ~ ~ ~ ~ ~ ~ ~ ~ ~ ~ ~ ~ ~ ~ ~ ~ ~ ~ ~ ~ ~ ~ ~ ~ T A T T A C  33bp del (9/9)
          G G T G A T C A A G T C A G C C A T C G A G G T G C T C T G A C T G G A G G T T A T T A C  wt (0/9)

Nf1       A T G C T G ~ ~ ~ ~ ~ ~ ~ ~ ~ ~ ~ ~ ~ ~ ~ ~ ~ ~ ~ ~ ~ ~ ~ ~ ~ ~ C C C T A T  36bp del (4/9)
          A T G C T G T C C T T C A A C A A C T C C C T C ~ A T G T G G C G G C T C A T C T G C C C T A T  1bp del (5/9)
          A T G C T G T C C T T C A A C A A C T C C C T C G A T G T G G C G G C T C A T C T G C C C T A T  wt (0/9)
```

FIG. 67

FIG. 68A-B

A)

sgRNA-Pool
CRISPR
72.9%

Gr1
CD11b
0.24
47
24.4
28.3

B)

C) Tet2
```
CCTACG--ATGTAG 1bp del (4/5)
CCTACGGGATGTAG wt (1/5)
```

Dnmt3a
```
GCGGCA------GGAAAG 4bp del (1/13)
GCGGCACCAGGGAAAG wt (2/13)*
CCTCCG--------------------------//-----------------------------CAGCGT 73bp del (2/13)
CCTCCGAGGTGTGTGAGGACTCCATC//ATGTACGTCGGGGACGTCCGCAGCGT wt (2/13)*
CCTGCT-------------------------------------------------GTCACA 216bp del (5/13)
CCTGCTGTGCATCTGGCAAGACAAGT//ACGTCGGGGACGTCCGCAGCGTCACA wt (2/13)*
```

Runx1
```
GGTTGA-------------------------------------------CGTCAT 48bp del (5/10)
GGTTGACGTCTAGGTGGTGGCACTGGCGGACGT-CCGGATGGCACTCTGGTCACCGTCAT 1bp del (5/10)
GGTTGACGTCTAGGTGGTGGCACTGGGGGACGTCCCGGATGGCACTCTGGTCACCGTCAT wt (0/10)
```

Nf1
```
TCAACA-----------------------GCTCATC 18bp del (1/8)
TCAACAACTCC----------------CTCATC 13bp del (2/8)
TCAACAACTCCCTCGCGATGTGGCGGCTCATC 2bp ins (5/8)
TCAACAACTCCCTCG--ATGTGGCGGCTCATC wt (0/8)
```

Ezh2
```
TCTTCT-----------------------------------------------GCAGTA 40bp del (4/8)
TCTTCTGCGGCCCCCTGGGAGCGTTAGGTGGTGTCTTTATACGCTCAGCAGTA 2bp ins (4/8)
TCTTCTGCGGCCCCCTGGG--CGTTAGGTGGTGTCTTTATACGCTCAGCAGTA wt (0/8)
```

FIG. 69A-C

FIG. 70A

B)

sgRNA-Pod

CRISPR
(Tet2)

FIG. 70B

FIG. 71A-C

FIG. 72A-C

FIG. 73A-C

A)

B)

FIG. 74A-B

FIG. 75A-F

FIG. 76A-D

FIG. 77A-G

A)

B)

C)

FIG. 78A-C

FIG. 79A-C

FIG. 80

A)

B)

Figure 81

Figure 82

Figure 83

Figure 84

A)

*Tet2*
GCGGG- - - - - - - - - - - - - - - - - - - - - - -AGTAAG 23bp del (5/8)
GCGGGAACAAGCTCTACA- - - - -TAGGAAGTAAG 5bp del (2/8)
GCGGGAACAAGCTCTACATCCCGTAGGAAGTAAG wt (1/8)

*Dnmt3a*
TCTTTCCCTGGTTGCCGCACCATGCC 1bp ins (6/12)
TCTTTC- - - - - - - - -CCGCACCATGCC 8bp del (4/12)
TCTTTCCCTGG- - - - - - - -CATGCC 9bp del (1/12)
TCTTTCCCTGG-TGCCGCACCATGCC wt (1/12)

*Nf1*
CAACTC- - - - -ATGTGG 5bp del (6/8)
CAACTCCCTCGATGTGG wt (2/8)

*Runx1*
ACTGGGGGACGT- -CCGGATGGCA 1bp del (5/10)
ACTGGGGGACGTCCCCGGATGGCA 1bp ins (3/8)
ACTGGGGGACGT-CCCGGATGGCA wt (2/8)

B)

*Tet2*
CTTCCTACGG-ATGTAGAGCTTG 1bp del (8/8)
CTTCCTACGGGATGTAGAGCTTG wt (0/8)

*Ezh2*
ACCACC- - - - - - -CCAGGG 7bp del (3/7)
ACCACCTAAAC- - -CCAGGG 2bp del (4/7)
ACCACCTAAACGCCCAGGG wt (0/7)

*Nf1*
TGTCCT- - - - - - - - - - - - - - - - - - - - - - - - - - - - - - -ATCTCT 36bp del (4/8)
TGTCCTTCAACAACTCCCTCGATGTGGCGGCTCATCTGCCCTATCTCT wt (4/8)

*Runx1*
CCATCCGGGACG 1bp ins (8/8)
CCATCC-GGGACG wt (0/8)

FIG. 85A-B

Dnmt3a
```
CCAAGT- - - - - - - - - - - - //- - - - - - - - - - -CCTTCC 181bp del (10/10)
CCAAGTGGACCGCTACAT// TTGCCATCCACTCCTTCC wt (0/10)
```

Ezh2
```
GACACC- - - - - - - - - - -CCCAGG 9bp del (6/11)
GACACCACCT- - - - - -CCCAGG 5bp del (5/11)
GACACCACCTAAACGCCAGG wt (0/11)
```

Smc3
```
GGTGAT- - - - - - - - - - - - - - - - - - - - - - - - - - -TATTAC 33bp del (9/9)
GGTGATCAAGTCAGCCATCGAGGTGCTCTGACTGGAGGTTATTAC wt (0/9)
```

Nf1
```
ATGCTG- - - - - - - - - - - - - - - - - - - - - - - - - - - - -CCCTAT 36bp del (4/9)
ATGCTGTCCTTCAACAACTCCCTC-ATGTGGCGGCTCATCTGCCCTAT 1bp del (5/9)
ATGCTGTCCTTCAACAACTCCCTCGATGTGGCGGCTCATCTGCCCTAT wt (0/9)
```

Figure 86

Figure 87

FIG. 88A-E

FIG. 89A-C

Figure 90

FIG. 91A-B

WBC

RBC

Spleen weight

| Figure | Donor | Colour |
|---|---|---|
| Figure 77 | C57Bl/6 | |
| Figure 89 | C57Bl/6 | |
| Figure 93 | Flt3-ITD het | |
| Figure 94 | Flt3-ITD het | |
| Figure 95 | Flt3-ITD het | |
| not further analyzed | Flt3-ITD het | |
| not further analyzed | Flt3-ITD-het | |

Figure 92

A)

43.5%  52.9%

sgRNA-Pool

Cas9

GFP-dim

22.4                48.3

Gr1

25.9                3.53

CD11b

GFP-pos

20.6                56

20.1                3.31

B)

C)

*Tet2* GFP dim
AGGTCT- - - - - - - - - - - - - - - - - - - - - - - - - - - - - -CACAAG 33bp del (3/7)
AGGTCTTACTTCCTTA- - - - - - - - - - - - - - - - - - - - - -CACAAG 25bp del/2bp ins (4/7)
AGGTCTTACTTCCTACGGGATGTAGAGCTTGTTCCCGCTCACAAG wt (0/7)

*Tet2* GFP pos
TGCAGG- - - - - - - - - - - - - - - - - - - - - - - - - - - - - -CTCACAAG 41bp del (4/7)
TGCAGGTTTGAGGTCTTACTTCCTTA- - - - - - - - - - - - - - - - - -CACAAG 25bp del/2bp ins (3/7)
TGCAGGTTTGAGGTCTTACTTCCTACGGGATGTAGAGCTTGTTCCCGCTCACAAG wt (0/7)

*Nf1* GFP dim
CCGCTACATGCTGATGCTGTCCTTCAACAACTCCCTCGA- - - - - ATGTGGCGGCTCATCTGCCCTATCTCTT 5bp ins (1/7)
CCGCTACATGCTGATGCTGTCCT- - - - - - - - - - - - - - - - - - - - - - - - - - - - -ATCTCTT 37bp del (2/7)
CCGCTA- - - - - - - - - - - - - - - - - - - - - - - - - - - - - - - - - - - - - -TCTCTT 54bp del(3/7)
CCGCTACATGCTGATGCTGTCCTTCAACAACTCCCTCGA- - - - -TGTGGCGGCTCATCTGCCCTATCTCTT wt (1/7)

*Nf1* GFP pos
CCGCTA- - - - - - - - - - - - - - - - - - - - - - - - - - - - - - - - - - - - - -TCTCTT 54bp del (4/8)
CCGCTACATGCTGATGCTGTCCT- - - - - - - - - - - - - - - - - - - - - - - - - - - - -ATCTCTT 37bp del/(4/8)
CCGCTACATGCTGATGCTGTCCTTCAACAACTCCCTCGATGTGGCGGCTCATCTGCCCTATCTCTT wt (0/8)

*Dnmt3a* GFP dim
-TGGGCA- - - - - - - - - - - - - - - - - - - - - - - - - - -CGTCGGGG 30bp del (1/7)
-TGGGCATGGTGCGGCAACCAGGGAAAGATCATGTACGTCGGGG wt (3/7)
-TGGGCATGGTGCG- - - - - - - - - - - - - - - - - - - - - -TCGGGG 23bp del(3/7)
-TGGGCATGGTGCGGCA- CCAGGGAAAGATCATGTACGTCGGGG wt (0/8)

*Dnmt3a* GFP pos
GGTGCG- - - - - - - - - - - - - - - - - - - - - -TCGGGG 20bp del (1/7)
GGTGCGG- A- CCAGGGAAAGATCATGTACGTCGGGG 1bp ins (2/7)
GGTGCGGCAA CCAGGGAAAGATCATGTACGTCGGGG 1bp del (3/7)
GGTGCGGCA- CCAGGGAAAGATCATGTACGTCGGGG wt (1/7)

*Runx1* GFP dim
GACGTCCCCGGATGG 1bp ins (2/8)
GACGTC- - - -GGATGG 2bp del (4/8)
GACGTCC- -GGATGG 1bp del (2/8)
GACGTCCC-GGATGG wt (0/7)

*Runx1* GFP pos
AGGTGG- - - - - - - - - - - - - - - -ATGGCA 20bp del (2/8)
AGGTGGTGGCACTCGGGGACGTC- -GGATGGCA 2bp del (1/8)
AGGTGGTGGCACTCGGGGACGTCC- CCATGGCA 1bp del (5/8)
AGGTGGTGGCACTCGGGGACGTCCCGGATGGCA| wt (0/7)

*Ezh2* GFP dim
CCTAAACG- -CAGGGG 2bp del(2/7)
CCTAAA- - -CCCAGGGG 2bp del (5/7)
CCTAAACGCCCAGGGG wt (0/7)

*Ezh2* GFP pos
CACCTA- - - - - -CCCAGGGG 4bp del(3/5)
CACCTAAA- -CCCAGGGG 2bp del (2/5)
CACCTAAACGCCCAGGGG wt (0/5)

FIG. 93A-C

A)

B)

C)

Runx1   GGGGACGTCCCCGGATGGCA 1bp ins (8/8)
        GGGGACGT-CCCGGATGGCA wt (0/8)

Nf1     GGCAGATGAGCCGCCACAT-CACGGAGTTGTTGAAGG 1bp del (5/8)
        GGCAGA-----------------------TGAAGG 25bp del(2/8)
        GGCAGATGAGCCGCCACATCGAGGGAGTTGTTGAAGG wt (1/8)

Ezh2    ACTGCT------------------------------------------------TCCGAA 53p del (5/9)
        ACTGCTGAGCGTATAAAGACACCACCTAAACGCCCAGGGGGCCCGCAGAAGAGGAGACTTCCGAA wt (4/9)

FIG. 94A-C

FIG. 95A-C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8454972 B2, Nabel
- US 7250299 B, Naldini
- US 7226780 B, Arya
- US 8404658 B2, Hajjar
- US 8454972 B
- US 8404658 B
- US 5846946 A
- WO 9703211 A
- WO 9639154 A
- US 81573004
- US 20040171156 A1
- US 491026
- WO 2011028929 A
- US 511940 A
- US 4873316 A
- EP 264166 A
- US 6750059 B
- US 092085
- US 7776321 B
- US 20110059502 A
- US 61736465 B
- US 61721283 B
- US 5049386 A
- US 4946787 A
- US 4897355 A
- WO 9117424 A
- WO 9116024 A
- US 4186183 A
- US 4217344 A
- US 4235871 A
- US 4261975 A
- US 4485054 A
- US 4501728 A
- US 4774085 A
- US 4837028 A
- US 9405700 W
- US 4797368 A
- WO 9324641 A
- US 5173414 A
- US 20120295960 A
- US 7303910 B
- US 7351585 B
- US 20060281180 A
- US 20090007284 A
- US 20110117189 A
- US 20090017543 A
- US 20070054961 A
- US 20100317109 A
- US 20110293571 A
- US 20040013648 A
- US 20070025970 A
- US 20090111106 A
- US 7259015 B
- US 20030087817 A
- US 5445934 A
- US 61736527
- US 61748427 B
- US 6603061 B
- US 7868149 B
- US 20090100536 A
- US 61064798
- US 61836127 A
- US 61836101
- US 61835936 B

### Non-patent literature cited in the description

- **KOMAROVA.** Loss- and Gain-of-Function Mutations in Cancer: Mass-action, Spatial and Hierarchical Models. *Journal of Statistical Physics,* July 2007, vol. 128 (1-2), 413-446
- **CONG, L. ; RAN, F.A. ; COX, D. ; LIN, S. ; BARRETTO, R. ; HABIB, N. ; HSU, P.D. ; WU, X. ; JIANG, W. ; MARRAFFINI, L.A.** Multiplex genome engineering using CRISPR/Cas systems. *Science,* 15 February 2013, vol. 339 (6121), 819-23
- **JIANG W. ; BIKARD D. ; COX D. ; ZHANG F ; MARRAFFINI LA.** RNA-guided editing of bacterial genomes using CRISPR-Cas systems. *Nat Biotechnol,* March 2013, vol. 31 (3), 233-9
- **WANG H. ; YANG H. ; SHIVALILA CS. ; DAWLATY MM. ; CHENG AW. ; ZHANG F. ; JAENISCH R.** One-Step Generation of Mice Carrying Mutations in Multiple Genes by CRISPR/Cas-Mediated Genome Engineering. *Cell,* 09 May 2013, vol. 153 (4), 910-8
- **KONERMANN S ; BRIGHAM MD ; TREVINO AE ; HSU PD ; HEIDENREICH M ; CONG L ; PLATT RJ ; SCOTT DA ; CHURCH GM ; ZHANG F.** Optical control of mammalian endogenous transcription and epigenetic states. *Nature,* 22 August 2013, vol. 500 (7463), 472-6

- **RAN, FA. ; HSU, PD. ; LIN, CY. ; GOOTENBERG, JS. ; KONERMANN, S. ; TREVINO, AE. ; SCOTT, DA. ; INOUE, A. ; MATOBA, S. ; ZHANG, Y.** Double Nicking by RNA-Guided CRISPR Cas9 for Enhanced Genome Editing Specificity. *Cell,* 28 August 2013
- **HSU, P. ; SCOTT, D. ; WEINSTEIN, J. ; RAN, FA. ; KONERMANN, S. ; AGARWALA, V. ; LI, Y. ; FINE, E. ; WU, X. ; SHALEM, O.** DNA targeting specificity of RNA-guided Cas9 nucleases. *Nat Biotechnol,* 2013
- **RAN, FA. ; HSU, PD. ; WRIGHT, J. ; AGARWALA, V. ; SCOTT, DA. ; ZHANG, F.** Genome engineering using the CRISPR-Cas9 system. *Nature Protocols,* November 2013, vol. 8 (11), 2281-308
- **SHALEM, O. ; SANJANA, NE. ; HARTENIAN, E. ; SHI, X. ; SCOTT, DA. ; MIKKELSON, T. ; HECKL, D. ; EBERT, BL. ; ROOT, DE. ; DOENCH, JG.** Genome-Scale CRISPR-Cas9 Knockout Screening in Human Cells. *Science,* 12 December 2013
- **NISHIMASU, H. ; RAN, FA. ; HSU, PD. ; KONERMANN, S. ; SHEHATA, SI. ; DOHMAE, N. ; ISHITANI, R. ; ZHANG, F. ; NUREKI, O.** Crystal structure of cas9 in complex with guide RNA and target DNA. *Cell,* 27 February 2014, vol. 156 (5), 935-49
- **WU X. ; SCOTT DA. ; KRIZ AJ. ; CHIU AC. ; HSU PD. ; DADON DB. ; CHENG AW. ; TREVINO AE. ; KONERMANN S. ; CHEN S.** Genome-wide binding of the CRISPR endonuclease Cas9 in mammalian cells. *Nat Biotechnol.*
- **HSU et al.** Development and Applications of CRISPR-Cas9 for Genome Engineering. *Cell,* 05 June 2014, vol. 157, 1262-1278
- **DUMITRACHE et al.** *Genetics,* August 2011, vol. 188 (4), 787-797
- REMINGTON'S PHARMACEUTICAL SCIENCES. Mack Pub. Co, 1991
- **SU X ; FRICKE J ; KAVANAGH DG ; IRVINE DJ.** In vitro and in vivo mRNA delivery using lipid-enveloped pH-responsive polymer nanoparticles. *Mol Pharm.,* 06 June 2011, vol. 8 (3), 774-87
- **MICHAEL S D KORMANN et al.** Expression of therapeutic proteins after delivery of chemically modified mRNA in mice. *Nature Biotechnology,* 09 January 2011, vol. 29, 154-157
- **RAN et al.** *Cell,* 12 September 2013, vol. 154 (6), 1380-9
- **XIA CF ; BOADO RJ ; PARDRIDGE WM.** Antibody-mediated targeting of siRNA via the human insulin receptor using avidin-biotin technology. *Mol Pharm,* May 2009, vol. 6 (3), 747-51
- **ZHANG Y ; SCHLACHETZKI F ; PARDRIDGE WM.** Global non-viral gene transfer to the primate brain following intravenous administration. *Mol Ther.,* January 2003, vol. 7 (1), 11-8
- **CHO, S. ; GOLDBERG, M. ; SON, S. ; XU, Q. ; YANG, F. ; MEI, Y. ; BOGATYREV, S. ; LANGER, R. ; ANDERSON, D.** Lipid-like nanoparticles for small interfering RNA delivery to endothelial cells. *Advanced Functional Materials,* 2010, vol. 19, 3112-3118
- **SCHROEDER, A. ; LEVINS, C. ; CORTEZ, C. ; LANGER, R. ; ANDERSON, D.** Lipid-based nanotherapeutics for siRNA delivery. *Journal of Internal Medicine,* 2010, vol. 267, 9-21
- **EL-ANDALOUSSI S et al.** Exosome-mediated delivery of siRNA in vitro and in vivo. *Nat Protoc.,* December 2012, vol. 7 (12), 2112-26
- **TANGRI S et al.** Rationally engineered therapeutic proteins with reduced immunogenicity. *J Immunol.,* 15 March 2005, vol. 174 (6), 3187-96
- Overview of principles of hybridization and the strategy of nucleic acid probe assay. **TIJSSEN.** Laboratory Techniques In Biochemistry And Molecular Biology-Hybridization With Nucleic Acid Probes. Elsevier, 1993
- **DEVEREUX et al.** Nucleic Acids Research. University of Wisconsin, U.S.A, 1984, vol. 12, 387
- **AUSUBEL et al.** NUCLEIC ACIDS RESEARCH. 1999
- **ATSCHUL et al.** *J. Mol. Biol.,* 1990, 403-410
- **AUSUBEL et al.** *NUCLEIC ACIDS RESEARCH,* 1999, 7-58, 7-60
- **DEVEREUX et al.** *Nuc. Acids Research,* 1984, vol. 12, 387
- **AUSUBEL et al.** *Short Protocols in Molecular Biology,* 1999
- **ALTSCHUL et al.** *J Mol. Biol.,* 1990, 403-410
- **AUSUBEL et al.** *Short Protocols in Molecular Biology,* 1999, 7-58, 7-60
- *FEMS Microbiol Lett.,* 1999, vol. 174 (2), 247-50
- *FEMS Microbiol Lett.,* 1999, vol. 177 (1), 187-8
- **HIGGINS DG ; SHARP PM.** *Gene,* 1988, vol. 73 (1), 237-244
- **LIVINGSTONE C.D. ; BARTON G.J.** Protein sequence alignments: a strategy for the hierarchical analysis of residue conservation. *Comput Appl. Biosci.,* 1993, vol. 9, 745-756
- **TAYLOR W.R.** The classification of amino acid conservation. *J. Theor. Biol.,* 1986, vol. 119, 205-218
- **SIMON RJ et al.** *PNAS,* 1992, vol. 89 (20), 9367-9371
- **HORWELL DC.** *Trends Biotechnol.,* 1995, vol. 13 (4), 132-134
- **SAMBROOK ; FRITSCH ; MANIATIS.** MOLECULAR CLONING: A LABORATORY MANUAL. 1989
- CURRENT PROTOCOLS IN MOLECULAR BIOLOGY. 1987
- PCR 2: A PRACTICAL APPROACH. METHODS IN ENZYMOLOGY. Academic Press, Inc, 1995

- ANTIBODIES, A LABORATORY MANUAL, and ANIMAL CELL CULTURE. 1988
- **GOEDDEL.** GENE EXPRESSION TECHNOLOGY: METHODS IN ENZYMOLOGY. Academic Press, 1990, vol. 185
- **BOSHART et al.** *Cell,* 1985, vol. 41, 521-530
- *Mol. Cell. Biol.,* 1988, vol. 8 (1), 466-472
- *Proc. Natl. Acad. Sci. USA.,* 1981, vol. 78 (3), 1527-31
- **SMITH ; JOHNSON.** Gene. Pharmacia Biotech Inc, 1988, vol. 67, 31-40
- **AMRANN et al.** *Gene,* 1988, vol. 69, 301-315
- **STUDIER et al.** GENE EXPRESSION TECHNOLOGY: METHODS IN ENZYMOLOGY. Academic Press, 1990, vol. 185, 60-89
- **BALDARI et al.** *EMBO J.,* 1987, vol. 6, 229-234
- **KUIJAN ; HERSKOWITZ.** *Cell,* 1982, vol. 30, 933-943
- **SCHULTZ et al.** *Gene,* 1987, vol. 54, 113-123
- **SMITH et al.** *Mol. Cell. Biol.,* 1983, vol. 3, 2156-2165
- **LUCKLOW ; SUMMERS.** *Virology,* 1989, vol. 170, 31-39
- **SEED.** *Nature,* 1987, vol. 329, 840
- **KAUFMAN et al.** *EMBO J.,* 1987, vol. 6, 187-195
- **SAMBROOK et al.** MOLECULAR CLONING: A LABORATORY MANUAL. Cold Spring Harbor Laboratory Press, 1989
- **PINKERT et al.** *Genes Dev.,* 1987, vol. 1, 268-277
- **CALAME ; EATON.** *Adv. Immunol.,* 1988, vol. 43, 235-275
- **WINOTO ; BALTIMORE.** *EMBO J.,* 1989, vol. 8, 729-733
- **BANEIJI et al.** *Cell,* 1983, vol. 33, 729-740
- **QUEEN ; BALTIMORE.** *Cell,* 1983, vol. 33, 741-748
- **BYRNE ; RUDDLE.** *Proc. Natl. Acad. Sci. USA,* 1989, vol. 86, 5473-5477
- **EDLUND et al.** *Science,* 1985, vol. 230, 912-916
- **KESSEL ; GRUSS.** *Science,* 1990, vol. 249, 374-379
- **CAMPES ; TILGHMAN.** *Genes Dev.,* 1989, vol. 3, 537-546
- **ISHINO et al.** *J. Bacteriol.,* 1987, vol. 169, 5429-5433
- **NAKATA et al.** *J. Bacteriol.,* 1989, vol. 171, 3553-3556
- **GROENEN et al.** *Mol. Microbiol.,* 1993, vol. 10, 1057-1065
- **HOE et al.** *Emerg. Infect. Dis.,* 1999, vol. 5, 254-263
- **MASEPOHL et al.** *Biochim. Biophys. Acta,* 1996, vol. 1307, 26-30
- **MOJICA et al.** *Mol. Microbiol.,* 1995, vol. 17, 85-93
- **JANSSEN et al.** *OMICS J. Integ. Biol.,* 2002, vol. 6, 23-33
- **MOJICA et al.** *Mol. Microbiol.,* 2000, vol. 36, 244-246
- **VAN EMBDEN et al.** *J. Bacteriol.,* 2000, vol. 182, 2393-2401
- **JANSEN et al.** *Mol. Microbiol.,* 2002, vol. 43, 1565-1575
- **KARGINOV ; HANNON.** The CRISPR system: small RNA-guided defence in bacteria and archaea. *Mole Cell,* 15 January 2010, vol. 37 (1), 7
- **SAPRANAUSKAS et al.** *Nucleic Acis Research,* 2011, vol. 39, 9275
- **GASIUNAS et al.** *Proc. Natl. Acad. Sci. USA,* vol. 109, E2579
- **NAKAMURA, Y. et al.** Codon usage tabulated from the international DNA sequence databases: status for the year 2000. *Nucl. Acids Res.,* 2000, vol. 28, 292
- **ZUKER ; STIEGLER.** *Nucleic Acids Res.,* 1981, vol. 9, 133-148
- **A.R. GRUBER et al.** *Cell,* 2008, vol. 106 (1), 23-24
- **PA CARR ; GM CHURCH.** *Nature Biotechnology,* 2009, vol. 27 (12), 1151-62
- **ANDERSON.** *Science,* 1992, vol. 256, 808-813
- **NABEL ; FELGNER.** *TIBTECH,* 1993, vol. 11, 211-217
- **MITANI ; CASKEY.** *TIBTECH,* 1993, vol. 11, 162-166
- **DILLON.** *TIBTECH,* 1993, vol. 11, 167-175
- **MILLER.** *Nature,* 1992, vol. 357, 455-460
- **VAN BRUNT.** *Biotechnology,* 1988, vol. 6 (10), 1149-1154
- **VIGNE.** *Restorative Neurology and Neuroscience,* 1995, vol. 8, 35-36
- **KREMER ; PERRICAUDET.** *British Medical Bulletin,* 1995, vol. 51 (1), 31-44
- **HADDADA et al.** Current Topics in Microbiology and Immunology. 1995
- **YU et al.** *Gene Therapy,* 1994, vol. 1, 13-26
- **CRYSTAL.** *Science,* 1995, vol. 270, 404-410
- **BLAESE et al.** *Cancer Gene Ther.,* 1995, vol. 2, 291-297
- **BEHR et al.** *Bioconjugate Chem.,* 1994, vol. 5, 382-389
- **REMY et al.** *Bioconjugate Chem.,* 1994, vol. 5, 647-654
- **GAO et al.** *Gene Therapy,* 1995, vol. 2, 710-722
- **AHMAD et al.** *Cancer Res.,* 1992, vol. 52, 4817-4820
- **BUCHSCHER et al.** *J. Virol.,* 1992, vol. 66, 2731-2739

- **JOHANN et al.** *J. Virol.,* 1992, vol. 66, 1635-1640
- **SOMMNERFELT et al.** *Virol.,* 1990, vol. 176, 58-59
- **WILSON et al.** *J. Virol.,* 1989, vol. 63, 2374-2378
- **MILLER et al.** *J. Virol.,* 1991, vol. 65, 2220-2224
- **WEST et al.** *Virology,* 1987, vol. 160, 38-47
- **KOTIN.** *Human Gene Therapy,* 1994, vol. 5, 793-801
- **MUZYCZKA.** *J. Clin. Invest.,* 1994, vol. 94, 1351
- **TRATSCHIN et al.** *Mol. Cell. Biol.,* 1985, vol. 5, 3251-3260
- **TRATSCHIN et al.** *Mol. Cell. Biol.,* 1984, vol. 4, 2072-2081
- **HERMONAT ; MUZYCZKA.** *PNAS,* 1984, vol. 81, 6466-6470
- **SAMULSKI et al.** *J. Virol.,* 1989, vol. 63, 03822-3828
- **GRIMM, D. et al.** *J. Virol.,* 2008, vol. 82, 5887-5911
- **BALAGAAN.** J Gene Med. Wiley InterScience, 21 November 2005, vol. 8, 275-285
- **BINLEY et al.** *HUMAN GENE THERAPY,* September 2012, vol. 23, 980-991
- **DIGIUSTO et al.** *Sci Transl Med,* 2010, vol. 2, 36ra43
- **SAMBROOK et al.** Nonradioactive In Situ Hybridization Application Manual. 1989
- **CHAN-HUI et al.** *Clinical Immunology,* 2003, vol. 111, 162-174
- **ROBERT D. WELLS ; TETSUO ASHIZAWA.** Genetic Instabilities and Neurological Diseases. Academic Press, 13 October 2011
- **MCIVOR EI ; POLAK U ; NAPIERALA M.** New insights into repeat instability: role of RNA•DNA hybrids. *RNA Biol.,* September 2010, vol. 7 (5), 551-8
- Genetics of Epilepsy and Genetic Epilepsies. Mariani Foundation Paediatric Neurology, vol. 20, 2009
- Genetic Diseases of the Eye. Oxford University Press, 2012
- **MORRELL et al.** Crop genomics:advances and applications. *Nat Rev Genet.,* 29 December 2011, vol. 13 (2), 85-96
- **LAWRENCE et al.** *Nature,* 23 January 2014, vol. 505, 495-503
- **IOANNIDIS et al.** *JNCI,* 16 June 2010, vol. 102 (12
- *TS GENE TUMOR SUPPRESSOR GENE DATABASE,* http://bioinfo.mc.vanderbilt.edu/TSGene/index.html
- **GUSCHIN et al.** *Methods Mol Biol,* 2010, vol. 649, 247
- **GRUBER et al.** *Nucleic Acids Research,* 2008, vol. 36, W70
- **SAPRANAUSAKS et al.** *Nucleic Acids Research,* 2011, vol. 39, 9275
- **GASIUNAS et al.** *Proc. Natl. Acad. Sci. USA,* 2012, vol. 109, E2579
- **JINEK et al.** *Science,* 2012, vol. 337, 816
- **MAKAROVA et al.** *Nat Rev Microbiol,* 2011, vol. 9, 467
- **PLOSKER GL et al.** *Drugs,* 1996, vol. 51 (3), 433-459
- **TRAPANI et al.** *IUBMB Life,* November 2011, vol. 63 (11), 964-971
- *Current Opinion in Drug Discovery & Development,* 2010, vol. 13 (4), 489-496
- **FUCHS et al.** *Oncogene,* 2002, vol. 21 (37), 5716-5724
- **MCMANAMAN JL et al.** *The Journal of Lipid Research,* 12 February 2013
- **TANG J et al.** *Cell Metabolism,* 05 January 2011, vol. 13 (1), 44-56
- **JOAO et al.** *Human Mutation,* 1995, vol. 5 (3), 191-196
- **SHALEK et al.** *Nano Letters,* 2012
- **PARDRIDGE et al.** *Cold Spring Harb Protoc,* 2010
- **TREMBLAY et al.** Transcription Activator-Like Effector Proteins Induce the Expression of the Frataxin Gene. *Human Gene Therapy,* August 2012, vol. 23 (8), 883-890
- Genomic and epigenomic landscapes of adult de novo acute myeloid leukemia. *The New England journal of medicine,* 2013, vol. 368, 2059-2074
- **JINEK, M. et al.** A programmable dual-RNA-guided DNA endonuclease in adaptive bacterial immunity. *Science,* 2012, vol. 337, 816-821
- **CONG, L. et al.** Multiplex genome engineering using CRISPR/Cas systems. *Science,* 2013, vol. 339, 819-823
- **MALI, P. et al.** RNA-guided human genome engineering via Cas9. *Science,* 2013, vol. 339, 823-826
- **BOGDANOVE, A.J. ; VOYTAS, D.F.** TAL effectors: customizable proteins for DNA targeting. *Science,* 2011, vol. 333, 1843-1846
- **REYON, D. et al.** FLASH assembly of TALENs for high-throughput genome editing. *Nature biotechnology,* 2012, vol. 30, 460-465
- **HWANG, W.Y. et al.** Efficient genome editing in zebrafish using a CRISPR-Cas system. *Nature biotechnology,* 2013, vol. 31, 227-229
- **WANG, H. et al.** One-step generation of mice carrying mutations in multiple genes by CRISPR/Cas-mediated genome engineering. *Cell,* 2013, vol. 153, 910-918

- **FRIEDLAND, A.E. et al.** Heritable genome editing in C. elegans via a CRISPR-Cas9 system. *Nature methods,* 2013, vol. 10, 741-743
- **SHAN, Q. et al.** Targeted genome modification of crop plants using a CRISPR-Cas system. *Nature biotechnology,* 2013, vol. 31, 686-688
- **WOOD, A.J. et al.** Targeted genome editing across species using ZFNs and TALENs. *Science,* 2011, vol. 333, 307
- **SANDER, J.D. et al.** Targeted gene disruption in somatic zebrafish cells using engineered TALENS. *Nature biotechnology,* 2011, vol. 29, 697-698
- **HOLKERS, M. et al.** Differential integrity of TALE nuclease genes following adenoviral and lentiviral vector gene transfer into human cells. *Nucleic acids research,* 2013, vol. 41, e63
- **HSU, P.D. et al.** DNA targeting specificity of RNA-guided Cas9 nucleases. *Nature biotechnology,* 2013, vol. 31, 827-832
- **FELLMANN, C. et al.** Functional identification of optimized RNAi triggers using a massively parallel sensor assay. *Molecular cell,* 2011, vol. 41, 733-746
- **LEE, B.H. et al.** FLT3 mutations confer enhanced proliferation and survival properties to multipotent progenitors in a murine model of chronic myelomonocytic leukemia. *Cancer cell,* 2007, vol. 12, 367-380
- **KELLY, L.M. ; GILLILAND, D.G.** Genetics of myeloid leukemias. *Annual review of genomics and human genetics,* 2002, vol. 3, 179-198
- **TANAKA, S. et al.** Ezh2 augments leukemogenicity by reinforcing differentiation blockage in acute myeloid leukemia. *Blood,* 2012, vol. 120, 1107-1117
- **CHALLEN, G.A. et al.** Dnmt3a is essential for hematopoietic stem cell differentiation. *Nature genetics,* 2012, vol. 44, 23-31
- **MORAN-CRUSIO, K. et al.** Tet2 loss leads to increased hematopoietic stem cell self-renewal and myeloid transformation. *Cancer cell,* 2011, vol. 20, 11-24
- **QUIVORON, C. et al.** TET2 inactivation results in pleiotropic hematopoietic abnormalities in mouse and is a recurrent event during human lymphomagenesis. *Cancer cell,* 2011, vol. 20, 25-38
- **MEAD, A.J. et al.** FLT3-ITDs instruct a myeloid differentiation and transformation bias in lymphomyeloid multipotent progenitors. *Cell reports,* 2013, vol. 3, 1766-1776
- **SCHAMBACH, A. et al.** Equal potency of gammaretroviral and lentiviral SIN vectors for expression of O6-methylguanine-DNA methyltransferase in hematopoietic cells. *Molecular therapy : the journal of the American Society of Gene Therapy,* 2006, vol. 13, 391-400
- **KIM, J.H. et al.** High cleavage efficiency of a 2A peptide derived from porcine teschovirus-1 in human cell lines, zebrafish and mice. *PloS one,* 2011, vol. 6, e18556
- **MOROZOVA, K.S. et al.** Far-red fluorescent protein excitable with red lasers for flow cytometry and superresolution STED nanoscopy. *Biophysical journal,* 2010, vol. 99, L13-15
- **FU, Y. et al.** High-frequency off-target mutagenesis induced by CRISPR-Cas nucleases in human cells. *Nature biotechnology,* 2013, vol. 31, 822-826
- **ZHANG, Y. ; TAYLOR, B.R. ; SHANNON, K. ; CLAPP, D.W.** Quantitative effects of Nfl inactivation on in vivo hematopoiesis. *The Journal of clinical investigation,* 2001, vol. 108, 709-715
- **WANG, T. ; WEI, J.J. ; SABATINI, D.M. ; LANDER, E.S.** Genetic screens in human cells using the CRISPR-Cas9 system. *Science,* 2014, vol. 343, 80-84
- **LI, Z. et al.** Murine leukemia induced by retroviral gene marking. *Science,* 2002, vol. 296, 497
- **ZYCHLINSKI, D. et al.** Physiological promoters reduce the genotoxic risk of integrating gene vectors. *Molecular therapy : the journal of the American Society of Gene Therapy,* 2008, vol. 16, 718-725
- **SCHAMBACH, A. et al.** Equal potency of gammaretroviral and lentiviral SIN vectors for expression of 06-methylguanine-DNA methyltransferase in hematopoietic cells. *Molecular therapy : the journal of the American Society of Gene Therapy,* 2006, vol. 13, 391-400
- **DING, Q. et al.** A TALEN genome-editing system for generating human stem cell-based disease models. *Cell Stem Cell,* 2013, vol. 12, 238-251
- **SOLDNER, F. et al.** Generation of isogenic pluripotent stem cells differing exclusively at two early onset Parkinson point mutations. *Cell,* 2011, vol. 146, 318-331
- **CARLSON, D.F. et al.** Efficient TALEN-mediated gene knockout in livestock. *Proc Natl Acad Sci U S A,* 2012, vol. 109, 17382-17387
- **GEURTS, A.M. et al.** Knockout Rats via Embryo Microinjection of Zinc-Finger Nucleases. *Science,* 2009, vol. 325, 433-433
- **TAKASU, Y. et al.** Targeted mutagenesis in the silkworm Bombyx mori using zinc finger nuclease mRNA injection. *Insect Biochem Molec,* 2010, vol. 40, 759-765
- **WATANABE, T. et al.** Non-transgenic genome modifications in a hemimetabolous insect using zinc-finger and TAL effector nucleases. *Nat Commun,* 2012, vol. 3
- **PORTEUS, M.H. ; BALTIMORE, D.** Chimeric nucleases stimulate gene targeting in human cells. *Science,* 2003, vol. 300, 763
- **MILLER, J.C. et al.** An improved zinc-finger nuclease architecture for highly specific genome editing. *Nat Biotechnol,* 2007, vol. 25, 778-785

- **SANDER, J.D. et al.** Selection-free zinc-finger-nuclease engineering by context-dependent assembly (CoDA). *Nat Methods,* 2011, vol. 8, 67-69
- **CHRISTIAN, M. et al.** Targeting DNA double-strand breaks with TAL effector nucleases. *Genetics,* 2010, vol. 186, 757-761
- **ZHANG, F. et al.** Efficient construction of sequence-specific TAL effectors for modulating mammalian transcription. *Nat Biotechnol,* 2011, vol. 29, 149-153
- **MILLER, J.C. et al.** A TALE nuclease architecture for efficient genome editing. *Nat Biotechnol,* 2011, vol. 29, 143-148
- **REYON, D. et al.** FLASH assembly of TALENS for high-throughput genome editing. *Nat Biotechnol,* 2012, vol. 30, 460-465
- **BOCH, J. et al.** Breaking the code of DNA binding specificity of TAL-type III effectors. *Science,* 2009, vol. 326, 1509-1512
- **MOSCOU, M.J. ; BOGDANOVE, A.J.** A simple cipher governs DNA recognition by TAL effectors. *Science,* 2009, vol. 326, 1501
- **SANJANA, N.E. et al.** A transcription activator-like effector toolbox for genome engineering. *Nat Protoc,* 2012, vol. 7, 171-192
- **DEVEAU, H. ; GARNEAU, J.E. ; MOINEAU, S.** CRISPR-Cas system and its role in phage-bacteria interactions. *Annu Rev Microbiol,* 2010, vol. 64, 475-493
- **HORVATH, P. ; BARRANGOU, R.** CRISPR-Cas, the immune system of bacteria and archaea. *Science,* 2010, vol. 327, 167-170
- **MAKAROVA, K.S. et al.** Evolution and classification of the CRISPR-Cas systems. *Nat Rev Microbiol,* 2011, vol. 9, 467-477
- **BHAYA, D. ; DAVISON, M. ; BARRANGOU, R.** CRISPR-Cas systems in bacteria and archaea: versatile small RNAs for adaptive defense and regulation. *Annu Rev Genet,* 2011, vol. 45, 273-297
- **CONG, L. et al.** Multiplex genome engineering using CRISPR-Cas systems. *Science,* 2013, vol. 339, 819-823
- **JINEK, M. et al.** RNA-programmed genome editing in human cells. *eLife,* 2013, vol. 2, e00471
- **CHO, S.W. ; KIM, S. ; KIM, J.M. ; KIM, J.S.** Targeted genome engineering in human cells with the Cas9 RNA-guided endonuclease. *Nat Biotechnol,* 2013, vol. 31, 230-232
- **GARNEAU, J.E. et al.** The CRISPR-Cas bacterial immune system cleaves bacteriophage and plasmid DNA. *Nature,* 2010, vol. 468, 67-71
- **GASIUNAS, G. ; BARRANGOU, R. ; HORVATH, P. ; SIKSNYS, V.** Cas9-crRNA ribonucleoprotein complex mediates specific DNA cleavage for adaptive immunity in bacteria. *Proc Natl Acad Sci U S A,* 2012, vol. 109, E2579-2586
- **URNOV, F.D. ; REBAR, E.J. ; HOLMES, M.C. ; ZHANG, H.S. ; GREGORY, P.D.** Genome editing with engineered zinc finger nucleases. *Nat Rev Genet,* 2010, vol. 11, 636-646
- **HSU, P.D. ; ZHANG, F.** Dissecting neural function using targeted genome engineering technologies. *ACS Chem Neurosci,* 2012, vol. 3, 603-610
- **PEREZ, E.E. et al.** Establishment of HIV-1 resistance in CD4(+) T cells by genome editing using zinc-finger nucleases. *Nat Biotechnol,* 2008, vol. 26, 808-816
- **CONG, L. et al.** Multiplex Genome Engineering Using CRISPR-Cas Systems. *Science,* 2013, vol. 339, 819-823
- **CHEN, F.Q. et al.** High-frequency genome editing using ssDNA oligonucleotides with zinc-finger nucleases. *Nat Methods,* 2011, vol. 8, 753-U796
- **BEDELL, V.M. et al.** In vivo genome editing using a high-efficiency TALEN system. *Nature,* 2012, vol. 491, 114-U133
- **SALEH-GOHARI, N. ; HELLEDAY, T.** Conservative homologous recombination preferentially repairs DNA double-strand breaks in the S phase of the cell cycle in human cells. *Nucleic Acids Res,* 2004, vol. 32, 3683-3688
- **DELTCHEVA, E. et al.** CRISPR RNA maturation by trans-encoded small RNA and host factor RNase III. *Nature,* 2011, vol. 471, 602-607
- **SAPRANAUSKAS, R. et al.** The Streptococcus thermophilus CRISPR-Cas system provides immunity in Escherichia coli. *Nucleic Acids Res,* 2011, vol. 39, 9275-9282
- **HWANG, W.Y. et al.** Efficient genome editing in zebrafish using a CRISPR-Cas system. *Nat Biotechnol,* 2013, vol. 31, 227-229
- **WANG, H. et al.** One-Step Generation of Mice Carrying Mutations in Multiple Genes by CRISPR-Cas-Mediated Genome Engineering. *Cell,* 2013, vol. 153, 910-918
- **SHEN, B. et al.** Generation of gene-modified mice via Cas9/RNA-mediated gene targeting. *Cell Res,* 2013, vol. 23, 720-723
- **QI, L.S. et al.** Repurposing CRISPR as an RNA-guided platform for sequence-specific control of gene expression. *Cell,* 2013, vol. 152, 1173-1183
- **TUSCHL, T.** Expanding small RNA interference. *Nat Biotechnol,* 2002, vol. 20, 446-448
- **SMITHIES, O. ; GREGG, R.G. ; BOGGS, S.S. ; KORALEWSKI, M.A. ; KUCHERLAPATI, R.S.** Insertion of DNA sequences into the human chromosomal beta-globin locus by homologous recombination. *Nature,* 1985, vol. 317, 230-234
- **THOMAS, K.R. ; FOLGER, K.R. ; CAPECCHI, M.R.** High frequency targeting of genes to specific sites in the mammalian genome. *Cell,* 1986, vol. 44, 419-428

- **HASTY, P. ; RIVERA-PEREZ, J. ; BRADLEY, A.** The length of homology required for gene targeting in embryonic stem cells. *Mol Cell Biol,* 1991, vol. 11, 5586-5591
- **WU, S. ; YING, G.X. ; WU, Q. ; CAPECCHI, M.R.** A protocol for constructing gene targeting vectors: generating knockout mice for the cadherin family and beyond. *Nat Protoc,* 2008, vol. 3, 1056-1076
- **GUSCHIN, D.Y. et al.** A rapid and general assay for monitoring endogenous gene modification. *Methods Mol Biol,* 2010, vol. 649, 247-256
- **OLIVEIRA, T.Y. et al.** Translocation capture sequencing: a method for high throughput mapping of chromosomal rearrangements. *J Immunol Methods,* 2012, vol. 375, 176-181
- **BOGENHAGEN, D.F. ; BROWN, D.D.** Nucleotide sequences in Xenopus 5S DNA required for transcription termination. *Cell,* 1981, vol. 24, 261-270
- **BULTMANN, S. et al.** Targeted transcriptional activation of silent oct4 pluripotency gene by combining designer TALEs and inhibition of epigenetic modifiers. *Nucleic Acids Res,* 2012, vol. 40, 5368-5377
- **VALTON, J. et al.** Overcoming transcription activator-like effector (TALE) DNA binding domain sensitivity to cytosine methylation. *J Biol Chem,* 2012, vol. 287, 38427-38432
- **MUSSOLINO, C. et al.** A novel TALE nuclease scaffold enables high genome editing activity in combination with low toxicity. *Nucleic acids research,* 2011, vol. 39, 9283-9293
- **BOBIS-WOZOWICZ, S. ; OSIAK, A. ; RAHMAN, S.H. ; CATHOMEN, T.** Targeted genome editing in pluripotent stem cells using zinc-finger nucleases. *Methods,* 2011, vol. 53, 339-346
- **JIANG, W. ; BIKARD, D. ; COX, D. ; ZHANG, F. ; MARRAFFINI, L.A.** RNA-guided editing of bacterial genomes using CRISPR-Cas systems. *Nat Biotechnol,* 2013, vol. 31, 233-239
- **MICHAELIS, L.M. ; MAUD.** Die kinetik der invertinwirkung. *Biochem.,* 1913
- **MAHFOUZ, M.M. et al.** De novo-engineered transcription activator-like effector (TALE) hybrid nuclease with novel DNA binding specificity creates double-strand breaks. *Proc Natl Acad Sci U S A,* 2011, vol. 108, 2623-2628
- **WILSON, E.B.** Probable inference, the law of succession, and statistical inference. *J Am Stat Assoc,* 1927, vol. 22, 209-212
- **LOPES, V.S.** Retinal gene therapy with a large MYO7A cDNA using adeno-assocaited virus. *Gene Ther,* 24 January 2013
- **KAPLITT, M.G. et al.** Safety and tolerability of gene therapy with an adeno-associated virus (AAV) borne GAD gene for Parkinson's disease: an open label, phase I trial. *Lancet,* 23 June 2007, vol. 369 (9579), 2097-105
- **NATHWANI, A.C. et al.** Adenovirus-associated virus vector-mediated gene transfer in hemophilia B. *N Engl J Med.,* 22 December 2011, vol. 365 (25), 2357-65
- **GRAY SJ ; FOTI SB ; SCHWARTZ JW ; BACHABOINA L ; TAYLOR-BLAKE B ; COLEMAN J ; EHLERS MD ; ZYLKA MJ ; MCCOWN TJ ; SAMULSKI RJ.** Optimizing promoters for recombinant adeno-associated virus-mediated gene expression in the peripheral and central nervous system using self-complementary vectors. *Hum Gene Ther.,* September 2011, vol. 22 (9), 1143-53
- **LIU D ; FISCHER I.** Two alternative promoters direct neuron-specific expression of the rat microtubule-associated protein 1B gene. *J Neurosci.,* 15 August 1996, vol. 16 (16), 5026-36
- **LEVITT N. ; BRIGGS D. ; GIL A. ; PROUDFOOT N.J.** Definition of an efficient synthetic poly(A) site. *Genes Dev.,* 1989, vol. 3, 1019-1025
- **MCCLURE C ; COLE KL ; WULFF P ; KLUGMANN M ; MURRAY AJ.** Production and titering of recombinant adeno-associated viral vectors. *J Vis Exp.,* 27 November 2011, vol. 57, e3348
- **BANKER G ; GOSLIN K.** Developments in neuronal cell culture. *Nature,* 10 November 1988, vol. 336 (6195), 185-6